# EUROPEAN PATENT APPLICATION

(11) **EP 2 270 034 A2**
(43) Date of publication of application: **05.01.2011**
(21) Application number: 10012173.0
(22) Date of filing: 03.06.2005
(51) Int. Cl.: C07K 14/47

(54) **Agents and methods for diagnosing stress**

(30) Priority: 03.06.2004 US 576285 P; 04.06.2004 AU 2004903003
(62) Divisional of application: 05746841.5
(71) Applicant: Athlomics Pty Ltd, Toowong, QLD 4066 (AU)
(72) Inventor: Brandon, Richard Bruce, Boonah Queensland 4310 (AU); Thomas, Mervyn Rees, Chapel Hill Queensland 4169 (AU)
(74) Representative: Dempster, Robert Charles

(57) **Abstract**

The present invention discloses molecules and assay for qualitatively or quantitatively determining the effect of stress on the immune system, the susceptibility to developing disease or illness through immune system dysfunction as a result of stress, and for monitoring the ability of an animal to cope with stress. The invention is useful *inter alia* in measuring response to immunomodulatory therapies, and monitoring the immune response to natural disease under stressful conditions.

## Description

### FIELD OF THE INVENTION

This invention relates generally to methods and agents for determining the status of the immune system. More particularly, the present invention relates to molecules and assays for qualitatively or quantitatively determining the effect of stress on the immune system, the susceptibility to developing disease or illness through immune system dysfunction as a result of stress, and for monitoring the ability of an animal to cope with stress. The invention is useful *inter alia* in measuring response to immunomodulatory therapies, and monitoring the immune response to natural disease under stressful conditions. In certain embodiments, the invention is useful for monitoring animals in athletic training, for measuring the effects of aging on ability to respond to stress and external stressors, and for enabling better treatment and management decisions to be made in animals at risk of exposure to disease, or susceptible to disease through the effects of stress.

### BACKGROUND OF THE INVENTION

The immune system functions to protect an organism from foreign invasion and insults. The host immune system of mammals can be functionally divided into adaptive and innate components. Innate immunity is often the first line of defense to external insults and consists of natural barriers, such as keratinous surfaces, secretions and chemicals, for example skin, mucous, lysozyme and acute phase proteins. The innate immune system can be found in most organisms, is non-specific, and many defense molecules that are part of the innate immune system are evolutionally conserved across a broad range of species (e.g., complement components appear early in evolution in invertebrates).

The adaptive immune system produces a specific response and "remembers" an infectious or invading agent to enable the host to engender an anamnestic response upon a subsequent challenge. The adaptive immune system can also be functionally divided intro humoral and cellular components. The humoral component consists of soluble factors, and-in mammals this consists of antibodies. Cells of the immune system of higher organisms consist of the lymphoid or myeloid lines. Lymphoid cells differentiate in the thymus (T cells) or bone marrow (B cells). B cells and T cells are morphologically identical. Myeloid cells are phagocytes and other cells such as platelets and mast cells. Phagocytes are either monocytes or polymorphonuclear cells. [For a general review of the immune system and its cellular and humoral components, see "Essential Immunology," 10th edition, Roitt and Delves, Blackwell Publishing 2001; and "Immunobiology. The Immune system in Health and Disease," 4th edition, Janeway et al., Garland Publishing 1999].

Functional studies of the immune system of mammals constitute a vast body of literature and there are numerous tests available to measure the functional capabilities of the immune system. The humoral immune system is more amenable to functional testing as compared to the cellular immune system. For example, antibodies bind specifically to their target molecules and can be measured directly in tests such as antibody diffusion and precipitation assays, enzyme-linked immunosorbent assays, or used to detect the presence of invading organisms in antigen capture assays.

The function of the cellular immune system is more difficult to measure and often involves simple counting of the numbers of various subpopulations of cells using stains or specific antibodies to cell surface proteins. For example, one of the most common blood tests in medicine is a complete blood count (CBC) that measures red and white blood cell and platelet numbers. A differential white cell count uses Wright stain to enable the enumeration of lymphocytes, neutrophils, basophils, eosinophils and monocytes. Infection with bacteria often results in increased numbers of neutrophils in peripheral blood samples, and parasitic infections often results in increased numbers of eosinophils. However, counting the numbers of white blood cell types in a peripheral blood sample is often a poor indicator of the functional capabilities of the immune system, it is non-specific (not capable of determining the nature of infection or insult) and lymphocytes of the B and T cell lineage cannot be distinguished.

B lymphocytes produce antibodies and T lymphocytes are one of the main regulators and effectors of the immune system. Various subpopulations of B and T cells can be distinguished on the basis of different proteins (markers) on their cell surface. B cells express immunoglobulin (antibody) proteins on their cell surface and T cells express various markers depending upon their stage of development and function. Many different reagents (often antibodies) have been developed to differentiate subpopulations of T and B cells in humans and experimental animal species and many can be bought commercially from companies such as Alexis Corporation (www.alexis-corp.com). Again, simply counting the numbers of B and T cells (including subpopulations) is not informative on the functional capabilities of the cells. The preparation of reagents for detecting cell surface markers is also laborious and a highly specialized activity.

There are more direct methods of measuring immune cell function, including; plaque forming, chemotaxis, random migration, superoxide anion release, concentration of ATP in circulating CD4⁺ cells following *in vitro* stimulation with phytohemagglutinin, and release of fluorescent dye from target cells assays. Many of these tests are laborious, require prior cell preparation and purification methods (often affecting the results of subsequent assays), and only measure the function of one particular subset of cells.

In summary, there currently exists a need for more effective modalities for measuring the functional capabilities of the immune system, and particularly the cellular immune system.

Athletic performance animals are unable to communicate their well-being to human owners or trainers. In addition, human athletes are often unaware of their well-being (due to heavy training) or are unable to communicate this effectively to trainers or medical practitioners. Therefore, there is also a need for more effective methods for monitoring the functional capabilities of the cellular immune system, especially in athletic performance animals.

It is almost 70 years since it was first recognized that stress can activate a physiological response that may be beneficial or damaging to the body (Seyle H. 1936, Nature 138:32). Stress is a physical, chemical or emotional factor that causes bodily or mental tension and may be a factor in disease causation. A publication by Pedersen et al. (1994, Inter J. Sports Med. 15:5116-5121) provides a review of work conducted in the area of stress and disease.

In recent years, rapid advances in the field of immunology have generated intense interest in the interaction between stress induced by psychosocial, nutritional and physical factors and the immune system. A major premise of this work is that stress may enhance vulnerability to disease by exerting an immunosuppressive effect. This may especially be true of diseases intimately connected with immunologic mechanisms such as infection, malignancy and autoimmune disease.

Studies demonstrating immune alterations in stress encompass a number of models in which most types of experimental and naturally occurring stresses have been associated with alteration of the components of the immune system. Some of the earliest work was conducted by the United States National Aeronautic Space Administration (NASA). The NASA studies showed that white blood cells and T-lymphocytes were elevated during the splash-down phase of space flight. However, there was impairment in the lymphoproliferative response to mitogenic stimulation during the first three (3) days after return to earth. A slight decrease in the stimulation response of lymphocytes was also observed prior to launch, possibly due to anticipation. A general overview ofstress and immune function can be found in "Stress, Immunity and Illness--A Review", authored by Dorian and Garfinkel, Psychological Medicine, 17:393-407 (1987).

Physical activity and exercise are also known to produce a variety of alterations to the immune system. The effects of vigorous exercise appear to depress immune function and may compromise host defenses against upper respiratory tract infections. Epidemiological studies have generally shown a greater risk of upper respiratory tract infections with vigorous levels of exercise. See Heath et al., 1992 Sports Medicine 14(6) 353-365.

In addition to physical activity and exercise, stress can be evinced by external factors such as trauma (physical), major life events, physical health status and lifestyle. The way in which these external factors are perceived and the way in which the body adjusts influence the ultimate physiological response. The body's response to stress is handled by an allostatic system (adaptive) consisting primarily of the sympathetic nervous system and the hypothalamic, pituitary, adrenal axis (HPA axis) (McEwen B. 1998, New England Journal of Medicine, 338:171-179). The term "allostatic load" refers to the amount of physiological response resulting from the balance between the initiation of a complex response and the shutting down of this response. Allostatic load can result from frequent stress, lack of adaptation to stress, inability to turn off an allostatic response, and lack of allostatic response in one system resulting in an increased response in another.

There is strong evidence to suggest that allostatic load leads to increased susceptibility to disease, risk of contracting disease and increased disease incidence. For example, stress induced increases in blood pressure can trigger myocardial infarction in humans and atherosclerosis in primates (Muller et al., 1989 Circulation 79:733-743, and Kaplan et al., 1991 Circulation, 84 Suppl VI:VI-23-VI-32,). Intense athletic training increases allostatic load resulting in weight loss, amenorrhoea and anorexia nervosa (Boyar et al., 1977 New Engl J Med. 296:190-193, and Loucks et al., 1989 J Clin. Endocrinol. Metabol. 68:402-411). Repeated social defeat (stressor) in mice is associated with (amongst other findings) increased plasma concentrations of corticosterone, which is a known immunosuppressant (Stark et al., Am. J. Physiol. Regul. Integrr. Comp. Physiol. 280: R1799-R1805). Age is correlated with the ability to turn off the HPA axis, and prolonged stimulation of physiological systems through the HPA axis can result in hippocampus damage and consequent cognitive deficits (Lupien et al., 1994 J Neurosci. 14:2893-2903). In Lewis rats, genetically determined to have hyporesponsiveness of the HPA axis, increased inflammatory responses result in an increased incidence of autoimmune and inflammatory disturbances (Sternburg et al., 1989 Proc. Natl. Acad. Sci (USA) 86: 4771-4775). Low HPA responsiveness is also considered to be involved in human fibromyalgia (Crofford et al., 1994 Arthritis Rheum. 37:1583-1592), chronic fatigue syndrome (Poteliakhoff A. 1981 J Psychosom. Res. 25:91-95), infant atopic dermatitis (Buske-Kirschbaum et al., 1997 Psychosom med. 59:419-426) and post-traumatic stress disorder *(*Yehuda et al., 1991 Bio. Psychiatry 30:1031-1048).

Approximating allostatic load has been attempted by using measures of metabolic and cardiovascular physiology including, systolic blood pressure, overnight urinary cortisol and catecholamine excretion, ratio of waist to hip measurement, glycosylated hemoglobin value, ratio of serum high density lipoprotein in the total serum cholesterol concentration, serum concentration of dehydroepiandrosterone sulfate, and serum concentration of high density lipoprotein cholesterol. Patients with a lower allostatic score from measuring these parameters had higher physical and mental functioning and a lower incidence of cardiovascular disease, hypertension and diabetes (Seeman et al., 1997 Arch. Intern. Med. 157:2259-2268). High serum fibrinogen concentrations have also been correlated to increased risk of coronary heart disease (Markowe et al., 1985 British Med. J. 291:1312-1314). In addition it has been noted that stress induces atrophy of the pyramidal neurones in the CA3 region of the hippocampus that can be detected using magnetic resonance imaging (Sapolsky R. M. 1996 Science 273:749-750.). These measures require multiple separate assays, are expensive and often laborious, and only provide an approximation of allostatic load.

In summary there is a need for more effective processes for measuring allostatic load.

It is well known that stress affects the immune system (Hawkley and Cacioppo, 2004 Brain Behav. Imm. 18:114-119; Engler et al., 2004 J Neuroimm. 148:106-115*;* Woods et al., 2003 Brain Behav. Imm. 17: 384-392; Mars et al., 1998 Biochem Biophys. Res. Comm. 249: 366-370; Bierhaus et al., 2003 Proc. Natl. Acad. Sci (USA) 100(4):1920-1925; Horohov et al., 1996 Vet Immunol. Immunopath. 53:221-233). Stress acts on the immune system mainly through the sympathetic nervous system and HPA axis causing the release of catecholamines, corticotrophin and cortisol (an example of a steroid). These molecules have known immunomodulatory effects but their mechanism of action is not fully understood. For example, glucocorticoids (steroids) such as cortisol bind to steroid receptors on the outside of cells and are then transported directly to the cell nucleus. Once inside the nucleus, steroid hormones can modulate gene expression, and hence immune function, through steroid responsive elements upstream of gene coding regions (Geng and Vedeckis, 2004 Mol. Endocrinol. 18(4):912-924). For the purposes of its effects on the immune system, stress can be classified into acute (once over a period of less than say two days) and chronic forms (persistent stress over a period of several days or months). Acute stress has been demonstrated to enhance the immune system by redistributing white blood cells from blood to various body compartments such as the skin, lymph nodes and bone marrow (Dhabhar et al., 1995 J. Immunol. 154:5511-5527) the effect of which, is partly due to release of endogenous glucocorticoids. The affect of acute stress has been reported to last for 3-5 days (Dhabhar et al., 1996 J Immunol. 157:1638-1644.). On the other hand, chronic stress elicits the HPA axis and the autonomic nervous system and reduces cellular immune responses and increases susceptibility to disease (McEwen et al., 1997 Brain Res. Rev. 23:79-113; Cohen et al., 1992 Psychol. Sci. 3:301-304; Cohen et al., 1993 JAMA, 277:1940-1944; Peijie et al., 2003 Life Sciences 72:2255-2262).

In summary, there is a need for better modalities for measuring and monitoring the effects of allostatic load on the function of the immune system.

### SUMMARY OF THE INVENTION

The present invention represents a significant advance over current technologies for quantifying allostatic load and for measuring and monitoring immune function. It is predicated in part on measuring the level of certain functional markers in cells, especially circulating leukocytes, of the host. More particularly, the present invention relates to molecules and assays, which are useful in screening and monitoring animals for the presence or risk of developing disease or illness through immune system dysfunction as a result of stress, in determining the ability of an animal to cope with, or adapt to, external stressors, and in monitoring immune function when administering immune-modulating drugs. The invention has practical use in monitoring animals under stress, especially those in athletic training, in measuring the effects of aging on the ability to respond to external stressors, and in enabling better treatment and management decisions to be made in animals at risk of exposure to disease, or susceptible to disease through the effects of stress. In certain embodiments, the invention has practical applications in measuring the response to vaccination or immune-modifying therapies, for example, in animals under stress, which may not develop an appropriate protective response to vaccination or therapy. In other embodiments, the invention has practical use in monitoring the immune response to natural disease when an animal is subject to stressful conditions or at risk due to inappropriate response to stress. This represents a significant and unexpected advance in the screening, monitoring and management of animals under stress.

Thus, the present invention addresses the problem of detecting the presence, absence or degree of a physiological stress response or of assessing well being including the function of the immune system by detecting, for example, a differential gene expression pattern that may be measured in host cells. Advantageous embodiments involve monitoring the expression of certain genes in peripheral leukocytes of the immune system, which may be reflected in changing patterns of RNA levels or protein production that correlate with allostatic stress load or with an immune-modulating event.

Accordingly, in one aspect, the present invention provides methods for determining the presence or degree of a physiological response to stress or a related condition in a test subject. These methods generally comprise detecting in the subject aberrant expression of at least one gene (also referred to herein as a "stress marker gene") selected from the group consisting of: (a) a gene comprising a nucleotide sequence that shares at least 50% (and at least 51 % to at least 99% and all integer percentages in between) sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof; (b) a gene comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a gene comprising a nucleotide sequence that encodes a polypeptide that shares at least 50% (and at least 51% to at least 99% and all integer percentages in between) sequence similarity with at least a portion of the sequence set forth in SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 15 contiguous amino acid residues of that sequence; and (d) a gene comprising a nucleotide sequence that hybridizes to the sequence of (a), (b), (c) or a complement thereof, under at least low, medium or high stringency conditions. In accordance with the present invention, these stress marker genes are aberrantly expressed in animals with a physiological response to stress or with an allostatic load. Suitably, the related condition is immunodepression.

Suitably, the-presence of the physiological response to stress or related condition is associated with psychological stress or physical stress (e.g., physical duress such as athletic training and physical trauma). Illustrative psychological conditions include depression, generalized anxiety disorder, post traumatic stress disorder, panic, chronic fatigue, myalgic encephalopathy, stress through restraint, sleep deprivation, overeating and behavioral (operant) conditioning. Other psychological conditions, especially relating to veterinary applications, include, but are not limited to, stress related to confinement, sheering, shipping or human-animal interaction. Illustrative examples of physical stress include physical duress such as athletic training and physical trauma.

As used herein, polynucleotide expression products of stress marker genes are referred to as "stress marker polynucleotides." Polypeptide expression products of the stress marker genes are referred to herein as "stress marker polypeptides."

Thus, in some embodiments, the methods comprise detecting aberrant expression of a stress marker polynucleotide selected from the group consisting of (a) a polynucleotide comprising a nucleotide sequence that shares at least 50% (and at least 51 % to at least 99% and all integer percentages in between) sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139; 141, 143, 144, 145, 147, 148, 150, 151, 153, 155, 156, 158, 160; 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof; (b) a polynucleotide comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100; 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a polynucleotide comprising a nucleotide sequence that encodes a polypeptide that shares at least 50% (and at least 51 % to at least 99% and all integer percentages in between) sequence similarity with at least a portion of the sequence set forth in SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106,110, 112, 114, 116, 120, 122, 124, 128, 132, 134, 136,138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 2151, 217, 219, 221, 223, 225, 227, 229, 231, 235, 237, 245, 247 or 249, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.

In other embodiments, the methods comprise detecting aberrant expression of a stress marker polypeptide selected from the group consisting of: (i) a polypeptide comprising an amino acid sequence that shares at least 50% (and at least 51% to at least 99% and all integer percentages in between) sequence similarity with the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (ii) a polypeptide comprising a portion of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 5 contiguous amino acid residues of that sequence; (iii) a polypeptide comprising an amino acid sequence that shares at least 30% (and at least 31% to at least 99% and all integer percentages in between) similarity with at least 15 contiguous amino acid residues of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 2-03, 205, 207, 209; 211, 213, 215, 217, 219, 221, 223, 225, 227, 229, 231, 235, 237, 245, 247 or 249; and (iv) a polypeptide comprising a portion of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 5 contiguous amino acid residues of that sequence and is immuno-interactive with an antigen-binding molecule that is immune-interactive with a sequence of (i), (ii) or (iii).

Typically, aberrant expression of a stress marker gene is detected by: (1) measuring in a biological sample obtained from the subjects the level or functional activity of an expression product of at least one stress marker gene and (2) comparing the measured level or functional-activity of each expression product to the level or functional activity of a corresponding expression product in a reference sample obtained from one or more normal subjects or from one or more subjects not under stress, wherein a difference in the level or functional activity of the expression product in the biological sample as compared to the level or functional activity of the corresponding expression product in the reference sample is indicative of the presence-of a physiological response to stress. In some embodiments, the method further comprises determining the degree of stress response (or stress level) or the degree of immunomodulation when the measured level or functional activity of the or each expression product is different than the measured level or functional activity of the or each corresponding expression product. In these embodiments, the difference typically represents an at least about 10%, 20%, 30%, 40%, 50%, 60%, 70%, 80% or 90%, or even an at least about 100%, 200%, 300%, 400%, 500%, 600%, 700%, 800%, 900% or 1000% increase, or an at least about 10%, 20%, 30% 40%, 50%, 60%, 70%, 80%, 90%, 92%, 94%, 96%, 97%, 98% or 99%, or even an at least about 99.5%, 99.9%, 99.95%, 99.99%, 99.995% or 99.999% decrease in the level or functional activity of an individual expression product as compared to the level or function activity of an individual corresponding expression product. , which is hereafter referred to as "aberrant expression." In illustrative examples of this type, the presence of a physiological response to stress is determined by detecting a decrease in the level or functional activity of at least one stress marker polynucleotide selected from (a) a polynucleotide comprising a nucleotide sequence that shares at least 50% (and at least 51% to at least 99% and all integer percentages in between) sequence identity with the sequence set forth in any one of SEQ ID NO: 1, 3, 4, 7, 9; 11, 19, 21, 24, 25, 33, 34, 38, 39, 40, 41, 42, 50, 51, 56, 57, 59, 62, 63, 66, 70, 71, 73, 75, 79, 81, 83, 89, 90, 91., 92, 93, 97, 99, 105, 107, 108, 111, 119, 121, 122, 123, 129, 130, 137, 139, 140, 141, 142, 143 or 185, or a complement thereof; (b) a polynucleotide comprising a nucleotide sequence that encodes a polypeptide comprising the amino acid sequence set forth in any one of SEQ ID NO: 2, 8, 10, 12, 20, 22, 43, 58, 60, 67, 71, 72, 74, 76, 80, 82, 84, 94, 98, 100, 106, 112, 120, 122, 123, 124 or 138; (c) a polynucleotide comprising a nucleotide sequence that encodes a polypeptide that shares at least 50% (and at least 51 % to at least 99% and all integer percentages in between) sequence similarity with at least a portion of the sequence set forth in SEQ ID NO: 2, 8, 10, 12, 20, 22, 43, 58, 60, 67, 71, 72, 74, 76, 80, 82, 84, 94, 98, 100, 106, 112, 120, 122, 123, 124 or 138, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In other illustrative examples, the presence of a physiological response to stress is determined by detecting an increase in the level or functional activity of at least one stress marker polynucleotide selected from (a) a polynucleotide comprising a nucleotide sequence that shares at least 50% (and at least 51% to at least 99% and all integer percentages in between) sequence identity with the sequence set forth in any one of SEQ ID NO:5, 13, 15, 16, 17, 23, 26, 28, 29, 30, 32, 35, 37, 44, 46, 48, 52, 54, 55, 64, 68, 77, 85, 87, 95, 96, 101, 103, 113, 115, 117, 118, 125, 126, 131, 133, 135, 144, 145, 147, 148, 150, 151, 153, 155, 156, 158, 160, 161, 163, 164, 165, 167, 169, 170, 171, 173, 175, 176, 178, 180, 182, 183, 184, 186, 187, 188, 190, 192, 194, 195, 196, 198, 200, 202, 204, 206 or 210, or a complement thereof; (b) a polynucleotide comprising a nucleotide sequence that encodes a polypeptide comprising the amino acid sequence set forth in any one of SEQ ID NO: 6, 14, 18, 27, 31, 36, 45, 47, 49, 53, 65, 69, 78, 86, 88, 102, 104, 114, 116, 132, 134, 136, 146, 149, 152, 154, 157, 159, 162, 166, 168, 172, 174, 177, 179, 181, 189, 191, 193, 197, 199, 201, 203, 205, 207 or 211; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 6, 14, 18, 27, 31, 36, 45, 47, 49, 53, 65, 69, 78, 86, 88, 102, 104, 114, 116, 132, 134, 136, 146, 149, 152, 154, 157, 159, 162, 166, 168, 172, 174, 177, 179, 181, 189, 191, 193, 197, 199, 201, 203, 205, 207 or 211, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In some embodiments, the method further comprises determining the absence of a physiological response to stress when the measured level or functional activity of the or each expression product is the same as or similar to the measured level or functional activity of the or each corresponding expression product. In these embodiments, the measured level or functional activity of an individual expression product varies from the measured level or functional activity of an individual corresponding expression product by no more than about 20%, 18%, 16%, 14%, 12%, 10%, 9%, 8%, 7%, 6%, 5%, 4%, 3%, 2%, 1% or 0.1%, which is hereafter referred to as "normal expression."

In some embodiments, the methods-comprise measuring the level or functional activity of individual expression products of at least about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44 or 45 stress marker genes. For example, the methods may comprise measuring the level or functional activity of a stress marker polynucleotide either alone or in combination with as much as 44, 43, 42, 41,40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1 other stress marker polynucleotide(s). In another example, the methods may comprise measuring the level or functional activity of a stress marker polypeptide either alone or in combination with as much as 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1 other stress marker polypeptides(-s). In illustrative examples of this type, the methods comprise measuring the level or functional activity of individual expression products of at least 1, 2, 3, 4, 5 or 6 stress marker genes that have a very high correlation with the presence or risk of a physiological response to stress (hereafter referred to as "level one correlation stress marker genes"), representative examples of which include, but are not limited to, (a) a polynucleotide comprising a nucleotide sequence that shares at least 50% (and at least 51% to at least 99% and all integer percentages in between) sequence identity with the sequence set forth in any one of SEQ ID NO: 89, 90, 103, 125, 126, 163, 178, 182, 184 or 190, or a complement thereof; (b) a polynucleotide comprising a nucleotide sequence that encodes a polypeptide comprising the amino acid sequence set forth in any one of SEQ ID NO: 104, 179, 183 or 189; (c) a polynucleotide comprising a nucleotide sequence that encodes a polypeptide that shares at least 50% (and at least 51 % to at least 99% and all integer percentages in between) sequence similarity with at least a portion of the sequence set forth in SEQ ID NO: 104, 179, 183 or 189, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In other illustrative examples, the methods comprise measuring the level or functional activity of individual expression products of at least 1, 2, 3, 4, 5, 6, 7 or 8 stress marker genes that have a high correlation with the presence or risk of a physiological response to stress (hereafter referred to as "level two correlation stress marker genes"), representative examples of which include, but are not limited to, (a) a polynucleotide comprising, nucleotide sequence that shares at least 50% (and at least 51% to at least 99% and all integer percentages in between) sequence identity with the sequence set forth in any one of SEQ ID NO: 17, 23, 44, 52, 133, 135, 144, 147, 148, 151, 155, 192, 196, 202 or 206, or a complement thereof; (b) a polynucleotide comprising a nucleotide sequence that encodes a polypeptide comprising the amino acid sequence set forth in any one of SEQ ID NO: 18, 20, 45, 53, 134, 136, 149, 152, 193, 197 or 207; (c) a polynucleotide comprising a nucleotide sequence that encodes a polypeptide that shares at least 50% (and at least 51 % to at least 99% and all integer percentages in between) sequence similarity with at least a portion of the sequence set forth in SEQ ID NO: 18, 20, 45, 53, 134, 136, 149, 152, 193, 197 or 207, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In still other illustrative examples, the methods comprise measuring the level or functional activity of individual expression products of at least 1, 2, 3, 4, 5, 6, 7, 8, 9 or 10 stress marker genes that have a medium correlation with the presence or risk of a physiological response to stress (hereafter referred to as "level three correlation stress marker genes"), representative examples of which include, but are not limited to, (a) a polynucleotide comprising a nucleotide sequence-that shares at least 50% (and at least 51% to at least 99% and all integer percentages in between) sequence identity with the sequence set forth in any one of SEQ ID NO: 5, 30, 37, 48, 54, 55, 64, 66, 70, 77, 79, 85, 91, 92, 95, 96, 101, 115, 117, 118, 121, 150, 153, 158, 164, 170, 180, 186 or 198, or a complement thereof; (b) a polynucleotide comprising a nucleotide sequence that encodes a polypeptide comprising the amino acid sequence set forth in any one of SEQ ID NO: 6, 31, 49, 65, 67, 78, 80, 86, 102, 116, 122, 154, 159, 181 or 199; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 6, 31, 49, 65, 67, 78, 80, 86, 102, 116, 122, 154, 159, 181 or 199, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In still other illustrative examples, the methods comprise measuring the level or functional activity of individual expression products of at least 1, 2, 3, 4, 5, 6, 7, 8, 9 or 10 stress marker genes that have a moderate correlation with the presence or risk of a physiological response to stress (hereafter referred to as "level four correlation stress marker genes"), representative examples of which include, but are not limited to, (a) a polynucleotide comprising a nucleotide sequence that shares at least 50% (and at least 51 % to at least 99% and all integer percentages in between) sequence identity with the sequence set forth in any one of SEQ ID NO: 7, 15, 16, 19, 21, 24, 25, 26, 28, 35, 38, 39, 42, 46, 57, 68, 73, 81, 83, 97, 99, 107, 113, 123, 160, 165, 175, 187, 188, 194, 195 or 200, or a complement thereof; (b) a polynucleotide comprising a nucleotide sequence that encodes a polypeptide comprising the amino acid sequence set forth in any one of SEQ ID NO: 20, 22, 27, 29, 36, 42, 43, 58, 69, 74, 82, 84, 98, 100, 108, 114, 124, 166, 189 or 201; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 20, 22, 27, 29, 36, 42, 43, 58, 69, 74, 82, 84, 98, 100, 108, 114, 124, 166, 189 or 201, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In still other illustrative examples, the methods comprise measuring the level or functional activity of individual expression products of at least 1, 2, 3, 4, 5, 6, 7, 8, 9 or 10 stress marker genes that have a lower correlation with the presence or risk of a physiological response to stress (hereafter referred to as "level five correlation stress marker genes"), representative examples of which include, but are not limited to, (a) a polynucleotide comprising a nucleotide sequence that shares at least 50% (and at least 51 % to at least 99% and all integer percentages in between) sequence identity with the sequence set forth in any one of SEQ ID NO: 1, 3, 9, 11, 13, 32, 33, 34, 40, 41, 50, 51, 56, 59, 62, 63, 71, 75, 87, 93, 105, 111, 119, 127, 129, 130, 131, 137, 139, 141, 143, 145, 156, 161, 167, 169, 171, 173, 176, 185, 204 or 210, or a complement thereof; (b) a polynucleotide comprising a nucleotide sequence that encodes a polypeptide comprising the amino acid sequence set forth in any one of SEQ ID NO: 2, 4, 12, 14, 60, 61, 72, 76, 88, 94, 106, 112, 120, 128, 132, 138, 140, 142, 146, 157, 162, 168, 172, 174, 177, 205 or 211; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 2, 4, 12, 14, 60, 61, 72, 76, 88, 94, 106, 112, 120, 128, 132, 138, 140, 142, 146, 157, 162, 168, 172, 174, 177, 205 or 211, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In some embodiments, the methods comprise measuring the level or functional activity of an expression product of at least 1 level one correlation stress marker gene. In other embodiments, the methods comprise measuring the level or functional activity of an expression product of at least 2 level one correlation stress marker genes. In still other embodiments, the methods comprise measuring the level or functional activity of an expression product of at least 1 level one correlation stress marker gene and the level or functional activity of an expression product of at least 1 level two stress marker gene. In still other embodiments, the methods comprise measuring the level or functional activity of an expression product of at least 2 level one correlation stress marker genes and the level or functional activity of an expression product of at least 1 level two correlation stress marker gene. In still other embodiments, the methods comprise measuring the level or functional activity of an expression product of at least 1 level one correlation stress marker gene and the level or functional activity of an expression product of at least 2 level two correlation stress marker genes.

In some embodiments, the methods comprise measuring the level or functional activity of an expression product of at least 1 level one correlation stress marker gene and the level or functional activity of an expression product of at least 1 level three correlation stress marker gene. In other embodiments, the methods comprise measuring the level or functional activity of an expression product of at least 2 level one correlation stress marker genes and the level or functional activity of an expression product of at least 1 level three correlation stress marker gene. In still other embodiments, the methods comprise measuring the level or functional activity of an expression product of at least 1 level one correlation stress marker gene and the level or functional activity of an expression product of at least 2 level three correlation stress marker genes. In still other embodiments, the methods comprise measuring the level or functional activity of an expression product of at least 1 level-one correlation stress marker gene and the level or functional activity of an expression product of at least 3 level three correlation stress marker genes.

In some embodiments, the methods comprise measuring the level or functional activity of an expression product of at least 1 level one correlation stress marker gene and the level or functional activity of an expression product of at least 1 level four correlation stress marker gene. In other embodiments, the methods comprise measuring the level or functional activity of an expression product of at least 2 level one correlation stress marker genes and the level or functional activity of an expression product of at least 1 level four correlation stress marker gene. In still other embodiments, the methods comprise measuring the level or functional activity of an expression product of at least 1 level one correlation stress marker gene and the level or functional activity of an expression product of at least 2 level four correlation stress marker gene. In still other embodiments, the methods comprise measuring the level or functional activity of an expression product of at least 1 level one correlation stress marker gene and the level or functional activity of an expression product of at least 3 level four correlation stress marker genes. In still other embodiments, the methods comprise measuring the level or functional activity of an expression product of at least 1 level one correlation stress marker gene and the level or functional activity of an expression product of at least 4 level four correlation stress marker genes.

In some embodiments, the methods comprise measuring the level or functional activity of an expression product of at least 1 level one correlation stress marker gene and the level or functional activity of an expression product of at least 1 level five correlation stress marker gene. In other embodiments, the methods comprise measuring the level or functional activity of an expression product of at least 2 level one correlation stress marker genes and the level or functional activity of an expression product of at least 1 level five correlation stress marker gene. In still other embodiments, the methods comprise measuring the level or functional activity of an expression product of at least 1 level one correlation stress marker gene and the level or functional activity of an expression product of at least 2 level five correlation stress marker gene. In still other embodiments, the methods comprise measuring the level or functional activity of an expression product of at least 1 level one correlation stress marker gene and the level or functional activity of an expression product of at least 3 level five correlation stress marker genes. In still other embodiments, the methods comprise measuring the level or functional activity of an expression product of at least 1 level one correlation stress marker gene and the level or functional activity of an expression product of at least 4 level five correlation stress marker genes.

In some embodiments, the methods comprise measuring the level or functional activity of an expression product of at least 1 level two correlation stress marker gene. In other-embodiments, the methods comprise measuring the level or functional activity of an expression product of at least 2 level two correlation stress marker genes. In still other embodiments, the methods comprise measuring the level or functional activity of an expression product of at least 1 level two correlation stress marker gene and the level or functional activity of an expression product of at least 1 level three correlation stress marker gene. In other embodiments, the methods comprise measuring the level or functional activity of an expression product of at least 2 level two correlation stress marker genes and the level or functional activity of an expression product of at least 1 level three correlation stress marker gene. In still other embodiments, the methods comprise measuring the level or functional activity of an expression product of at least 1 level two correlation stress marker gene and the level or functional activity of an expression product of at least 2 level three correlation stress marker genes. In still other embodiments, the methods comprise measuring the level or functional activity of an expression product of at least 1 level two correlation stress marker gene and the level or functional activity of an expression product of at least 3 level three correlation stress marker genes. In still other embodiments, the methods comprise measuring the level or functional activity of an expression product of at least 1 level two correlation stress marker gene and the level or functional activity of an expression product of at least 4 level three correlation stress marker genes.

In some embodiments, the methods comprise measuring the level or functional activity of an expression product of at least 1 level two correlation stress marker gene and the level or functional activity of an expression product of at least 1 level four correlation stress marker gene. In other embodiments, the methods comprise measuring the level or functional activity of an expression product of at least 2 level two correlation stress marker genes and the level or functional activity of an expression product of at least 1 level four correlation stress marker gene. In still other embodiments, the methods comprise measuring the level or functional activity of an expression product of at least 1 level two correlation stress marker gene and the level or functional activity of an expression product of at least 2 level four correlation stress marker genes. In still other embodiments, the methods comprise measuring the level or functional activity of an expression product of at least 1 level two correlation stress marker gene and the level or functional activity of an expression product of at least 3 level four correlation stress marker genes. In still other embodiments, the methods comprise measuring the level or functional activity of an expression product of at least 1 level two correlation stress marker gene and the level or functional activity of an expression product of at least 4 level four correlation stress marker genes. In still other embodiments, the methods comprise measuring the level or functional activity of an expression product of at least 1 level two correlation stress marker gene and the level or functional activity of an expression product of at least 5 level four correlation stress marker genes.

In some embodiments, the methods comprise measuring the level or functional activity of an expression product of at least 1 level two correlation stress marker gene. In other embodiments, the methods comprise measuring the level or functional activity of an expression product of at least 2 level two correlation stress marker gene. In still other embodiments, the methods comprise measuring the level or functional activity of an expression product of at least 1 level two correlation stress marker gene and the level or functional activity of an expression product of at least 1 level five correlation stress marker gene. In other embodiments, the methods comprise measuring the level or functional activity of an expression product of at least 2 level two correlation stress marker genes and the level or functional activity of an expression product of at least 1 level five correlation stress marker gene. In still other embodiments, the methods comprise measuring the level or functional activity of an expression product of at least 1 level two correlation stress marker gene and the level or functional activity of an expression product of at least 2 level five correlation stress marker genes. In still other embodiments, the methods comprise measuring the level or functional activity of an expression product of at least 1 level two correlation stress marker gene and the level or functional activity of an expression product of at least 3 level five correlation stress marker genes. In still other embodiments, the methods comprise measuring the level or functional activity of an expression product of at least 1 level two correlation stress marker gene and the level or functional activity of an expression product of at least 4 level five correlation stress marker genes. In still other embodiments, the methods comprise measuring the level or functional activity of an expression product of at least 1 level two correlation stress marker-gene and the level or functional activity-of an expression product of at least 5 level five correlation stress marker genes.

In some embodiments, the methods comprise measuring the level or functional activity of an expression product of at least 1 level three correlation stress marker gene. In other embodiments, the methods comprise measuring the level or functional activity of an expression product of at least 2 level three correlation stress marker genes. In still other embodiments, the methods comprise measuring the level or functional activity of an expression product of at least 1 level three correlation stress marker gene and the level or functional activity of an expression product of at least 1 level four correlation stress marker gene. In other embodiments, the methods comprise measuring the level or functional activity of an expression product of at least 2 level three correlation stress marker genes and the level or functional activity of an expression product of at least 1 level four correlation stress marker gene. In still other embodiments, the methods comprise measuring the level or functional activity of an expression product of at least 1 level three correlation stress marker gene and the level or functional activity of an expression product of at least 2 level four correlation stress marker genes. In still other embodiments, the methods comprise measuring the level or functional activity of an expression product of at least 1 level three correlation stress marker gene and the level or functional activity of an expression product of at least 3 level four correlation stress marker genes. In still other embodiments, the methods comprise measuring the level or functional activity of an expression product of at least 1 level three correlation stress marker gene and the level or functional activity of an expression product of at least 4 level four correlation stress marker genes. In still other embodiments, the methods comprise measuring the level or functional activity of an expression product of at least 1 level three correlation stress marker gene and the level or functional activity of an expression product of at least 5 level four correlation stress marker genes.

In some embodiments, the methods comprise measuring the level or functional activity of an expression product of at least 1 level three correlation stress marker gene and the level or functional activity of an expression product of at least 1 level five correlation stress marker gene. In other embodiments, the methods comprise measuring the level or functional activity of an expression product of at least 2 level three correlation stress marker genes and the level or functional activity of an expression product of at least 1 level five correlation stress marker gene. In still other embodiments, the methods comprise measuring the level or functional activity of an expression product of at least 1 level three correlation stress marker gene and the level or functional activity of an expression product of at least 2 level five correlation stress marker genes. In still other embodiments, the methods comprise measuring the level or functional activity of an expression product of at least 1 level three correlation stress marker gene and the level or functional activity of an expression product of at least 3 level five correlation stress marker genes. In still other embodiments, the methods comprise measuring the level or functional activity of an expression product of at least 1 level three correlation stress marker gene and the level or functional activity of an expression product of at least 4 level five correlation stress marker genes. In still other embodiments, the methods comprise measuring the level or functional activity of an expression product of at least 1 level three correlation stress marker gene and the level or functional activity of an expression product of at least 5 level five correlation stress marker genes.

In some embodiments, the methods comprise measuring the level or functional activity of an expression product of at least 1 level four correlation stress marker gene. In other embodiments, the methods comprise measuring the level or functional activity of an expression product of at least 2 level four correlation stress marker genes. In other embodiments, the methods comprise measuring the level or functional activity of an expression product of at least 3 level four correlation stress marker genes. In still other embodiments, the methods-comprise measuring the level or functional activity of an expression product of at least 3- level four correlation stress marker genes: In still other embodiments, the methods comprise measuring the level or functional activity of an expression product of at least 4 level four correlation stress marker genes. In still other embodiments, the methods comprise measuring the level or functional activity of an expression product of at least 5 level four correlation stress marker genes. In still other embodiments, the methods comprise measuring the level or functional activity of an expression product of at least 6 level four correlation stress marker genes.

In some embodiments, the methods comprise measuring the level or functional activity of an expression product of at least 1 level four correlation stress marker gene and the level or functional activity of an expression product of at least 1 level five correlation stress marker gene. In other embodiments, the methods comprise measuring the level or functional activity of an expression product of at least 2 level four correlation stress marker genes and the level or functional activity of an expression product of at least 1 level five correlation stress marker gene. In still other embodiments, the methods comprise measuring the level or functional activity of an expression product of at least 1 level four correlation stress marker gene and the level or functional activity of an expression product of at least 2 level five correlation stress marker genes. In still other embodiments, the methods comprise measuring the level or functional activity of an expression product of at least 1 level four correlation stress. marker gene and the level or functional activity of an expression product of at least 3 level five correlation stress marker genes. In still other embodiments, the methods comprise measuring the level or functional activity of an expression product of at least 1 level four correlation stress marker gene and-the level or functional activity of an expression product of-at least 4 level five correlation stress marker genes. In still other embodiments, the-methods comprise measuring the level or functional activity of an expression product of at least 1 level four correlation stress marker gene and the level or functional activity of an expression product of at least 5 level five correlation stress marker genes. In still other embodiments, the methods comprise measuring the level or functional activity of an expression product of at least 1 level four correlation stress marker gene and the level or functional activity of an expression product of at least 6 level five correlation stress marker genes.

In some embodiments, the methods comprise measuring the level or functional activity of an expression product of at least 1 level five correlation stress marker gene. In other embodiments, the methods comprise measuring the level or functional activity of an expression product of at least 2 level five correlation stress marker genes. In other embodiments, the methods comprise measuring the level or functional activity of an expression product of at least 3 level five correlation stress marker genes. In still other embodiments, the methods-comprise measuring the level or functional activity of an expression product of at least 3 level five correlation stress marker genes. In still other embodiments; the methods comprise measuring the level or functional activity of an expression product of at least 4 level five correlation stress marker genes. In still other embodiments, the methods comprise measuring the level or functional activity of an expression product of at least 5 level five correlation stress marker genes. In still other embodiments, the methods comprise measuring the level or functional activity of an expression product of at least 6 level five correlation stress marker genes.

Advantageously, the biological sample comprises blood, especially peripheral blood, which typically includes leukocytes. Suitably, the expression product is selected from a RNA molecule or a polypeptide. In some embodiments, the expression product is the same as the corresponding expression product. In other embodiments, the expression product is a variant (e.g., an allelic variant) of the corresponding expression product.

In certain embodiments, the expression product or corresponding expression product is a target RNA (e.g., mRNA) or a DNA copy of the target RNA whose level is measured using at least one nucleic acid probe that hybridises under at least low stringency conditions to the target RNA or to the DNA copy, wherein the nucleic acid probe comprises at least 15 contiguous nucleotides of a stress marker gene. In these embodiments, the measured level or abundance of the target RNA or its DNA copy is normalised to the level or abundance of a reference RNA or a DNA copy of the reference RNA that is present in the same sample. Suitably, the nucleic acid probe is immobilized on a solid or semi-solid support. In illustrative examples of this type, the nucleic acid-probe forms part of a spatial array of nucleic acid probes. In some embodiments, the level of nucleic acid probe that is bound to the target RNA or to the DNA copy is measured by hybridization (e.g., using a nucleic acid array). In other embodiments, the level of nucleic acid probe that is bound to the target RNA or to the DNA copy is measured by nucleic acid amplification (e.g., using a polymerase chain reaction (PCR)): In still other embodiments, the level of nucleic acid probe that is bound to the target RNA or to the DNA copy is measured by nuclease protection assay.

In other embodiments, the expression product or corresponding expression product is a target polypeptide whose level is measured using at least one antigen-binding molecule that is immune-interactive with the target polypeptide. In these embodiments, the measured level of the target polypeptide is normalized to the level of a reference polypeptide that is present in the same sample. Suitably, the antigen-binding molecule is immobilized on a solid or semi-solid support. In illustrative examples of this type, the antigen-binding molecule forms part of a spatial array of antigen-binding molecule. In some embodiments, the level of antigen-binding molecule that is bound to the target polypeptide is measured by immunoassay (e.g., using an ELISA).

In still other embodiments, the expression product or corresponding expression product is a target polypeptide whose level is measured using at least one substrate for the target polypeptide with which it reacts to produce a reaction product In these embodiments, the measured functional activity of the target polypeptide is normalized to the functional activity of a reference polypeptide that is present in the same sample.

In some embodiments, a system is used to perform the method, which suitably comprises at least one end station coupled to a base station. The base station is suitably caused (a) to receive subject data from the end station via a communications network, wherein the subject data represents parameter values corresponding to the measured or normalized level or functional activity of at least one expression product in the biological sample, and (b) to compare the subject data with predetermined data representing the measured or normalized level or functional activity of at least one corresponding expression product in the reference sample to thereby determine any difference in the level or functional activity of the expression product in the biological sample as compared to the level or functional activity of the corresponding expression product in the reference sample. Desirably, the base station is further caused to provide a diagnosis for the presence, absence, degree, or risk of development, of a stress response. In these embodiments, the base station may be further caused to transfer an indication of the diagnosis to the end station via the communications network.

In another aspect, the invention provides methods for determining the presence or degree of immunosuppression in a test subject. These methods generally comprise detecting in the subject aberrant expression of at least one stress marker gene as broadly described above.

In yet another aspect, the present invention provides methods for treating or preventing the development of stress or a related condition in a test subject. These methods generally comprise detecting aberrant expression of at least one stress marker gene in the subject, and managing the environment of the subject to prevent or minimize exposure of the subject to a causative stressor and/or administering to the subject an effective amount of an agent that treats or ameliorates the symptoms or reverses or inhibits the development of stress in the subject. In certain embodiments, the related condition is immunosuppression.

Accordingly, in a related aspect, the present invention provides methods for treating or preventing the development of immunosuppression in a test subject. These methods generally comprise detecting aberrant expression of at least one stress marker gene in the subject, and managing the environment of the subject to prevent or minimize exposure of the subject to a causative stressor and/or administering to the subject an effective amount of an agent that treats or ameliorates the-symptoms or reverses or inhibits the development of stress in the subject.

In still another aspect, the present invention provides methods for assessing the-capacity of a subject's immune system to produce an immunogenic response to a selected antigen. These methods generally comprise determining whether at least one stress marker gene as broadly described above is normally or aberrantly expressed in the-subject, whereby normal expression of the or each stress marker gene is indicative of a normal capacity to produce the immunogenic response and whereby aberrant expression of the or each stress marker gene is indicative of an impaired capacity to produce the immunogenic response.

In a further aspect, the present invention provides methods for eliciting an immune response to a selected antigen in a test subject via administration of a composition comprising the antigen. These methods generally comprise detecting normal expression of at least one stress marker gene as broadly described above in the subject and administering the composition to the subject.

In some embodiments, the methods further comprise detecting in the subject aberrant expression of at least one stress marker gene as broadly described above and managing the environment of the subject to prevent or minimize exposure of the subject to a causative stressor and/or administering to the subject an effective amount of an agent that reverses or inhibits the development of stress in the subject, and administering the composition to the subject. In some embodiments, the composition is administered to the subject when the or each stress marker gene is normally expressed in the subject.

In a related aspect, the invention provides methods for improving an immune response to a selected antigen in a test subject to whom/which has been administered a composition comprising the antigen. These methods generally comprise detecting aberrant expression of at least one stress marker gene as broadly described above in the subject and managing the environment of the subject to prevent or minimize exposure of the subject to a causative stressor and/or administering to the subject an effective amount of an agent that reverses or inhibits the development of stress in the subject, whereby the management or administration leads to normal expression of the or each stress marker gene in the subject.

In another aspect, the present invention provides isolated polynucleotides, referred to herein as "stress marker polynucleotides," which are generally selected from: (a) a polynucleotide comprising a nucleotide sequence that shares at least 50% (and at least 51% to at least 99% and all integer percentages in between) sequence identity with the sequence set forth in any one of SEQ ID NO: 15, 16, 23, 24, 25, 28, 29, 32, 33, 34, 37, 38, 39, 40, 41, 50, 51, 54, 55, 62, 63, 89, 90, 91, 92, 95, 96,107, 108, 117, 118, 125, 126, 129, 130, 143, 144, 147, 150, 155, 163, 164, 169, 170, 175, 184,185, 186, 187, 194, 195, 232, 233, 238, 239, 240, 241, 242 or 243, or a complement thereof; (b) a polynucleotide comprising a portion of the sequence set forth in any one of SEQ ID NO: 15, 16, 23, 24, 25, 28, 29, 32, 33, 34, 37, 38, 39, 40, 41, 50, 51, 54, 55, 62, 63, 89, 90, 91, 92, 95, 96, 107, 108, 117, 118, 125, 126, 129, 130, 143, 144, 147, 150, 155, 163, 164, 169, 170, 176, 184, 185, 186, 187, 194, 195, 232, 233, 238, 239, 240, 241, 242 or 243, or a complement thereof, wherein the portion comprises at least 15 contiguous nucleotides of that sequence or complement; (c) a polynucleotide that hybridizes to the sequence of (a) or (b) or a complement thereof, under at least low, medium or high stringency conditions; and (d) a polynucleotide comprising a portion of any one of SEQ ID NO: 15, 16, 23, 24, 25, 28, 29, 32, 33, 34, 37, 38, 39, 40, 41, 50, 51, 54, 55, 62, 63, 89, 90, 91, 92, 95, 96, 107, 108, 117, 118, 125, 126, 129, 130, 143, 144, 147, 150, 155, 163, 164, 169, 170, 175, 184, 185, 186, 187, 194, 195, 232, 233, 238, 239, 240, 241, 242 or 243, or a complement thereof, wherein the portion comprises at least 15 contiguous nucleotides of that sequence or complement and hybridizes to a sequence of (a), (b) or (c), or a complement thereof, under at least low, medium or high stringency conditions.

In another aspect, the present invention provides a nucleic acid construct comprising a polynucleotide as broadly described above in operable connection with a regulatory element, which is operable in a host cell. In certain embodiments, the construct is in the form of a vector, especially an expression vector.

In yet another aspect, the present invention provides isolated host cells containing a nucleic acid construct or vector as broadly described above. In certain advantageous embodiments, the host cells are selected from bacterial cells, yeast cells and insect cells.

In still another aspect, the present invention provides probes for interrogating nucleic acid for the presence of a polynucleotide as broadly described above. These probes generally comprise a nucleotide sequence that hybridizes under at least low stringency conditions to a polynucleotide as broadly described above. In some embodiments, the probes consist essentially of a nucleic acid sequence which corresponds or is complementary to at least a portion of a nucleotide sequence encoding the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion is at least 15 nucleotides in length. In other embodiments, the probes comprise a nucleotide sequence which is capable of hybridizing to at least a portion of a nucleotide sequence encoding the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 99, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249 under at least low stringency conditions, wherein the portion is at least 15 nucleotides in length. In still other embodiment, the.probes comprise a nucleotide sequence that is capable of hybridizing to at least a portion of any one of SEQ ID NO: 1, 3, 4, 5, 7, 9, 11, 13, 15, 16, 17, 19, 21, 23, 24, 25, 26, 28, 29, 30, 32, 33, 34, 35; 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248 under at least low stringency conditions, wherein the portion is at least 15 nucleotides in length. Representative probes for detecting the stress marker polynucleotides according to the resent invention are set forth in SEQ ID NO: 250-1807 (see Table 2).

In a related aspect, the invention provides a solid or semi-solid support comprising at least one nucleic acid probe as broadly described above immobilized thereon. In some embodiments, the solid or semi-solid support comprises a spatial array of nucleic acid probes immobilized thereon.

In a further aspect, the present invention provides isolated polypeptides, referred to herein as-"stress marker polypeptides," which are generally selected from: (i) a polypeptide comprising an amino acid sequence that shares at least 50% (and at least 51% to at least 99% and all integer percentages in between) sequence similarity with a polypeptide expression product of a stress marker gene as broadly described above, for example, especially a stress marker gene that comprises a nucleotide sequence that shares at least 50% (and at least 51% to at least 99% and all integer percentages in between) sequence identity with the sequence set forth in any one of SEQ ID NO: 15, 16, 23, 24, 25, 28, 29, 32, 33, 34, 37, 38, 39, 40, 41, 50, 51, 4, 5, 62, 63, 89, 90, 91, 92, 95, 96, 107, 108, 117, 118, 125, 126, 129, 130, 143, 144, 147, 150, 155, 163, 164, 169, 170, 175, 184, 185, 186, 187, 194, 195, 232, 233, 238, 239, 240, 241, 242 or 243; (ii) a portion of the polypeptide according to (i) wherein the portion comprises at least 5 contiguous amino acid residues of that polypeptide; (iii) a polypeptide comprising an amino acid sequence that shares at least 30% similarity (and-at least 31% to at least 99% and all integer percentages in between) with at least 15 contiguous amino acid residues of the polypeptide according to (i); and (iv) a polypeptide comprising an amino acid sequence that is immune interactive with an antigen-binding molecule that is immune-interactive with a sequence of (i), (ii) or (iii).

Still a further aspect of the present invention provides an antigen-binding molecule that is immune interactive with a stress marker polypeptide as broadly described above:

In a related aspect, the invention provides a solid or semi-solid support comprising at least one antigen-binding molecule as broadly described above immobilized thereon. In some embodiments, the solid or semi-solid support comprises a spatial array of antigen-binding molecules immobilized thereon.

Still another aspect of the invention provides the use of one or more stress marker polynucleotides as broadly described above, or the use of one or more probes as broadly described above, or the use of one or more stress marker polypeptides as broadly described above, or the use of one or more antigen-binding molecules as broadly described above, in the manufacture of a kit for assessing the physiological response to stress or immune function in a subject.

### BRIEF DESCRIPTION OF THE DRAWINGS

Figure 1 is a graphical representation of a receiver operating curve (ROC) for comparison of gene expression at 28 days after stressor to Day 0 (Day 0 is following 2 days of road transport). ROC curves are based on cross validated components discriminant function scores.

Figure 2 is a graphical representation of a receiver operating curve (ROC) for comparison of gene expression at 28 days after stressor to Day 2 (Day 0 is following 2 days of road transport). ROC curves are based on cross validated components discriminant function scores.

Figure 3 is a graphical representation of a receiver operating curve (ROC) for comparison of gene expression at 28 days after stressor to Day 4 (Day 0 is following 2 days of road transport). ROC curves are based on cross validated components discriminant function scores.

Figure 4 is a graphical representation of a receiver operating curve (ROC) for comparison of gene expression at 28 days after stressor to Day 7 (Day 0 is following 2 days of road transport). ROC curves are based on cross validated components discriminant function scores.

Figure 5 is a graphical representation of a receiver operating curve (ROC) for comparison of gene expression at 28 days after stressor to Day 9 (Day 0 is following 2 days of road transport). ROC curves are based on cross validated components discriminant function scores.

Figure 6 is a graphical representation of a receiver operating curve (ROC) for comparison of gene expression at 28 days after stressor to Day L1 (Day 0 is following 2 days of road transport). ROC curves are based on cross validated components discriminant function scores.

Figure 7 is a graphical representation of a receiver operating-curve (ROC) for comparison of gene expression at 28 days after stressor to Day 14 (Day 0 is following 2. days of road transport). ROC curves are based on cross validated components discriminant function scores.

Figure 8 is a graphical representation of a receiver operating curve (ROC) for comparison of gene expression at 28 days after stressor to Day 17 (Day 0 is following 2 days of road transport). ROC curves are based on cross validated components discriminant function scores.

Figure 9 is a graphical representation of a receiver operating curve (ROC) for comparison of gene expression at 28 days after stressor to Day 21 (Day 0 is following 2 days of road transport). ROC curves are based on cross validated components discriminant function scores.

Figure 10 is a graphical representation of a receiver operating curve (ROC) for comparison of gene expression at 28 days after stressor to Day 24 (Day 0 is following 2 days of road transport). ROC curves are based on cross validated components discriminant function scores.

### DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e. to at least one) of the grammatical object of the article. By way of example, "an element" means one element or more than one element.

The term "aberrant expression," as used herein to describe the expression of a stress marker gene, refers to the overexpression or underexpression of a stress marker gene relative to the level of expression of the stress marker gene or variant thereof in cells obtained from a healthy subject or from a subject free of stress, and/or to a higher or lower level of a stress marker gene product (e.g., transcript or polypeptide) in a tissue sample or body fluid obtained from a healthy subject or from a subject not under stress. In particular, a stress marker gene is aberrantly expressed if the level of expression of the stress marker gene is higher by at least about 10%, 20%, 30%, 40%, 50%, 60%, 70%, 80% or 90%, or even an at least about 100%, 200%, 300%, 400%, 500%, 600%, 700%, 800%, 900% or 1000%, or lower by at least about 10%, 20%, 30% 40%, 50%, 60%, 70%, 80%, 90%, 92%, 94%, 96%, 97%, 98%-or 99%, or even an at least about 99.5%, 99.9%, 99.95%, 99.99%, 99.995% or 99.999% that the level of expression of the stress marker gene by cells-obtained from a healthy subject or from a subject not under stress, and/or relative to the level of expression of the stress-marker gene in a tissue sample or body fluid obtained from a healthy subject or from a subject not under stress.

By "about" is meant a quantity, level, value, number, frequency, percentage, dimension, size, amount, weight or length that varies by as much as 30, 25, 20, 25,10,9, 8, 7,6, 5, 4, 3, 2 or 1 % to a reference quantity, level, value, number, frequency, percentage, dimension, size, amount, weight or length.

The term "amplicon" refers to a target sequence for amplification, and/or the amplification products of a target sequence for amplification. In certain other embodiments an "amplicon" may include the sequence of probes or primers used in amplification.

By "antigen-binding molecule" is meant a molecule that has binding affinity for a target antigen. It will be understood that this term extends to immunoglobulins, immunoglobulin fragments and non-immunoglobulin derived protein frameworks that exhibit antigen-binding activity.

As used herein, the term "binds specifically," "specifically immuno-interactive" and the like when referring to an antigen-binding molecule refers to a binding reaction which is determinative of the presence of an antigen in the presence of a heterogeneous population of proteins and other biologics. Thus, under designated immunoassay conditions, the specified antigen-binding molecules bind to a particular antigen and do not bind in a significant amount to other proteins or antigens present in the sample. Specific binding to an antigen under such conditions may require an antigen-binding molecule that is selected for its specificity for a particular antigen. For example, antigen-binding molecules can be raised to a selected protein antigen, which bind to that antigen but not to other proteins present in a sample. A variety of immunoassay formats may be used to select antigen-binding molecules specifically immuno-interactive with a particular protein. For example, solid-phase ELISA immunoassays are routinely used to select monoclonal antibodies specifically immune-interactive with a protein. See Harlow and Lane (1988) "Antibodies, A Laboratory Manual," Cold Spring Harbor Publications, New York, for a description of immunoassay formats and conditions that can be used to determine specific immunoreactivity.

By "biologically active portion" is meant a portion of a full-length parent peptide or polypeptide which portion retains an activity of the parent molecule. As used herein, the term "biologically active portion" includes deletion mutants and peptides, for example of at least about 8, 9, 10, 11, 12, 13, 14, 15, 16, 17, 18, 19, 20, 21, 22, 23, 24, 25, 30, 40, 50, 60, 70, 80, 90, 100, 120, 150, 300, 400, 500, 600, 700, 800, 900, 1000 contiguous amino acids, which comprise an activity of a parent molecule. Portions ofthis type may be obtained through the application of standard recombinant nucleic acid techniques or synthesized using conventional liquid or solid phase synthesis techniques. For example, reference may be made to solution synthesis or solid phase synthesis as described, for example, in Chapter 9 entitled "Peptide Synthesis"-by Atherton and Shephard which is included in a publication entitled "Synthetic Vaccines" edited by Nicholson and published by Blackwell Scientific Publications. Alternatively, peptides can be produced by digestion of a peptide or polypeptide of the invention with proteinases such as endoLys-C, endoArg-C, endoGlu-C and staphylococcus V8-protease. The digested fragments can be purified by, for example, high performance liquid chromatographic (HPLC) techniques. Recombinant nucleic acid techniques can also be used to produce such portions.

The term "biological sample" as used herein refers to a sample that may be extracted, untreated, treated, diluted or concentrated from an animal. The biological sample may include a biological fluid such as whole blood, serum, plasma, saliva, urine, sweat, ascitic fluid, peritoneal fluid, synovial fluid, amniotic fluid, cerebrospinal fluid, tissue biopsy, and the like. In certain embodiments, the biological sample is blood, especially peripheral blood.

As used herein, the term "cis-acting sequence", "cis-acting element" or "cis-regulatory region" or "regulatory region" or similar term shall be taken to mean any sequence of nucleotides, which when positioned-appropriately relative to an expressible genetic sequence, is capable of regulating, at least in part, the expression of the genetic sequence. Those skilled in the art will be aware that a cis-regulatory region may be capable of activating, silencing, enhancing, repressing or otherwise altering the level of expression and/or cell type-specificity and/or developmental specificity of a gene sequence at the transcriptional or post-transcriptional leveL In certain embodiments of the present invention, the cis-acting sequence is an activator sequence that enhances or stimulates the expression of an expressible genetic sequence.

Throughout this specification, unless the context requires otherwise, the words "comprise," "comprises" and "comprising" will be understood to imply the inclusion of a stated step or element or group of steps or elements but not the exclusion of any other step or element or group of steps or elements.

By "corresponds to" or "corresponding to" is meant a polynucleotide (a) having a nucleotide sequence that is substantially identical or complementary to all or a portion of a reference polynucleotide sequence or (b) encoding an amino acid sequence identical to an amino acid sequence in a peptide or protein. This phrase also includes within its scope a peptide or polypeptide having an amino acid sequence that is substantially identical to a sequence of amino acids in a reference peptide or protein.

By "effective amount", in the context of treating or preventing a condition, is meant the administration of that amount of active to an individual in need of such treatment or prophylaxis, either in a single dose or as part of a series, that is effective for the prevention of incurring a symptom, holding in check such symptoms, and/or treating existing symptoms, of that condition. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

The terms "expression" or "gene expression" refer to either production of RNA message or translation of RNA message into proteins or polypeptides. Detection of either types of gene expression in use of any of the methods described herein are part of the invention.

By "expression vector" is meant any autonomous genetic element capable of directing the transcription of a polynucleotide contained within the vector and suitably the synthesis of a peptide or polypeptide encoded by the polynucleotide. Such expression vectors are known to practitioners in the art.

The term "gene" as used herein refers to any and all discrete coding regions of the cell's genome, as well as associated non-coding and regulatory regions. The gene is also intended to mean the open reading frame encoding specific polypeptides, introns, and adjacent 5' and 3' non-coding nucleotide sequences involved in the regulation of expression. In this regard, the gene may further comprise control signals such as promoters, enhancers, termination and/or polyadenylation signals that are naturally associated.with a given gene, or heterologous control signals. The DNA sequences may be cDNA or genomic DNA or a fragment thereof. The gene may be introduced into an appropriate vector for extrachromosomal maintenance or for integration into the host.

By "high density polynucleotide arrays" and the like is meant those arrays that contain at least 400 different features per cm².

The phrase "high discrimination hybridization conditions" refers to hybridization conditions in which single base mismatch may be determined.

"Hybridization" is used herein to denote the pairing of complementary nucleotide sequences to produce a DNA-DNA hybrid or a DNA-RNA hybrid. Complementary base sequences are those sequences that are related by the base-pairing rules. In DNA, A pairs with T and C pairs with G. In RNA U pairs with A and C pairs with G. In this regard, the terms "match" and "mismatch" as used herein refer to the hybridization potential of paired nucleotides in complementary nucleic acid-strands. Matched nucleotides hybridize efficiently, such as the classical A-T and G-C base pair mentioned above. Mismatches are other combinations of nucleotides that do not hybridize efficiently.

The phrase "hybridizing specifically to" and the like refer to the binding, duplexing, or hybridizing of a molecule only to a particular nucleotide sequence under stringent conditions when that sequence is present in a complex mixture (e.g., total cellular) DNA or RNA.

Reference herein to "immuno-interactive" includes reference to any interaction, reaction, or other form of association between molecules and in particular where one of the molecules is, or mimics, a component of the immune system.

"Immune function" or "immunoreactivity" refers to the ability of the immune system to respond to foreign antigen as measured by standard assays well known in the art.

The term "immunosuppression" refers to a decrease in the overall immunoreactivity of the immune system resulting from stress or the physiological response to stress. Suitably, the decrease is by at least 20-40%, or by at least 50-75%, or even by at least 80% relative to the immunoreactivity in the absence of stress. Additionally, the term "immunosuppression" includes within its scope a delay in the occurrence of the immune response as compared to a subject not under stress. A delay in the occurrence of an immune response can be a short delay, for example 1 hr-10 days, i.e., 1 hr, 2, 5 or 10 days. A delay in the occurrence of an immune response can also be a long delay, for example, 10 days-10 years (i.e., 30 days, 60 days, 90 days, 180 days, 1, 2, 5 or 10 years). "Immunosuppression" according to the invention can also mean a decrease in the intensity of an immune response, e.g., a reduced intensity such that it is 5-100%, 25-100% or 75 -100% less than the intensity of the immune response of a subject not compromised by stress.

By "isolated" is meant material that is substantially or essentially free from-components that normally accompany it in its native state. For example, an "isolated polynucleotide", as used herein, refers to a polynucleotide, which has been purified from the sequences which flank it in a naturally-occurring state, e.g., a DNA fragment which has been removed from the sequences that are normally adjacent to the fragment. Alternatively, an "isolated peptide" or an "isolated polypeptide" and the like, as used herein, refer to in vitro isolation and/or purification of a peptide or polypeptide molecule from its natural cellular environment, and from association with other components of the cell, i.e., it is not associated with in vivo substances.

By "marker gene" is meant a gene that imparts a distinct phenotype to cells expressing the marker gene and thus allows such transformed cells to be distinguished from cells that do not have the marker. A selectable marker gene confers a trait for which one can 'select' based on resistance to a selective agent (e.g., a herbicide, antibiotic; radiation; heat, or other treatment damaging to untransformed cells). A screenable-marker gene (or reporter gene) confers a trait that one can identify through observation or testing, i.e., by 'screening' (e.g. β-glucuronidase, luciferase, or other enzyme activity not present in untransformed, cells).

As used herein, a "naturally-occurring" nucleic acid molecule refers to a RNA or DNA molecule having a nucleotide sequence that occurs in nature. For example a naturally-occurring nucleic acid molecule can encode a protein that occurs in nature.

By "obtained from" is meant that a sample such as, for example, a cell extract or nucleic acid or polypeptide extract is isolated from, or derived from, a particular source. For instance, the extract may be isolated directly from biological fluid or tissue of the subject.

The term "oligonucleotide" as used herein refers to a polymer composed of a multiplicity of nucleotide residues (deoxyribonucleotides or ribonucleotides, or related structural variants or synthetic analogues thereof, including nucleotides with modified or substituted sugar groups and the like) linked via phosphodiester bonds (or related structural variants or synthetic analogues thereof). Thus, while the term "oligonucleotide" typically refers to a nucleotide polymer in which the nucleotide residues and linkages between them are naturally-occurring, it will be understood that the term also includes within its scope various analogues including, but not restricted to, peptide nucleic acids (PNAs), phosphorothioate, phosphorodithioate, phophoroselenoate, phosphorodiselenoate, phosphoroanilothioate, phosphoraniladate, phosphoroamidate, methyl phosphonates, 2-O-methyl ribonucleic acids, and the like. The exact size of the molecule can vary depending on the particular application. Oligonucleotides are a polynucleotide subset with 200 bases or fewer in length. Preferably, oligonucleotides are 10 to 60 bases in length and most preferably 12, 13, 14, 15, 16, 17, 18, 19, or 20 to 40 bases in length. Oligonucleotides are usually single stranded, e.g., for probes; although oligonucleotides may be double stranded, e.g., for use in the construction of a variant nucleic acid sequence. Oligonucleotides of the invention can be either sense or antisense oligonucleotides.

The term "oligonucleotide array" refers to a substrate having oligonucleotide probes with different known sequences deposited at discrete known locations associated with its surface. For example, the substrate can be in the form of a two dimensional substrate as described in U.S. Patent No. 5,424,186. Such substrate may be used to synthesize two-dimensional spatially addressed oligonucleotide (matrix) arrays. Alternatively, the substrate may be characterized in that it forms a tubular array in which a two dimensional planar sheet is rolled into a three-dimensional tubular configuration. The substrate may also be in the form of a microsphere or bead connected to the surface of an optic fibre as, for example, disclosed by Chee et al. in WO 00/39587. Oligonucleotide arrays have at least two different features and a density of at least 400 features per cm². In certain embodiments, the-arrays can have a density of about 500, at least one thousand, at least 10 thousand, at least 100 thousand, at least one million or at least 10 million features per cm². For example, the substrate may be silicon or glass and can have the thickness of a glass microscope slide or a glass cover slip, or may be composed of other synthetic polymers. Substrates that are transparent to light are useful when the method of performing an assay on the substrate involves optical detection. The term also refers to a probe array and the substrate to which it is attached that form part of a wafer.

The term "operably connected" or "operably linked" as used herein means placing a structural gene under the regulatory control of a promoter, which then controls the transcription and optionally translation of the gene. In the construction of heterologous promoter/structural gene combinations, it is generally preferred to position the genetic sequence or promoter at a distance from the gene transcription start site that is approximately the same as the distance between that genetic sequence or promoter and the gene it controls in its natural setting; i.e., the gene from which the genetic sequence or promoter is derived. As is known in the art, some variation in this distance can be accommodated without loss of function. Similarly, the preferred positioning of a regulatory sequence element with respect to a heterologous gene to be placed under its control is defined by the positioning of the element in its natural setting; i.e., the genes from which it is derived.

The term "polynucleotide" or "nucleic acid" as used herein designates mRNA, RNA, cRNA, cDNA or DNA. The term typically refers to polymeric form of nucleotides of at least 10 bases in length, either ribonucleotides or deoxynucleotides or a modified form of either type of nucleotide. The term includes single and double stranded forms of DNA.

The terms "polynucleotide variant" and "variant" refer to polynucleotides displaying substantial sequence identity with a reference polynucleotide sequence or polynucleotides that hybridize with a reference sequence under stringent conditions that are defined hereinafter. These terms also encompass polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains a biological function or activity of the reference polynucleotide. The terms "polynucleotide variant" and "variant" also include naturally-occurring allelic variants.

"Polypeptide", "peptide" and "protein" are used interchangeably herein to refer to a polymer of amino acid residues and to variants and synthetic analogues of the same. Thus, these terms apply to amino acid polymers in which one or more amino acid residues is a synthetic non-naturally-occurring amino acid, such as a chemical analogue of a corresponding naturally-occurring amino acid, as well as to-naturally-occurring-amino acid polymers.

The term "polypeptide variant" refers to polypeptides which are distinguished from a reference polypeptide by the addition, deletion or substitution of at least one amino acid residue. In certain embodiments, one or more amino acid residues of a reference polypeptide-are replaced by different amino acids. It-is well understood in the art that some amino acids may be changed to others with broadly similar properties without changing the nature of the activity of the polypeptide (conservative substitutions) as described hereinafter.

By "primer" is meant an oligonucleotide which, when paired with a strand of DNA, is capable of initiating the synthesis of a primer extension product in the presence of a suitable polymerizing agent. The primer is preferably single-stranded for maximum efficiency in amplification but can alternatively be double-stranded. A primer must be sufficiently long to prime the synthesis of extension products in the presence of the polymerization agent. The length of the primer depends on many factors, including application, temperature to be employed, template reaction conditions, other reagents, and source of primers. For example, depending on the complexity of the target sequence, the primer may be at least about 5, 6, 7, 8, 9, 10, 11, 12, 13, 14, 15, 16, 17, 18, 19, 20, 21, 22, 23, 24, 25, 26, 27, 28, 29, 30, 35, 40, 50, 75, 100, 150, 200, 300, 400, 500, to one base shorter in length than the template sequence at the 3' end of the primer to allow extension of a nucleic acid chain, though the 5' end of the primer may extend in length beyond the 3' end of the template sequence. In certain embodiments, primers can be large polynucleotides, such as from about 35 nucleotides to several kilobases or more. Primers can be selected to be "substantially complementary" to the sequence on the template to which it is designed to hybridise and serve as a site for the initiation of synthesis. By "substantially complementary", it is meant that the primer is sufficiently complementary to hybridise with a target polynucleotide. Desirably, the primer contains no mismatches with the template to which it is designed to hybridise but this is not essentiaL For example, non-complementary nucleotide residues can be attached to the 5' end of the primer, with the remainder of the primer sequence being complementary to the template. Alternatively, non-complementary nucleotide residues or a stretch of non-complementary nucleotide residues can be interspersed into a primer, provided that the primer sequence has sufficient complementarity with the sequence of the template to hybridise therewith and thereby form a template for synthesis of the extension product of the primer.

"Probe" refers to a molecule that binds to a specific sequence or subsequence or other moiety of another molecule. Unless otherwise indicated, the term "probe" typically refers to a polynucleotide probe that binds to another polynucleotide, often called the "target polynucleotide", through complementary base pairing. Probes can bind target polynucleotides lacking complete sequence complementarity with the probe, depending on the stringency of the hybridization conditions. Probes can be labeled directly or indirectly and include primers within their scope.

The term "recombinant polynucleotide" as used herein refers to a polynucleotide formed in vitro by the manipulation of nucleic acid into a form not normally found in nature. For example, the recombinant polynucleotide may be in the form of an expression vector. Generally, such expression vectors include transcriptional and translational regulatory nucleic acid operably linked to the nucleotide sequence.

By "recombinant polypeptide" is meant a polypeptide made using recombinant techniques, i.e., through the expression of a recombinant or synthetic polynucleotide.

By "regulatory element" or "regulatory sequence" is meant nucleic acid sequences (e.g., DNA) necessary for expression of an operably linked coding sequence in a particular host cell. The regulatory sequences that are suitable for prokaryotic cells for example, include a promoter, and optionally a cis-acting sequence such as an operator sequence and a ribosome binding site. Control sequences that are suitable for eukaryotic cells include promoters, polyadenylation signals, transcriptional enhancers, translational enhancers, leader or trailing sequences that modulate mRNA stability, as well as targeting sequences that target a product encoded by a transcribed polynucleotide to an intracellular compartment within a cell or to the extracellular environment.

The term "sequence identity" as used herein refers to the extent that sequences are identical on a nucleotide-by-nucleotide basis or an amino acid-by-amino acid basis over a window of comparison. Thus, a "percentage of sequence identity" is calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. For the purposes of the present invention, "sequence identity" will be understood to mean the "match percentage" calculated by the DNASIS computer program (Version 2.5 for windows; available from Hitachi Software engineering Co., Ltd., South San Francisco, California, USA) using standard defaults as used in the reference manual accompanying the software.

"Similarity" refers to the percentage number of amino acids that are identical or constitute conservative substitutions as defined in Table 3 *infra.* Similarity may be determined using sequence comparison programs such as GAP (Deveraux et al. 1984, Nucleic Acids Research 12, 387-395). In this way, sequences of a similar or substantially different length to those cited herein might be compared by insertion of gaps into the alignment, such gaps being determined, for example, by the comparison algorithm used by GAP.

Terms used to describe sequence relationships between two or more polynucleotides or polypeptides include "reference sequence," "comparison window," "sequence identity," "percentage of sequence identity" and "substantial identity". A "reference sequence" is at least 12 but frequently 15 to 18 and often at least 25 monomer units, inclusive of nucleotides and amino acid residues, in length. Because two polynucleotides may each comprise (1) a sequence (i.e., only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of at least 6 contiguous positions, usually about 50 to about 100, more usually about 100 to about 150 in which a sequence is compared to a reference sequence of the same number of contiguous positions after the two sequences are optimally aligned. The comparison window may comprise additions or deletions (i.e., gaps) of about 20% or less as compared to the reference sequence (which does not comprise additions or deletions) for optimal alignment of the two sequences. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e., resulting in the highest percentage homology over the comparison window) generated by any of the various methods selected. Reference also may be made to the BLAST family of programs as for example disclosed by Altschul et al., 1997, Nucl. Acids Res. 25:3389. A detailed discussion of sequence analysis can be found in Unit 19.3 of Ausubel et al., "Current Protocols in Molecular Biology", John Wiley & Sons Inc, 1994-1998, Chapter 15.

The terms "subject" or "individual" or "patient", used interchangeably herein, refer to any subject, particularly a vertebrate subject, and even more particularly a mammalian subject, for whom therapy or prophylaxis is desired. Suitable vertebrate animals that fall within the scope of the invention include, but are not restricted to, primates, avians, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes). A preferred subject is an equine animal in need of treatment or prophylaxis for stress. However, it will be understood that the aforementioned terms do not imply that symptoms are present.

The phrase "substantially similar affinities" refers herein to target sequences having similar strengths of detectable hybridization to their complementary or substantially complementary oligonucleotide probes under a chosen set of stringent conditions.

The term "template" as used herein refers-to a nucleic acid that is-used in the creation of a complementary nucleic acid strand to the "template" strand. The template may be either RNA-and/or DNA, and the complementary strand may also be RNA and/or DNA. In certain embodiments, the complementary strand may comprise all or part of the complementary sequence to the "template," and/or may include mutations so that it is not an exact, complementary strand to the "template". Strands that are not exactly complementary to the template strand may hybridise specifically to the template strand in detection assays described here, as well as other assays known in the art, and such complementary strands that can be used in detection assays are part of the invention.

The term "transformation" means alteration of the genotype of an organism, for example a bacterium, yeast, mammal, avian, reptile, fish or plant, by the introduction of a foreign or endogenous nucleic acid.

By "vector" is meant a polynucleotide molecule, suitably a DNA molecule derived, for example, from a plasmid, bacteriophage, yeast, virus, mammal, avian, reptile or fish into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly, the vector can be an autonomously replicating vector, i.e., a vector that exists as an extrachromosomal entity, the replication of which is independent of chromosomal replication, e.g., a linear or closed circular plasmid, an extrachromosomal element, a minichromosome, or an artificial chromosome. The vector can contain any means for assuring self-replication. Alternatively, the vector can be one which, when introduced into the host cell, is integrated into the genome and replicated together with the chromosome(s) into which it has been integrated. A vector system can comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. The vector can also include a selection marker such as an antibiotic resistance gene that can be used for selection of suitable transformants. Examples of such resistance genes are known to those of skill in the art.

The terms "wild-type" and "normal" are used interchangeably to refer to the phenotype that is characteristic of most of the members of the species occurring naturally and contrast for example with the phenotype of a mutant.

### 2. Abbreviations

The following abbreviations are used throughout the application:
nt =nucleotide
nts =nucleotides
aa =amino acid(s)
kb =kilobase(s) or kilobase pair(s)
kDa =kilodalton(s)
d =day
h =hour
s =seconds

### 3. Markers of stress and uses therefor

The present invention concerns measuring the stress level or physiological response to stress in a subject of interest. Markers of stress, in the form of RNA molecules of specified sequences, or polypeptides expressed from these RNA molecules in cells, especially in blood cells, and more especially in peripheral blood cells, of subjects subjected to stress or perceived to be under stressful conditions, are disclosed. These markers are indicators of stress and, when differentially expressed, are diagnostic for a physiological response to stress in tested subjects. Such markers provide considerable advantages over the prior art in this field. In certain advantageous embodiments where peripheral blood is used for the analysis, it is possible to monitor the reaction to stress, and in addition, the drawing of a blood sample is minimally invasive and relatively inexpensive. The detection methods disclosed herein are thus suitable for widespread screening of subjects.

It will be apparent that the nucleic acid sequences disclosed herein will find utility in a variety of applications in assessing the response to stress, as well as managing and treating stress. Examples of such applications within the scope of the present disclosure include amplification of stress markers using specific primers, detection of stress markers by hybridisation with oligonucleotide probes, incorporation of isolated nucleic acids into vectors, expression of vector-incorporated nucleic acids as RNA and protein, and development of immunological reagents corresponding to marker encoded products.

The identified stress markers may in turn be used to design specific oligonucleotide probes and primers. Such probes and primers may be of any length that would specifically hybridize to the identified marker gene sequences and may be at least about 10, 11, 12, 13, 14, 15, 16, 17, 18, 19, 20, 21, 22, 23, 24, 25, 26, 27, 28, 29, 30, 35, 40, 50, 75, 100, 150, 200, 300, 400, 500 nucleotides in length and in the case of probes, up to the full length of the sequences of the marker genes identified herein. Probes may also include additional sequence at their 5' and/or 3' ends so that they extent beyond the target sequence with which they hybridize.

When used in combination with nucleic acid amplification procedures, these probes and primers enable the rapid analysis of biological samples (e.g., peripheral blood samples) for detecting or quantifying marker gene transcripts. Such procedures include any method or technique known in the art or described herein for duplicating or increasing the number of copies or amount of a target nucleic acid or its complement.

The identified markers may also be used to identify and isolate full length gene sequences, including regulatory elements for gene expression, from genomic DNA libraries, which are suitably but not exclusively of equine origin. The cDNA sequences identified in the present disclosure may be used as hybridization probes to screen genomic DNA libraries by conventional techniques. Once partial genomic clones have been identified, full-length genes may be isolated by "chromosomal walking" (also called "overlap hybridization") using, for example, the method disclosed by Chinault & Carbon (1979, Gene 5: 111-126). Once a partial genomic clone has been isolated using a cDNA hybridization probe, non-repetitive segments at or near the ends of the partial genomic clone may be used as hybridization probes in further genomic library screening, ultimately allowing isolation of entire gene sequences for the stress markers of interest. It will be recognized that full-length genes may be obtained using the partial cDNA sequences or short expressed sequence tags (ESTs) described in this disclosure using standard techniques as disclosed for example by Sambrook, et al. (MOLECULAR CLONING. A LABORATORY MANUAL (Cold Spring Harbor Press, 1989) and Ausubel et al., (CURRENT PROTOCOLS IN MOLECULAR BIOLOGY, John Wiley & Sons, Inc. 1994). In addition, the disclosed sequences may be used-to identify and isolate full-length cDNA sequences using standard techniques as disclosed, for example, in the above-referenced texts. Sequences identified and isolated by such means may be useful in the detection of the stress marker genes using the detection methods described herein, and are part of the invention.

One of ordinary skill in the art could select segments from the identified marker genes for use in determining susceptibility, the different detection, diagnostic, or prognostic methods, vector constructs, antigen-binding molecule production, kit, and/or any of the embodiments described herein as part of the present invention. Marker gene sequences that are desirable for use in the invention are those set fort in SEQ ID NO: 1,3,4, 5,7,9,11,13,15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 12, 125, 126, 127, 129, 130, 131, 133, 135, 137, 139, 141, 143, 144, 145, 147, 148, 150, 151, 153, 155, 156, 158, 160, 161, 163, 164, 165, 167, 169; 170, 171, 173, 175, 175, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248 (see Table 1).

### 4. Nucleic acid molecules of the invention

As described in the Examples and in Table 1, the present disclosure provides 134 markers of stress (i.e., 134 stress marker genes), identified by GeneChip™ analysis of blood obtained from normal horses and from horses subjected to stress. Of the 134 marker genes, 96 have full-length or substantially full-length coding sequences and the remaining 38 have partial sequence information at one or both of their 5' and 3' ends. The identified stress marker genes include 38 previously uncharacterised equine genes.

In accordance with the present invention, the sequences of isolated nucleic acids disclosed herein find utility *inter alia* as hybridization probes or amplification primers. These nucleic acids may be used, for example, in diagnostic evaluation of biological samples or employed to clone full-length cDNAs or genomic clones corresponding thereto. In certain embodiments, these probes and primers represent oligonucleotides, which are of sufficient length to provide specific hybridization to a RNA or DNA sample extracted from the biological sample. The sequences typically will be about 10-20 nucleotides, but may be longer. Longer sequences, e.g., of about 30, 40, 50, 100, 500 and even up to full-length, are desirable for certain embodiments.

Nucleic acid molecules having contiguous stretches of about 10, 15, 17, 20, 30, 40, 50, 60, 75 or 100 or 500 nucleotides of a sequence set forth in any one of SEQ ID NO: 1, 3, 4, 5,7, 9, 11, 13, 15, 16, 17, 19, 21, 23, 24, 25, 26, 28, 29, 30, 32, 33, 34,35, 37, 38, 39, 40, 41, 42, 44, 46, 48, 50, 51, 52, 54, 55, 56, 57, 59, 62, 63, 64, 66, 68, 70; 71, 73, 75, 77 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248 are contemplated. Molecules that are complementary to the above mentioned sequences and that bind to these sequences under high stringency conditions are also contemplated. These probes are useful in a variety of hybridization embodiments, such as Southern and northern blotting. In some cases, it is contemplated that probes may be used that hybridize to multiple target sequences without compromising their ability to effectively measure a stress response. In general, it is contemplated that the hybridization probes described herein are useful both as reagents in solution hybridization, as in PCR, for detection of expression of corresponding genes; as well as in embodiments employing a solid phase.

Various probes-and primers may be designed around the disclosed nucleotide sequences. For example, in certain embodiments, the sequences used to design probes and primers may include repetitive stretches of adenine nucleotides (poly-A tails) normally attached at the ends of the RNA for the identified marker genes. In other embodiments, probes and primers may be specifically designed to not include these or other segments from the identified marker genes, as one of ordinary skilled in the art may deem certain segments more suitable for use in the detection methods disclosed. In any event, the choice of primer or probe sequences for a selected application is within the realm of the ordinary skilled practitioner. Illustrative probe sequences for detection of stress marker genes are presented in Table 2.

Primers may be provided in double-stranded or single-stranded form, although the single-stranded form is desirable. Probes, while perhaps capable of priming, are designed to bind to a target DNA or RNA and need not be used in an amplification process. In certain embodiments, the probes or primers are labelled with radioactive species ³²P, ¹⁴C, ³⁵S, ³H, or other label), with a fluorophore (e.g., rhodamine, fluorescein) or with a chemillumiscent label (e.g., luciferase).

The present invention provides 96 substantially full-length cDNA sequences as well as 59 EST or partial cDNA sequences that are useful as markers of stress. It will be understood, however, that the present disclosure is not limited to these disclosed sequences and is intended particularly to encompass at least isolated nucleic acids that are hybridizable to nucleic acids comprising the disclosed sequences or that are variants of these nucleic acids. For example, a nucleic acid of partial sequence may be used to identify a structurally-related gene or the full-length genomic or cDNA clone from which it is derived. Methods for generating cDNA and genomic libraries which may be used as a target for the above-described probes are known in the art (see, for example, Sambrook *et al.,* 1989, *supra* and Ausubel *et al.,* 1994, *supra).* All such nucleic acids as well as the specific nucleic acid molecules disclosed herein are collectively referred to as *"stress marker polynucleotides."* Additionally, the present invention includes within its scope isolated or purified expression products of stress marker polynucleotides (i.e., RNA transcripts and polypeptides).

Accordingly, the present invention encompasses isolated or substantially purified nucleic acid or protein compositions. An "isolated" or "purified" nucleic acid molecule or protein, or biologically active portion thereof, is substantially or essentially free from components that normally accompany or interact with the nucleic acid molecule or protein as found in its naturally occurring environment. Thus, an isolated or purified polynucleotide or polypeptide is substantially free of other cellular-material, or culture medium when produced by recombinant techniques, or substantially free of chemical precursors or other chemicals when chemically synthesized. Suitably, an "isolated" polynucleotide is free of sequences (especially protein encoding sequences) that naturally flank the polynucleotide (i.e., sequences located at the 5' and 3' ends of the polynucleotide) in the genomic DNA of the organism from which the polynucleotide was derived. For example, in various embodiments, an isolated stress marker polynucleotide can contain less than about 5 kb, 4 kb, 3 kb, 2 kb, 1 kb, 0.5 kb, or 0.1 kb of nucleotide sequences that naturally flank the polynucleotide in genomic DNA of the cell from which the polynucleotide was derived. A polypeptide that is substantially free of cellular material includes preparations of protein having less than about 30%, 20%, 10%, 5%, (by dry weight) of contaminating protein. When the protein of the invention or biologically active portion thereof is recombinantly produced, culture medium suitably represents less than about 30%, 20%, 10%, or 5% (by dry weight) of chemical precursors or non-protein-of-interest chemicals.

The present invention also encompasses portions of the full-length or substantially full-length nucleotide sequences of the stress marker genes or their transcripts or DNA copies of these transcripts. Portions of a stress marker nucleotide sequence may encode polypeptide portions or segments that retain the biological activity of the native polypeptide. Alternatively, portions of a stress marker nucleotide sequence that are useful as hybridization probes generally do not encode amino acid sequences retaining such biological activity. Thus, portions of a stress marker nucleotide sequence may range from at least about 15, 16, 17, 18, 19, 20, 21, 22, 23, 24, 25, 26, 27, 28, 29, 30, 40, 50, 60, 80, 90, 100 nucleotides, or almost up to the full-length nucleotide sequence encoding the stress marker polypeptides of the invention.

A portion of a stress marker nucleotide sequence that encodes a biologically active portion of a stress marker polypeptide of the invention may encode at least about 5, 6, 7, 8, 9, 10, 11, 12, 13, 14, 15, 16, 17, 18, 19, 20, 21, 22, 23, 24, 25, 30, 40, 50, 60, 70, 80, 90, 100, 120, 150, 300, 400, 500, 600, 700, 800, 900 or 1000, or even at least about 2000, 3000, 4000 or 5000 contiguous amino acid residues, or almost up to the total number of amino acids present in a full-length stress marker polypeptide. Portions of a stress marker nucleotide sequence that are useful as hybridization probes or PCR primers generally need not encode a biologically active portion of a stress marker polypeptide.

Thus, a portion of a stress marker nucleotide sequence may encode a biologically active portion of a stress marker polypeptide, or it may be a fragment that can be used as a hybridization probe or PCR primer using standard methods known in the art. A biologically active portion of a stress marker polypeptide can be prepared by isolating a portion of one of the stress marker nucleotide sequences of the invention, expressing the encoded portion of the stress marker polypeptide (e.g., by recombinant expression *in vitro*), and assessing the activity of the encoded portion of the stress marker polypeptide. Nucleic acid molecules that are portions of a stress marker nucleotide sequence comprise at least about 15, 16, 17, 18, 19, 20, 25, 30, 50, 75, 100, 150, 200, 250, 300, 350, 400, 450, 500, 550, 600, or 650 nucleotides, or almost up to the number of nucleotides present in a full-length stress marker nucleotide sequence.

The invention also contemplates variants of the stress marker nucleotide sequences. Nucleic acid variants can be naturally-occurring, such as allelic variants (same locus), homologues (different locus), and orthologues different organism) or can be non naturally-occurring. Naturally occurring variants such as these can be identified with the use of well-known molecular biology techniques, as, for example, with polymerase chain reaction (PCR) and hybridization techniques as known in the art. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product). For nucleotide sequences, conservative variants include those sequences that, because of the degeneracy of the genetic code, encode the amino acid sequence of one of the stress marker polypeptides of the invention. Variant nucleotide sequences also include synthetically derived nucleotide sequences, such as those generated, for example, by using site-directed mutagenesis but which still encode a stress marker polypeptide of the invention. Generally, variants of a particular nucleotide sequence of the invention will have at least about 30%, 40% 50%, 55%, 60%, 65%, 70%, generally at least about 75%, 80%, 85%, desirably about 90% to 95% or more, and more suitably about 98% or more sequence identity to that particular nucleotide sequence as determined by sequence alignment programs described elsewhere herein using default parameters.

The stress marker nucleotide sequences of the invention can be used to isolate corresponding sequences and alleles from other organisms, particularly other mammals, especially other equine species. Methods are readily available in the art for the hybridization of nucleic acid sequences. Coding sequences from other organisms may be isolated according to well known techniques based on their sequence identity with the coding sequences set forth herein. In these techniques all or part of the known coding sequence is used as a probe which selectively hybridizes to other stress marker coding sequences present in a population of cloned genomic DNA fragments or cDNA fragments (i.e., genomic or cDNA libraries) from a chosen organism. Accordingly, the present invention also contemplates polynucleotides that hybridize to the stress marker gene nucleotide sequences, or to their complements, under stringency conditions described below. As used herein, the term "hybridizes under low stringency, medium stringency, high stringency, or very high stringency conditions" describes conditions for hybridization and washing. Guidance for performing hybridization reactions can be found in *Ausubel et al.,* (1998, *supra*), Sections 6.3.1-63.6. Aqueous and non-aqueous methods are described in that reference and either can be used. Reference herein to low stringency conditions include and encompass from at least about 1% v/v to at least about 15% v/v formamide and from at least about 1 M to at least about 2 M salt for hybridization at 42° C, and at least about 1 M to at least about 2 M salt for washing at 42° C. Low stringency conditions also may include 1% Bovine Serum Albumin (BSA), 1 mM EDTA, 0.5 M NaHPO₄ (pH 7.2), 7% SDS for hybridization at 65° C, and (i) 2 x SSC, 0.1% SDS; or (ii) 0.5% BSA, 1 mM EDTA, 40 mM NaHPO₄ (pH 7.2), 5% SDS for washing at room temperature. One embodiment of low stringency conditions includes hybridization in 6 x sodium chloride/sodium citrate (SSC) at about 45° C, followed by two washes in 0.2 x SSC, 0.1% SDS at least at 50° C (the temperature of the washes can be increased to 55° C for low stringency conditions). Medium stringency conditions include and encompass from at least about 16% v/v to at least about 30% v/v formamide and from at least about 0.5 M to at least about 0.9 M salt for hybridization at 42° C, and at least about 0.1 M to at least about 0.2 M salt for washing at 55° C. Medium stringency conditions also may include 1% Bovine Serum Albumin (BSA), 1 mM EDTA, 0.5 M NaHPO₄ (pH 7.2), 7% SDS for hybridization at 65° C, and (i) 2 × SSC, 0.1% SDS; or (ii) 0.5% BSA, 1 mM EDTA, 40 mM NaHPO₄ (pH 7.2), 5% SDS for washing at 60-65° C. One embodiment of medium stringency conditions includes hybridizing in 6 × SSC at about 45° C, followed by one or more washes in 0.2 × SSC, 0.1% SDS at_60° C. High stringency conditions-include and encompass from at least about 31% v/v to at least about 50% v/v formamide and from about 0.01 M to about 0.15 M salt for hybridization at 42° C, and about 0.01 M to about 0.02 M salt for washing at 55° C. High stringency conditions also may include 1% BSA, 1 mM EDTA, 0.5 M NaHPO₄ (pH 7.2), 7% SDS for hybridization-at 65° C, and (i) 0.2 × SSC, 0.1% SDS; or (ii) 0.5% BSA, 1 mM EDTA, 40 mM NaHPO₄ (pH 7.2), 1% SDS for washing at a temperature in excess of 65° C. One embodiment of high stringency conditions includes hybridizing in 6 × SSC at about 45° C, followed by one or more washes in 0.2 × SSC, 0.1% SDS at 65° C.

In certain embodiments, a stress marker polynucleotide of the invention hybridises to a disclosed nucleotide sequence under very high stringency conditions. One embodiment of very high stringency conditions includes hybridising in 0.5 M sodium phosphate, 7% SDS at 65° C, followed by one or more washes at 0.2 × SSC, 1% SDS at 65° C.

Other stringency conditions are well known in the art and a skilled person will recognize that various factors can be manipulated to optimize the specificity of the hybridization. Optimization of the stringency of the final washes can serve to ensure a high degree of hybridization. For detailed examples, see Ausubel *et al., supra* at pages 2.10.1 to 2.10.16 and Sambrook *et al.* (1989, *supra)* at sections 1.101 to 1.104.

While stringent washes are typically carried out at temperatures from about 42° C to 68° C, one skilled in the art will appreciate that other temperatures may be suitable for stringent conditions. Maximum hybridization rate typically occurs at about 20° C to 25° C below the Tₘ for formation of a DNA-DNA hybrid. It is well known in the art that the Tₘ is the melting temperature, or temperature at which two complementary polynucleotide sequences dissociate. Methods for estimating Tₘ are well known in the art (see Ausubel *et al., supra* at page 2.10.8). In general, the Tₘ of a perfectly matched duplex of DNA may be predicted as an approximation by the formula:

Tₘ = 81.5 + 16.6 (log₁₀ M) + 0.41 (%G+C) - 0.63 (% formamide)-(600/length)

wherein: M is the concentration of Na⁺, preferably in the range of 0.01 molar to 0.4 molar; %G+C is the sum of guanosine and cytosine bases as a percentage of the total number of bases, within the range between 30% and 75% G+C; % formamide is the percent formamide concentration by volume; length is the number of base pairs in the DNA duplex. The Tₘ of a duplex DNA decreases by approximately 1° C with every increase of 1% in the number of randomly mismatched base pairs. Washing is generally carried out at Tₘ - 15° C for high stringency, or Tₘ - 30° C for moderate stringency.

In one example of a hybridization procedure, a membrane (e.g., a nitrocellulose membrane or a nylon membrane) containing immobilized DNA is hybridized overnight at 42° C in a hybridization buffer (50%-deionised formamide, 5 × SSC, 5 × Denhardt's solution (0.1% ficoll, 0.1 % polyvinylpyrollidone and 0.1% bovine serum albumin), 0.1 % SDS and 200 mg/mL denatured salmon sperm DNA) containing labeled probe. The membrane is then subjected to two sequential medium stringency washes (i.e., 2 × SSC, 0.1% SDS for 15 min at 45° C, followed by 2 × SSC, 0.1% SDS for 15 min at 50° C), followed by two sequential higher stringency washes (i.e., 0.2 × SSC, 0.1% SDS for 12 min at 55°C followed by 0.2 × SSC and 0.1%SDS solution for 12 min at 65-68° C.

### 5. Polypeptides of the invention

The present invention also contemplates full-length polypeptides encoded by the stress marker genes of the invention as well as the biologically active portions of those polypeptides, which are referred to collectively herein as "stress marker polypeptides". Biologically active portions of full-length stress marker polypeptides include portions with immune-interactive activity of at least about 6, 8, 10, 12, 14, 16, 18, 20, 25, 30, 40, 50, 60 amino acid residues in length. For example, immuno-interactive fragments contemplated by the present invention are at least 6 and desirably at least 8 amino acid residues in length, which can elicit an immune response in an animal for the production of antigen-binding molecules that are-immuno-interactive with a stress marker polypeptide of the invention. Such antigen-binding molecules can be used to screen other mammals, especially equine mammals, for structurally and/or functionally related stress marker polypeptides. Typically, portions of a full-length stress marker polypeptide may participate in an interaction, for example, an intramolecular or an inter-molecular interaction. An inter-molecular interaction can be a specific binding interaction or an enzymatic interaction (e.g., the interaction can be transient and a covalent bond is formed or broken). Biologically active portions of a full-length stress marker polypeptide include peptides comprising amino acid sequences sufficiently similar to or derived from the amino acid sequences of a (putative) full-length stress marker polypeptide, for example, the amino acid sequences shown in SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, which include less amino acids than a full-length stress marker polypeptide; and exhibit at least one activity of that polypeptide. Typically, biologically active portions comprise a domain or motif with at least one activity of a full-length stress marker polypeptide. A biologically active portion of a full-length stress marker polypeptide can be a polypeptide which is, for example, 5, 6, 7, 8, 9, 10, 11, 12, 13, 14, 15, 16, 17, 18, 19, 20, 21, 22, 23, 24, 25, 30, 40, 50, 60, 70, 80, 90, 100, 120, 150, 300, 400, 500, 600, 700, 800, 900 or 1000, or even at least-about 2000, 3000, 4000 or 5000, or more amino acid residues in length. Suitably, the portion is a "biologically-active portion" having no less than about 1%, 10%, 25% 50% of the activity of the full-length polypeptide from which it is derived.

The present invention also contemplates variant stress marker polypeptides. "Variant" polypeptides include proteins derived from the native protein by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Variant proteins encompassed by the present invention are biologically active, that is, they continue to possess the desired biological activity of the native protein. Such variants may result from, for example, genetic polymorphism or from human manipulation. Biologically active variants of a native stress marker protein of the invention will have-at least 40%, 50%, 60%, 70%, generally at least 75%, 80%, 85%, preferably about 90% to 95% or more, and more preferably about 98% or more sequence similarity with the amino acid sequence for the native protein as determined by sequence alignment programs described elsewhere herein using default-parameters. A biologically active variant of a protein of the invention may differ from that protein generally by as much 1000, 500, 400, 300, 200, 100, 50, or 20 amino acid residues or suitably by as few as 1-15 amino acid residues, as few as 1-10, such as 6-10, as few as 5, as few -as 4, 3, 2, or even 1 amino acid residue.

A stress marker polypeptide of the invention may be altered in various ways including amino acid substitutions, deletions, truncations, and insertions. Methods for such manipulations are generally known in the art. For example, amino acid sequence variants of a stress marker protein can be prepared by mutations in the DNA. Methods for mutagenesis and nucleotide sequence alterations are well known in the art. See, for example, Kunkel (1985, Proc. Natl. Acad. Sci. USA 82:488-492), Kunkel et al. (1987, Methods in Enzymol. 154:367-382), U.S. Pat. No. 4,873,192, Watson, J. D. et al. ("Molecular Biology of the Gene", Fourth Edition, Benjamin/Cummings, Menlo Park, Calif., 1987) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.). Methods for screening gene products of combinatorial libraries made by point mutations or truncation, and for screening cDNA libraries for gene products having a selected property are known in the art. Such methods are adaptable for rapid screening of the gene libraries generated by combinatorial mutagenesis of stress marker polypeptides. Recursive ensemble mutagenesis (REM), a technique which enhances the frequency of functional mutants in the libraries, can be used in combination with the screening assays to identify stress marker polypeptide variants (Arkin and Yourvan (1992) Proc. Natl. Acad. Sci. USA 89:7811-7815; Delgrave et al. (1993) Protein Engineering 6:327-331). Conservative substitutions, such as exchanging one amino acid with another having similar properties, may be desirable as discussed in more detail below.

Variant stress marker polypeptides may contain conservative amino acid substitutions at various locations along their sequence, as compared to the parent stress marker amino acid sequence. A "conservative amino acid substitution" is one in which the amino acid residue is replaced with an amino acid residue having a similar side chain. Families of amino acid residues having similar side chains have been defined in the art, which can be generally sub-classified as follows:

Acidic: The residue has a negative charge due to loss of H ion at physiological pH and the residue is attracted by aqueous solution so as to seek the surface positions in the conformation of a peptide in which it is contained when the peptide is in aqueous medium at physiological pH. Amino acids having an acidic side chain include glutamic acid and aspartic acid.

Basic: The residue has a positive charge due to association with H ion at physiological pH or within one or two pH units thereof (e.g., histidine) and the residue is attracted by aqueous solution so as to seek the surface positions in the conformation of a peptide in which it is contained when the peptide is in aqueous medium at physiological pH. Amino acids-having a basic side chain include arginine, lysine and histidine.

Charged: The residues-are charged at physiological pH and, therefore, include amino acids having acidic or basic side chains (i.e., glutamic acid, aspartic acid, arginine, lysine and histidine).

Hydrophobic: The residues are not charged at physiological pH and the residue is repelled by aqueous solution so as to seek the inner positions in the conformation of a peptide in which it is contained when the peptide is in aqueous medium. Amino acids having a hydrophobic side chain include tyrosine, valine, isoleucine, leucine, methionine, phenylalanine and tryptophan.

Neutral/polar: The residues are not charged at physiological pH, but the residue is not sufficiently repelled by aqueous solutions so that it would seek inner positions in the conformation of a peptide in which it is contained when the peptide is in aqueous medium. Amino acids having a neutral/polar side chain include asparagine, glutamine, cysteine, histidine, serine and threonine.

This description also characterizes certain amino acids as "small" since their side chains are not sufficiently large, even if polar groups are lacking, to confer hydrophobicity. With the exception of proline, "small" amino acids are those with four carbons or less when at least one polar group is on the side chain and three carbons or less when not. Amino acids having a small side chain include glycine, serine, alanine and threonine. The gene-encoded secondary amino acid proline is a special case due to its known effects on the secondary conformation of peptide chains. The structure of proline differs from all the other naturally-occurring amino acids in that its side chain is bonded to the nitrogen of the α-amino group, as well as the α-carbon. Several amino acid similarity matrices (e.g., PAM120 matrix and PAM250 matrix as disclosed for example by Dayhoff et al. (1978) A model of evolutionary change in proteins. Matrices for determining distance relationships In M. O. Dayhoff, (ed.), Atlas of protein sequence and structure, Vol. 5, pp. 345-358, National Biomedical Research Foundation, Washington DC; and by Gonnet et al., 1992, Science 256(5062): 144301445), however, include proline in the same group as glycine, serine, alanine and threonine. Accordingly, for the purposes of the present invention, proline is classified as a "small" amino acid.

The degree of attraction or repulsion required for classification as polar or nonpolar is arbitrary and, therefore, amino acids specifically contemplated by the invention have been classified as one or the other. Most amino acids not specifically named can be classified on the basis of known behavior.

Amino acid residues can be further sub-classified as cyclic or noncyclic, and aromatic or nonaromatic, self-explanatory classifications with respect to the side-chain substituent groups of the residues, and as small or large. The residue is considered small if it contains a total of four carbon atoms or less, inclusive of the carboxyl carbon, provided an additional polar substituent is present; three or less if not. Small residues are, of course, always nonaromatic. Dependent on their structural properties, amino acid residues may fall in two or more classes. For the naturally-occurring protein amino acids, sub-classification according to the this scheme is presented in the Table 3.

Conservative amino acid substitution also includes groupings based on side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. For example, it is reasonable to expect that replacement of a leucine with an isoleucine or valine, an-aspartate with a glutamate, a threonine with a serine, or a similar replacement of an amino acid with a structurally related amino acid will not have a major effect on the properties of the resulting variant polypeptide. Whether an amino acid change results in a functional stress marker polypeptide can readily be determined by assaying its activity. Conservative substitutions are shown in Table 4 under the heading of exemplary substitutions. More preferred substitutions are shown under the heading of preferred substitutions. Amino acid substitutions falling within the scope of the invention, are, in general, accomplished by selecting substitutions that do not differ significantly in their effect on maintaining (a) the structure of the peptide backbone in the area of the substitution, (b) the charge or hydrophobicity of the molecule at the target site, or (c) the bulk of the side chain. After the substitutions are introduced, the variants are screened for biological activity.

Alternatively, similar amino acids for making conservative substitutions can be grouped into three categories based on the identity of the side chains. The first group includes glutamic acid, aspartic acid, arginine, lysine, histidine, which all have charged side chains; the second group includes glycine, serine, threonine, cysteine, tyrosine, glutamine, asparagine; and the third group includes leucine, isoleucine, valine, alanine, proline, phenylalanine, tryptophan, methionine, as described in Zubay, G., Biochemistry, third edition, Wm.C. Brown Publishers (1993).

Thus, a predicted non-essential amino acid residue in a stress marker polypeptide is typically replaced with another amino-acid residue from the same side chain family. Alternatively, mutations can be introduced randomly along all or part of a stress-marker gene coding sequence, such as by saturation mutagenesis, and the resultant mutants can be screened for an activity of the parent polypeptide to-identify mutants which retain that activity. Following mutagenesis of the coding sequences, the encoded peptide can be expressed recombinantly and the activity of the peptide can be determined.

Accordingly, the present invention also contemplates variants of the naturally-occurring stress marker polypeptide sequences or their biologically-active fragments, wherein the variants are distinguished from the naturally-occurring sequence by the addition, deletion, or substitution of one or more amino acid residues. In general, variants will display at least about 30, 40, 50, 55, 60, 65, 70, 75, 80, 85, 90, 91, 92, 93, 94, 95, 96, 97, 98, 99 % similarity to a parent stress marker polypeptide sequence as, for example, set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249. Desirably, variants will have at least 30, 40, 50, 55, 60, 65, 70, 75, 80, 85, 90, 91, 92, 93, 94, 95, 96, 97, 98, 99 % sequence identity to a parent stress marker polypeptide sequence as, for example, set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249. Moreover, sequences differing from the native or parent sequences by the addition, deletion, or substitution of 1, 2, 3, 4, 5, 6, 7, 8, 9, 10, 11, 12, 13, 14, 15, 16, 17, 18, 19, 20, 30, 40, 50, 60 70, 80 90, 100, 150, 200, 300, 500 or more amino acids but which retain the properties of the parent stress marker polypeptide are contemplated. stress marker polypeptides also include polypeptides that are encoded by polynucleotides that hybridise under stringency conditions as defined herein, especially high stringency conditions, to the stress marker polynucleotide sequences of the invention, or the non-coding strand thereof, as described above.

- In one embodiment, variant polypeptides differ from a stress marker sequence by at least one but by less than 50, 40, 30, 20, 15, 10, 8, 6, 5, 4, 3 or 2 amino acid residue(s). In another, variant polypeptides differ from the corresponding sequence in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229,231, 235, 237, 245, 247 or 249 by at least 1% but less than 20%, 15%, 10% or 5% of the residues. (If this comparison requires alignment the sequences should be aligned for maximum similarity. "Looped" out sequences from deletions or insertions, or mismatches, are considered differences.) The differences are, suitably, differences or changes at a non-essential residue or a conservative substitution.

A "non-essential" amino acid residue is a residue that can be altered from the wild-type sequence of an embodiment polypeptide without abolishing or substantially altering one or more of its activities. Suitably, the alteration does not substantially alter one of these activities, for example, the activity is at least 20%, 40%, 60%, 70% or 80% of wild-type. An "essential" amino acid residue is a residue that, when altered from the wild-type sequence of a stress marker polypeptide of the invention, results in abolition of an activity of the parent molecule such that less than 20% of the wild-type activity is present.

In other embodiments, a variant polypeptide includes an amino acid sequence having at least about 50%, 55%, 60%, 65%, 70%, 75%, 80%, 85%, 90%, 91%, 92%, 93%, 94% 95%, 96%, 97%, 98% or more similarity to a corresponding sequence of a stress marker polypeptide as, for example, set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, and has the activity of that stress marker polypeptide.

Stress marker polypeptides of the invention may be prepared by any suitable procedure known to those of skill in the-art. For example, the polypeptides may be prepared by a procedure including the steps of: (a) preparing a chimeric construct comprising a nucleotide sequence that encodes at least a portion of a stress marker polynucleotide and that is operably linked to a regulatory element; (b) introducing the chimeric construct into a host cell; (c) culturing the host cell to express the stress marker polypeptide; and (d) isolating the stress marker polypeptide from the host cell. In illustrative examples, the nucleotide sequence encodes at least a portion of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140,142, 146, 149, 152, 154, 157, 159, 162, 166, 168, 172, 174, 77, 179, 181, 183, 189, 191, 193, 197, 199, 201, 203, 205, 207, 209, 211, 213, 215, 217, 219, 221, 223, 225, 227, 229, 231, 235, 237, 245, 247 or 249, or a variant thereof.

The chimeric construct is typically in the form of an expression vector, which is suitably selected from self replicating extra-chromosomal vectors (e.g., plasmids) and vectors that integrate into a host genome.

The regulatory element will generally be appropriate for the host cell employed for expression of the stress marker polynucleotide: Numerous types of expression vectors and regulatory elements are known in the art for a variety of host cells. Illustrative elements of this type include, but are not restricted to, promoter sequences (e.g., constitutive or inducible promoters which may be naturally occurring or combine elements of more than one promoter), leader or signal sequences, ribosomal binding sites, transcriptional start and stop sequences, translational start and termination sequences, and enhancer or activator sequences.

In some embodiments, the expression vector comprises a selectable marker gene to permit the selection of transformed host cells. Selectable marker genes are well known in the art and will vary with the host cell employed.

The expression vector may also include a fusion partner (typically provided by the expression vector) so that the stress marker polypeptide is produced as a fusion polypeptide with the fusion partner. The main advantage of fusion partners is that they assist identification and/or purification of the fusion polypeptide. In order to produce the fusion polypeptide, it is necessary to ligate the stress marker polynucleotide into an expression vector so that the translational reading frames of the fusion partner and the stress marker polynucleotide coincide. Well known examples of fusion partners include, but are not limited to, glutathione-S-transferase (GST), Fc potion of human IgG, maltose binding protein (MBP) and hexahistidine (HIS₆), which are particularly useful for isolation of the fusion polypeptide by affinity chromatography. In some embodiments, fusion polypeptides are purified by affinity chromatography using matrices to which the fusion partners bind such as but not limited to glutathione-, amylose-, and nickel- or cobalt-conjugated resins. Many such matrices are available in "kit" form, such as the QIAexpress™ system (Qiagen) useful with (HIS₆) fusion partners and the Pharmacia GST purification system. Other fusion partners known in the art are light-emitting proteins such as green fluorescent protein (GFP) and luciferase, which serve as fluorescent "tags" that permit the identification and/or isolation of fusion polypeptides by fluorescence microscopy or by flow cytometry. Flow cytometric methods such as fluorescence activated cell sorting (FACS) are particularly useful in this latter application.

Desirably, the fusion partners also possess protease cleavage sites, such as for Factor Xₐ or Thrombin, which permit the relevant protease to partially digest the fusion polypeptide and thereby liberate the stress marker polypeptide from the fusion construct. The liberated polypeptide can then be isolated from the fusion partner by subsequent chromatographic separation.

Fusion partners also include within their scope "epitope tags," which-are usually short peptide sequences for which a specific antibody is available. Well known examples of epitope tags for which specific monoclonal antibodies are readily available include e-Myc, influenza virus, hemagglutinin and FLAG tags.

The chimeric constructs of the invention are introduced into a host by any suitable means including "transduction" and "transfection," which are art recognized as meaning the introduction of a nucleic acid, for example, an expression vector, into a recipient cell by nucleic acid-mediated gene transfer. "Transformation," however, refers to a process in which a host's genotype is changed as a result of the cellular uptake of exogenous DNA or RNA, and, for example, the transformed cell comprises the expression system of the invention. There are many methods for introducing chimeric constructs into cells. Typically, the method employed will depend on the choice of host cell. Technology for introduction of chimeric constructs into host cells is well known to those of skill in the art. Four general classes of methods for delivering nucleic acid molecules into cells have been described: (1) chemical methods such as calcium phosphate precipitation, polyethylene glycol (PEG)-mediate precipitation and lipofection; (2) physical methods such as microinjection, electroporation, acceleration methods and vacuum infiltration; (3) vector based methods such as bacterial and viral vector-mediated transformation; and (4) receptor-mediated. Transformation techniques that fall within these and other classes are well known to workers in the art, and new techniques are continually becoming known. The particular choice of a transformation technology will be determined by its efficiency to transform certain host species as well as the experience and preference-of the person practising the invention with a particular methodology of choice. It will be apparent to the skilled person that the particular choice of a transformation system to introduce a chimeric construct into cells is not essential to or a limitation of the invention, provided it achieves an acceptable level of nucleic acid transfer.

Recombinant stress marker polypeptides may be produced by culturing a host cell transformed with a chimeric construct. The conditions appropriate for expression of the stress marker polynucleotide will vary with the choice of expression vector and the host cell and are easily ascertained by one skilled in the art through routine experimentation. Suitable host cells for expression may be prokaryotic or eukaryotic. An illustrative host cell for expression of a polypeptide of the invention is a bacterium. The bacterium used may be *Escherichia coli.* Alternatively, the host cell may be a yeast cell or an insect cell such as, for example, *SF9* cells that may be utilized with a baculovirus expression system.

Recombinant stress marker polypeptides can be conveniently prepared using standard protocols as described for example in Sambrook, *et al.,* (1989, *supra*), in particular Sections 16 and 17; Ausubel *et al.,* (1994, *supra*), in particular Chapters 10 and 16; and Coligan et al., CURRENT PROTOCOLS IN PROTEIN SCIENCE (John Wiley & Sons, Inc. 1995-1997), in particular Chapters 1, 5 and 6. Alternatively, the stress marker polypeptides may be synthesized by chemical synthesis; e.g., using solution synthesis or solid phase synthesis as described, for example, in Chapter 9 of Atherton and Shephard *(supra)* and in Roberge et al (1995, Science 269:202).

### 6. Antigen-binding-molecules

The invention also provides antigen-binding molecules that are specifically immuno-interactive with a stress marker polypeptide of the invention. In one embodiment, the antigen-binding molecule comprise whole polyclonal antibodies. Such antibodies may be prepared, for example, by injecting a stress marker polypeptide of the invention into a production species, which may include mice or rabbits, to obtain polyclonal antisera. Methods of producing polyclonal antibodies are well known to those skilled in the art. Exemplary protocols which may be used are described for example in Coligan et al., CURRENT PROTOCOLS IN IMMUNOLOGY, (John Wiley & Sons, Inc, 1991), and Ausubel *et al.,* (1994-1998, *supra*), in particular Section III of Chapter 11.

In lieu of polyclonal antisera obtained in a production species, monoclonal antibodies may be produced using the standard method as described, for example, by Köhler and Milstein (1975, Nature 256, 495-497), or by more recent modifications thereof as described, for example, in Coligan *et al.,* (1991, *supra*) by immortalizing spleen or other antibody producing cells derived from a production species which has been inoculated with one or more of the stress marker polypeptides of the invention.

The invention also contemplates as antigen-binding molecules Fv, Fab, Fab' and F(ab')₂ immunoglobulin fragments. Alternatively, the antigen-binding molecule may comprise a synthetic stabilized Fv fragment. Exemplary fragments of this type include single chain Fv fragments (sFv, frequently termed scFv) in which a peptide linker is used to bridge the N terminus or C terminus of a V*_{H}* domain with the C terminus or N-terminus, respectively, of a V*_{L}* domain. ScFv lack all constant parts of whole antibodies and are not able to activate complement. ScFvs may be prepared, for example, in accordance with methods outlined in Kreber *et al* (Kreber et al. 1997, J. Immunol. Methods; 201(1): 35-55). Alternatively, they may be prepared by methods described in U.S. Patent No 5,091,513, European Patent No 239,400 or the articles by Winter and Milstein (1991, Nature 349:293) and Plückthun et al (1996, In Antibody engineering: A practical approach. 203-252). In another embodiment, the synthetic stabilised Fv fragment comprises a disulphide stabilised Fv (dsFv) in which cysteine residues are introduced into the V*_{H}* and V*_{L}* domains such that in the fully folded Fv molecule the two residues will form a disulphide bond between them. Suitable methods of producing dsFv are described for example in (Glockscuther et al. Biochem. 29: 1363-1367; Reiter et al. 1994, J. Biol. Chem. 269: 18327-18331; Reiter et al. 1994, Biochem. 33: 5451-5459; Reiter et al. 1994. Cancer Res. 54: 2714-2718; Webber et al. 1995, Mol. Immunol. 32: 249-258).

Phage display and combinatorial methods for generating anti-stress marker polypeptide antigen-binding molecules are known in the art (as described in, e.g., Ladner et al. U.S. Patent No. 5,223,409; Kang *et al.* International Publication No. WO 92/18619; Dower *et al*. International Publication No. WO 91/17271; Winter *et al*. International Publication WO 92/20791; Markland *et al.* International Publication No. WO 92/15679; Breitling *et al.* International Publication WO 93/01288; McCafferty *et al.* International Publication No. WO 92/01047; Garrard *et al.* International Publication No. WO 92/09690; Ladner *et al.* International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982). The antigen-binding molecules can be used to screen expression libraries for variant stress marker polypeptides. They can also be used to detect and/or isolate the stress marker polypeptides of the invention. Thus, the invention also contemplates the use of antigen-binding molecules to isolate stress marker polypeptides using, for example, any suitable immunoaffinity based method including, but not limited to, immunochromatography and immunoprecipitation. A suitable method utilises solid phase adsorption in which anti- stress marker polypeptide antigen-binding molecules are attached to a suitable resin, the resin is contacted with a sample-suspected of containing a stress marker polypeptide, and the stress marker polypeptide, if any, is subsequently eluted from the resin. Illustrative resins include: Sepharose® (Pharmacia), Poros® resins (Roche Molecular Biochemicals, Indianapolis), Actigel Superflow™ resins (Sterogene Bioseparations Inc., Carlsbad Calif.), and Dynabeads™ (Dynal Inc., Lake Success, N.Y.).

The antigen-binding molecule can be coupled to a compound, e.g., a label such as a radioactive nucleus, or imaging agent, e.g. a radioactive, enzymatic, or other, e.g., imaging agent, e.g., a NMR contrast agent. Labels which produce detectable radioactive emissions or fluorescence are preferred. An anti- stress marker polypeptide antigen-binding molecule (e.g., monoclonal antibody) can be used to detect stress marker polypeptides (e.g., in a cellular lysate or cell supernatant) in order to evaluate the abundance and pattern of expression of the protein. In certain advantageous application in accordance with the present invention, such antigen-binding molecules can be used to monitor stress marker polypeptides levels in biological samples (including whole cells and fluids) for diagnosing the presence, absence, degree, of stress or risk of development of disease as a consequences of stress. Detection can be facilitated by coupling (i.e., physically linking) the antibody to a detectable substance (i.e., antibody labeling). Examples of detectable substances include various enzymes, prosthetic groups, fluorescent materials, luminescent materials, biolumniescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ^{12S}I, ¹³¹I, ³⁵S or ³H. The label may be selected from a group including a chromogen, a catalyst, an enzyme, a fluorophore, a chemiluminescent molecule, a lanthanide ion such as Europium (Eu³⁴), a radioisotope and a direct visual label. In the case of a direct visual label, use may be made of a colloidal metallic or non-metallic particle, a dye particle, an enzyme or a substrate, an organic polymer, a latex particle, a liposome, or other vesicle containing a signal producing substance and the like.

A large number of enzymes useful as labels is disclosed in United States Patent Specifications U.S. 4,366,241, U.S. 4,843,000, and U.S. 4,849,338. Enzyme labels useful in the present invention include alkaline phosphatase, horseradish peroxidase, luciferase, β-galactosidase, glucose oxidase, lysozyme, malate dehydrogenase and the like. The enzyme label may be used alone or in combination with a second enzyme in solution.

### 7. Methods of detecting aberrant stress marker gene expression or alleles

The present invention is predicated in part on the discovery that horses subjected to stress have aberrant expression of certain genes or certain alleles of genes, referred to herein as stress marker genes, as compared to horses not subjected to stress. It is proposed that aberrant expression of these genes or their homologues or orthologues will be found in other animals under stress. Accordingly, the present invention features a method for assessing stress or for diagnosing stress or a stress-related condition (stress sequelae) in a subject, which is suitably a mammal, by detecting aberrant expression of a stress marker gene in abiological sample obtained from the subject. According to some embodiments, the related condition is characterized by elevated levels of corticosteroids or their modulators-(e.g., corticotropin releasing factor). Illustrative examples of such related conditions include: physical stress such as athletic training and physical trauma; mood disorders such as depression, including major depression, single episode depression, recurrent depression, child abuse induced depression, seasonal affective disorder, postpartum depression, dysthemia, bipolar disorders, and cyclothymia; anxiety disorders including panic, phobias, obsessive-compulsive disorder; post-traumatic stress disorder; and sleep disorders induced by stress; inflammation; pain; chronic fatigue syndrome; stress-induced headache; cancer; human immunodeficiency virus (HIV) infections; neurodegenerative diseases such as Alzheimer's disease, Parkinson's disease and Huntington's disease; gastrointestinal diseases such as ulcers, irritable bowel syndrome, Crohn's disease, spastic colon, diarrhea, and post operative ileus, and colonic hypersensitivity associated by psychopathological disturbances or stress; eating disorders such as anorexia and bulimia nervosa; supranuclear palsy; amyotrophic lateral sclerosis; a decrease in immune function or immunosuppression; hemorrhagic stress; stress-induced psychotic episodes; euthyroid sick syndrome; syndrome of inappropriate antidiarrhetic hormone (ADH); overeating or obesity; infertility; head traumas; spinal cord trauma; ischemic neuronal damage (e.g., cerebral ischemia such as cerebral hippocampal ischemia); excitotoxic neuronal damage; epilepsy; cardiovascular disorders including hypertension, tachycardia and congestive heart failure; stroke; immune dysfunctions including stress-induced immune dysfunctions (e.g., stress induced fevers, porcine stress syndrome, bovine shipping fever, equine paroxysmal fibrillation, and dysfunctions induced by confinement in chickens, sheering stress in sheep or human-animal interaction related stress in dogs); restraint; behavioral (operant) conditioning; muscular spasms; urinary incontinence; senile dementia of the Alzheimer's type; multiinfarct dementia; amyotrophic lateral sclerosis; chemical dependencies and addictions (e.g., dependencies on alcohol, cocaine, heroin, benzodiazepines, or other drugs); drug and alcohol withdrawal symptoms; osteoporosis; psychosocial dwarfism; hypoglycemia; hair loss; abnormal circadian rhythm; and disorders related to abnormal circadian rhythm such as time zone change syndrome, seasonal affective disorder, sleep deprivation, irregular sleep-wake pattern, delayed sleep phase syndrome, advanced sleep phase syndrome, non-24 hour-sleep wake disorder, light-induced clock resetting, REM sleep disorder, hypersomnia, parasomnia, narcolepsy, nocturnal enuresis, restless legs syndrome, sleep apnea, dysthymia, and abnormal circadian rhythm associated with chronic administration and withdrawal of antidepressant agents.

In order to make the assessment or the diagnosis, it will be desirable to qualitatively or quantitatively determine the levels of stress marker gene transcripts, or the presence of levels of particular alleles of a stress marker gene, or the level or functional activity of stress marker polypeptides. In some embodiments, the presence; degree or stage of stress or risk of development of stress sequelae is diagnosed when a stress marker gene product is present at a detectably lower level in the biological sample as compared to the level at which that gene is present in a reference sample obtained from normal subjects or from subjects not under stress. In other embodiments, the presence, degree or stage of stress or risk of development of stress sequelae is diagnosed when a stress marker gene product is present at a detectably higher level in the biological sample as compared to the level at which that gene is present in a reference sample obtained from normal subjects or from subjects free of stress. Generally, such diagnoses are made when the level or functional activity of a stress marker gene product in the biological sample varies from the level or functional activity of a corresponding stress marker gene product in the reference sample by at least 10%, 20%, 30%, 40%, 50%, 60%, 70%, 80%, 90%, 92%, 94%, 96%, 97%, 98% or 99%, or-even by at least about 99.5%, 99.9%, 99.95%, 99.99%, 99.995% or 99.999%, or even by at least about 100%, 200%, 300%, 400%, 500%, 600%, 700%, 800%, 900% or 1000%. Illustrative increases or decreases in the expression level of representative stress marker genes are shown in Table 6.

The corresponding gene product is generally selected from the same gene product that is present in the biological sample, a gene product expressed from a variant gene (e.g., an homologous or orthologous gene) including an allelic variant, or a splice variant or protein product thereof. In some embodiments, the method comprises measuring the level or functional activity of individual expression products of at least about 2, 3, 4, 5, 6, 7, 8, 9, 10, 11, 12, 13, 14, 15, 16, 17, 18, 19, 20, 21, 22, 23, 24, 25, 26, 27, 28, 29 or 30 stress marker genes.

Generally, the biological sample contains blood, especially peripheral blood, or a fraction or extract thereof. Typically, the biological sample comprises blood cells such as mature, immature and developing leukocytes, including lymphocytes, polymorphonuclear leukocytes, neutrophils, monocytes, reticulocytes, basophils, coelomocytes, hemocytes, eosinophils, megakaryocytes, macrophages, dendritic cells natural killer cells, or fraction of such cells (e.g., a nucleic acid or protein fraction). In specific embodiments, the biological sample comprises leukocytes including peripheral blood mononuclear cells (PBMC).

### 7.1 Nucleic acid-based diagnostics

Nucleic acid used in polynucleotide-based assays can be isolated from cells contained in the biological sample, according to standard methodologies (Sambrook, *et al.,* 1989, *supra;* and Ausubel *et al.,* 1994, *supra*). The nucleic acid is typically fractionated (e.g., poly A⁺ RNA) or whole cellRNA. Where RNA is used as the subject of detection, it may be desired to convert the RNA to a complementary DNA. In some embodiments, the nucleic acid is amplified by a template-dependent nucleic acid amplification technique. A number of template dependent processes are available to amplify the stress marker sequences present in a given template sample. An exemplary nucleic acid amplification technique is the polymerase chain reaction (referred to as PCR) which is described in detail in U.S. Pat. Nos. 4,683,195, 4,683,202 and 4,800,159, Ausubel *et al. (supra),* and in Innis et al., ("PCR-Protocols", Academic Press, Inc., San Diego Calif., 1990). Briefly, in PCR, two primer sequences are prepared that are complementary to regions on opposite complementary strands of the marker sequence. An excess of deoxynucleoside triphosphates are added to a reaction mixture along with a DNA polymerase, e.g., *Taq* polymerase. If a cognate stress marker sequence is present in a sample, the primers will bind to the marker and the polymerase will cause the primers to be extended along the marker sequence by adding on nucleotides. By raising and lowering the temperature of the reaction mixture, the extended primers will dissociate from the marker to form reaction products, excess primers will bind to the marker and to the reaction products and the process is repeated. A reverse transcriptase PCR amplification procedure may be performed in order to quantify the amount of mRNA amplified. Methods of reverse transcribing RNA into cDNA are well known and described in Sambrook *et al.,* 1989, *supra.* Alternative methods for reverse transcription utilize thermostable, RNA-dependent DNA polymerases. These methods are described in WO 90/07641. Polymerase chain reaction methodologies are well known in the art.

In certain advantageous embodiments, the template-dependent amplification involves the quantification of transcripts in real-time. For example, RNA or DNA may be quantified using the Real-Time PCR technique (Higuchi, 1992, et al., Biotechnology 10: 413-417). By determining the concentration of the amplified products of the target DNA in PCR reactions that have completed the same number of cycles and are in their linear ranges, it is possible to determine the relative concentrations of the specific target sequence in the original DNA mixture. If the DNA mixtures are cDNAs synthesized from RNAs isolated from different tissues or cells, the relative abundance of the specific mRNA from which the target sequence was derived can be determined for the respective tissues or cells. This direct proportionality between the concentration of the PCR products and the relative mRNA abundance is only true in the linear range of the PCR reaction. The final concentration of the target DNA in the plateau portion of the curve is determined by the availability of reagents in the reaction mix and is independent of the original concentration of target DNA.

Another method for amplification is the ligase chain reaction ("LCR"), disclosed in EPO No. 32.0 308. In LCR, two complementary probe pairs are prepared, and in the presence of the target sequence, each pair will bind to opposite complementary strands of the target such that they abut. In the presence of a ligase, the two probe pairs will link to form a single unit. By temperature cycling, as in PCR, bound ligated units dissociate from the target and then serve as "target sequences" for ligation of excess probe pairs. U.S. Pat. No. 4,883,750 describes a method similar to LCR for binding probe pairs to a target sequence.

Qβ Replicase, described in PCT Application No. PCT/US87/00880, may also be used. In this method, a replicative sequence of RNA that has a region complementary to that of a target is added to a sample in the presence of an RNA polymerase. The polymerase will copy the replicative sequence that can then be detected.

An isothermal amplification method, in which restriction endonucleases and ligases are used to achieve the amplification of target molecules-that contain nucleotide 5'α-thio-triphosphates in one strand of a restriction site may also be-useful in the amplification of nucleic acids in the present invention, Walker et al., (1992, Proc. Natl. Acad. Sci. U.S.A 89: 392-396).

Strand Displacement Amplification (SDA) is another method of carrying out isothermal amplification of nucleic acids which involves multiple rounds of strand displacement and synthesis, i.e., nick translation. A similar method, called Repair Chain Reaction (RCR), involves annealing several probes throughout a region-targeted for amplification, followed by a repair reaction in which only two of the four bases-are present. The other two bases can be added as biotinylated derivatives for easy detection. A similar approach is used in SDA. Target-specific sequences can also be detected using a cyclic probe reaction (CPR). In CPR, a probe having 3' and 5' sequences of non-specific DNA and a middle sequence of specific RNA is hybridized to DNA that is present in a sample. Upon hybridization, the reaction is treated with RNase H, and the products of the probe identified as distinctive products that are released after digestion. The original template is annealed to another cycling probe and the reaction is repeated.

Still another amplification method described in GB Application No. 2 202 328, and in PCT Application No. PCT/US89/01025, may be used. In the former application, "modified" primers are used in a PCR-like, template- and enzyme-dependent synthesis. The primers may be modified by labeling with a capture moiety (e.g., biotin) and/or a detector moiety (e.g., enzyme). In the latter application, an excess of labeled probes are added to a sample. In the presence of the target sequence, the probe binds and is cleaved catalytically. After cleavage, the target sequence is released intact to be bound by excess probe. Cleavage of the labeled probe signals the presence of the target sequence.

Other nucleic acid amplification procedures include transcription-based amplification systems (TAS), including nucleic acid sequence based amplification (NASBA) and 3SR (Kwoh et al., 1989, Proc. Natl. Acad. Sci. U.S.A., 86: 1173; Gingeras et al., PCT Application WO 88/10315). In NASBA, the nucleic acids can be prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer which has target specific sequences. Following polymerization, DNA/RNA hybrids are digested with RNase H while double stranded DNA molecules are heat denatured again. In either case the single stranded DNA is made fully double stranded by addition of second target specific primer, followed by polymerization. The double-stranded DNA molecules are then multiply transcribed by an RNA polymerase such as T7 or SP6. In an isothermal cyclic reaction, the RNAs are reverse transcribed into single stranded DNA, which is then converted to double stranded DNA, and then transcribed once again with an RNA polymerase such as T7 or SP6. The resulting products, whether truncated or complete, indicate target specific sequences.

Davey et al., EPO No. 329 822 disclose a nucleic acid amplification process involving cyclically synthesizing single-stranded RNA ("ssRNA"), ssDNA, and double-stranded DNA (dsDNA), which may be used in accordance with the present invention. The ssRNA is a template for a first primer oligonucleotide, which is elongated by reverse transcriptase (RNA-dependent DNA polymerase). The RNA is then removed from the resulting DNA:RNA duplex by the action of ribonuclease H (RNase H, an RNase specific for RNA in-duplex with either DNA or RNA). The resultant ssDNA is a template for a second primer, which also includes the sequences of an RNA polymerase promoter (exemplified by T7 RNA polymerase) 5' to its homology to the template. This primer is then extended by DNA polymerase (exemplified by the large "Klenow" fragment of E. coli DNA polymerase I), resulting in a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

Miller et al. in PCT Application WO 89/06700 disclose a nucleic acid sequence amplification scheme based on the hybridization of a promoter/primer sequence to a target single-stranded DNA ("ssDNA") followed by transcription of many RNA copies of the sequence. This scheme is not cyclic, *i.e*., new templates are not produced from the resultant RNA transcripts. Other amplification methods include "RACE" and "one-sided PCR" (Frohman, M. A., In: "PCR Protocols: A Guide to Methods and Applications", Academic Press, N.Y., 1990; Ohara et al., 1989, Proc. Natl Acad Sci. U.S.A., 86: 5673-567).

Methods based on ligation of two (or more) oligonucleotides in the presence of nucleic acid having the sequence of the resulting "di-oligonucleotide", thereby amplifying the di-oligonucleotide, may also be used for amplifying target nucleic acid sequences. Wu et al., (1989, Genomics 4: 560).

Depending on the format, the stress marker nucleic acid of interest is identified in the sample directly using a template-dependent amplification as described, for example, above, or with a second, known nucleic acid following amplification. Next, the identified product is detected. In certain applications, the detection may be performed by visual means (e.g., ethidium bromide staining of a gel). Alternatively, the detection may involve indirect identification of the product *via* chemiluminescence, radioactive scintigraphy of radiolabel or fluorescent label or even via a system using electrical or thermal impulse signals (Affymax Technology; Bellus, 1994, J Macromol. Sci. Pure, Appl. Chem., A31(1):1355-1376).

In some embodiments, amplification products or "amplicons" are visualized in order to confirm amplification of the stress marker sequences. One typical. visualization method involves staining of a gel with ethidium bromide and visualization under UV light. Alternatively, if the amplification products are integrally labeled with-radio- or fluorometrically-labeled nucleotides, the amplification products can then be exposed to x-ray film or visualize under the appropriate stimulating spectra, following separation. In some embodiments, visualization is achieved indirectly. Following separation of amplification products, a labeled nucleic acid probe is brought into contact with the amplified stress marker sequence. The probe is suitably conjugated to a chromophore but may be radiolabeled. Alternatively, the probe is conjugated to a binding partner, such as an antigen-binding molecule, or biotin, and the other member of the binding pair carries a detectable moiety or reporter molecule. The techniques involved are well known to those of skill in the art and can be found in many standard texts on molecular protocols (e.g., see Sambrook *et al.,* 1989, *supra* and Ausubel *et al.* 1994, *supra*). For example, chromophore or radiolabel probes or primers identify the target during or following amplification.

In certain embodiments, target nucleic acids are quantified using blotting techniques, which are well known to those of skill in the art. Southern blotting involves the use of DNA as a target, whereas Northern blotting involves the use ofRNA as a target. Each provide different types of information, although cDNA blotting is analogous, in many aspects, to blotting or RNA species. Briefly, a probe is used to target a DNA or RNA species that has been immobilized on a suitable matrix, often a filter of nitrocellulose. The different species should be spatially separated to facilitate analysis. This often is accomplished by gel electrophoresis of nucleic acid species followed by "blotting" on to the filter. Subsequently, the blotted target is incubated with a probe (usually labeled) under conditions that promote denaturation and rehybridisation. Because the probe is designed to base pair with the target, the probe will bind a portion of the target sequence under renaturing conditions. Unbound probe is then removed, and detection is accomplished as described above.

Following detection/quantification, one-may compare the results seen in a given subject with a control reaction or a statistically significant reference group of normal subjects or of subjects free of stress. In this way, it is possible to correlate the amount of a stress marker nucleic acid detected with the progression or severity of the disease.

Also contemplated are genotyping methods and allelic discrimination methods and technologies such as those described by Kristensen et al. (Biotechniques 30(2): 318-322), including the use of single nucleotide polymorphism analysis, high performance liquid-chromatography, TaqMan™, liquid chromatography, and mass spectrometry.

Also contemplated are biochip-based technologies such as those described by Hacia et al. (1996, Nature Genetics 14: 441-447) and Shoemaker et al. (1996, Nature Genetics 14: 450-456). Briefly, these techniques involve quantitative methods for analyzing large numbers of genes rapidly and accurately. By tagging genes with oligonucleotides or using fixed probe arrays, one can employ biochip technology to segregate target molecules as high density arrays and screen these molecules on the basis of hybridization. See also Pease et al. (1994, Proc. Natl. Acad. Sci. U.S.A. 91: 5022-5026); Fodor et al. (1991, Science 251: 767-773). Briefly, nucleic acid probes to stress marker polynucleotides are made and attached to biochips to be used in screening and diagnostic methods, as outlined herein. The nucleic acid probes attached to the biochip are designed to be substantially complementary to specific expressed stress marker nucleic acids, i.e., the target sequence (either the target sequence of the sample or to other probe sequences, for example in sandwich assays), such that hybridization of the target sequence and the probes of the present invention occurs. This complementarity need not be perfect; there may be any number of base pair mismatches which will interfere with hybridization between the target sequence and the nucleic acid probes of the present invention. However, if the number of mismatches is so great that no hybridization can occur under even the least stringent of hybridization conditions, the sequence is not a complementary target sequence. In certain embodiments, more than one probe per sequence is used, with either overlapping probes or probes to different sections of the target being used. That is, two, three, four or more probes, with three being desirable, are used to build in a redundancy for a particular target. The probes can be overlapping (i.e. have some sequence in common), or separate.

. As will be appreciated by those of ordinary skill in the art, nucleic acids can be attached to or immobilized on a solid support in a wide variety of ways. By "immobilized" and grammatical equivalents herein-is meant the association or binding between the nucleic acid probe and the solid support is sufficient to be stable under the conditions of binding, washing, analysis, and removal as outlined below. The binding can be covalent or non-covalent. By "non-covalent binding" and grammatical equivalents herein is meant one or more of either electrostatic, hydrophilic, and hydrophobic interactions. Included in non-covalent binding is the covalent attachment of a molecule, such as, streptavidin to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.

In general, the probes are attached to the biochip in a wide variety of ways, as will be appreciated by those in the art. As described-herein, the nucleic acids can-either be synthesized first, with subsequent attachment to the biochip, or can be directly synthesized on the biochip.

The biochip comprises a suitable solid or semi-solid substrate or solid support. By "substrate" or "solid support" is meant any material that can be modified to contain discrete individual sites appropriate for the attachment or association of the nucleic acid probes and is amenable to at least one detection method. As will be appreciated by practitioners in the art, the number of possible substrates are very large, and include, but are not limited to, glass and modified or functionalized glass, plastics (including acrylics, polystyrene and copolymers of styrene and other materials, polypropylene, polyethylene, polybutylene, polyurethanes, Teflon™, etc.), polysaccharides, nylon or nitrocellulose, resins, silica or silica-based materials including silicon and modified silicon, carbon, metals, inorganic glasses, plastics, etc. In general, the substrates allow optical detection and do not appreciably fluorescese.

Generally the substrate is planar, although as will be appreciated by those of skill in the art, other configurations of substrates may be used as well. For example, the probes may be placed on the inside surface of a tube, for flow-through sample analysis to minimize sample volume. Similarly, the substrate may be flexible, such as a flexible foam, including closed cell foams made of particular plastics.

In certain embodiments, oligonucleotides probes are synthesized on the substrate, as is known in the art. For example, photoactivation techniques utilizing photopolymerisation compounds and techniques can be-used. In an illustrative example, the nucleic acids are synthesized *in situ,* using well known photolithographic-techniques, such as those described in WO 95/25116; WO 95/35505; U.S. Pat. Nos. 5,700,637 and 5,445,934; and references cited within; these methods of attachment form the basis of the Affymetrix GeneChip™ technology.

In an illustrative biochip analysis, oligonucleotide probes on the biochip are exposed to or contacted with a nucleic acid sample suspected of containing one or more stress polynucleotides under conditions favoring specific hybridization. Sample extracts of DNA or RNA, either single or double-stranded, may be prepared from fluid suspensions of biological materials, or by grinding biological materials, or following a cell lysis step which includes, but is not limited to, lysis effected by treatment with SDS (or other detergents), osmotic shock, guanidinium isothiocyanate and lysozyme. Suitable DNA, which may be used in the method of the invention, includes cDNA. Such DNA may be prepared-by-any one of a number of commonly used protocols as for example described in Ausubel, *et al*., 1994, *supra,* and Sambrook *et al*., *et al*., 1989, *supra.*

Suitable RNA, which may be used in the method of the invention, includes messenger RNA, complementary RNA transcribed from DNA (cRNA) or genomic or subgenomic RNA. Such RNA may be prepared using standard protocols as for example described in the relevant sections of Ausubel, *et al.* 1994, *supra* and Sambrook, *et al.* 1989, *supra*).

cDNA may be fragmented, for example, by sonication or by treatment with restriction endonucleases. Suitably, cDNA is fragmented such that resultant DNA fragments are of a length greater than the length of the immobilized oligonucleotide probe(s) but small enough to allow rapid access thereto under suitable hybridization conditions. Alternatively, fragments of cDNA may be selected and amplified using a suitable nucleotide amplification technique, as described for example above, involving appropriate random or specific primers.

Usually the target stress marker polynucleotides are detectably labeled so that their hybridization to individual probes can be determined. The target polynucleotides are typically detectably labeled with a reporter molecule illustrative examples of which include chromogens, catalysts, enzymes, fluorochromes, chemiluminescent molecules, bioluminescent molecules, lanthanide ions (e.g., Eu³⁴), a radioisotope and a direct visual label. In the case of a direct visual label, use may be made of a colloidal metallic or non-metallic particle, a dye particle, an enzyme or a substrate, an organic polymer, a latex particle, a liposome, or other vesicle containing a signal producing substance and the like. Illustrative labels of this type include large colloids, for examples, metal colloids such as those from gold, selenium, silver, tin and titanium oxide. In some embodiments in which an enzyme is used as a direct visual label, biotinylated bases are incorporated into a target polynucleotide. Hybridization is detected by incubation with streptavidin-reporter molecules.

Suitable fluorochromes include, but are not limited to, fluorescein isothiocyanate (FITC), tetramethylrhodamine isothiocyanate (TRITC), R-Phycoerythrin (RPE), and Texas Red. Other exemplary-fluorochromes include those discussed by Dower et al. (International Publication WO 93/06121). Reference also may be made to the fluorochromes described in U.S. Patents 5,573,909 (Singer et an*,* 5,326,692 (Brinkley et al). Alternatively, reference may be made to the fluorochromes described in U.S. PatentNos. 5,227,487, 5,274,113, 5,405,975, 5,433,896, 5,442,045, 5,451,663, 5,453-,517, 5,459,276, 5,516,864, 5,648,270 and 5,723,218. Commercially available fluorescent labels-include, for example, fluorescein phosphoramidites such as Fluoreprime™ (Pharmacia), FTuoredite™ (Millipore) and FAM (Applied Biosystems International)

Radioactive-reporter molecules include, for example, ³²P, which can be detected by an X-ray or phosphoimager techniques.

The hybrid-forming step can be performed under suitable conditions for hybridizing oligonucleotide probes to test nucleic acid including DNA or RNA. In this regard,-reference may be made, for example, to NUCLEIC ACID HYBRIDIZATION, A PRACTICAL APPROACH (Homes and Higgins, eds.) (IRL press, Washington D.C., 1985). In general, whether hybridization takes place is influenced by the length of the oligonucleotide probe and the polynucleotide sequence under test, the pH, the temperature, the concentration of mono- and divalent cations, the proportion of G and C nucleotides in the hybrid-forming region, the viscosity of the medium and the possible presence of denaturants. Such variables also influence the time required for hybridization. The preferred conditions will therefore depend upon the particular application. Such empirical conditions, however, can be routinely determined without undue experimentation.

In certain advantageous embodiments, high discrimination hybridization conditions are used. For example, reference may be made to Wallace et al. (1979, Nucl. Acids Res. 6: 3543) who describe conditions that differentiate the hybridization of 11 to 17 base long oligonucleotide probes that match perfectly and are completely homologous to a target sequence as compared to similar oligonucleotide probes that contain a single internal base pair mismatch. Reference also may be made to Wood et al. (1985, Pro. Natl. Acid. Sci. USA 82: 1585) who describe conditions for hybridization of 11 to 20 base long oligonucleotides using 3M tetramethyl ammonium chloride wherein the melting point of the hybrid depends only on the length-of the oligonucleotide probe, regardless of its GC content. In addition, Drmanac *et al*. *(supra)* describe hybridization conditions that allow stringent hybridization of 6-10 nucleotide long oligomers, and similar conditions may be obained most readily by using nucleotide analogues such as 'locked nucleic acids (Christensen et al., 2001 Biochem J 354: 481-4).

Generally, a hybridization reaction can be performed in the presence of a hybridization buffer that optionally includes a hybridization optimizing agent, such as an isostabilising agent, a denaturing agent and/or a renaturation accelerant. Examples of isostabilising agents include, but are not restricted to, betaines and lower tetraalkyl ammonium salts. Denaturing agents are compositions that lower the melting temperature of double stranded nucleic acid molecules by interfering with hydrogen bonding between bases in a double stranded nucleic acid or the hydration of nucleic acid molecules. Denaturing agents include, but are not restricted to, formamide, formaldehyde, dimethylsulfoxide, tetraethyl acetate, urea, guanidium isothiocyanate, glycerol and chaotropic salts. Hybridization accelerants include heterogeneous nuclear ribonucleoprotein (hnRP) A1 and-cationic detergents such as cetyltrimethylammonium bromide (CTAB) and dodecyl trimethylammonium bromide (DTAB), polylysine, spermine, spermidine, single stranded binding protein (SSB), phage T4 gene 32 protein and a mixture of ammonium acetate and ethanol. Hybridization buffers may include target polynucleotides at a concentration between about 0.005 nM and about 50 nM, preferably between about 0.5 nM and 5 nM, more preferably between about 1 nM and 2 nM.

A hybridization-mixture containing the target stress marker polynucleotides is placed in contact with the array of probes and incubated at a temperature and for a time appropriate to permit hybridization between the target sequences in the target polynucleotides and any complementary probes. Contact can take place in any suitable container, for example, a dish or a cell designed to hold the solid support on which the probes are bound. Generally, incubation will be at temperatures normally used for hybridization of nucleic acids, for example, between about 20° C and about 75° C, example, about 25° C, about 30° C, about 35° C, about 40° C, about 45° C, about 50° C, about 55° C, about 60° C, or about 65° C. For probes longer than 14 nucleotides, 20° C to 50° C is desirable. For shorter probes, lower temperatures are preferred. A sample of target polynucleotides is incubated with the probes for a time sufficient to allow the desired level of hybridization between the target sequences in the target polynucleotides and any complementary probes. For example, the hybridization may be carried out at about 45° C +/-10° C in formamide for 1-2 days.

After the hybrid-forming step, the probes are washed to remove any unbound nucleic acid with a hybridization buffer, which can typically comprise a hybridization optimising agent in the same range of concentrations as for the hybridization step. This washing step leaves only bound target polynucleotides. The probes are then examined to identify which probes have hybridized to a target polynucleotide.

The hybridization reactions are then detected to determine which of the probes has hybridized to a corresponding target sequence. Depending on the nature of the reporter molecule associated with a target polynucleotide, a signal may be instrumentally detected by irradiating a fluorescent label with light and detecting fluorescence in a fluorimeter; by providing for an enzyme system to produce a dye which could be detected using a spectrophotometer; or detection of a dye particle or a colored colloidal metallic or non metallic particle using a reflectometer; in the case of using a radioactive label or chemiluminescent molecule employing a radiation counter or autoradiography. Accordingly, a detection means may be adapted to detect or scan light associated with the label which light may include fluorescent, luminescent, focussed beam or laser light. In such a case, a charge couple device (CCD) or a photocell can be used to scan for emission of light from a probe:target polynucleotide hybrid from each location in the micro-array and record the data directly in a digital computer.-In some cases; electronic detection of the signal may not be necessary. For example, with enzymatically generated colour spots associated with nucleic acid array format, visual examination of the array will allow interpretation of the pattern on the array. In the case of a nucleic acid array, the detection means is suitably interfaced with pattern recognition software to convert the pattern of signals from the array into a plain language genetic profile. In pertain embodiments, oligonucleotide probes specific for different stress marker gene products are in the form of a nucleic acid array and detection of a signal generated from a reporter molecule on the array is performed using a 'chip reader'. A detection system that-can be used by a 'chip reader' is described for example by Pirrung et al (U.S. Patent No. 5,143,854). The chip reader will typically also incorporate some signal processing to determine whether the signal at a particular array position or feature is a true positive or maybe a spurious signal. Exemplary chip readers are described for example by Fodor et al (U.S. Patent No., 5,925,525). Alternatively, when the array is made using a mixture of individually addressable kinds of labeled microbeads, the reaction may be detected using flow cytometry.

### 7.2 Protein-based diagnostics

Consistent with the present invention, the presence of an aberrant concentration of a stress marker protein is indicative of the presence, degree or stage of stress or risk of development of stress sequelae. Stress marker protein levels in biological samples can be assayed using any suitable method known in the art. For example, when a stress marker protein is an enzyme, the protein can be quantified based upon its catalytic activity or based upon the number of molecules of the protein contained in a sample. Antibody-based techniques may be employed, such as, for example, immunohistological and immunohistochemical methods for measuring the level of a protein of interest in a tissue sample. For example, specific recognition is provided by a primary antibody (polyclonal or monoclonal) and a secondary detection system is used to detect presence (or binding) of the primary antibody. Detectable labels can be conjugated to the secondary antibody, such as a fluorescent label, a radiolabel, or an enzyme (e.g., alkaline phosphatase, horseradish peroxidase) which produces a quantifiable, e.g., colored, product. In another suitable method, the primary antibody itself can be detectably labeled. As a result, immunohistological labeling of a tissue section is provided. In some embodiments, a protein extract is produced from a biological sample (e.g., tissue, cells) for analysis. Such an extract (e.g., a detergent extract) can be subjected to western-blot or dot/slot assay of the level of the protein of interest, using routine immunoblotting methods (Jalkanen et al., 1985, J. Cell. Biol. 101: 976-985; Jalkanen et al., 1987, J. Cell. Biol. 105: 3087-3096).

Other useful antibody-based methods include immunoassays, such as the enzyme-linked immunosorbent assay (ELISA) and the radioimmunoassay (RIA). For example, a protein-specific monoclonal antibody, can be used both as an immunoadsorbent and as an enzyme-labeled probe to detect and quantify a stress marker protein of interest. The amount of such protein present in a sample can be calculated by reference to the amount present in a standard preparation using a linear regression computer algorithm (see Lacobilli et al., 1988, Breast Cancer Research and Treatment 11: 19-30). In other embodiments, two different monoclonal antibodies to the protein of interest can be employed, one as the immunoadsorbent and the other as an enzyme-labeled probe.

Additionally-recent developments in the field of protein capture arrays permit the simultaneous detection and/or quantification of a large number of proteins. For example, low-density protein arrays on filter membranes, such as the universal protein array system (Ge, 2000 Nucleic Acids Res. 28(2):e3) allow imaging of arrayed antigens using standard ELISA techniques and a scanning charge-coupled device (CCD) detector. Immuno-sensor arrays have also been developed that enable the simultaneous detection of clinical analytes. It is now possible using protein arrays, to profile protein expression in bodily fluids, such as in sera of healthy or diseased subjects, as well as in subjects pre- and post-drug treatment.

Protein capture arrays typically comprise a plurality of protein-capture agents each of which defines a spatially distinct feature of the array. The protein-capture agent can be any molecule or complex of molecules which has the ability to bind a protein and immobilize it to the site of the protein-capture agent on the array. The protein-capture agent may be a protein whose natural function in a cell is to specifically bind another protein, such as an antibody or a receptor. Alternatively, the protein-capture agent may instead be a partially or wholly synthetic or recombinant protein which specifically binds a protein. Alternatively, the protein-capture agent may be a protein which has been selected *in vitro* from a mutagenized, randomized, or completely random and synthetic library by its binding affinity to a specific protein or peptide target. The selection method used may optionally have been a display method such as ribosome display or phage display, as known in the art. Alternatively, the protein-capture agent obtained *via in vitro* selection may be a DNA or RNA aptamer which specifically binds a protein target (see, e.g., Potyrailo et al., 1998 Anal. Chem. 70:3419-3425; Cohen et al., 1998, Proc. Natl. Acad. Sci. USA 95:14272-14277; Fukuda, et al., 1997 Nucleic Acids Symp. Ser. 3.7:237-238; available from SomaLogic). For example, aptamers are selected from libraries of oligonucleotides by the Selex™ process and their interaction with protein can be enhanced by covalent attachment, through incorporation of brominated deoxyuridine and UV-activated crosslinking (photoaptamers). Aptamers have the advantages of ease of production by automated oligonucleotide synthesis and the stability and robustness of DNA; universal fluorescent protein stains can be used to detect binding. Alternatively, the *in vitro* selected protein-capture agent may be a polypeptide (e.g., an antigen) (see, e.g., Roberts and Szostak, 1997 Proc. Natl. Acad. Sci. USA, 94:12297-12302).

An alternative to an array of capture molecules is one made through 'molecular imprinting' technology, in which peptides (e.g., from the C-terminal regions-of proteins) are used as templates to generate structurally complementary, sequence-specific cavities in a polymerizable matrix; the cavities can then specifically capture (denatured) proteins which have the appropriate primary amino acid sequence (e.g., available from ProteinPrint™ and Aspira Biosystems).

Exemplary protein capture arrays include arrays comprising spatially addressed antigen-binding molecules, commonly referred to as antibody arrays, which can facilitate extensive parallel analysis of numerous proteins defining a proteome or subproteome. Antibody arrays have been shown to have the required properties of specificity and acceptable background, and some are available commercially (e.g., BD Biosciences, Clontech, BioRad and Sigma). Various methods for the preparation of antibody arrays have been reported (see, e.g., Lopez et al., 2003 J. Chromatogr. B 787:19-27; Cahill, 2000 Trends in Biotechnology 7:47-51; U.S. Pat. App. Pub. 2002/0055186; U.S. Pat. App. Pub. 2003/0003599; PCT publication WO 03/062444; PCT publication WO 03/077851; PCT publication WO 02/59601; PCT publication WO 02/39120; PCT publication WO 01/79849; PCT publication WO 99/39210). The antigen-binding molecules of such arrays may recognize at least a subset of proteins expressed by a cell or population of cells, illustrative examples of which include growth factor receptors, hormone receptors, neurotransmitter receptors, catecholamine receptors, amino acid derivative receptors, cytokine receptors, extracellular matrix receptors, antibodies, lectins, cytokines, serpins, proteases, kinases, phosphatases, ras-like GTPases, hydrolases, steroid hormone receptors, transcription factors, heat-shock transcription factors, DNA-binding proteins, zinc-finger proteins, leucine-zipper proteins, homeodomain proteins, intracellular signal transduction modulators and effectors, apoptosis-related factors, DNA synthesis factors, DNA repair factors, DNA recombination factors, cell-surface antigens, hepatitis C virus (HCV) proteases and HIV proteases.

Antigen-binding molecules for antibody arrays are made either by conventional immunization (e.g., polyclonal sera and hybridomas), or as recombinant fragments, usually expressed in *E*. *coli,* after selection from phage display or ribosome display libraries (e.g., available from Cambridge-Antibody Technology, BioInvent, Affitech and Biosite). Alternatively, 'combibodies' comprising non-covalent associations of VH and VL domains, can be produced in a matrix format created from combinations of diabody-producing bacterial clones (e.g., available from Domantis). Exemplary antigen-binding molecules for use -as protein-capture agents include monoclonal antibodies, polyclonal antibodies,. Fv, Fab, Fab' and F(ab')₂ immunoglobulin fragments, synthetic stabilized Fv fragments, e.g., single chain Fv fragments (scFv), disulfide stabilized Fv fragments (dsFv), single variable region domains (dAbs) minibodies, combibodies and multivalent-antibodies such as diabodies and multi-scFv, single domains from camelids or engineered human equivalents.

Individual spatially distinct protein-capture agents are typically attached to a support surface, which is generally planar or contoured. Common physical supports include glass slides, silicon, microwells, nitrocellulose or PVDF membranes, and magnetic and other microbeads.

While microdrops of protein delivered onto planar surfaces are widely used, related alternative architectures include CD centrifugation devices based on developments in microfluidics (e.g., available from Gyros) and specialized chip designs, such as engineered microchannels in a plate (e.g., The Living Chip™, available from Biotrove) and tiny 3D posts on a silicon surface (e.g., available from Zyomyx).

Particles in suspension can also be used as the basis of arrays, providing they are coded for identification; systems include color-coding for microbeads (e.g., available from Luminex, Bio-Rad and Nanomics Biosystems) and semiconductor nanocrystals (e.g., Qdots™, available from Quantum Dots), and barcoding for beads (UltraPlex™, available from Smartbeads)-and multimetal microrods (Nanobarcodes™ particles, available from Surromed). Beads can also be assembled into planar arrays on semiconductor chips (e.g., available from LEAPS technology and BioArray Solutions). Where particles are used, individual protein-capture agents are typically attached to an individual particle to provide the spatial definition or separation of the array. The particles may then be assayed-separately, but in parallel, in a compartmentalized way, for example in the wells of a microtiter plate or in separate test tubes.

In operation, a protein sample; which is optionally fragmented to form peptide fragments (see, e.g., U.S. Pat. App. Pub. 2002/0055186), is delivered to a protein-capture array under conditions suitable for protein or peptide binding, and the array is washed to remove unbound or non-specifically bound components of the sample from the array. Next, the presence or amount of protein or peptide bound to each feature of the array is detected using a suitable detection system. The amount of protein bound to a feature of the array may be determined relative to the amount of a second protein bound to a second feature of the array. In certain embodiments, the amount of the second protein in the-sample is already known or known to be invariant.

For analyzing differential expression of proteins between two cells or cell. populations, a protein sample of a first cell or population of cells is delivered to the array under conditions suitable for protein binding. In an analogous manner, a protein sample of a second cell or population of cells to a second array, is delivered to a second array which is identical to the first array. Both-arrays are then washed to remove unbound or non-specifically bound components of the sample from the arrays. In a final step, the amounts of protein remaining bound to the features of the first array are compared to the amounts of protein remaining bound to the corresponding features of the second array. To determine the differential protein expression pattern of the two cells or populations of cells, the amount of protein bound to individual features of the first array is subtracted from the amount of protein-bound to the corresponding features of the second array.

In an illustrative example, fluorescence labeling can be used for detecting protein bound to the array. The same instrumentation as used for reading DNA microarrays is applicable to protein-capture arrays. For differential display, capture arrays (e.g. antibody arrays) can be probed with fluorescently labeled proteins from two different cell states, in which cell lysates are labeled with different fluorophores (e.g., Cy-3 and Cy-5) and mixed, such that the color acts as a readout for changes in target abundance. Fluorescent readout sensitivity can be amplified 10-100 fold by tyramide signal amplification (TSA) (e.g., available from PerkinElmer Lifesciences). Planar waveguide technology (e.g., available from Zeptosens) enables ultrasensitive fluorescence detection, with the additional advantage of no washing procedures. High sensitivity can also be achieved with suspension beads and particles, using phycoerythrin as label (e.g., available from Luminex) or the properties of semiconductor nanocrystals (e.g., available from Quantum Dot). Fluorescence resonance energy transfer has been adapted to detect binding of unlabelled ligands, which may be useful on arrays (e.g., available from Affibody). Several alternative readouts have been developed, including adaptations of surface plasmon resonance (e.g., available from HTS Biosystems and Intrinsic Bioprobes), rolling circle DNA amplification (e.g., available from Molecular Staging), mass spectrometry (e.g., available from Sense Proteomic, Ciphergen, Intrinsic and Bioprobes), resonance light scattering (e.g., available from Genicon Sciences) and atomic force microscopy (e.g., available from BioForce Laboratories). A microfluidics system for automated sample incubation with arrays on glass slides and washing has been co-developed by NextGen and Perkin Elmer Life Sciences.

In certain embodiments, the techniques used for detection of stress marker expression products will include internal or external standards to permit quantitative or semiquantitative determination of those products, to thereby enable a valid comparison of the level or functional activity of these expression products in a biological sample with the corresponding expression products in a reference sample or samples. Such standards can be determined by the skilled practitioner using standard protocols. In specific examples, absolute values for the level or functional activity of individual expression products are determined.

In specific embodiments, the diagnostic method is implemented using a system as disclosed, for example, in International Publication No. WO 02/090579 and in copending PCT Application No. PCT/AU03/01517 filed November 14, 2003, comprising at least one end station coupled to a base station. The base station is typically coupled to one or more databases comprising predetermined data from a number of individuals representing the level or functional activity of stress marker expression products, together with indications of the actual status of the individuals (e.g., presence, absence, degree, stage of stress or risk of development of stress sequelae) when the predetermined data was collected. In operation, the base station is adapted to receive from the end station, typically via a communications network, subject data representing a measured or normalized level or functional activity of at least one expression product in a biological sample obtained from a test subject and to compare the subject data to the predetermined data stored in the database(s). Comparing the subject and predetermined data allows the base station to determine the status of the subject in accordance with the results of the comparison. Thus, the base station attempts to identify individuals having similar parameter values to the test subject and once the status has been determined on the basis of that identification, the base station provides an indication of the diagnosis to the end station.

### 7.3 Kits

All the essential materials and reagents required for detecting and quantifying stress marker gene expression products may be assembled together in a kit. The kits may also optionally include appropriate reagents for detection of labels, positive and negative controls, washing solutions, blotting membranes, microtiter plates dilution buffers and the like. For example, a nucleic acid-based detection kit may include (i) a stress marker polynucleotide (which may be used as a positive control), (ii) a primer or probe that specifically hybridizes to a stress marker polynucleotide. Also included may be enzymes-suitable for amplifying nucleic acids including various polymerases (Reverse Transcriptase, Taq, Sequenase™ DNA ligase etc. depending on the nucleic acid amplification technique employed), deoxynucleotides and buffers to provide the necessary reaction mixture for amplification. Such kits also generally will comprise, in suitable means, distinct containers for each individual reagent and enzyme as well as for each primer or probe. Alternatively, a protein-based detection kit may include (i) a stress marker polypeptide (which may be used as a positive control), (ii) an antigen-binding molecule that is immuno-interactive with a stress marker polynucleotide. The kit can also feature various devices and reagents for performing one of the assays described herein; and/or printed instructions for using the kit to quantify the expression of a stress marker gene.

### 7.4 Monitoring immune function

The present invention also provides methods for monitoring immune function by measuring the level or functional activity of an expression product of one or more stress marker genes in a subject When the-measured level or functional activity is the same as or similar to the measured-level or functional activity of a corresponding expression product in a reference sample obtained from one or more normal subjects or from one or more subjects not under stress, this generally indicates that the subject is not under stress and has normal immune function. Conversely, when the measured level or functional activity is different than the measured level or functional activity of the corresponding expression product, this generally indicates that the subject is under stress and consequently has reduced immune function (or immunosuppression).

The normalcy of immune function is important to the effective combat of disease and ultimate protection to natural challenge. In addition, it is vital to obtain an effective immune response to vaccination, and, in this regard, the identified stress markers can also be used to monitor the immune system of individuals so that vaccination can be timed to produce an immune response that leads to the best level of protection. For instance, in the context of athletic performance animals such as human athletes and racehorses, monitoring the immune system in this fashion allows the performance animal or his/her/its trainer to reduce potential stressors that may lead to an inappropriate or non-protective immune response to vaccination. When the performance animal's immune system has recovered, as determined by monitoring using the identified stress markers, vaccination can be performed.

Also, the identified stress markers can be used to assess the immune system's response to vaccine preparations. An inappropriate immune response to an initial vaccination may lead to a decision to revaccinate, or to modify the vaccination regimen, or to delay a vaccination regimen until potential stressors (that affect immune function) are removed and the animal's immune system has recovered.

By way of example, there are known vaccine preparations available for Equine Herpes Virus. It is widely used in the veterinary field, especially in pregnant mares so that foals will be afforded some protection through transfer of milk antibodies (colostrum). Pregnancy and the puerperal periods are times of high stress and immune modulation. Immune function can be monitored during these periods using the identified stress markers, to time vaccination so that appropriate and protective vaccine responses are generated. Alternatively, stress marker levels could be used to modify the vaccination regimen depending upon the monitored immune response to vaccination.

### 8. Methods of treatment or prophylaxis

The present invention also-extends to the treatment or prevention of stress in subjects following positive diagnosis for the risk of development of stress sequelae in the subjects. Generally, the treatment will include administering to a positively diagnosed subject an effective amount of an agent or therapy that ameliorates the symptoms or reverses the development of stress or that reduces or abrogates a stress-related condition as described for example above, or that reduces potential of the subject to developing a stress-related condition. Current agents suitable for treating stress include, but are not limited to corticotropin releasing factor antagonists as described, for example, in U.S. Patent Nos. 6,723,721, 6,670,371, 6,664,261, 6,586,456, 6,548,509, 6,323,312, 6,255,310; glucocorticoid receptor antagonists as disclosed in U.S. Patent Application Publication No. 20020169132; adenosine compounds as described, for example, in U.S. Patent No. 6,642,209; nitric oxide donors as described, for example in U.S. Patent No. 6,455,542; nutritional compositions as described for example in U.S. Patent Nos. 6,444,700, 6,391,332 and 6,218,420; herbal extracts as disclosed, for example, in U.S. Patent No. 6,416,795; NK-1 receptor antagonists as disclosed, for example, in U.S. Patent No. 6,087,348; fatty acid-based compositions as described, for example, in U.S. Patent No. 6,077,867; peptide derivatives from yeast as disclosed, for example, in U.S. Patent Application Publication No. 20040101934; and zinc ionophores as described, for example, in U.S. Patent Application No. 20020183300;

Alternatively, the subject may be treated using stress-relieving processes known in the art including for example: removing or decreasing the level of stressor in the subject's environment; and altering ion flux across cell membranes with electric fields as described in U.S. Patent Application Publication No. 20030233124.

However, it will be understood that the present invention encompasses any agent or process that is useful for treating or preventing stress and is not limited to the aforementioned illustrative compounds and formulations.

Typically, stress-relieving agents will be administered in pharmaceutical (or veterinary) compositions together -with a pharmaceutically acceptable carrier and in an effective amount to achieve their intended purpose. The dose of active compounds administered to a subject should be sufficient to achieve a beneficial response in the subject over time such as a reduction in, or relief from, the symptoms of stress. The quantity of the pharmaceutically active compounds(s) to be administered may depend on the subject to be treated inclusive of the age, sex, weight and general health condition thereof. In this regard, precise amounts of the active compound(s) for administration will depend on the judgement of the practitioner. In determining the effective amount of the active compound(s) to be administered in the treatment or prevention of stress, the physician or veterinarian may evaluate severity of any symptom associated with the presence of stress including symptoms related to stress sequelae as mentioned above. In any event, those of skill in the art may readily determine suitable dosages of the stress relieving agents and suitable treatment regimens without undue experimentation.

The stress relieving agents may be administered in concert with adjunctive therapies to reduce an aberrant immune response in the subject. Illustrative examples of such adjunctive therapies include but are not limited to, removal of the stressor, yoga, meditation, acupuncture, massage, mild exercise and breathing exercises.

It order that the invention may be readily understood and put into practical effect, particular preferred embodiments will nowbe described by way of the following nonlimiting examples.

### EXAMPLE 1

### IDENTIFICATION OF SPECIFIC DIAGNOSTIC GENES FOR STRESS

Blood samples obtained from 20 animals exposed to transport stress over 48 hours were analyzed using GeneChips™ (method of use is described below in detail in "Generation of Gene Expression Data") containing thousands of genes expressed in white blood cells of horses. Analysis of these data (see identification of Responding Genes and Demonstration of Diagnostic Potential" below) reveals specific genes that are expressed differentially at day 0 through to day 28. It is possible to design an assay that measures the RNA level in the sample using at least one and desirably at least two stress marker genes representative sequences of which are set forth in SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 1-Oi, 103, 105, 107, 108, 109, 111, 113, 115, 117, 118, 119, 121,123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248.

### Materials and methods

### Blood Collection

Blood is collected from a horse (in a non-agitated state) for the purpose of extraction of high quality RNA or protein. Suitable blood collection tubes for the collection, preservation, transport and isolation of RNA include PAXgene™ tubes (PreAnalytix Inc., Valencia, CA, USA). Alternatively, blood can be collected into tubes, containing solutions designed for the preservation of nucleic acids (available from Roche, Ambion, Invitrogen and ABI). For the determination of protein levels, 50 mL of blood is prevented from clotting by collection into a tube containing 4 mL of 4% sodium citrate. White blood cells and plasma are isolated and stored frozen for later analysis and detection of specific proteins. PAXgene tubes can be kept at room temperature prior to RNA extraction. Clinical signs are recorded in a standard format.

### Total RNA Extraction

A kit available from Qiagen Inc (Valencia, CA, USA) has the reagents and instructions for the isolation of total RNA from 2.5 mL blood collected in the PAXgene Blood RNA Tube. Isolation begins with a centrifugation step to pellet nucleic acids in the PAXgene blood RNA tube. The pellet is washed and resuspended and incubated in optimized buffers together with Proteinase K to bring about protein digestion. An additional centrifugation is carried out to remove residual cell debris and the supernatant is transferred to a fresh microcentrifuge tube. Ethanol is added to adjust binding conditions, and the lysate is applied to the PAXgene RNA spin column. During brief centrifugation, RNA is selectively bound to the silica-gel membrane as contaminants pass through. Remaining contaminants are removed in three efficient wash steps and RNA is then eluted in Buffer BR5.

Determination of RNA quantity and quality is necessary poor to proceeding and can be achieved using an Agilent Bioanalyzer and Absorbance 260/280 ratio using a spectrophotometer.

### DNA Extraction

A kit available from Qiagen Inc (Valencia, CA, USA) has the reagents and instructions for the isolation of total DNA from 8.5 mL blood collected in the PAXgene Blood DNA Tube. Isolation begins with the addition of additional lysis solution followed by a centrifugation step. The pellet is washed and resuspended and incubated in optimized buffers together with Proteinase K to bring about protein digestion. DNA is precipitated using alcohol and an additional centrifugation is carried out to pellet the nucleic acid. Remaining contaminants are removed in a wash step and the DNA is then resuspended in Buffer BG4.

Determination of DNA quantity and quality is necessary prior to proceeding and can be achieved using a spectrophotometer or agarose gel electrophoresis.

### Generation of Gene Express-ion Data

### Choice of Method

Measurement of specific RNA levels in a tissue sample can be-achieved using a variety of technologies. Two common and readily available technologies that are well known in the art are:

GeneChip® analysis using Affymetrix technology.

Real-Time Polymerase Chain Reaction (TaqMan™ from Applied Biosystems for example).

GeneChips® quantitate RNA by detection of labeled cRNA hybridized to short oligonucleotides built on a silicon substrate. Details on the technology and methodology can be found at www.affymetrix.com.

Real-Time Polymerase Chain Reaction (RT-PCR) quantitates RNA using two PCR primers, a labeled probe and a thermostable DNA polymerase. As PCR product is generated a dye is released into solution and detected. Internal controls such as 18S RNA probes are often used to determine starting levels of total RNA in the sample. Each gene and the internal control are run separately. Details on the technology and methods can be found at www.appliedbiosytems.com or www.qiagen.com or www.biorad..com. Applied Biosystems offer a service whereby the customer provides DNA sequence information and payment and is supplied in return all of the reagents required to perform RT-PCR analysis on individual genes.

GeneChip® analysis has the advantage of being able to analyze thousands of genes at a time. However it is expensive and takes over 3 days to perform a single assay. RT-PCR generally only analyses one gene at a time, but is inexpensive .and can be completed within a single day.

RT-PCR is the method of choice for gene expression analysis if the number of specific genes to be analyzed is less than 20. GeneChip® or other gene expression analysis technologies (such as Illumina Bead Arrays) are the method of choice when many genes need to be analysed simultaneously.

The methodology for GeneChip® data generation and analysis and Real Time PCR is presented below in brief.

### GeneChip® Data Generation

### cDNA & cRNA Generations

The following method for cDNA and cRNA generation from total RNA has been adapted from the protocol provided and recommended by Affymetrix (www.affymetrix.com).

The steps are:

• A total of 3 µg of total RNA is used as a template to generate double. stranded cDNA.

• cRNA is generated and labeled using biotinylated Uracil (dUTP).

• biotin-labeled-cRNA is cleaned and the quantity determined using a spectrophotometer and MOPS gel analysis.

• labeled cRNA is fragmented to ~ 300bp in size.

• RNA quantity is determined on an Agilent "Lab-on-a-Chip" system (Agilent Technologies).

### Hybridization, Washing & Staining:

The steps are:

• A hybridization cocktail is prepared containing 0.05 µg/µL of labeled and fragmented cRNA, spike-in positive hybridization controls, and the Affymetrix oligonucleotides B2, bioB, bioC, bioD and cre.

• The final volume (80 µL) of the hybridization cocktail is added to the GeneChip™ cartridge.

• The cartridge is-placed in a hybridization oven at constant rotation for 16 hours.

• The fluid is removed from the GeneChip™ and stored.

• The GeneChip™ is placed in the fluidics station.

• The experimental conditions for each GeneChip™ are recorded as an .EXP file.

• All washing and staining procedures are carried out by the Affymetrix fluidics station with an attendant providing the appropriate solutions.

• The GeneChip™ is washed, stained with steptavidin-phycoerythin dye and then washed again using low salt solutions.

• After the wash protocols are completed, the dye on the probe array is 'excited' by laser and the image-captured by a CCD camera using an Affymetrix Scanner (manufactured-by Agilent).

### Scanning & Data File Generation:

The scanner and MAS 5 software generates-an image file from a single GeneChip™ called a .DAT file (see figure overleaf).

The .DAT file is then pre-processed prior to any statistical analysis.

Data pre-processing steps (prior to any statistical analysis) include:

• DAT File Quality Control (QC).

• CEL File Generation.

• Scaling and Normalization.

### DAT File Quality Control

The DAT file is an image. The image is inspected manually for artifacts (e.g. high/low intensity spots, scratches, high regional or overall background). (The B2 oligonucleotide hybridization performance is easily identified by an alternating pattern of intensities creating a border and array name.) The MAS 5 software used the B2 oligonucleotide border to align a grid over the image so that each square of oligonucleotides was centered and identified.

The other spiked hybridization-controls (bioB, bioC, bioD and cre) are used to evaluate-sample hybridization efficiency by reading "present" gene detection calls with increasing signal values, reflecting their relative concentrations. (If the DAT file is of suitable quality it is converted to an intensity data file (.CEL file) by Affymetrix MAS 5 software).

### CEL File Generation

The .CEL files generated by the MAS 5 software from .DAT files contain calculated raw intensities for the probe sets. Gene expression data is obtained by subtracting a calculated background from each cell value. To eliminate negative intensity values, a noise correction fraction based from a local noise value from the standard deviation of the lowest 2% of the background is applied.

All .CEL files generated from the GeneChip™ are subjected to specific quality metrics parameters.

Some metrics are routinely recommended by Affymetrix and can be determined from Affymetrix internal controls provided as part of the GeneChip™. Other metrics are based on experience and the processing of many GeneChip™.

### Analysis of GeneChip® Data

Three illustrative approaches to normalizing data might be used:

• Affymetrix MAS 5 Algorithm.

• Robust Multi-chip Analysis (RMA) algorithm of Irizarry (Irizarray *et al.,* 2002, *Biostatistics* (in print)).

• Robust Multi-chip Analysis Saved model (RMAS).

Those of skill in the art will recognize that many other approaches might be adopted, without materially affecting the invention.

### Affymetrix MAS 5 Algorithm

.CEL files are used by Affymetrix MAS 5 software to normalize or scale the data. Scaled data from one chip are compared to similarly scaled data from other chips.

Affymetrix MAS 5 normalization is achieved by applying the default "Global Scaling" option of the MAS 5 algorithm to the .CEL files. This procedure-subtracts a robust estimate of the center of the distribution of probe values, and divides by a robust estimate of the probe variability. This produces a set of chips with common location and scale at the probe level.

Gene expression indices are generated by a robust averaging procedure on all the probe pairs for a given gene. The results are constrained to be non-negative.

. Given that scaling takes place at the level of the probe, rather than at the level of the gene, it is possible that even after normalization there may be chip-to-chip differences in overall gene expression level. Following standard MAS5 normalization, values for each gene were de-trended with respect to median chip intensity. That is, values for each gene were regressed on the median chip intensity, and residuals were calculated. These residuals were taken as the de-trended estimates of expression for each gene

Median chip intensity was calculated using the Affymetrix MAS5 algorithm, but with a scale factor fixed at one.

### RMAS Analysis

This method is identical to the RMA method, with the exception that probe weights and target quantiles are established using a long term library of chip cel files, and are not re-calculated for these specific chips. Again, normalization occurs at the probe level.

### Real-Time PCR Data Generation

: Background information for conducting Real-time PCR may be obtained, for example, at http://dorakmt.tripod.com/genetics/realtime.html and in a review by Bustin SA (2000, J Mol Endocrinol 25:169-193).

### TaqMan™ Primer and Probe Design Guidelines:

1. The Primer Express™ (ABI) software designs primers with a melting temperature (Tm) of 58-60° C, and probes with a Tm value of 10° C higher. The Tm of both primers should be equal;

2. Primers should be 15-30 bases in length;

3. The G+C content should ideally be 30-80%. If a higher G+C content is unavoidable, the use of high annealing and melting temperatures, cosolvents such as glycerol, DMSO, or 7-deaza-dGTP may be necessary;

4. The run of an identical nucleotide should be avoided. This is especially true for G, where runs of four or more Gs is not allowed;

5. The total number of Gs and Cs in the last five nucleotides at the 3' end of the primer should not exceed two (the newer version of the software has an option to do this automatically). This helps to introduce relative instability to the 3' end of primers to reduce non-specific priming. The primer conditions are the same for SYBR Green assays;

6. Maximum amplicon size should not exceed 400 bp (ideally 50-150 bases). Smaller amplicons give more consistent results because PCR is more efficient and more tolerant of reaction conditions (the short length requirement has nothing to do with the efficiency of 5' nuclease activity);

7. The probes should not have runs of identical nucleotides (especially four or more consecutive Gs), G+C content should be 30-80%, there should be more Cs than Gs, and not a G at the 5' end. The higher number of Cs produces a higher ΔRn. The choice of probe should be made-first;

8. To avoid false-positive results due to amplification of contaminating genomic DNA in the cDNA preparation, it is preferable to have primers spanning exon-exon junctions. This way, genomic DNA will not be amplified (the PDAR kit for human GAPDH amplification has such primers);

9: If TaqMan™ probe is designed for allelic-discrimination, the mismatching nucleotide (the polymorphic site) should be in the middle of the probe rather than at the ends;

10. Use primers that contain dA nucleotides near the 3' ends so that any primer-dimer generated is efficiently degraded by AmpErase™ UNG (mentioned in p.9 of the manual for EZ RT-PCR kit; P/N 402877). If primers cannot be selected with dA nucleotides, near the ends, the use of primers with 3' terminal dU-nucleotides should be considered.

(See also the general principles of PCR Primer Design by Invitrogen.)

### General Method:

1. Reverse transcription of total RNA to cDNA should be done with random hexamers (not with oligo-dT). If oligo-dT has to be used long mRNA transcripts or amplicons greater than two kilobases upstream should be avoided, and 18S RNA cannot be used as normaliser:

2. Multiplex PCR will only work properly if the control primers are limiting (ABI control reagents do not have their primers limited);

3. The range of target cDNA used is 10 ng to 1 µg. If DNA is used (mainly for allelic discrimination studies), the optimum amount is 100 ng to 1 µg;

4. It is ideal to treat each RNA preparation with RNAse free DNAse to avoid genomic DNA contamination. Even the best RNA extraction methods yield some genomic DNA. Of course, it is ideal to have primers not amplifying genomic DNA at all but sometimes this may not be possible;

5. For optimal results, the reagents (before the preparation of the PCR mix) and the PCR mixture itself (before loading) should be vortexed and mixed well. Otherwise there may be shifting Rn value during the early (0 - 5) cycles of PCR. It is also important to add probe to the buffer component and allow it to equilibrate at room temperature prior to reagent mix formulation.

### TaqMan™ Primers and Probes:

The TaqMan™ probes ordered from ABI at midi-scale arrive already resuspended at 100 µM. If a 1/20 dilution is made, this gives a 5 µM solution. This stock solution should be aliquoted, frozen and kept in the dark. Using 1 µL of this in a 50 µL reaction gives the recommended 100 nM final concentration.

The primers arrive lyophilized with the amount given on the tube in pmols (such as 150.000 pmol which is equal to 150 nmol). If X nmol of primer is resuspended in X µL of H₂O, the resulting solution is 1 mM. It is best to freeze this stock solution in aliquots. When the 1 mM stock solution is diluted 1/100, the resulting working solution will be 10 µM. To get the recommended 50- 900 nM final primer concentration in 50 µL reaction volume, 0.25- 4.50 µL should be used per reaction (2.5 µL for 500 nM final concentration).

The PDAR primers and probes are supplied as a mix in one tube. They have to be used 2.5 µL in a 50 µL reaction volume.

### Setting up One-step TaqMan™ Reaction:

One-step real-time PCR uses RNA (as opposed to cDNA) as a template. This is the preferred method if the RNA solution has a low concentration but only if singleplex reactions are run. The disadvantage is that RNA carryover prevention enzyme AmpErase cannot be used in one-step reaction format. In this method, both reverse transcriptase and real-time PCR take place in the same tube. The downstream PCR primer also acts as the primer for reverse transcriptase (random hexamers or oligo-dT cannot be used for reverse transcription in one-step RT-PCR). One-step reaction requires higher dNTP concentration (greater than or equal to 300 mM vs 200 mM) as it combines two reactions needing dNTPs in one. A typical reaction mix for one-step PCR by Gold RT-PCR kit is as follows:

| **Reagents** | **Volume** |
|---|---|
| H₂O + RNA: | 20.5 µL [24 µL if PDAR is used] |
| 10X TaqMan buffer : | 5.0 µL |
| MgCl₂ (25 mM): | 11.0 µL |
| dATP (10mM): | 1.5 µL [for final concentration of 300 µM] |
| dCTP (10mM): | 1.5 µL [for fmal concentration of 300 µM] |
| dGTP (10mM) : | 1.5 µL [for final concentration of 300 µM] |
| dUTP (20mM) : | 1:5 µL [for final concentration of 600 µM] |
| Primer F (10 µM) * : | 2.5 µL [for final concentration of 500 nM] |
| Primer R (10 µM) * : | 2.5 µL [for final concentration of 500 nM] |
| TaqMan Probe * : | 1.0 µL [for-final concentration of 100 nM] |
| AmpliTaq Gold : | 0.25 µL [can be increased for higher efficiency] |
| Reverse Transcriptase : | 0.25 µL |
| RNAse inhibitor: | 1.00 µL |

If a PDAR is used, 2.5 µL of primer + probe mix used.

Ideally 10 µg - 100 ng RNA should be used in this reaction. Note that decreasing the amount of template from 100 ng to 50 ng will increase the C_{T} value by 1. To decrease a C_{T} value by 3, the initial amount of template should be increased 8-fold. ABI claims that 2 picograms of RNA can be detected by this system and the maximum amount of RNA that can be used is 1 microgram. For routine analysis, 10 pg - 100 ng RNA and 100 pg - 1 µg genomic DNA can be used.

### Cycling Parameters for One-step-PCR:

-Reverse transcription (by MuLV) 48° C for 30 min.

AmpliTaq activation 95°C for 10 min.

PCR: denaturation 95° C for 15 sec and annealing/extension 60° C for 1 min (repeated 40 times) (On ABI 7700, minimum holding time is 15 seconds.)

The recently introduced EZ one-step™ RT-PCR kit allows the use of UNG as the incubation time for reverse transcription is 60° C thanks to the use of a thermostable reverse transcriptase. This temperature also a better option to avoid primer dimers and non-specific bindings at 48° C.

### Operating the ABI 7700:

Make sure the following before starting a run:

1. Cycle parameters are correct for the run;

2. Choice of spectral compensation is correct (*off* for singleplex, on for multiplex reactions);

3. Choice of "Number of PCR Stages" is correct in the Analysis Options box (Analysis/Options). This=may have to be manually assigned after a run if the data is absent in the amplification plot but visible in the plate view, and the X-axis of the amplification is displaying a range of 0-1 cycles;

4. No Template Control is labeled as such (for accurate ΔRn calculations);

5. The choice of dye component should be made correctly before data analysis;

6. You must save the run before it starts by giving it a name (not leaving as untitled);

7. Also at the end of the run, first-save the data before starting to analyze.

The ABI software requires extreme caution. Do not attempt to stop a run after clicking on the Run button. You will have problems and if you need to switch off and on the machine, you have to wait for at least an-hour to restart the run.

When analyzing the data, remember that the default setting for baseline is 3 15. If any C_{T} value is <15, the baseline should be changed accordingly (the baseline stop value should be 1-2 smaller than the smallest C_{T} value). For a useful discussion of this matter, see the ABTTutorial on Setting Baselines and Thresholds. (Interestingly, this issue is best discussed in the manual for TaqMan™ Human Endogenous Control Plate.)

If the results do not make sense, check the raw spectra for a possible CDC camera saturation during the run. Saturation of CDC camera may be prevented by using optical caps rather than optical adhesive cover. It is also more likely to happen when SYBR Green I is used, when multiplexing and when a high concentration of probe is used.

### Interpretation of Results:

At the end of each reaction, the recorded fluorescence intensity is used for the following calculations:

Rn⁺ is the Rn value of a reaction containing all components, Rn is the Rn value of an unreacted sample (baseline value or the value detected in NTC). ΔRn is the difference between Rn⁺ and Rn⁻. It is an indicator of the magnitude of the signal generated by the PCR.

There are three illustrative methods to quantitate the amount of template:

1. Absolute standard method: In this method, a known amount of standard such as in vitro translated RNA (cRNA) is used;

2. Relative standard: Known amounts of the target nucleic acid are included in the assay design in each run;

3. Comparative C_{T} method: This method uses no known amount of standard but compares the relative amount of the target sequence to any of the reference values chosen and the result is given as relative to the reference value (such as the expression level of resting lymphocytes or a standard cell line).

### The Comparative CT Method (ΔΔCT) for Relative Quantitation of Gene Expression:

This method enables relative quantitation of template and increases sample throughput by eliminating the need for standard curves when looking at expression levels relative to an active reference control (normaliser). For this method to be successful; the dynamic range of both the target and reference should be similar. A sensitive method to control this is to look at how ΔC_{T} (the difference between the two CT values of two PCRs for the same initial template amount) varies with template dilution. If the efficiencies of the to amplicons are approximately equal, the plot of log input amount versus ΔC_{T} will have a nearly horizontal line (a slope of <0.10). This means that both ECRs perform equally efficiently across the range of initial template amounts. If the plot shows unequal efficiency, the standard curve method should-be used for quantitation of gene expression. The dynamic range should be determined for both (1) minimum and maximum concentrations of the targets for which the results are accurate and (2) minimum and maximum ratios of two gene quantities for which the results are accurate. In conventional competitive RT-PCR, the dynamic range is limited to a target-to-competitor ratio of about 10:1 to 1:10 (the best accuracy is obtained for 1:1 ratio). The real-time PCR is able to achieve a much wider dynamic range.

Running the target and endogenous control amplifications in separate tubes and using the standard curve method requires the least amount of optimization and validation. The advantage of using the comparative C_{T} method is that the need for a standard curve is eliminated (more wells are available for samples). It also eliminates the adverse effect of any dilution errors made in creating the standard curve samples.

As long as the target and normaliser have similar dynamic ranges, the comparative C_{T} method (ΔΔC_{T} method) is the most practical method. It is expected that the normaliser will have a higher expression level than the target (thus, a smaller C_{T} value). The calculations for the quantitation start with getting the difference (ΔC_{T}) between the C_{T} values of the target and the normaliser:

ΔC_{T}= C_{T} (target) - C_{T} (normaliser)

This value is calculated for each sample to be quantitated (unless, the target is expressed at a higher level than the normaliser, this should be a positive value. It is no harm if it is negative). One of these samples should be chosen as the reference (baseline) for each comparison to be made. The comparative ΔΔC_{T} calculation involves fmding the difference between each sample's ΔC_{T} and the baseline's ΔC_{T}. If the baseline value is representing the minimum level of expression, the ΔΔC_{T} values are expected to be negative (because the ΔC_{T} for the baseline sample will be the largest as it will have the greatest C_{T} value). If the expression is increased in some samples and decreased in others, the ΔΔC_{T} values will be a mixture of negative and positive ones. The last step in quantitation is to transform these values to absolute values. The formula for this is:

comparative expression level = 2 ^{- ΔΔCT}

For expressions increased compared to the baseline level this will be something like 2³ = 8 times increase, and for decreased expression it will be something like 2⁻³= 1/8 of the reference level. Microsoft Excel can be used to do these calculations by simply entering the C_{T} values (there is an online ABI tutorial at http://www.appliedbiosystems.com/support/tutorials/7700amp/ on the use of spread sheet programs to produce amplification plots; the TaqMan™ Human Endogenous Control Plate protocol also contains detailed instructions on using MS Excel for real-time PCR data analysis).

The other (absolute) quantification methods are-outlined in the ABI User Bulletins (http://docs.appliedbiosystems.com/search.taf?_UserReference=A86583Z7189850A13A0C598 E). The Bulletins #2 and #5 are most useful for the general understanding of real-time PCR and quantification.

### Recommendations on Procedures:

1. Use positive-displacement pipettes to avoid inaccuracies in pipetting;

2. The sensitivity of real-time PCR allows detection of the target in 2 pg of total RNA. The number of copies of total RNA used in the reaction should ideally be enough to give a signal by 25-30 cycles (preferably less than 100 ng). The amount used should be decreased or increased to achieve this;

3. The optimal concentrations of the reagents are as follows;

i. Magnesium chloride concentration should be between 4 and 7 mM. It is optimized as 5.5 mM for the primers/probes designed using the Primer Express software;

ii. Concentrations of dNTPs should be balanced with the exception of dUTP (if used). Substitution of dUTP for dTTP for control of PCR product carryover requires twice dUTP that of other dNTPs. While the optimal range for dNTPs is 500 µM to 1 mM (for one-step RT-PCR), for a typical TaqMan reaction (PCR only), 200 µM of each dNTP (400 µM of dUTP) is used;

iii. Typically 0.25 µL (1.25 U) AmpliTaq DNA Polymerase (5.0 U/µL) is added into each 50 µL reaction. This is the minimum requirement. If necessary, optimization can be done by increasing this amount by 0.25 U increments;

iv. The optimal probe concentration is 50-200 nM, and the primer concentration is 100-900 nM. Ideally, each primer pair should be optimised at three different temperatures (58, 60 and 62° C for TaqMan primers) and at each combination of three concentrations (50, 300, 900 nM). This means setting up three different sets (for three temperatures) with nine reactions in each (50/50 mM, 50/300 mM, 50/900, 300/50, 300/300, 300/900, 900/50, 900/300, 900/900 mM) using a fixed amount of target template. If necessary, a second round of optimization may improve the-results. Optimal performance is achieved by selecting the primer concentrations that provide the lowest C_{T} and highest ΔRn. Similarly, the probe concentration should be optimized for 25-225 nM;

- 4. If AmpliTaq-Gold DNA Polymerase is being used, there has to be a 9-12 min pre-PCR heat step at 92 - 95° C to activate it. If AmpliTaq Gold DNA Polymerase is used, there is no need to set up the reaction on ice. A typical TaqMan reaction consists of 2 min at 50° C for UNG (see below) incubation, 10 min at 95° C for Polymerase activation, and 40 cycles of 15 sec at 95° C (denaturation) and 1 min at 60° C (annealing and extension). A typical reverse transcription cycle (for cDNA synthesis), which should precede the TaqMan reaction if the starting material is total RNA, consists of 10 min at 25° C (primer-incubation), 30 min at 48° C (reverse transcription with conventional reverse transcriptase) and 5 min at 95° C (reverse transcriptase inactivation);

5. AmpErase uracil-N-glycosylase (UNG) is added in the reaction to prevent the reamplification of carry-over PCR products by removing any uracil incorporated into amplicons. This is why dUTP is used rather than dTTP in PCR reaction. UNG does not function above 55 °C and does not cut single-stranded DNA with terminal dU nucleotides. UNG-containing master mix should not be used with one-step RT-PCR unless rTth DNA polymerase is being used for reverse transcription and PCR (TaqMan EZ RT-PCR kit);

6. It is necessary to include at least three No Amplification Controls (NAC) as well as three No Template Controls (NTC) in each reaction plate (to achieve a 99.7% confidence level in the definition of +/- thresholds for the target amplification, six replicates of NTCs must be run). NAC former contains sample and no enzyme. It is necessary to rule out the presence of fluorescence contaminants in the sample or in the heat block of the thermal cycler (these would cause false positives). If the absolute fluorescence of the NAC is greater than that of the NTC after PCR, fluorescent contaminants may be present in the sample or in the heating block of the thermal cycler;

7. The dynamic range of a primer/probe system and its normaliser should be examined if the ΔΔC_{T} method is going to be used for relative quantitation. This is done by running (in triplicate) reactions of five RNA concentrations (for example, 0, 80 µg/µL, 400 pg/µL, 2 ng/µL and 50 ng/µL). The resulting plot of log of the initial amount vs C_{T} values (standard curve) should be a (near) straight line for both the target and normaliser real time RT-PCRs for the same range of total RNA concentrations;

8. The passive reference is a dye (ROX) included in the reaction (present in the TaqMan universal PCR master mix). It does not participate in the 5' nuclease reaction. It provides-an internal reference for background fluorescence emission. This is used to normalize the reporter-dye signal. This normalization is for non-PCR-related fluorescence fluctuations occurring well-to-well (concentration or volume differences) or over time and different from the normalization for the amount of cDNA or efficiency of the PCR. Normalization is achieved by dividing the emission intensity of reporter dye by the emission intensity of the passive reference. This gives the ratio defined as Rn;

9. If multiplexing is- done, the more abundant of the targets will use up all the ingredients of the reaction before the other target gets a chance to amplify. To avoid this, the primer concentrations for the more abundant target should be limited;

10. TaqMan Universal PCR master mix should be stored at 2 to 8° C (not at -20° C);

11. The GAPDH probe supplied with the TaqMan Gold RT-PCR kit is labeled with a JOE reporter dye, the same probe provided within the Pre-Developed TaqMan™ Assay Reagents (PDAR) kit is labeled with VIC. Primers for these human GAPDH assays are designed not to amplify genomic DNA;

12. The carryover prevention enzyme, AmpErase UNG, cannot be used with one-step RT-PCR which requires incubation at 48° C but may be used with the EZ RT-PCR kit;

13. One-step RT-PCR can only be used for singleplex reactions, and the only choice for reverse transcription is the downstream primer (not random hexamers or oligo-dT);

14. It is ideal to mn duplicates to control pipetting errors but this inevitably increases the cost;

15. If multiplexing, the spectral compensation option (in Advanced Options) should be checked before the run;

16. Normalization for the fluorescent fluctuation by using a passive reference (ROX) in the reaction and for the amount of cDNA/PCR efficiency by using an endogenous control (such as GAPDH, active reference) are different processes;

17.ABI 7700 can be used not only for quantitative RT-PCR but also endpoint PCR. The latter includes presence/absence assays or allelic discrimination assays (such as SNP typing);

18. Shifting Rn values during the early cycles (cycle 0-5) of PCR means initial disequilibrium of the reaction components and does not affect the final results as long as the lower value of baseline range is reset;

19. If an abnormal amplification plot has been noted (C_{T} value <15 cycles with amplification signal detected in early cycles), the upper value of the baseline range should be lowered and the samples should be diluted to increase the C_{T} value (a high C_{T} value may also be due to contamination);

20. A small ΔRn value (or greater than expected C_{T} value) indicates either poor PCR-efficiency or low copy number of the target;

21. A standard deviation >0.16 for C_{T} value indicates inaccurate pipetting;

22. SYBR Green entry in the Pure Dye Setup should be abbreviated as "SYBR" in capitals. Any other abbreviation or lower case letters will cause-problems;

23. The SDS software for ABI 7700 have conflicts with the Macintosh Operating System version 8.1. The data should not be analyzed on such computers;

24. The ABI 7700 should not be deactivated for extended periods of time. If it has ever been shutdown, it should be allowed to warm up for at least one hour before a run. Leaving the instrument on all times is recommended and is beneficial for the laser. If the machine has been switched on just before a run, an error box stating a firmware version conflict may appear. If this happens, choose the "Auto Download" option;

25. The ABI 7700 is only one of the real-time PCR systems available, others include systems from BioRad, Cepheid, Corbett Research, Roche and Stratagene.

### Genotyping Analysis

Many methods are available to genotype DNA. A review of allelic discrimination methods can be found in Kristensen et al. (Biotechniques 30(2): 318-322 (2001). Only one method, allele-specific PCR is described here.

### Primer Design

Upstream and downstream PCR primers specific for particular alleles can be designed using freely available computer programs, such as Primer3 (http://frodo.wi.mit.edu/primer3/primer3-code.html). Alternatively the DNA sequences of the various alleles can be aligned using a program such as ClustalW (http://www.ebi.ac.uk/clustalw/) and specific primers designed to areas where DNA sequence differences exist but retaining enough specificity to ensure amplification of the correct amplicon. Preferably a PCR amplicon is designed to have a restriction enzyme site in one allele but not the other. Primers are generally 18-25 base pairs in length with similar melting temperatures.

### PCR Amplification

The composition of PCR reactions has been described elsewhere (Clinical Applications of PCR, Dennis Lo (Editor), Blackwell Publishing, 1998). Briefly, a reaction contains primers, DNA, buffers and a thermostable polymerase enzyme. The reaction is cycled (up to 50 times) through temperature steps of denaturation, hybridization and DNA extension on a thermocycler such as the MJ Research Thermocycler model PTC-96V.

### DNA Analysis

PCR products can be analyzed using a variety of methods including size differentiation using mass spectrometry, capillary gel electrophoresis and agarose gel electrophoresis. If the PCR amplicons have been designed to contain differential restriction enzyme sites, the DNA in the PCR reaction is purified using DNA-binding columns or precipitation-and re-suspended in-water, and then restricted using the appropriate restriction enzyme. The restricted DNA can then be run on an agarose gel where DNA is separated by size using electric current. Various alleles of a gene will have different sizes depending on whether they contain restriction sites.

### EXAMPLE 2

### IDENTIFICATION OF GENES AND PRIORITY RANKING OF GENES

Significant genes were ranked according to an Empirical Bayes approach (Lonnstedt and Speed, 2002, *Statistica Sinica* **12**: 31-46), based on a comparison of all animals at Day 28 compared to animals on days 0, 2, 4, 7, 9, 11, 14, 17,21, and 24. The empirical Bayes approach was used to provide a shrinkage estimator of the within groups variance for each gene.

Individual p values were based on a t Test using this shrinkage estimator. The p values of the t test were adjusted using Holms method to maintain strong control of the family wise type I error rate.

The genes listed in Table 5 were generated from a total of 783 genes that were significant (p<0.05) across the various days. This gene list was trimmed by eliminating those genes-that were significant for less than two days and where p>0.001. The remaining genes were then ranked in increasing order of their p value..

It should be noted that this gene list is not inclusive of the genes that can act as diagnostics for stress (see also the minimally predictive set and gene ontology).

The genes listed in Table 6 are ranked in order of their t statistic or value which may be interpreted as a signal-to-noise ratio. The tabulation also displays the log 2 fold change (M value), and the adjusted p values. Genes with a negative t value (and hence a negative M value) are down regulated. Genes with positive t and M values are up-regulated. The priority ranking of genes is based on increasing value of t value for the first day each gene is significant (p<0.001) following stress induction, and for genes that were significant for at least three sampling times.

### EXAMPLE 3

### DEMONSTRATION OF DIAGNOSTIC POTENTIAL TO DETERMINE STRESS RESPONSE

The diagnostic potential of the entire set of genes was assessed using discriminant analysis (Venables and Ripley, 2002, Modern Applied Statistics in S, Springer) on the principal component scores (Jolliffe, I.T. Principal components analysis, Springer-Verlag, 1986) calculated from gene expression. Comparisons were made between-samples taken immediately after the stressor, and at 2, 4, 7, 9, 11, 14, 17, 21, 24 and 28 days after the stressor.

The entire process was cross-validated Sensitivity and-specificity were calculated for a uniform prior. This may be interpreted as a form of shrinkage regularization, where the estimates are shrunken to lie in a reduced space.

Cross validated estimates of discriminant function scores were then used to construct an ROC curve (Lloyd C.J., 1998, The use of smoothed ROC curves to summarize and compare diagnostic systems, *Journal of the American Statistical Association* **93**:1356-1364). The ROC curves were based both on empirical cumulative distribution functions, and on kernel density estimates with a smoothing window chosen using Lloyd's method (*loc. cit*).

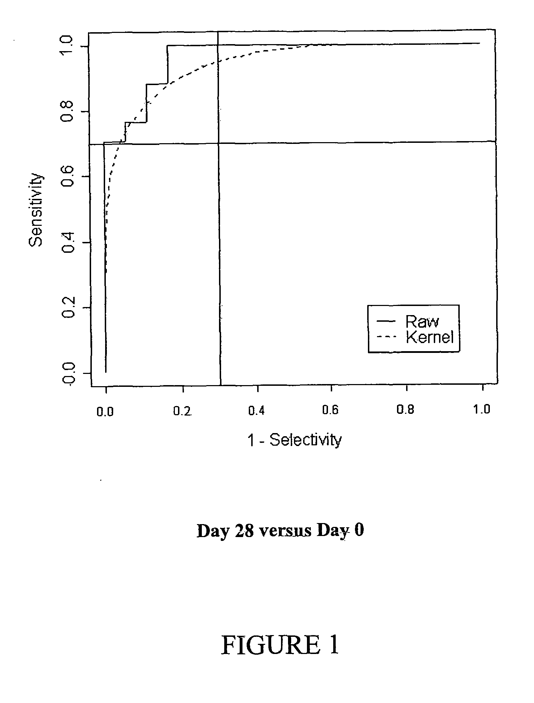

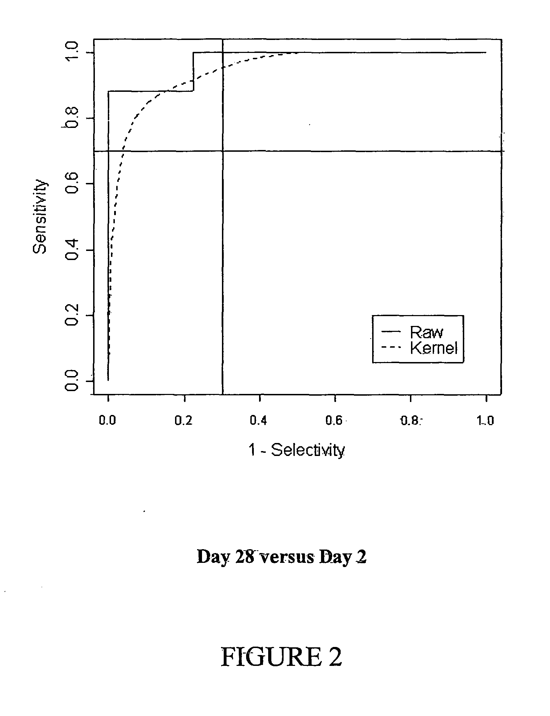

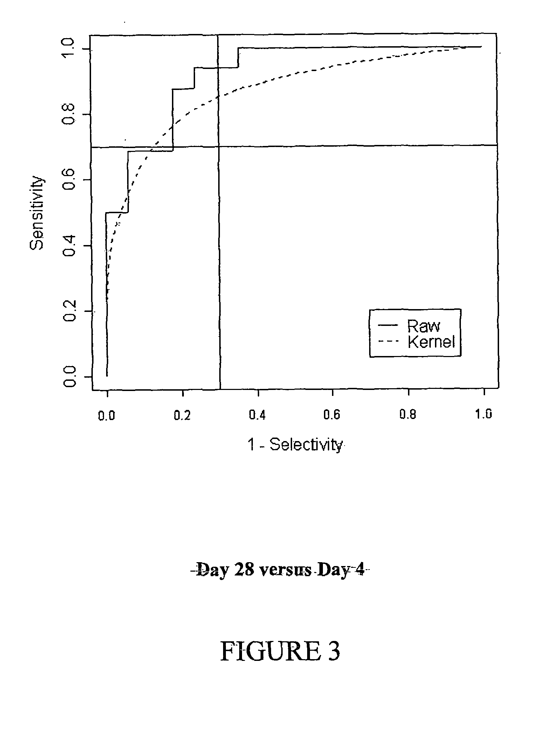

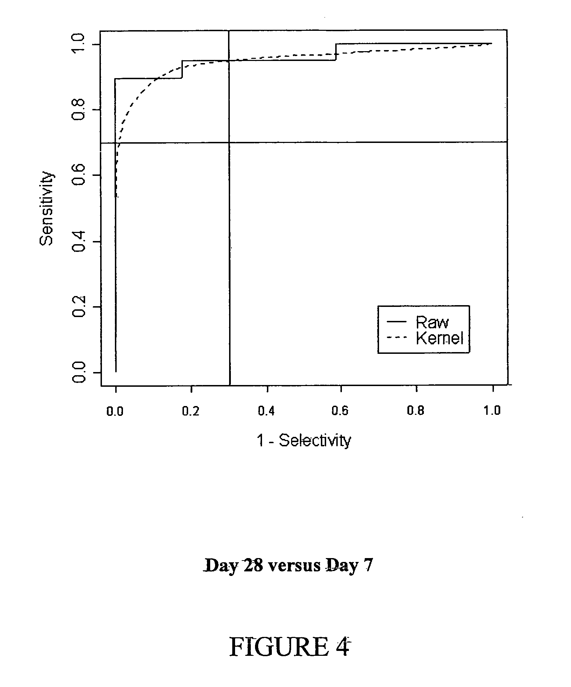

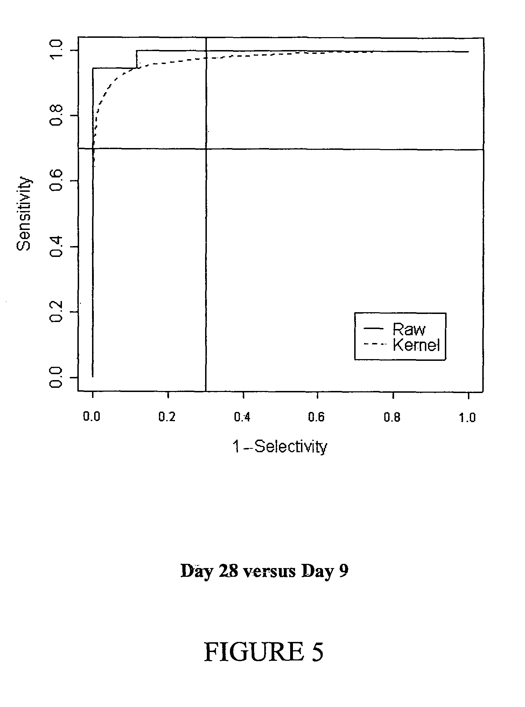

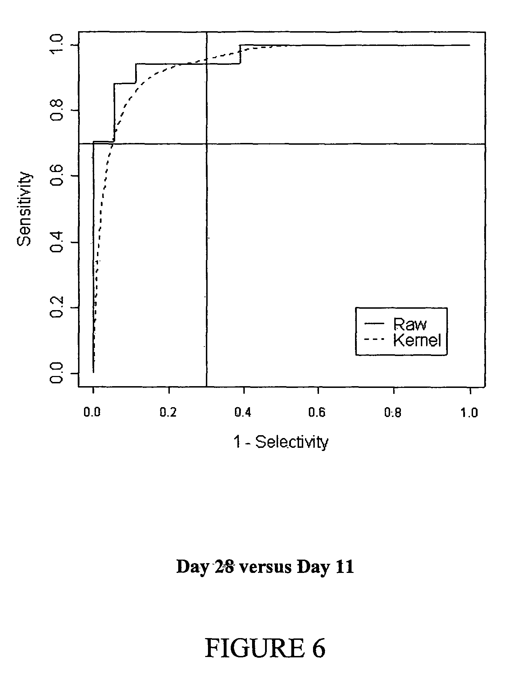

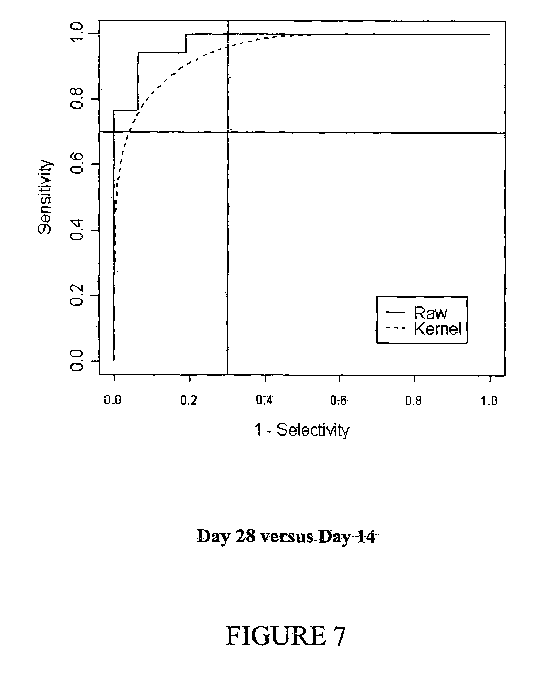

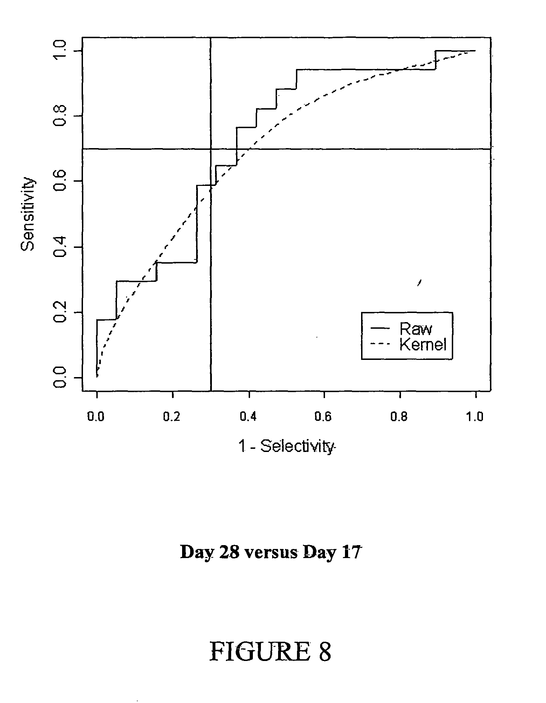

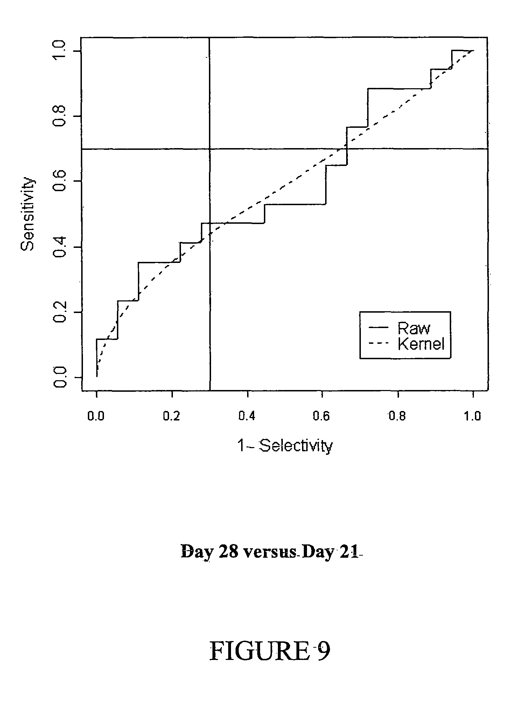

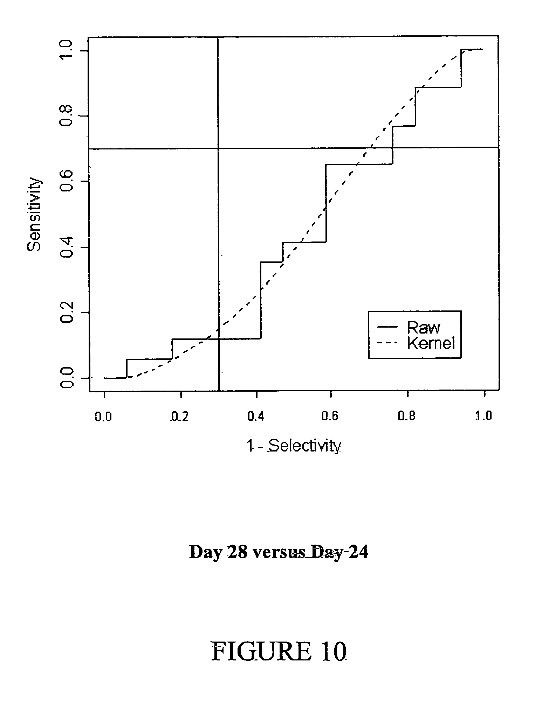

ROC curves for the comparison of each day with Day 28 are shown in Figures 1 to 10, respectively.

Changes in gene expression following transport stress are of sufficient magnitude to produce excellent diagnostic potential.

### EXAMPLE 4

### MINIMALLY PREDICTIVE GENE SETS

Although a large number of genes has been identified as having diagnostic potential, a much fewer number are generally required for acceptable diagnostic performance.

Table 7 shows the cross validated classification success, sensitivity and specificity obtained from a linear discriminant analysis, based on two genes selected from the set of potential diagnostic genes. The pairs presented are those producing the highest prediction success, many other pairs of genes produce acceptable classification success. The identification of alternate pairs of genes would be readily apparent to those skilled in the art. Techniques for identifying pairs include (but are not limited to) forward variable selection (Venables W.N. and Ripley B.D. Modem Applied Statistics in S 4^{th} Edition 2002. Springer), best subsets selection, backwards elimination (Venables W.N. and Ripley B.D., 2002, *supra),* stepwise selection (Venables W.N. and Ripley B.D., 2002, *supra*) and stochastic variable elimination (Figuerado M.A. Adaptive Sparseness for Supervised Learning).

Table 8 shows the cross-validated classification success obtained from a linear discriminant analysis based on three genes selected from the diagnostic set. Only twenty sets of three genes are presented. It will be readily apparent to those of skill in the art that other suitable diagnostic selections based on three stress marker genes can be made.

Table 9 shows the cross validated classification success obtained from a linear discriminant analysis based on four genes selected from the diagnostic set. Only twenty sets of four genes are presented. It will be readily apparent to practitioners in the art that other suitable diagnostic selections-based on four stress marker genes can be made.

Table 10 shows the cross-validated classification success obtained from a linear discriminant analysis based on five genes selected from the diagnostic set. Only twenty sets of-five genes are presented. It will be readily apparent to practitioners in the art that other suitable diagnostic selections based on five stress marker genes can be made.

Table 11 shows the cross-validated classification success obtained from a linear discriminant analysis based on six genes selected from the diagnostic set. Only twenty sets of six genes are presented. It will be readily apparent to practitioners in the art that other suitable diagnostic selections based on six stress marker genes can be made.

Table 12 shows the cross-validated classification success obtained from a linear discriminant analysis based on seven genes selected from the diagnostic set. Only twenty sets of seven genes are presented. It will be readily apparent to practitioners in the art that other suitable diagnostic selections based on seven stress marker genes can be made.

Table 13 shows the cross-validated classification success obtained from a linear discriminant analysis based on eight genes selected from the diagnostic set. Only twenty sets of eight genes are presented. It will be readily apparent to practitioners in the art that other suitable diagnostic selections based on eight stress marker genes can be made.

Table 14 shows the cross-validated classification success obtained from a linear discriminant analysis based on nine genes selected from the diagnostic set. Only twenty sets of nine genes are presented. It will be readily apparent to practitioners in the art that other suitable diagnostic selections based on nine stress marker genes can be made.

Table 15 shows the cross-validated classification success obtained from a linear discriminant analysis based on ten genes selected from the diagnostic set. Only twenty sets of ten genes are presented. It will be readily apparent to practitioners in the art that other suitable diagnostic selections based on ten stress marker genes can be made.

Table 16 shows the cross-validated classification success obtained from a linear discriminant analysis based on 20 genes selected from the diagnostic set. Only 20 sets of twenty genes are presented. It will be readily apparent to practitioners in the art that other suitable diagnostic selections based on twenty stress marker genes can be made.

### EXAMPLE 5

### DEMONSTRATION OF SPECIFICITY

The specificity of a stress gene signature is difficult to define because the test is an assessment rather than a diagnostic.

Nonetheless, the-entire set of "stress genes" were used as a training set against a gene expression database of over 850 GeneChip™. Gene expression results in the database were obtained from samples from horses with various diseases and conditions including; chronic and acute induced EPM, clinical cases of EPM, herpes virus infection, degenerative osteoarthritis, Rhodococcus infection, endotoxemia, laminitis, gastric ulcer syndrome, animals in athletic training and clinically normal animals. The stress status of these animals was not known *a priori.*

A stress index score was calculated for each GeneChip™, using the genes in the training set. The score was calculated from a regularized discriminant function, so that large values would be associated with high probability of stress, and the variance of the score should be approximately 1. GeneChip™ were ranked on this score, from the largest to the smallest.

Specificity was investigated by varying a threshold value for a positive diagnosis. At each value of the threshold, specificity was defined as the proportion of positive results (i.e. GeneChip™ index score greater than the threshold) which were true positives. A threshold value of two (i.e. two standard deviations) was adopted.

59 animals from the database that were not part of the induced stress trial were identified as having immune modification associated with stress and were two standard deviations above zero on discriminant function when using four principal components and the entire gene-set (3105). Of these 59 animals, 10 were in a laminitis trial, 14 had R. equi infection and nine had gastritis. Thirteen animals were "controls," and of these, three had been recently transported, two were in a trial, three were not clinically normal and five were foals with exposure to R. equi. Twelve animals deemed to be clinically normal were identified by the signature as stressed. Based on this information, it can be stated that the specificity of the stress signature is over 90% when used against a database of over 850 samples.

79 animals from the database that were not part of the induced stress trial were identified as having immune modification associated with stress and were two standard deviations above zero on discriminant function when using four principal components and the unique stress signature genes listed in Table 1. Of these 79 animals, 15 were in a laminitis trial, 8 had R equi infection and 24 had gastritis. Twenty-one were "controls", and of these, 12 were in a trial, and three were not clinically normal. Nine animals deemed to be clinically normal were identified by-the signature as stressed. Based on this information, it can be stated that the specificity of the stress signature is over 90% when used against a database of over 850 samples.

### EXAMPLE 7

### GENE ONTOLOGY

Gene sequences were compared against the GenBank database using the BLAST algorithm (Altschul, S.F., Gish, W., Miller, W., Myers, E.W. & Lipman, D.J. (1990) "Basic local alignment search tool." J. Mol. Biol. 215:403-410), and gene homology and gene ontology searches were performed in order to group genes based on function, metabolic processes or cellular component (using UniProt and GenBank). Table 17 lists and groups the genes based on these criteria and information available at the time. See also Table 1, which contains sequence information for each gene.

The disclosure of every patent, patent application, and publication cited herein is hereby incorporated herein by reference in its entirety.

The citation of any reference herein should not be construed as an admission that such reference is available as "Prior Art" to the instant application.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

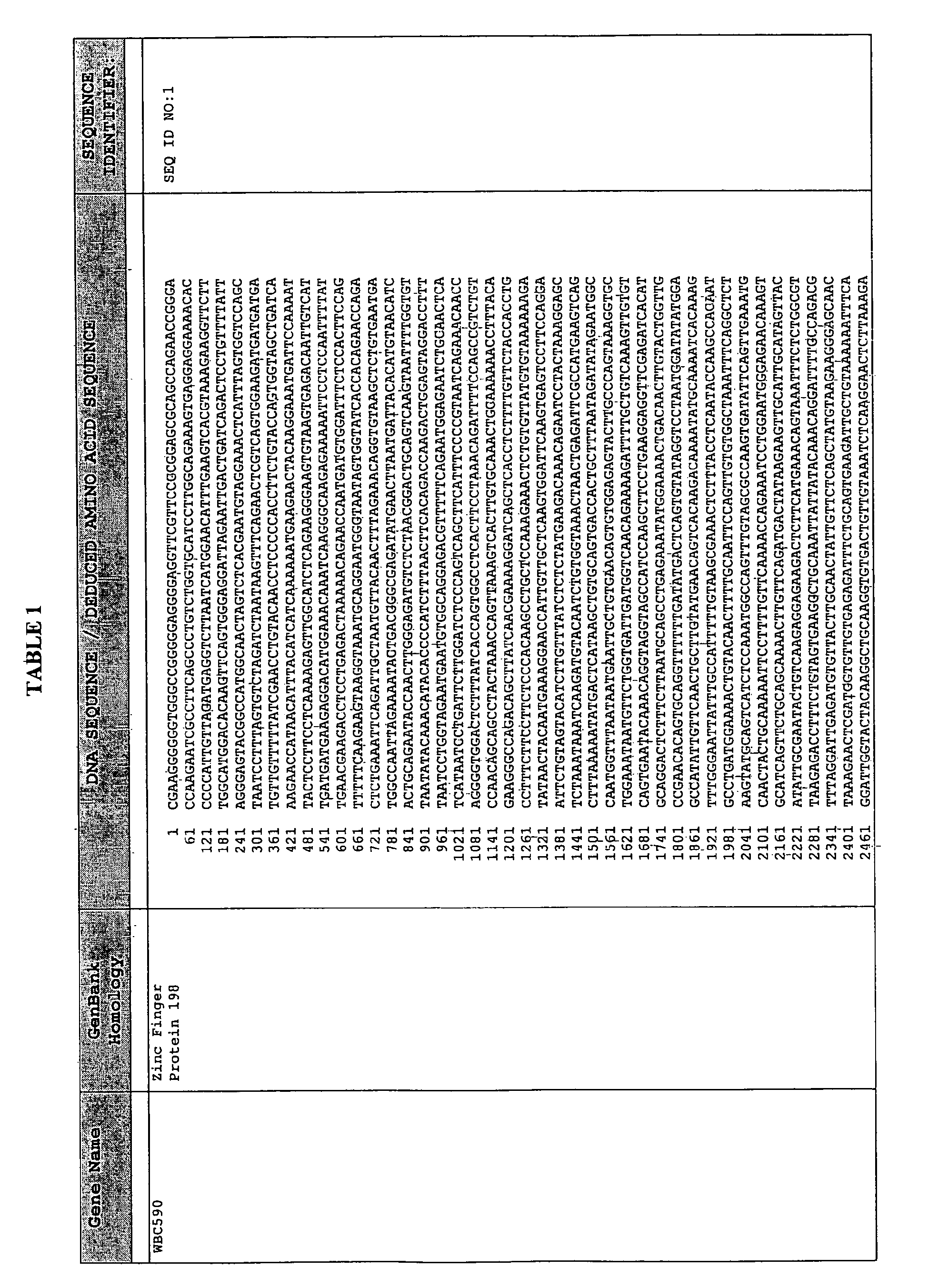

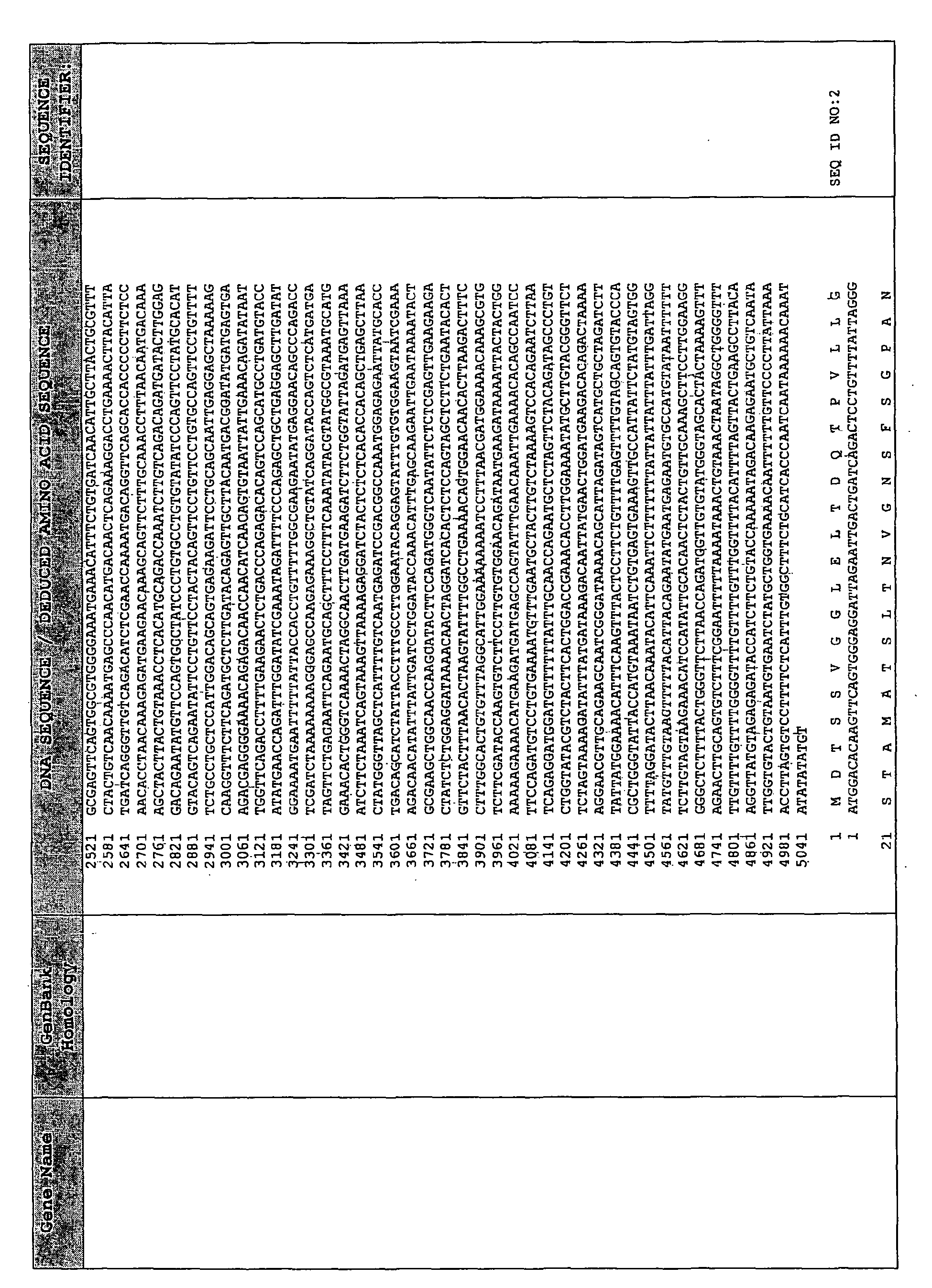

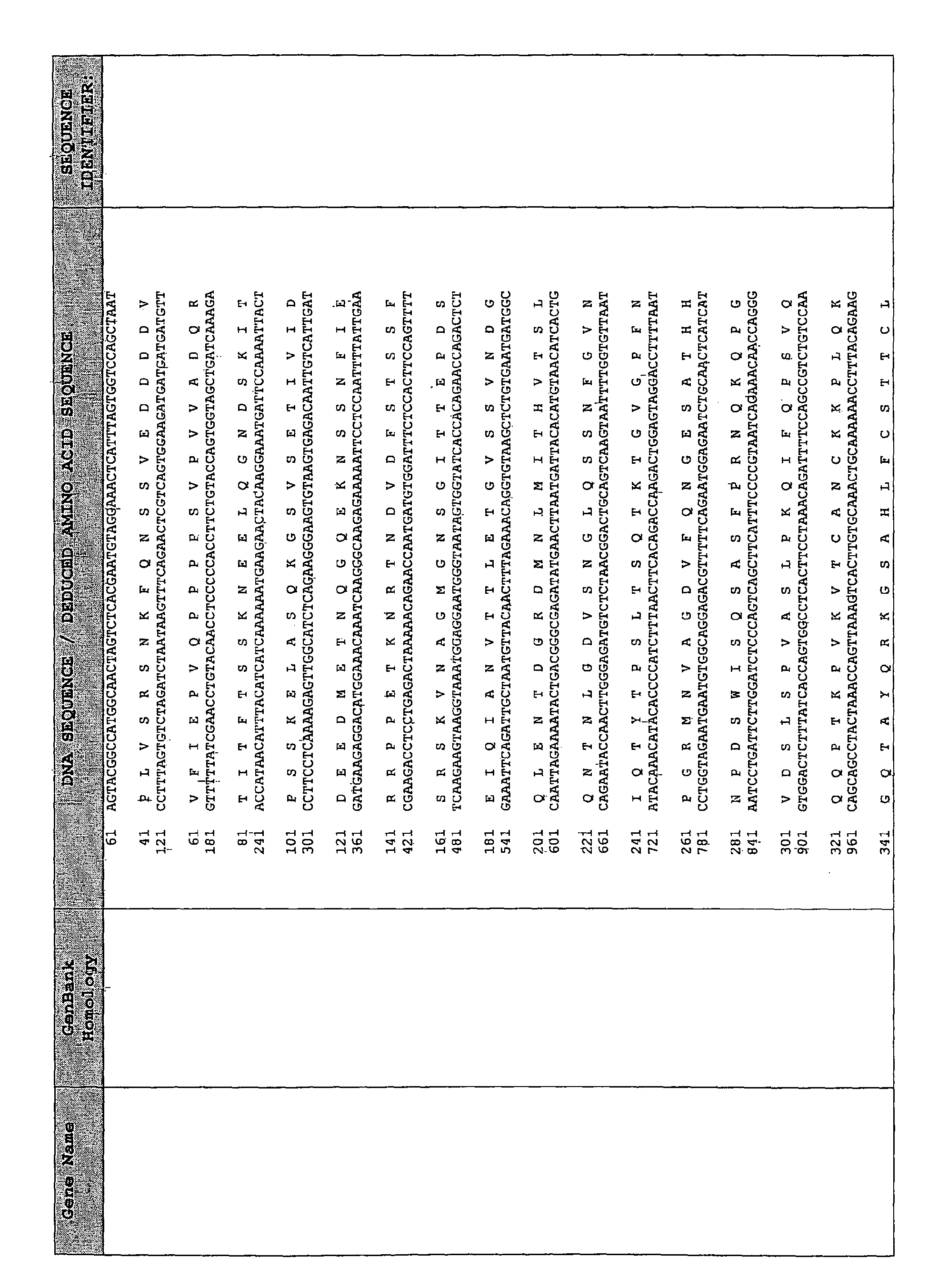

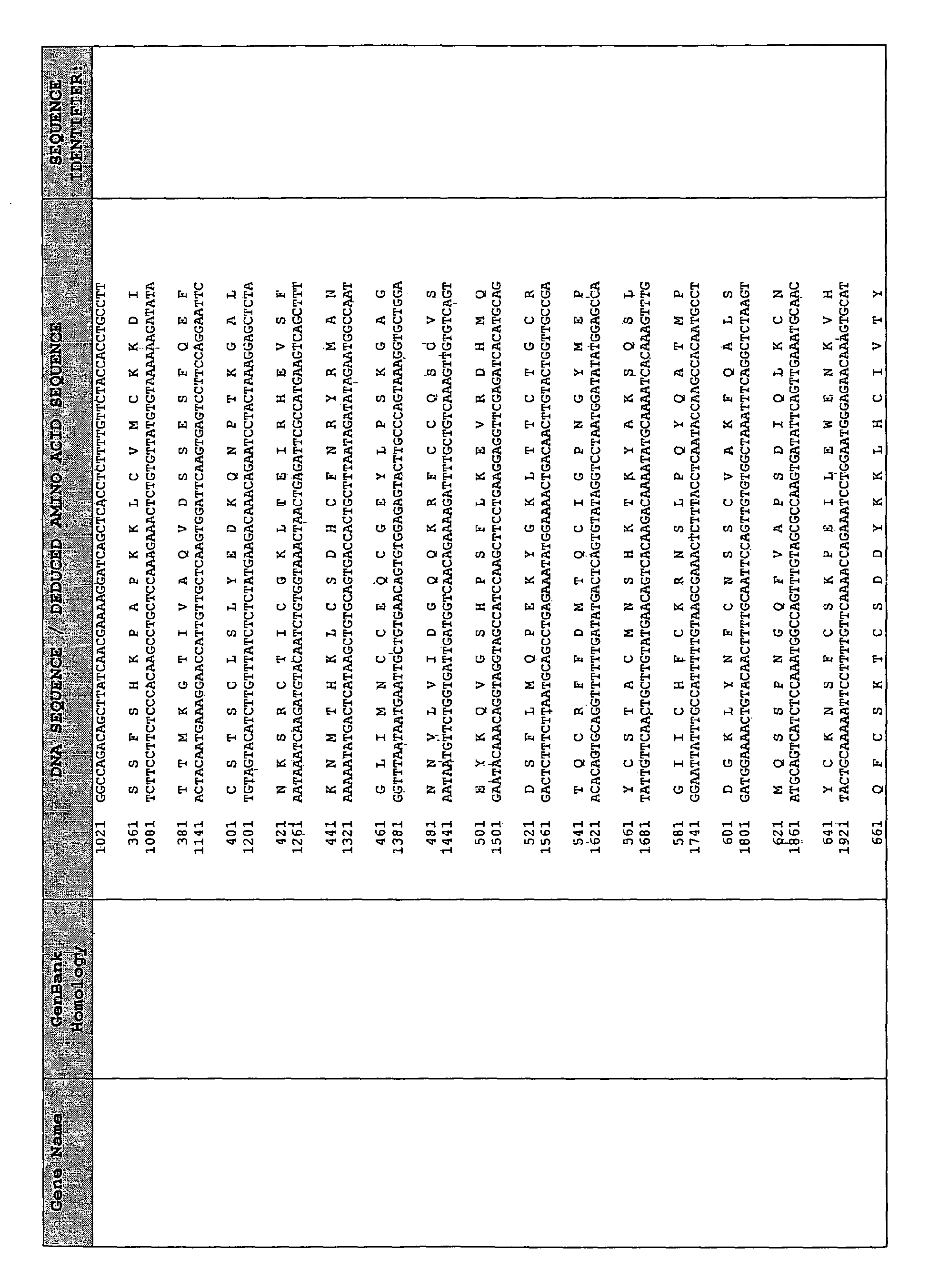

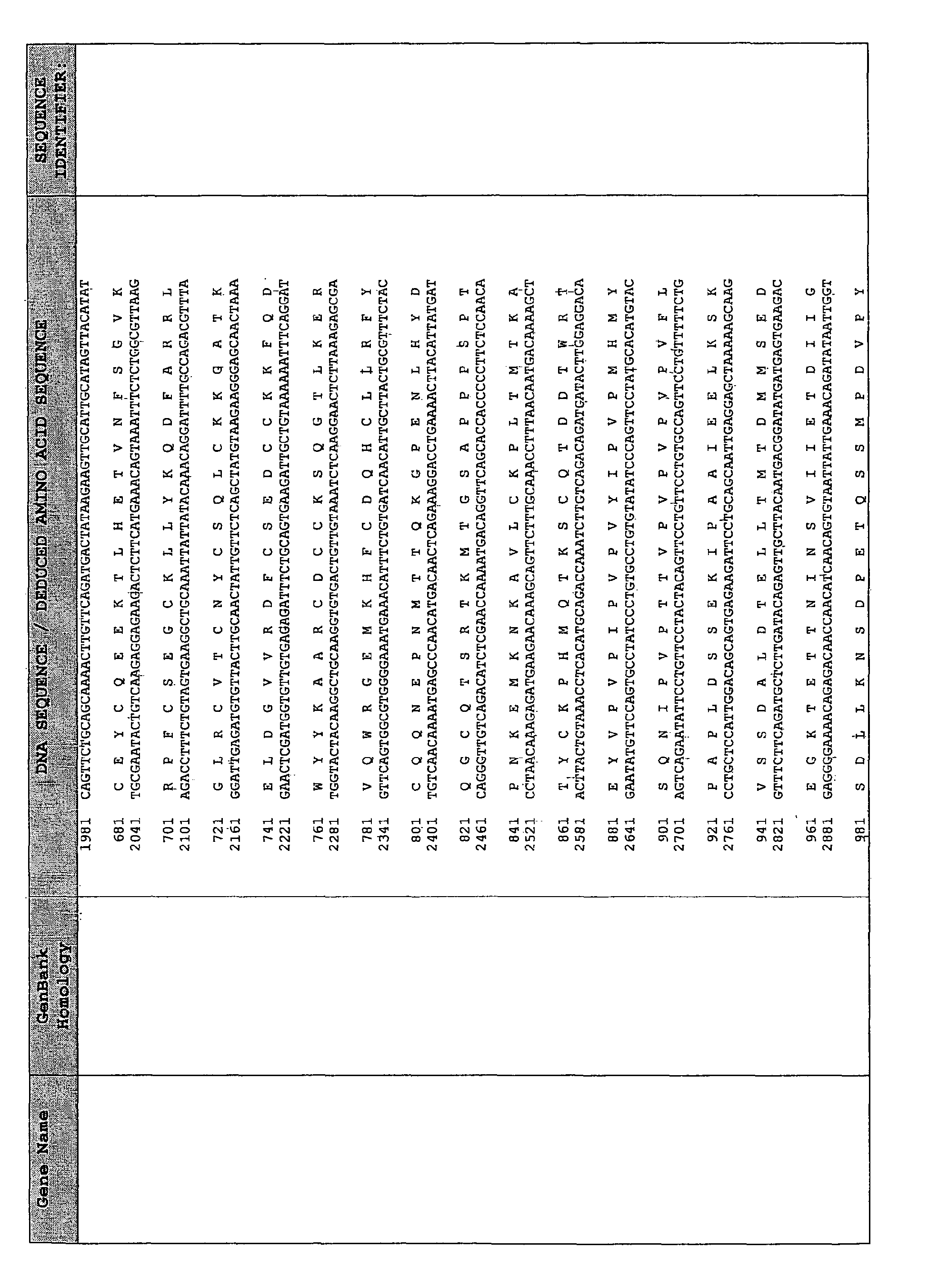

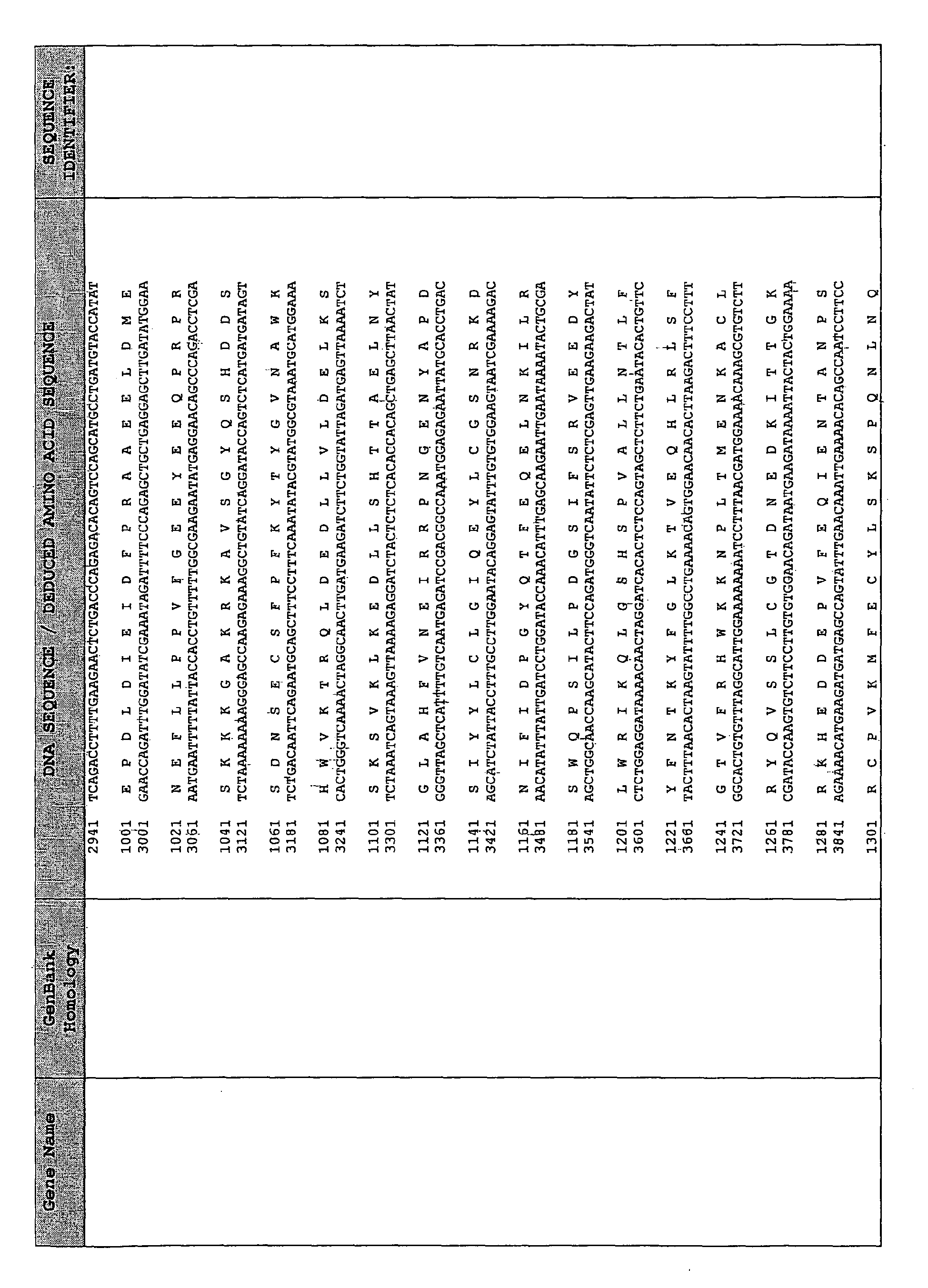

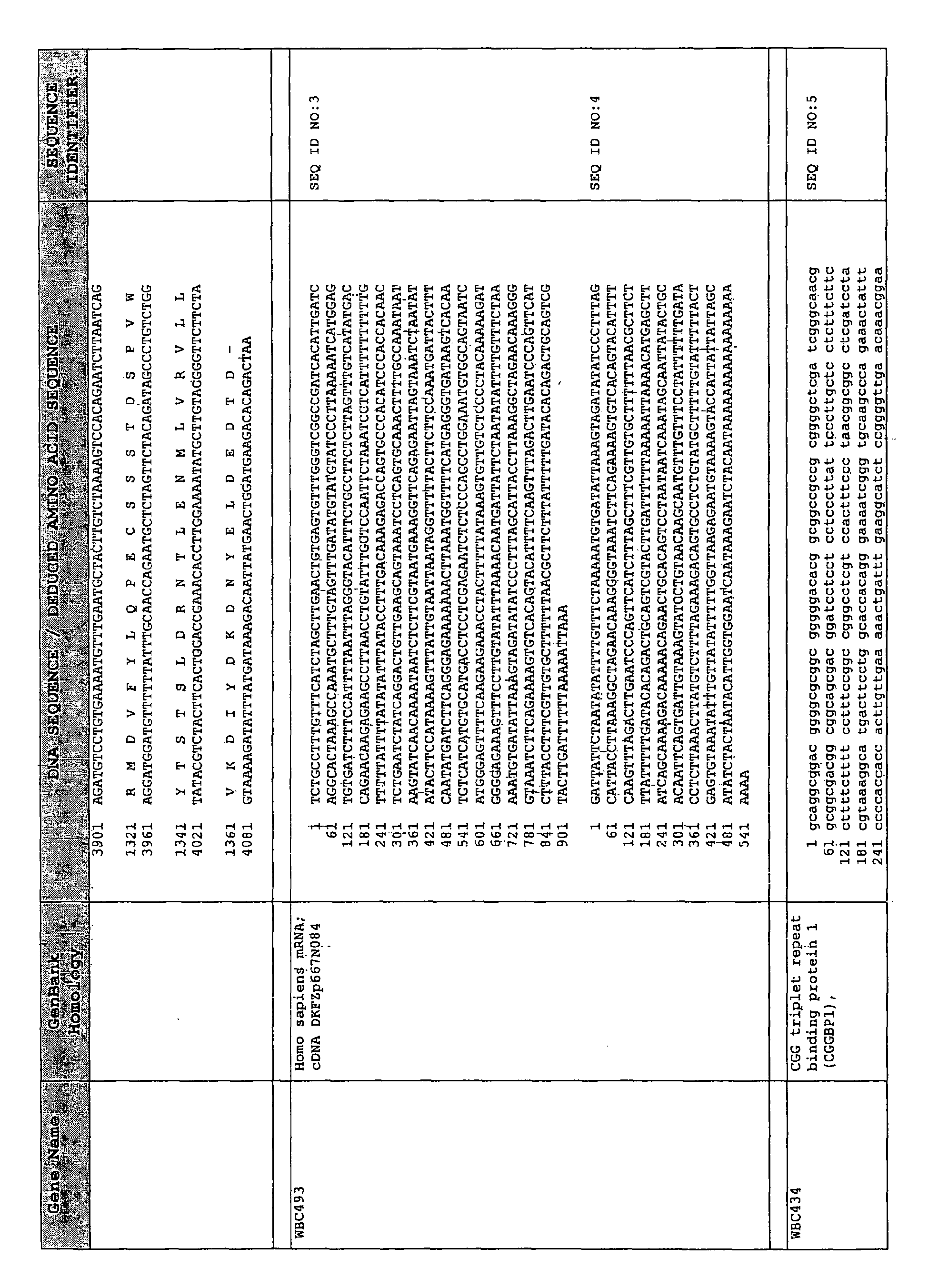

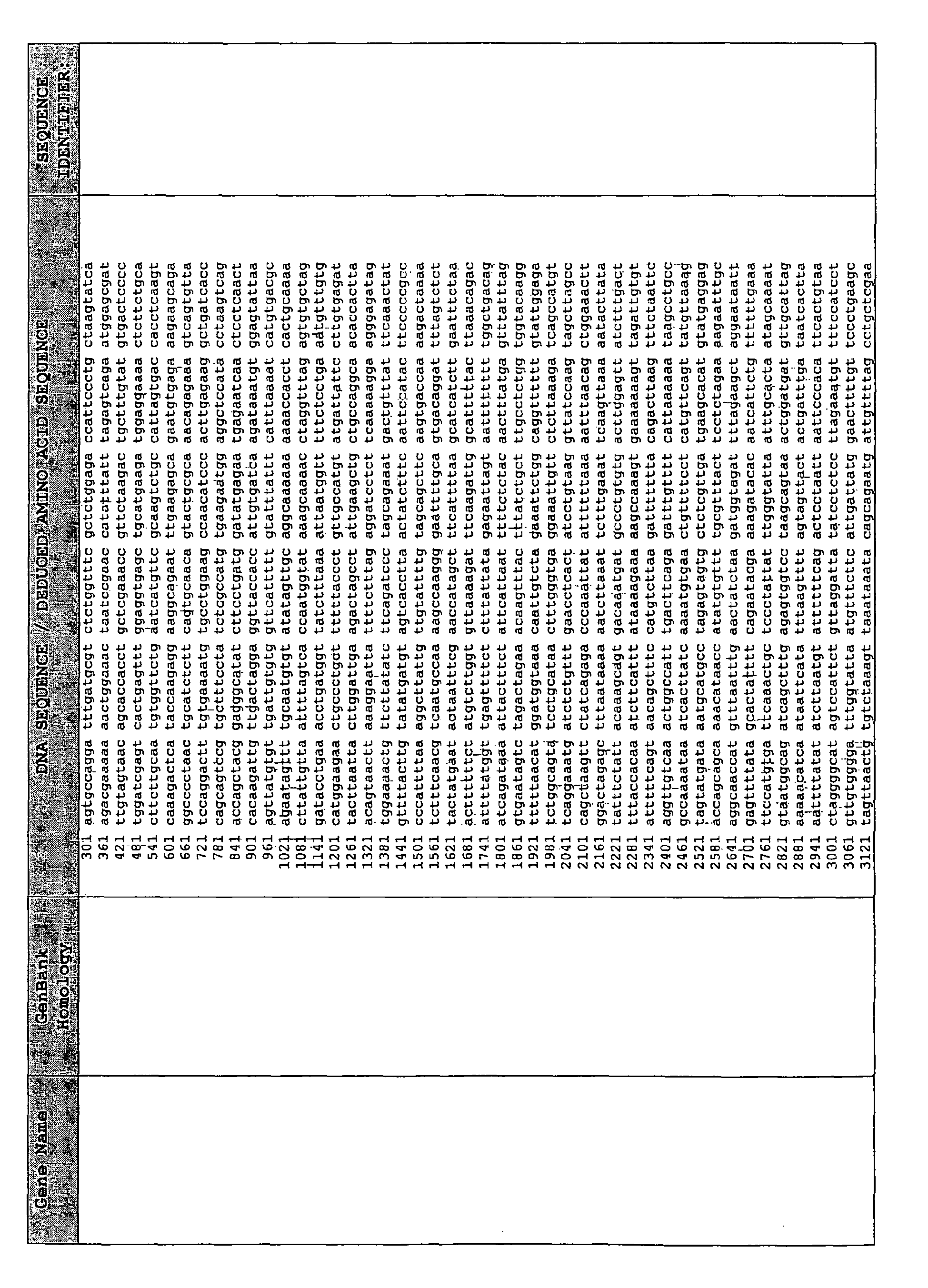

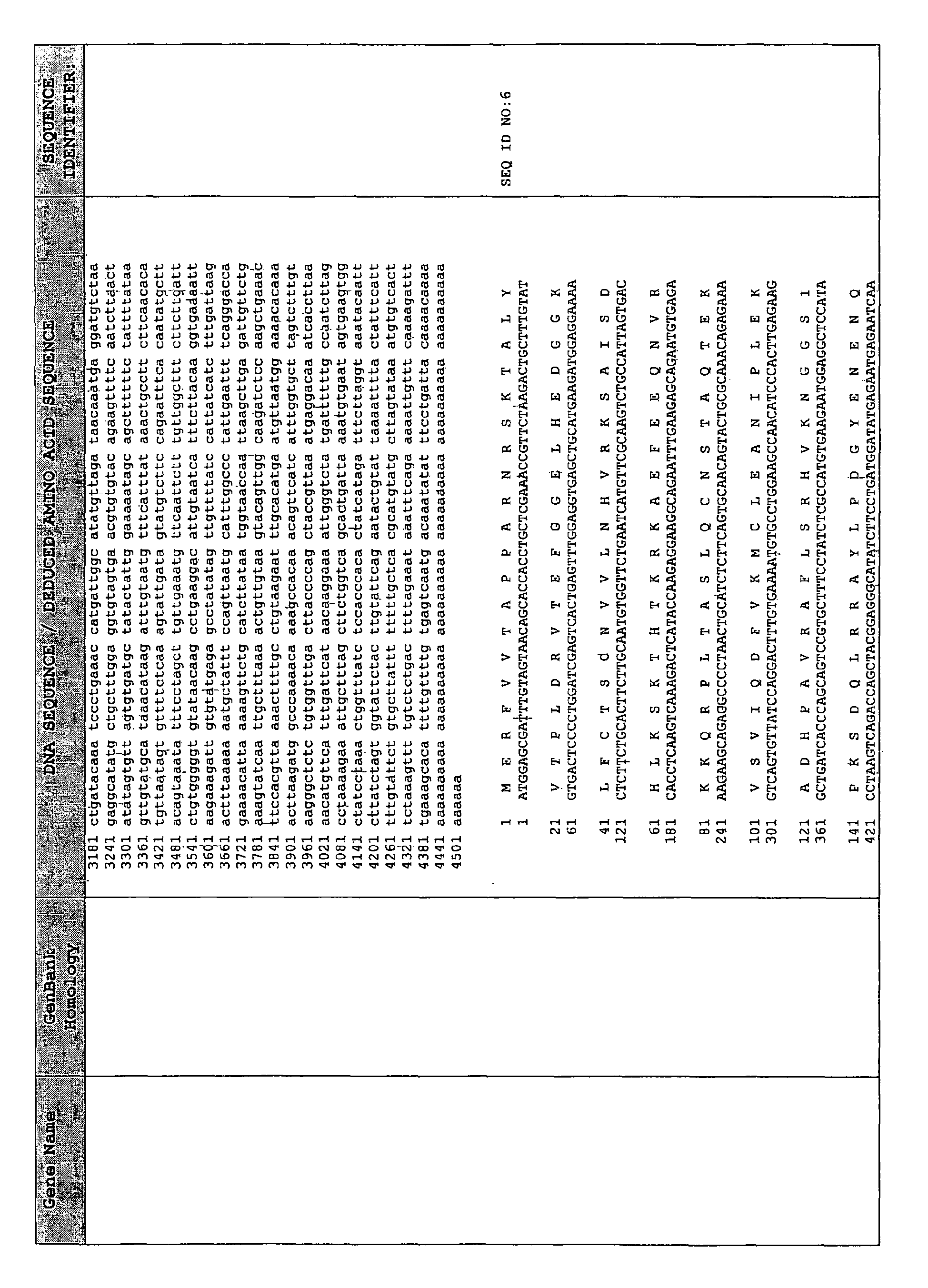

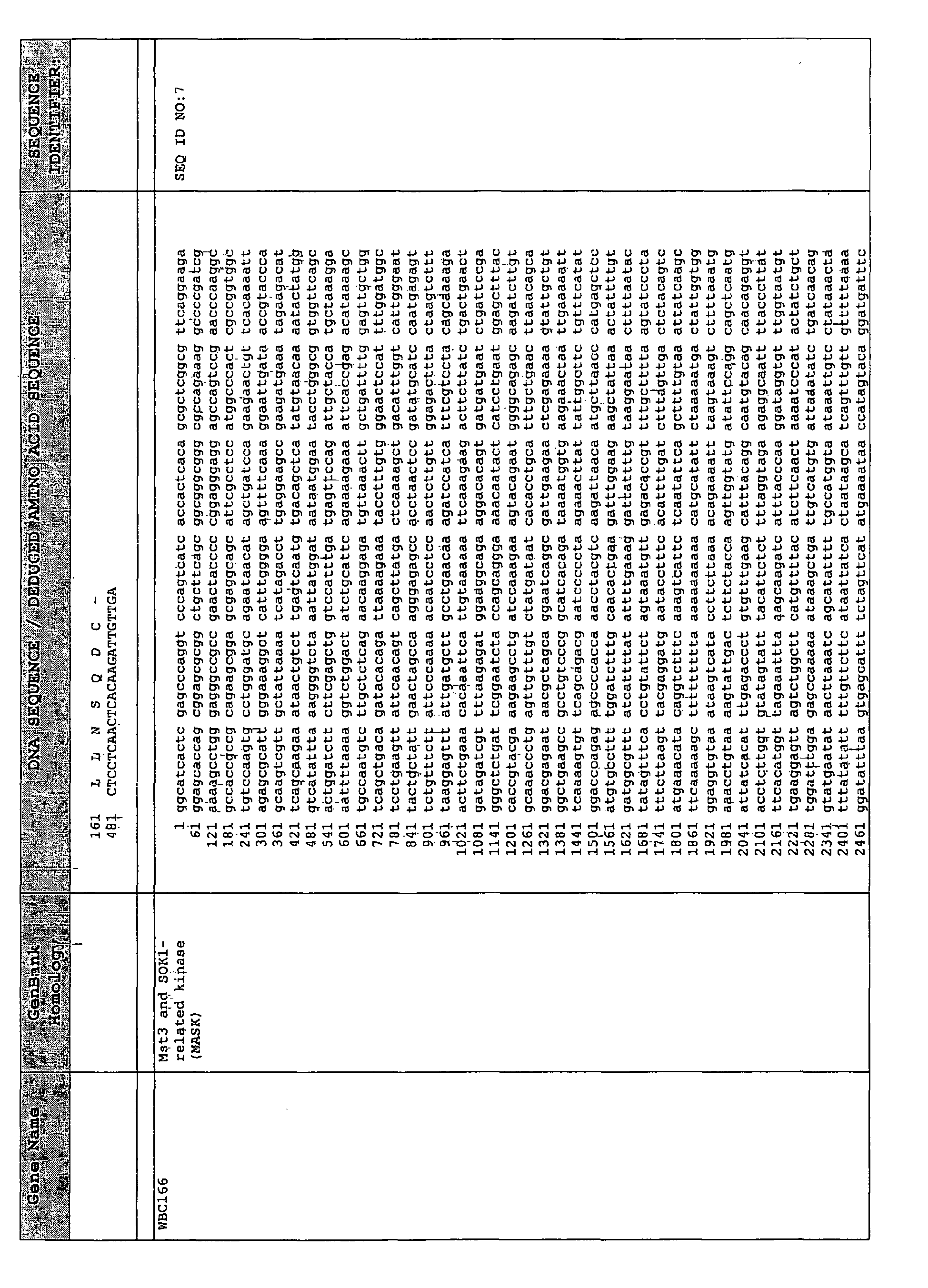

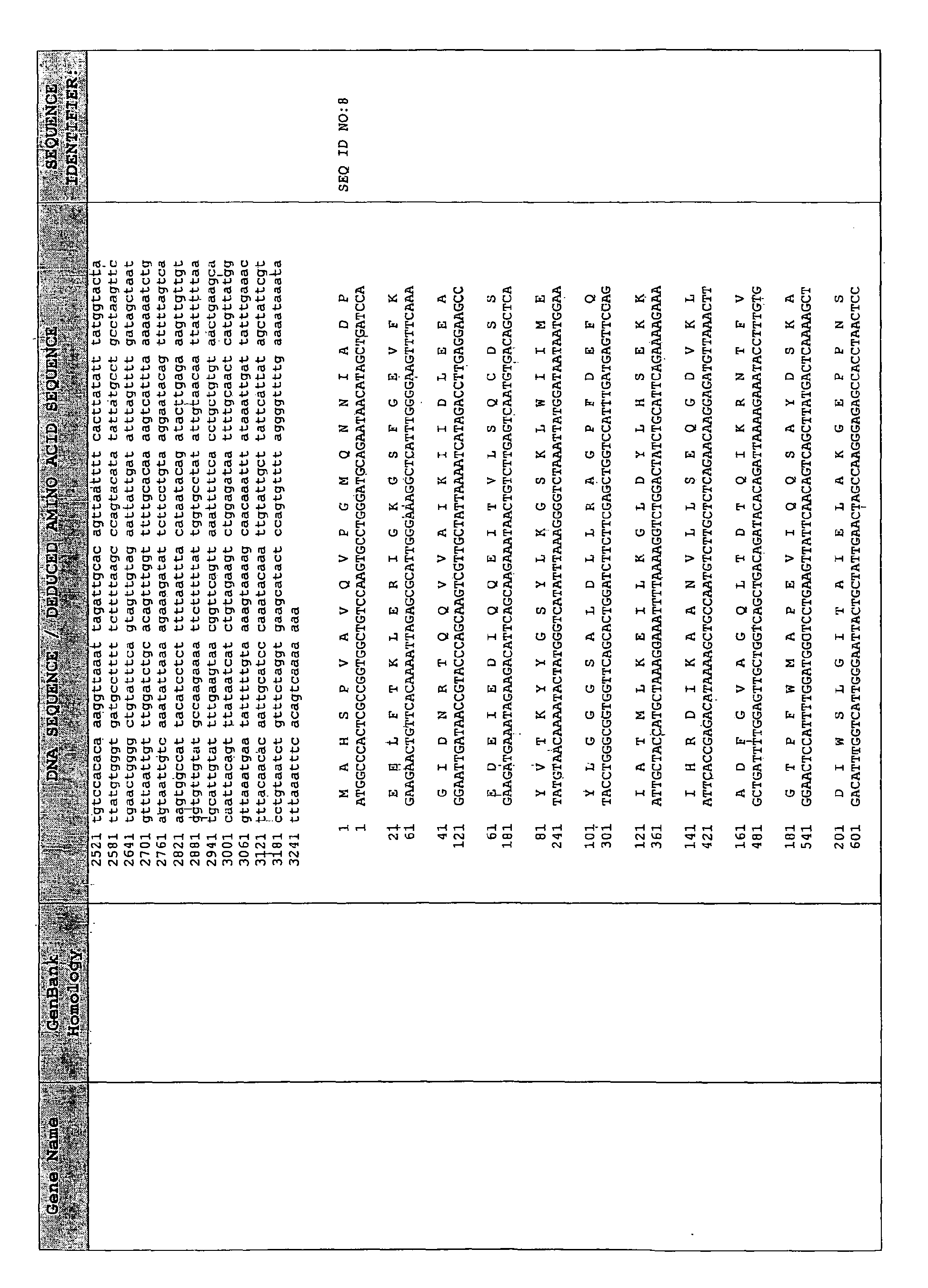

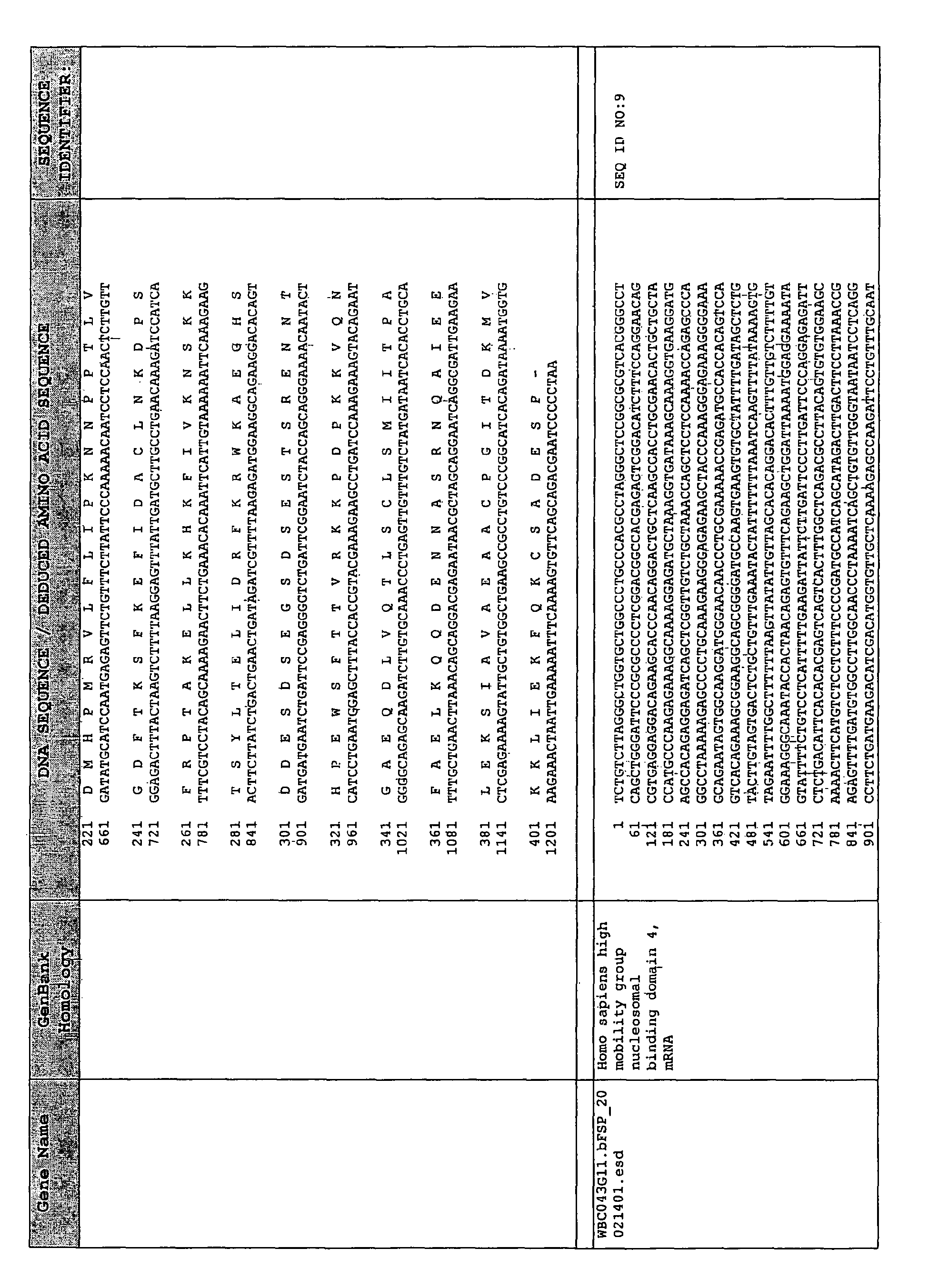

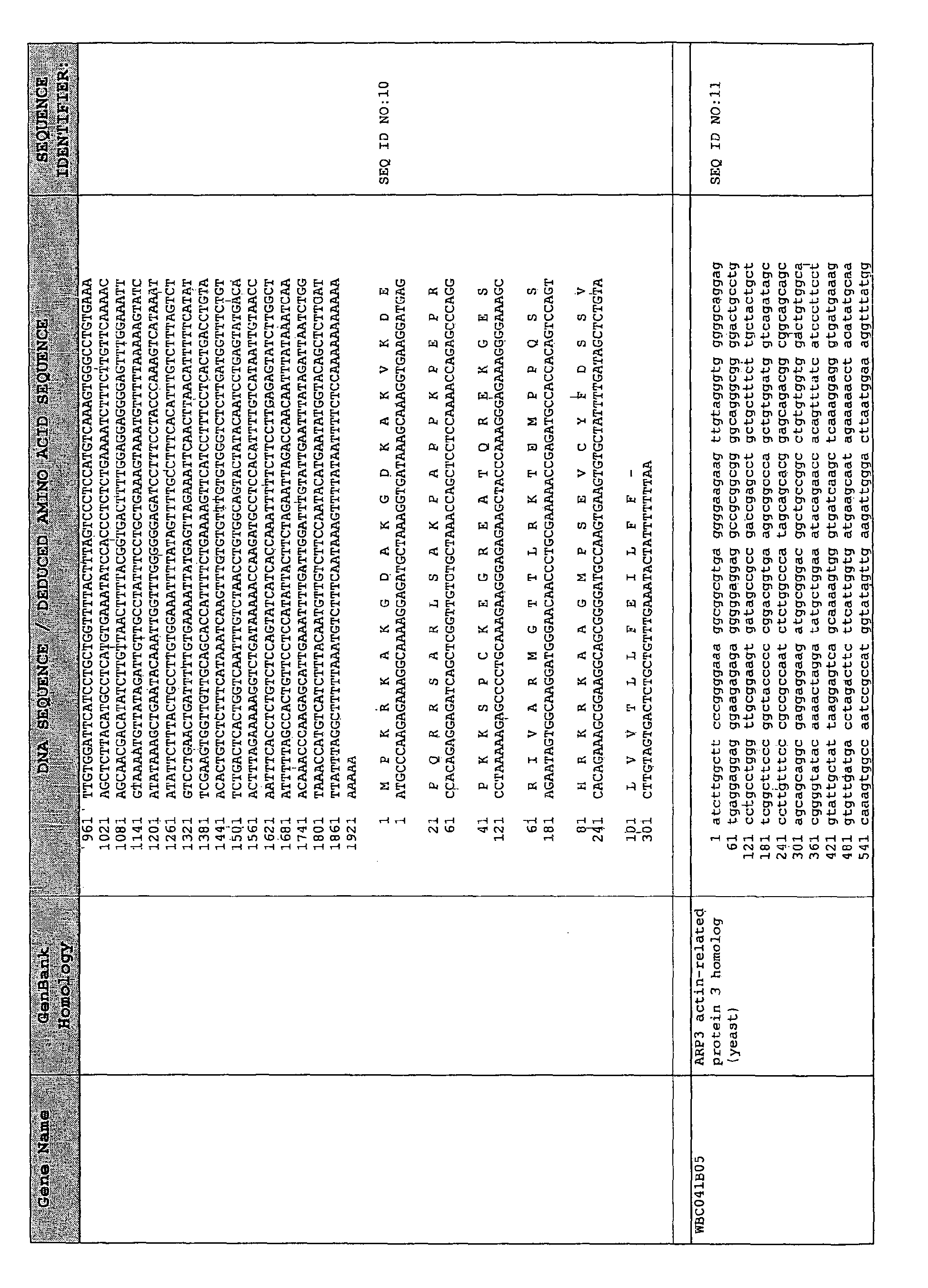

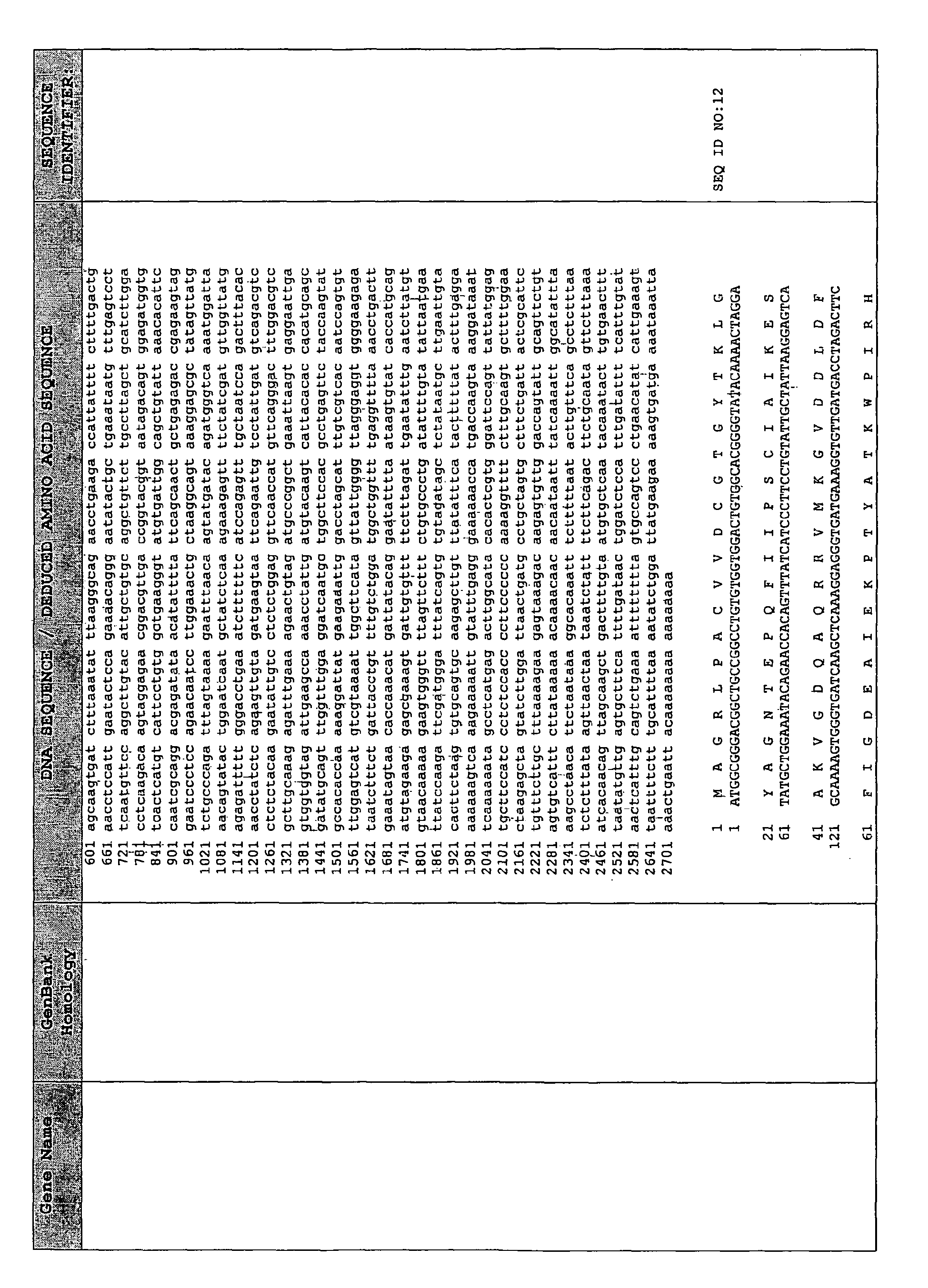

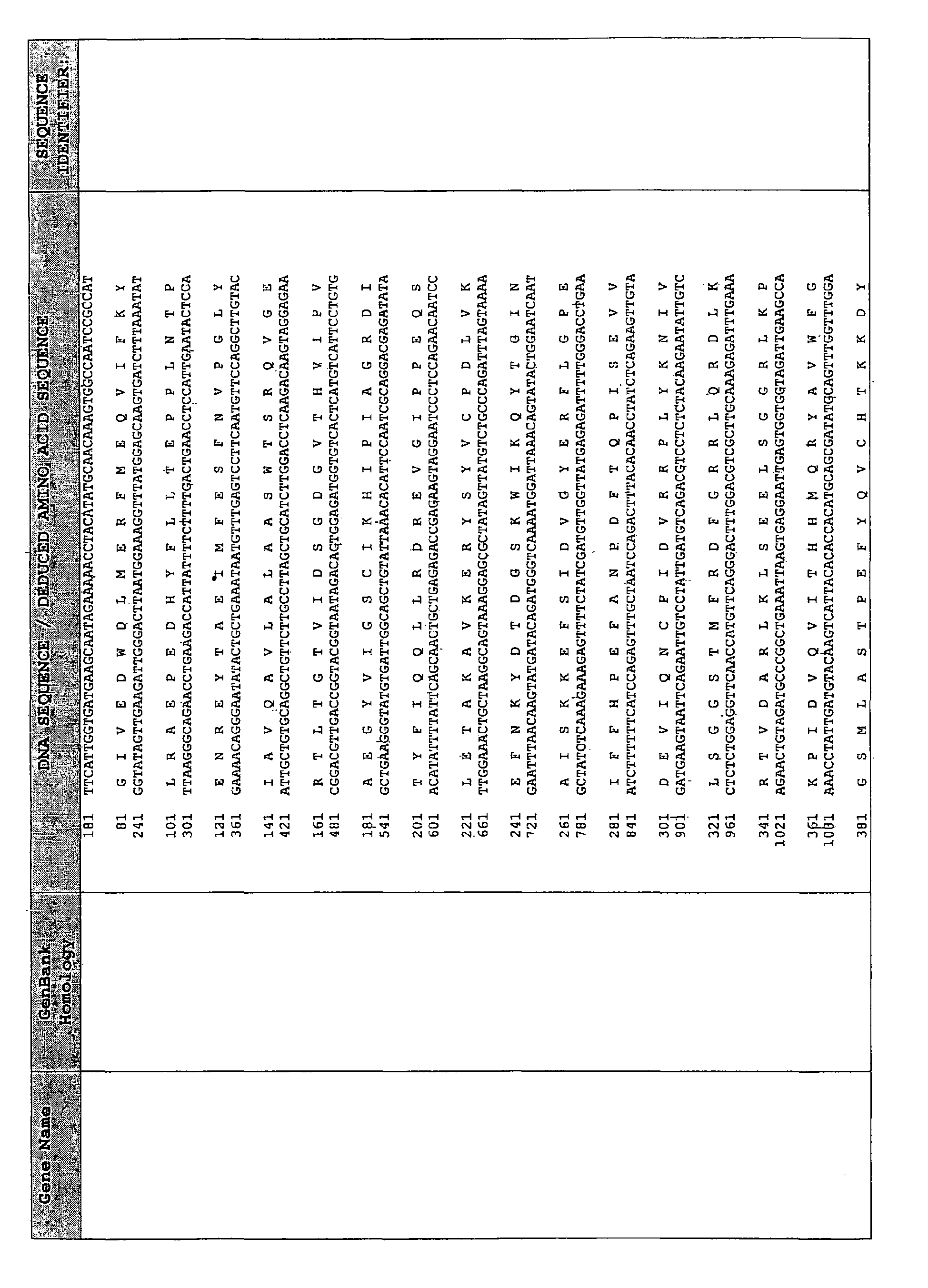

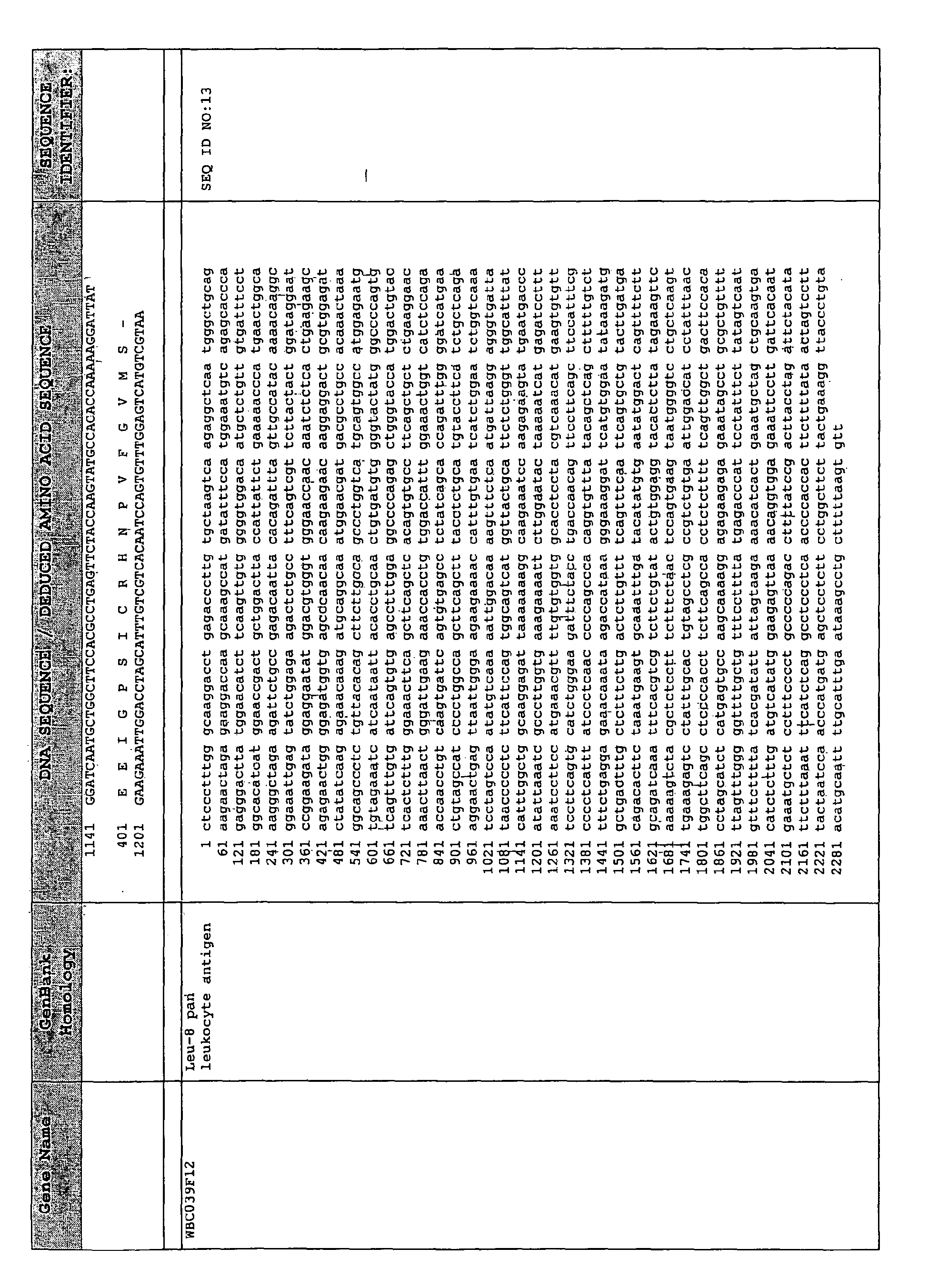

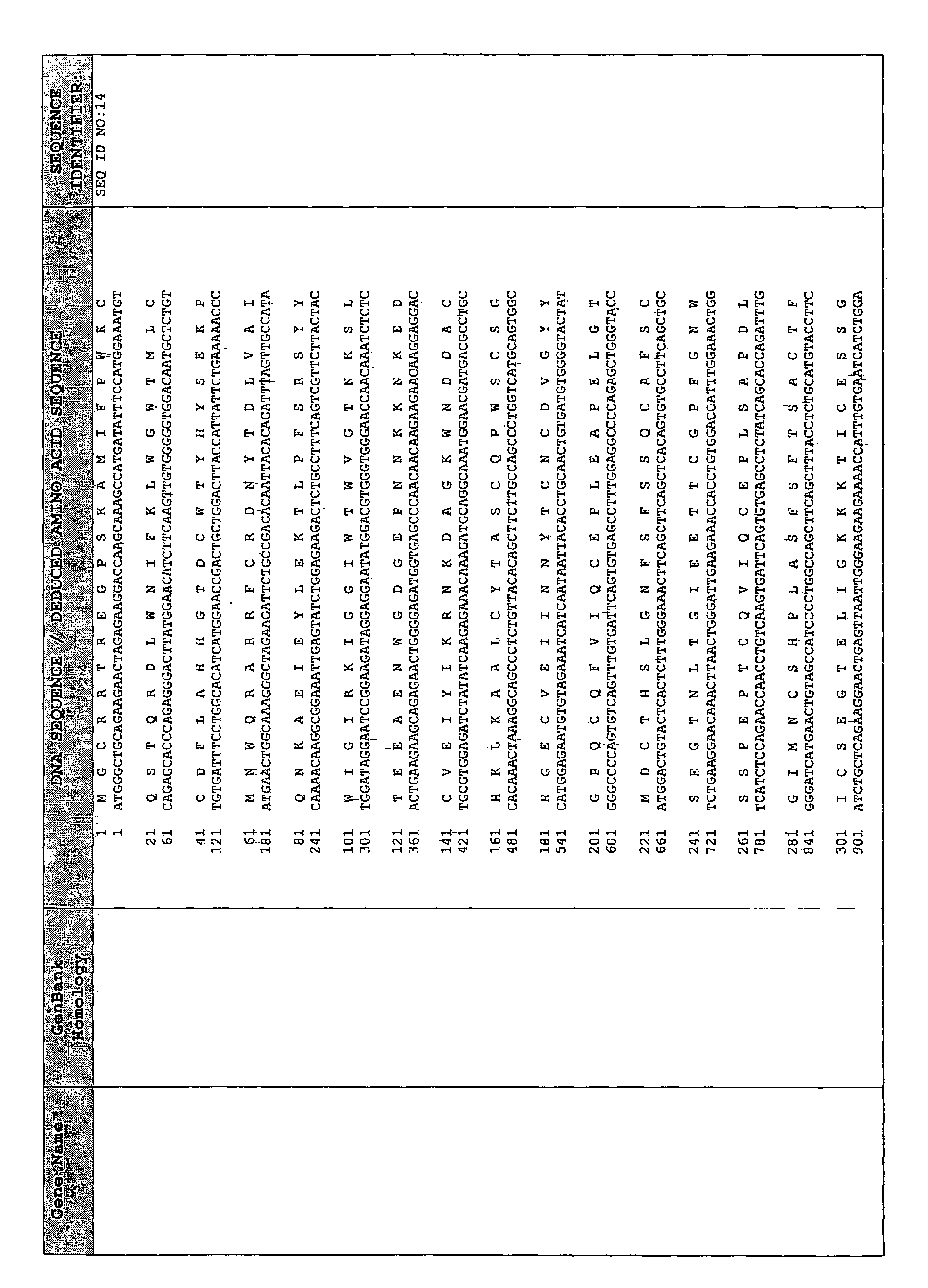

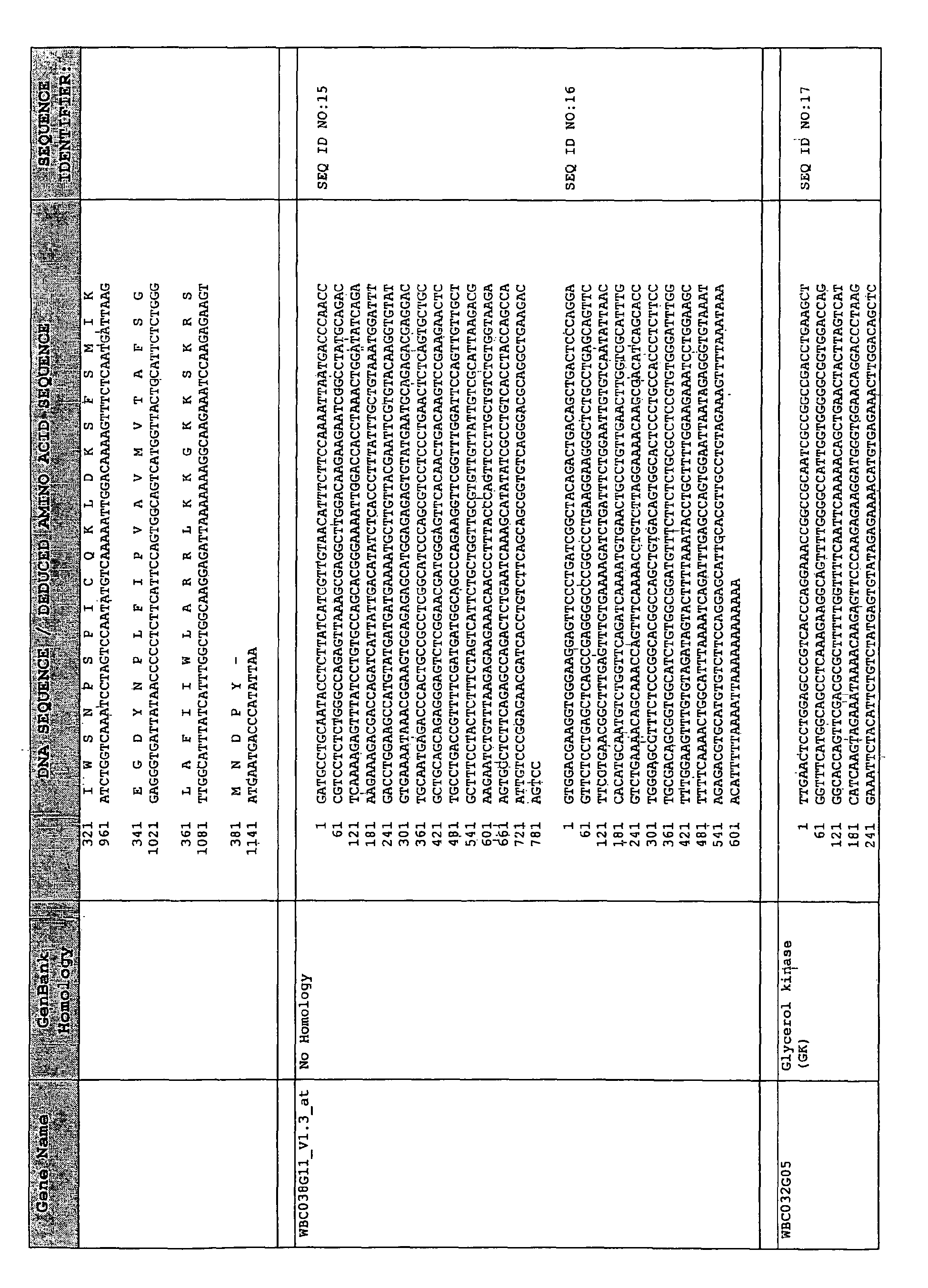

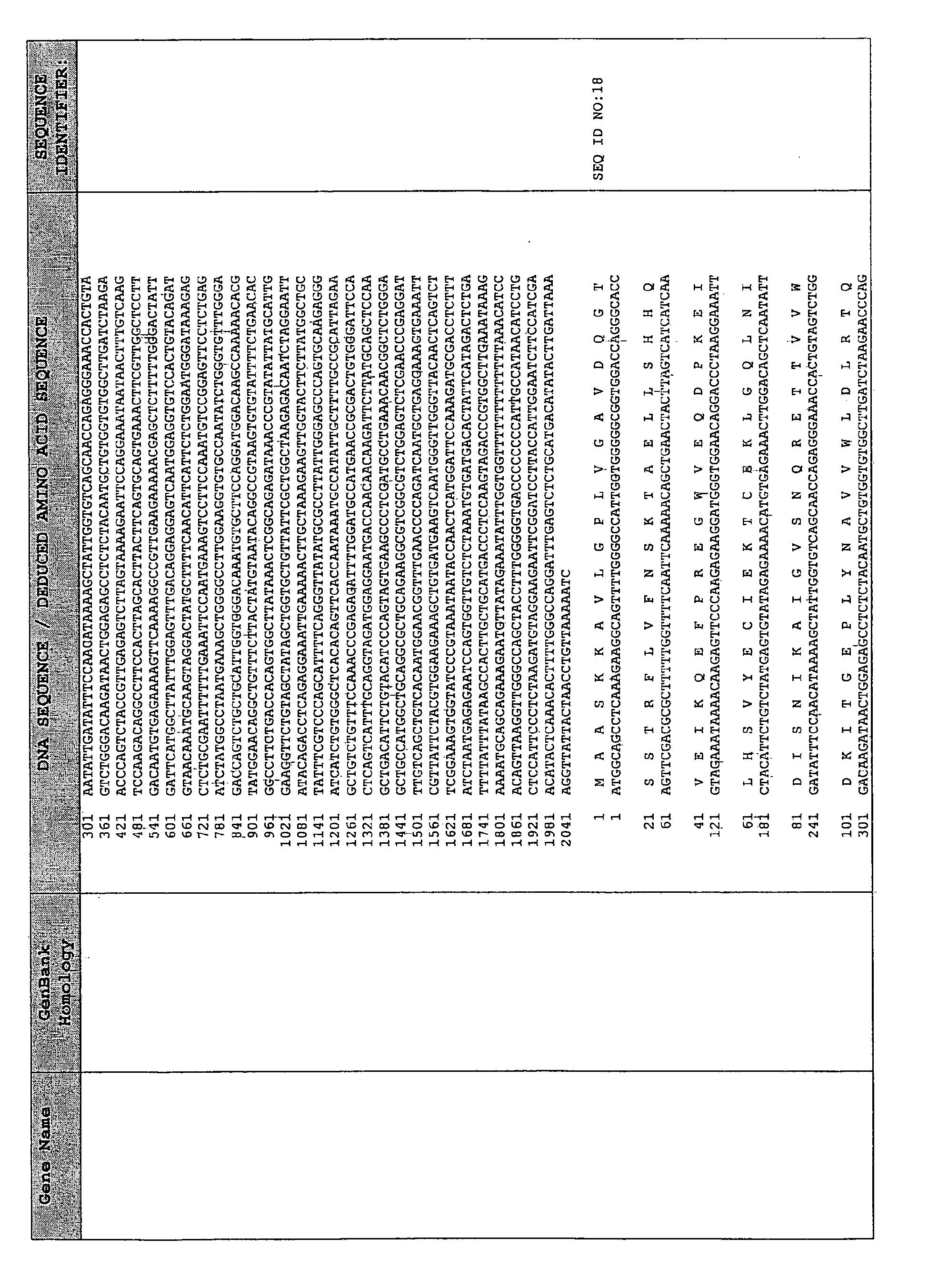

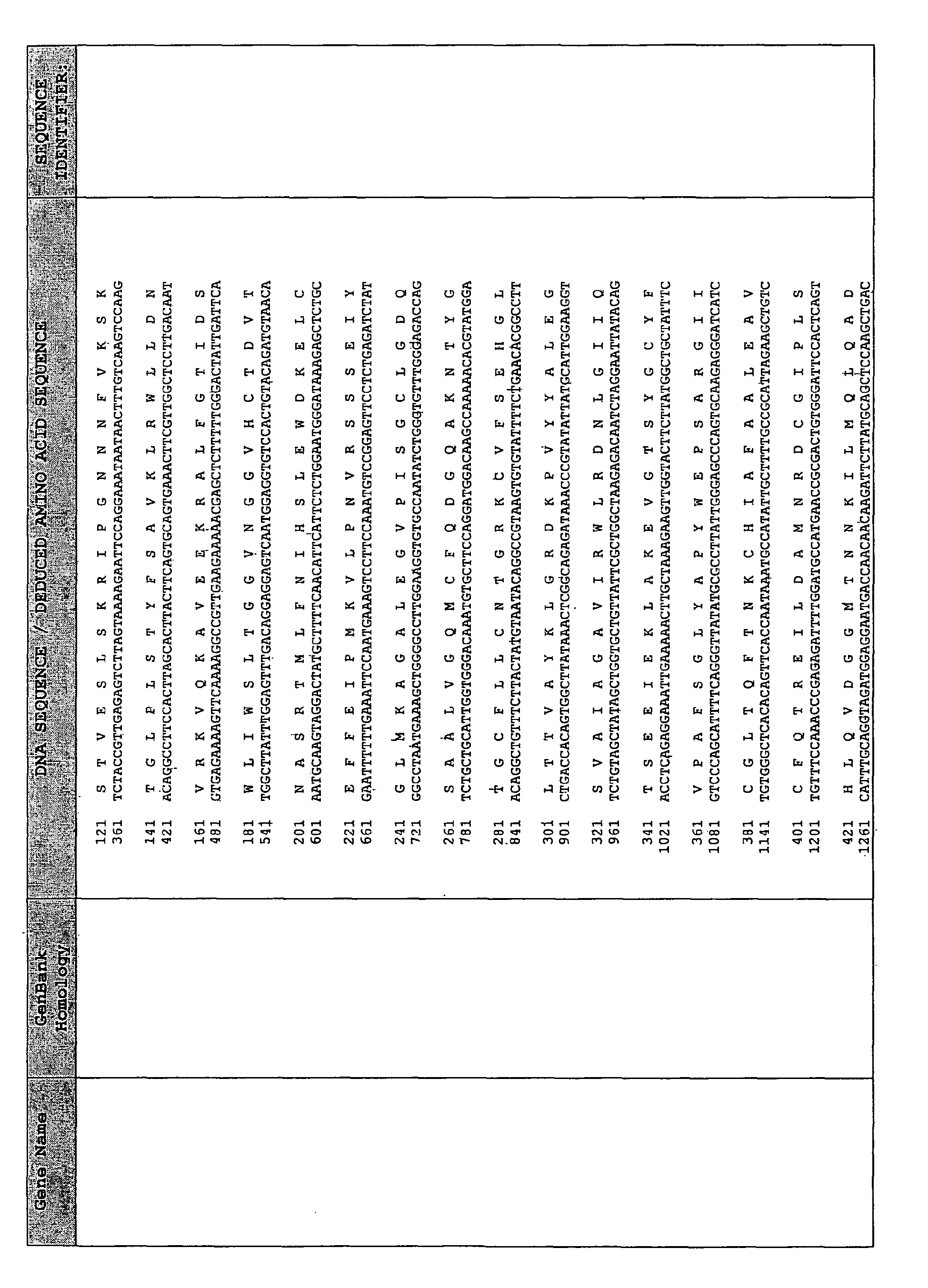

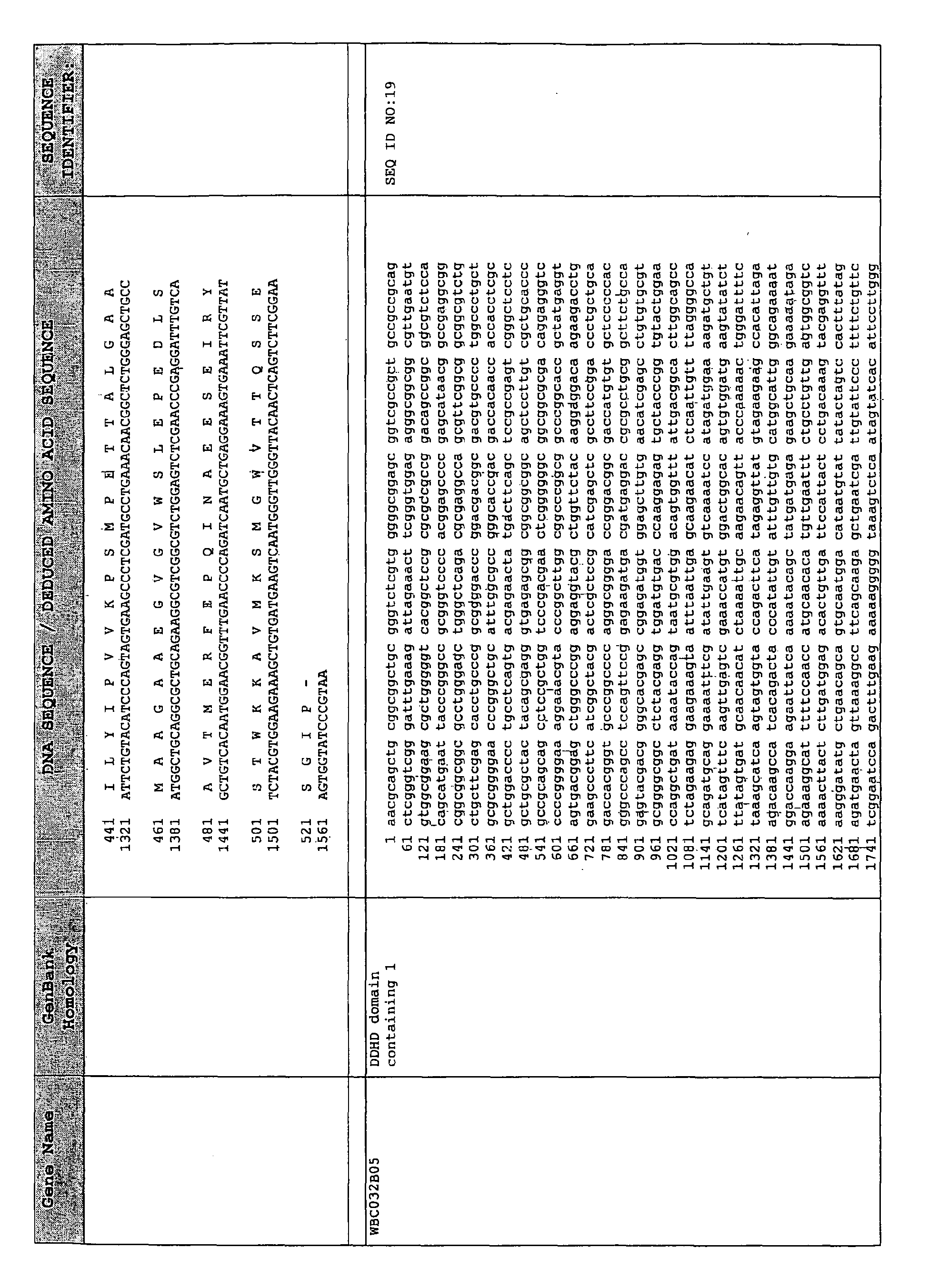

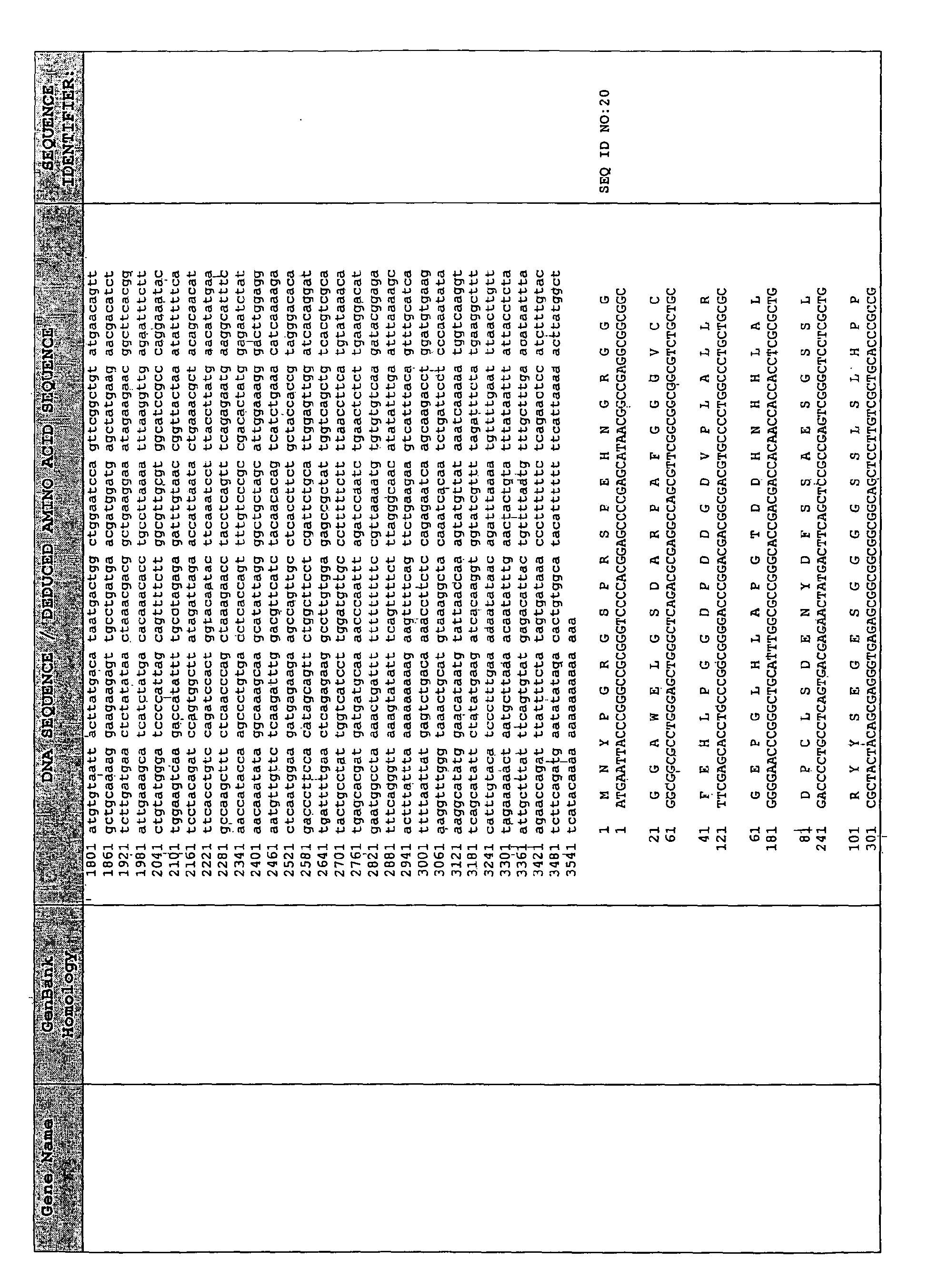

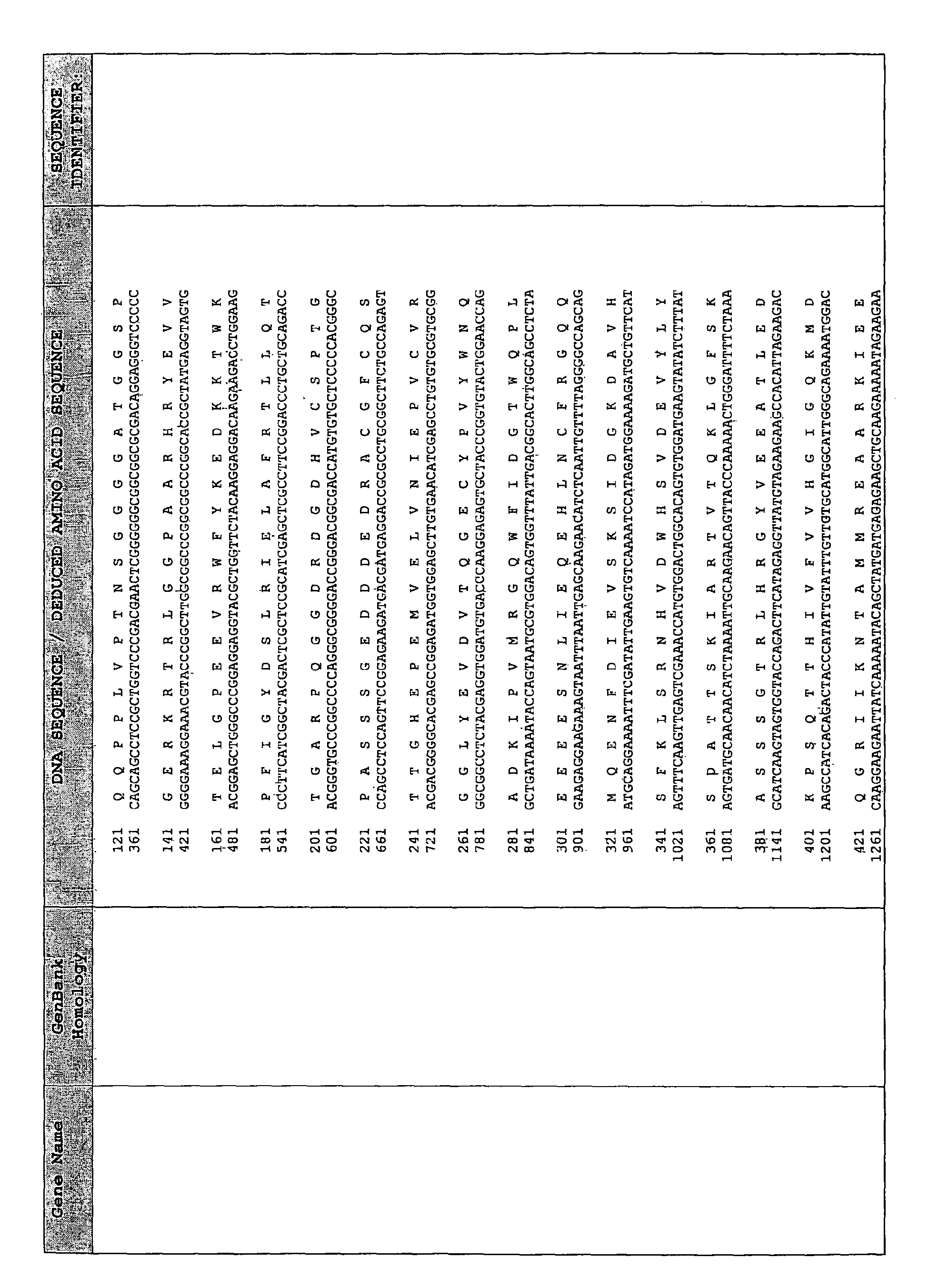

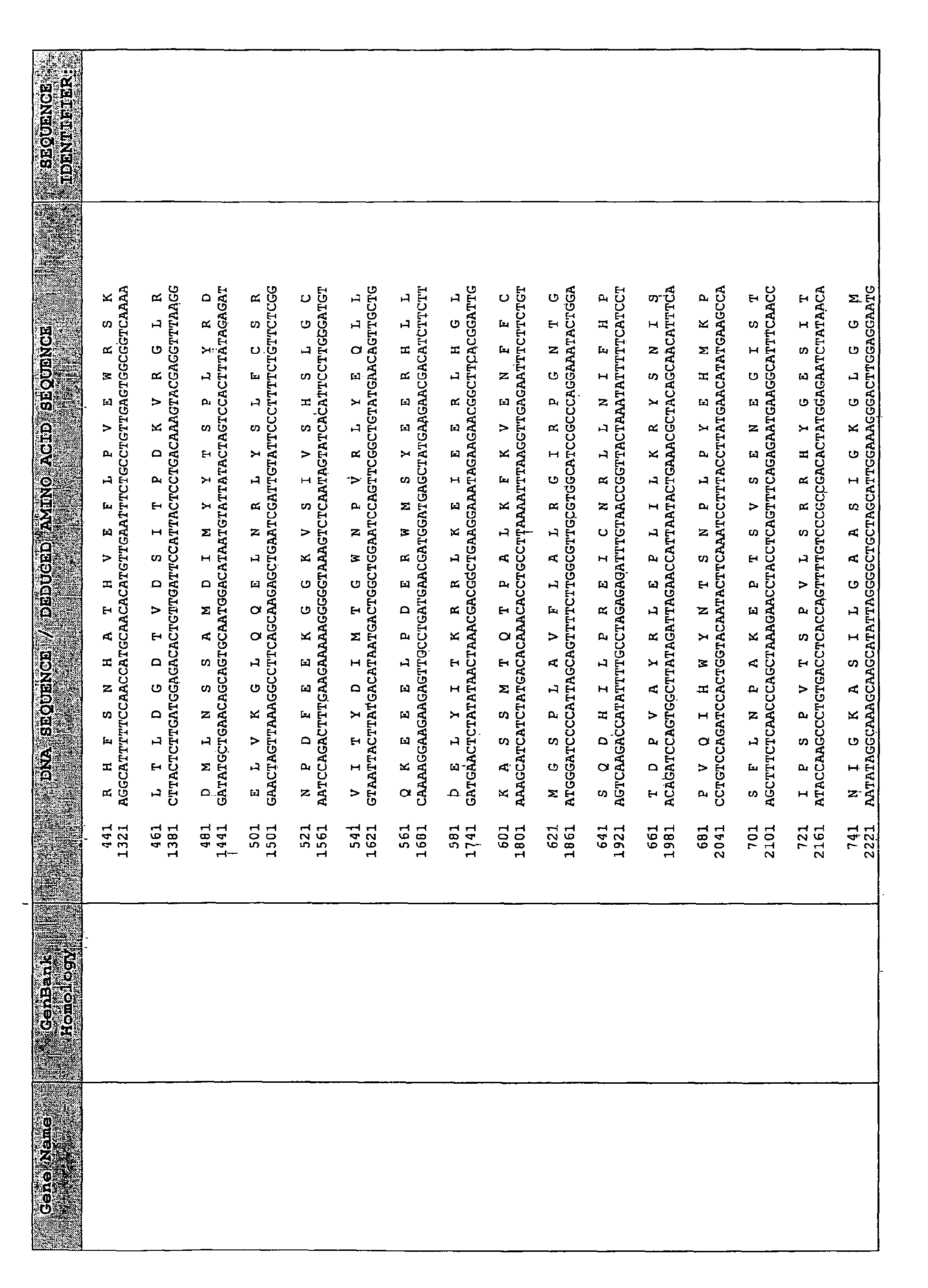

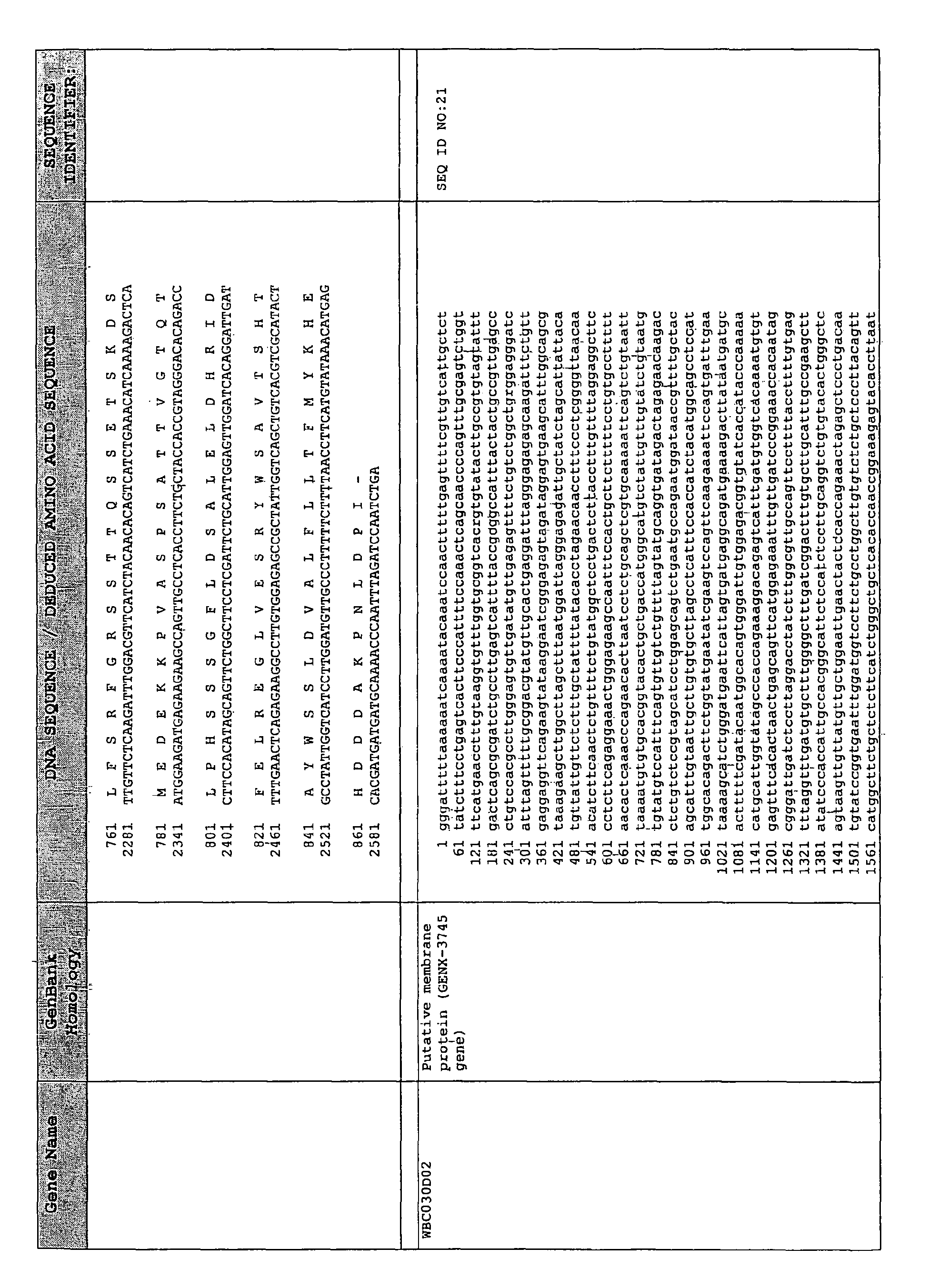

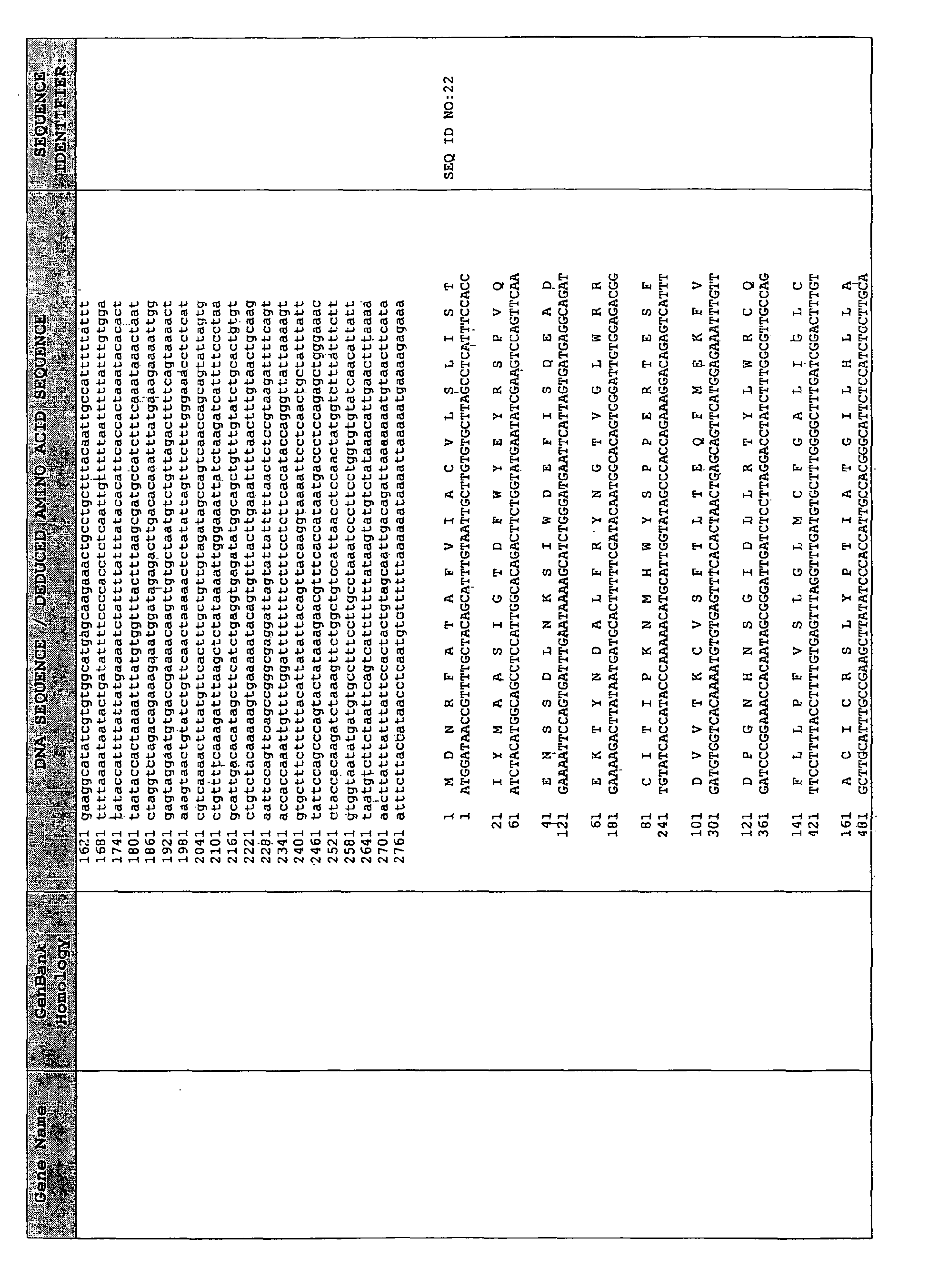

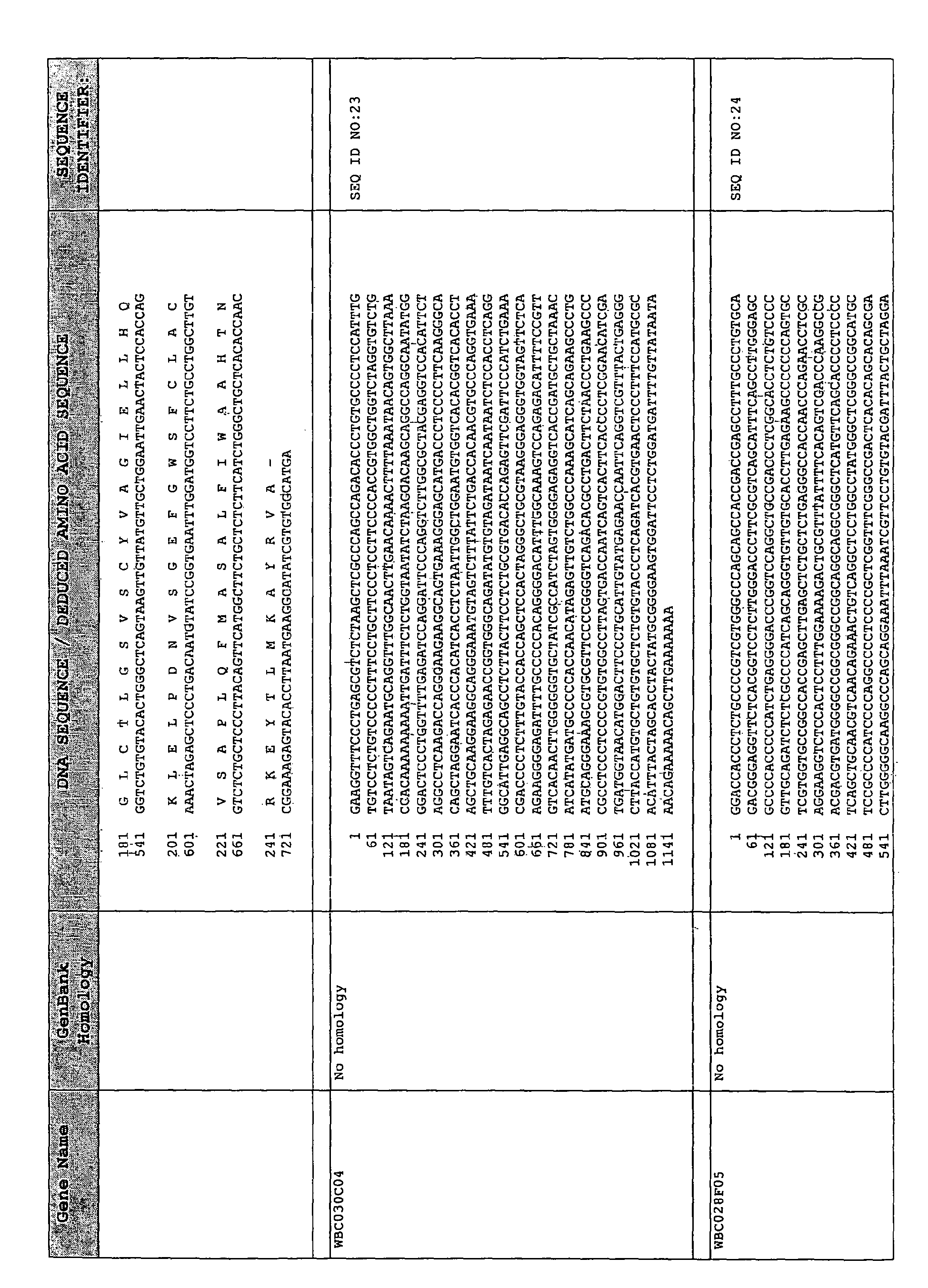

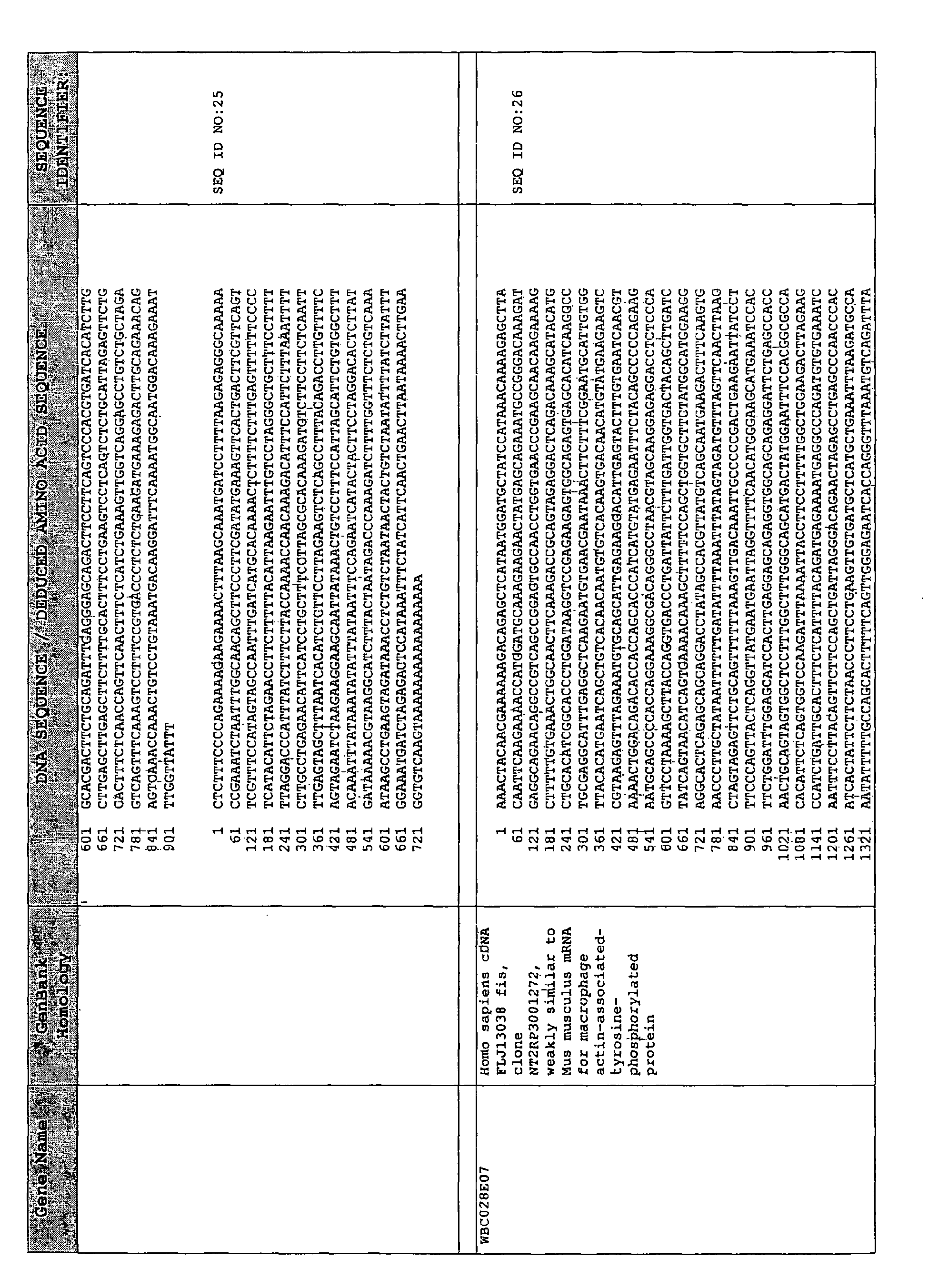

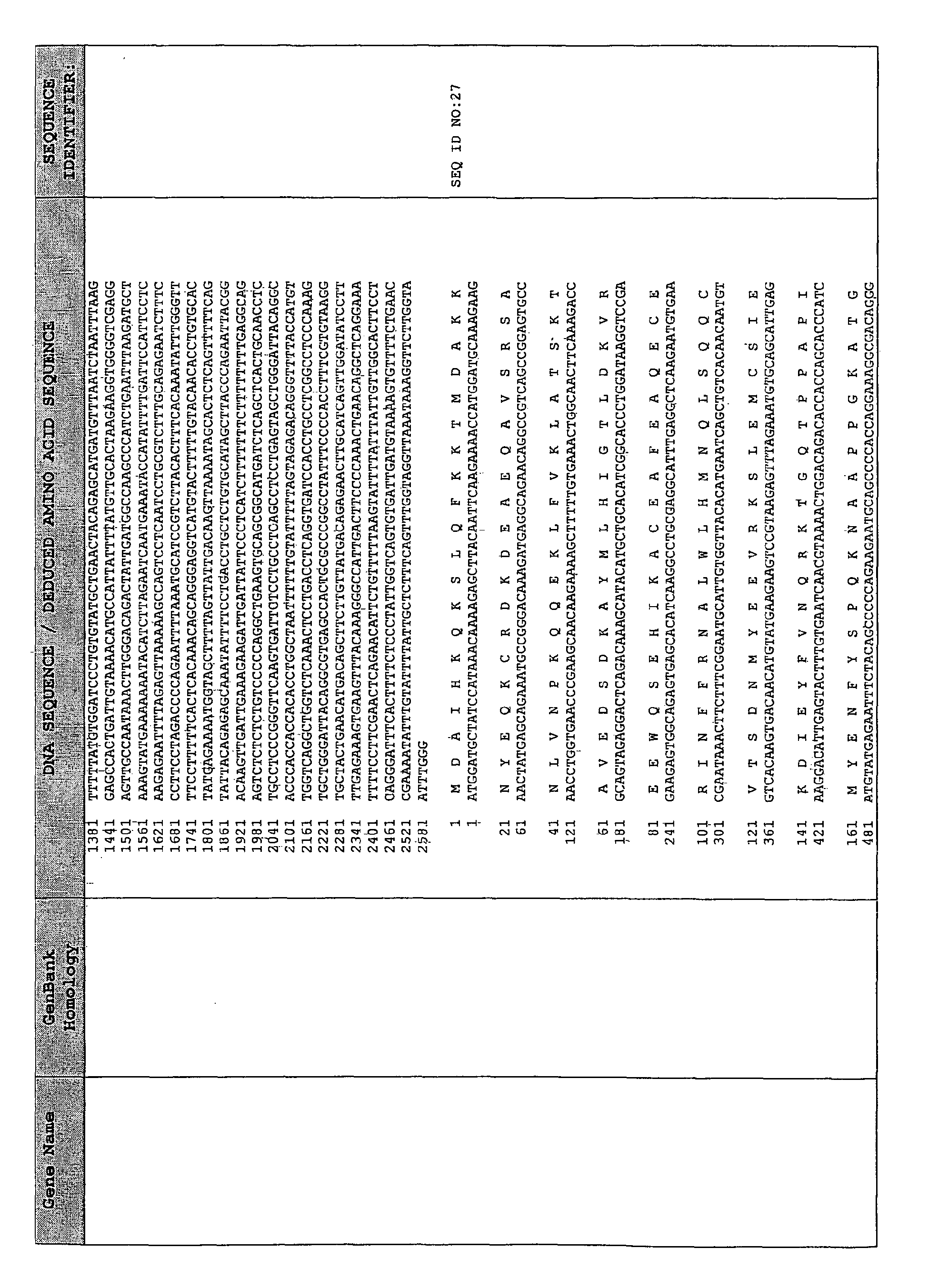

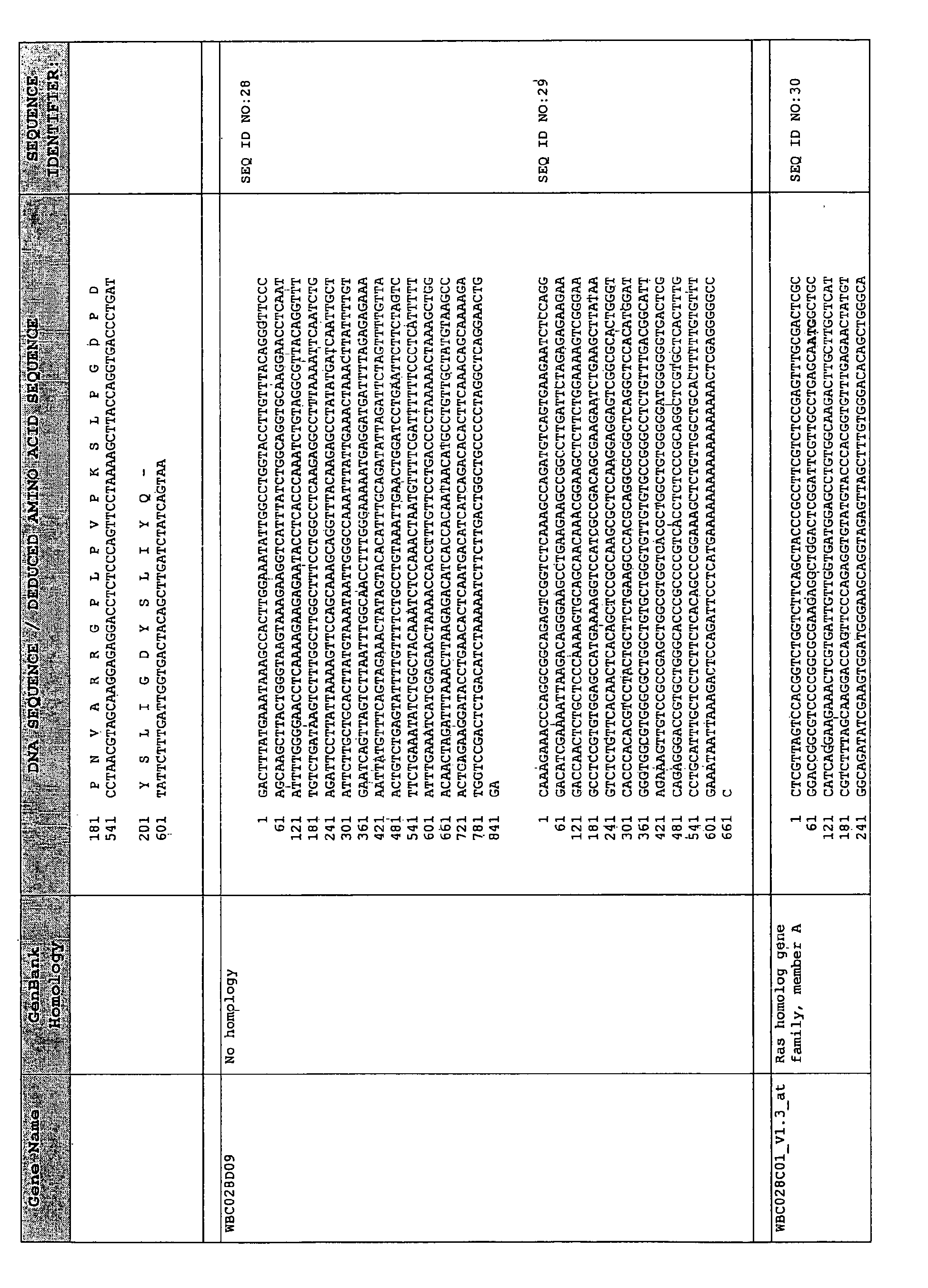

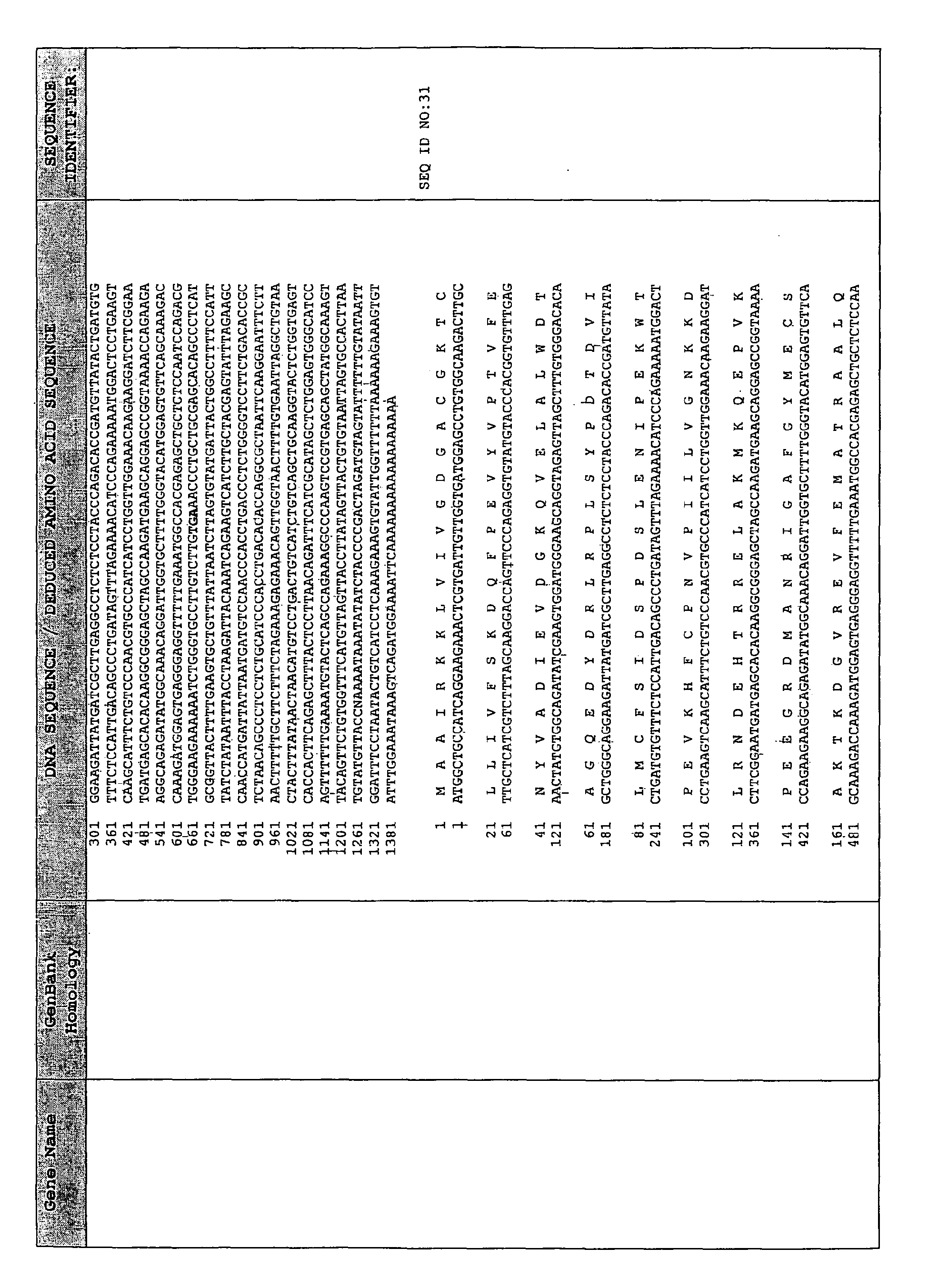

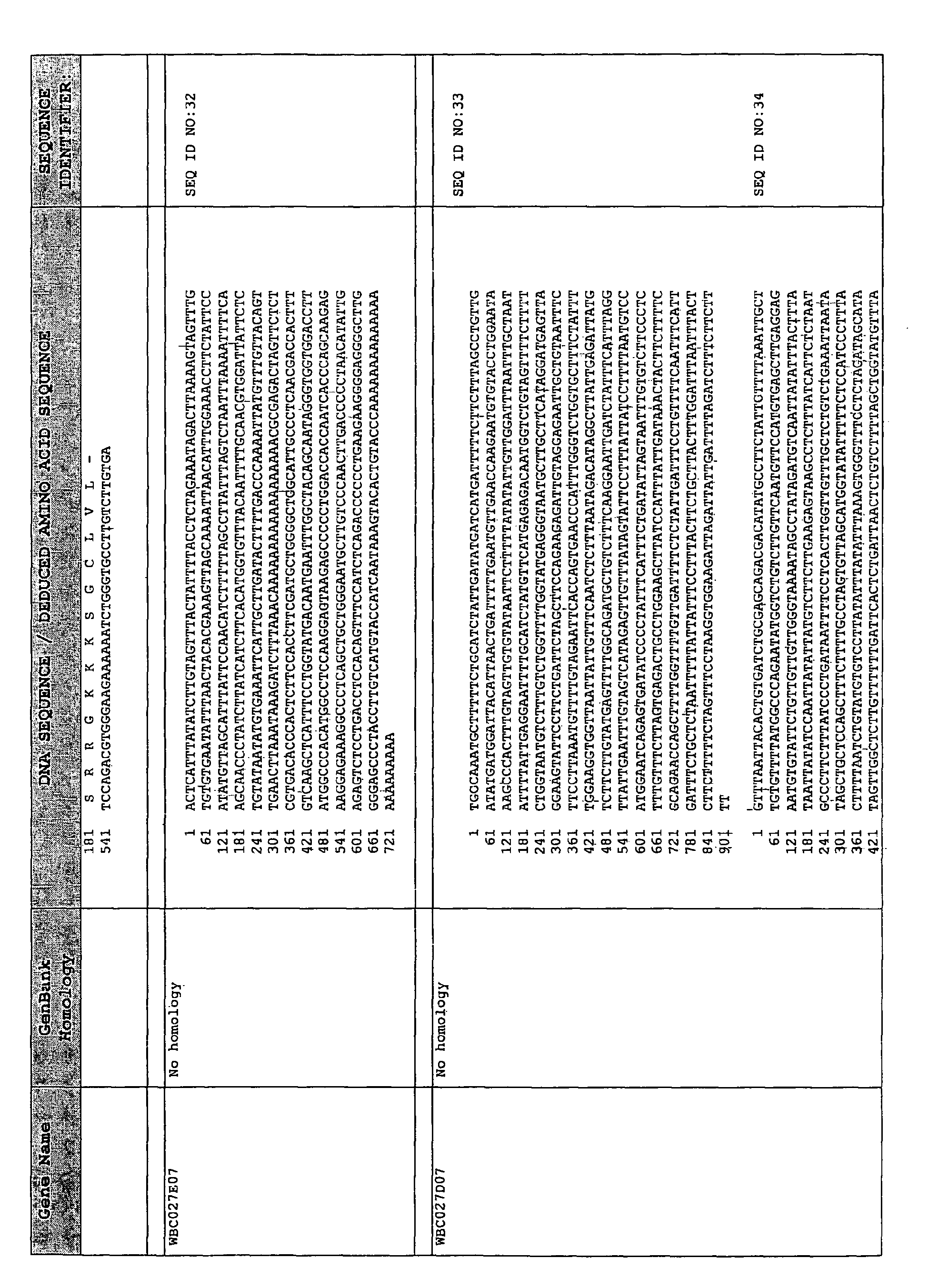

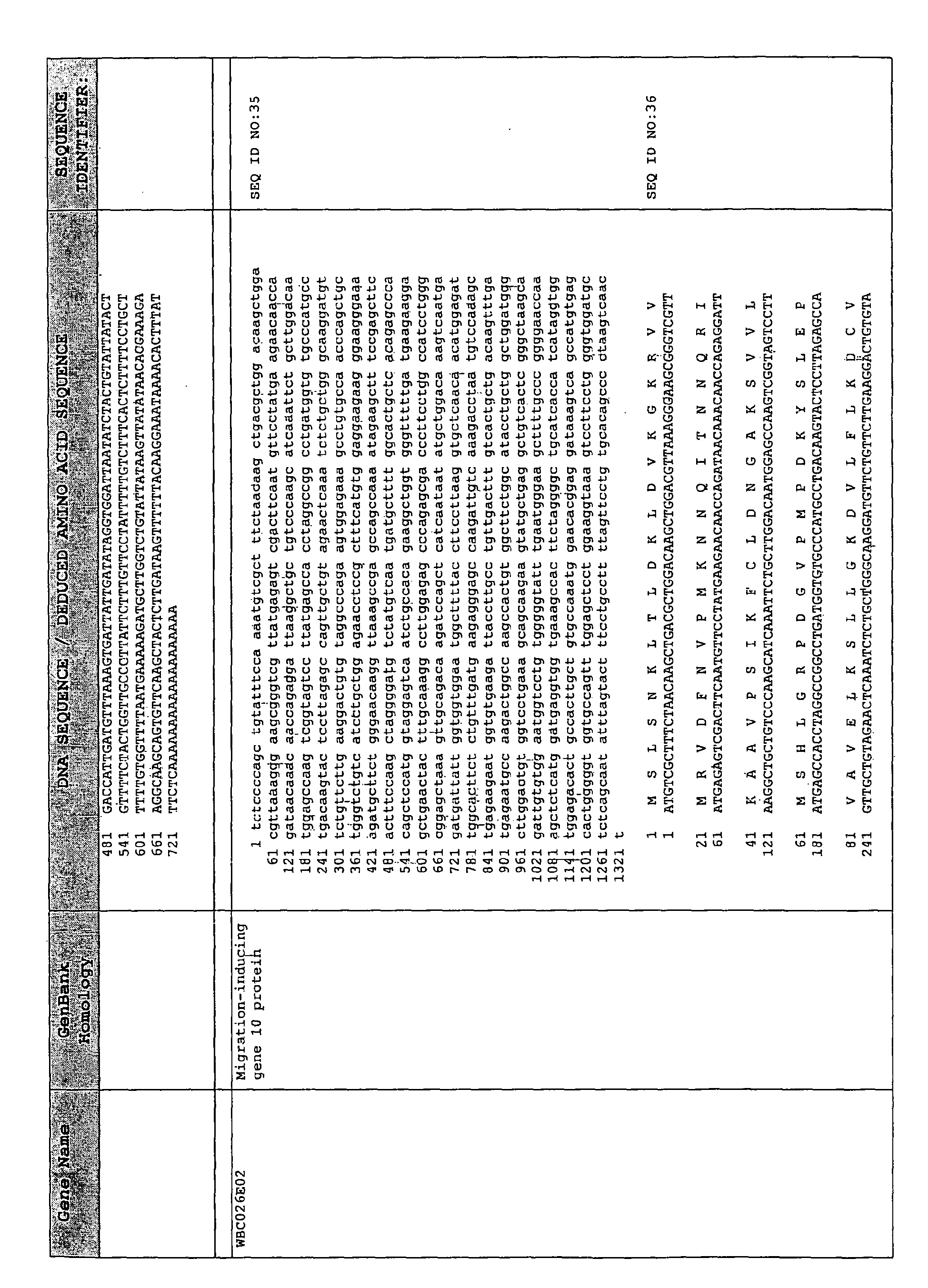

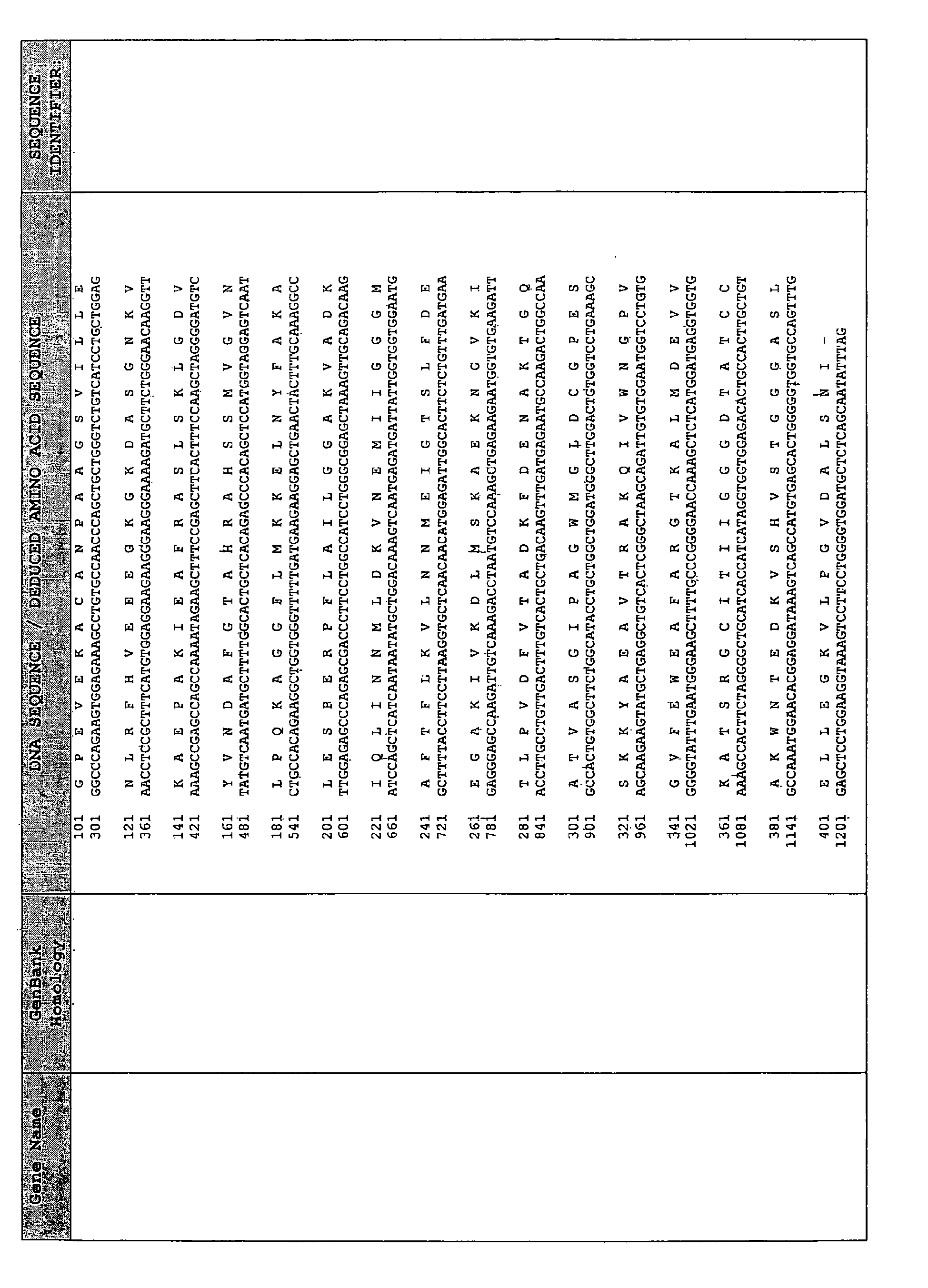

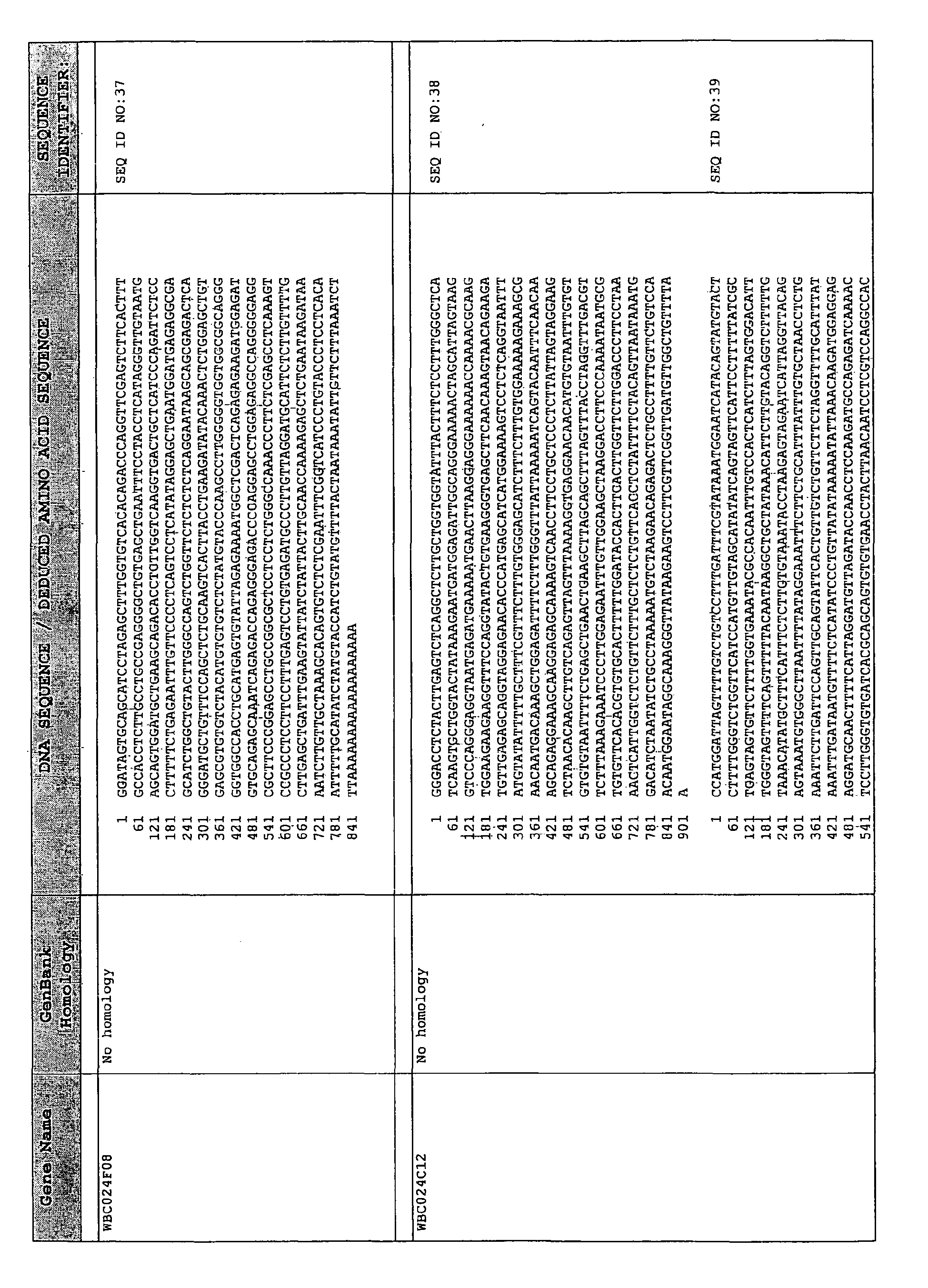

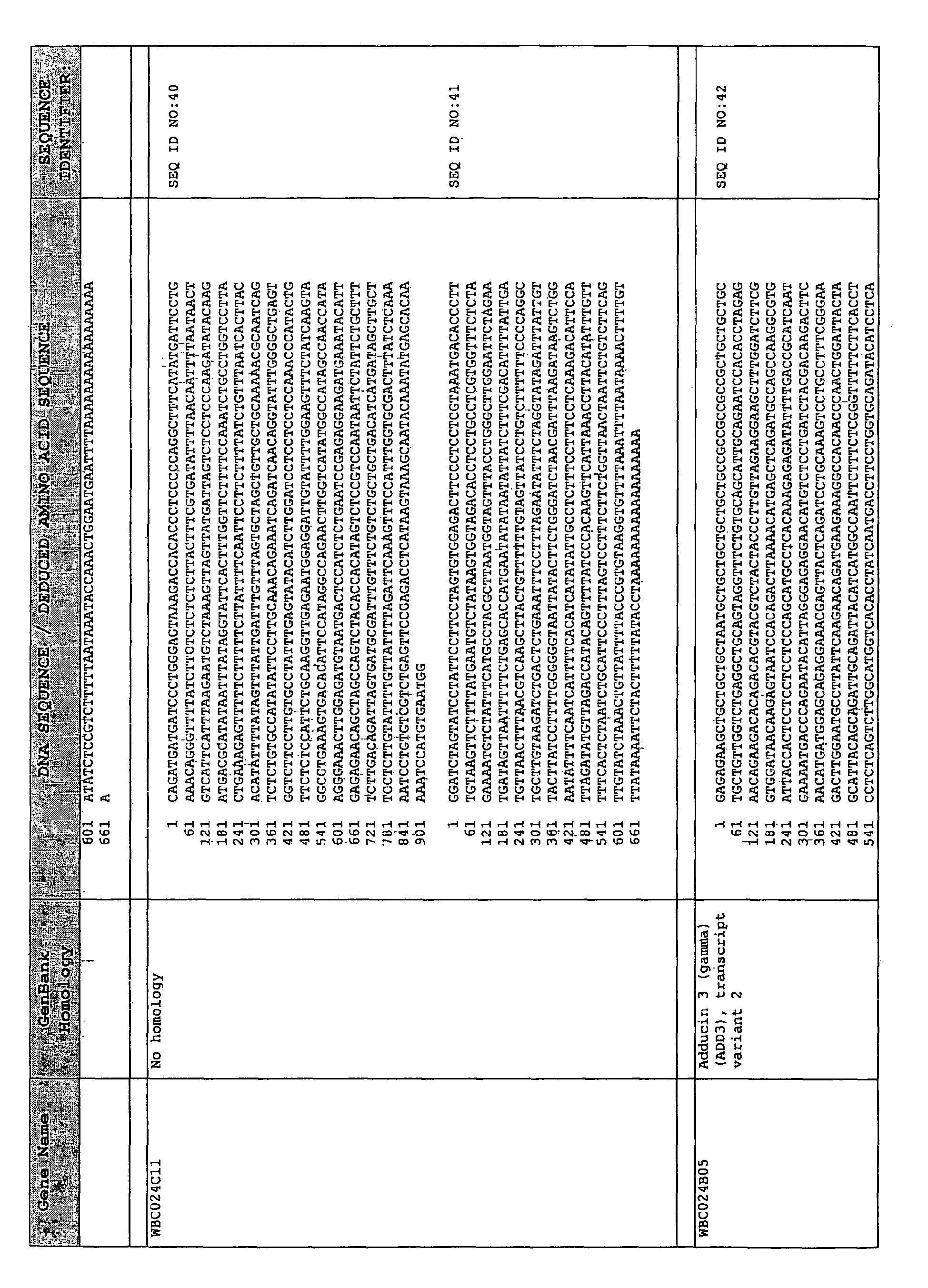

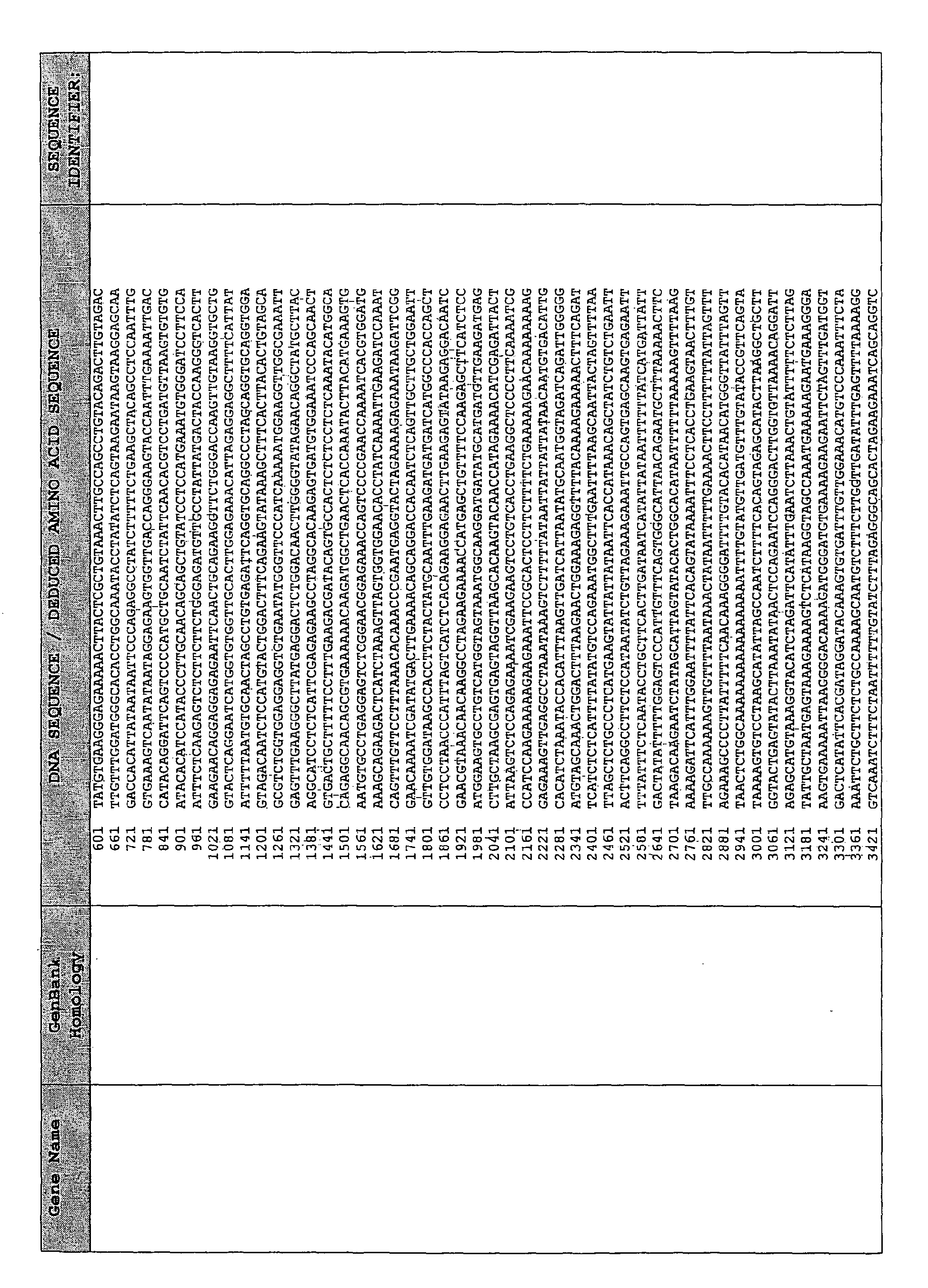

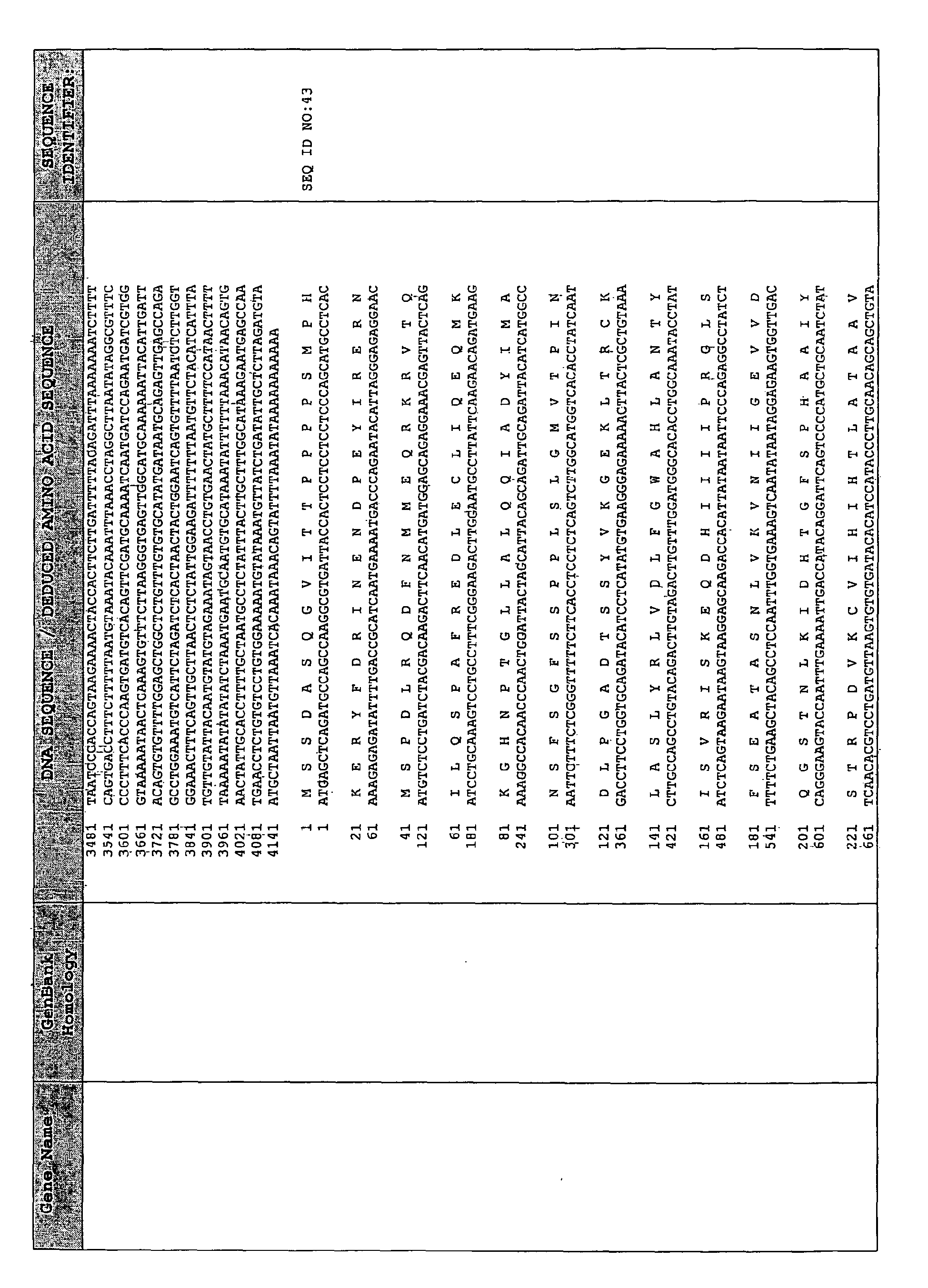

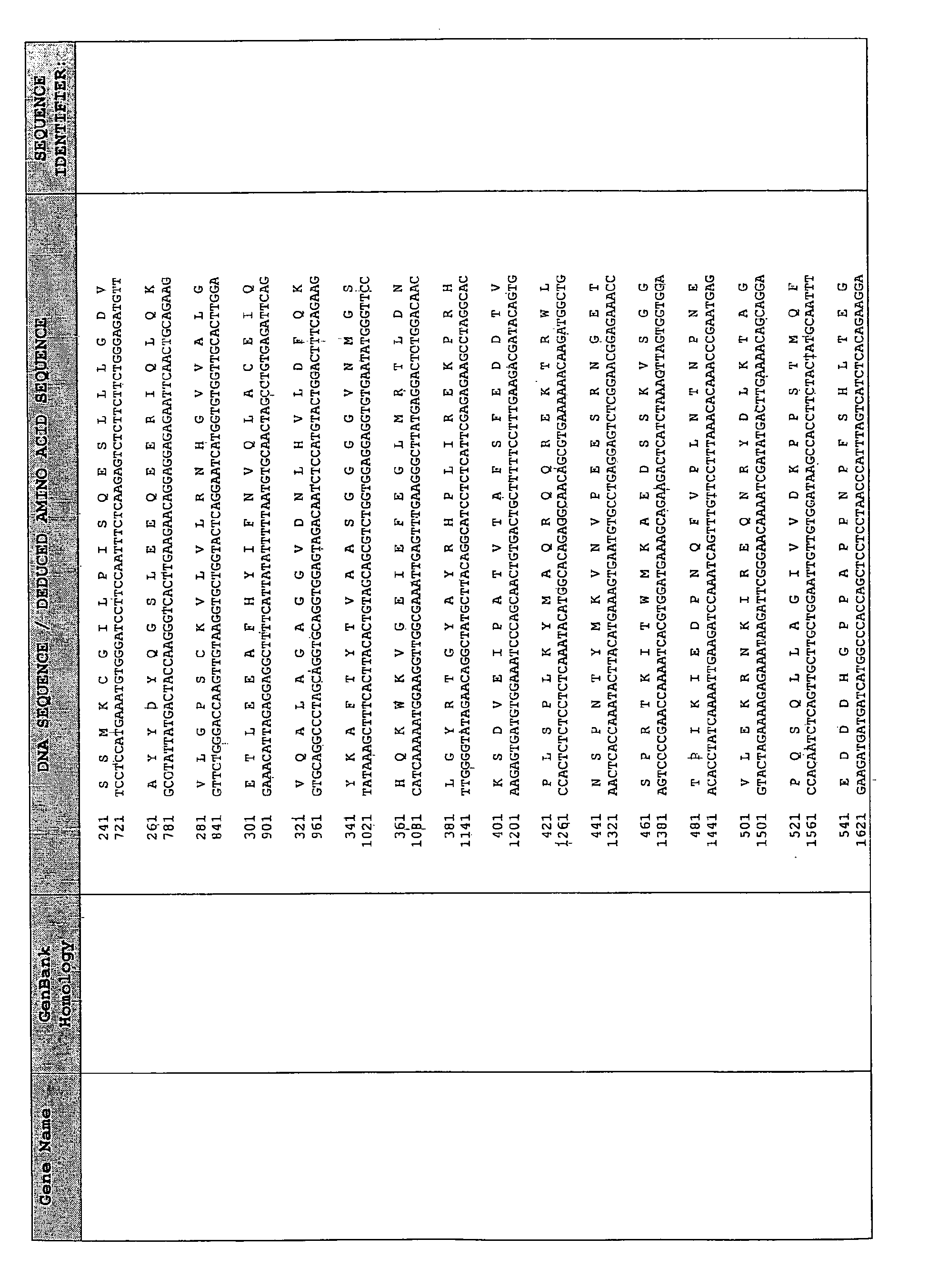

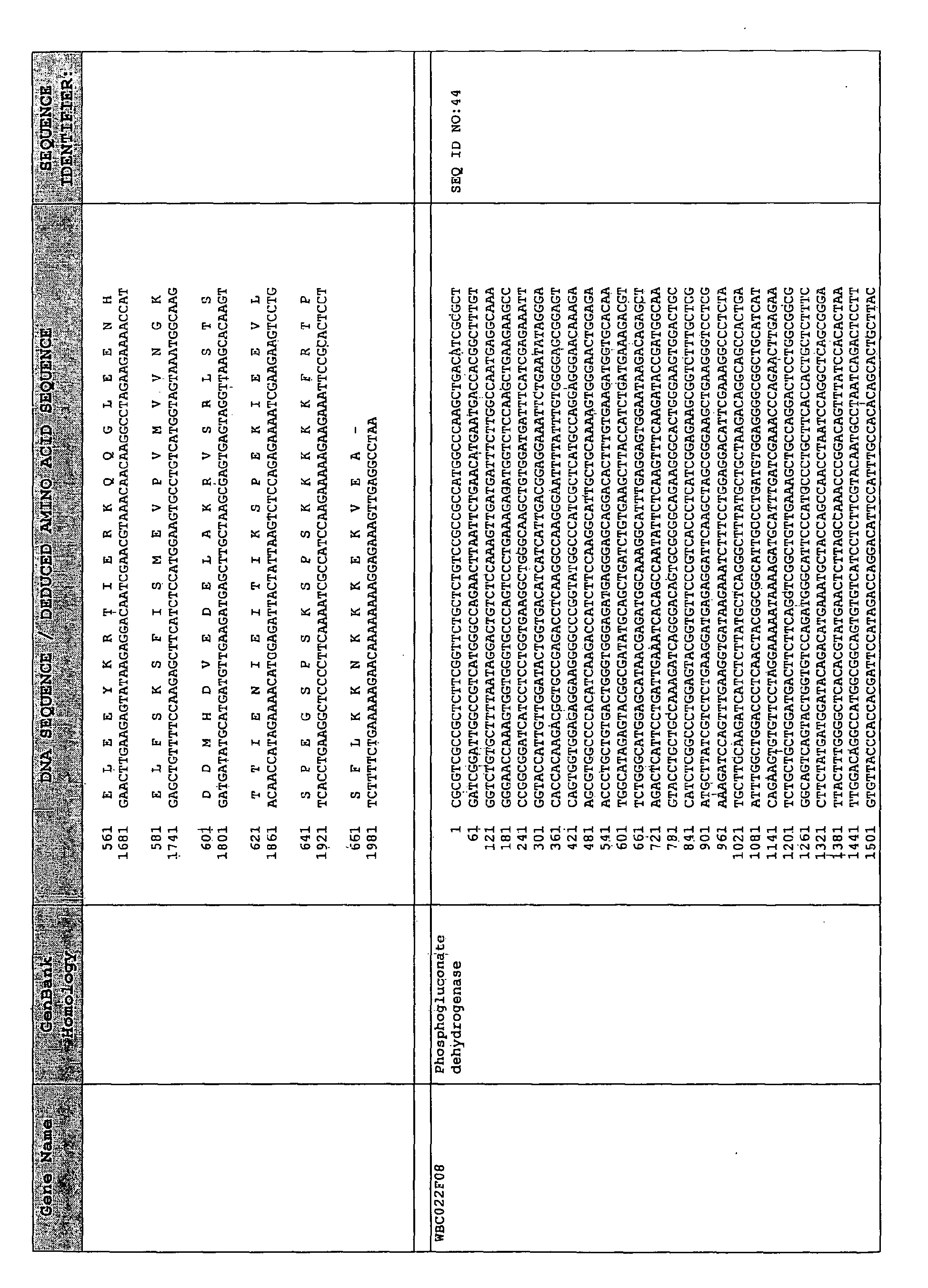

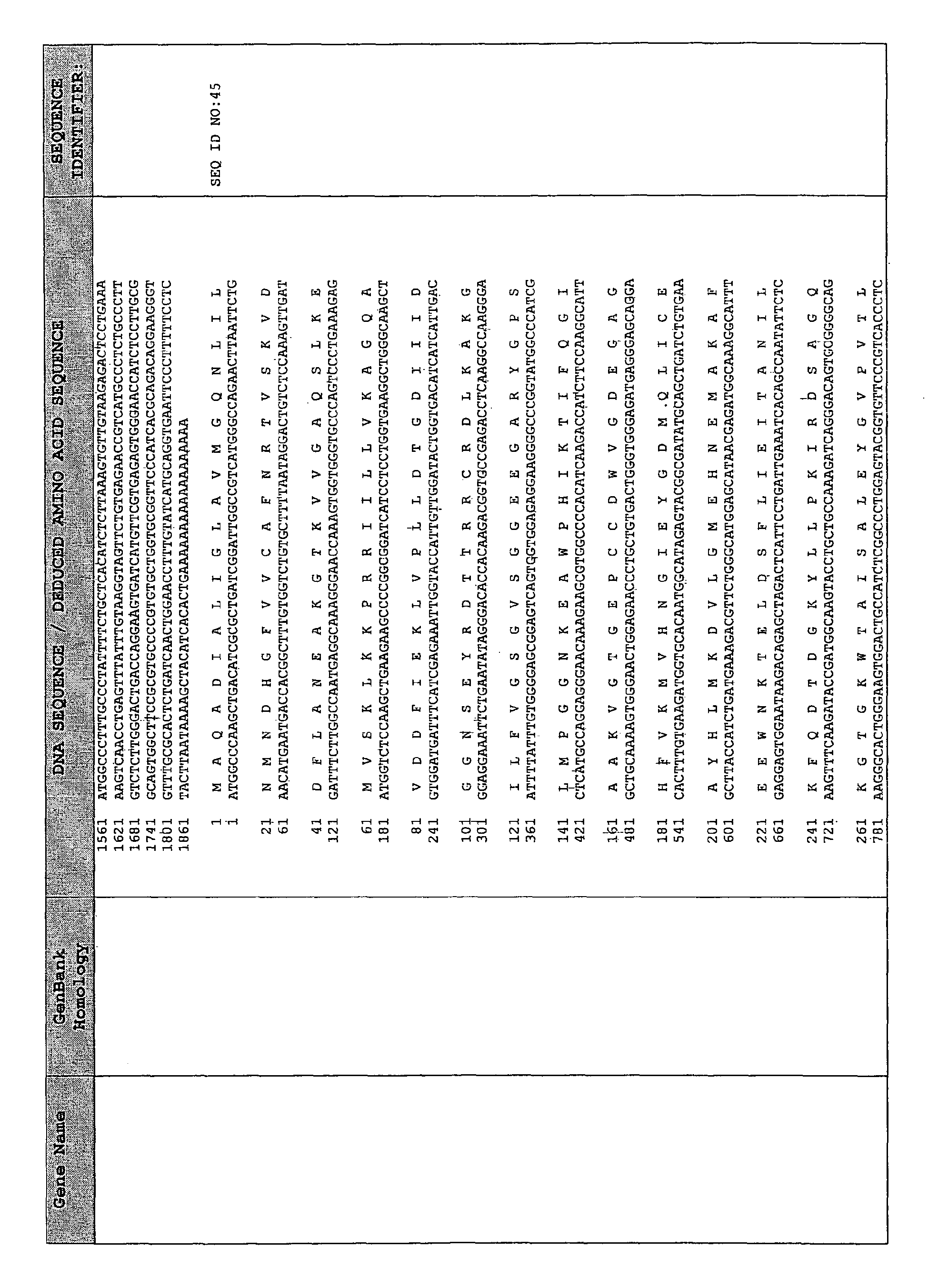

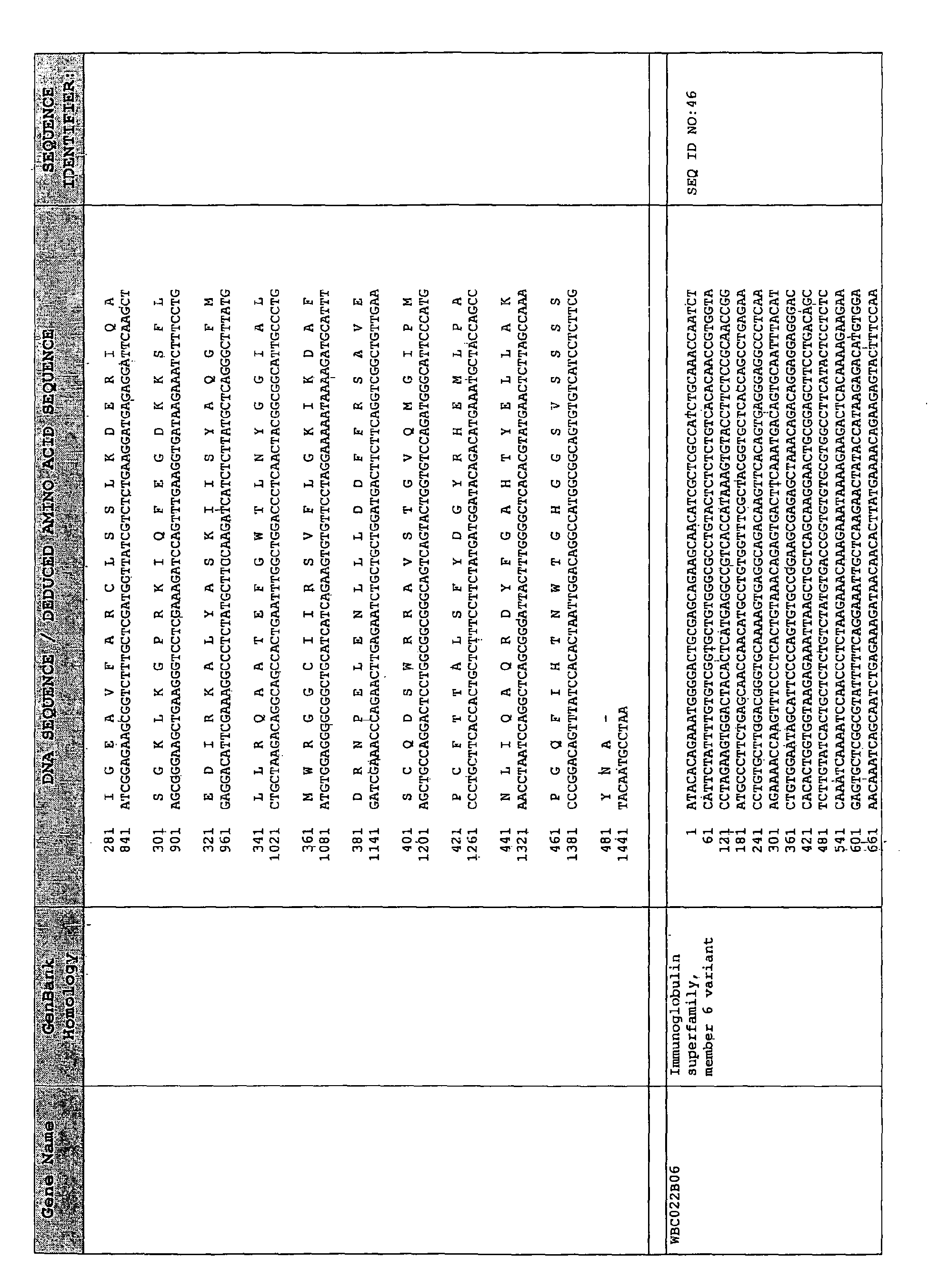

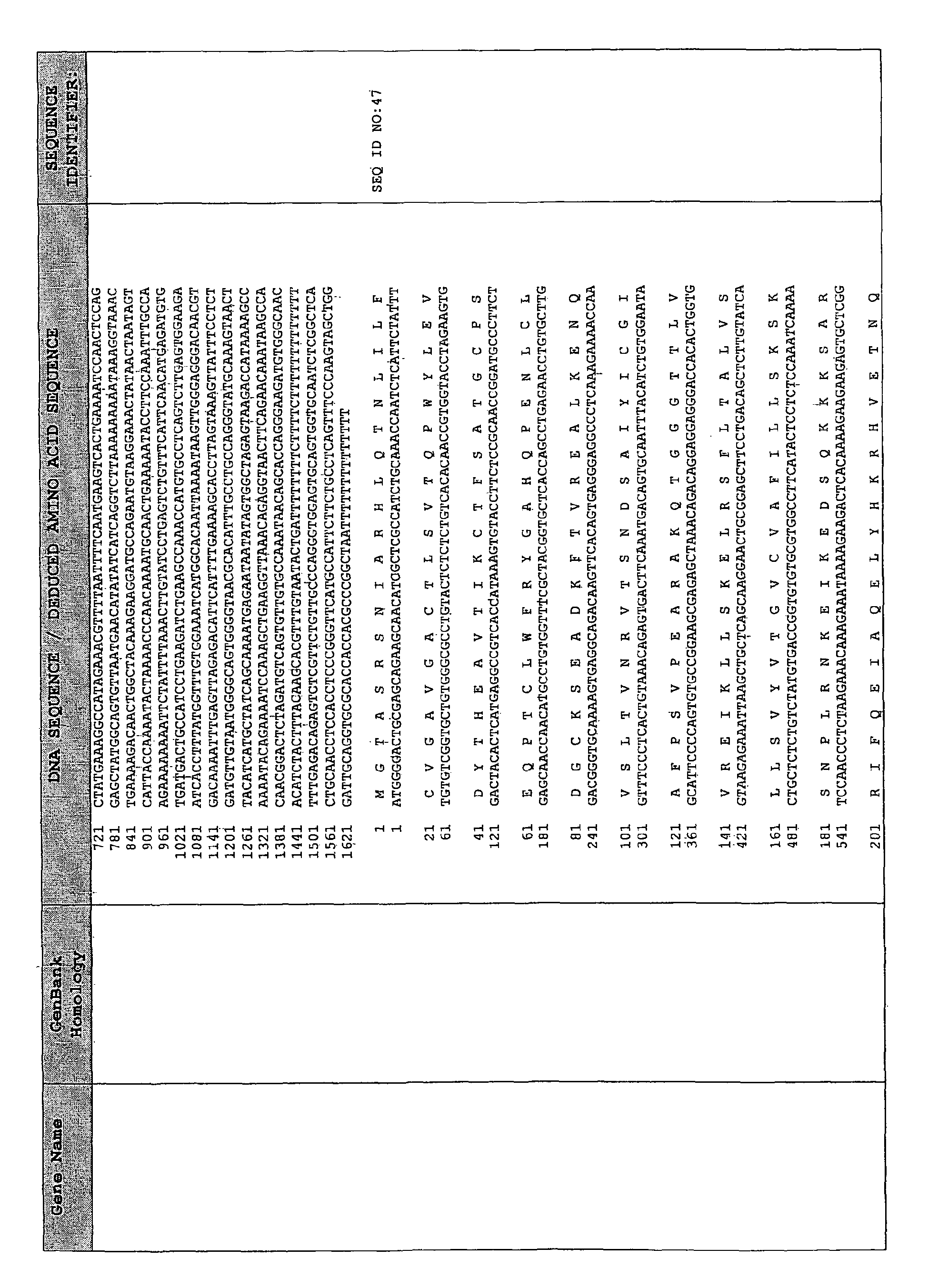

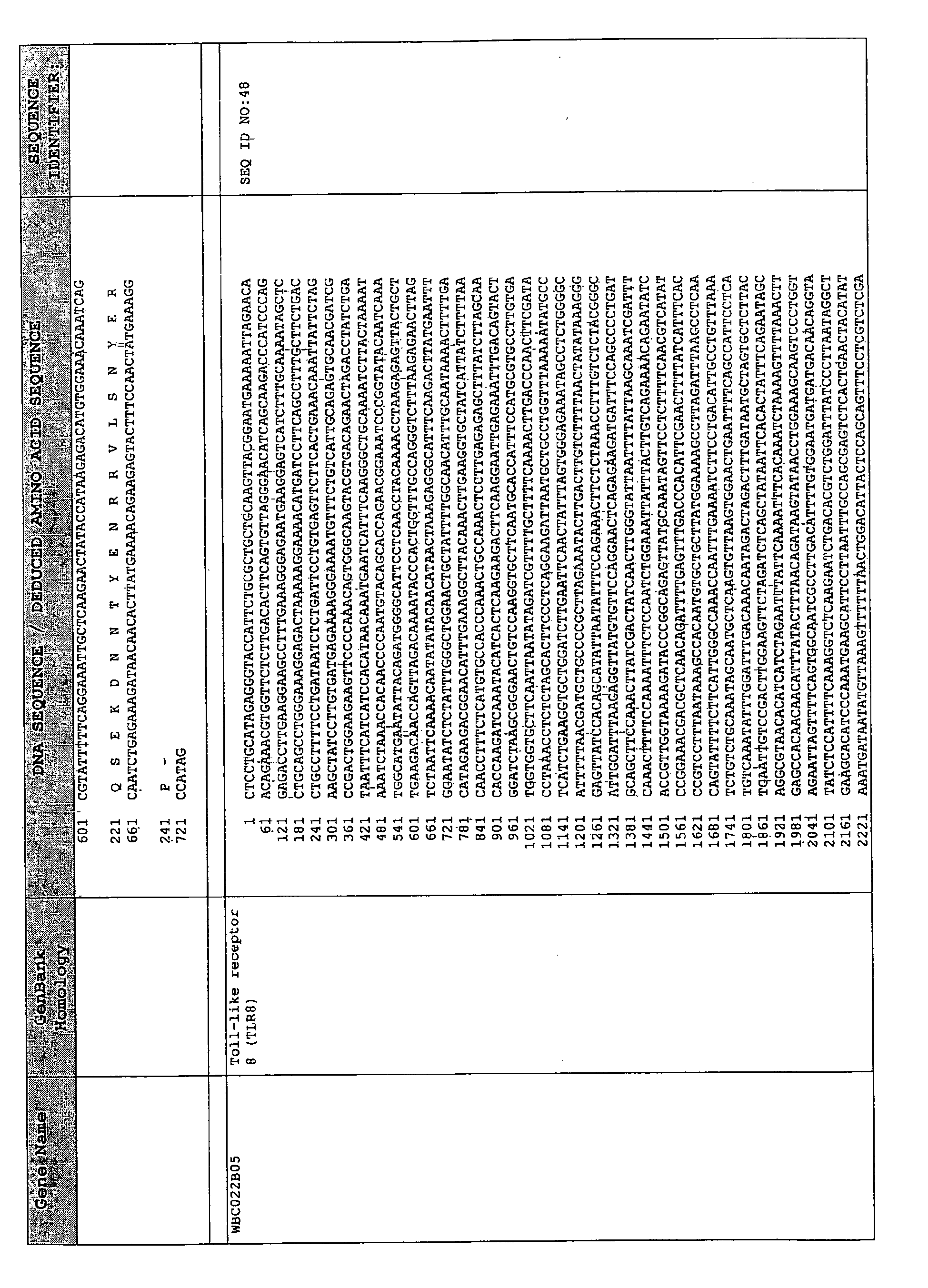

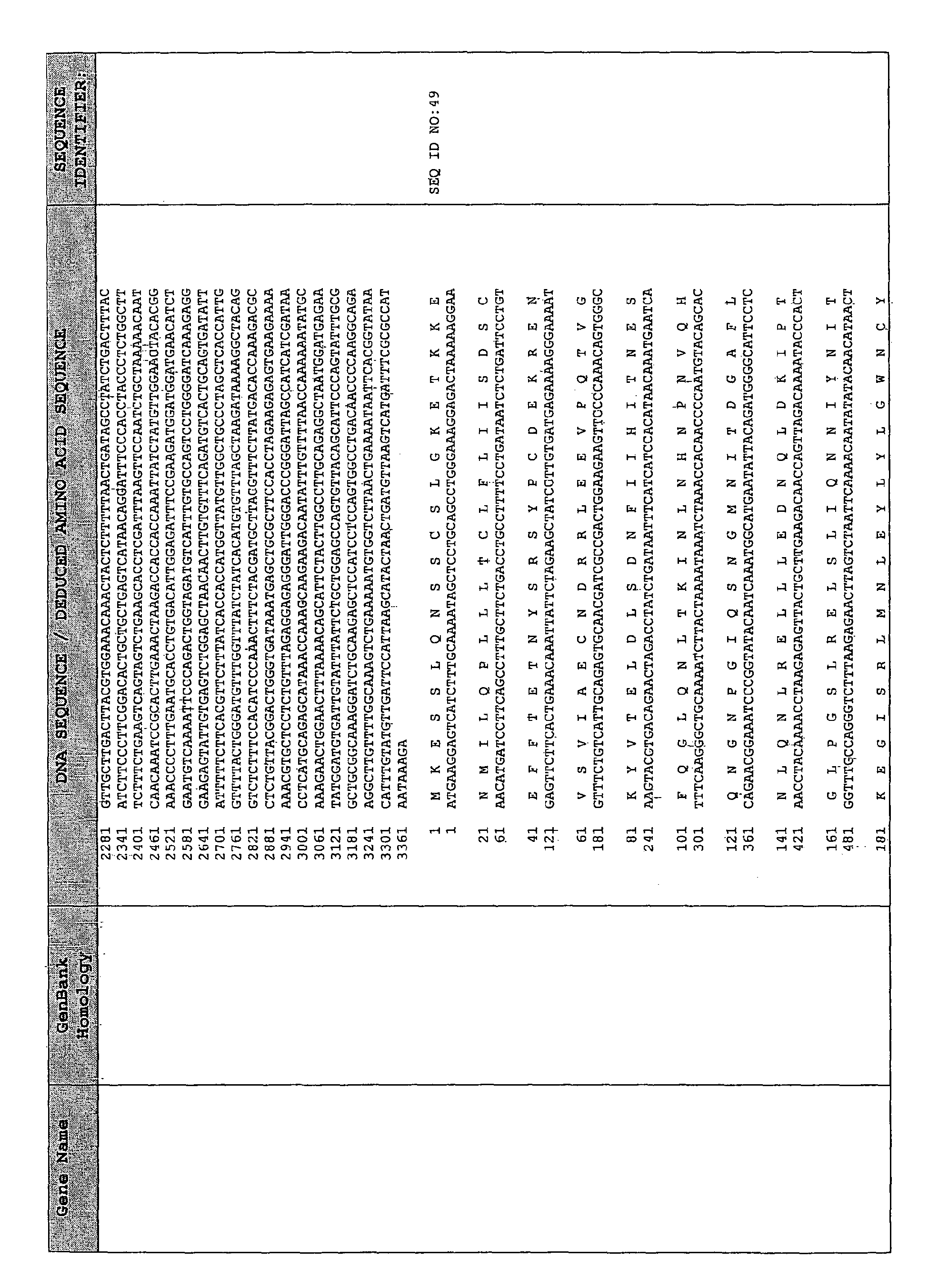

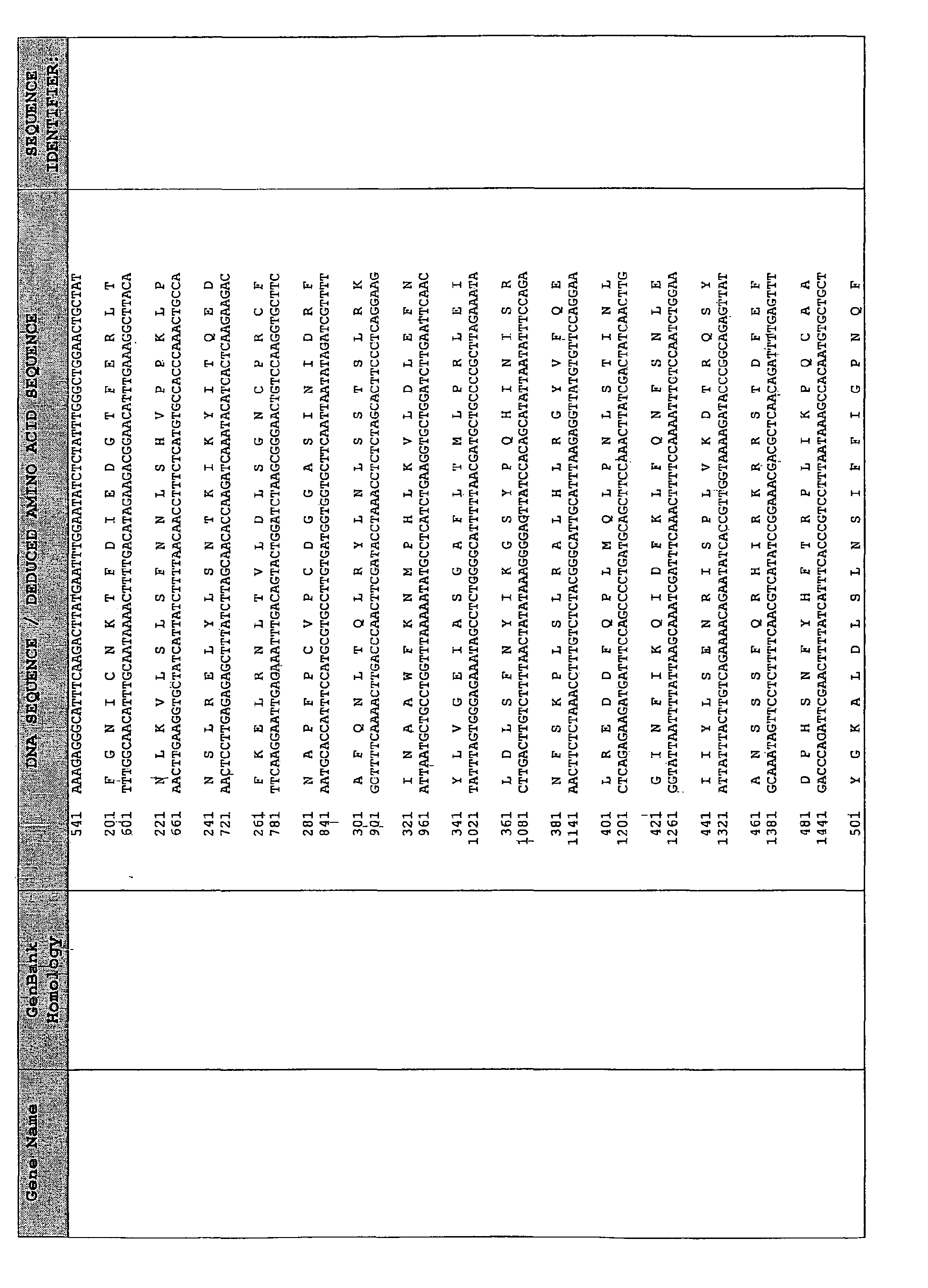

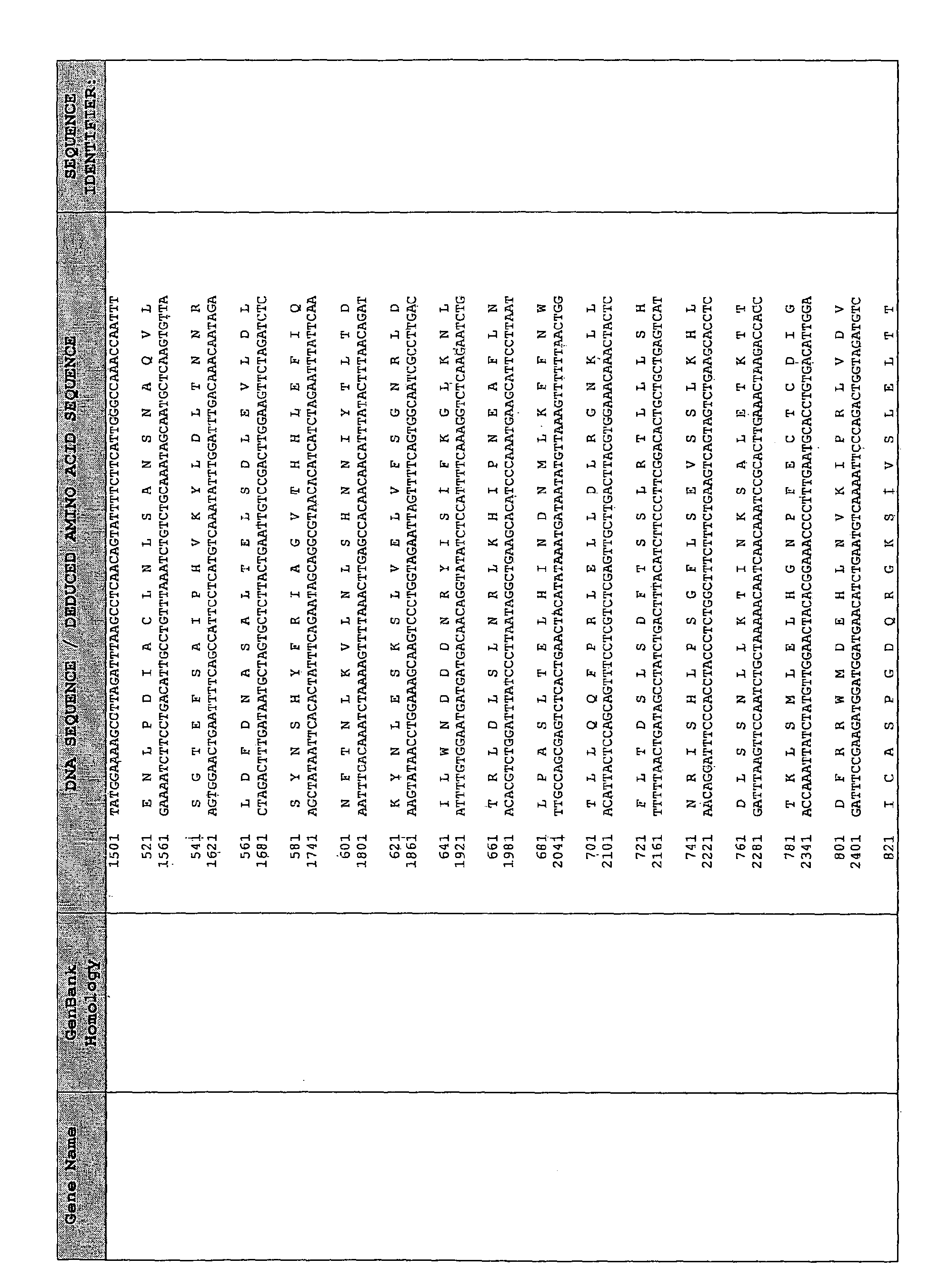

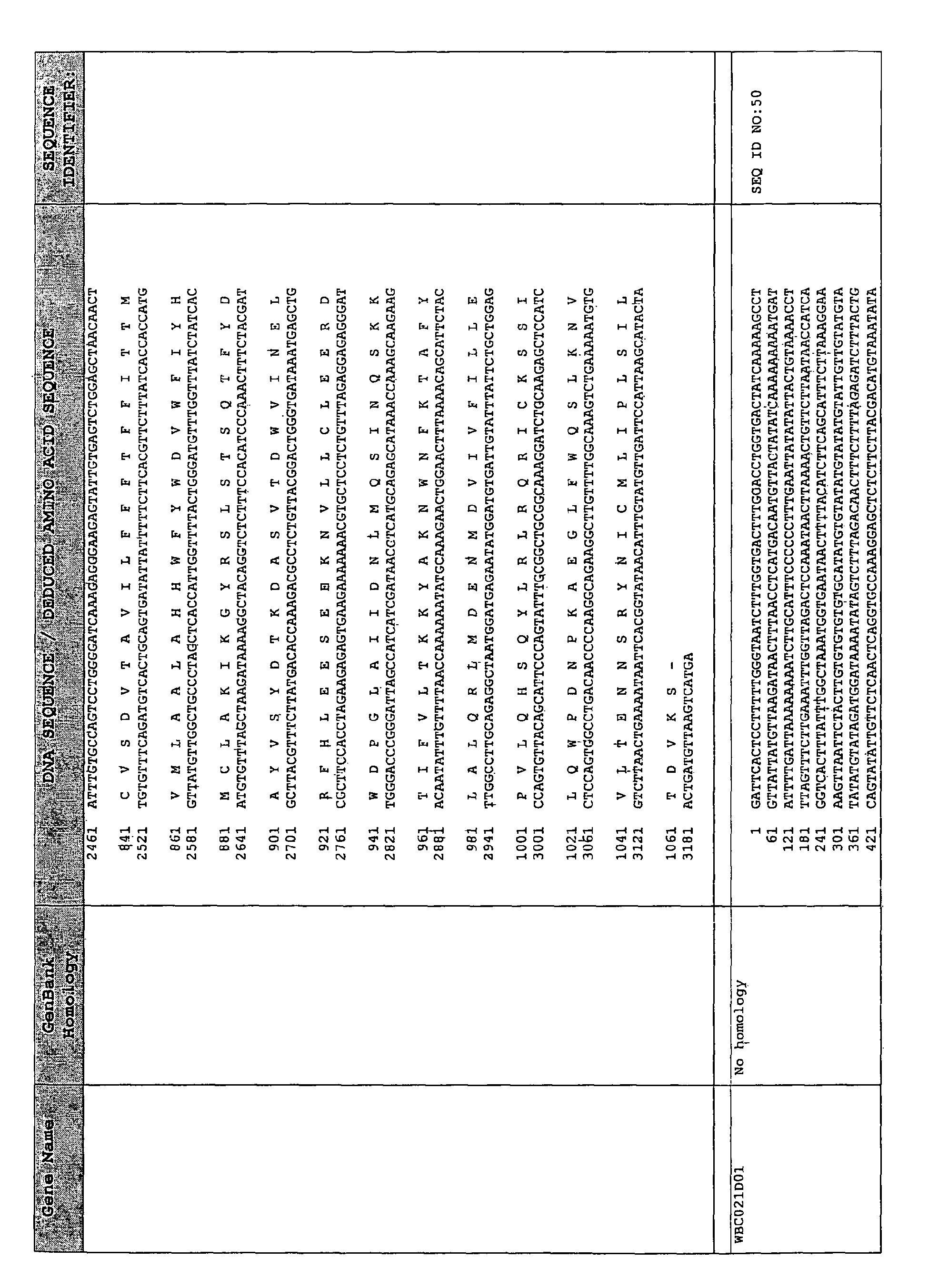

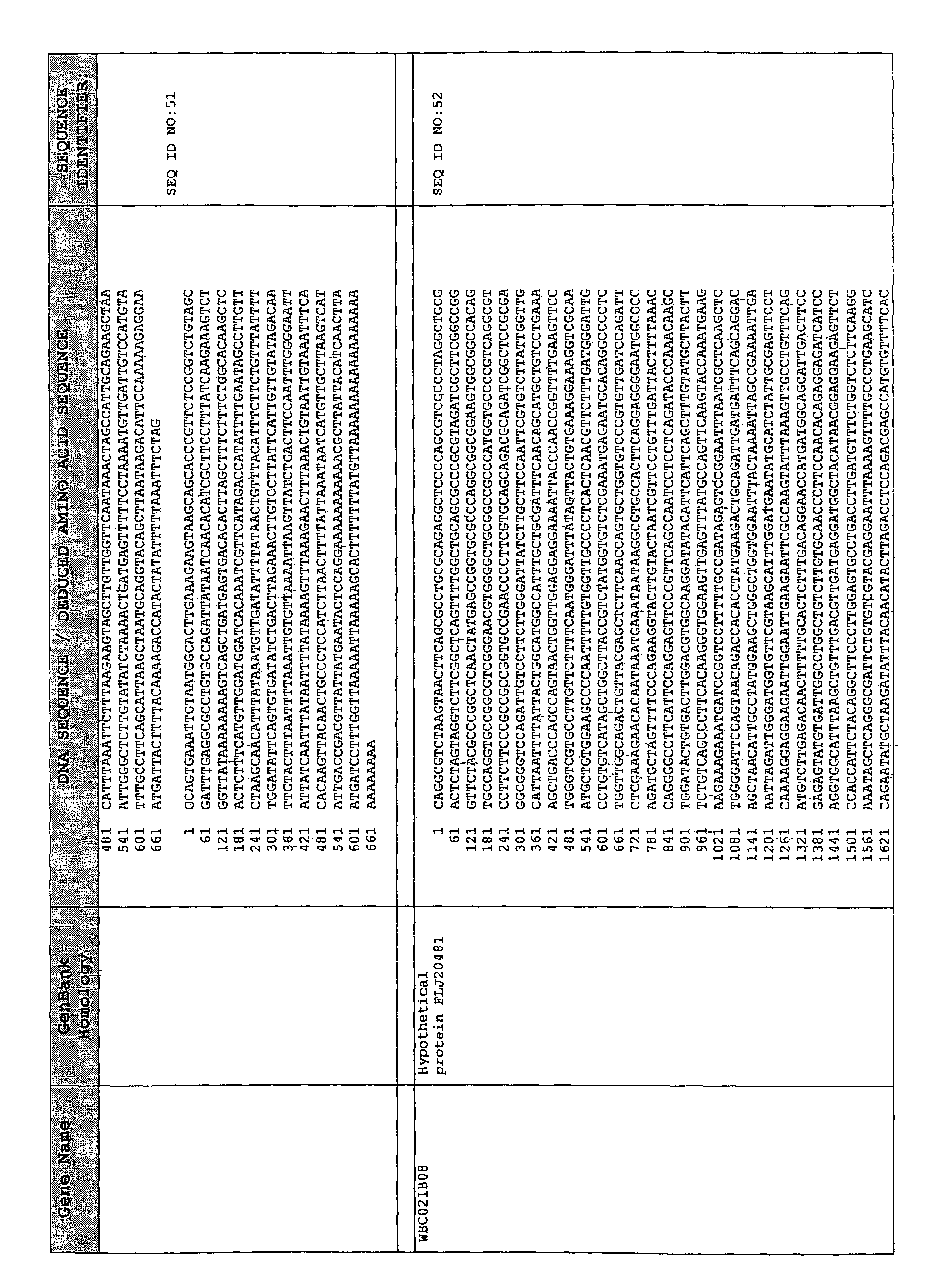

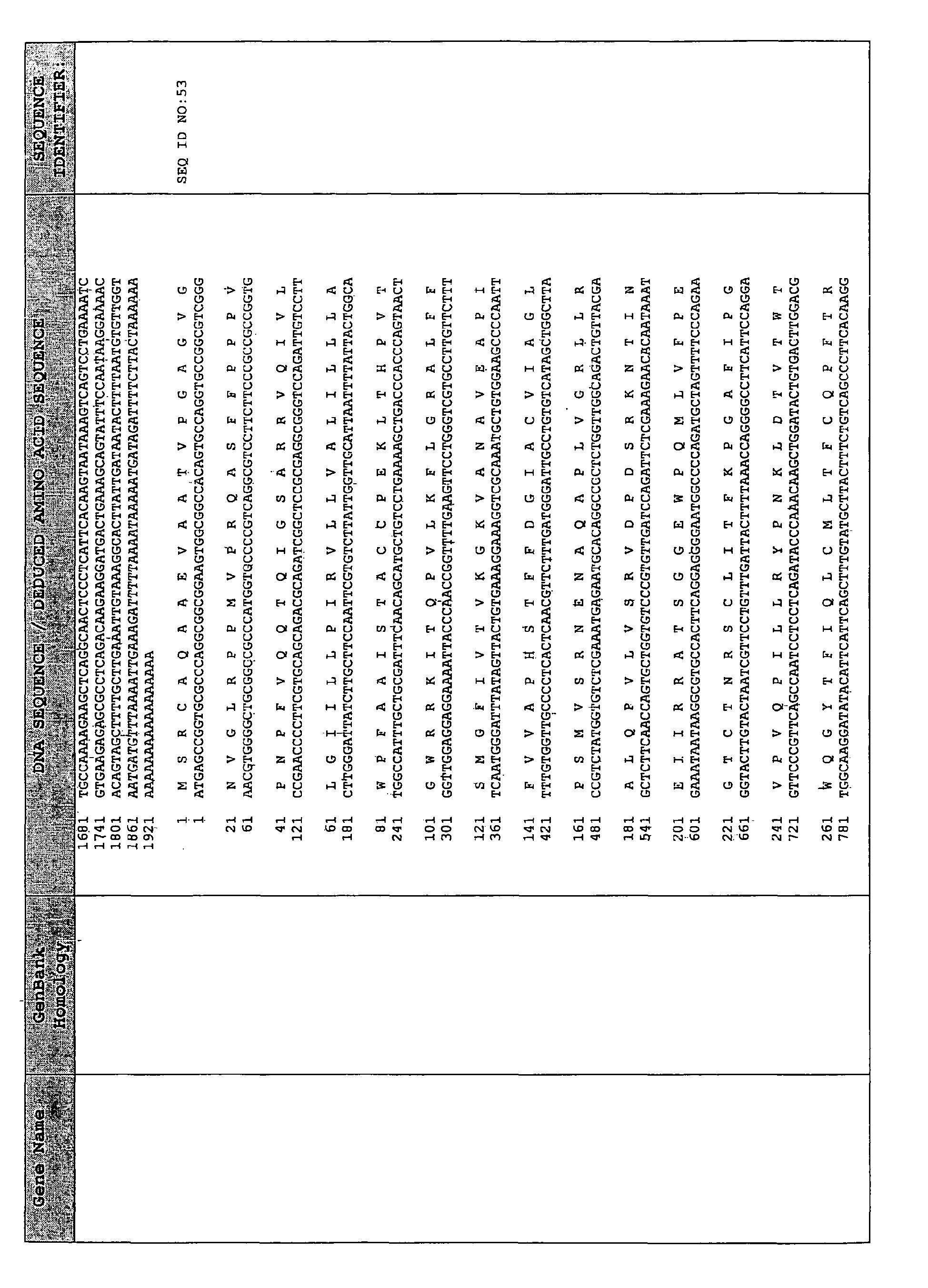

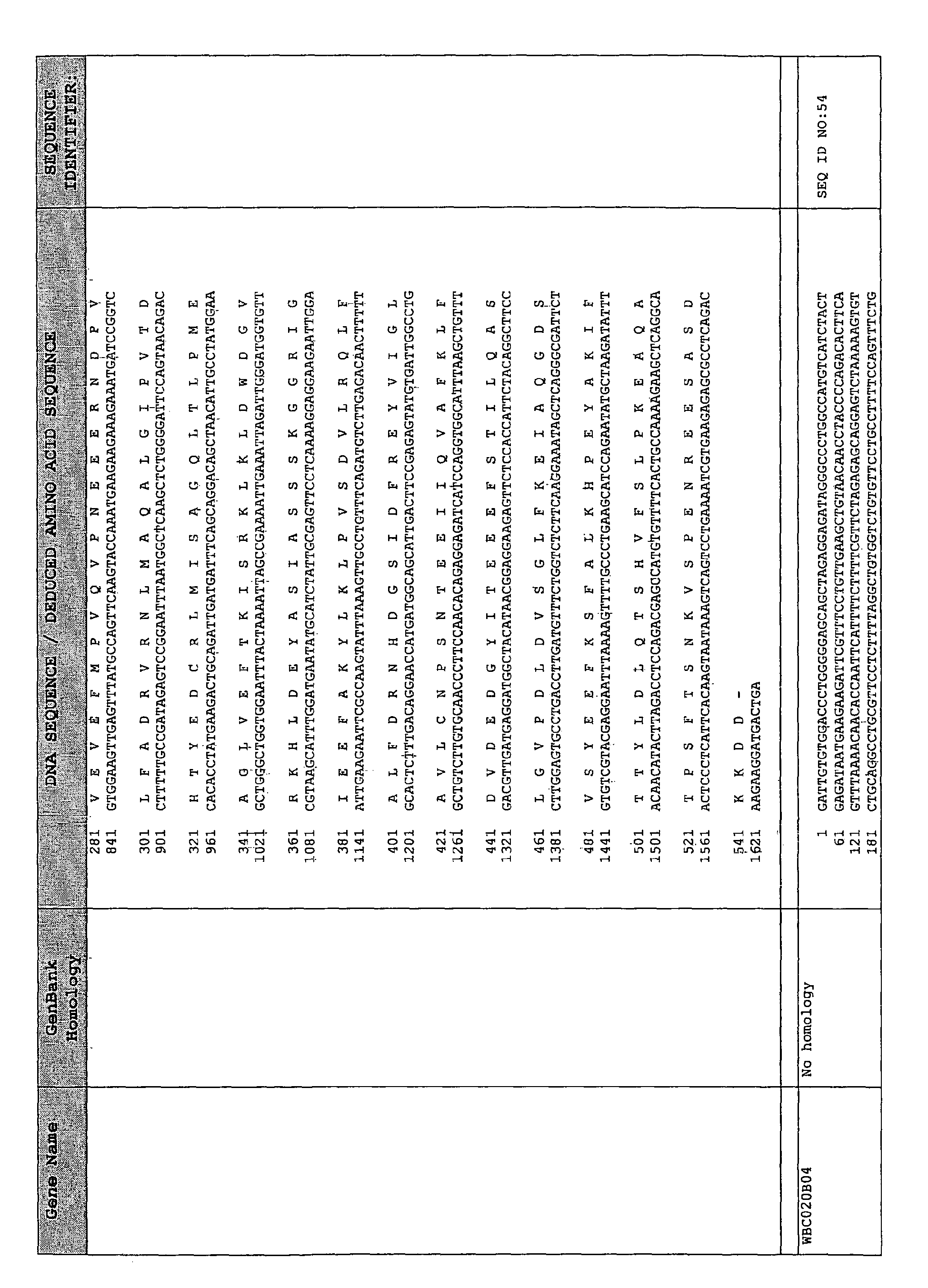

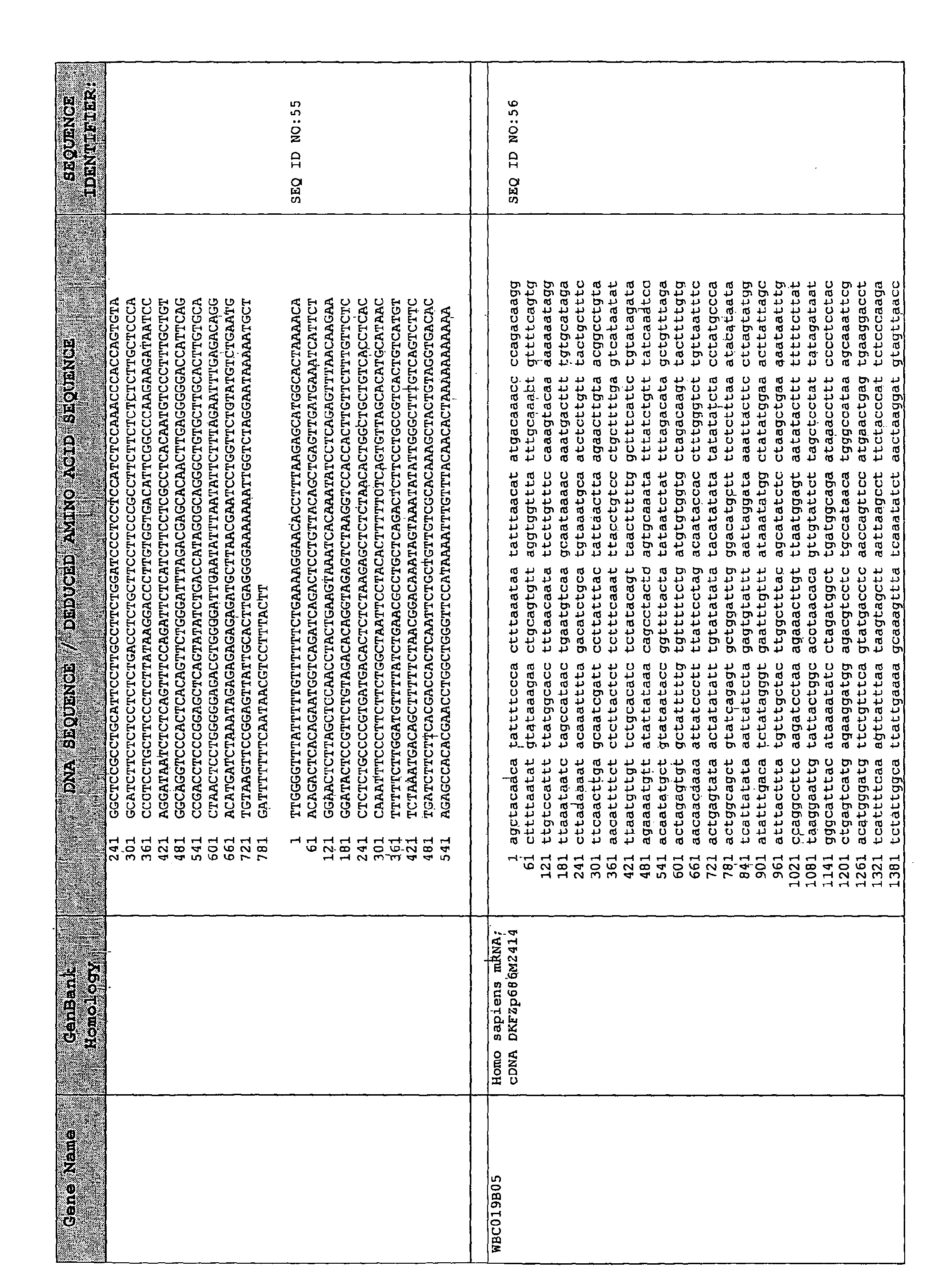

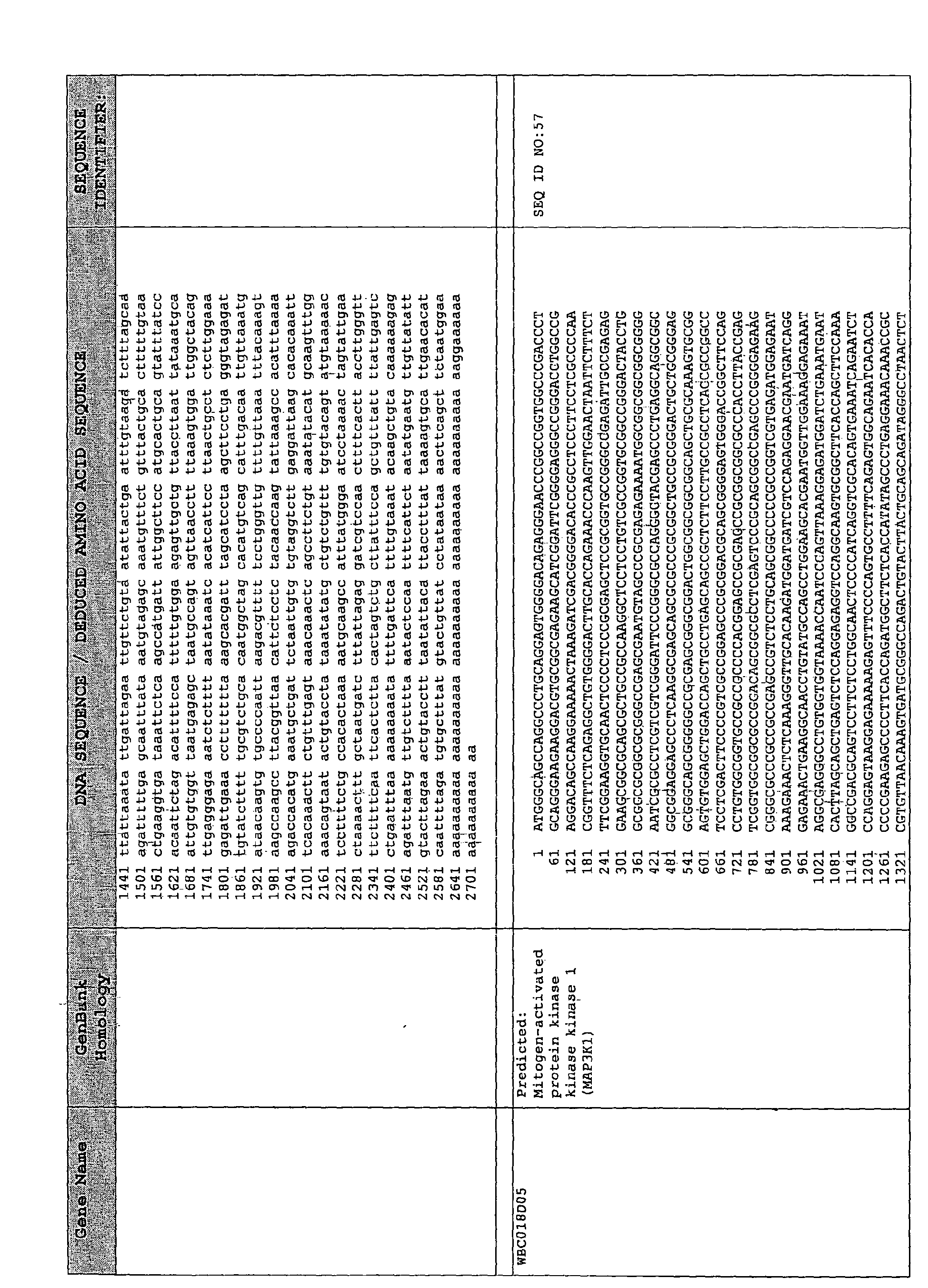

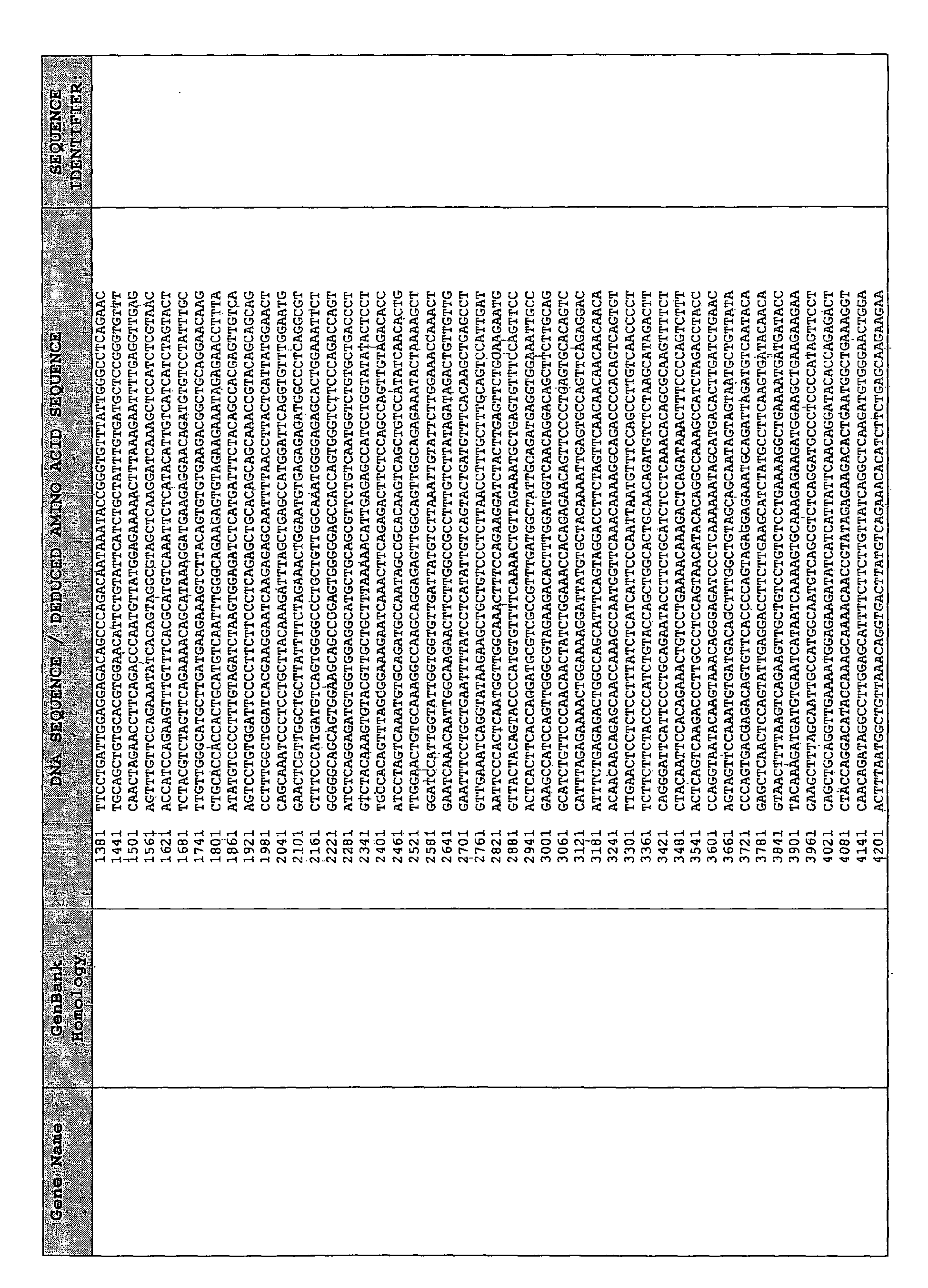

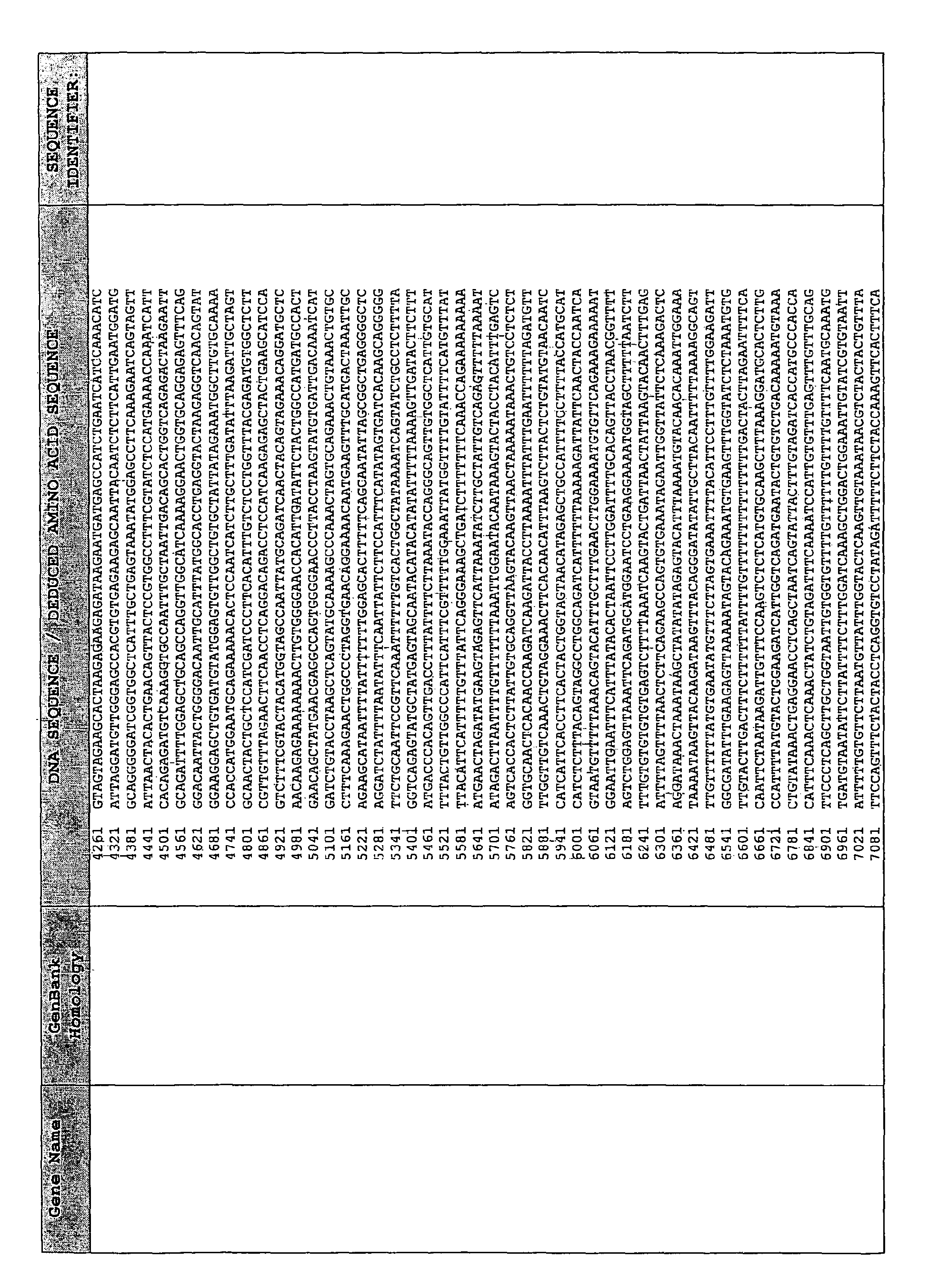

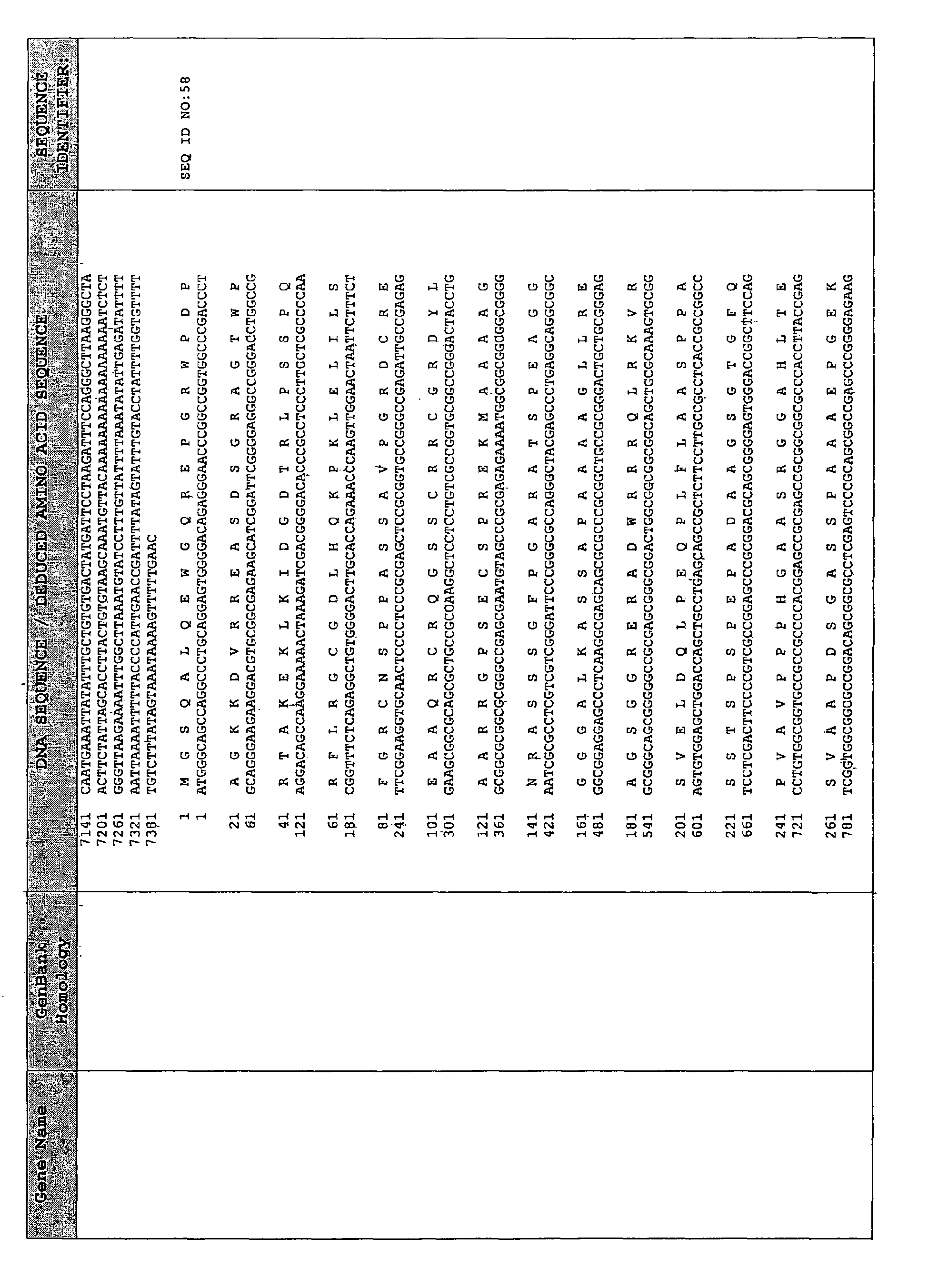

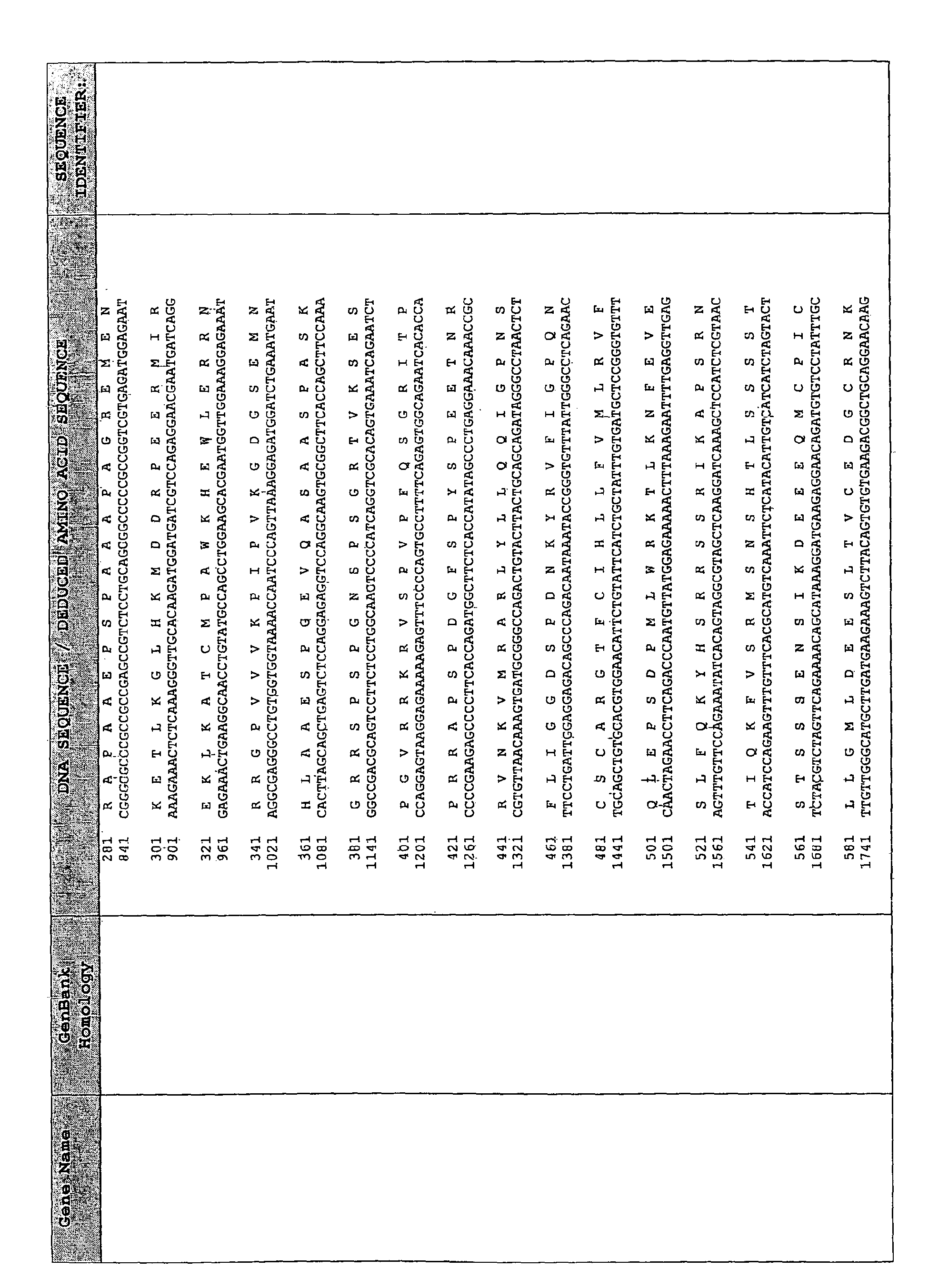

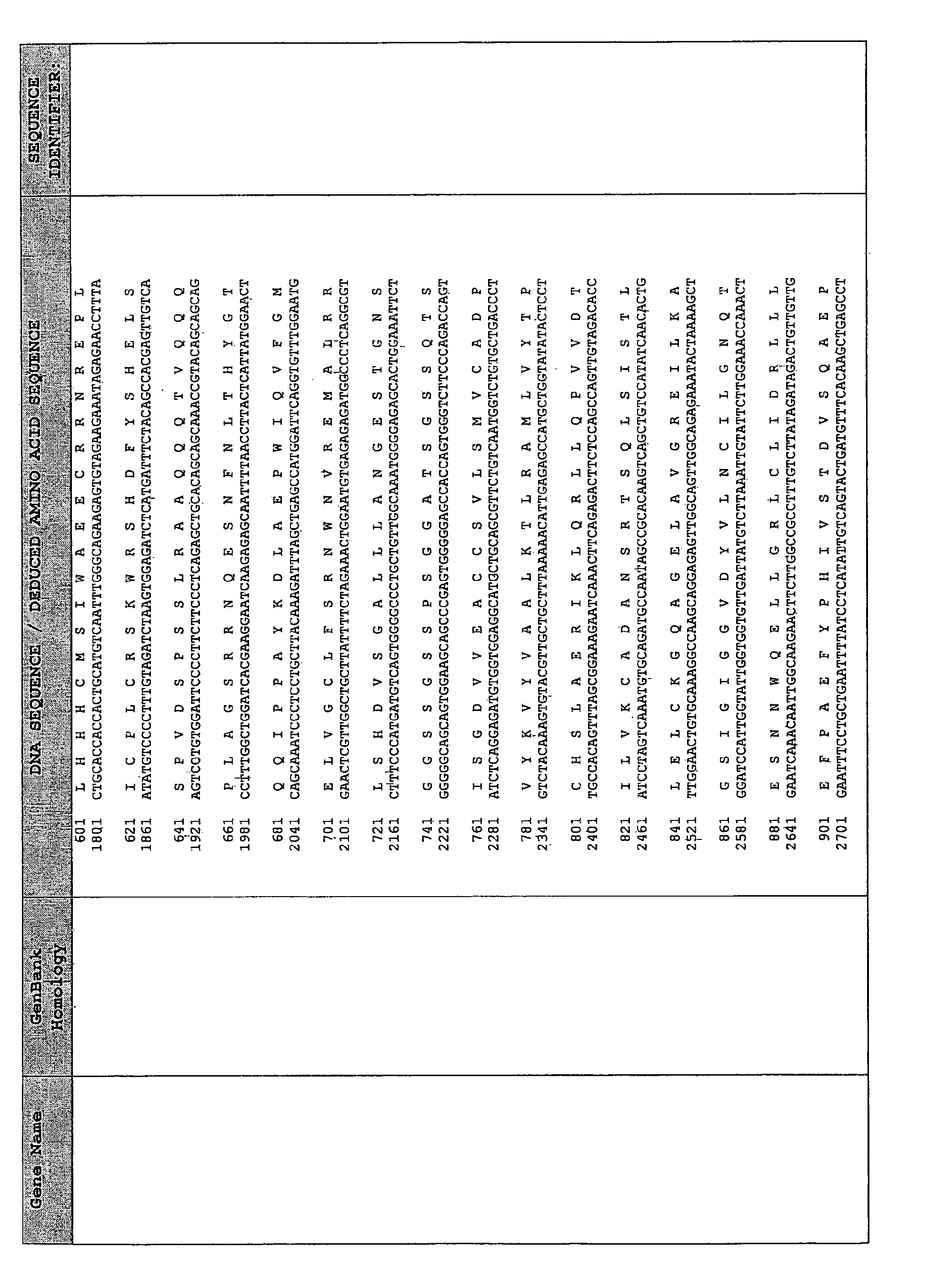

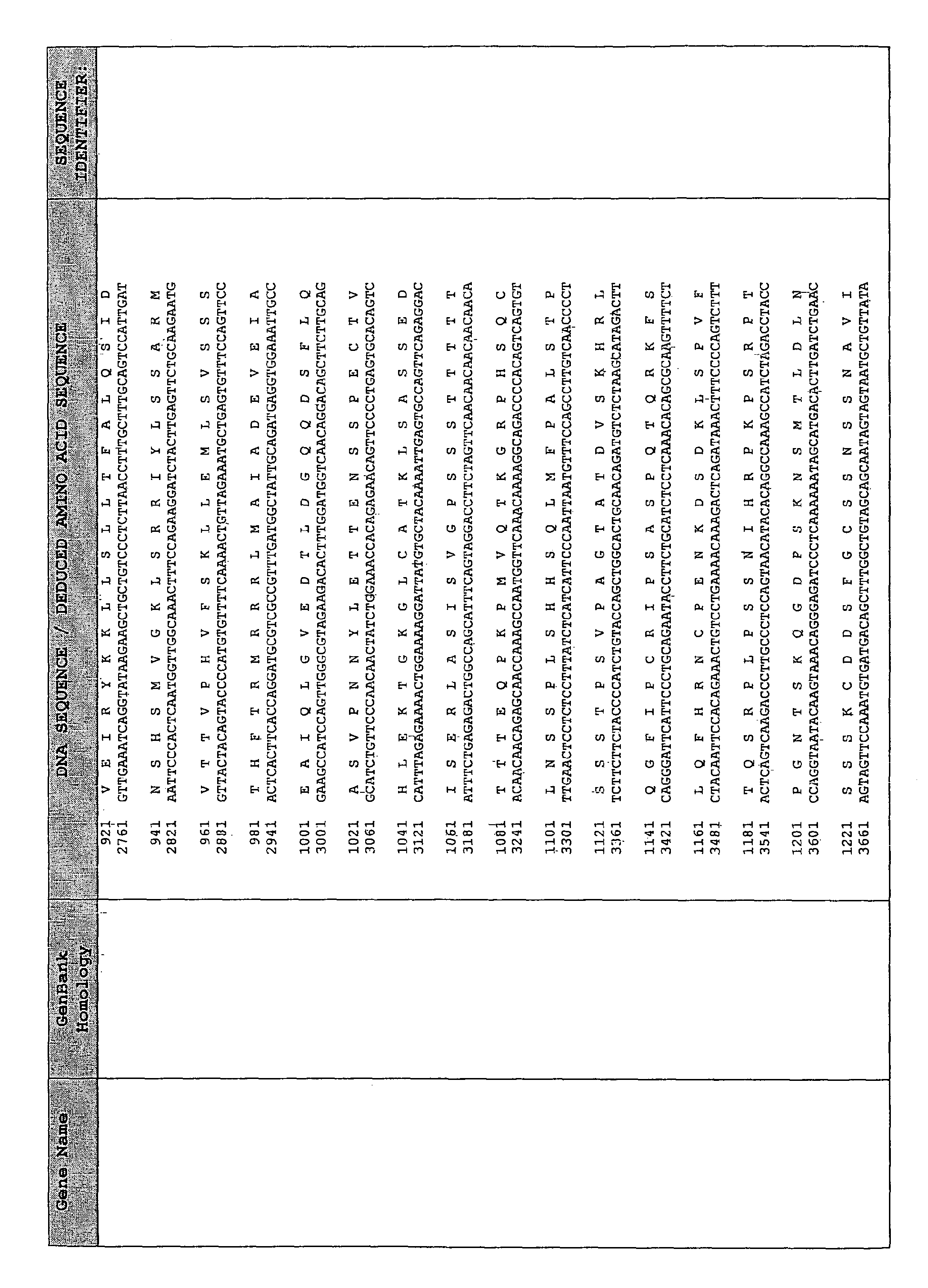

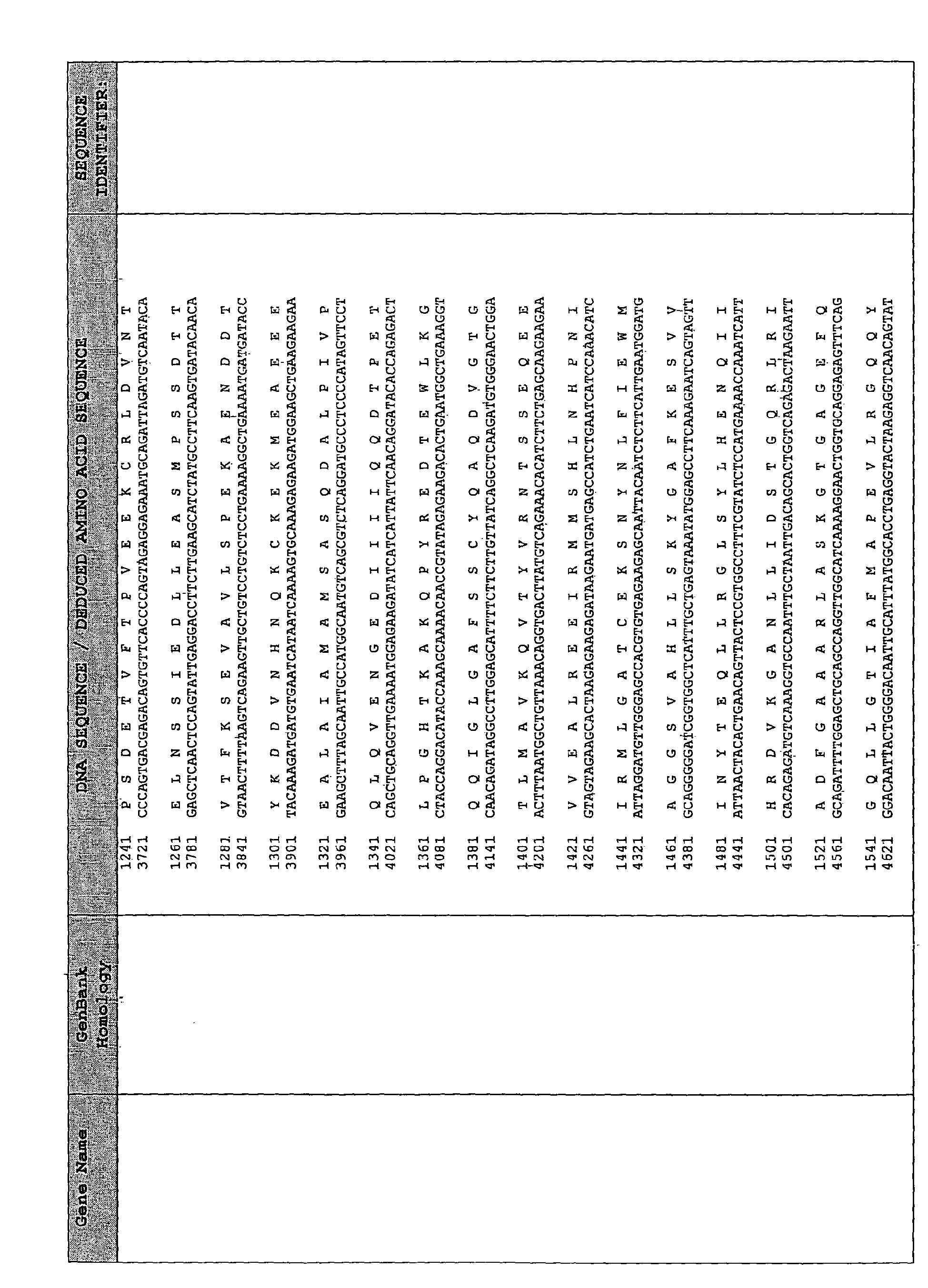

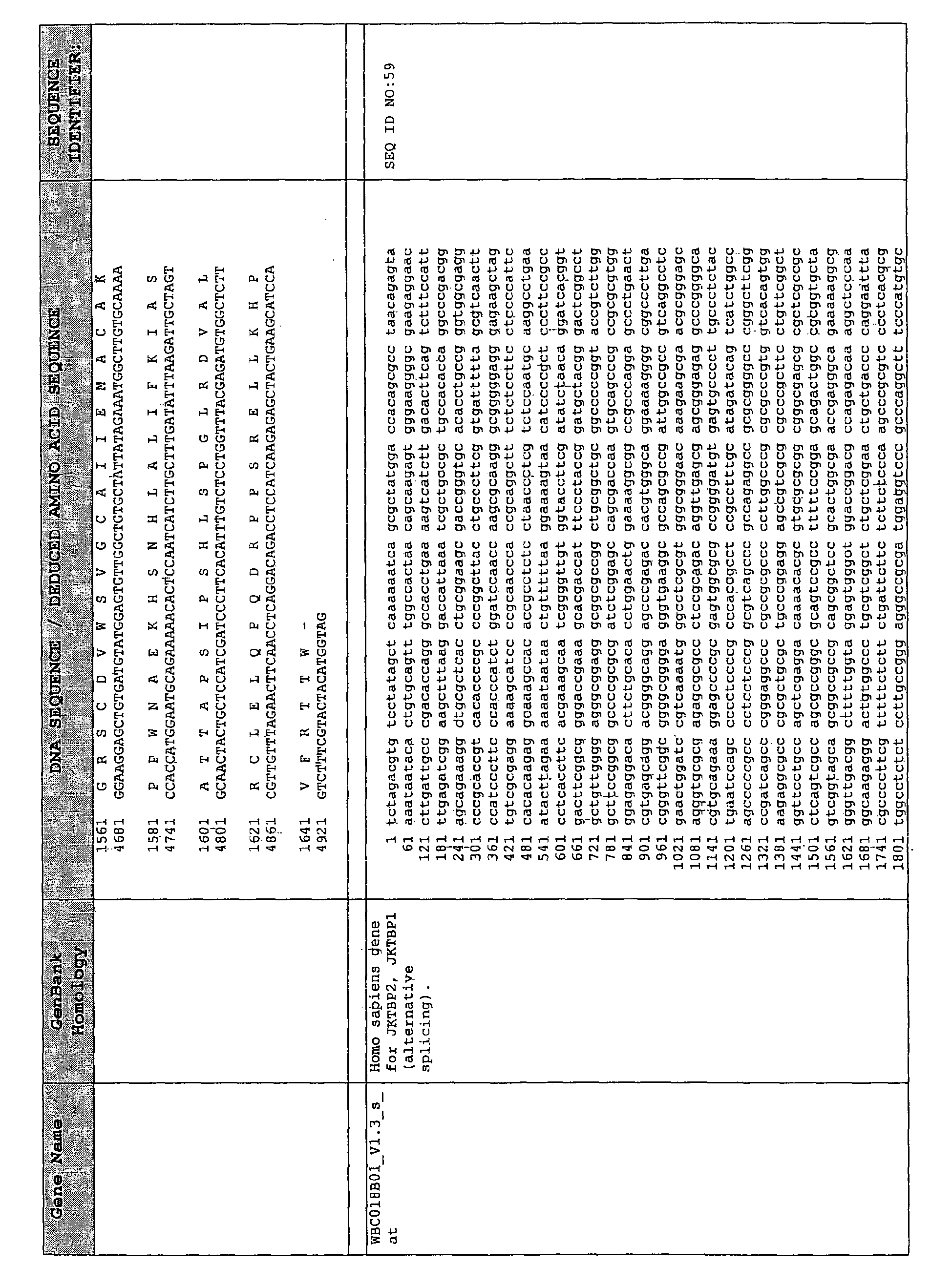

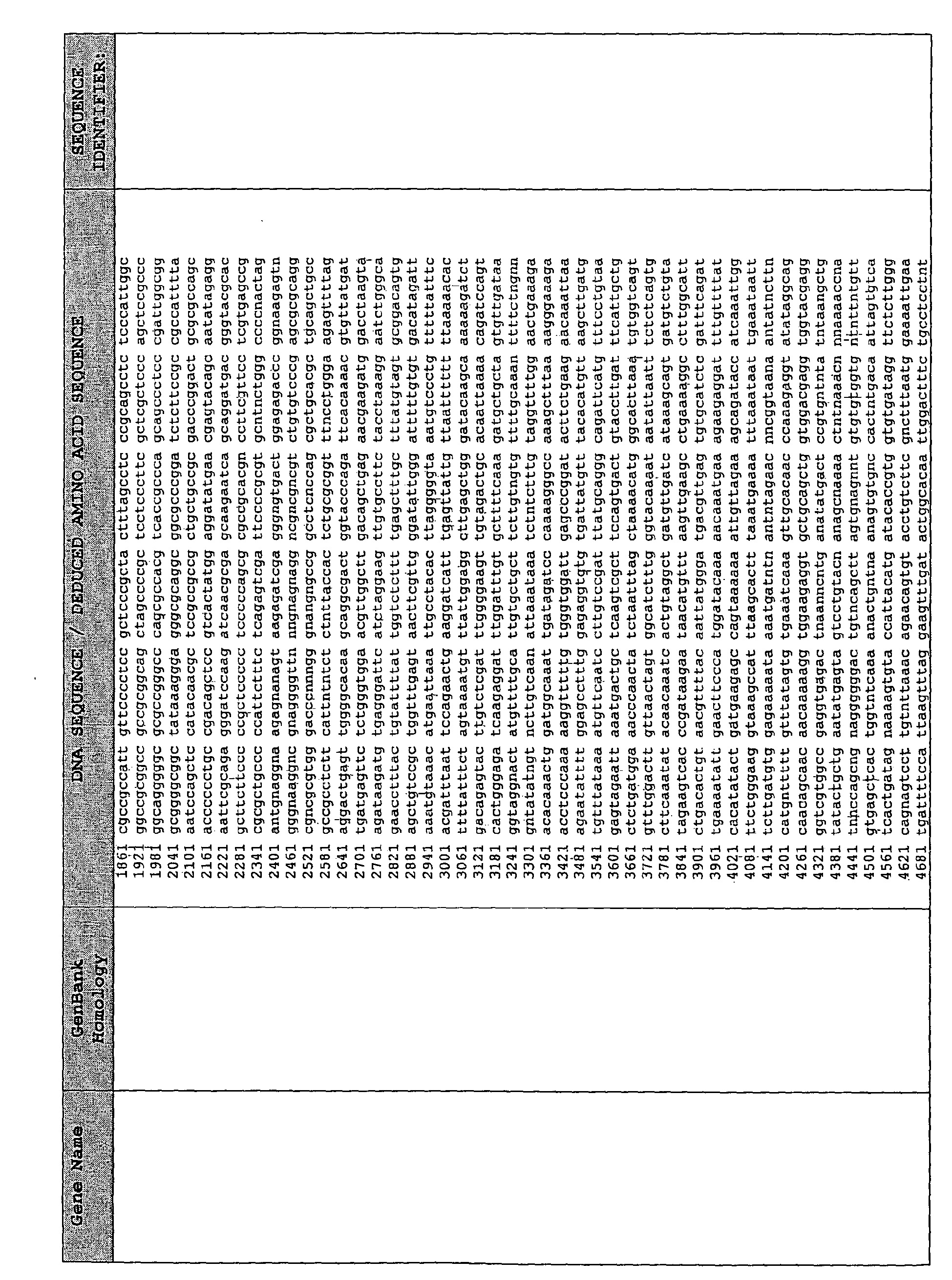

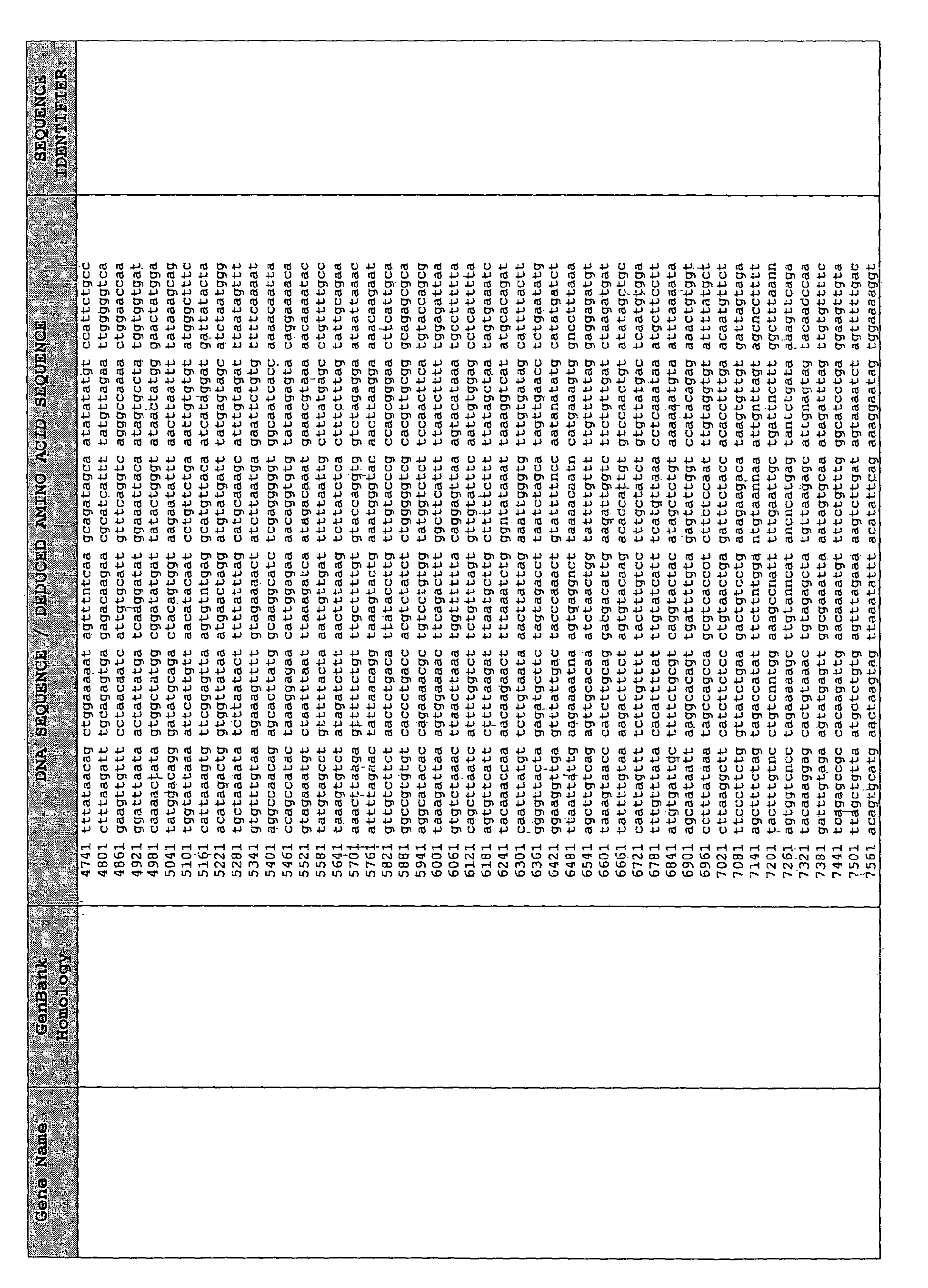

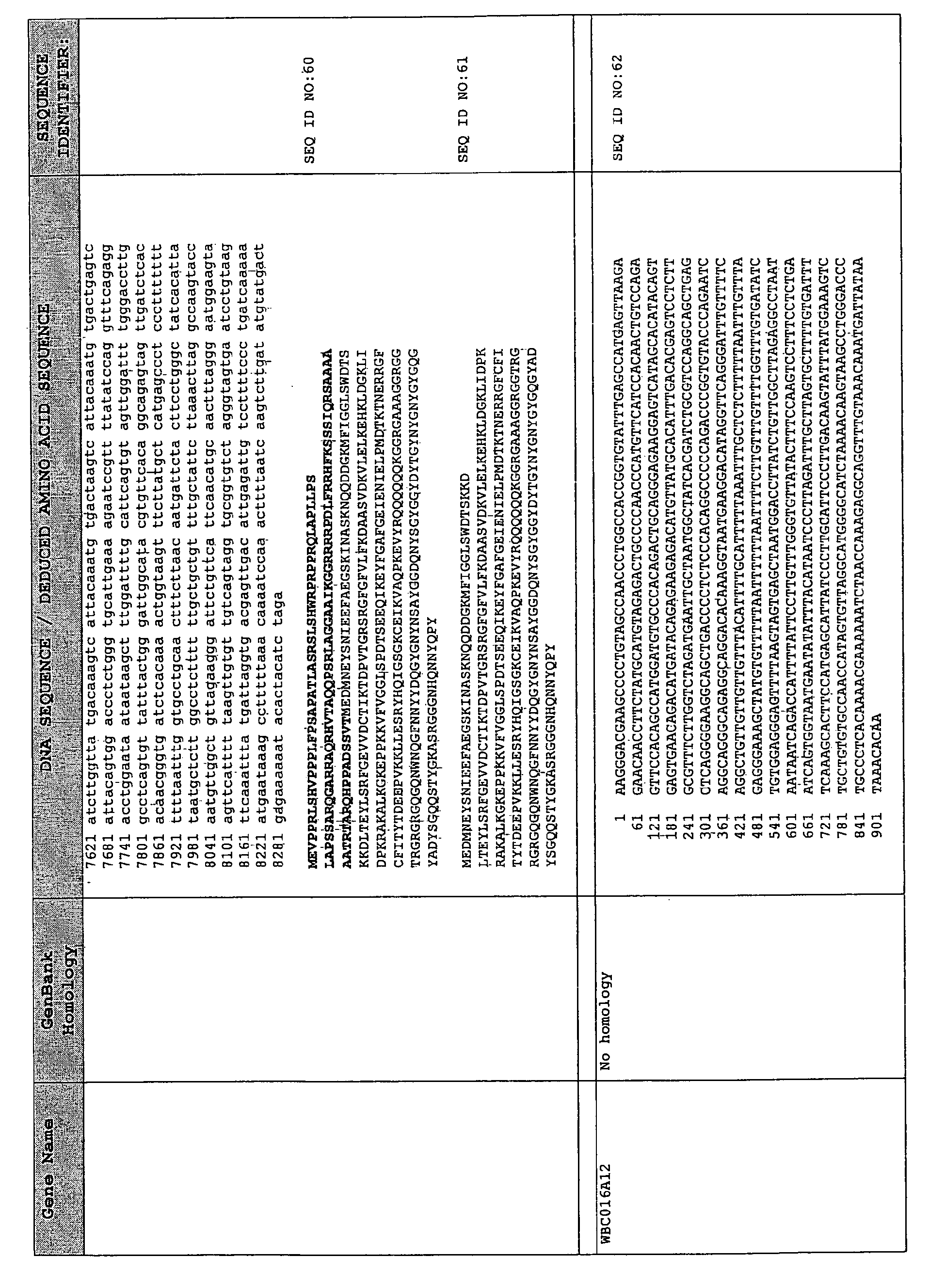

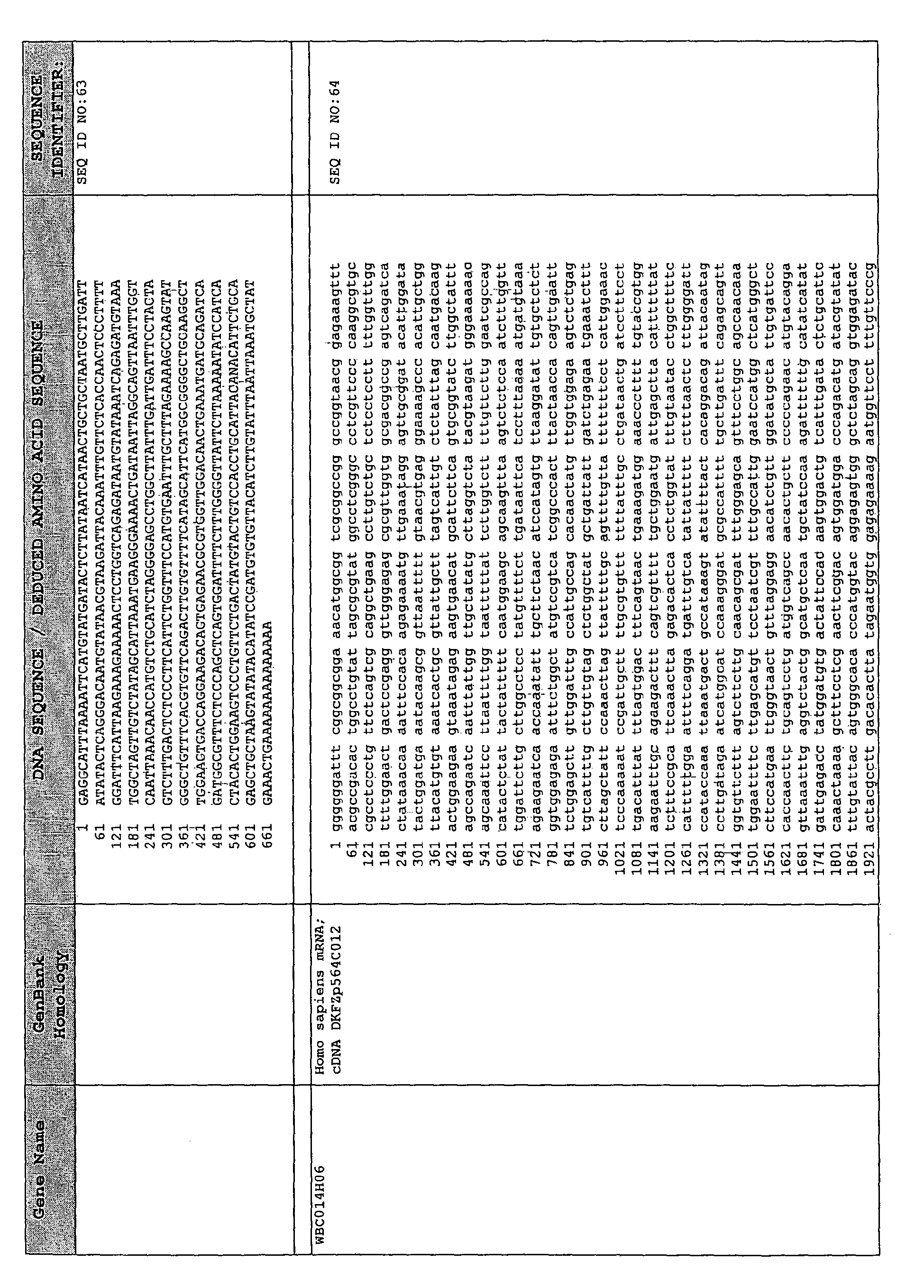

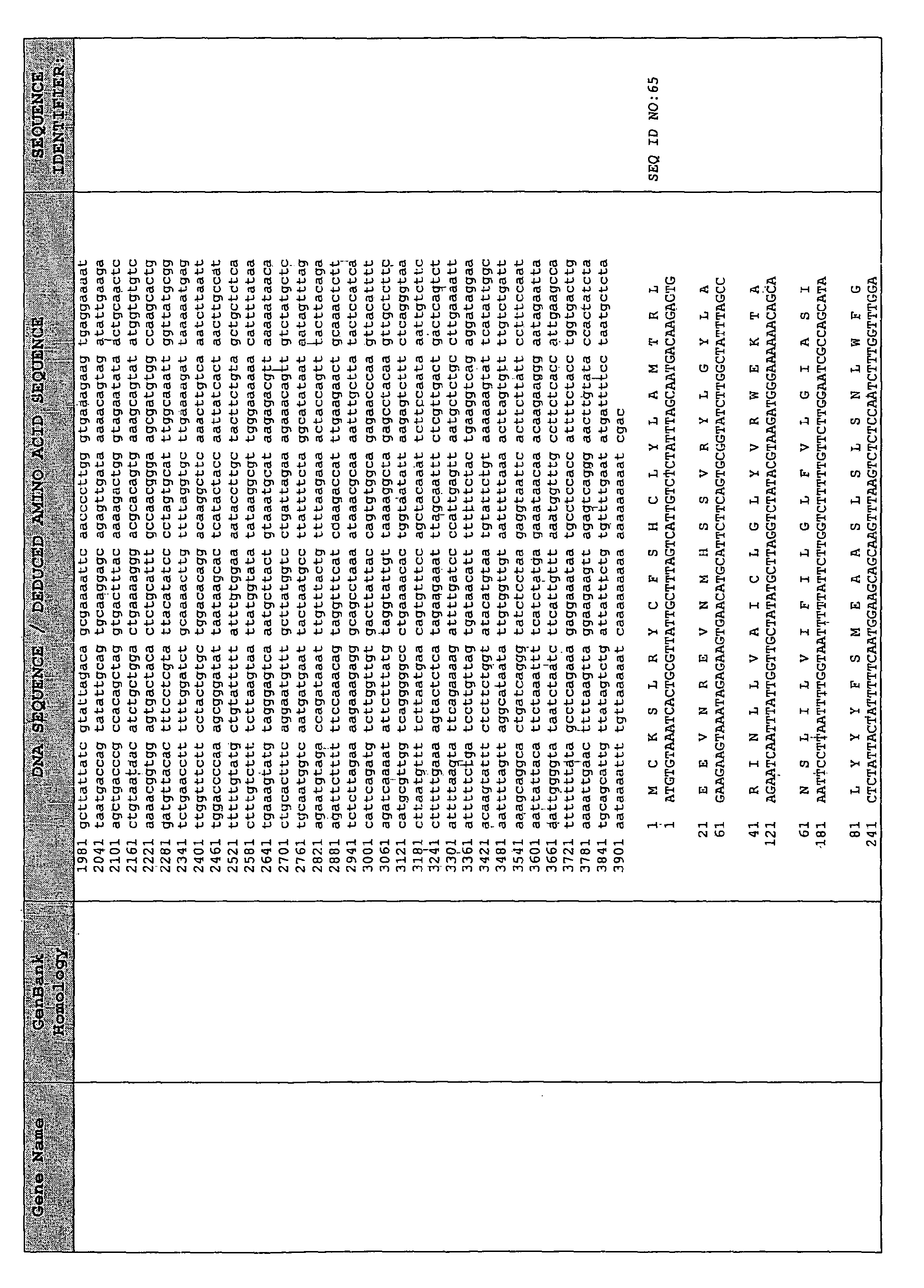

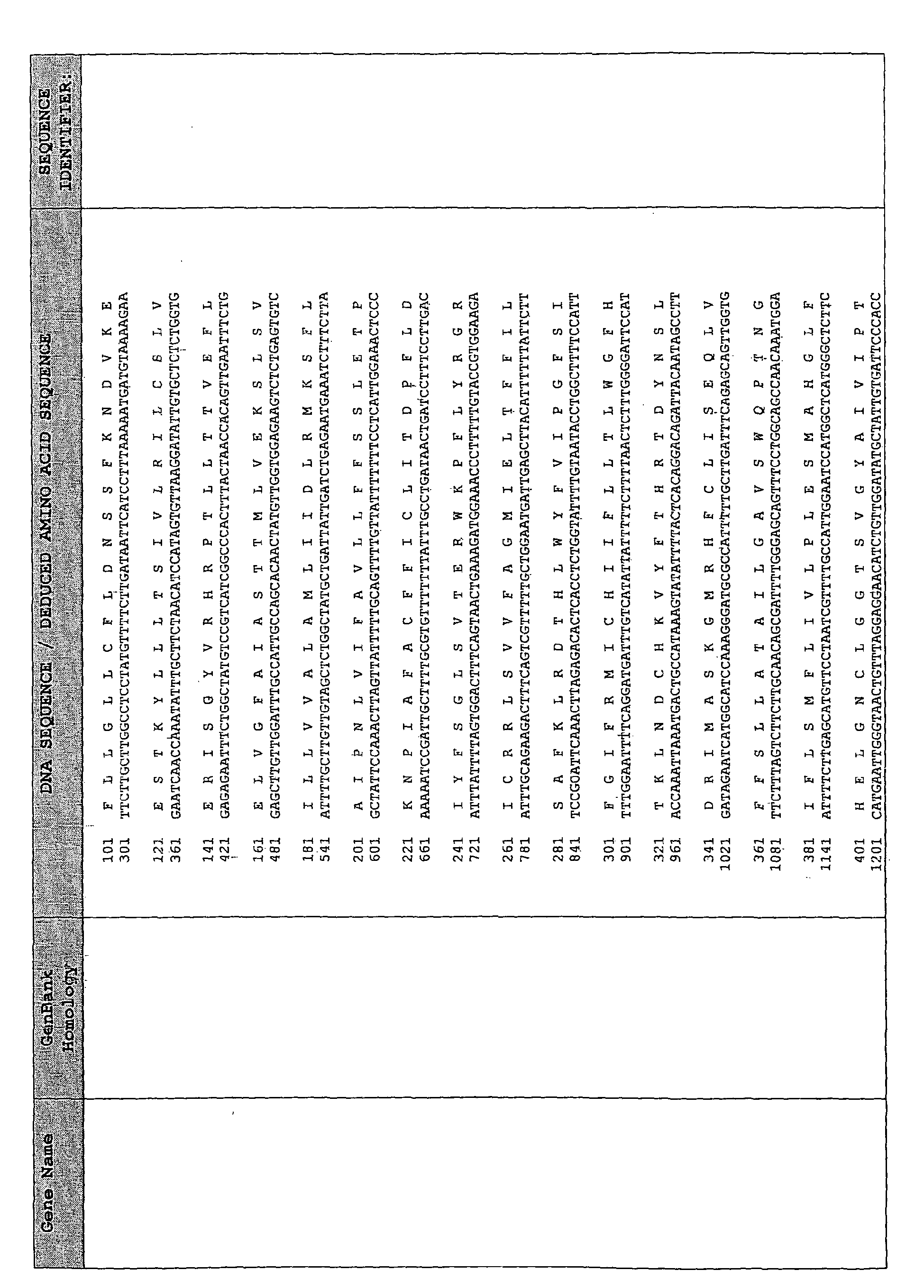

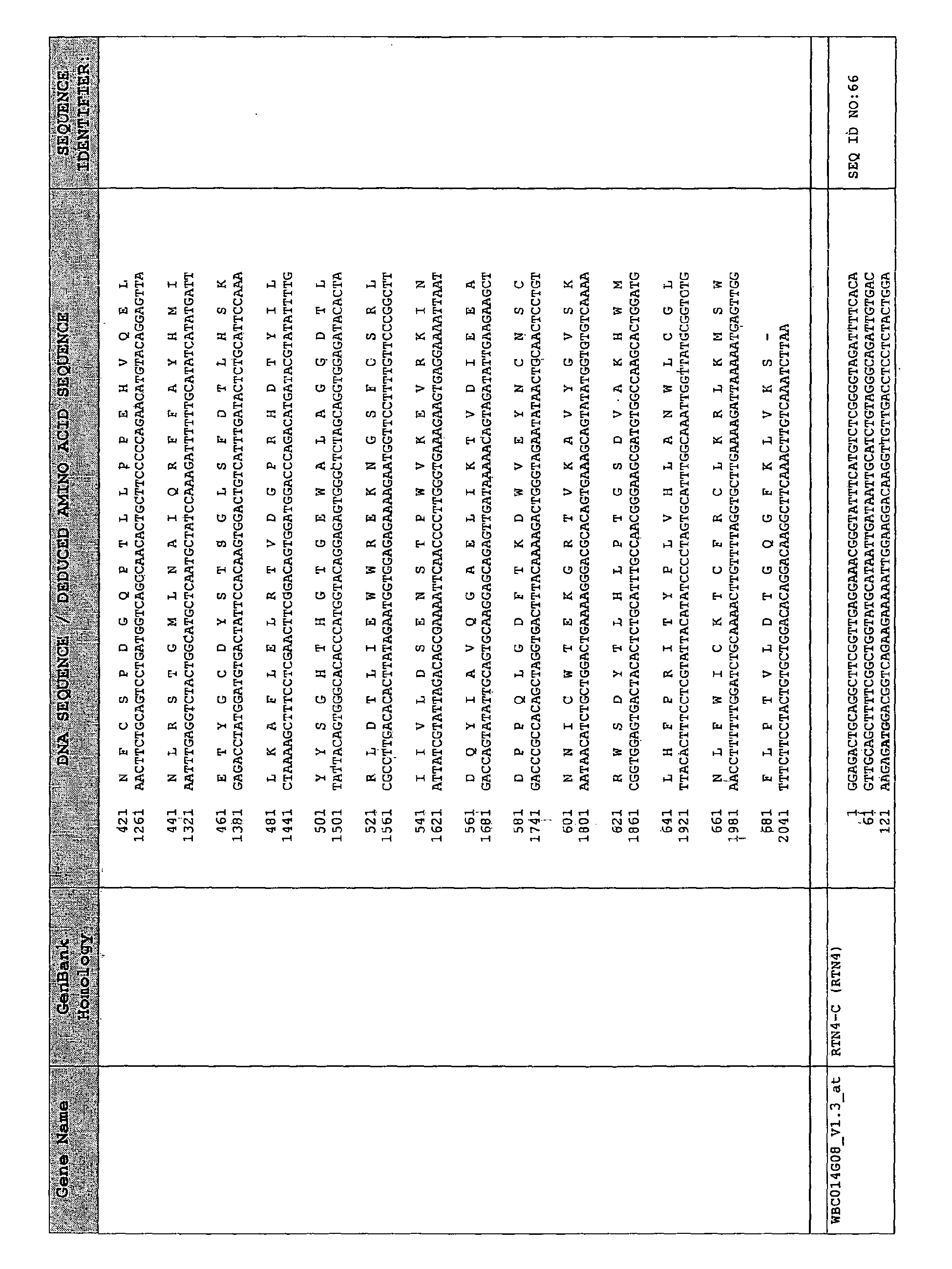

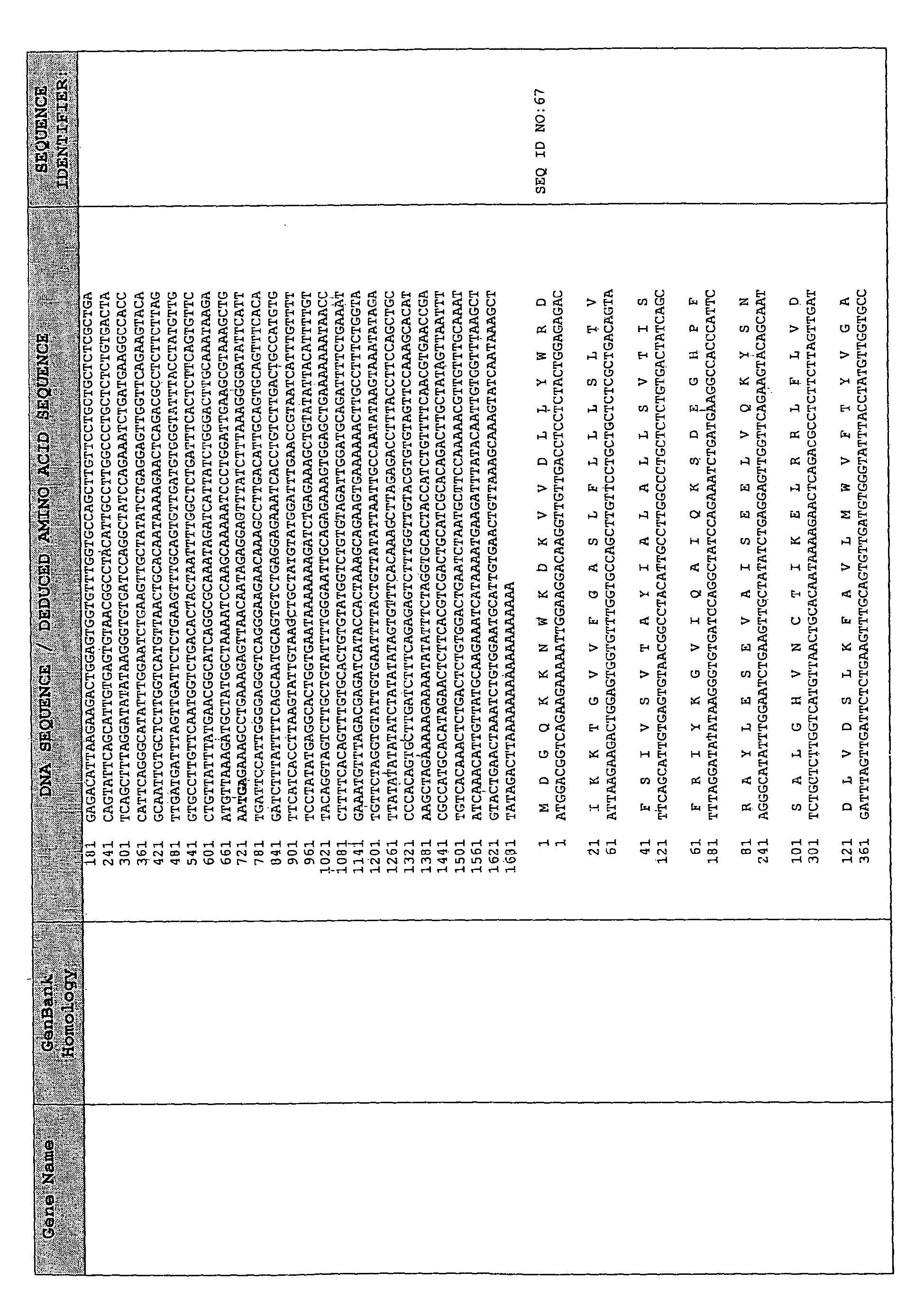

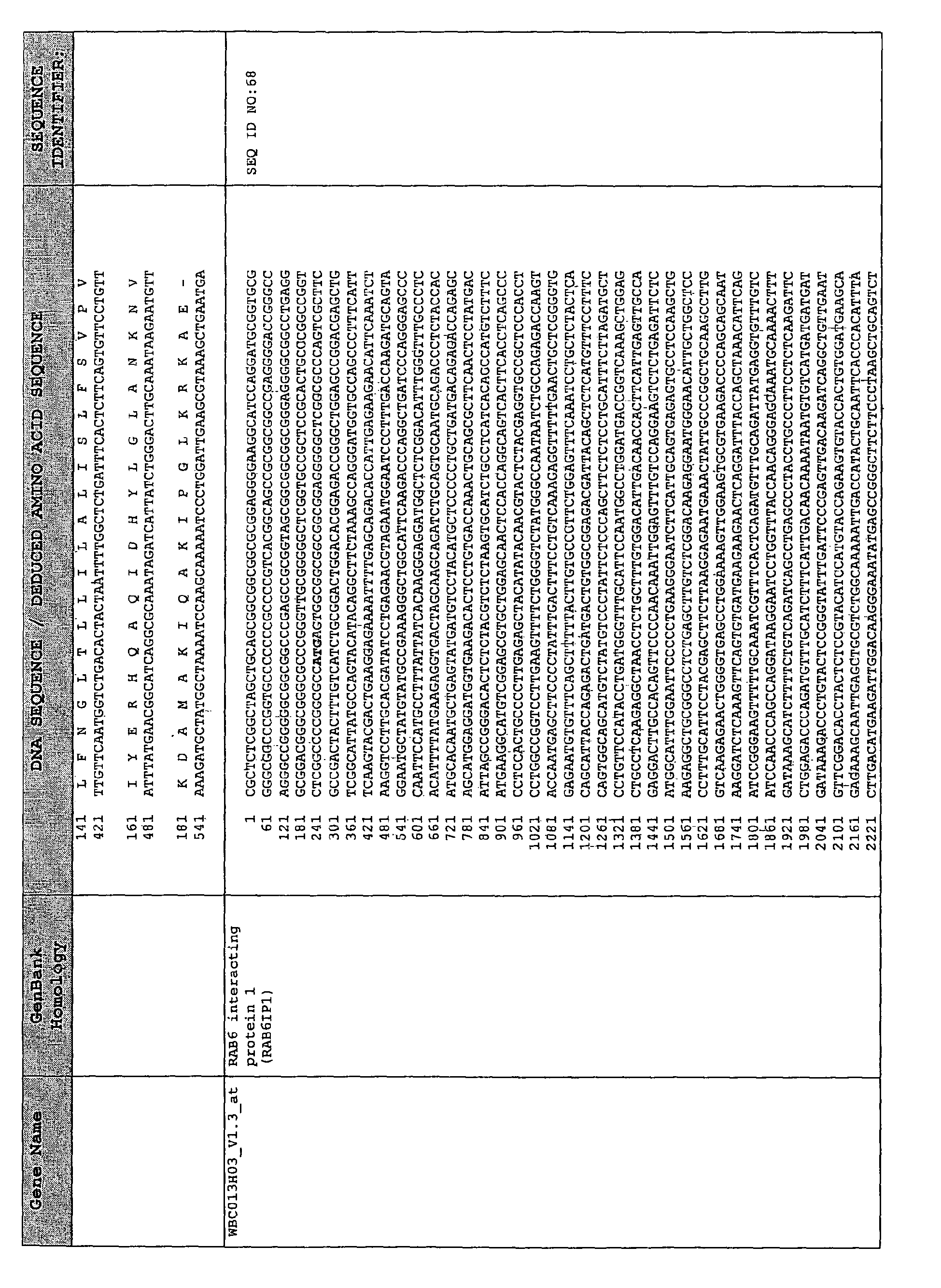

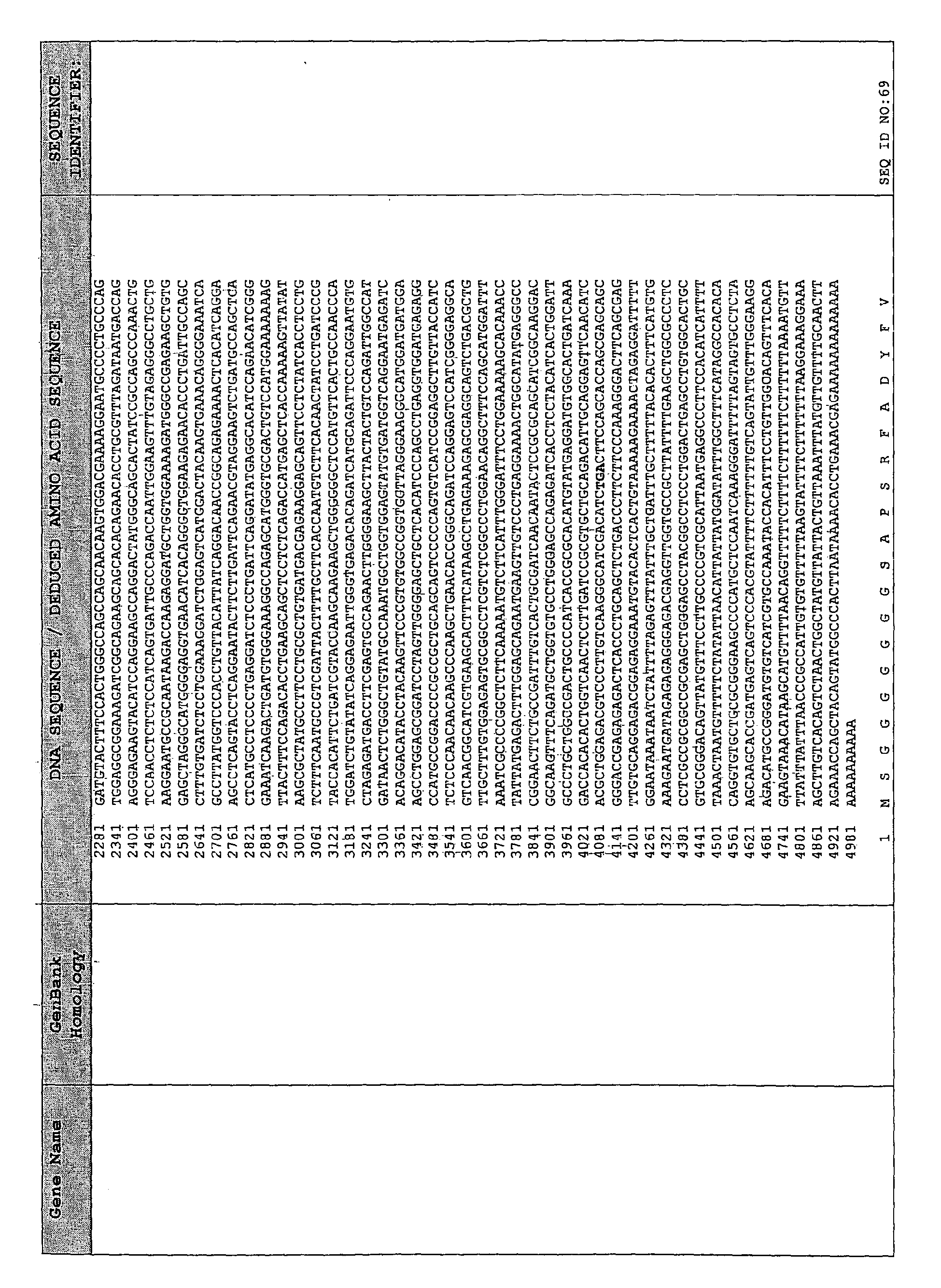

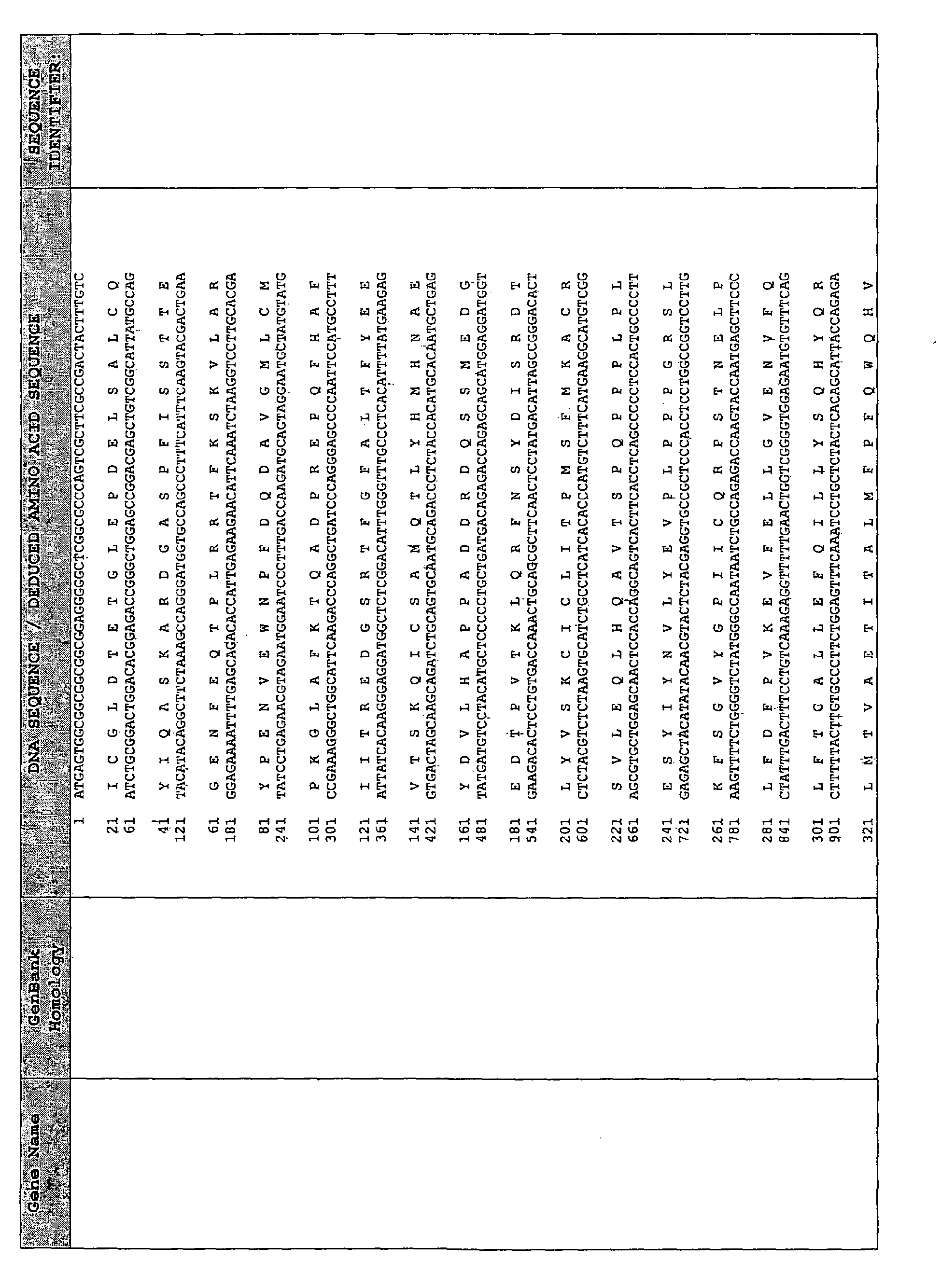

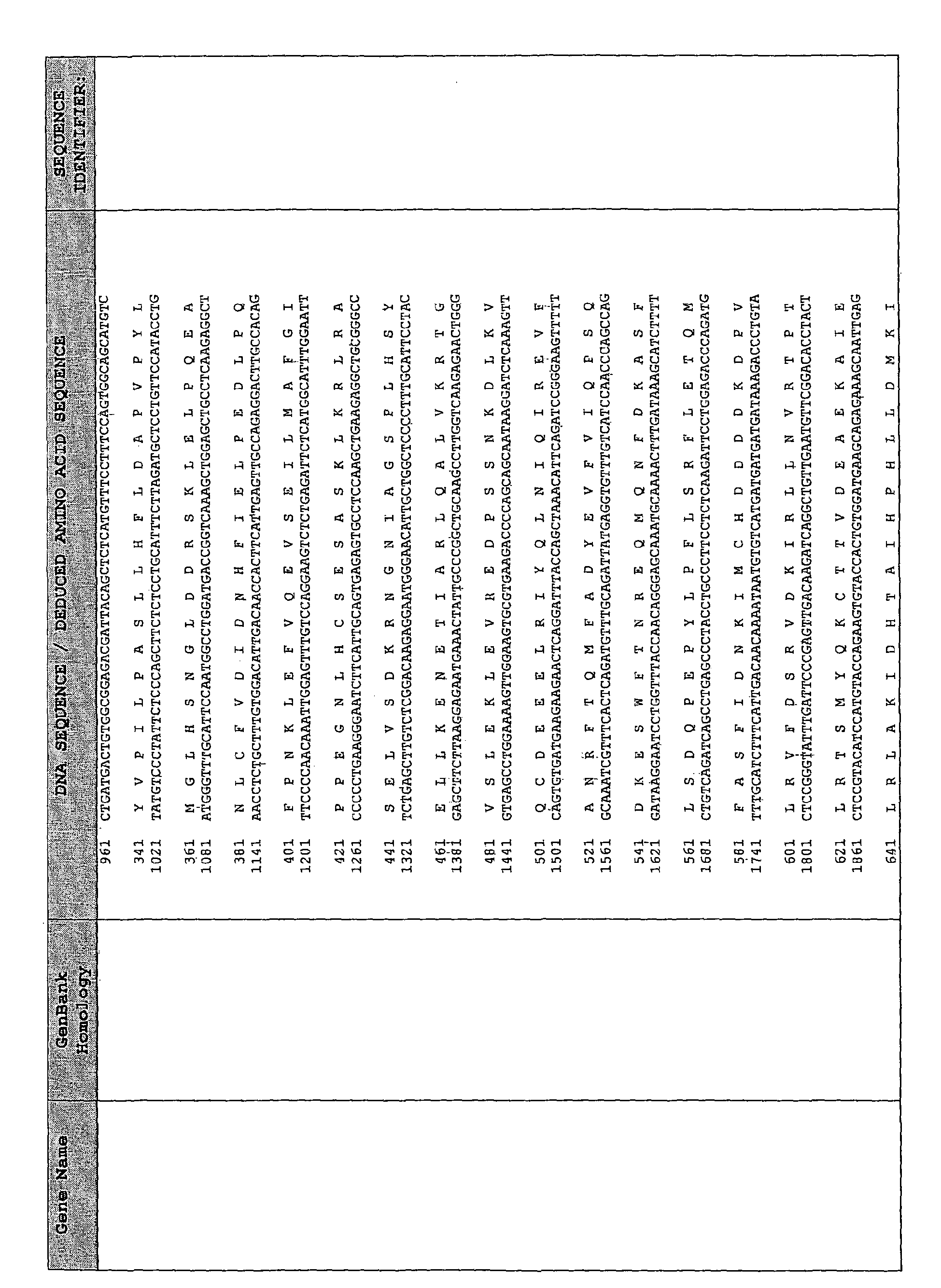

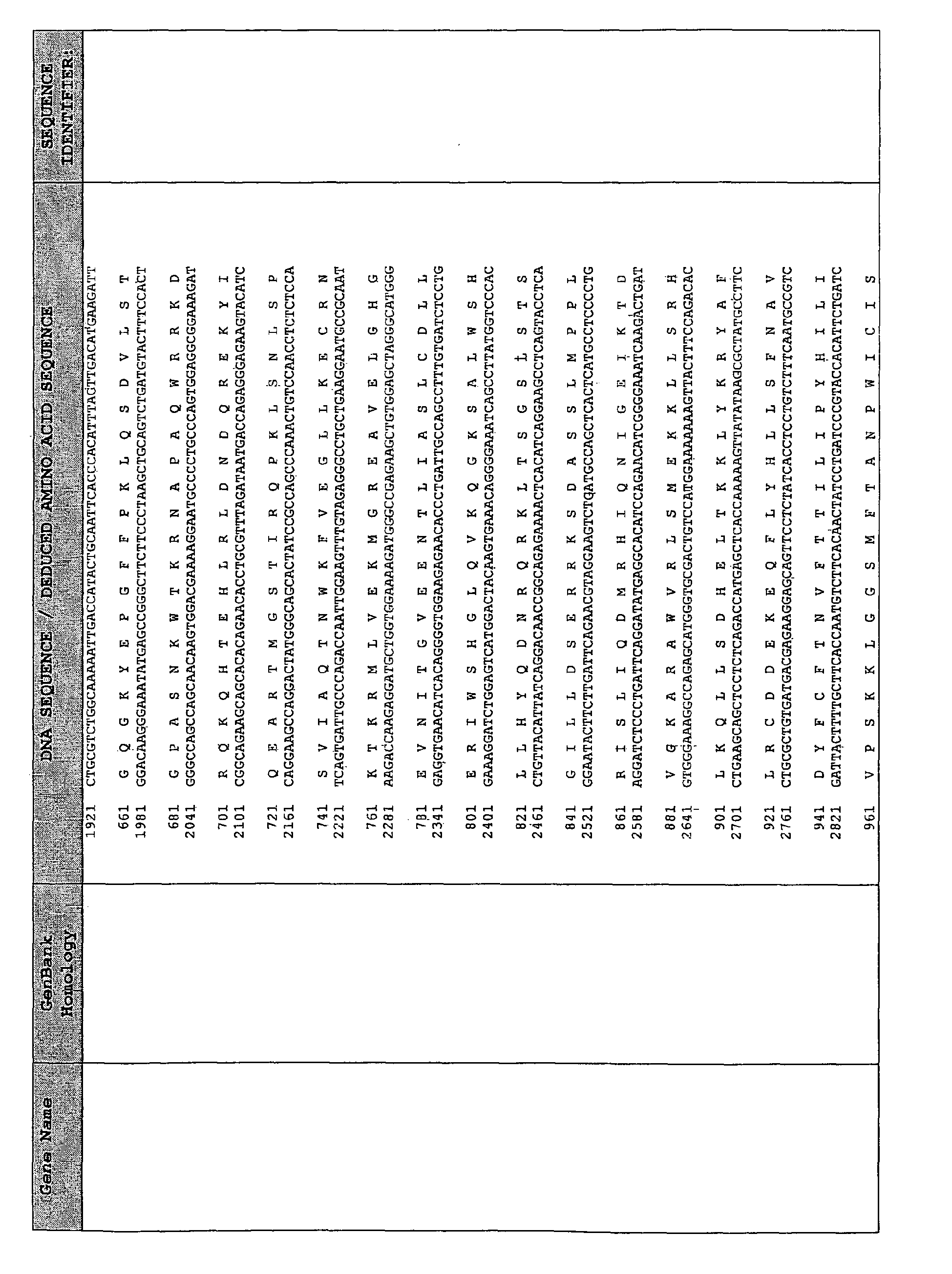

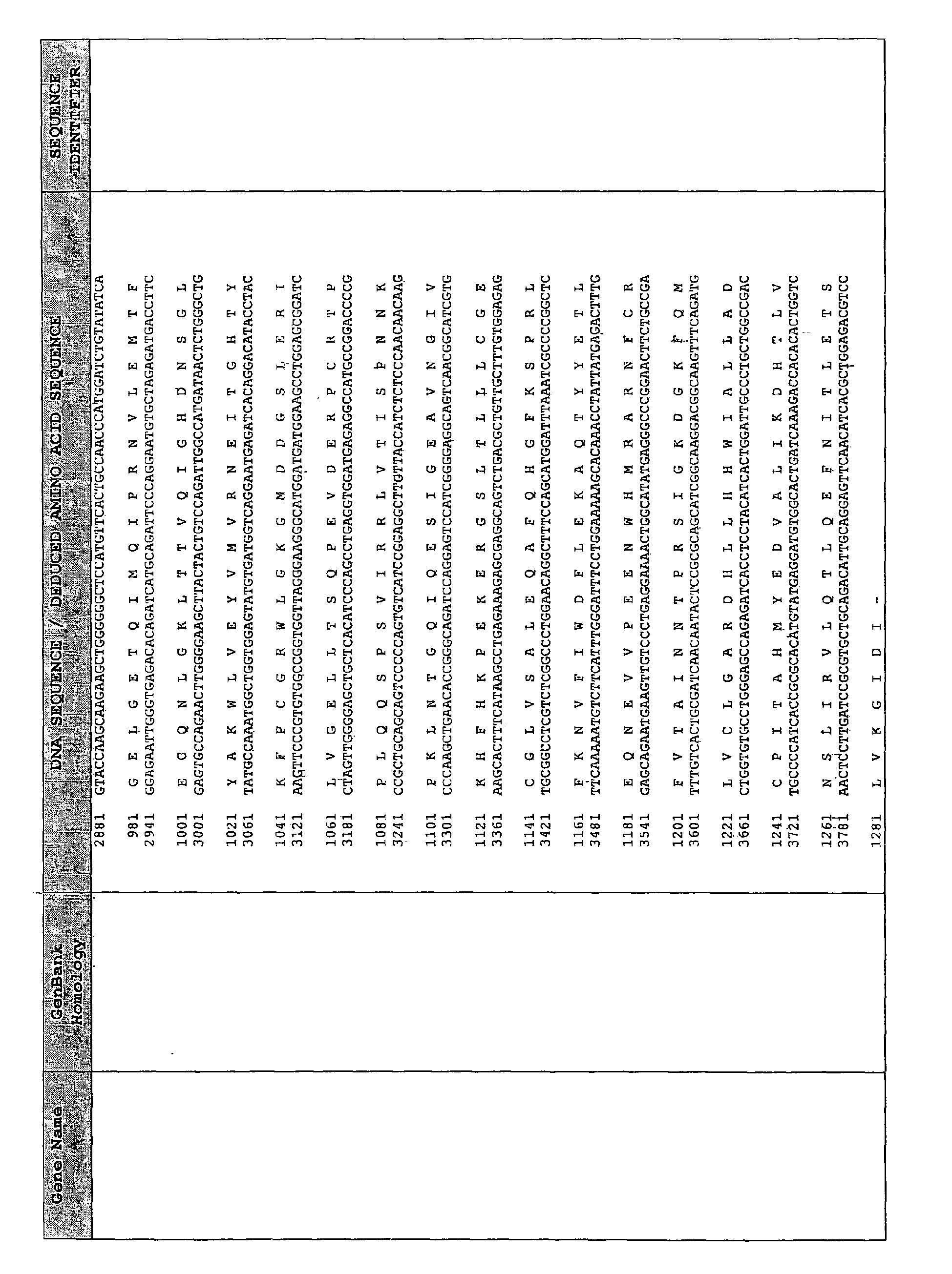

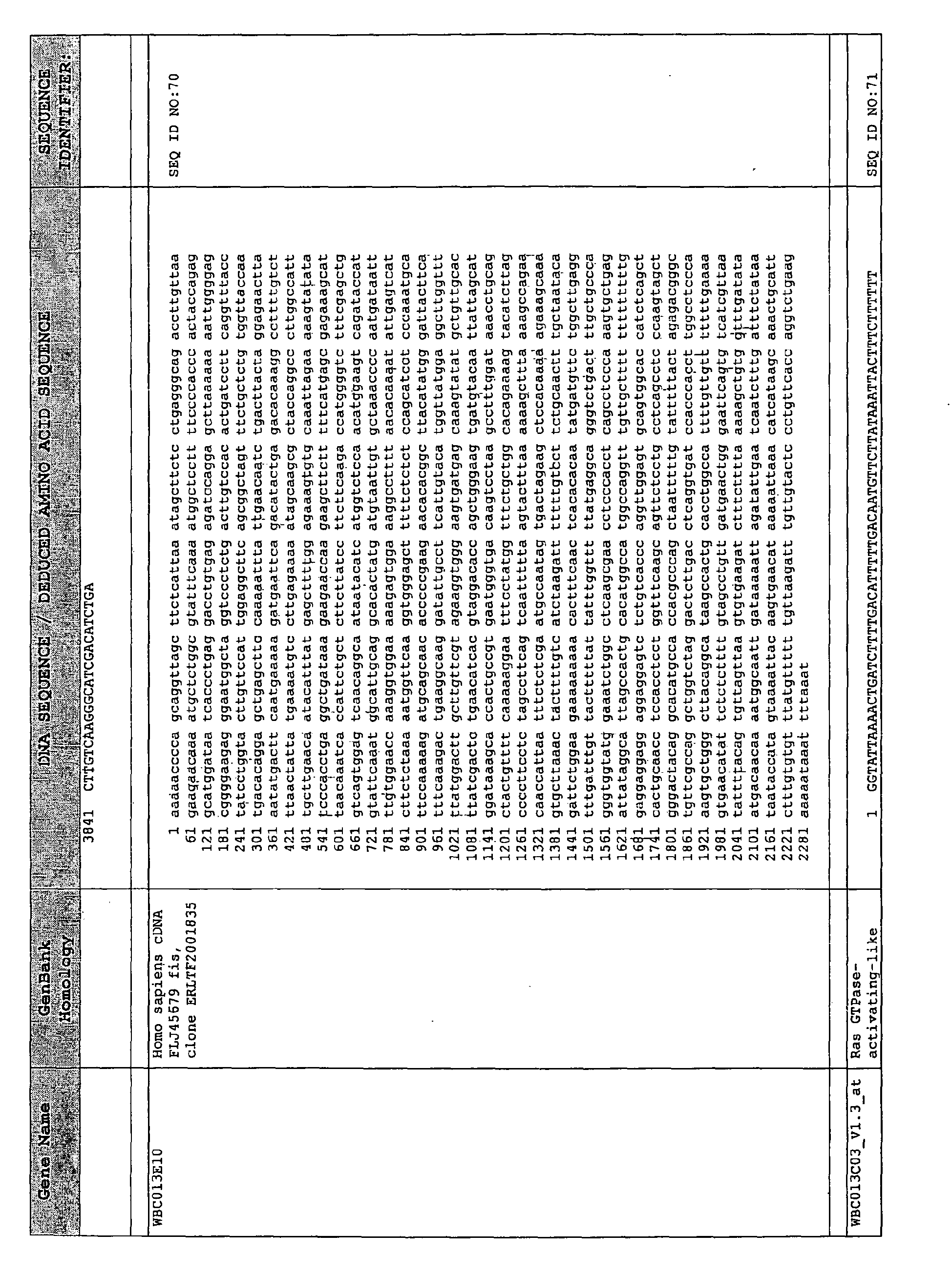

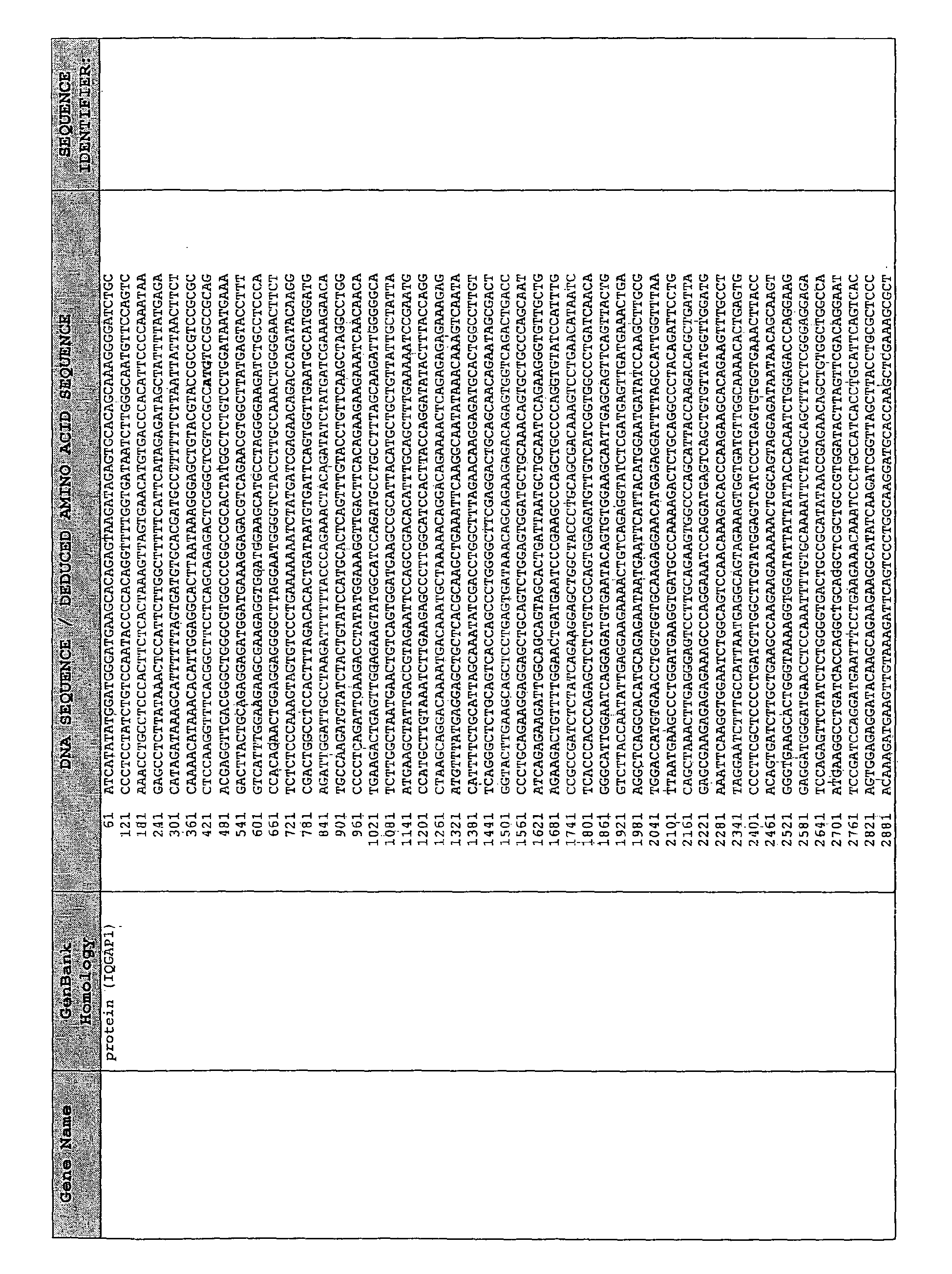

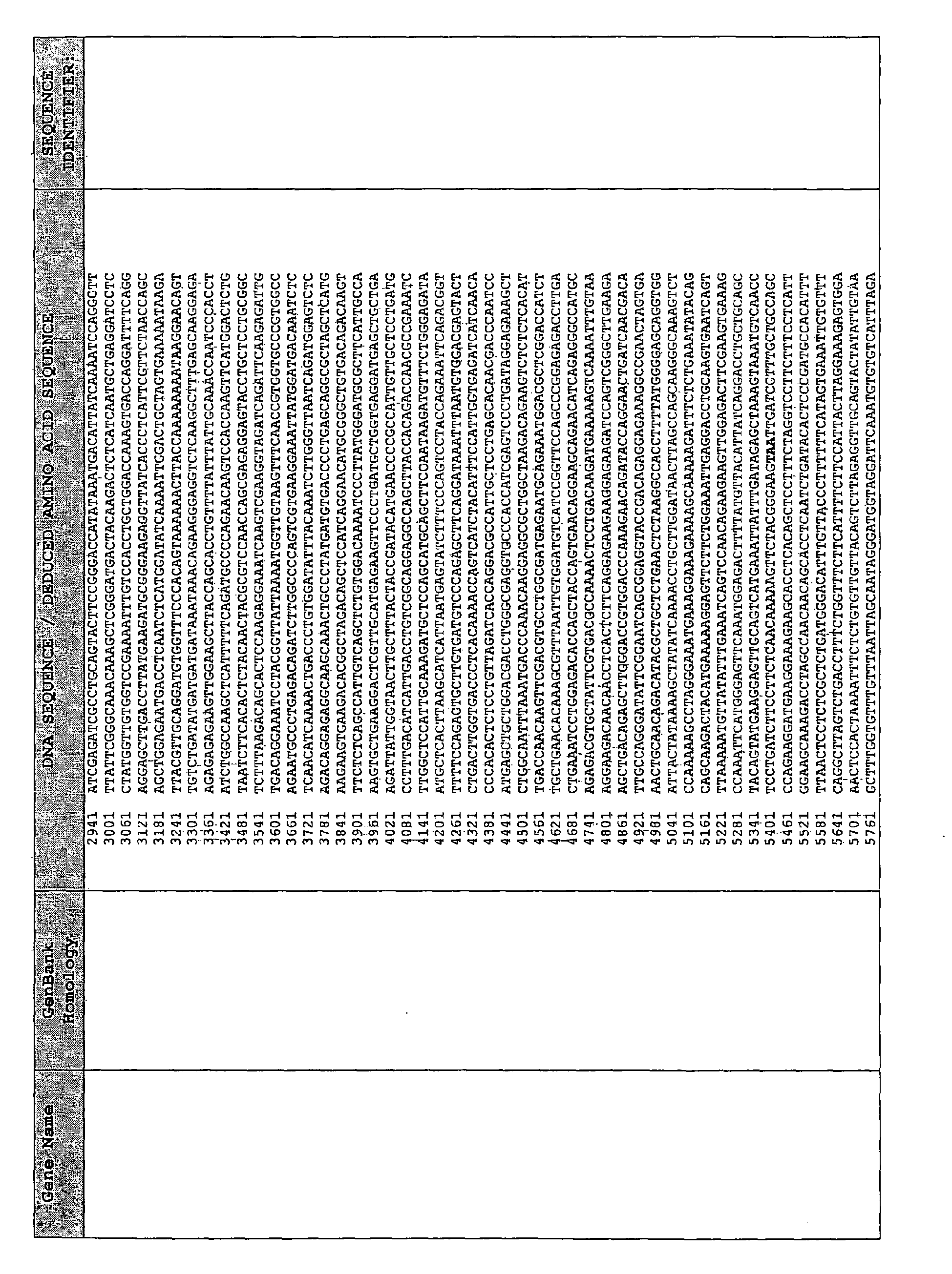

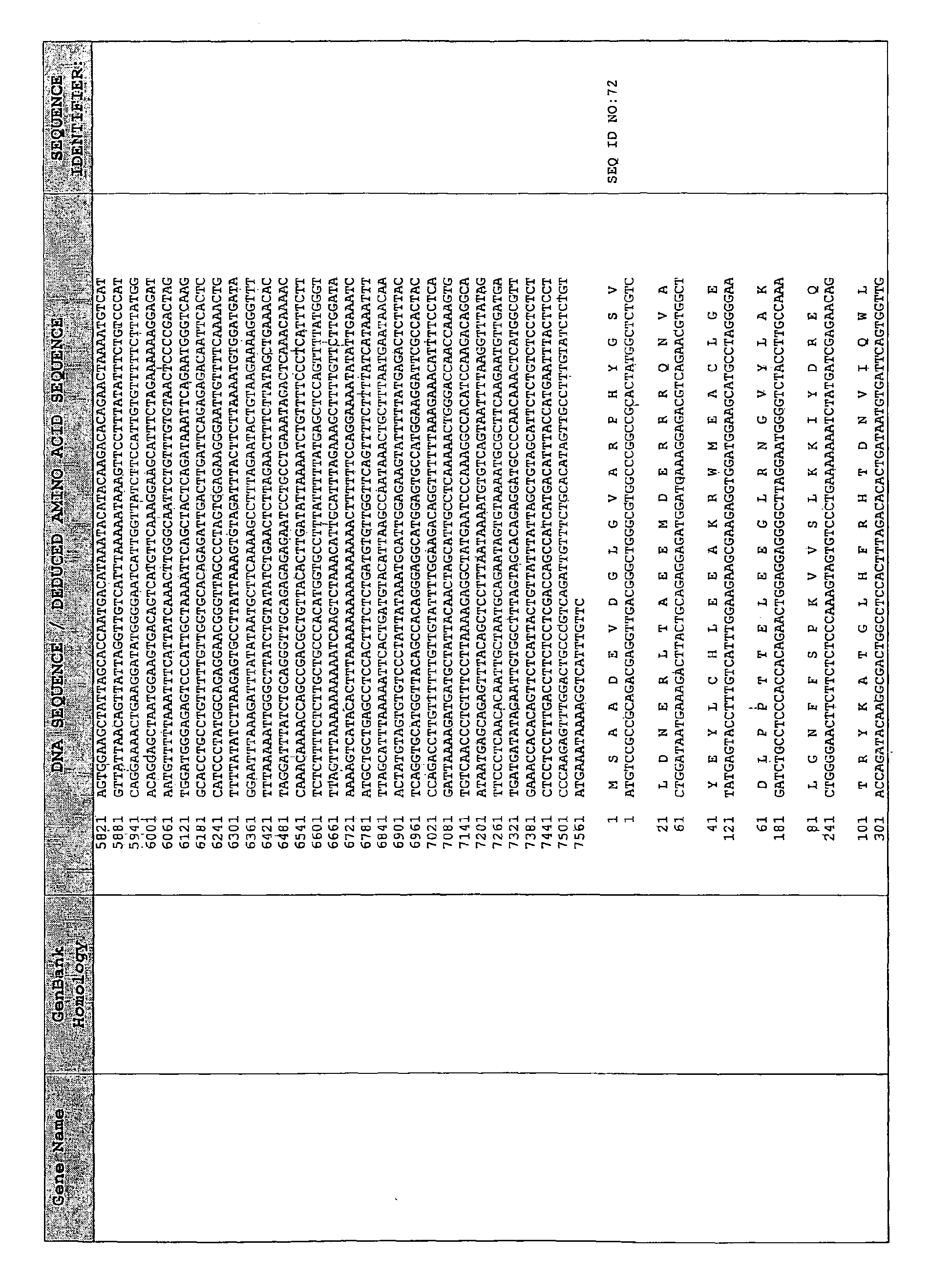

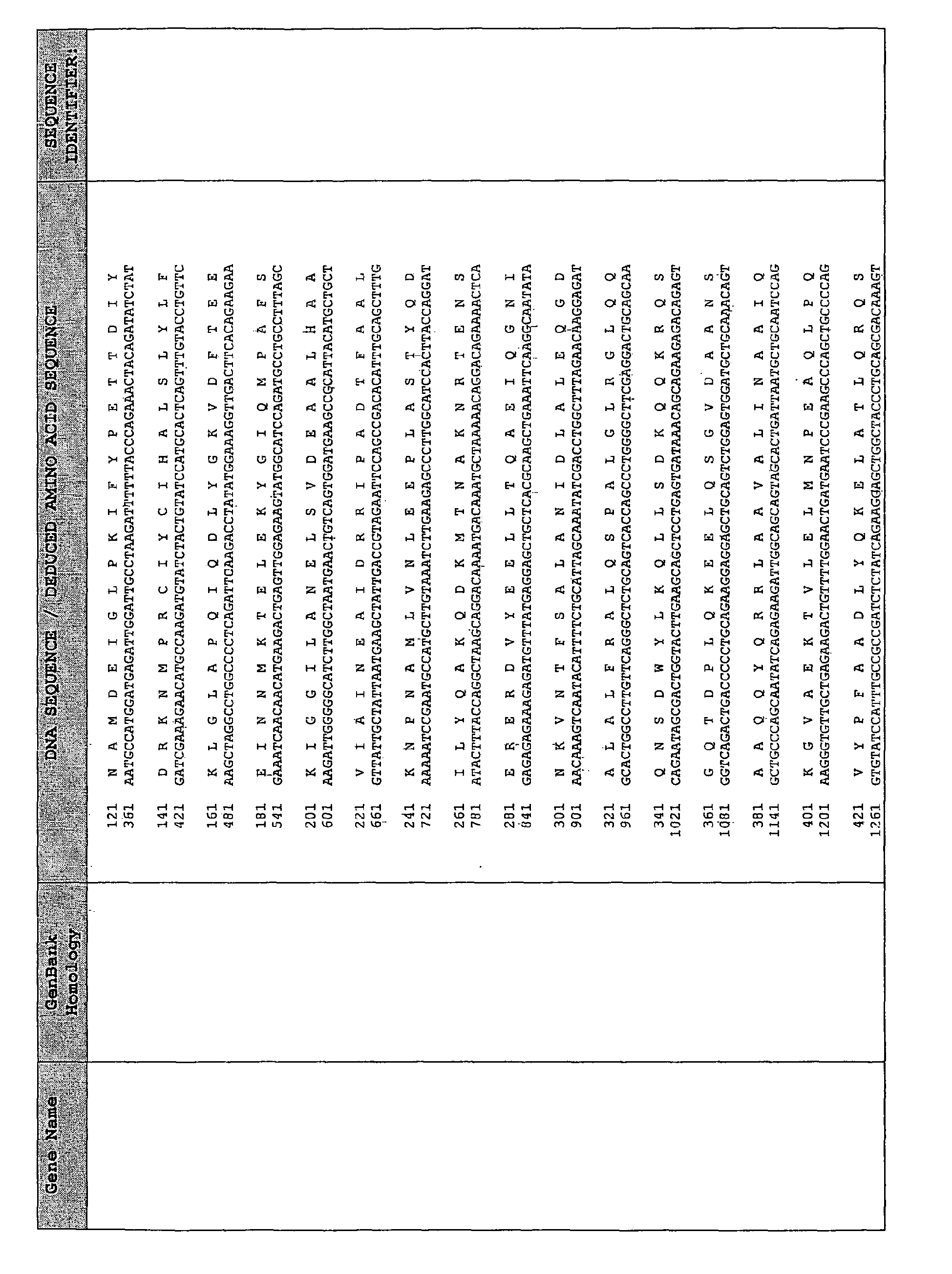

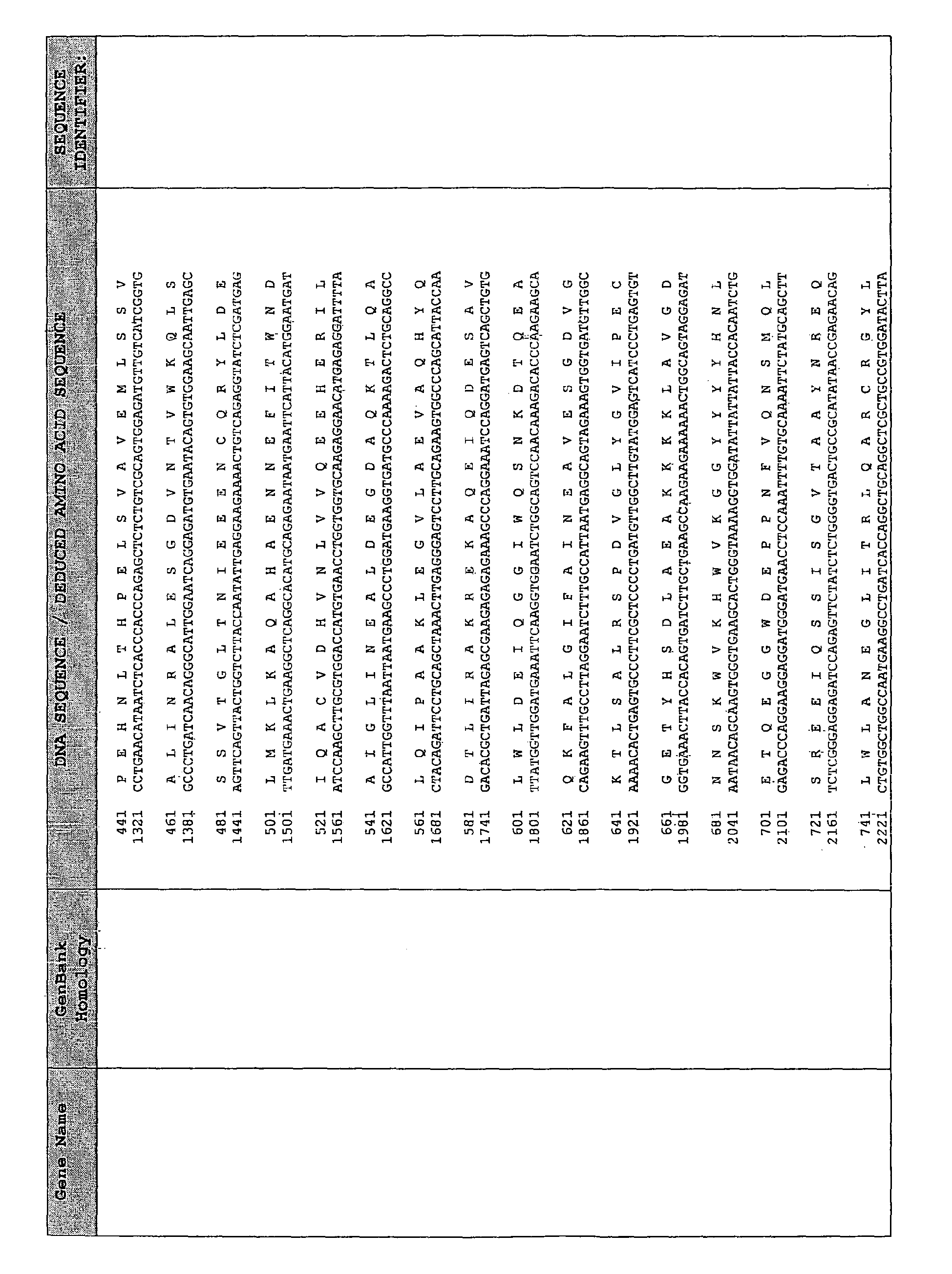

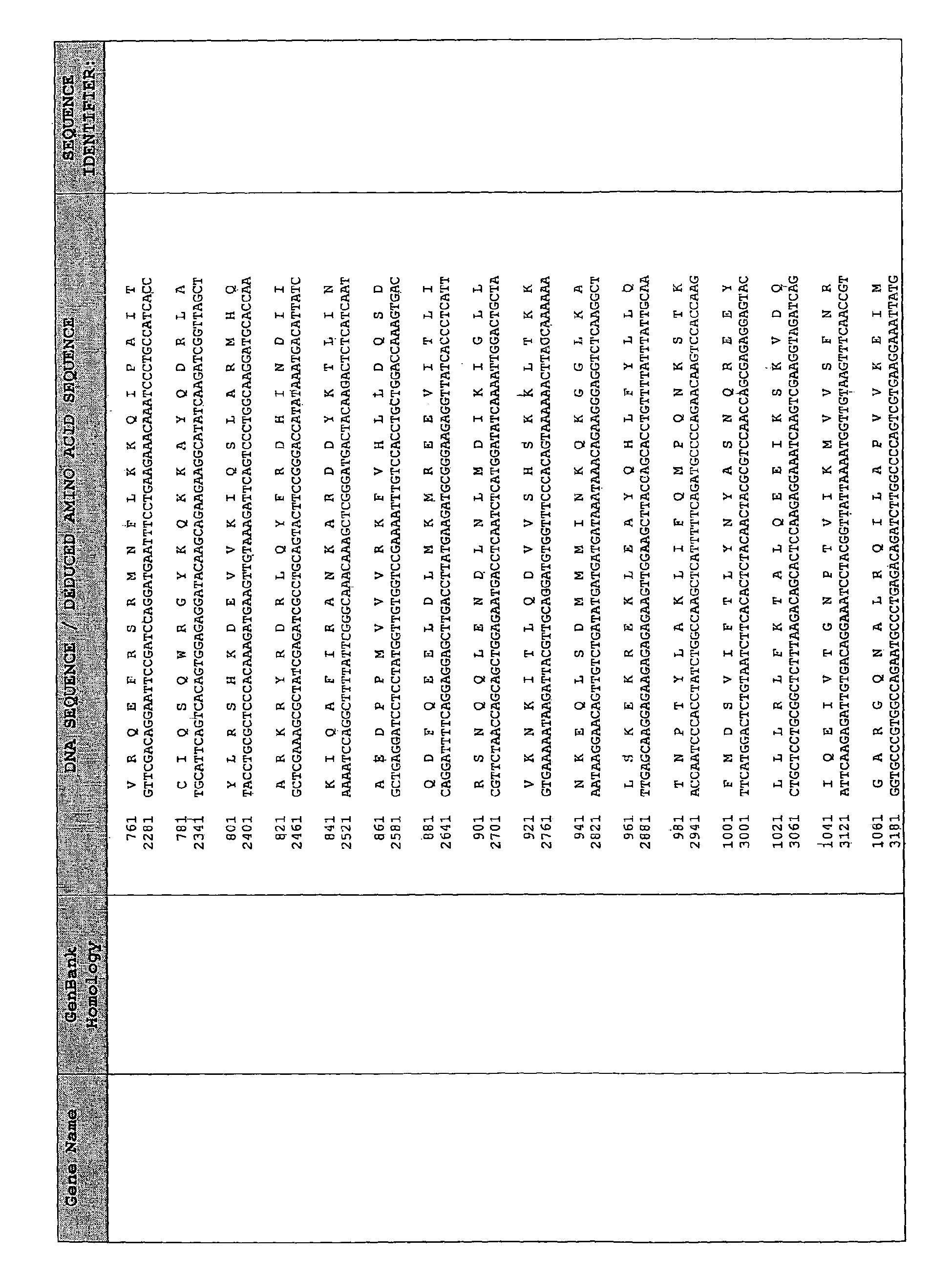

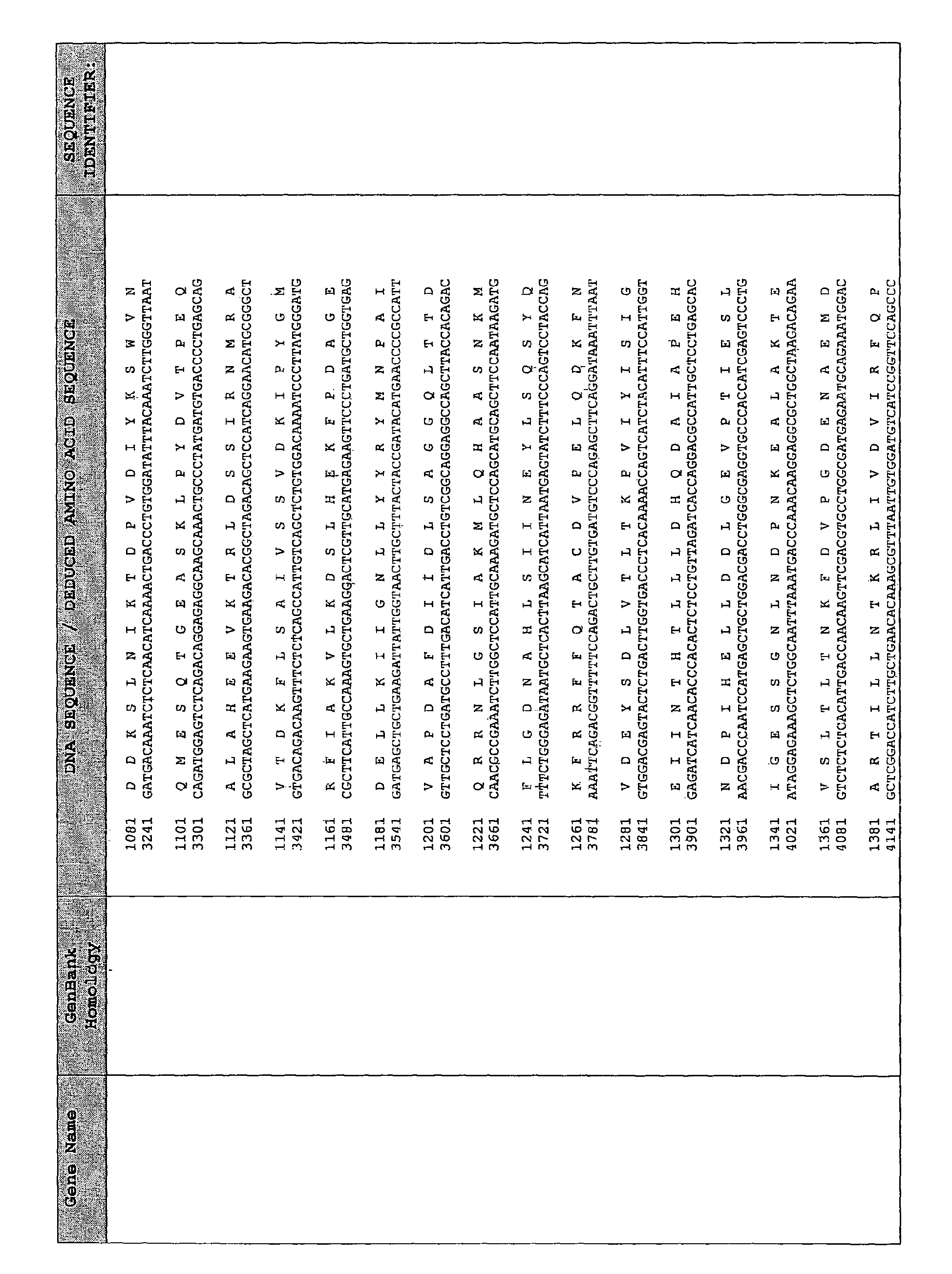

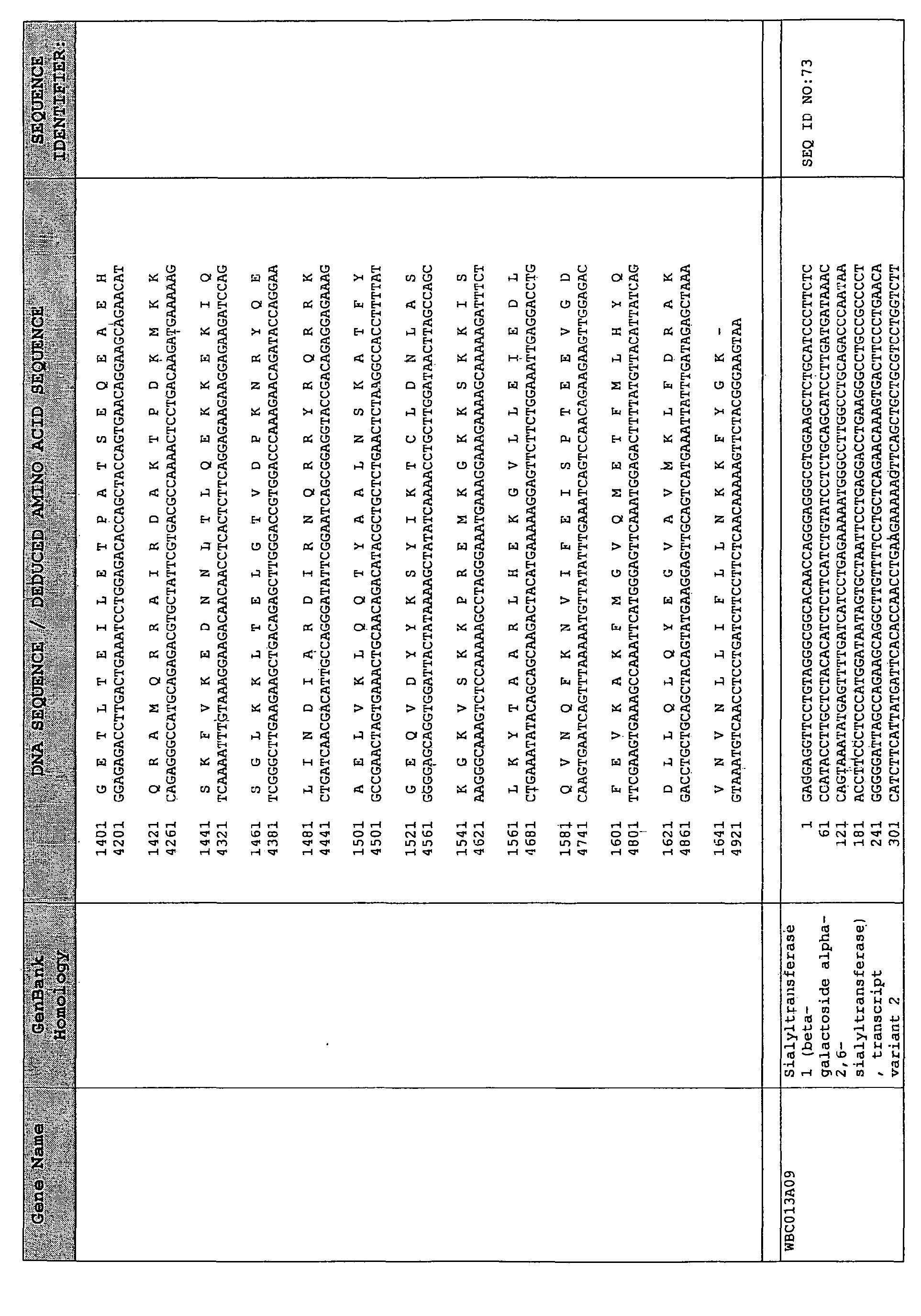

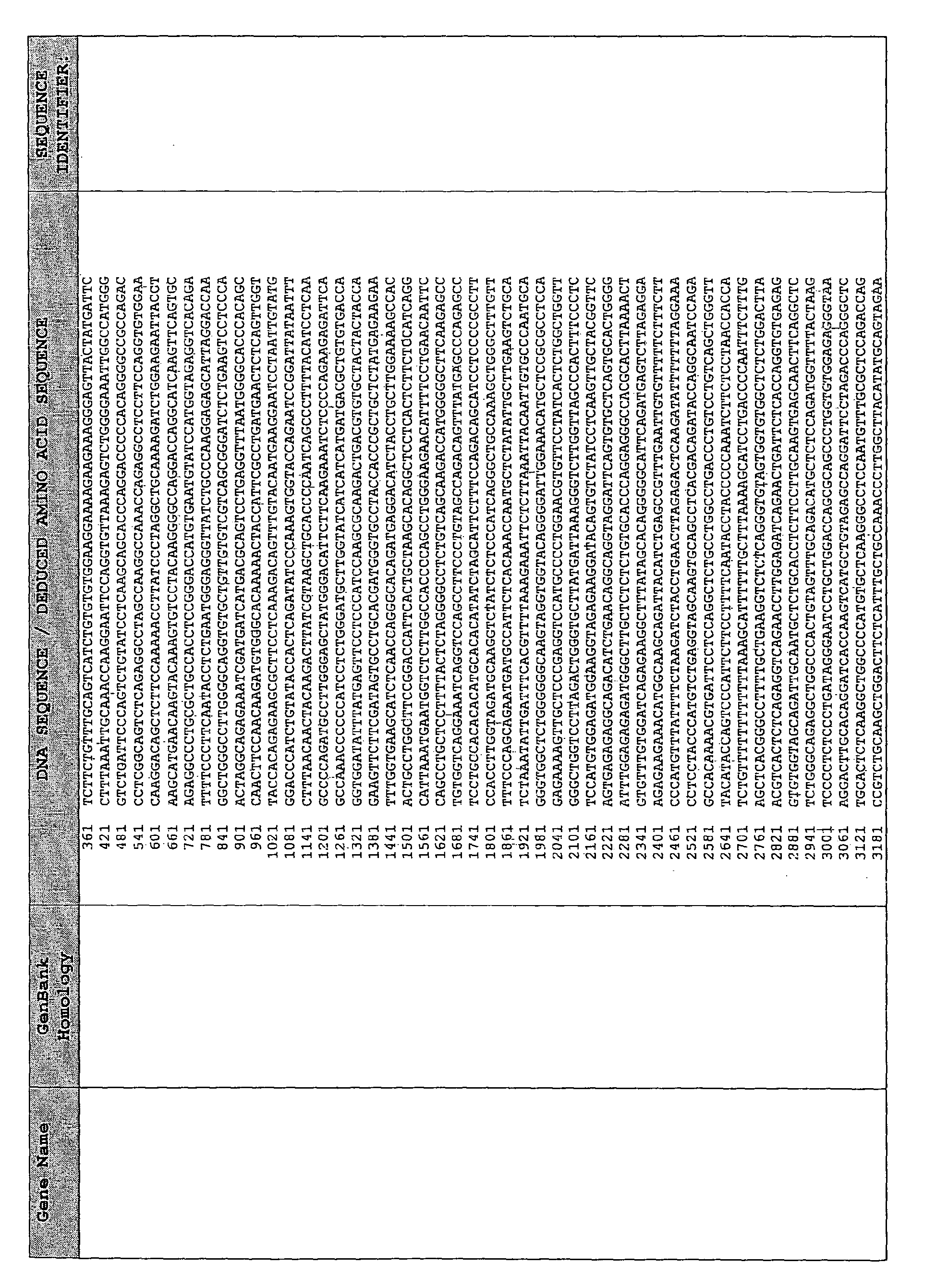

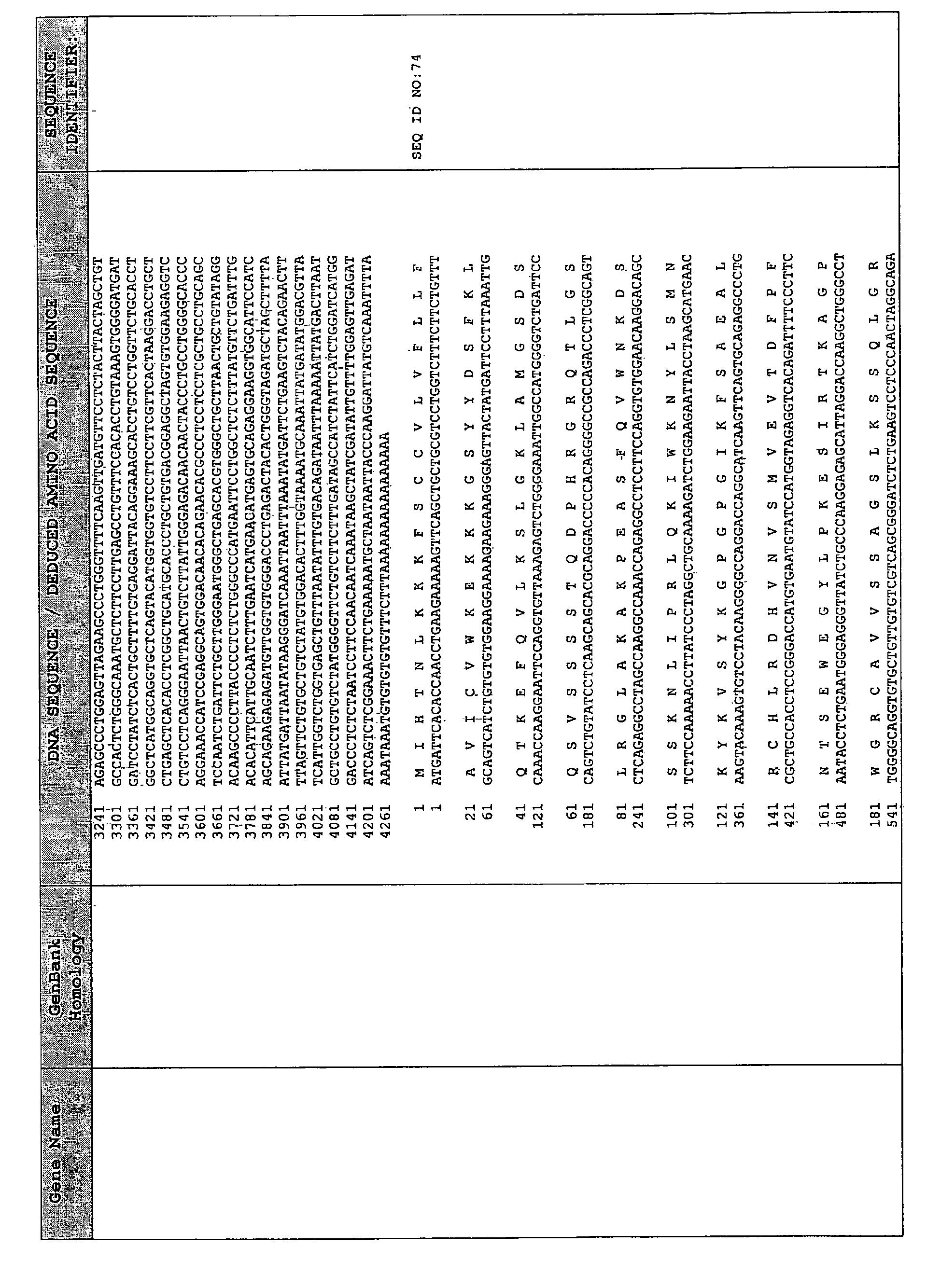

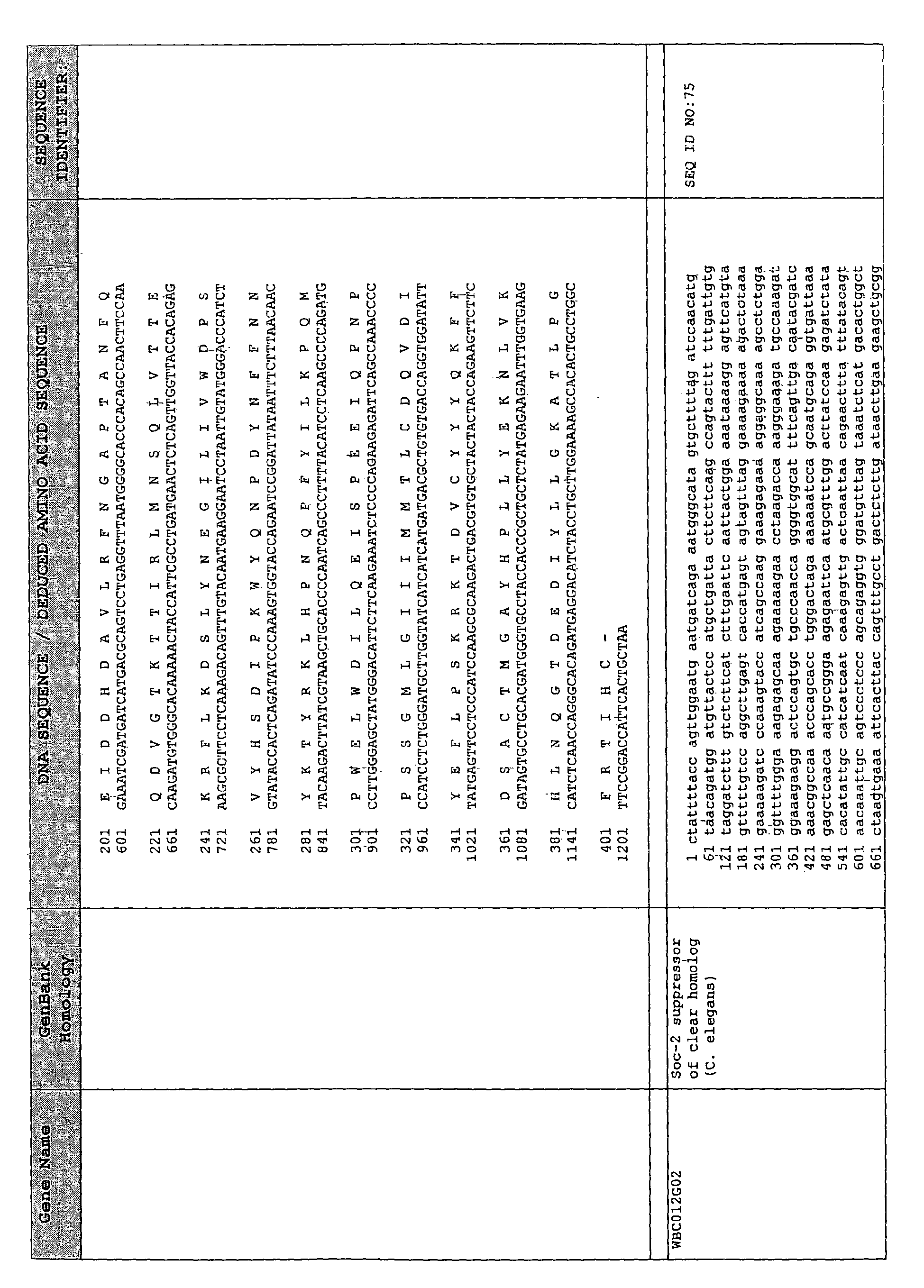

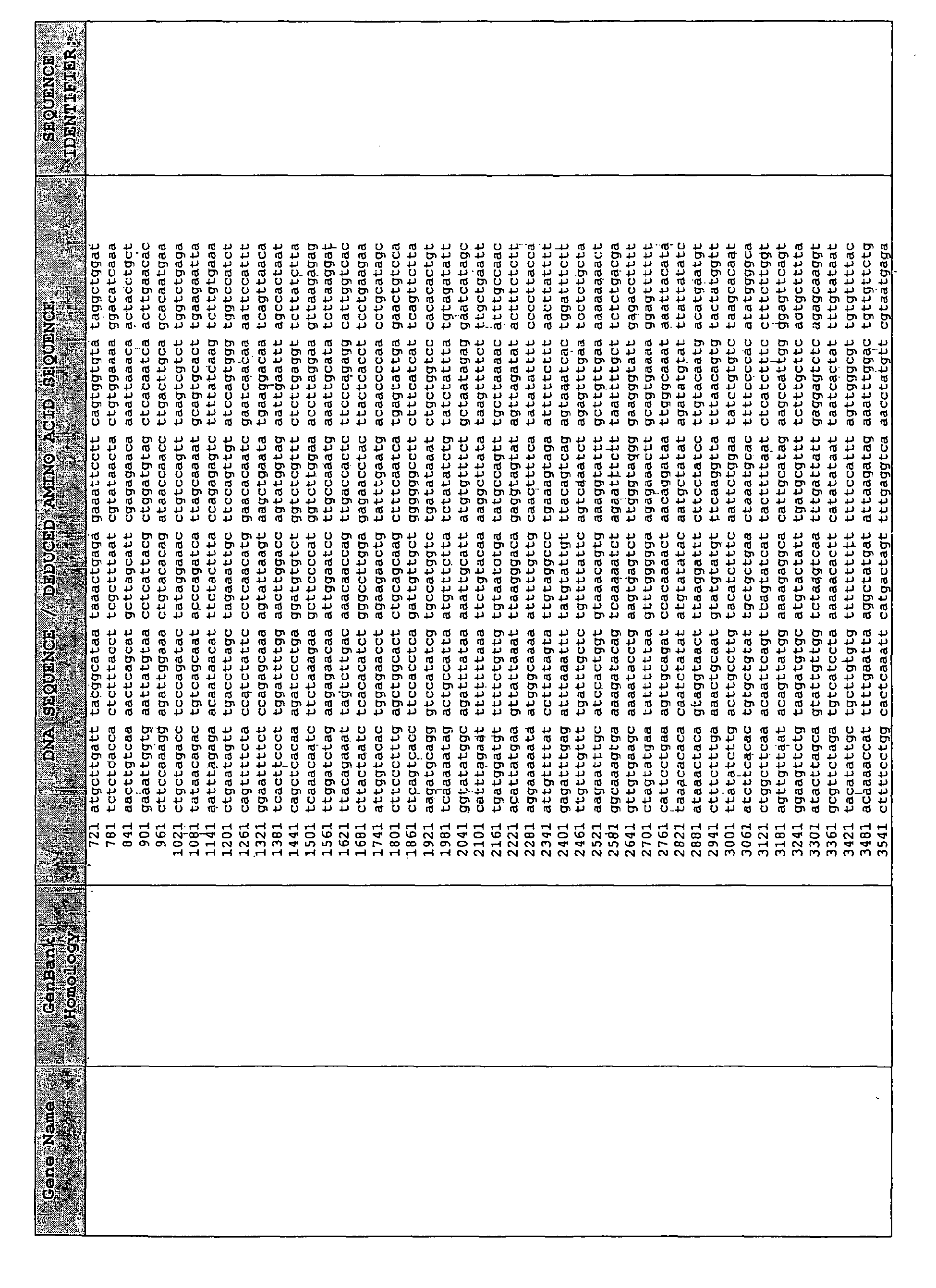

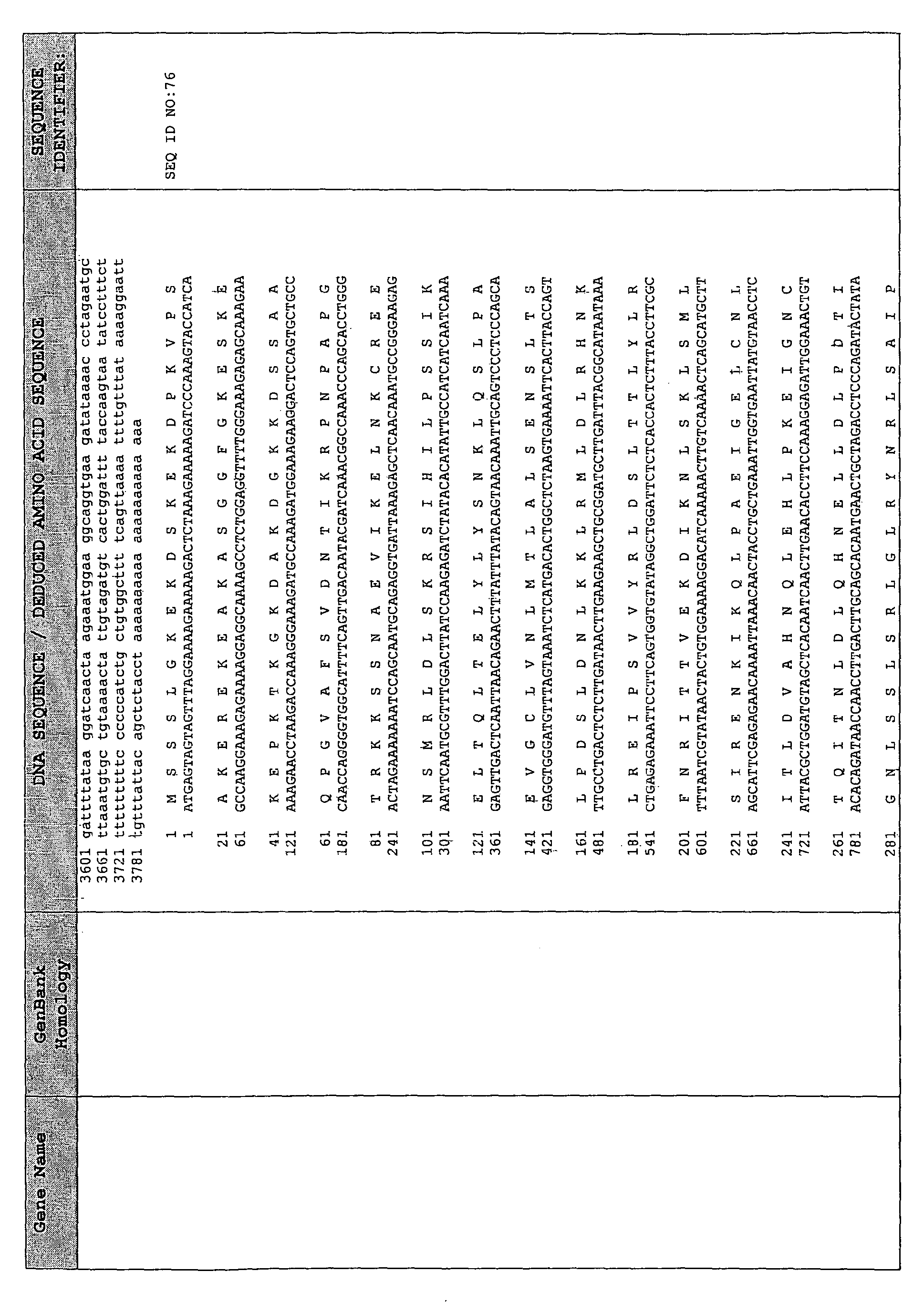

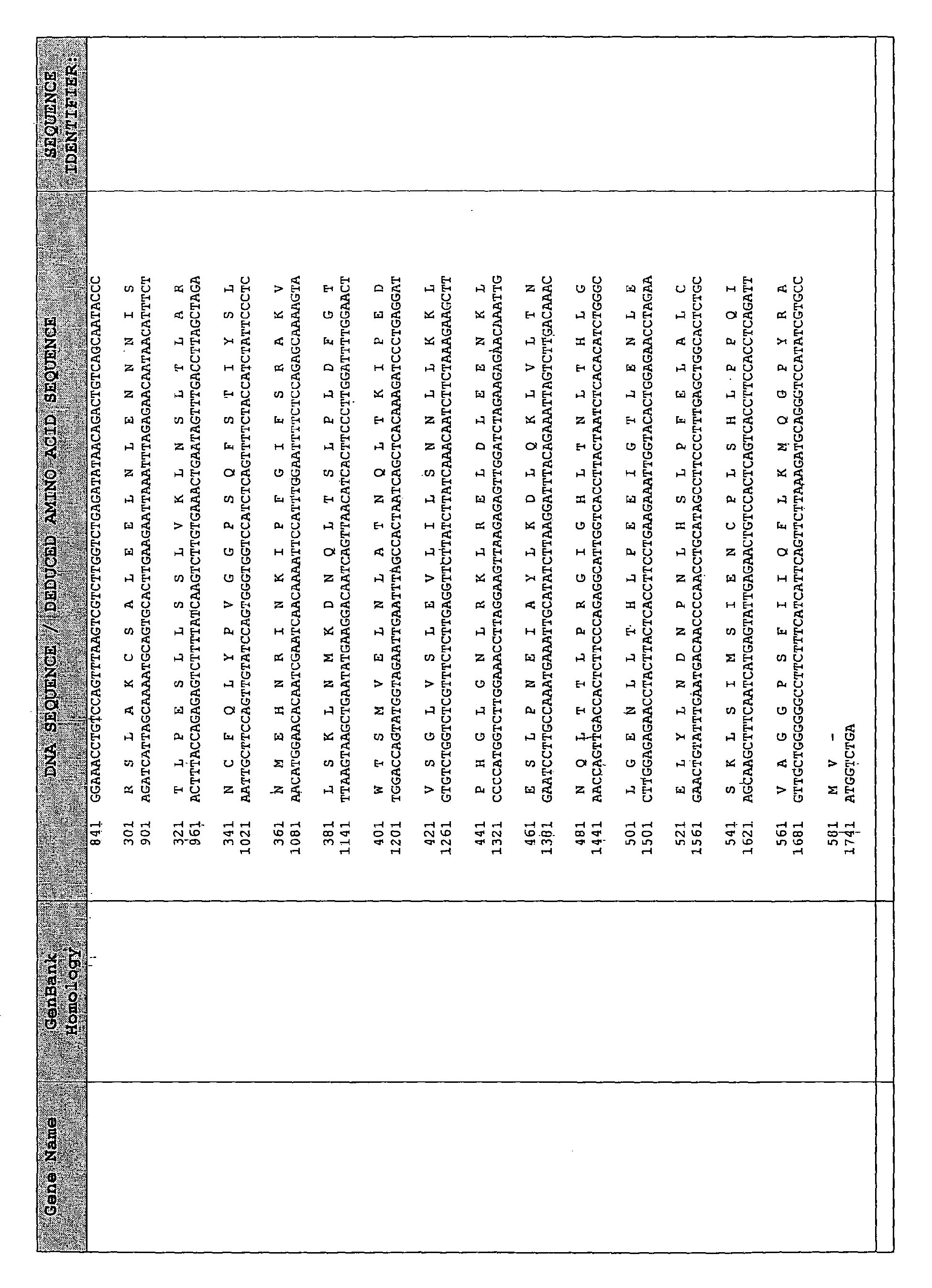

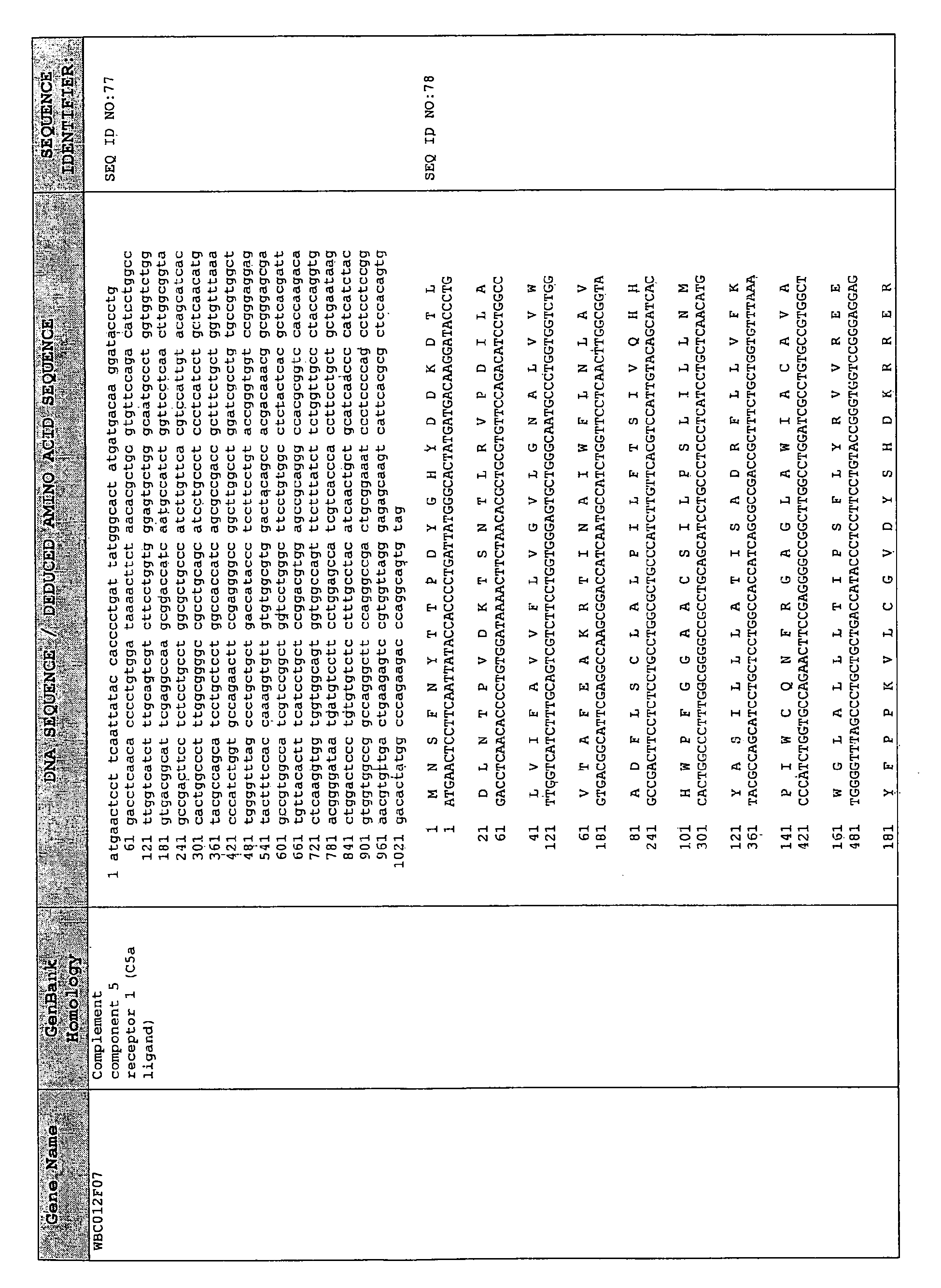

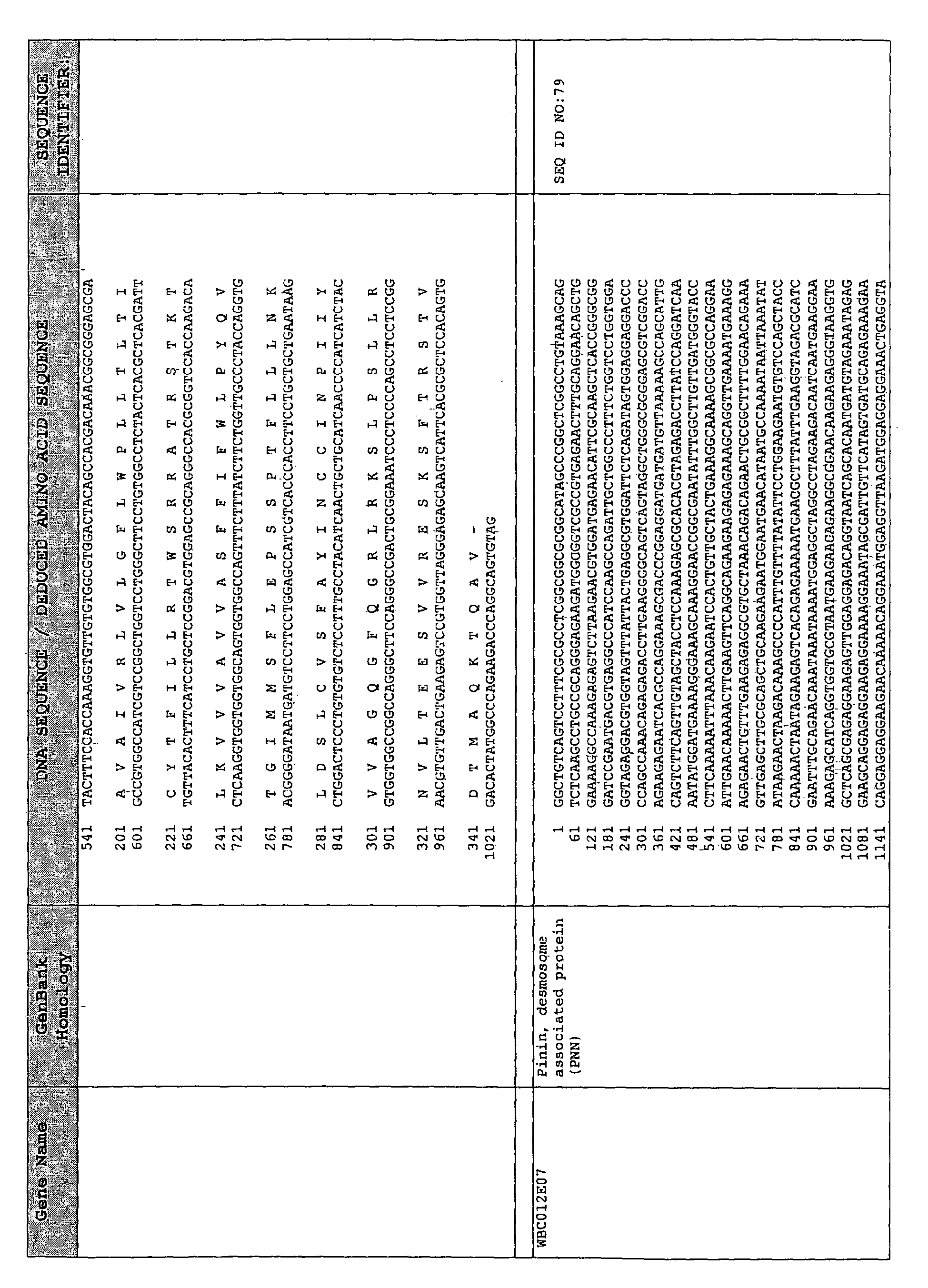

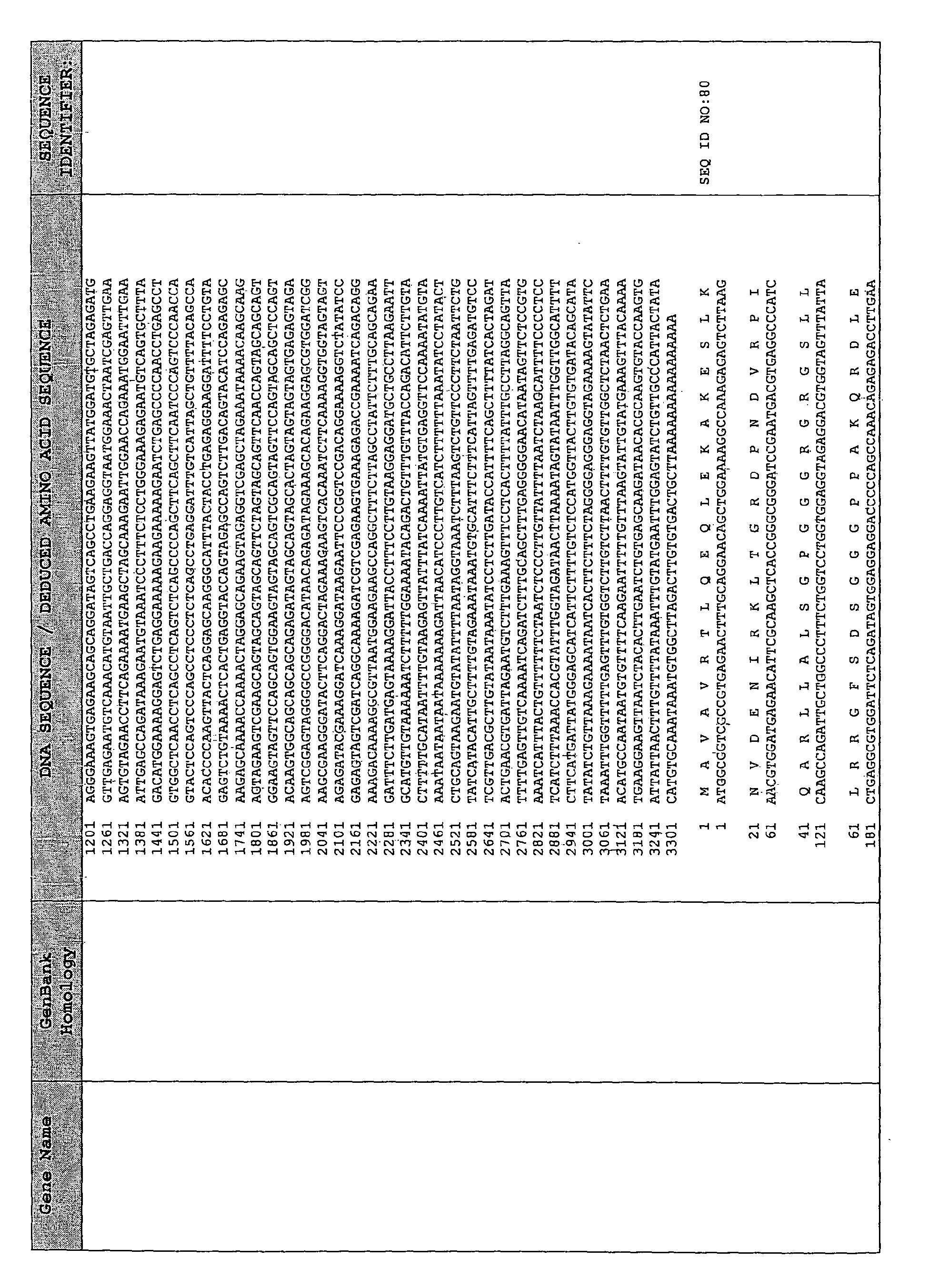

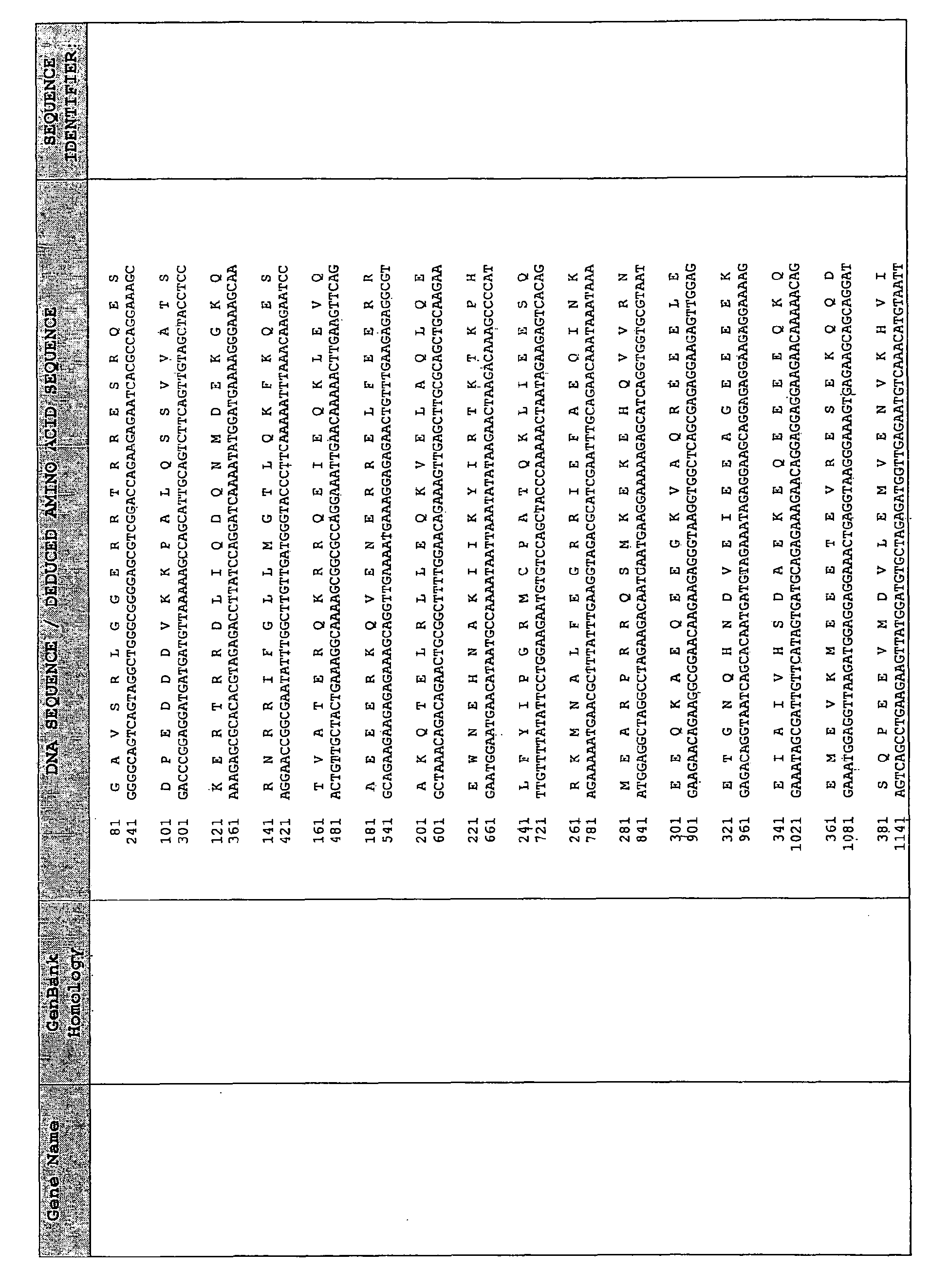

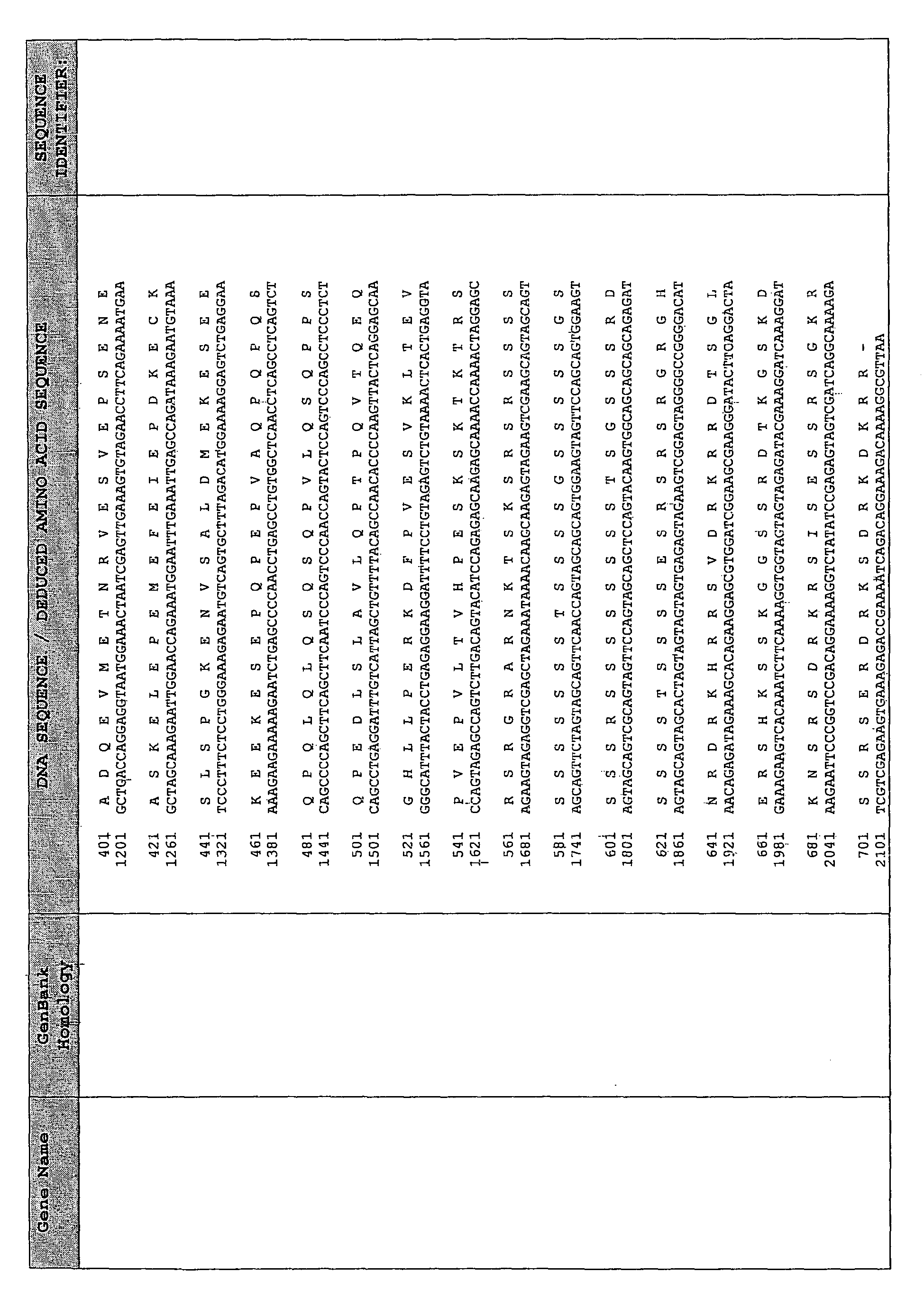

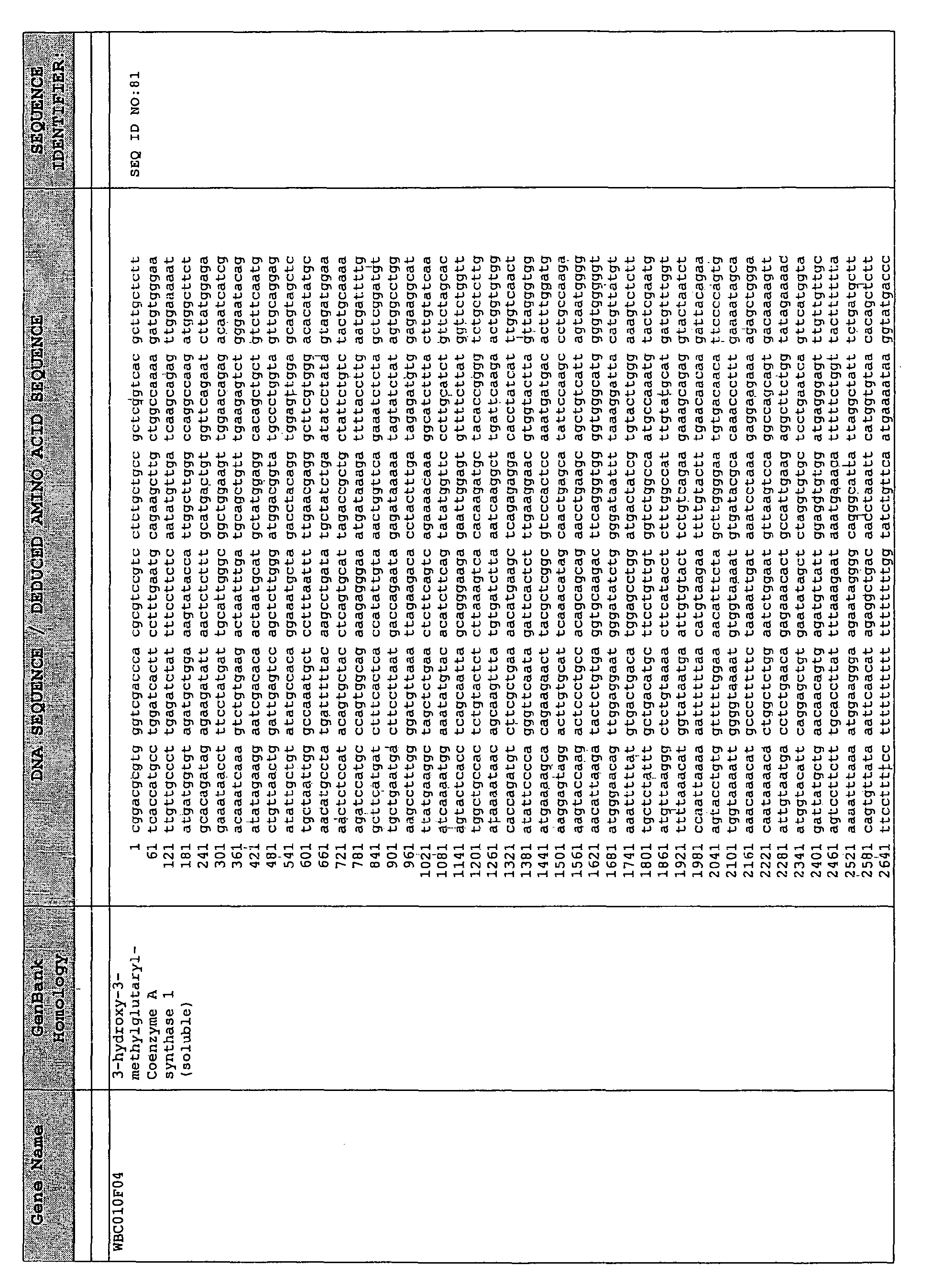

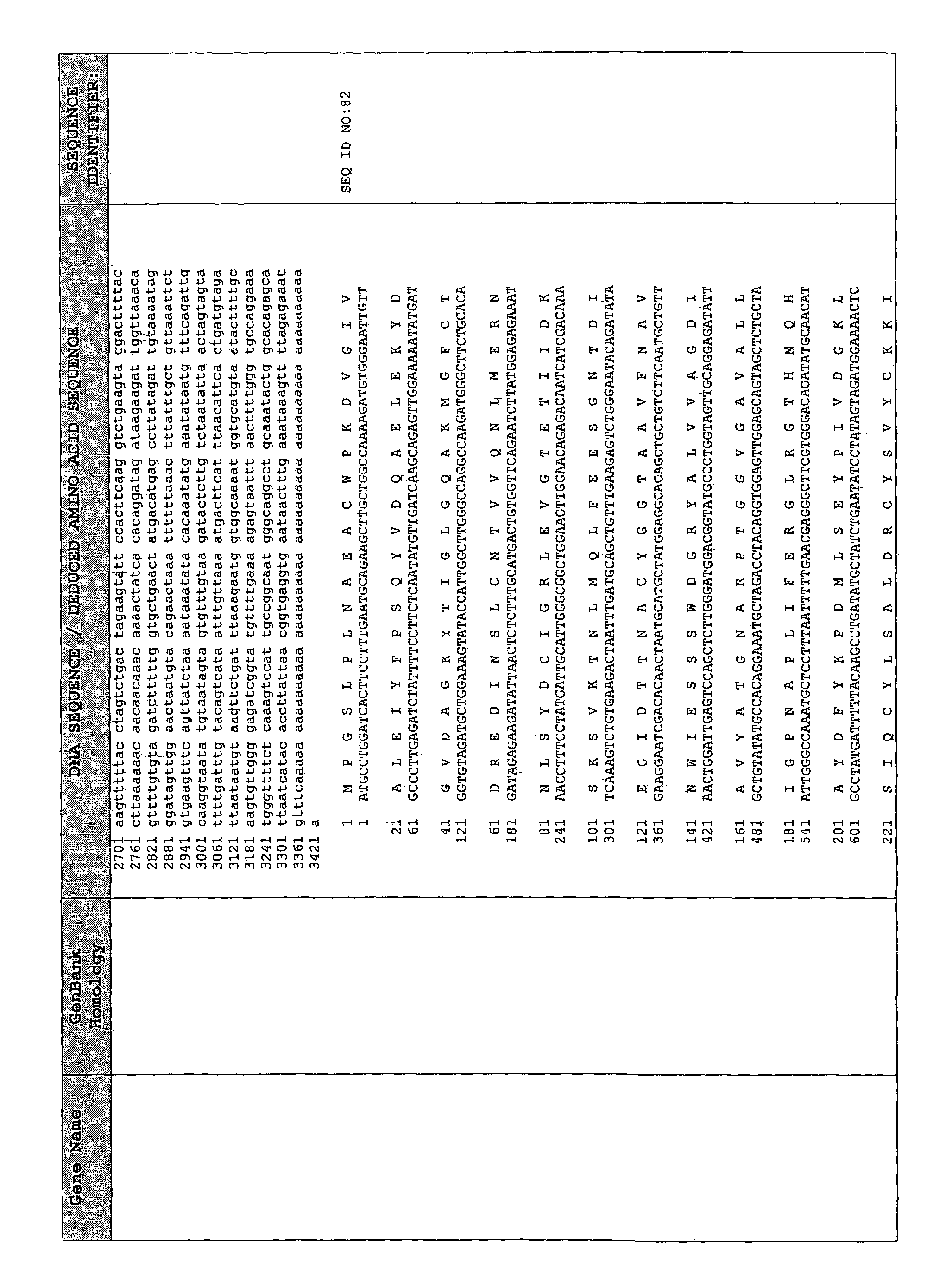

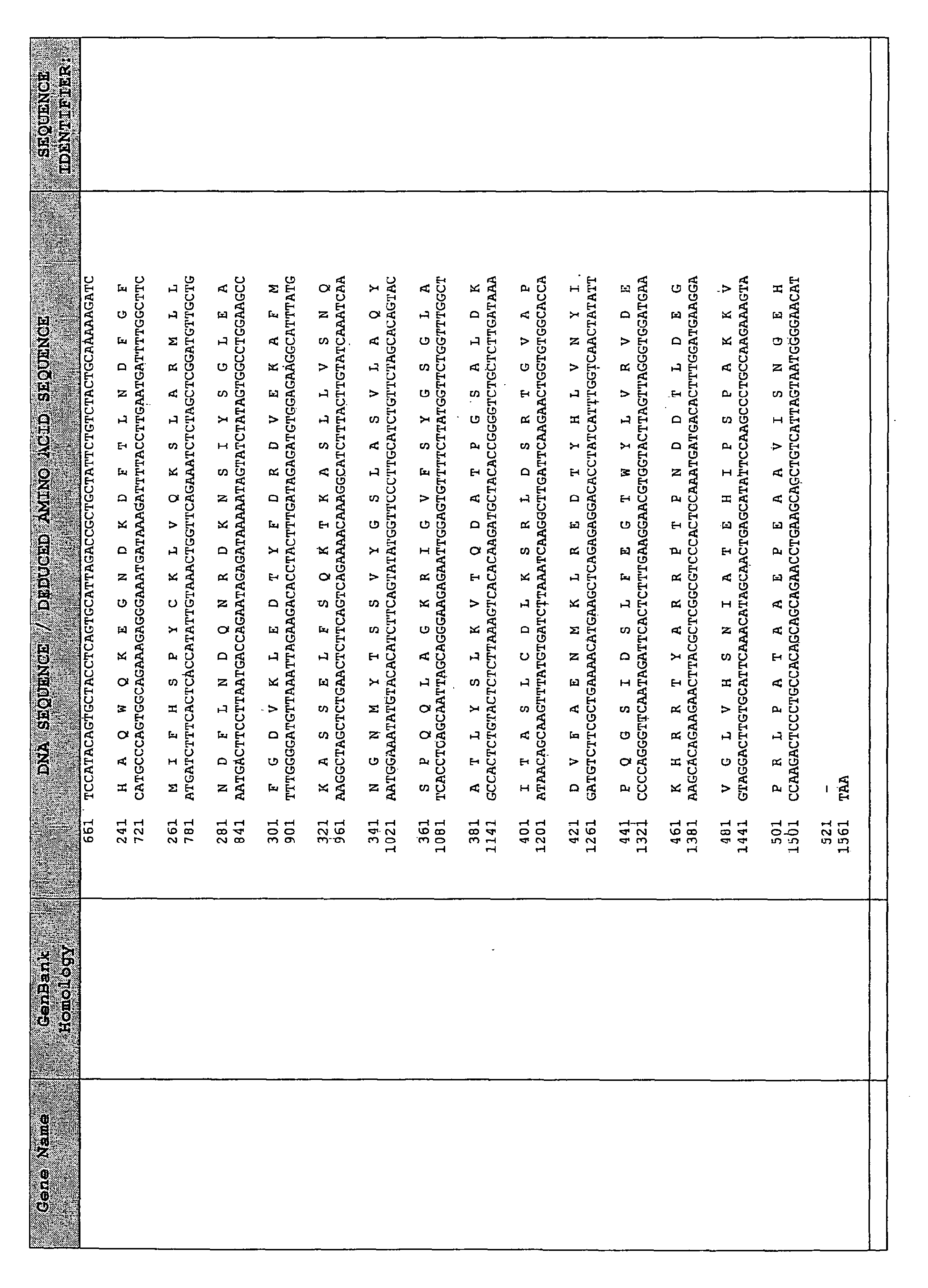

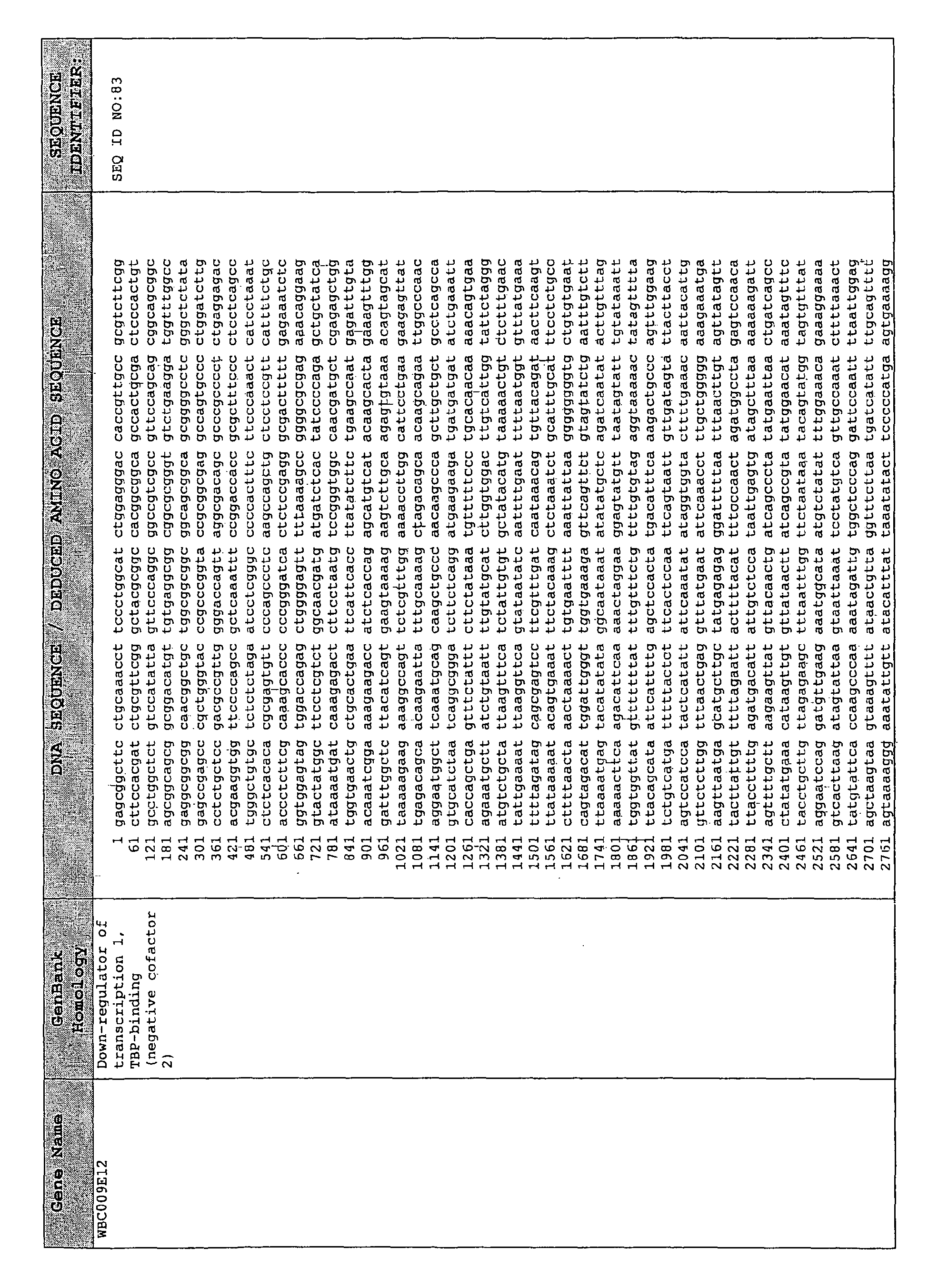

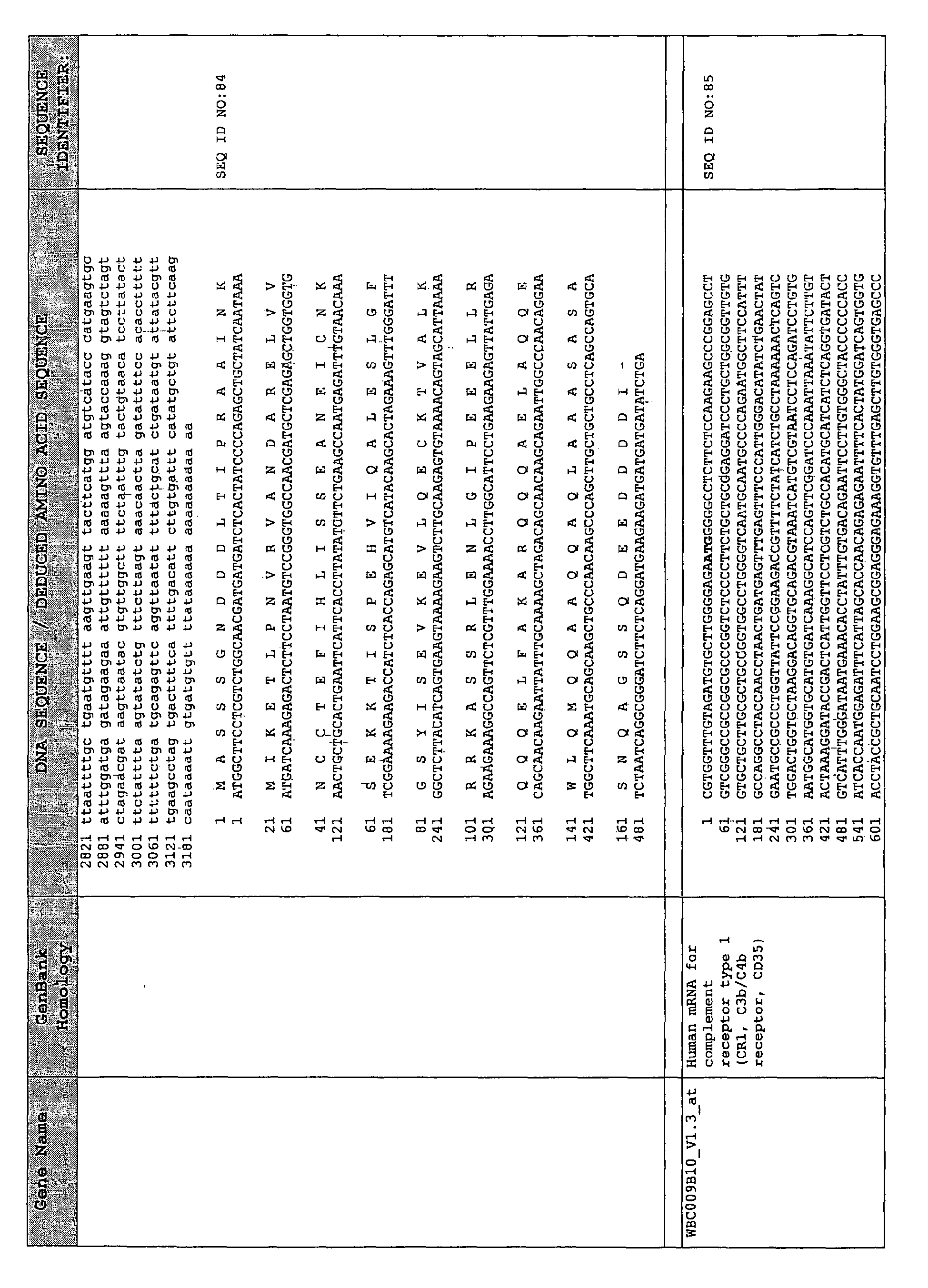

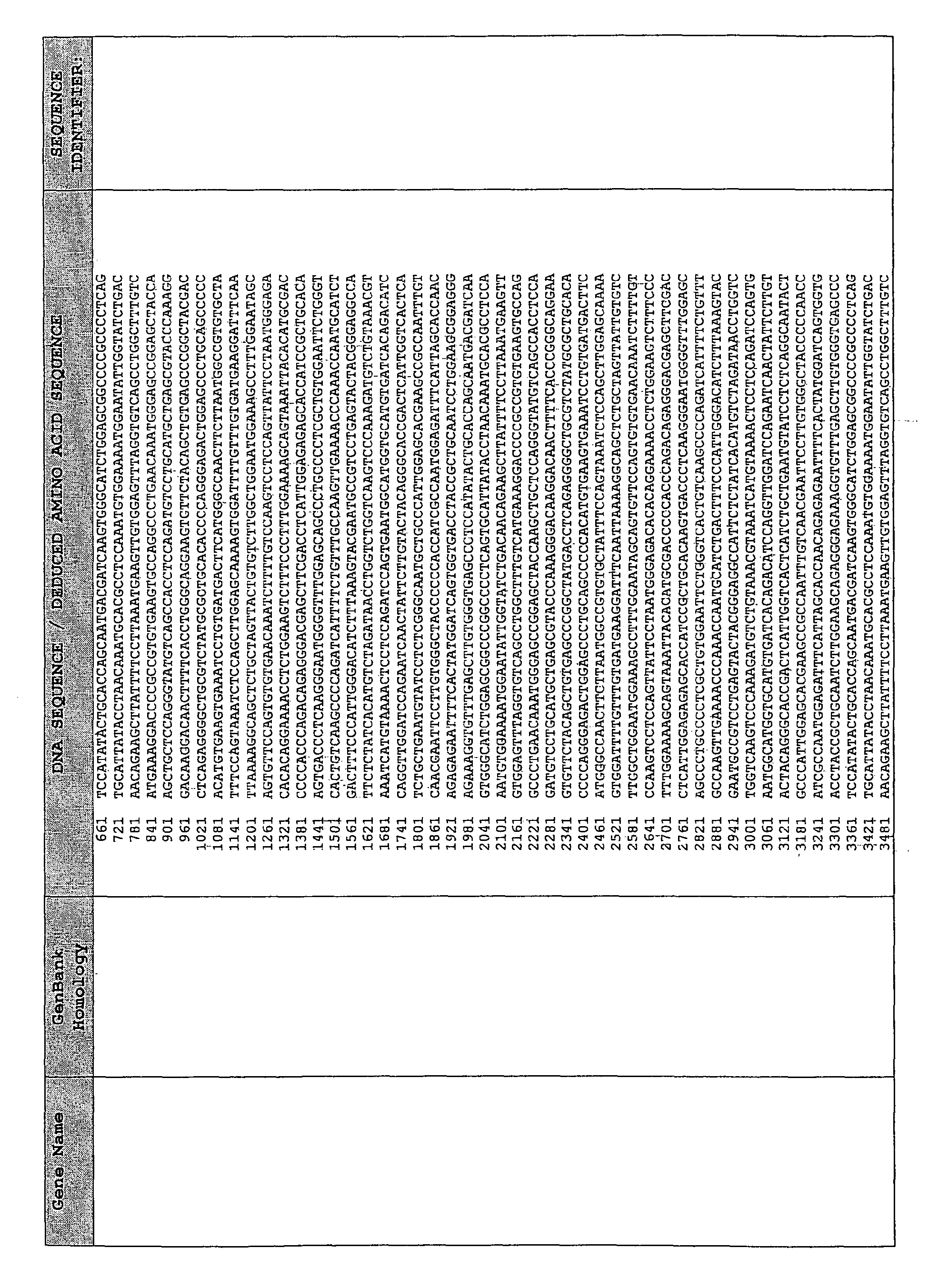

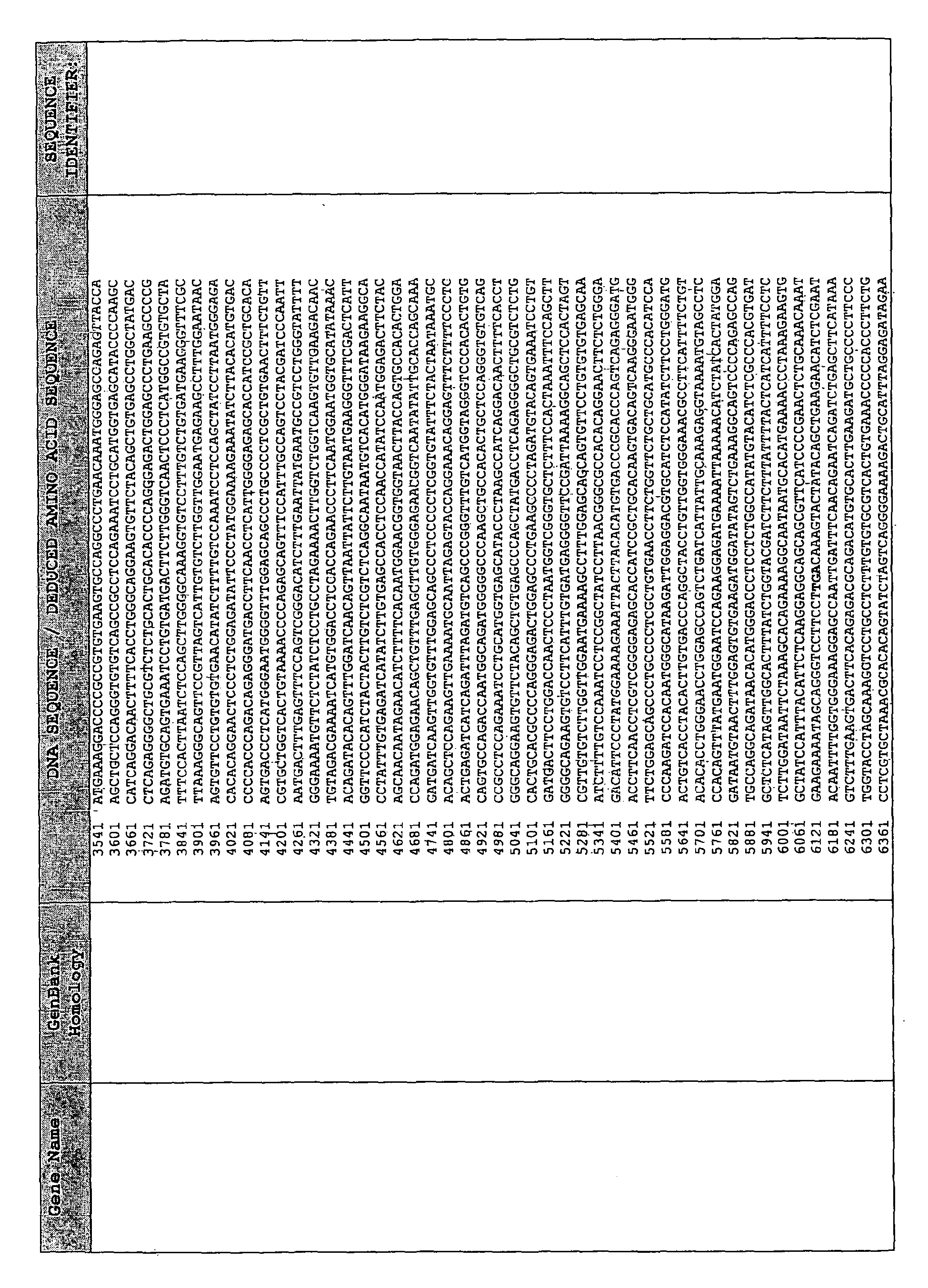

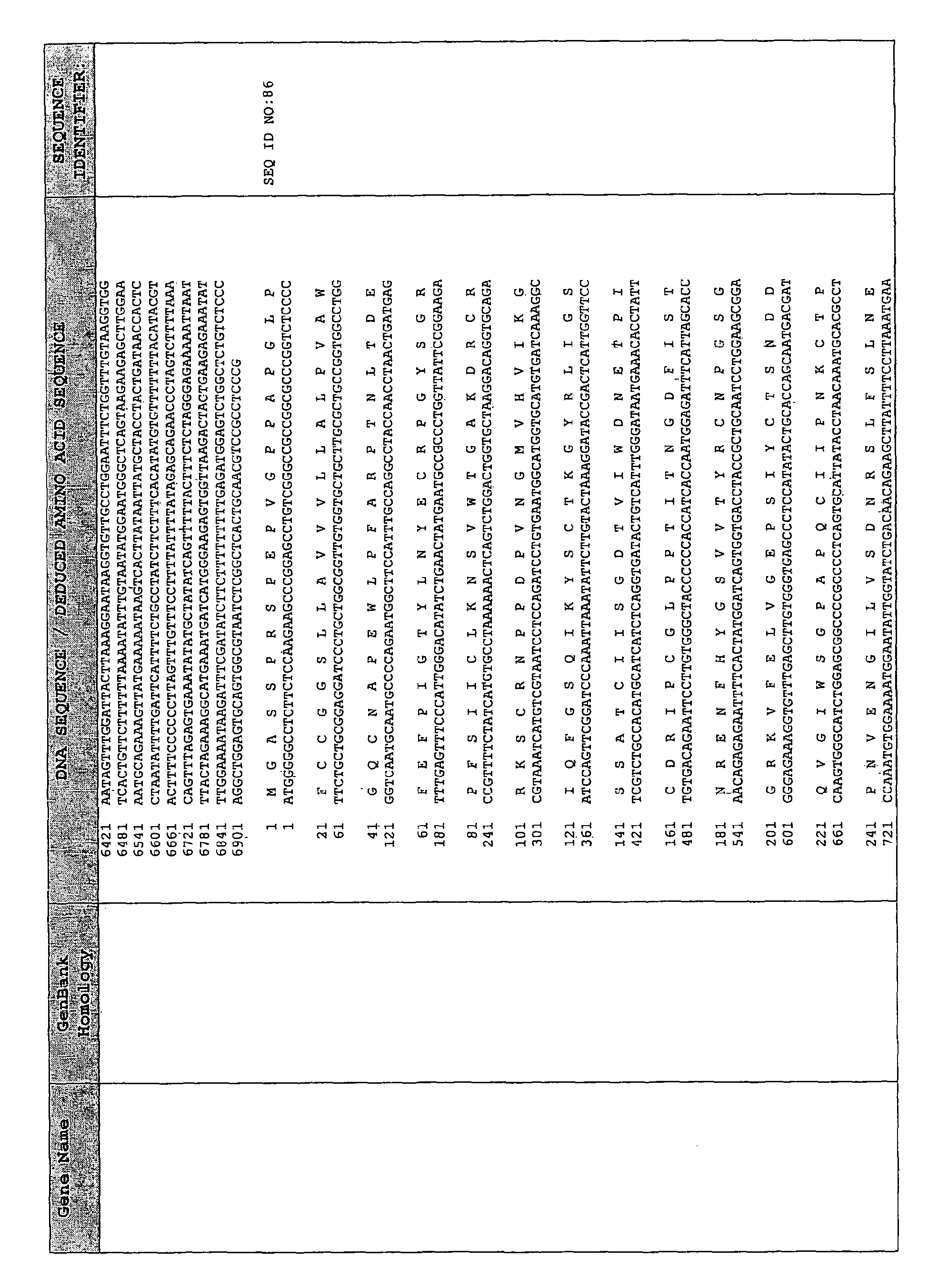

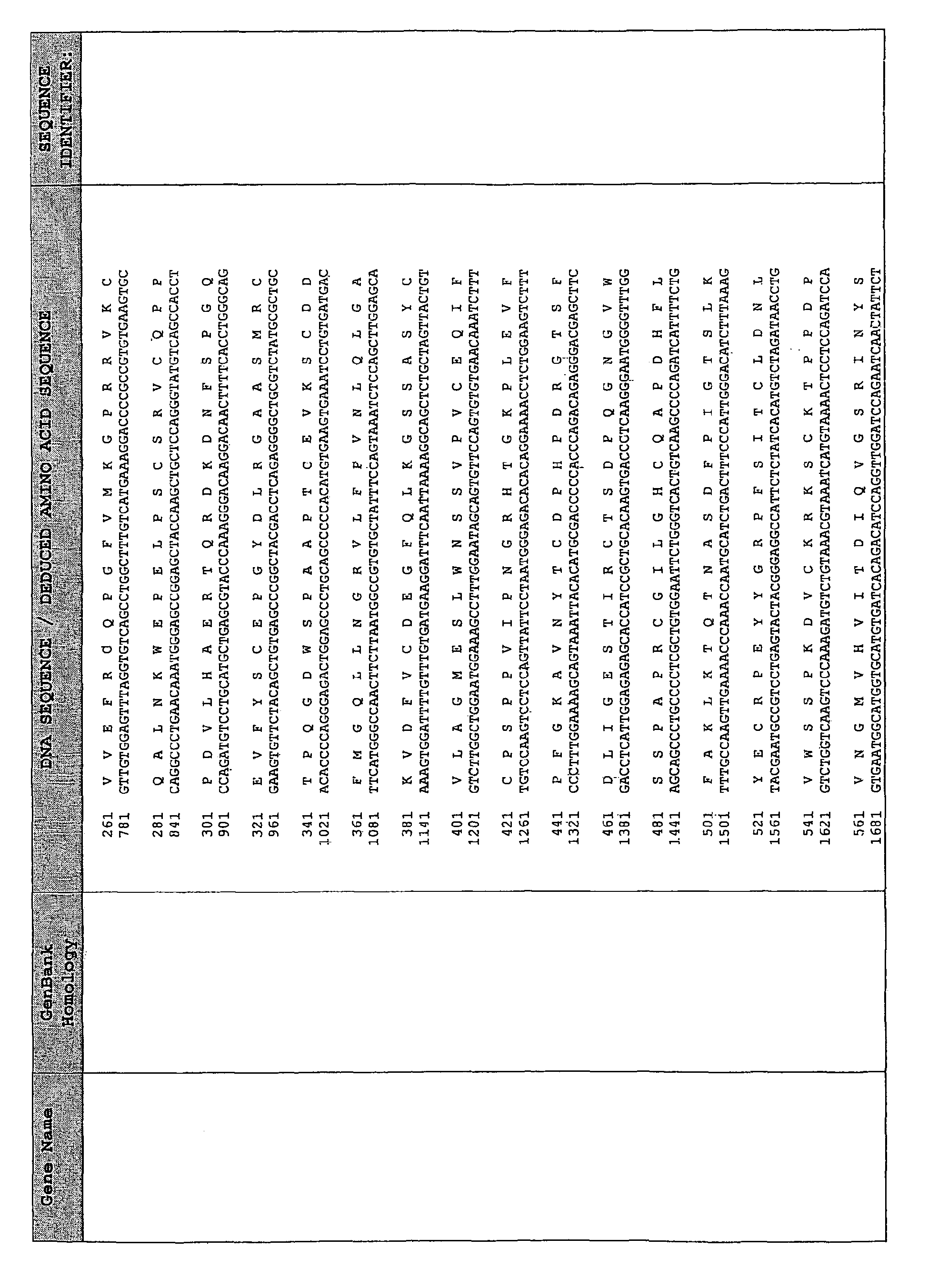

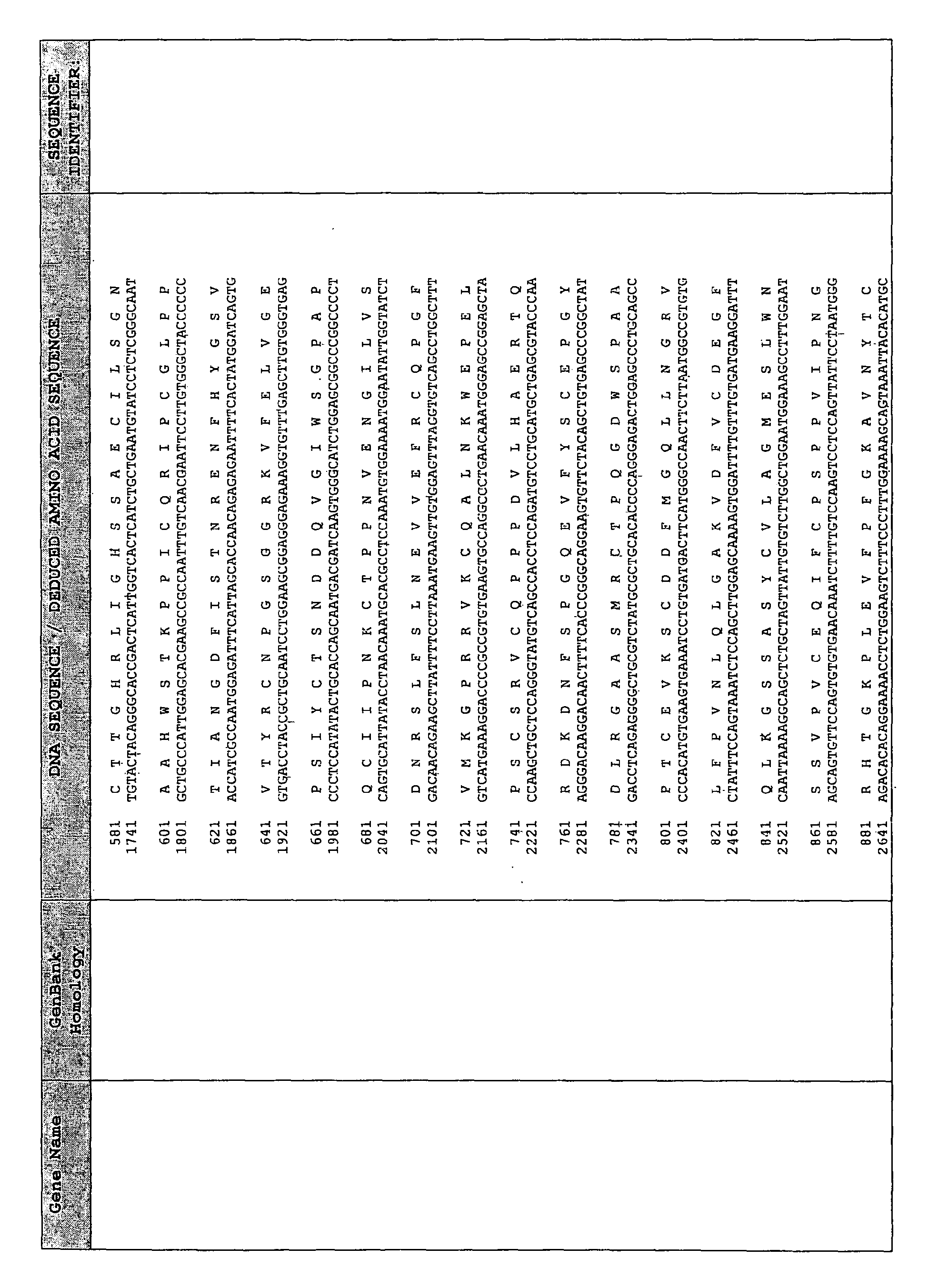

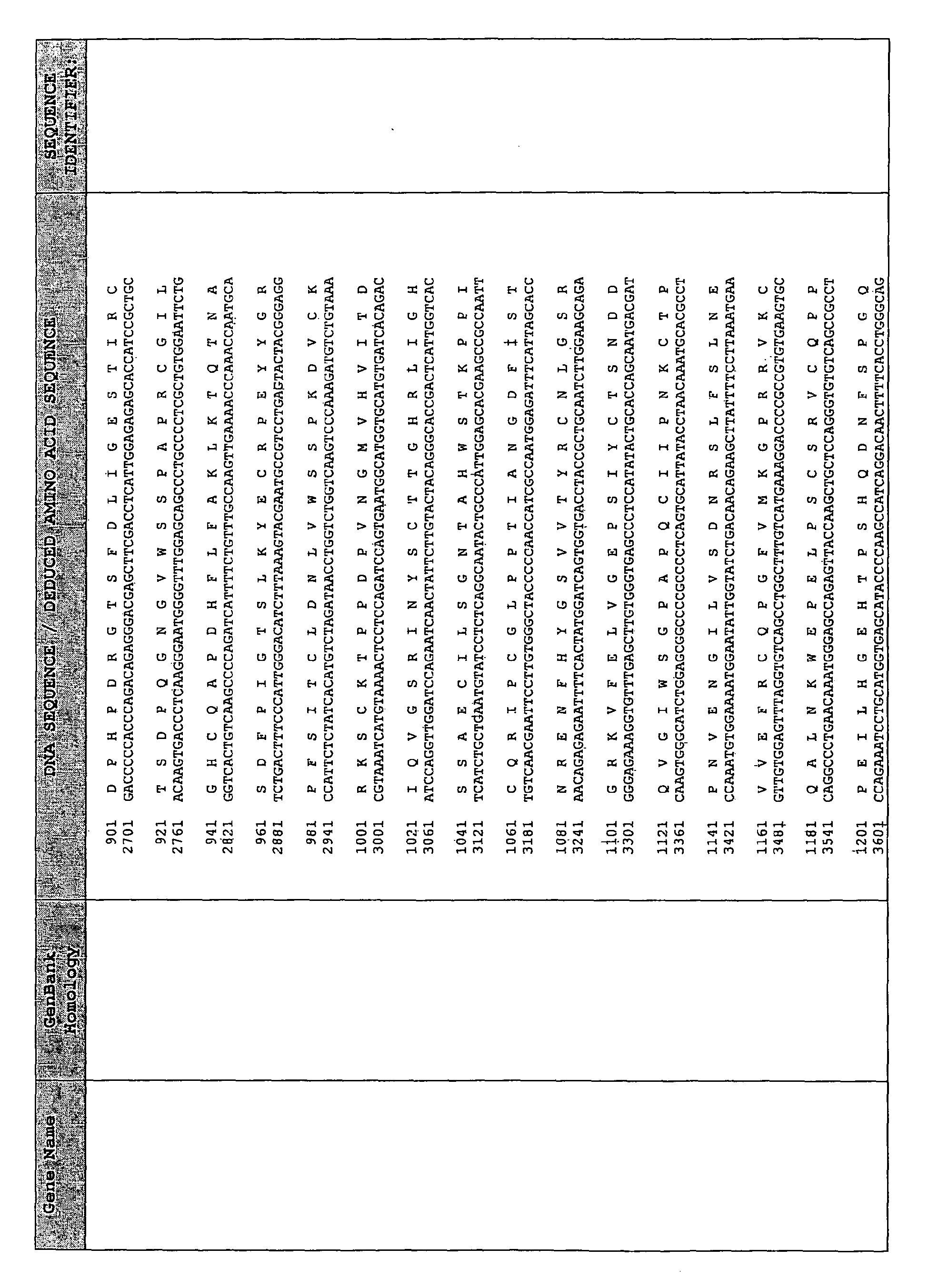

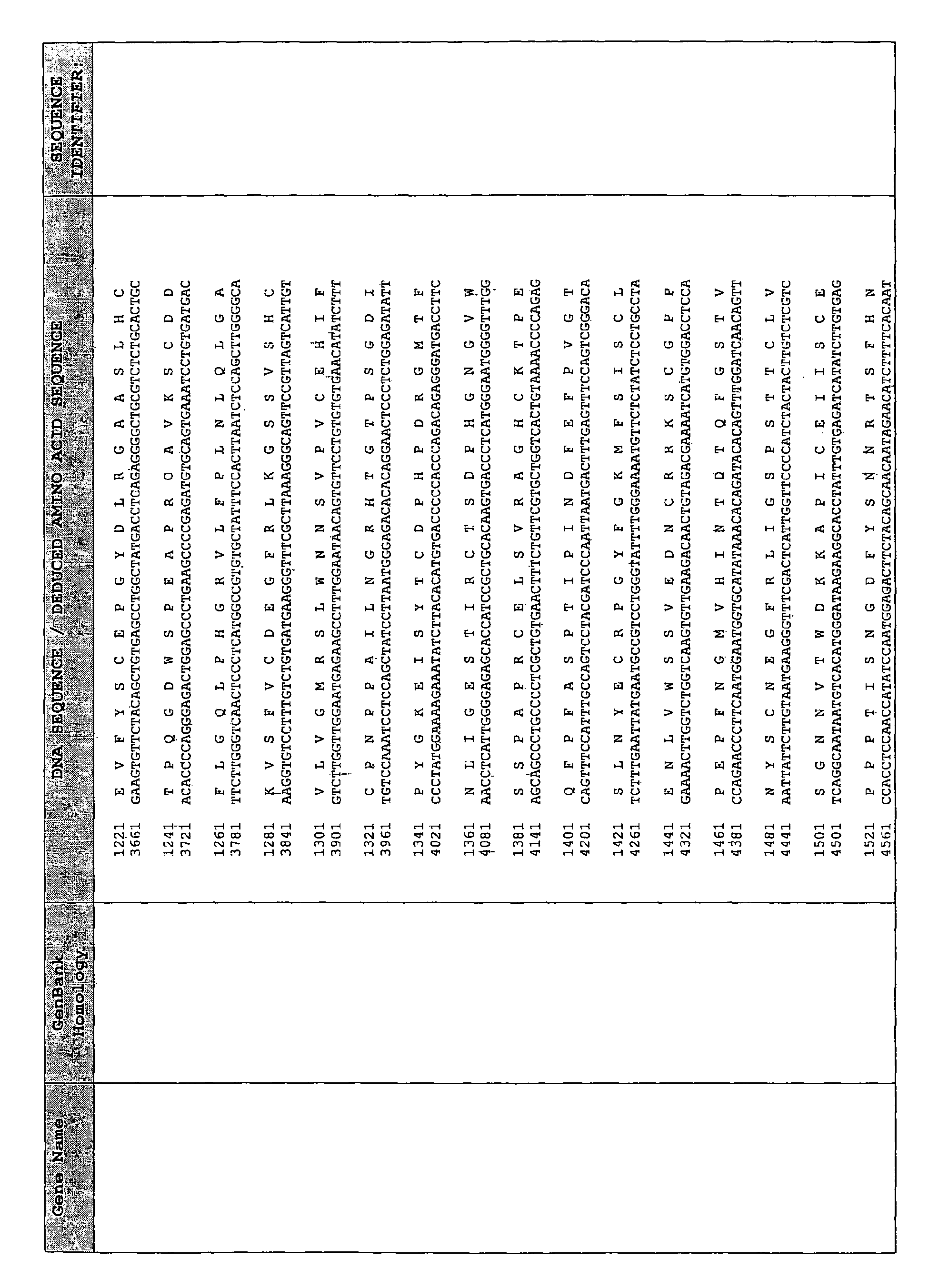

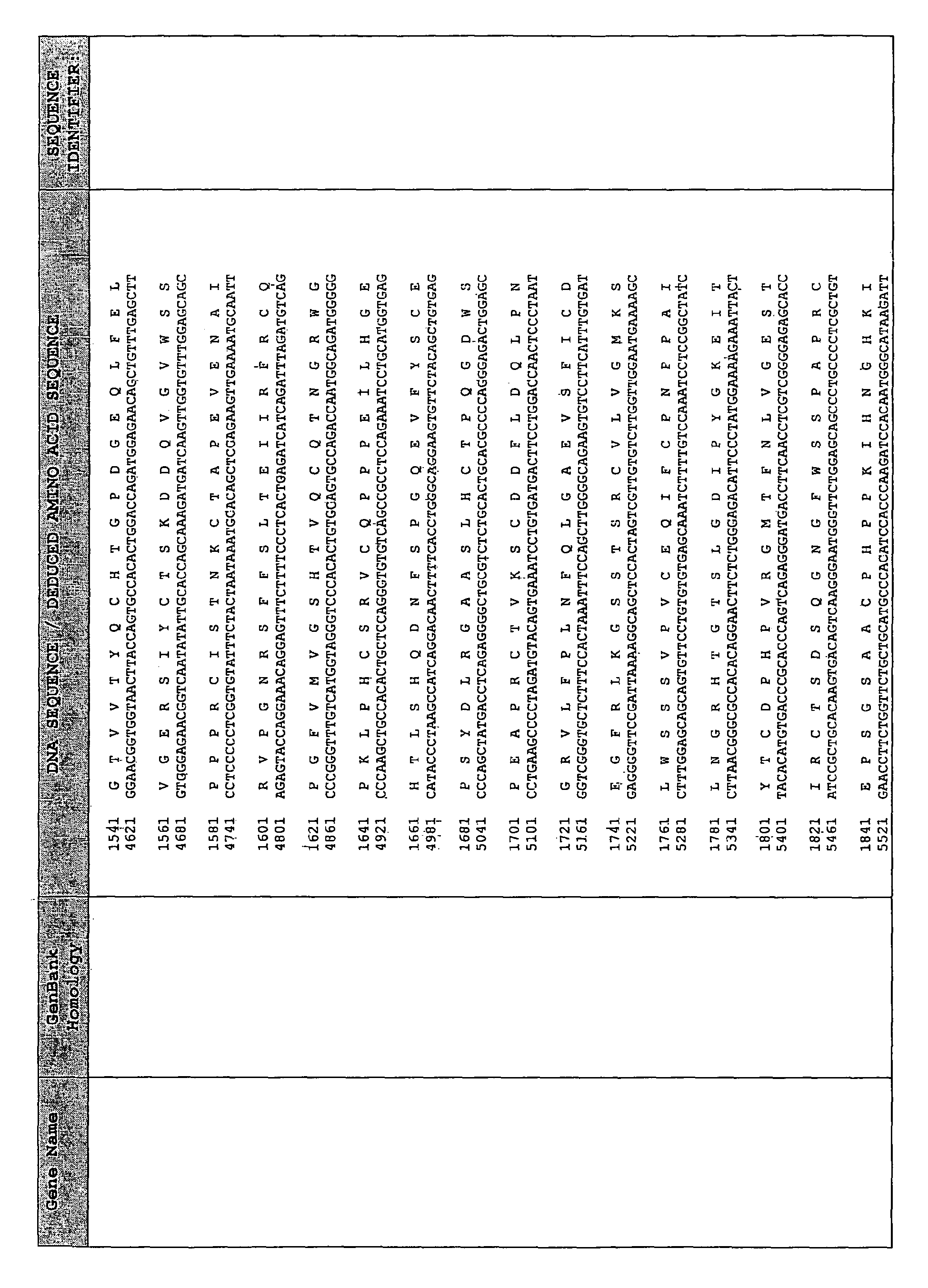

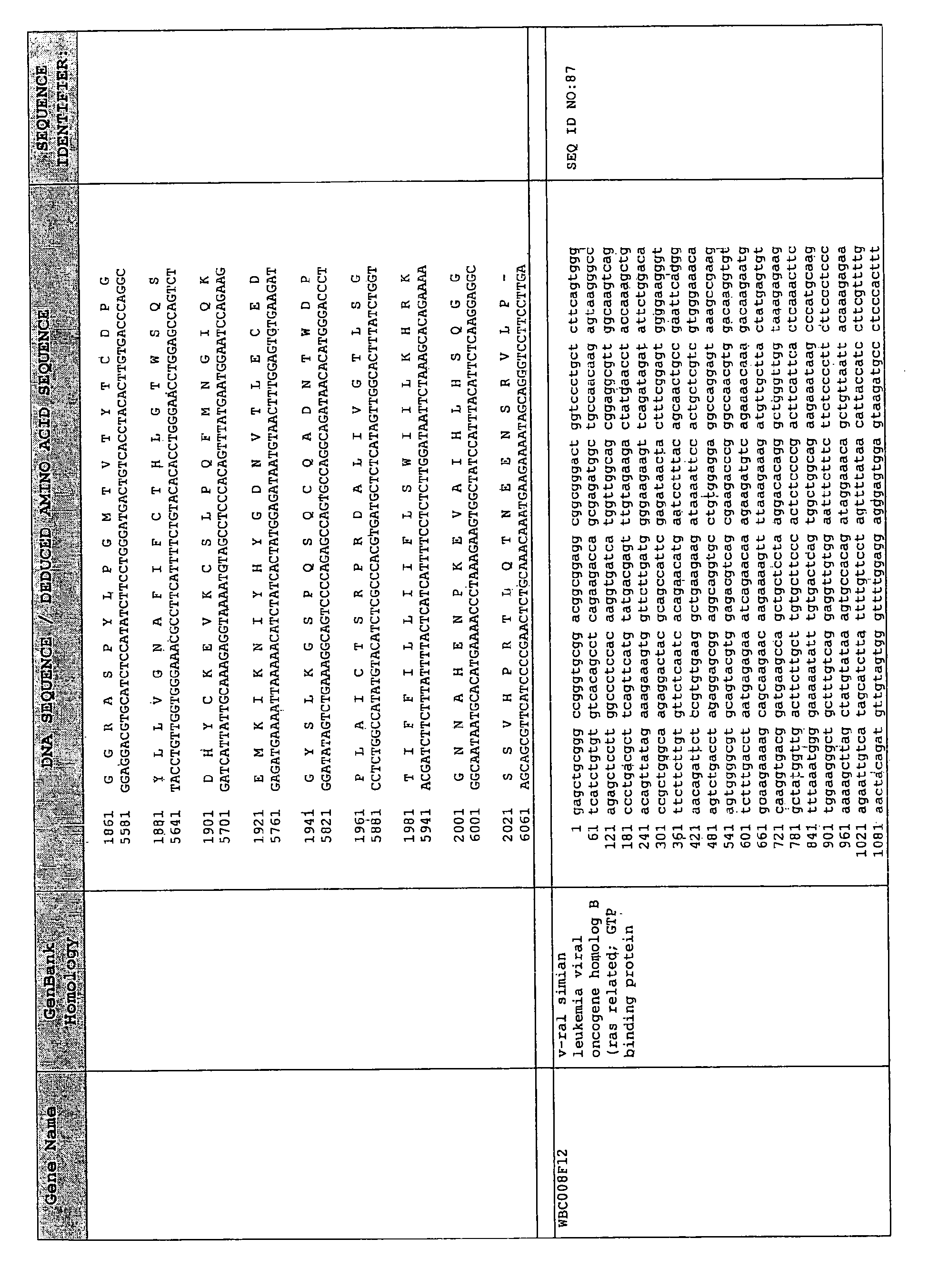

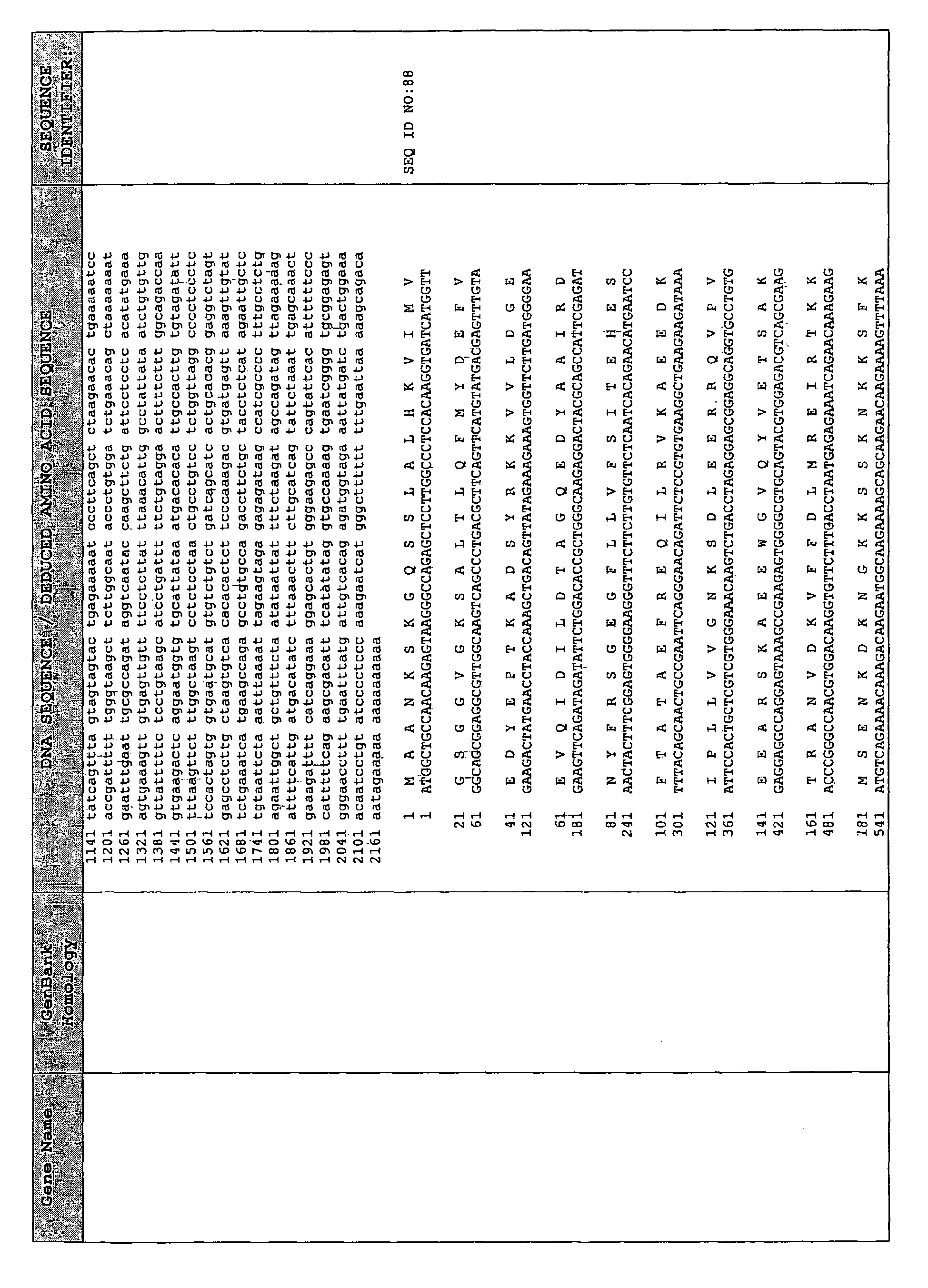

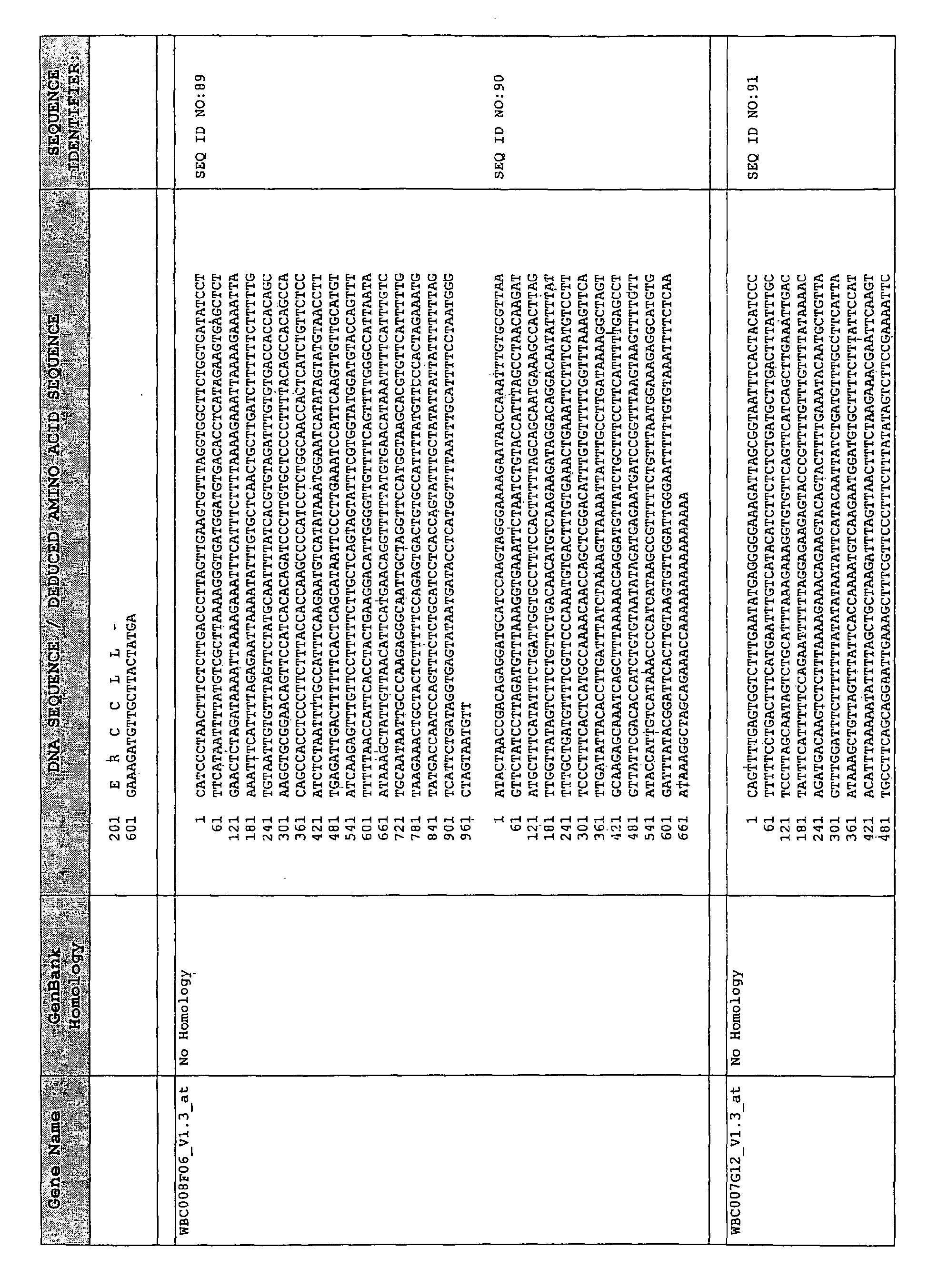

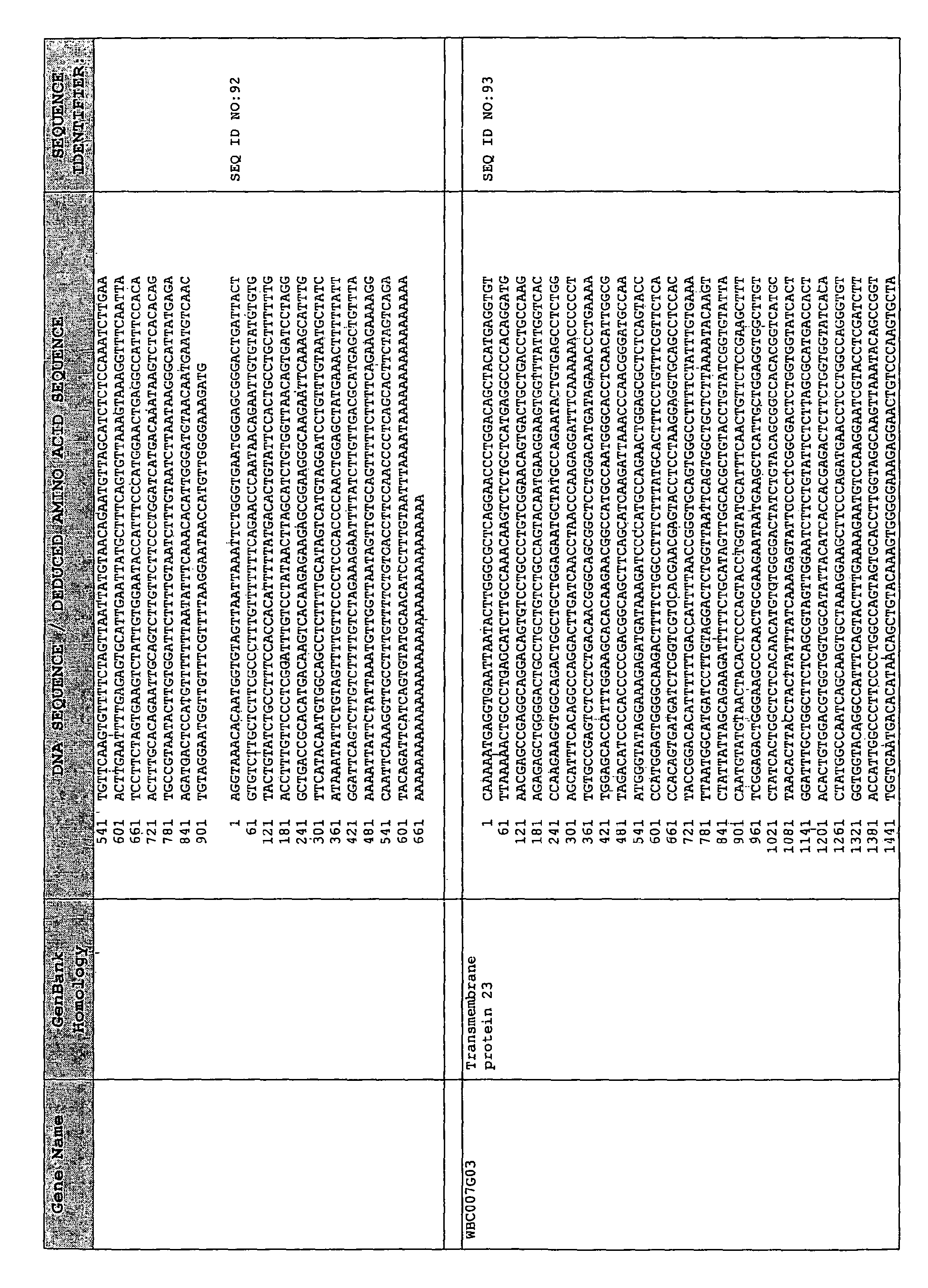

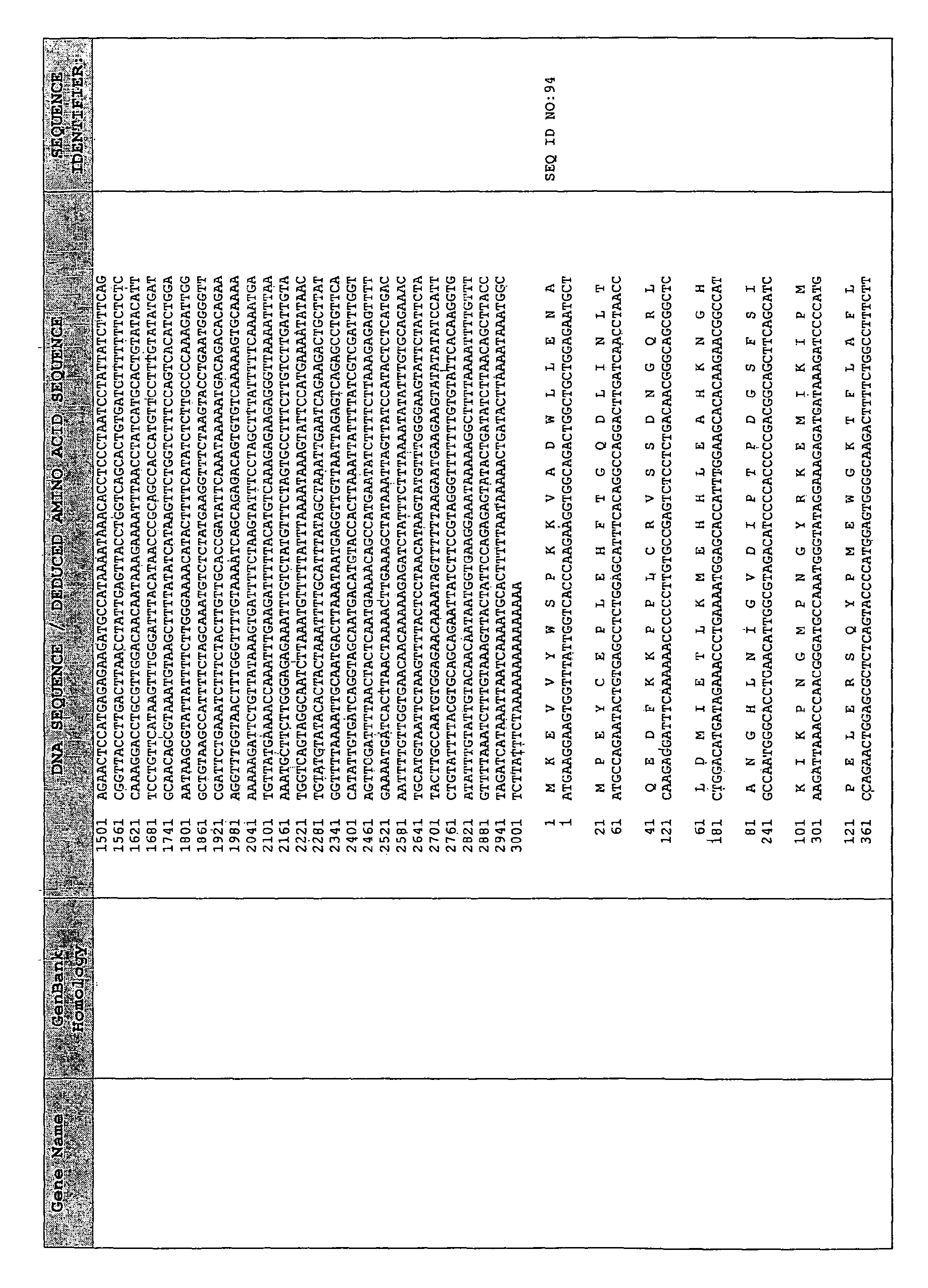

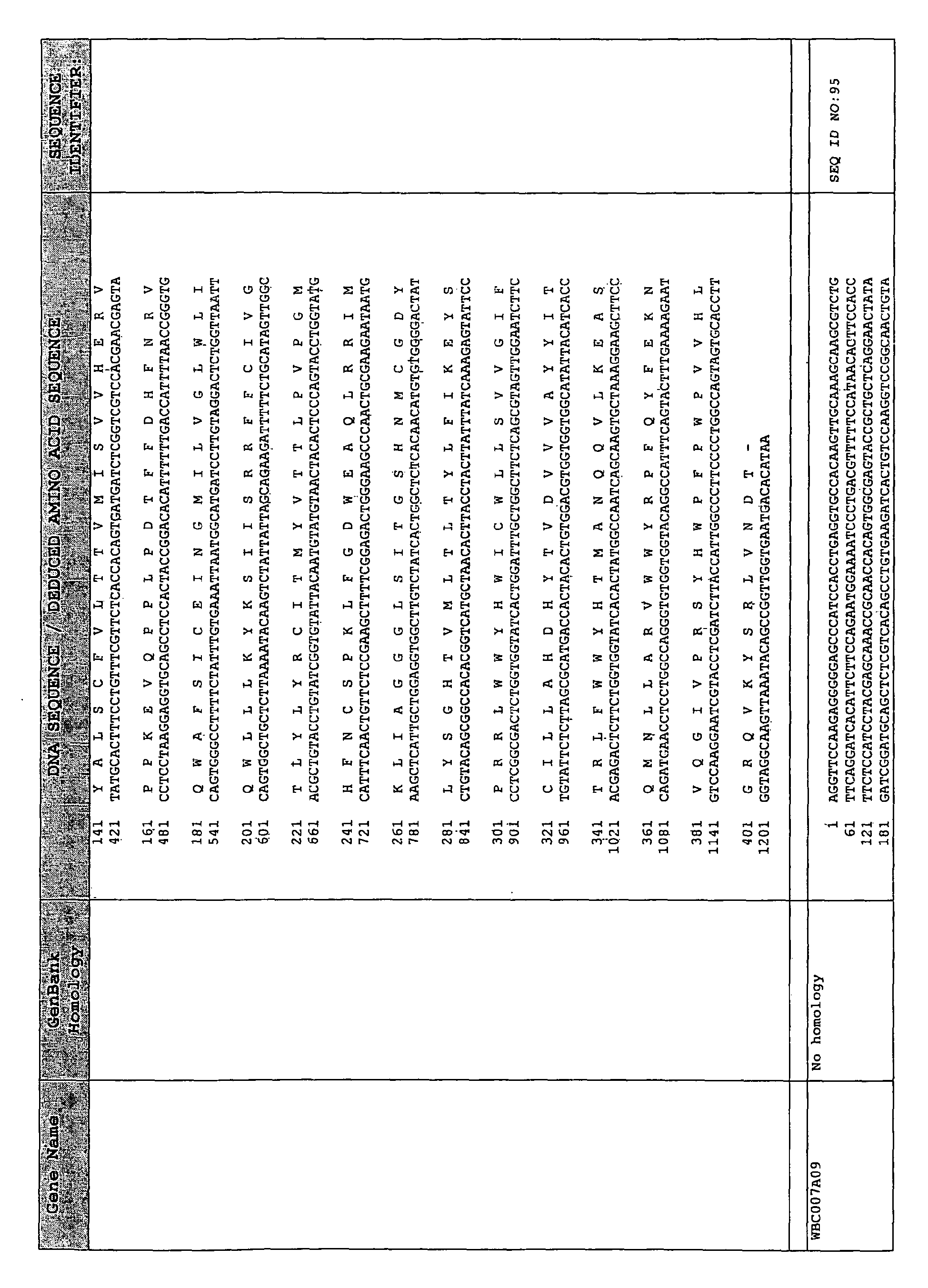

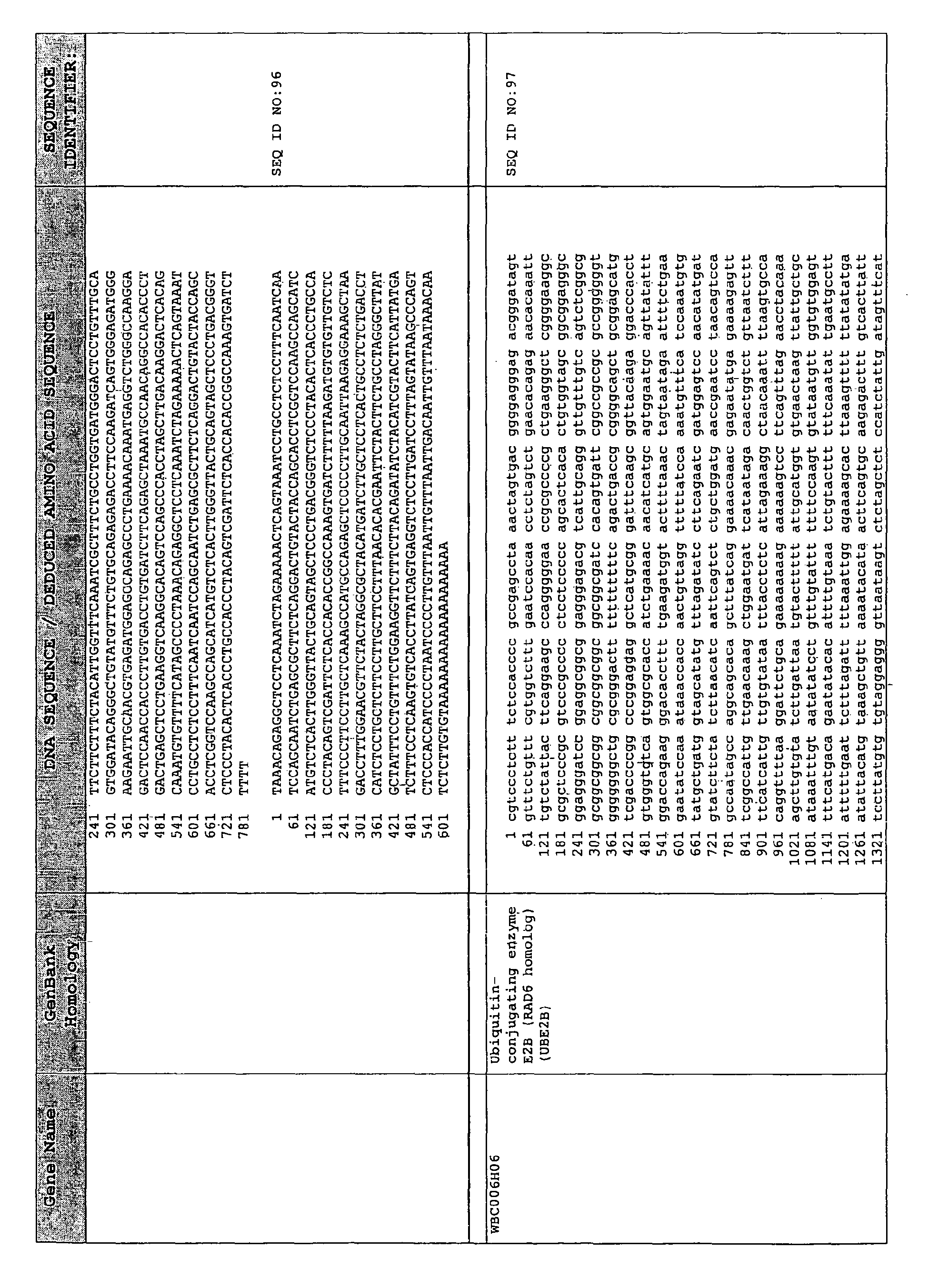

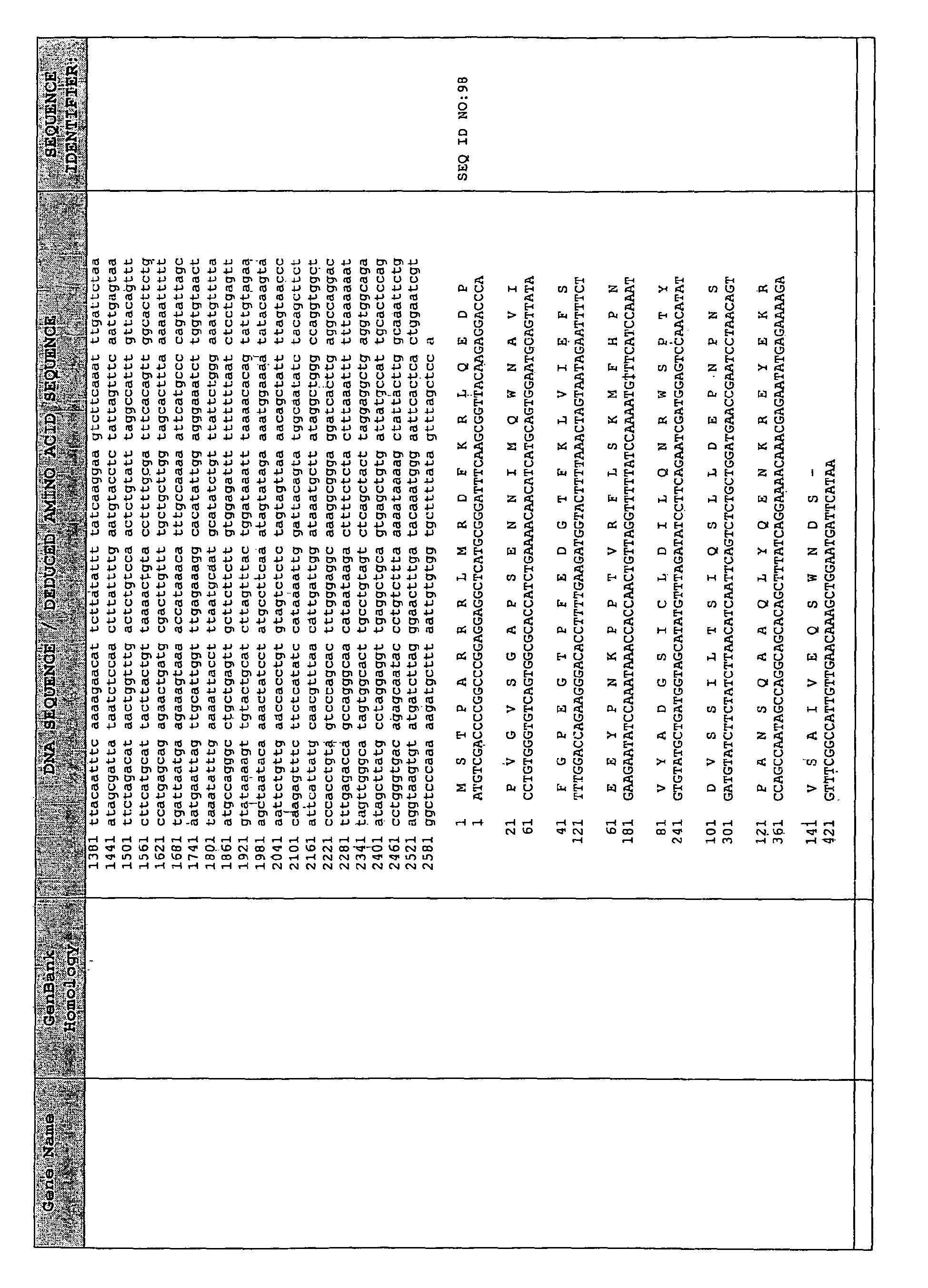

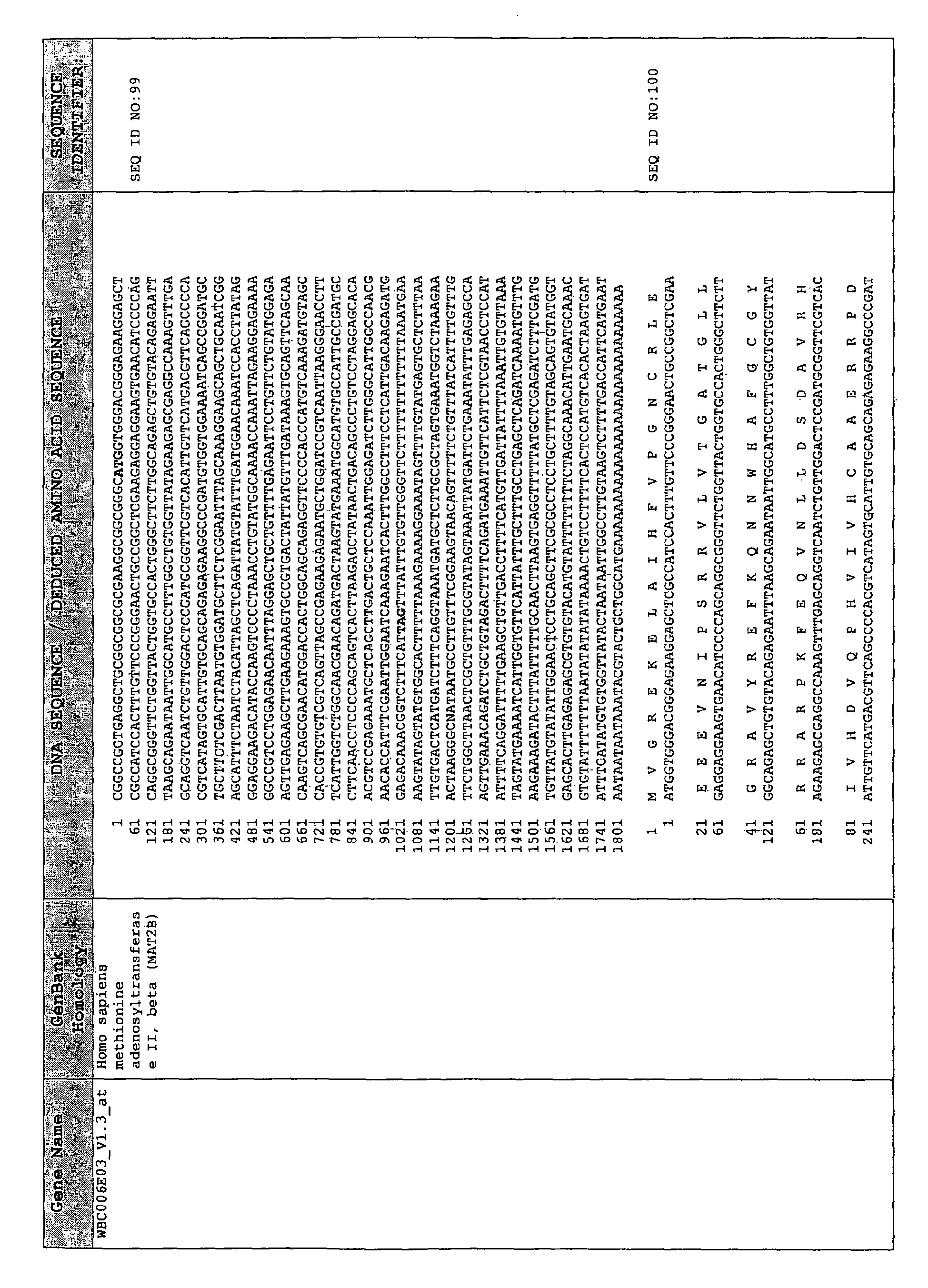

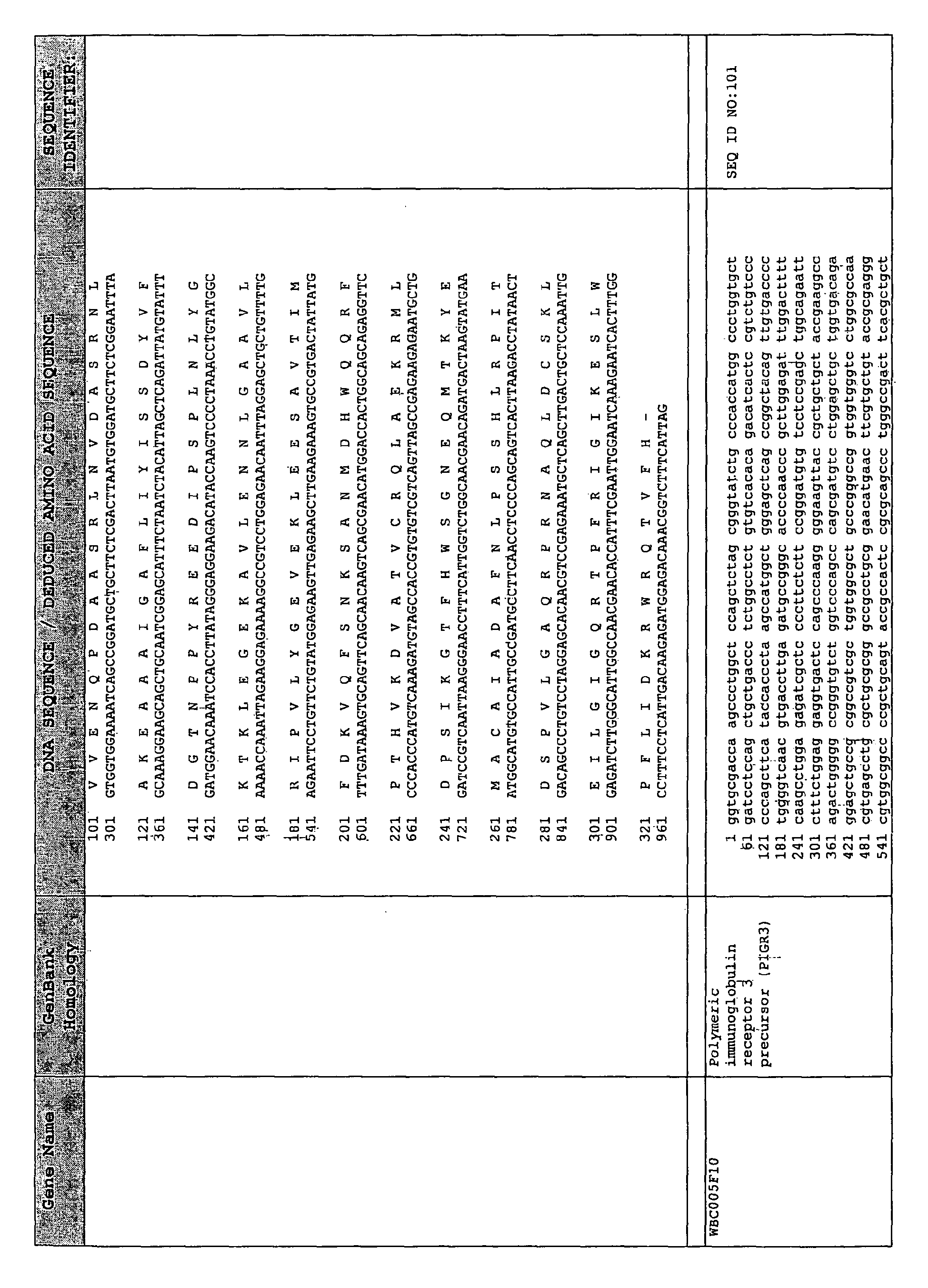

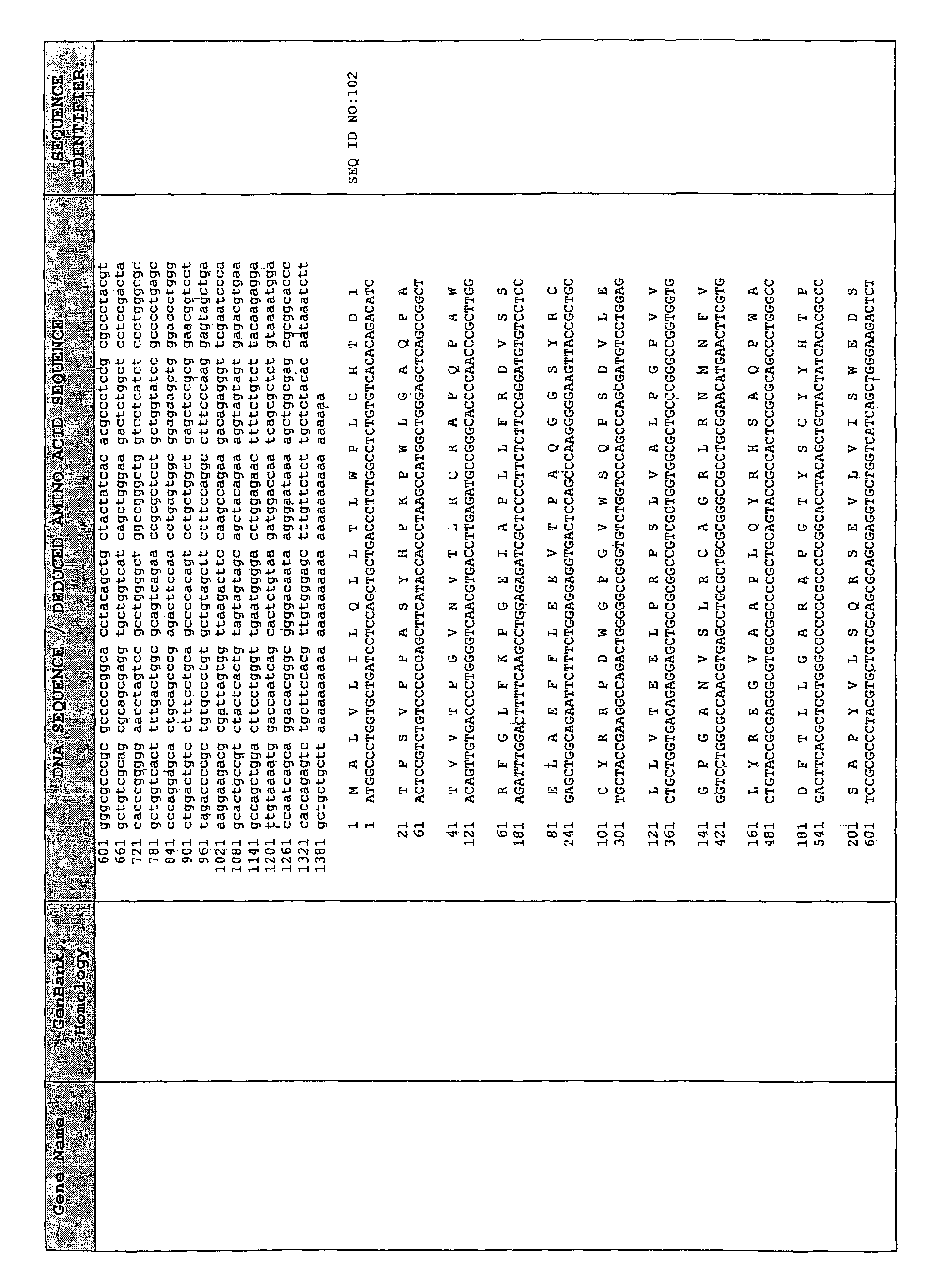

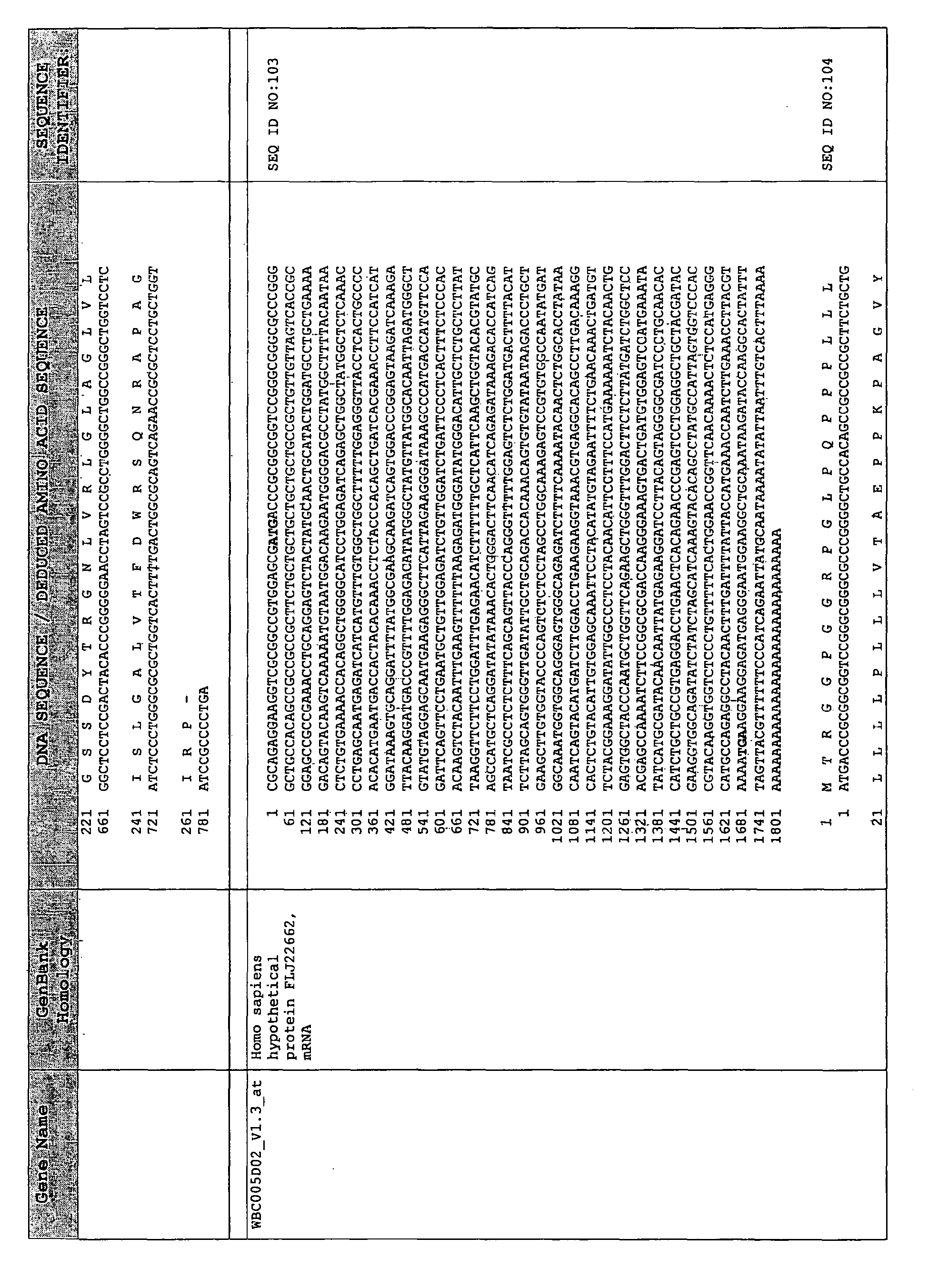

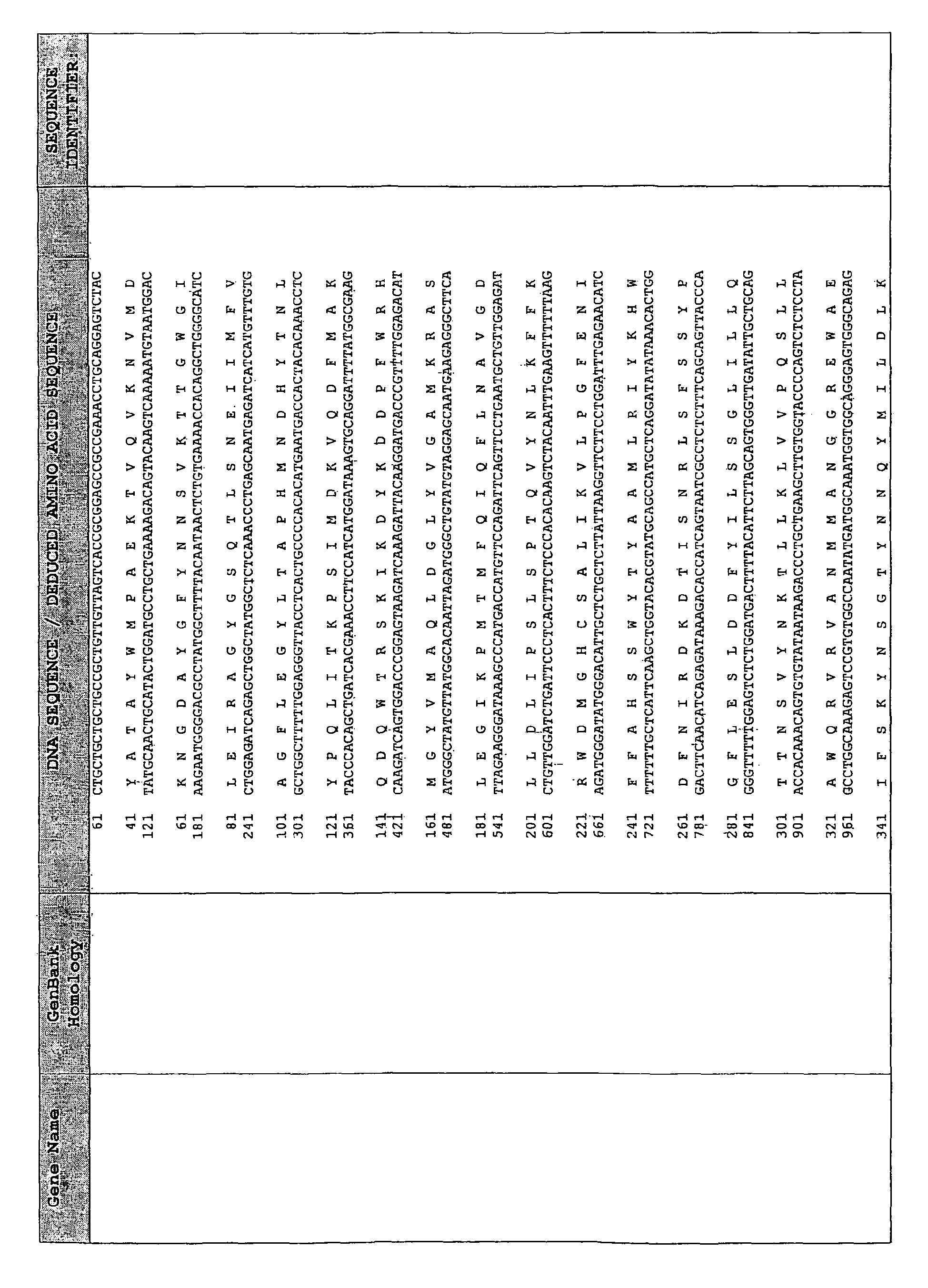

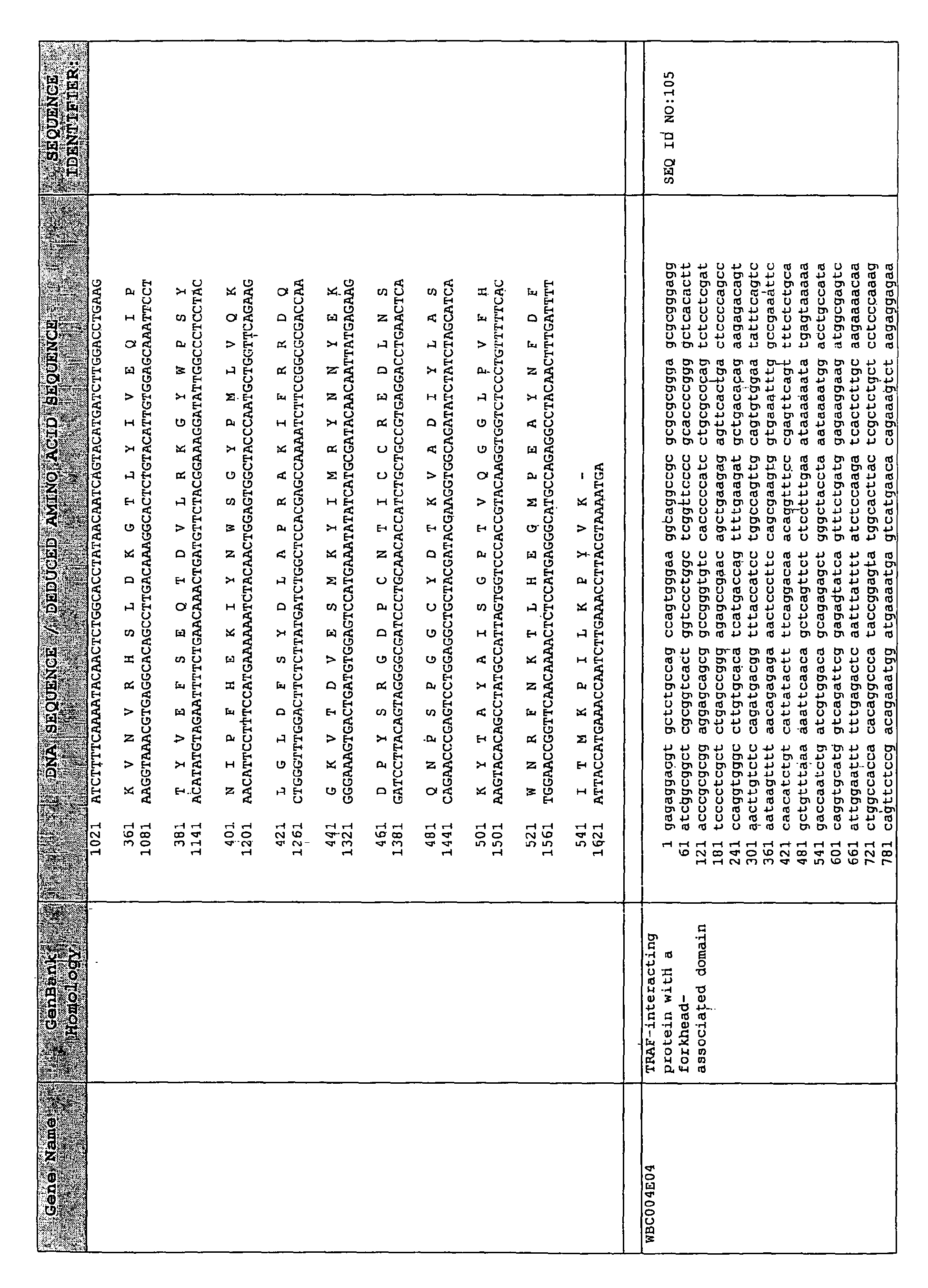

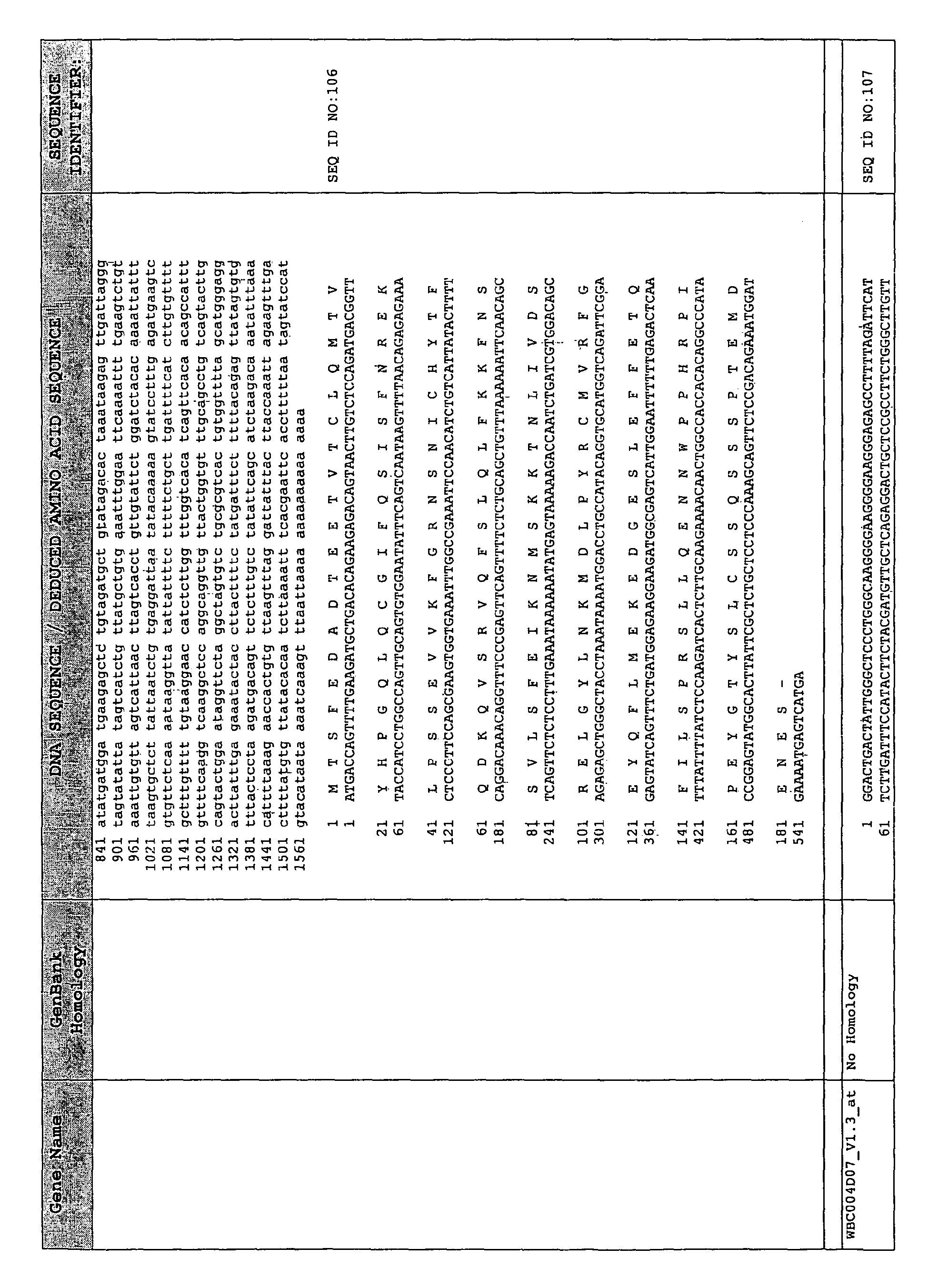

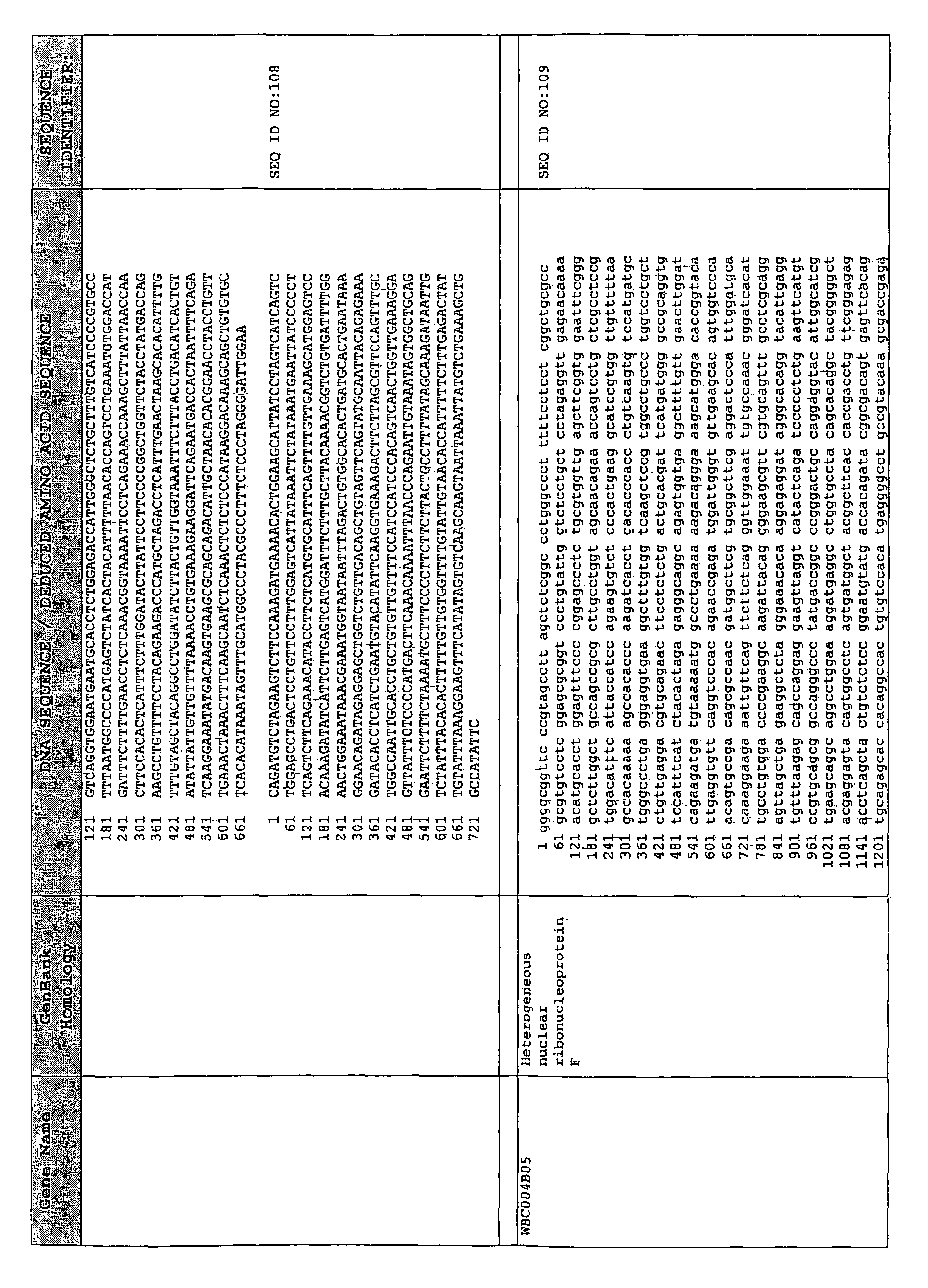

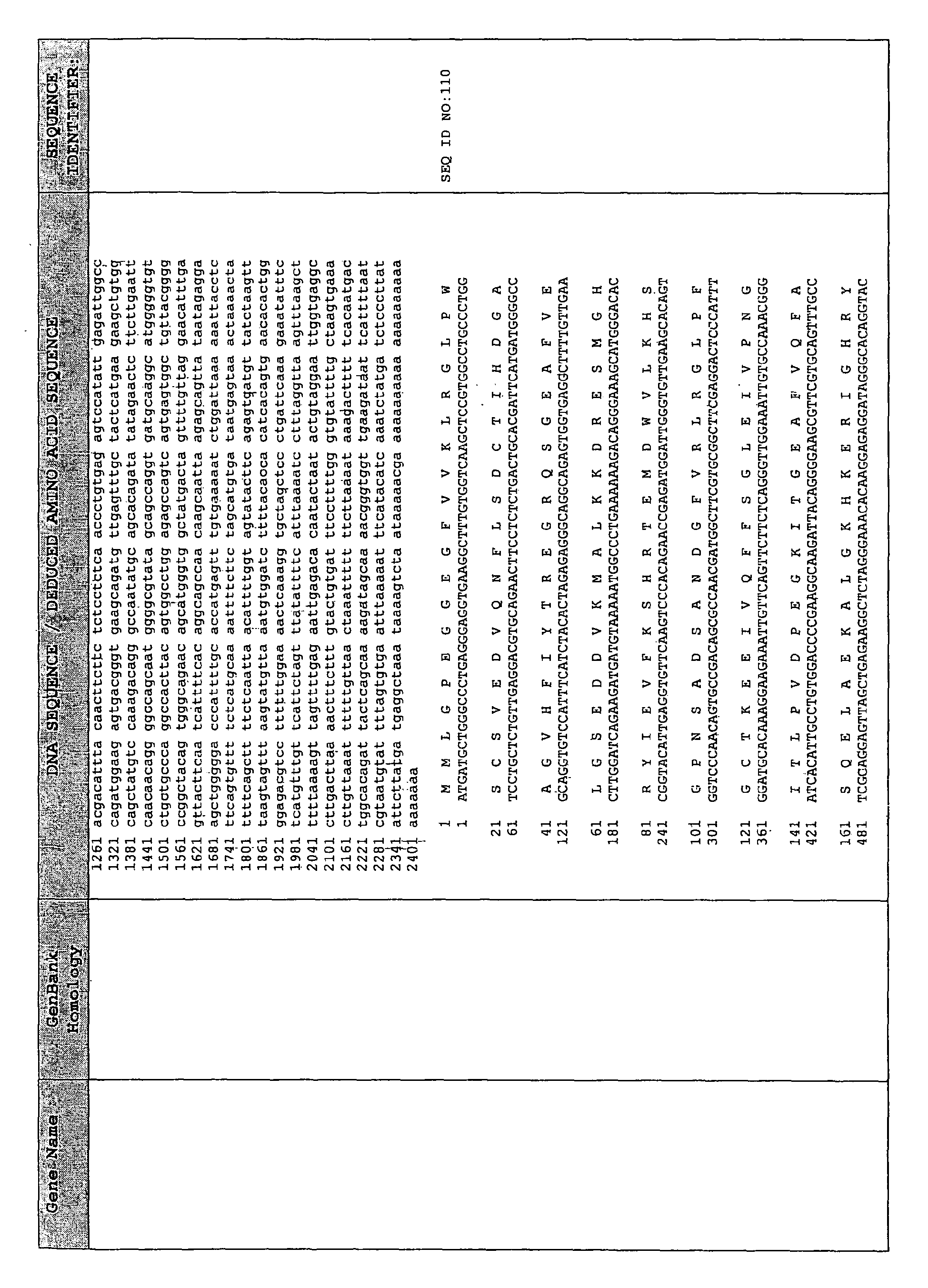

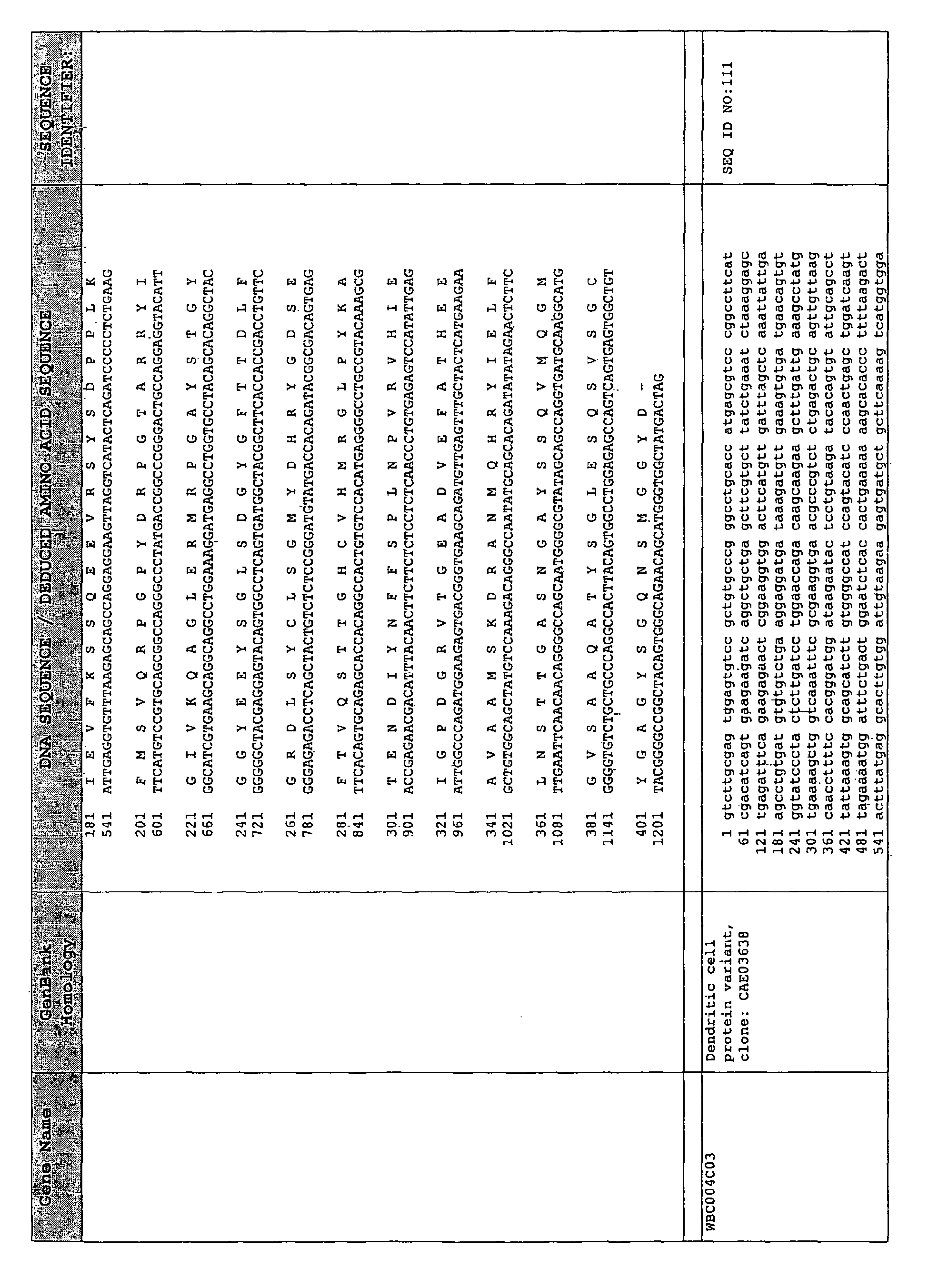

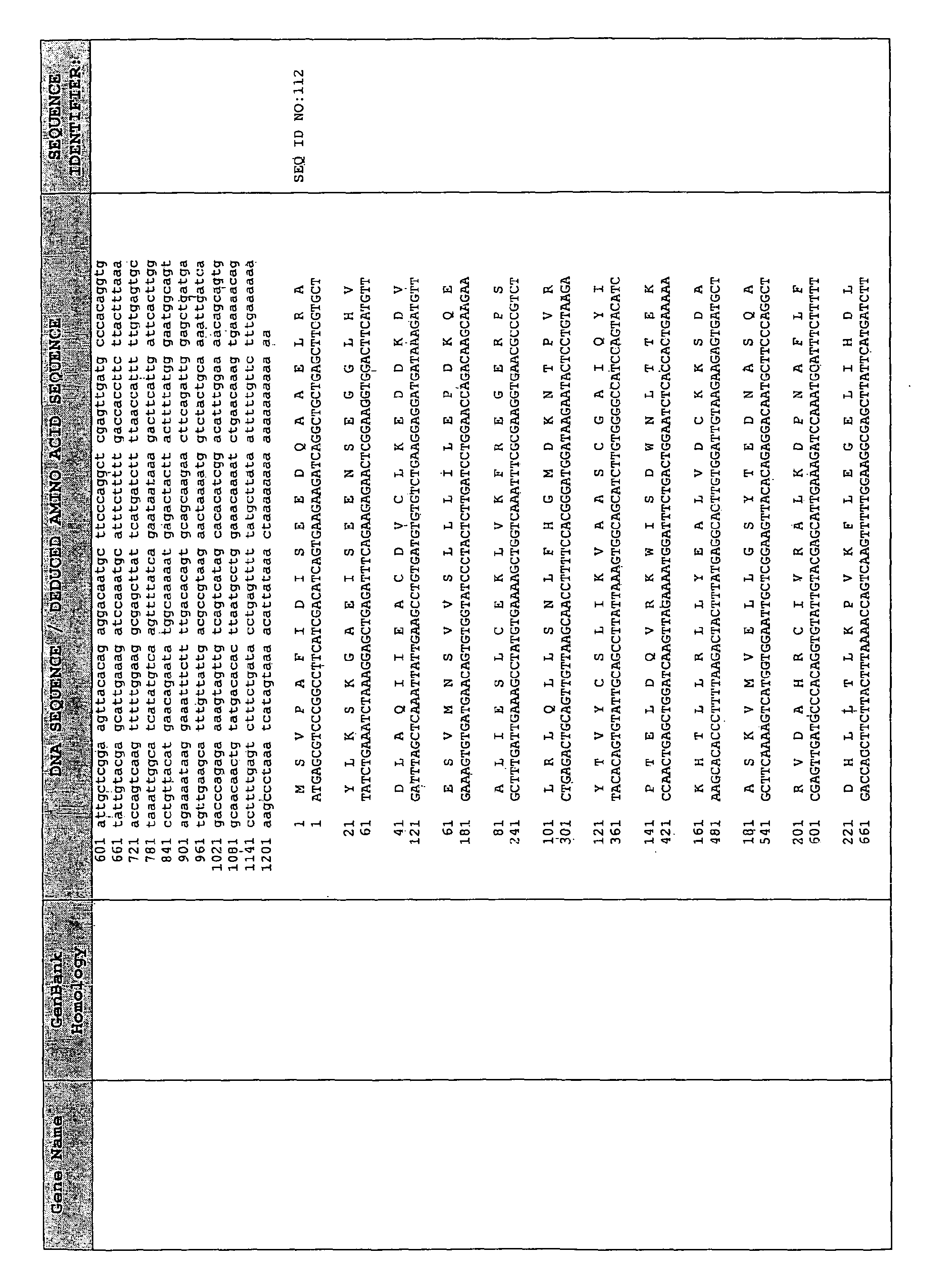

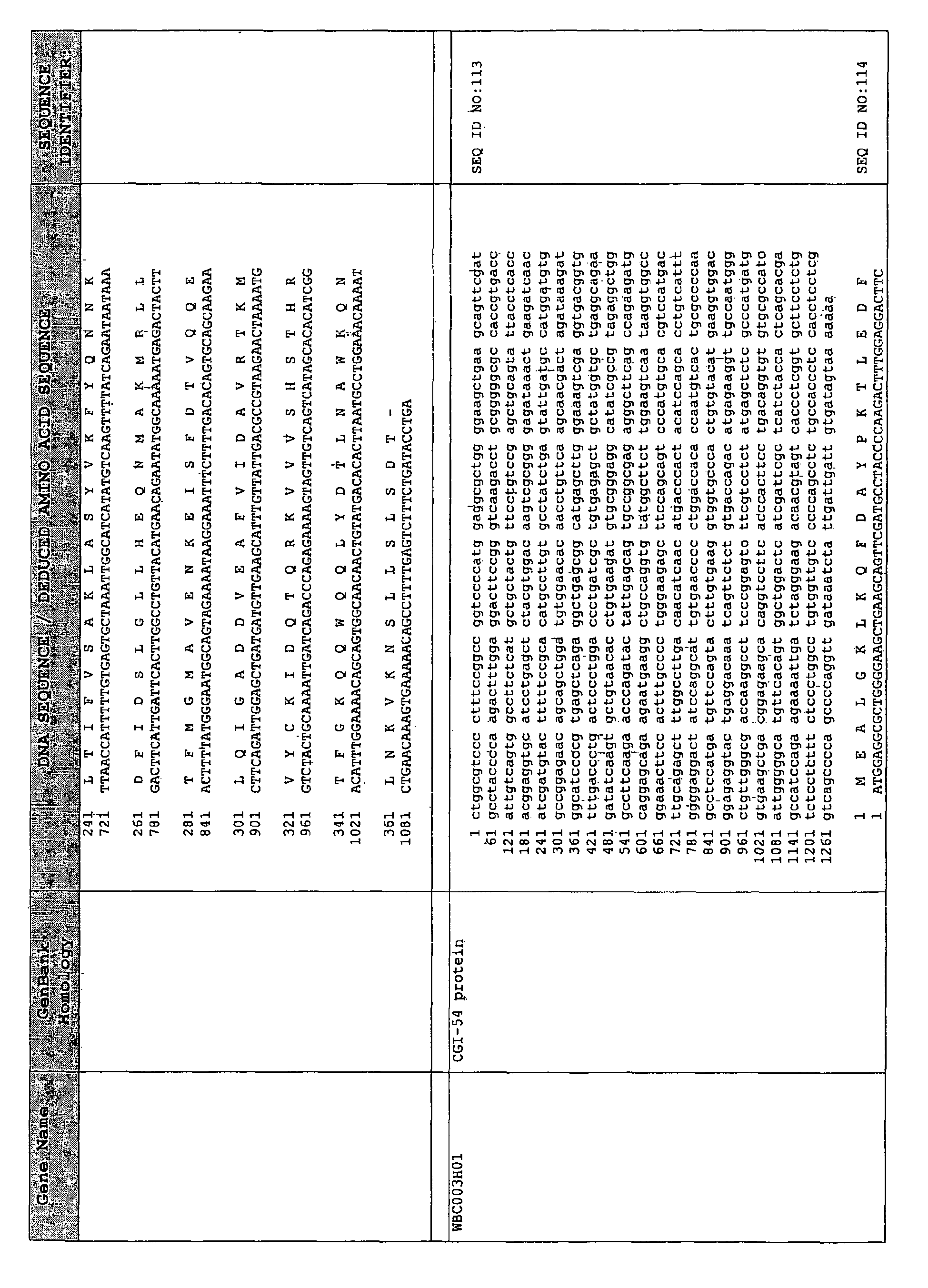

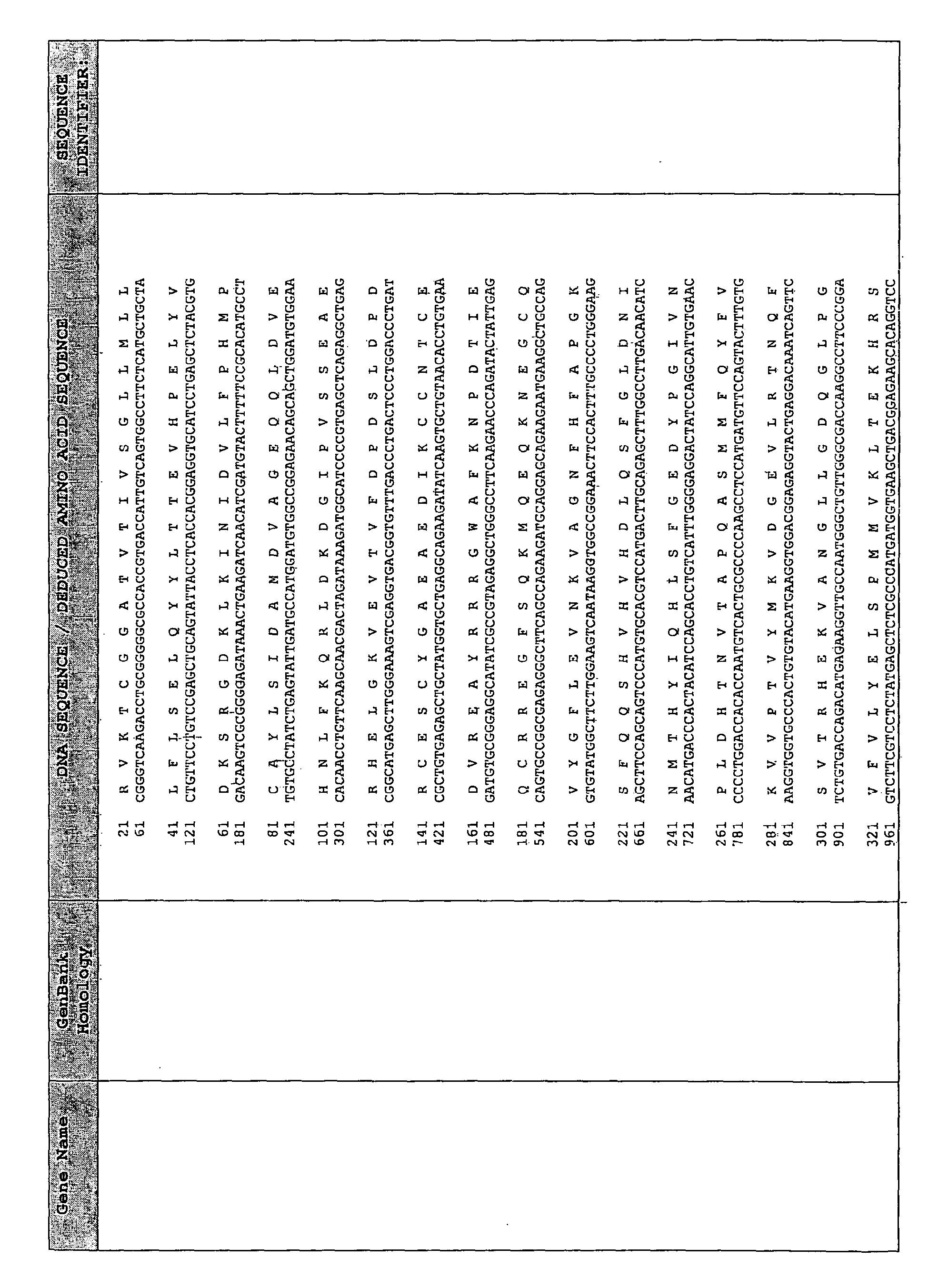

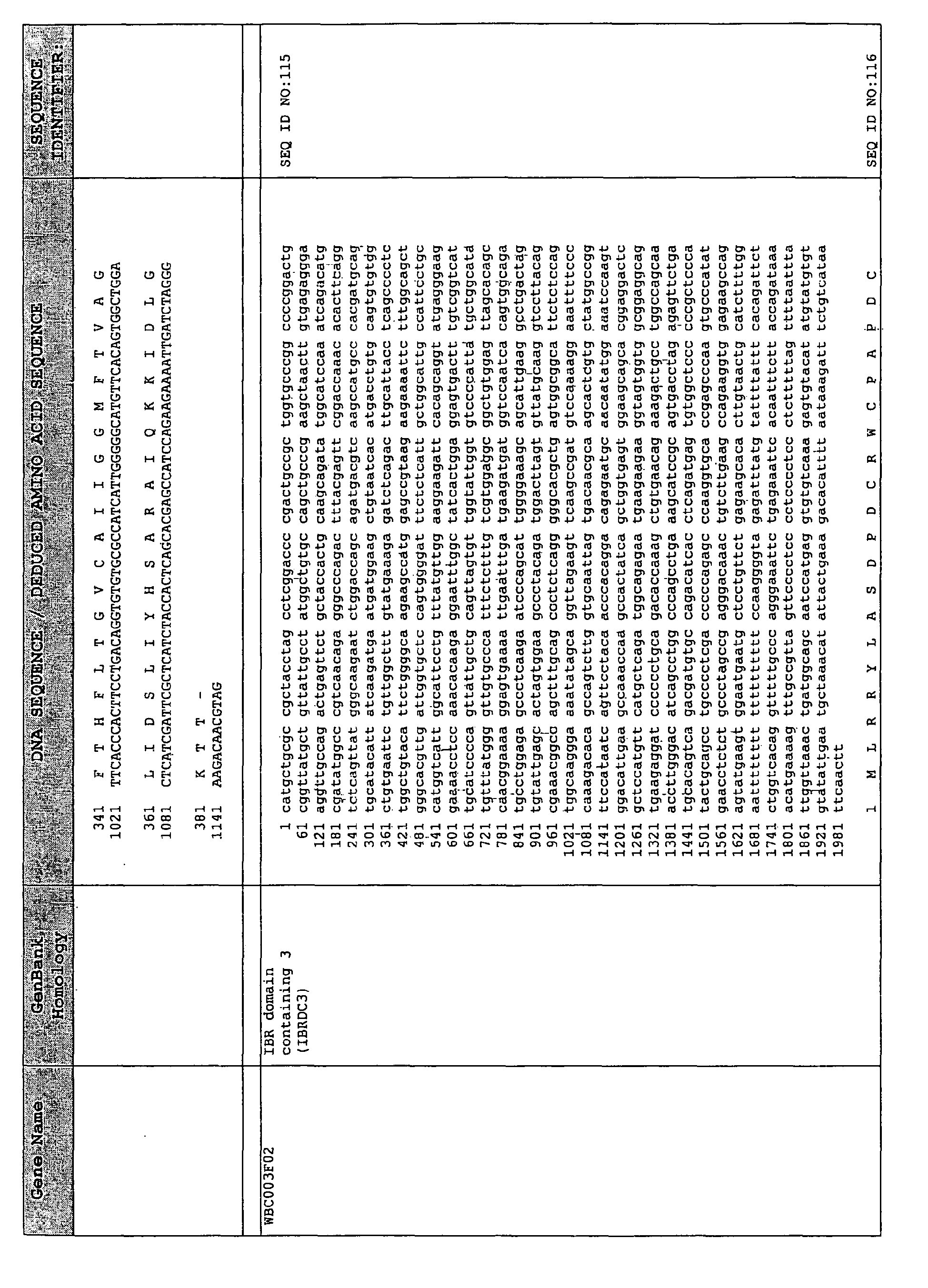

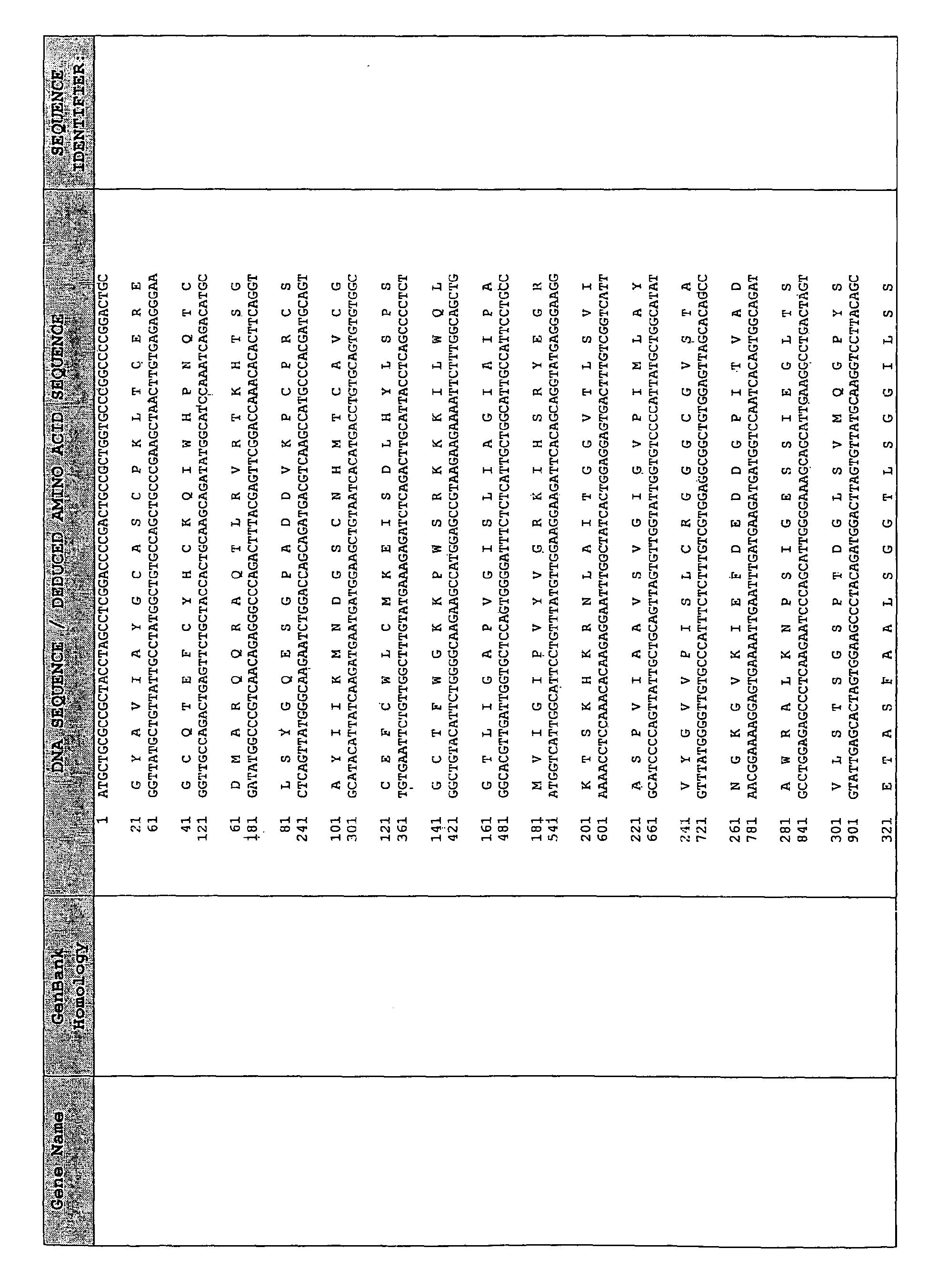

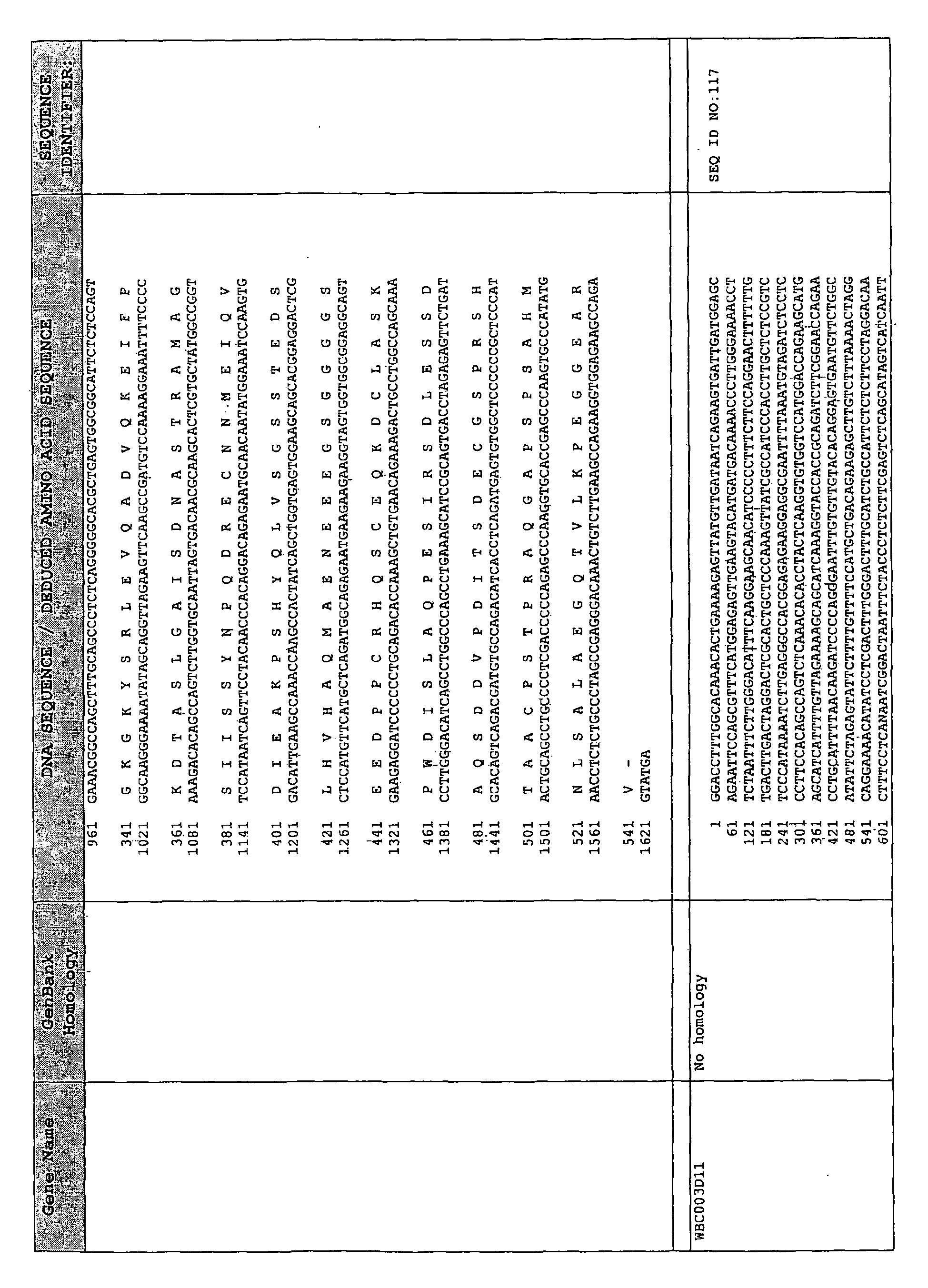

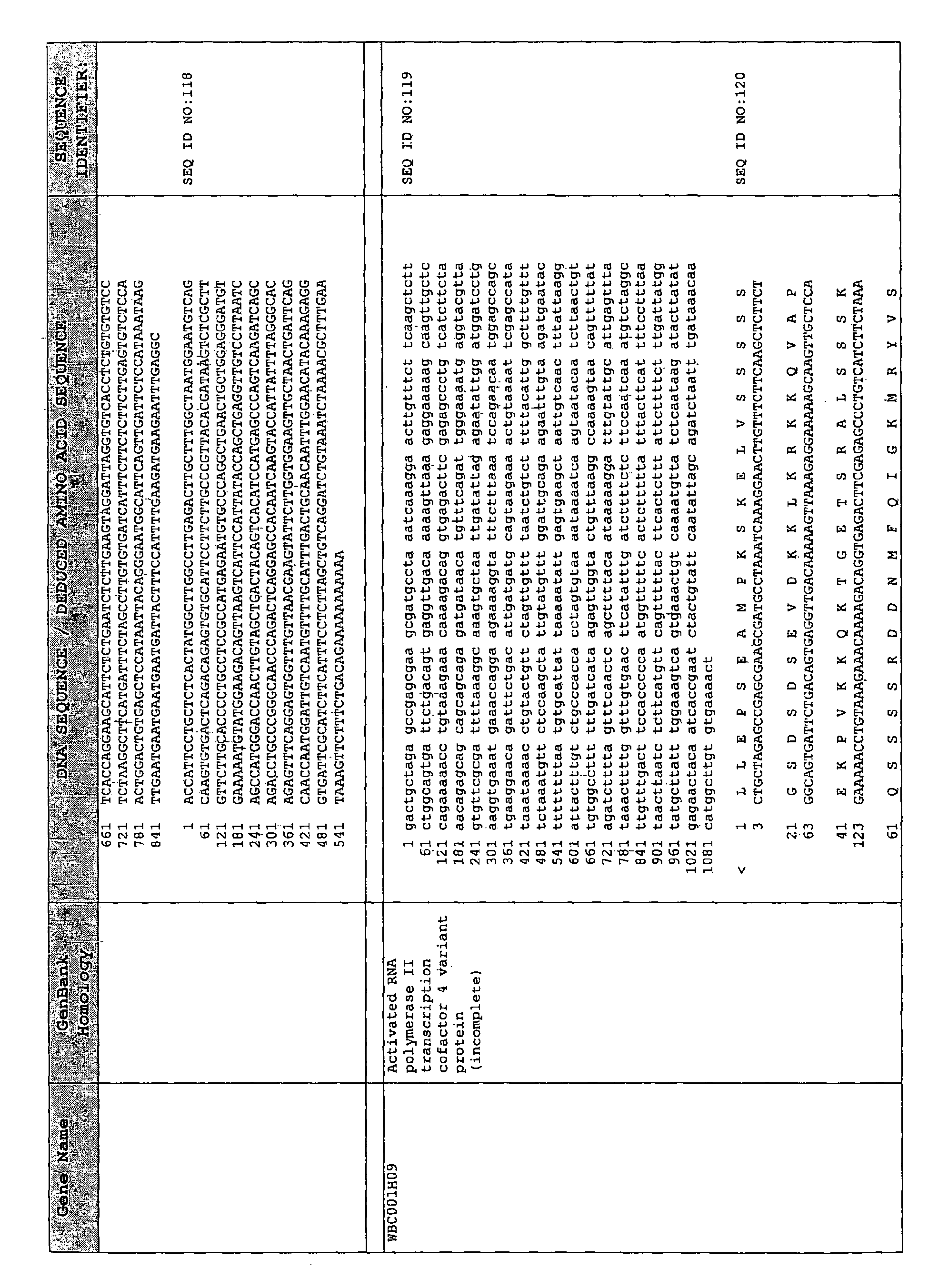

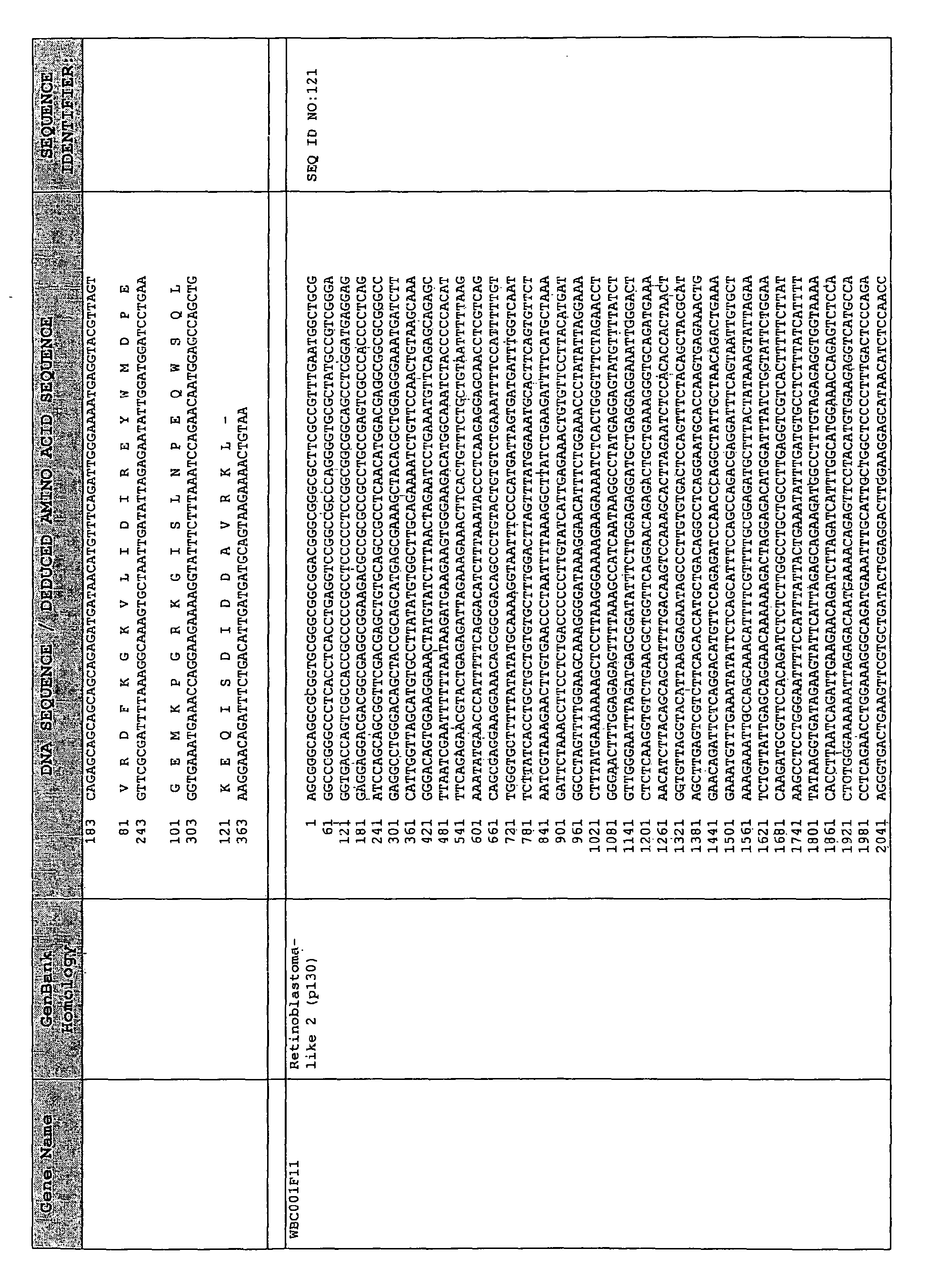

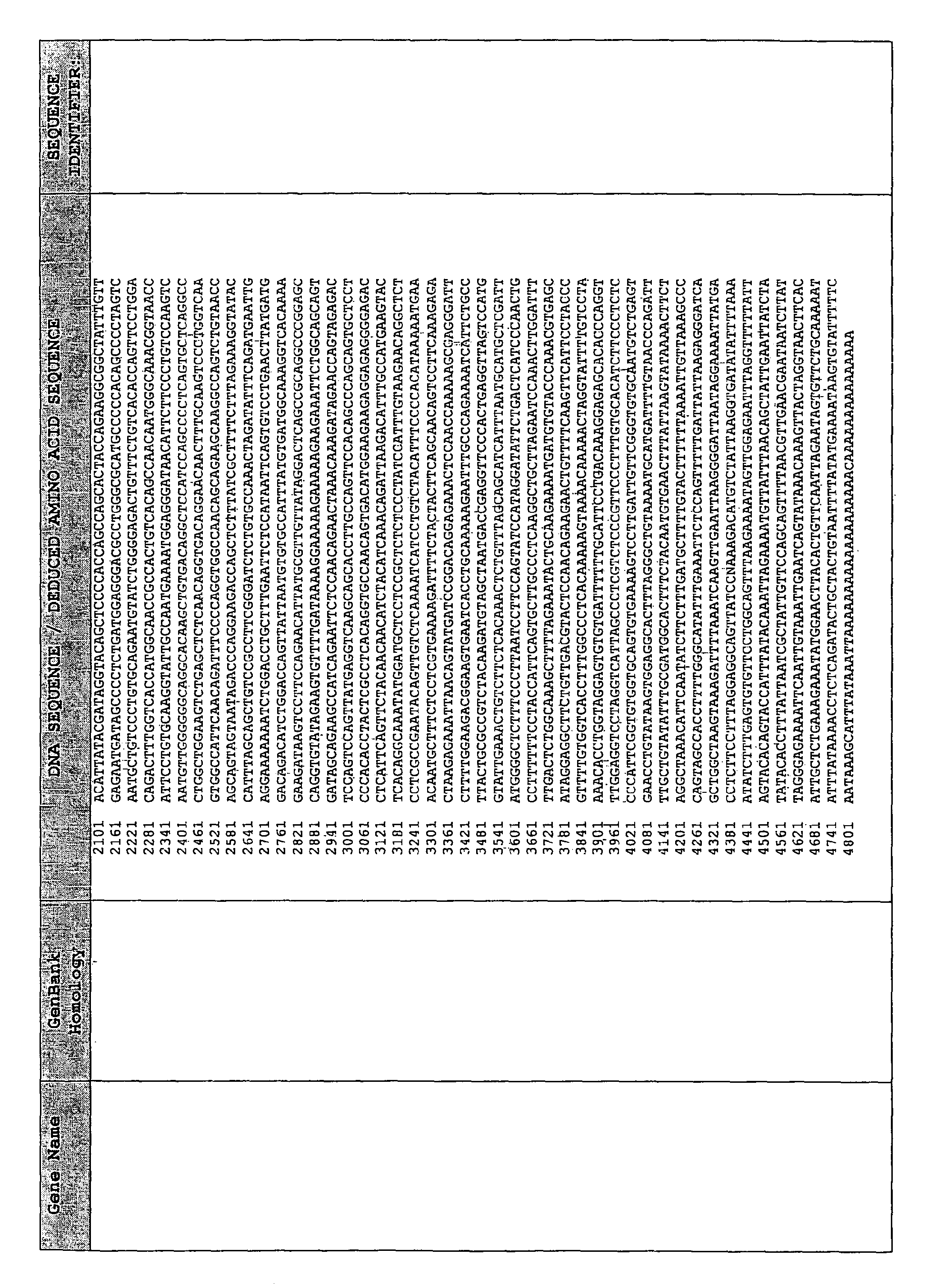

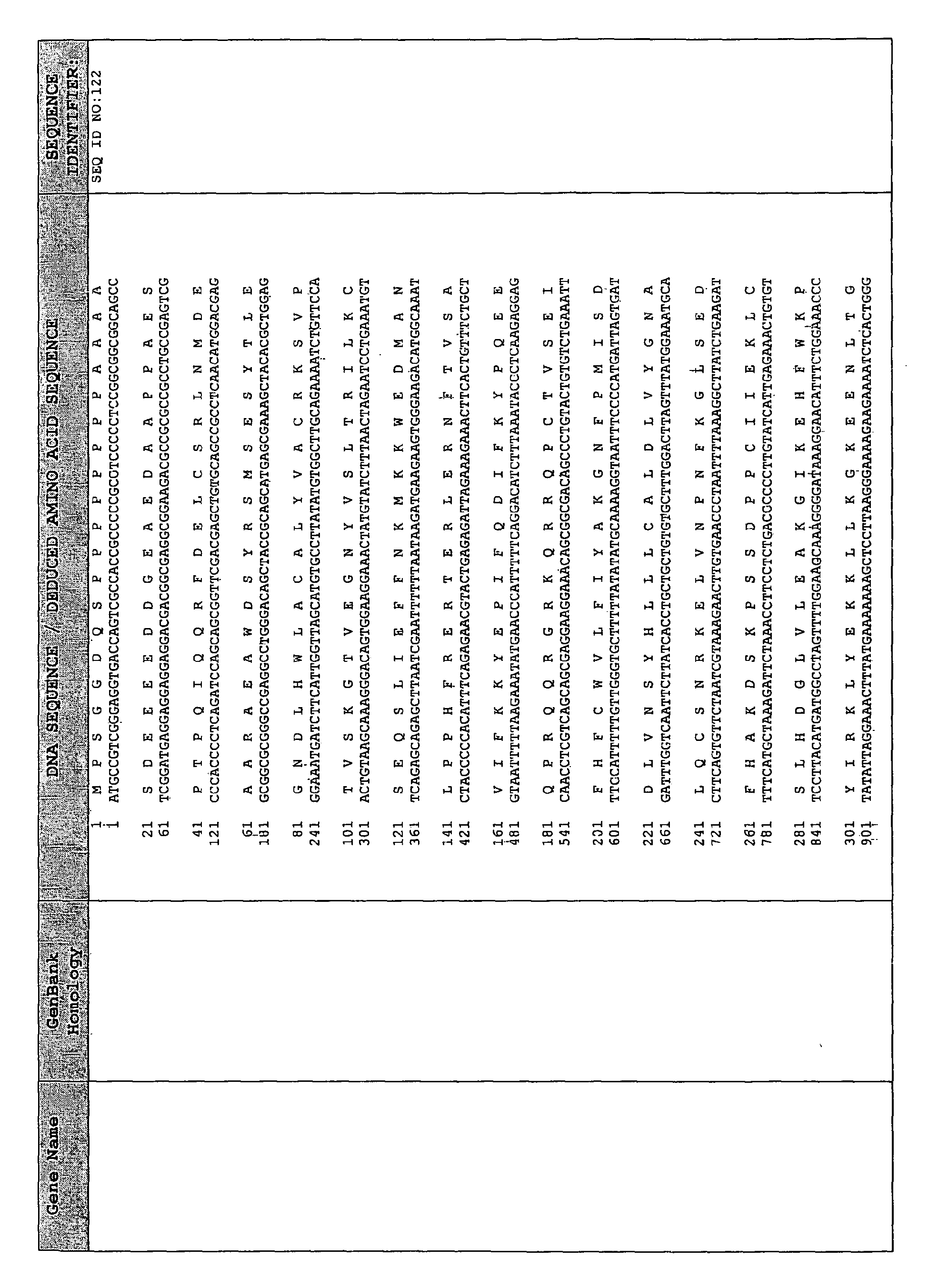

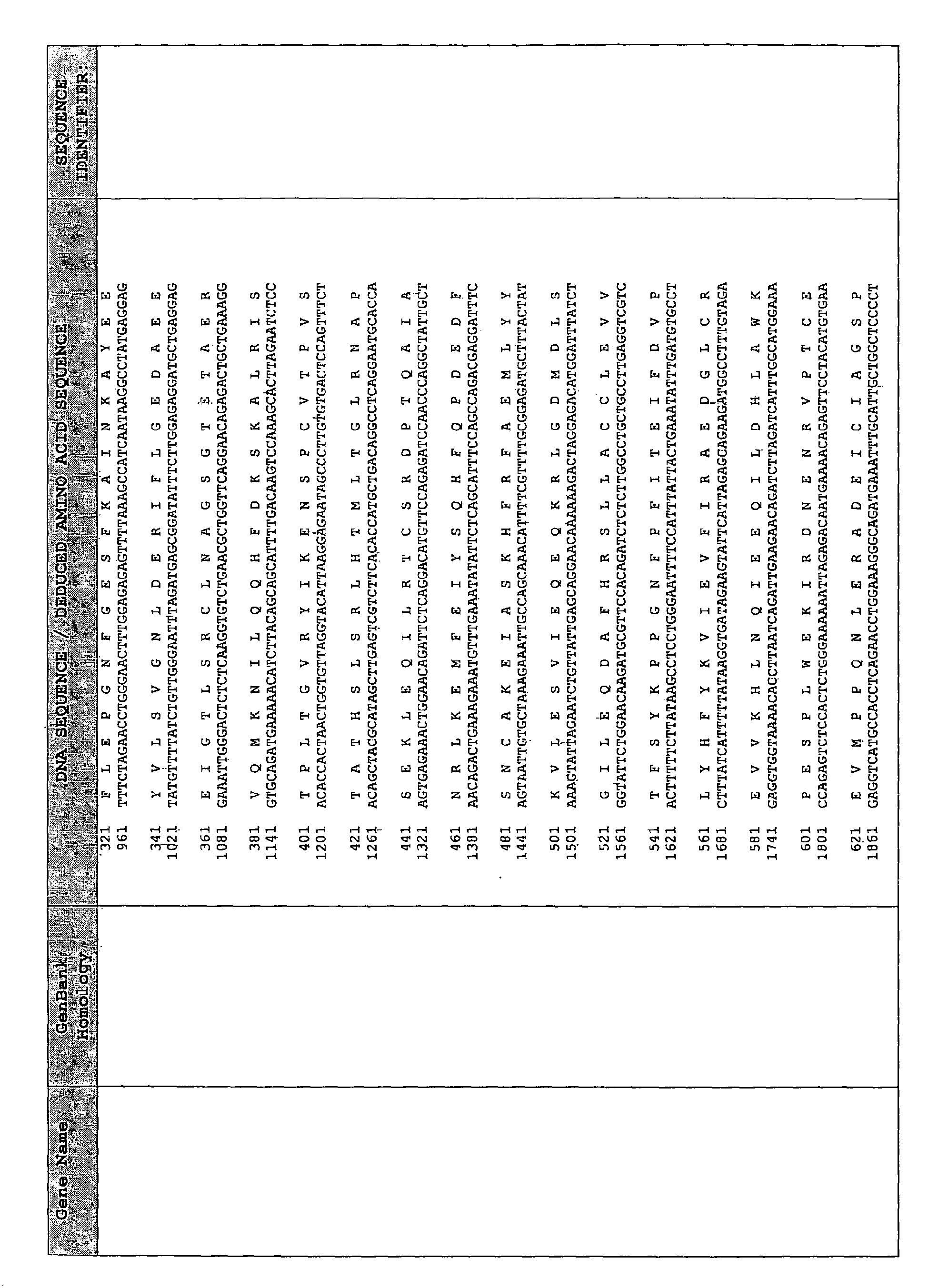

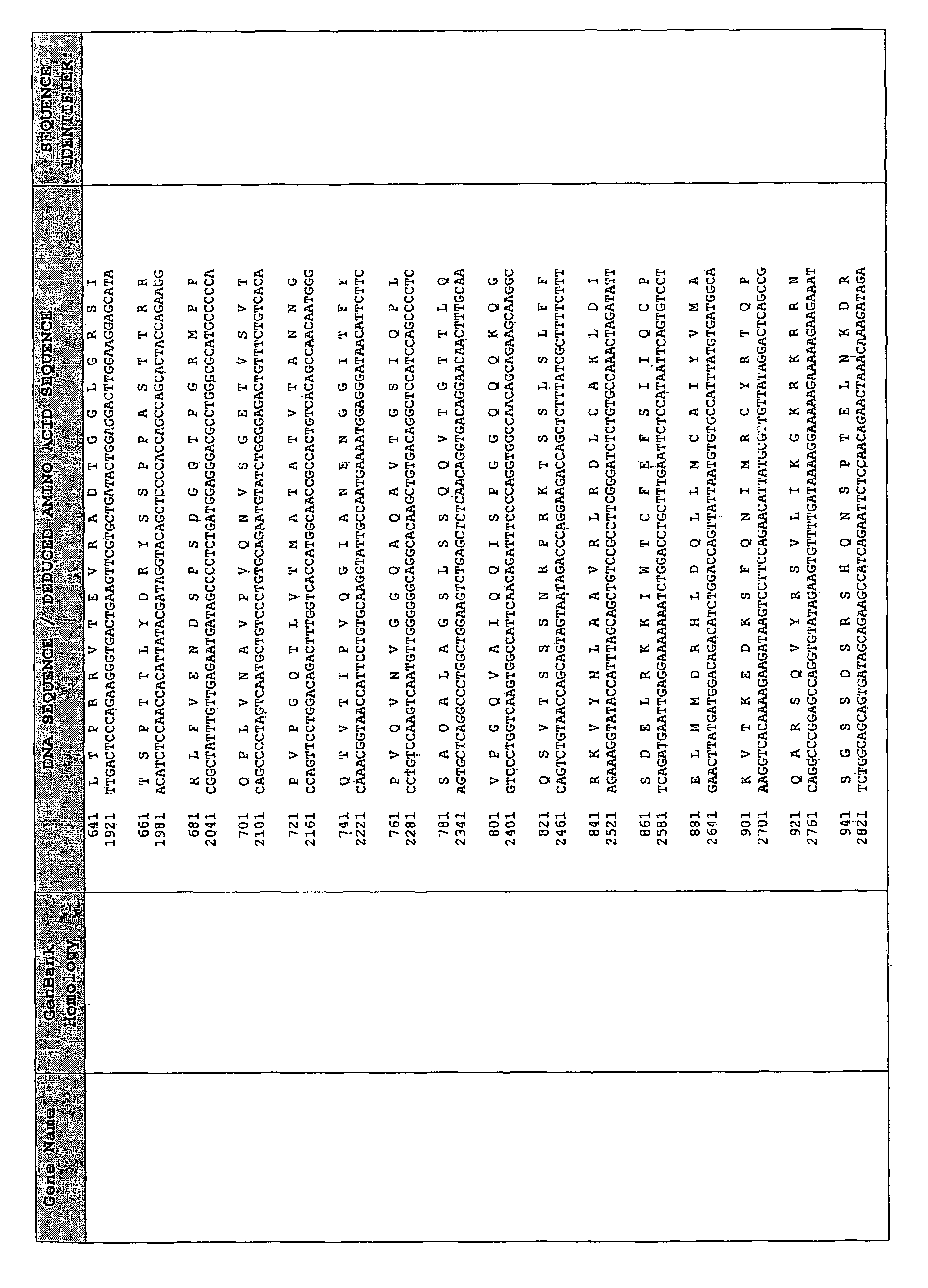

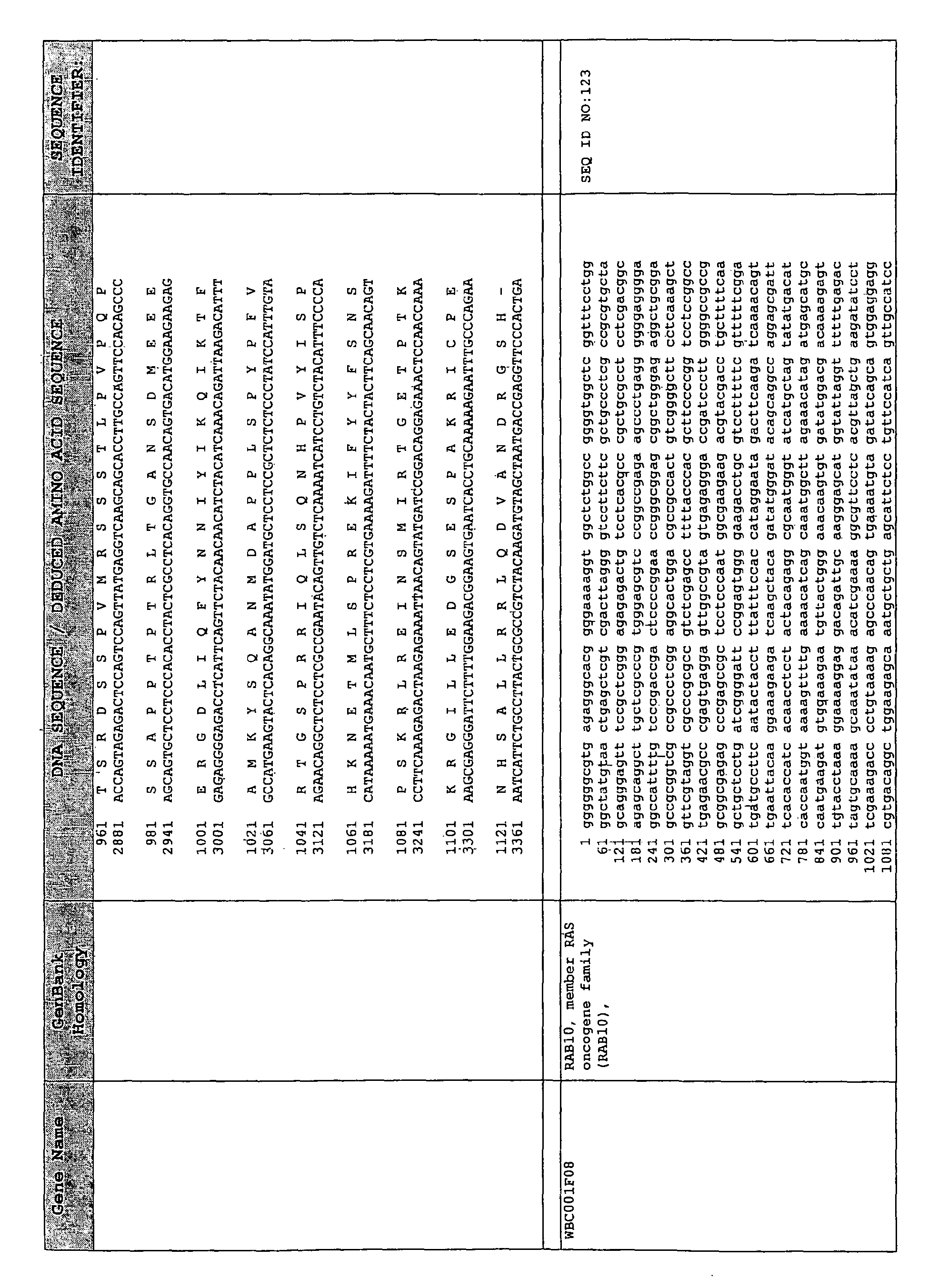

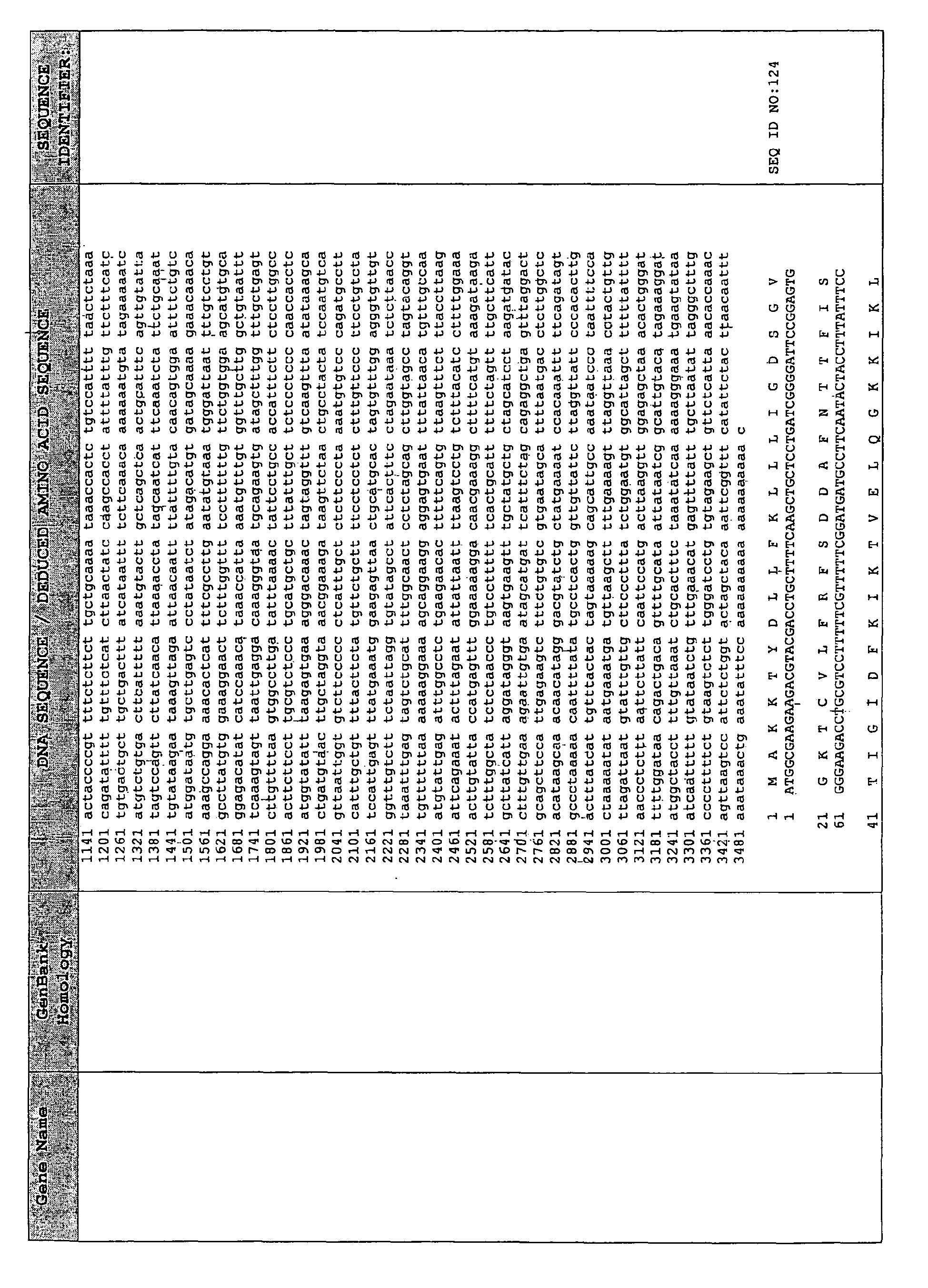

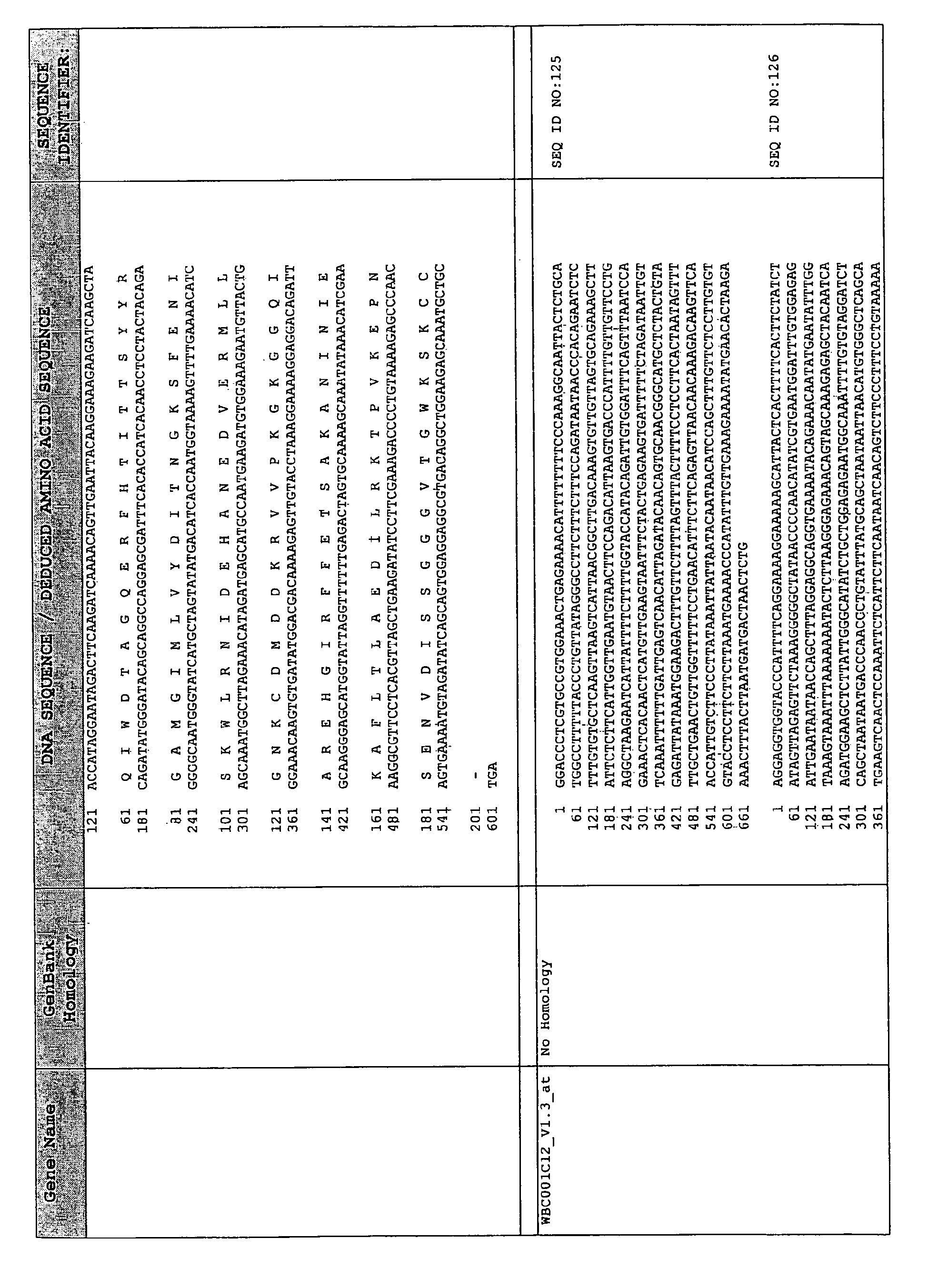

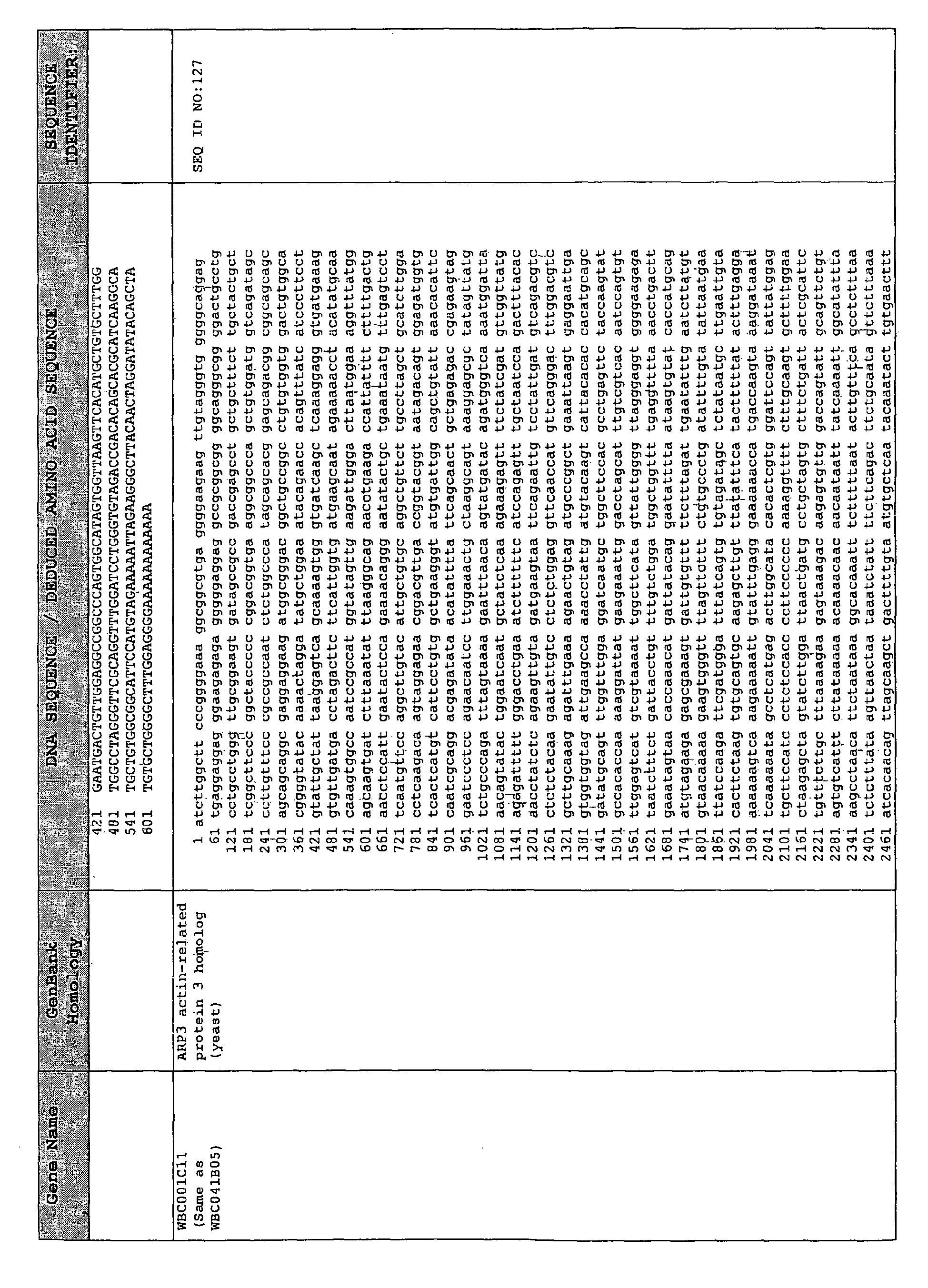

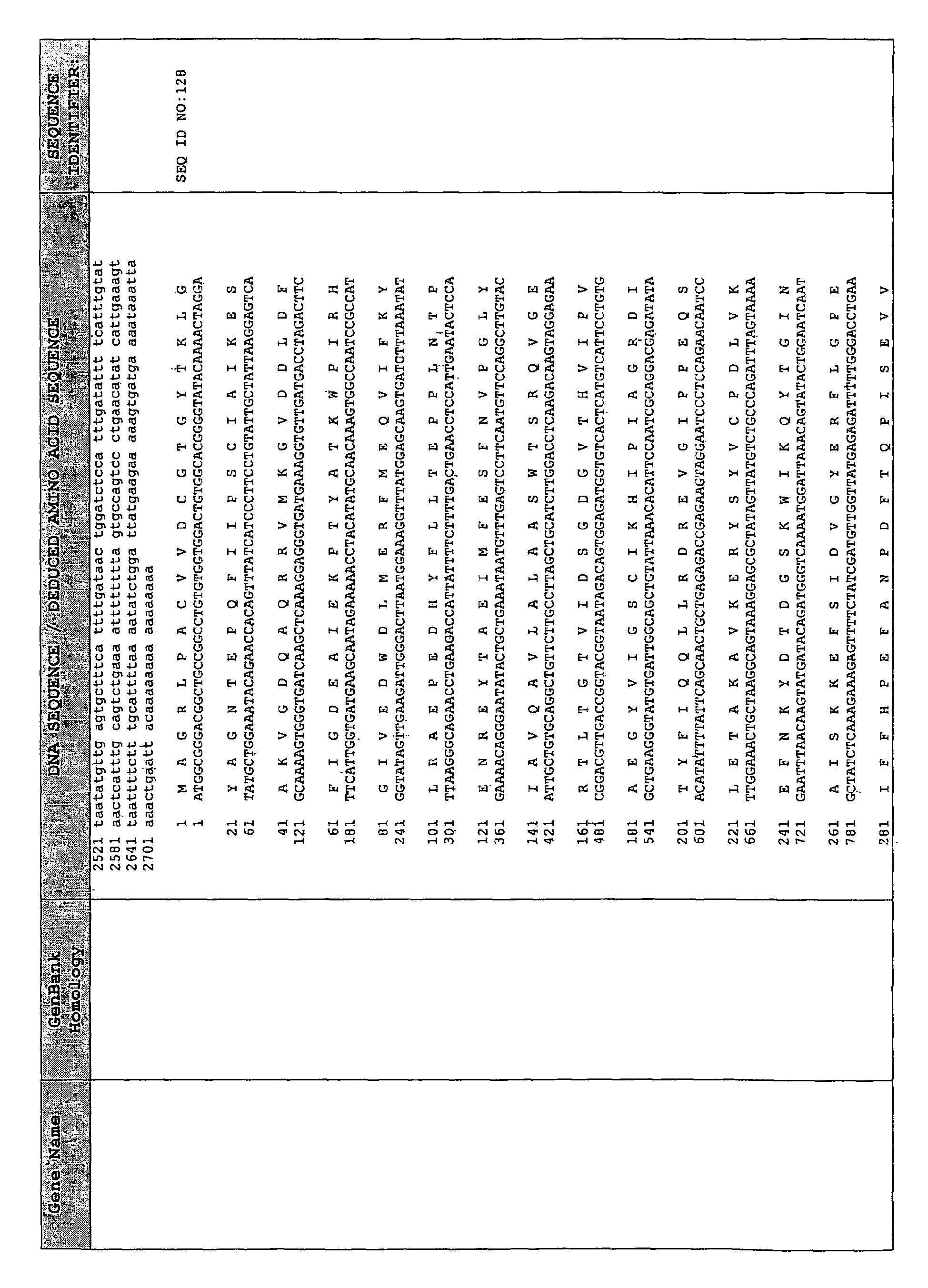

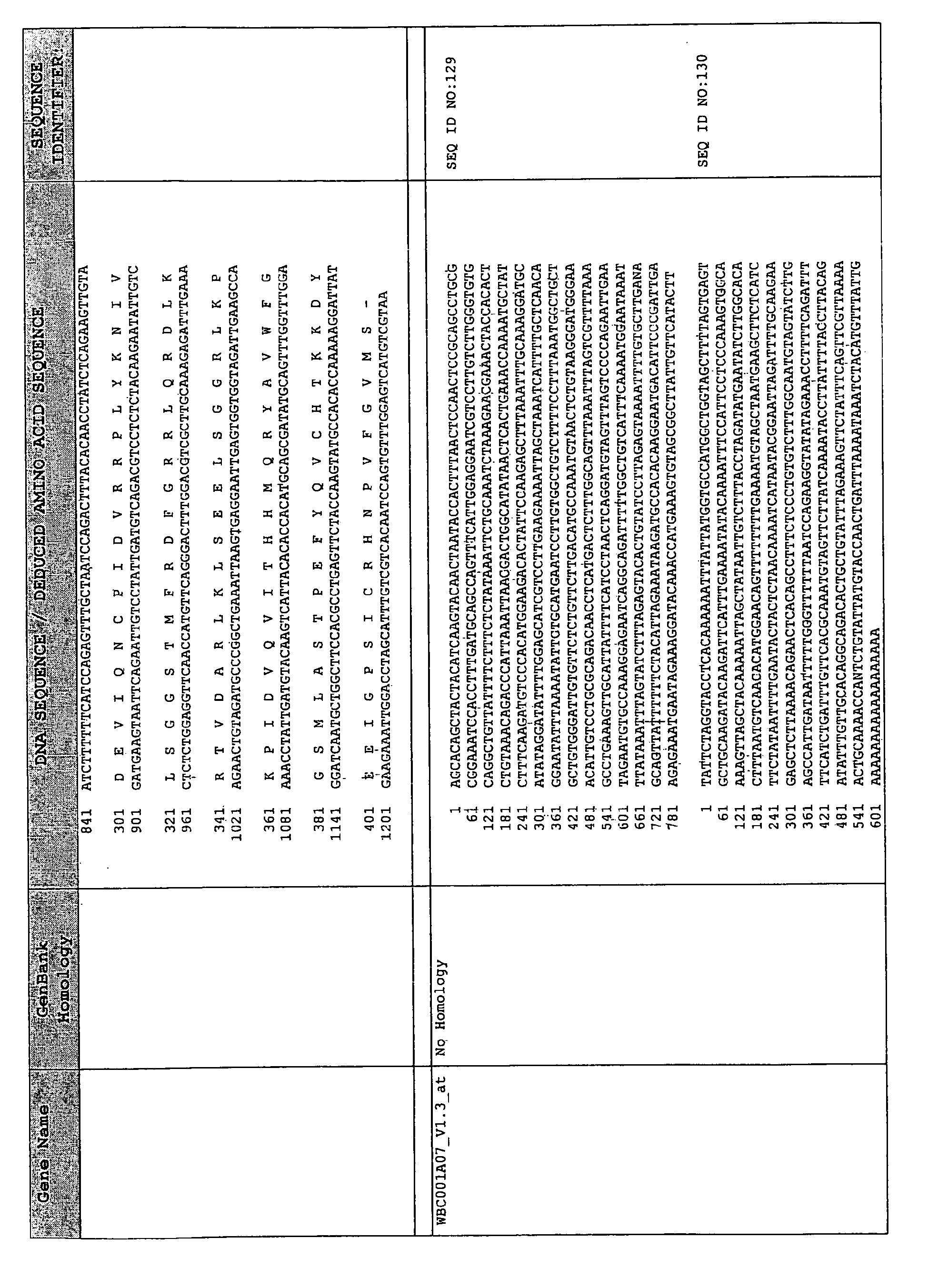

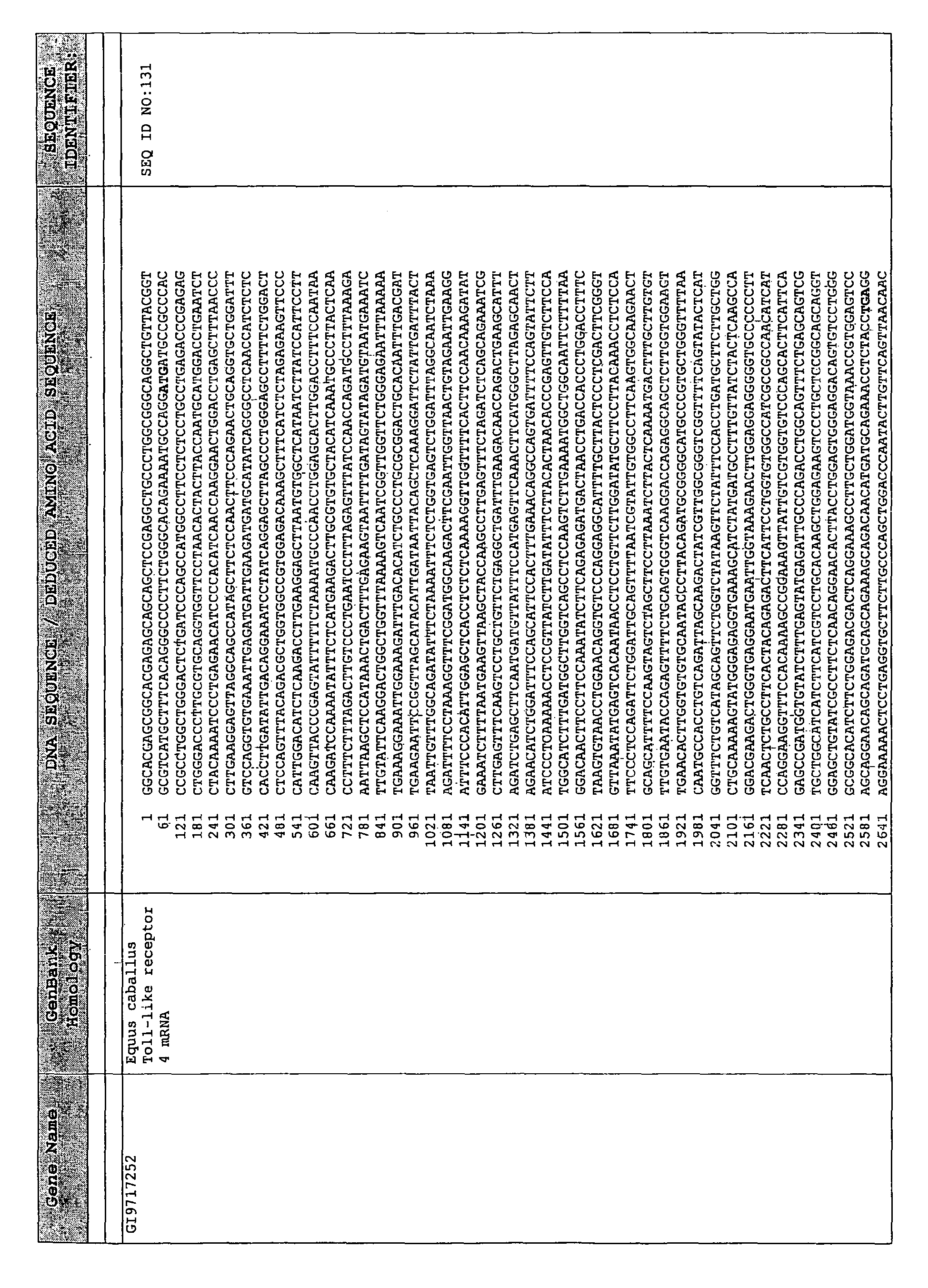

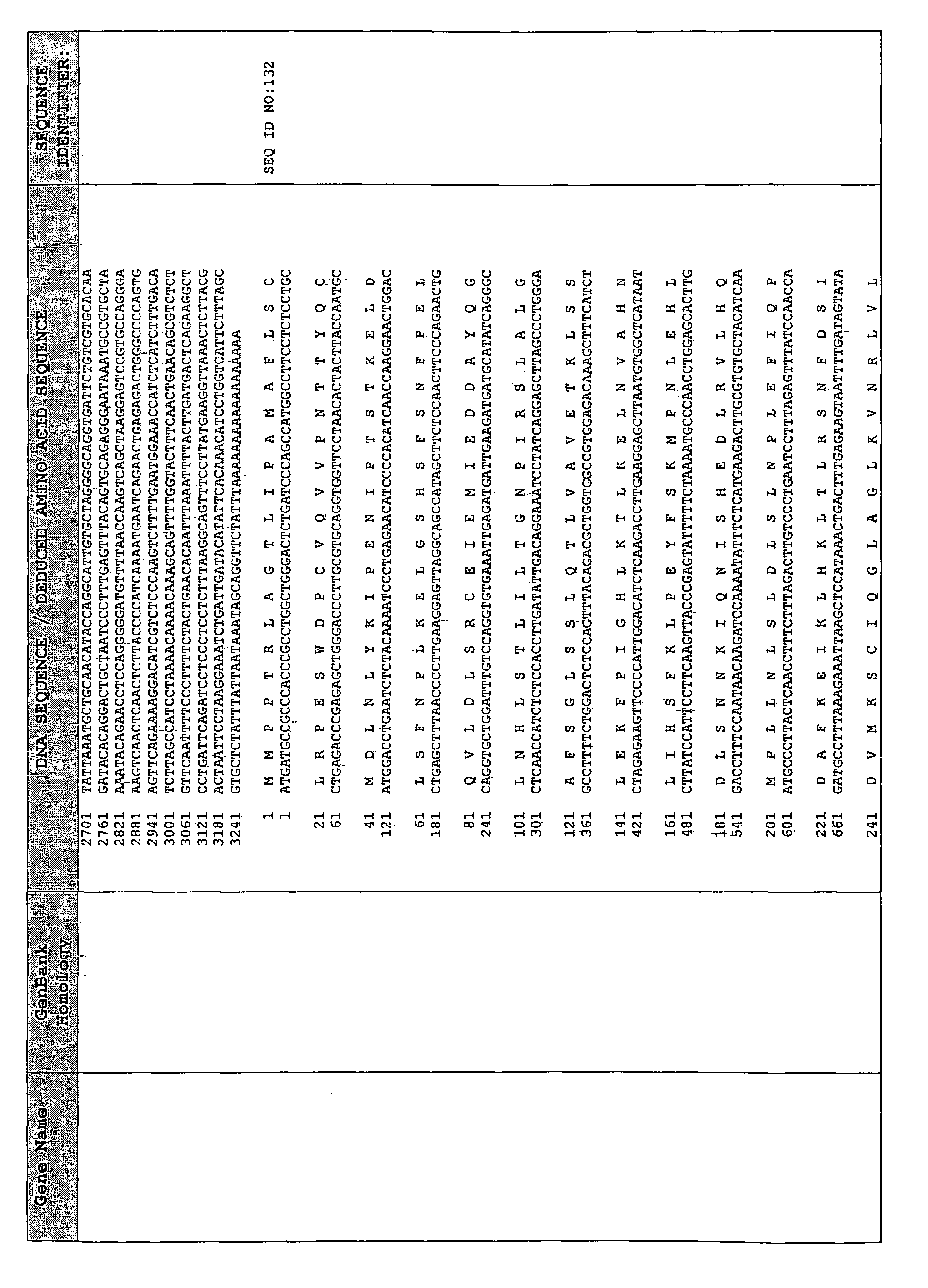

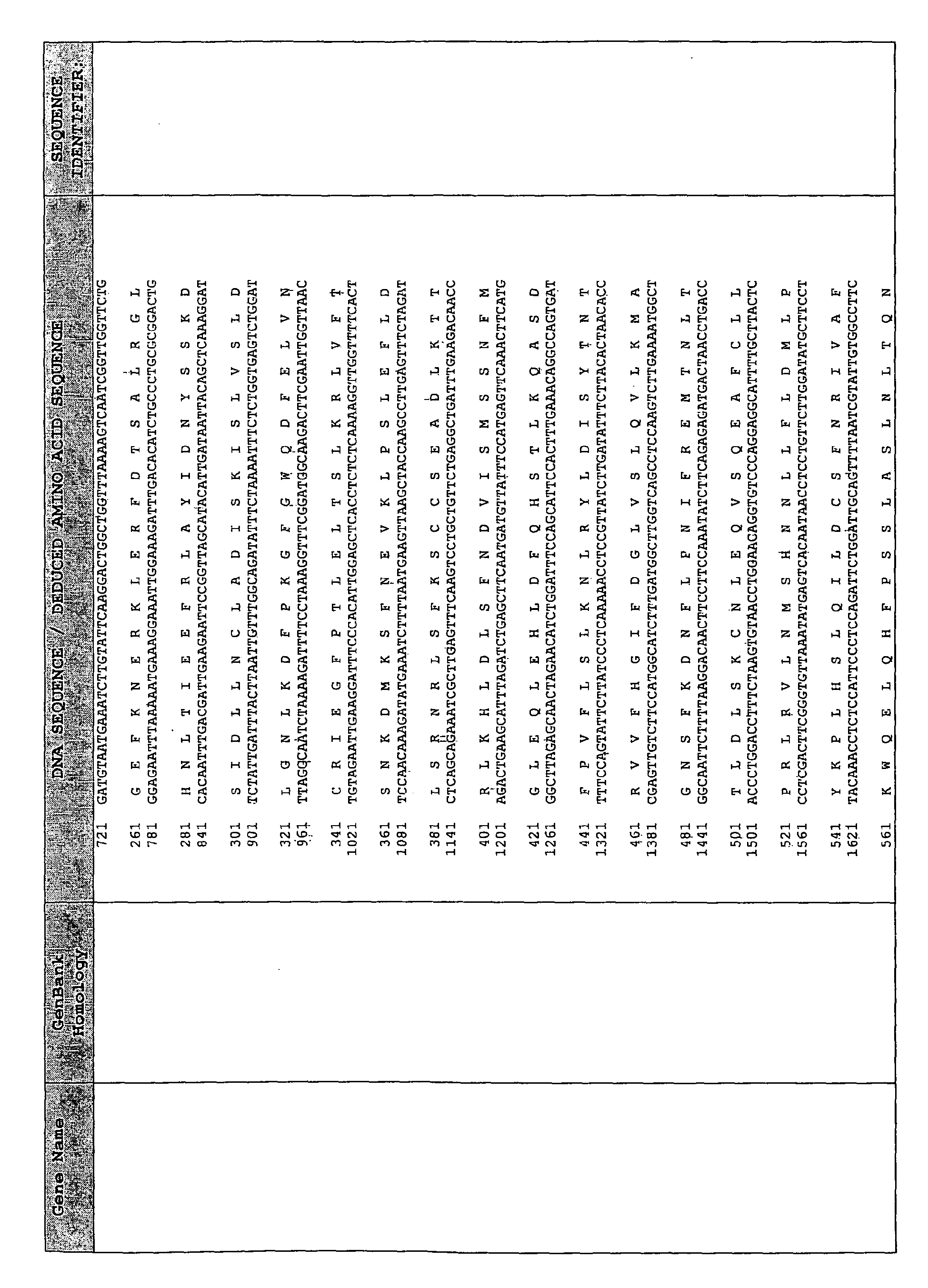

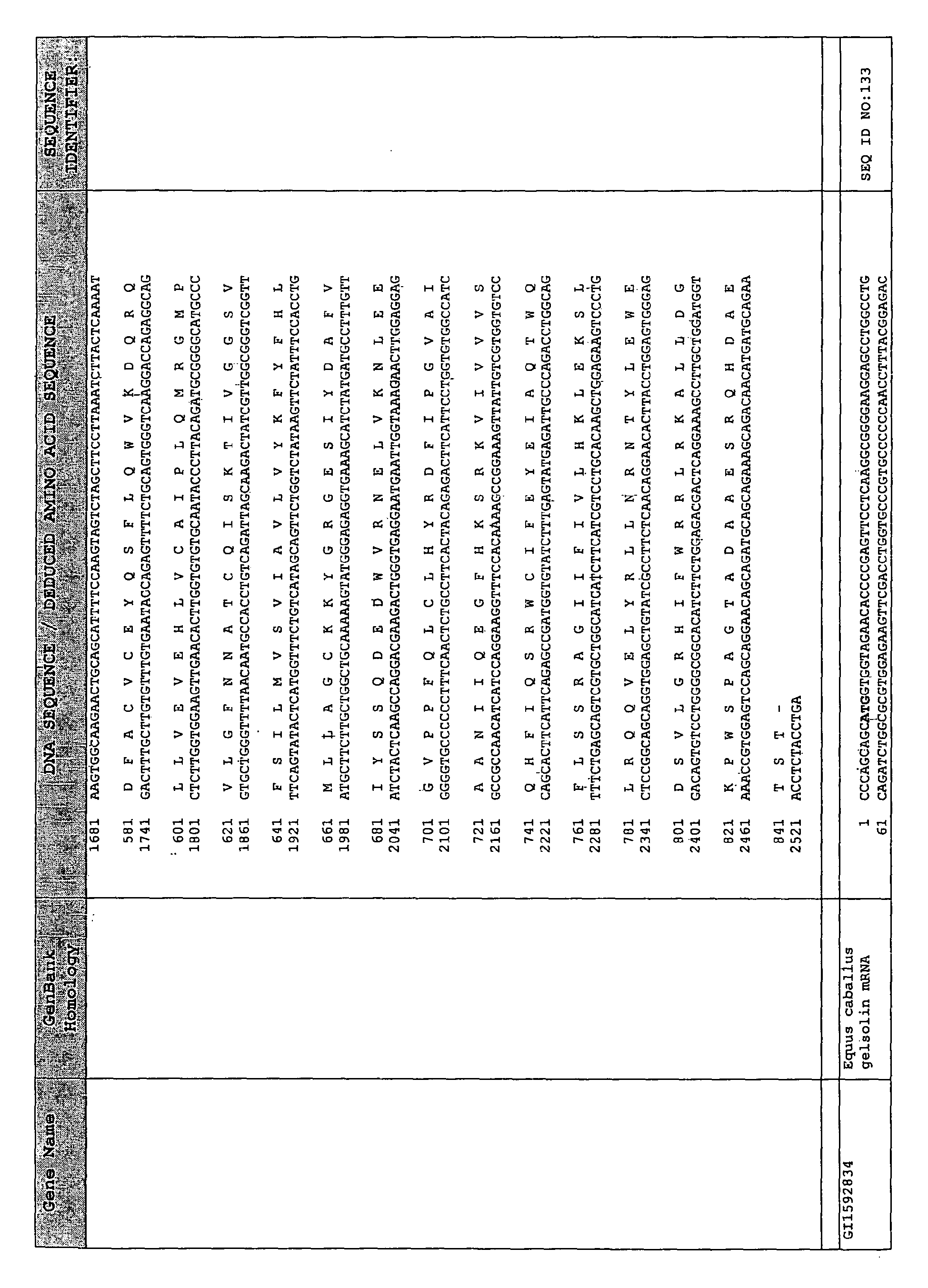

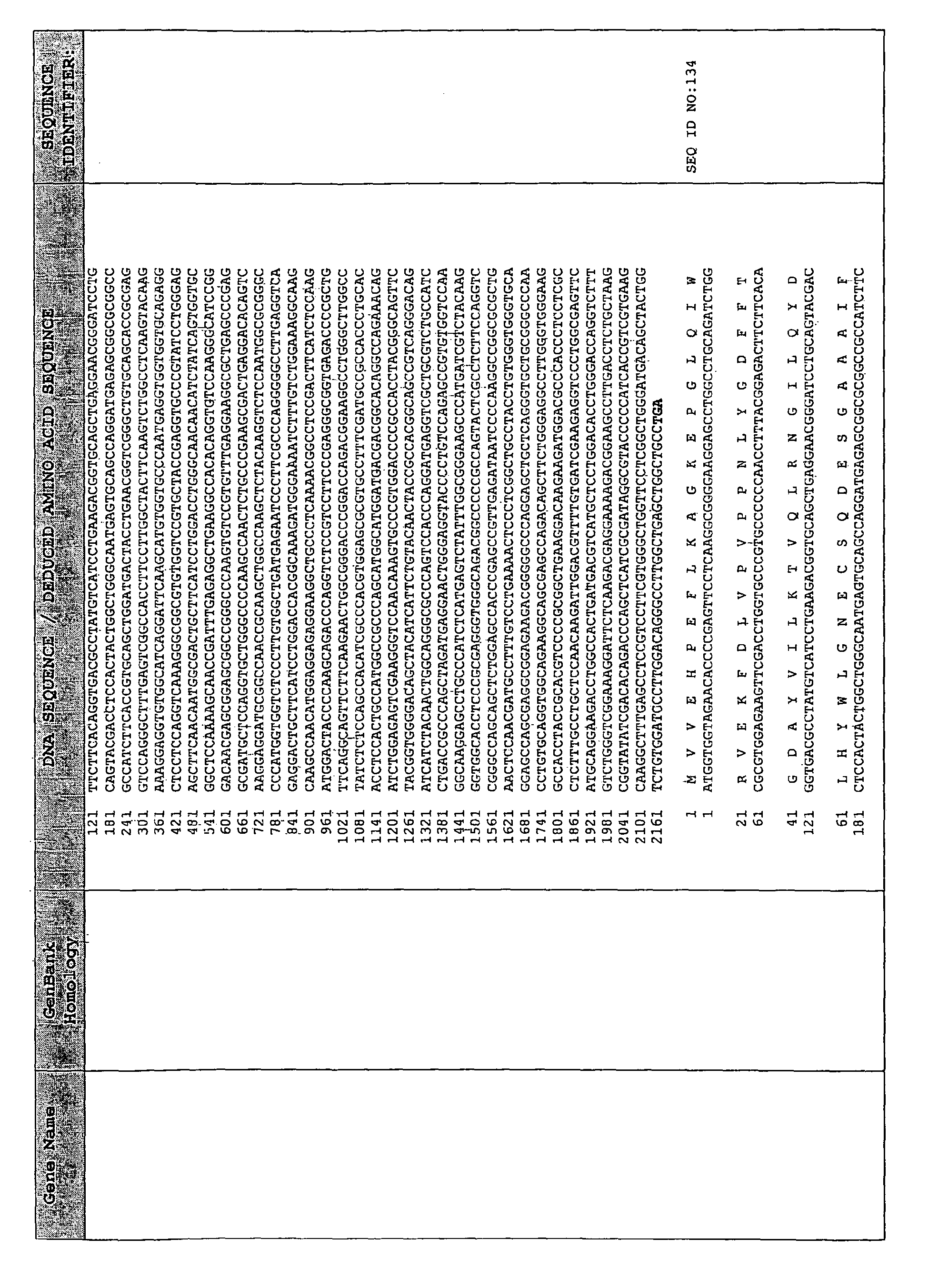

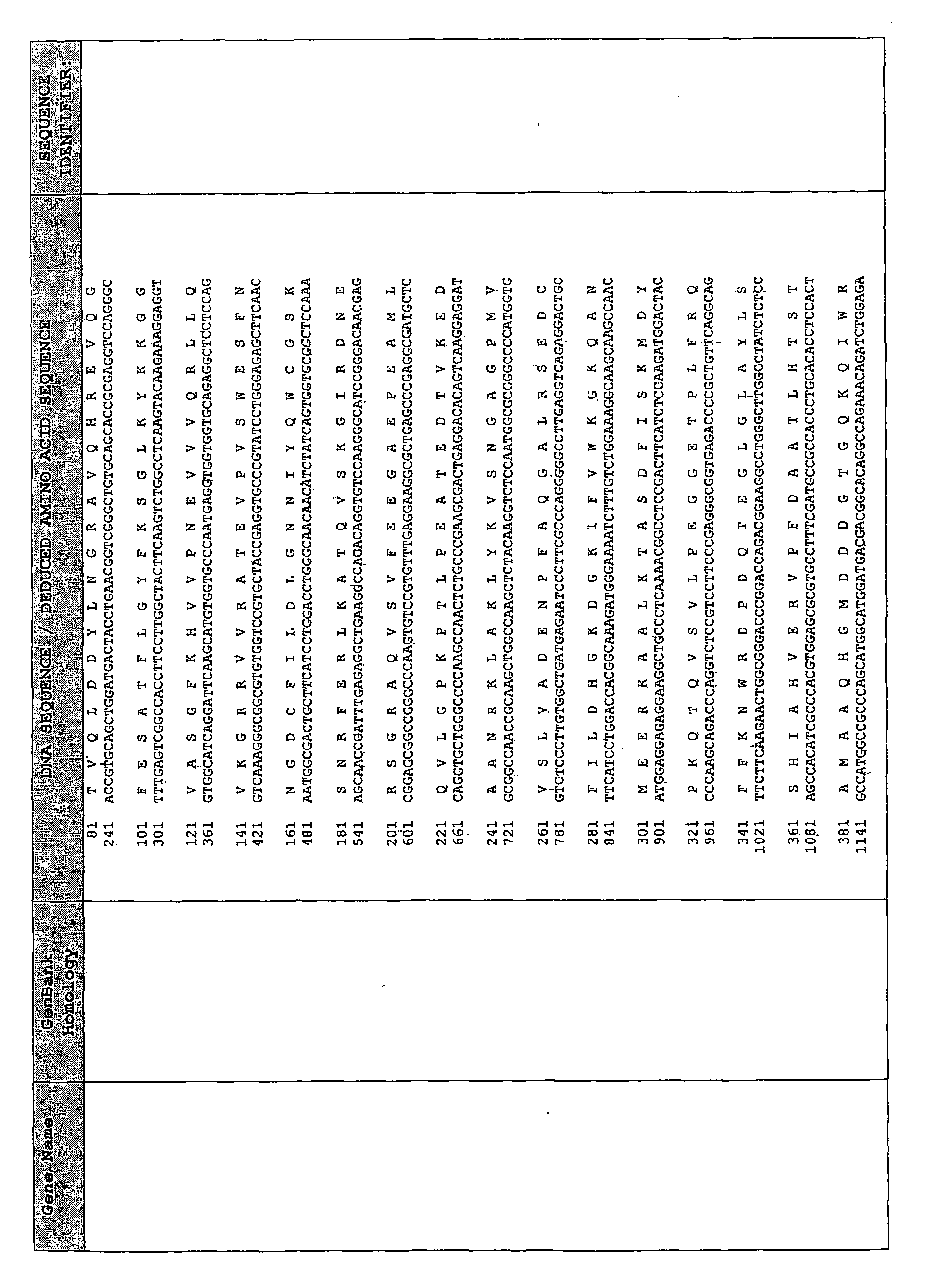

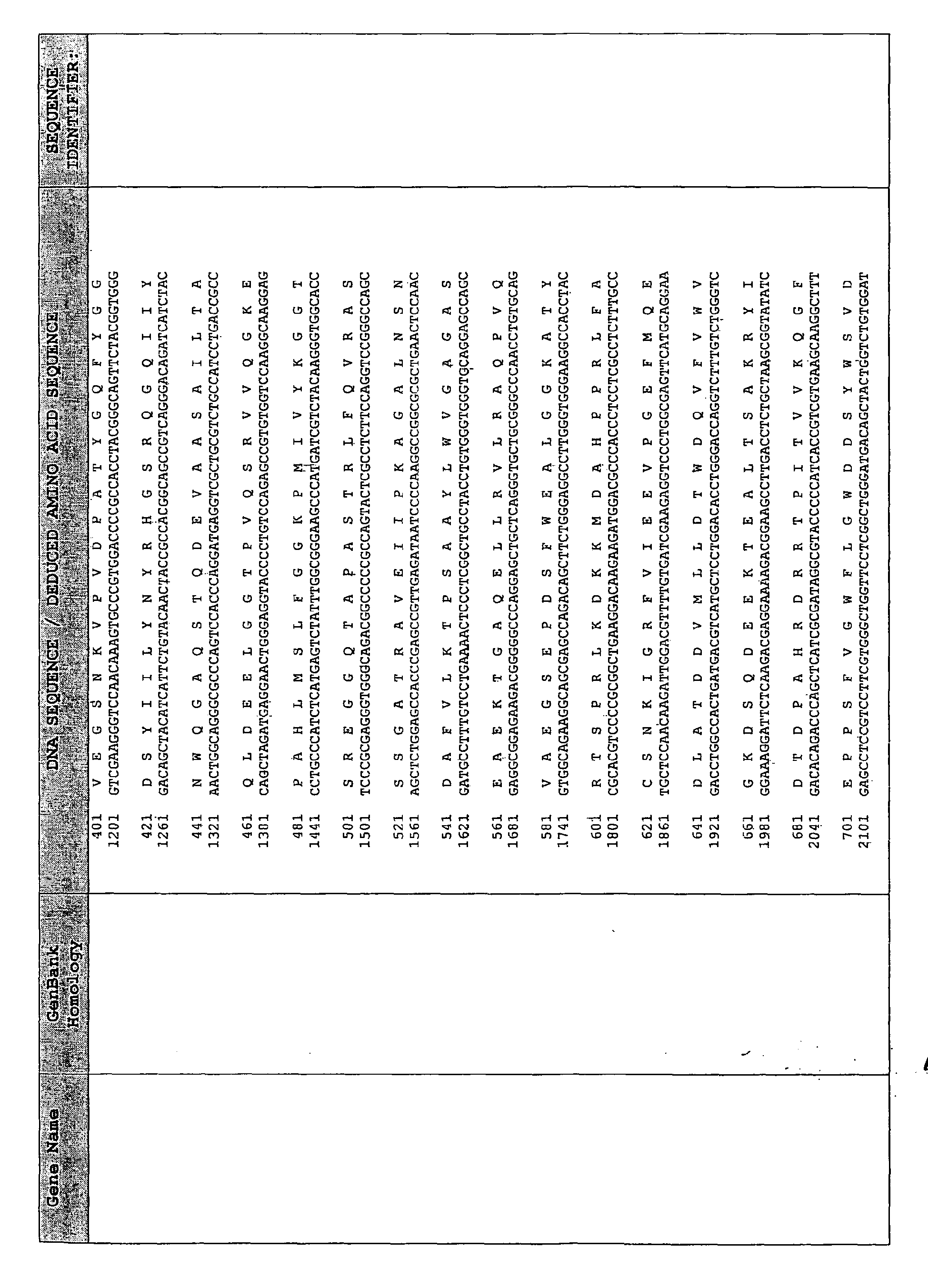

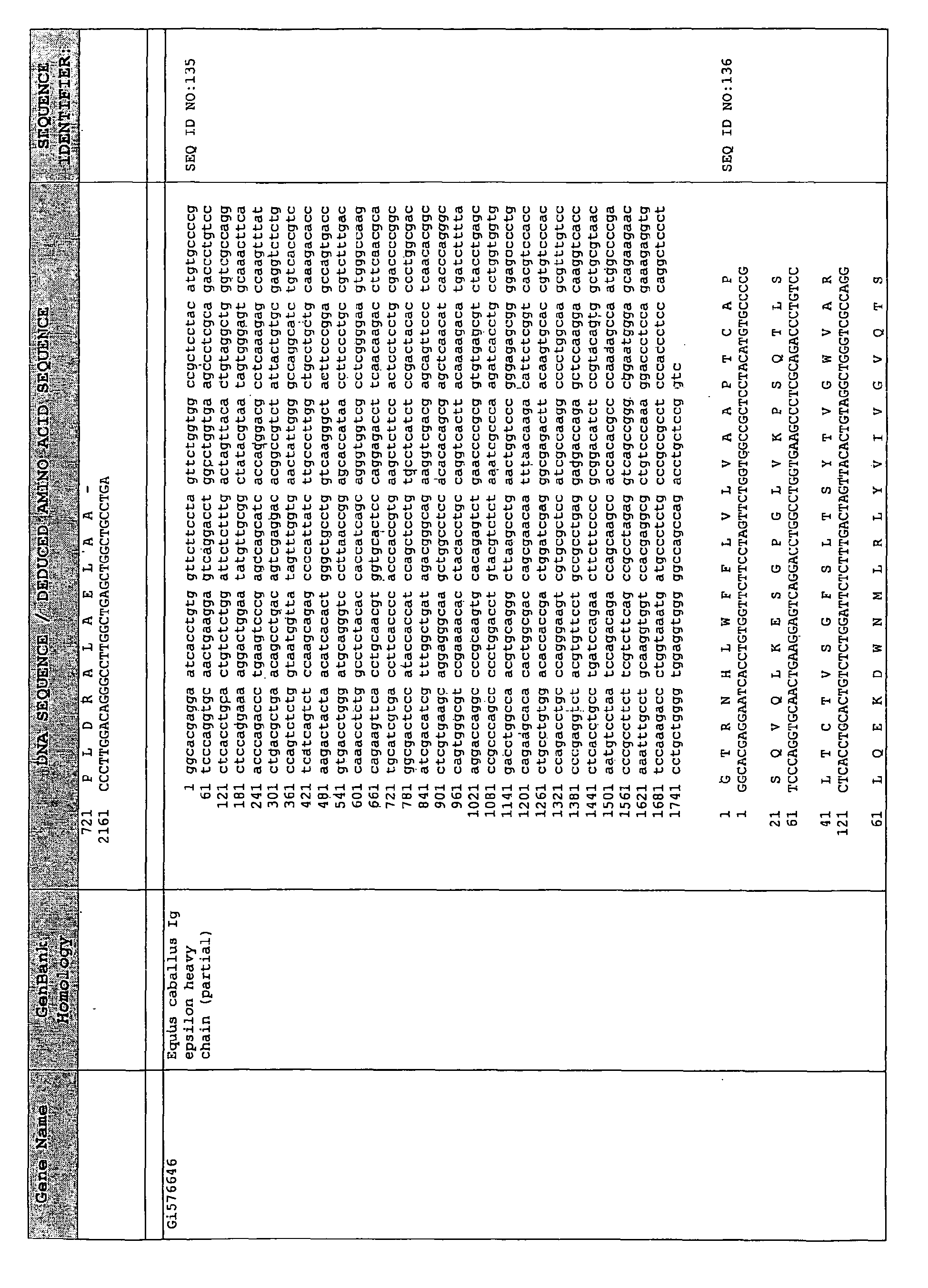

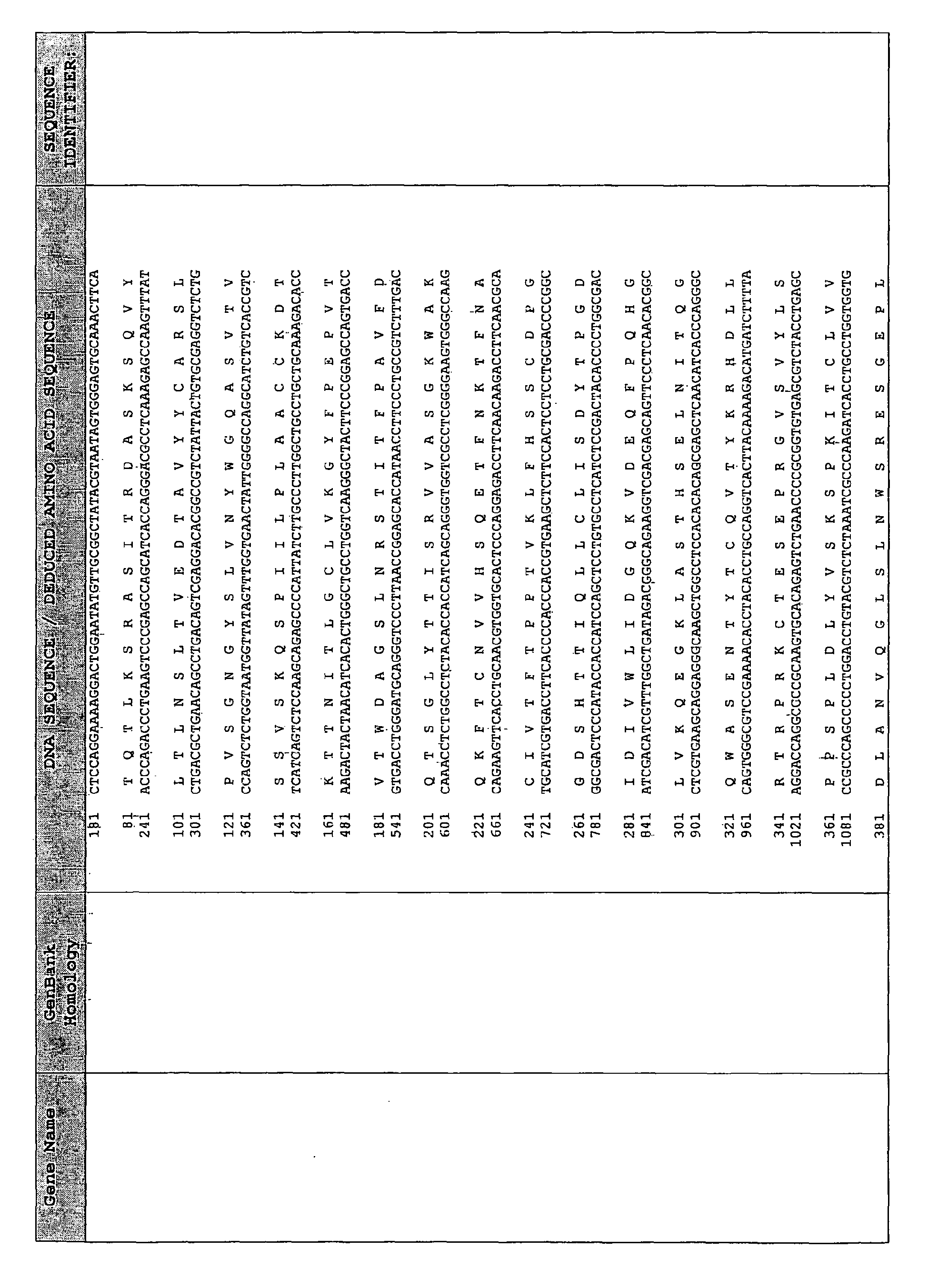

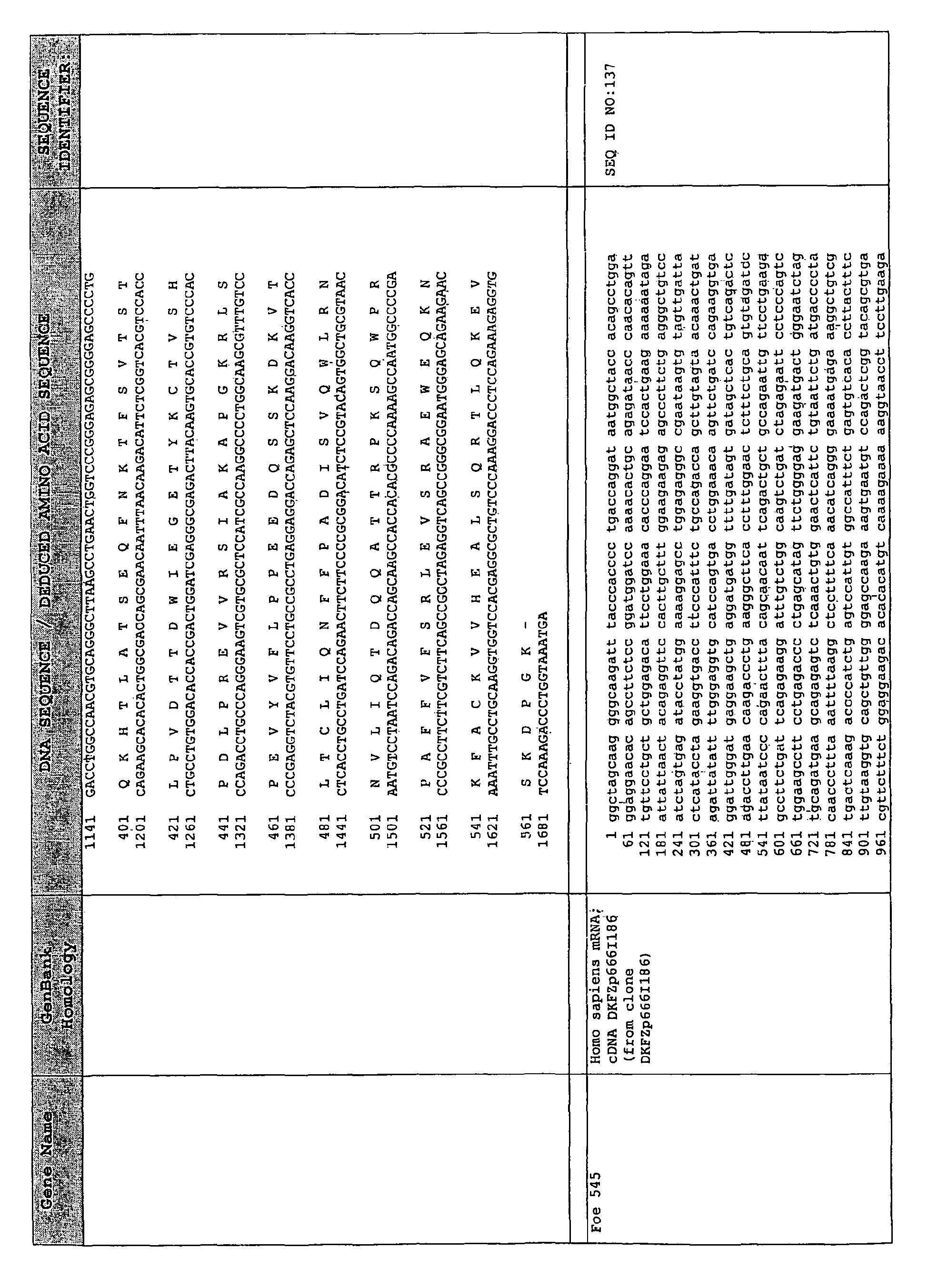

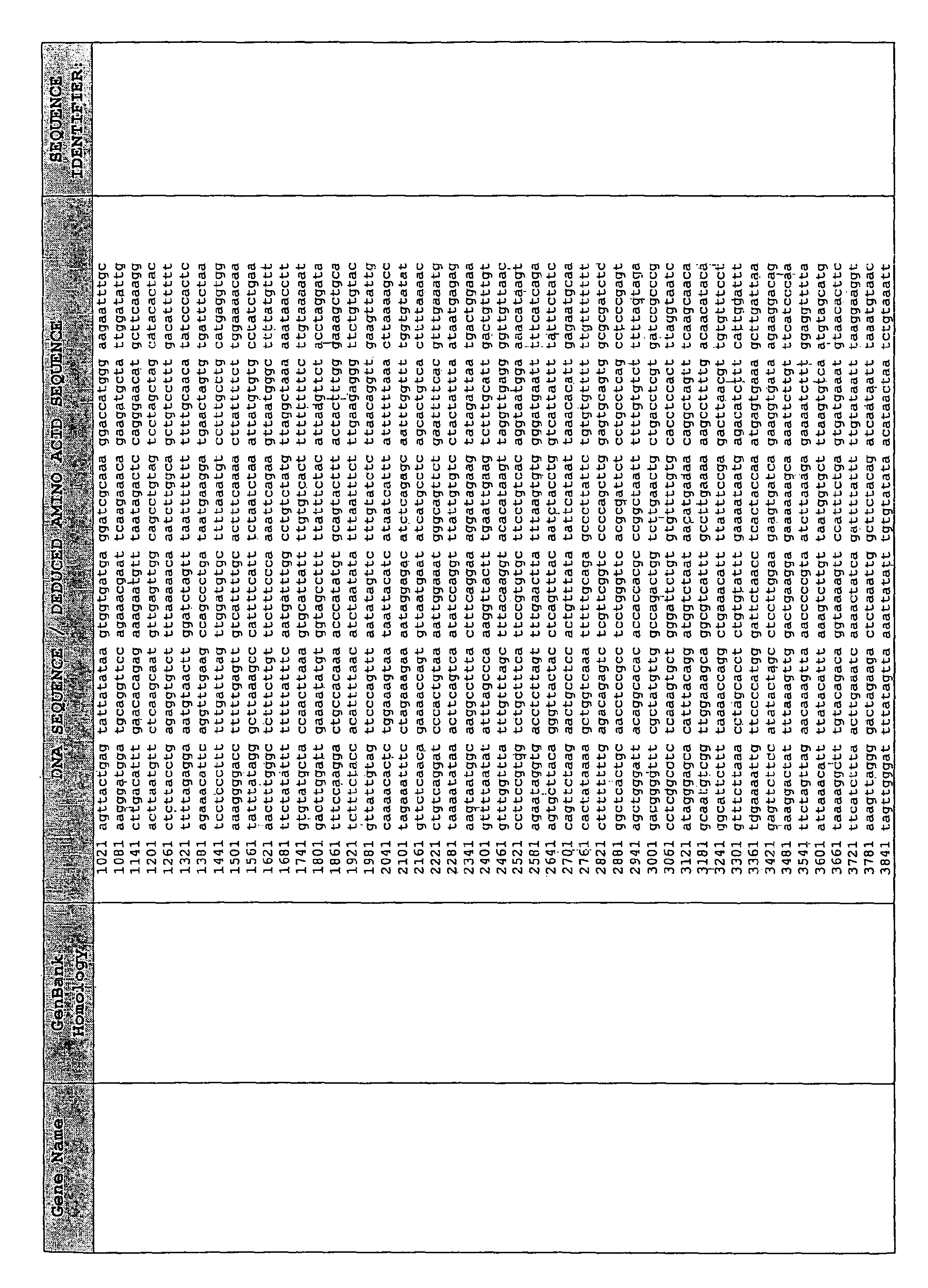

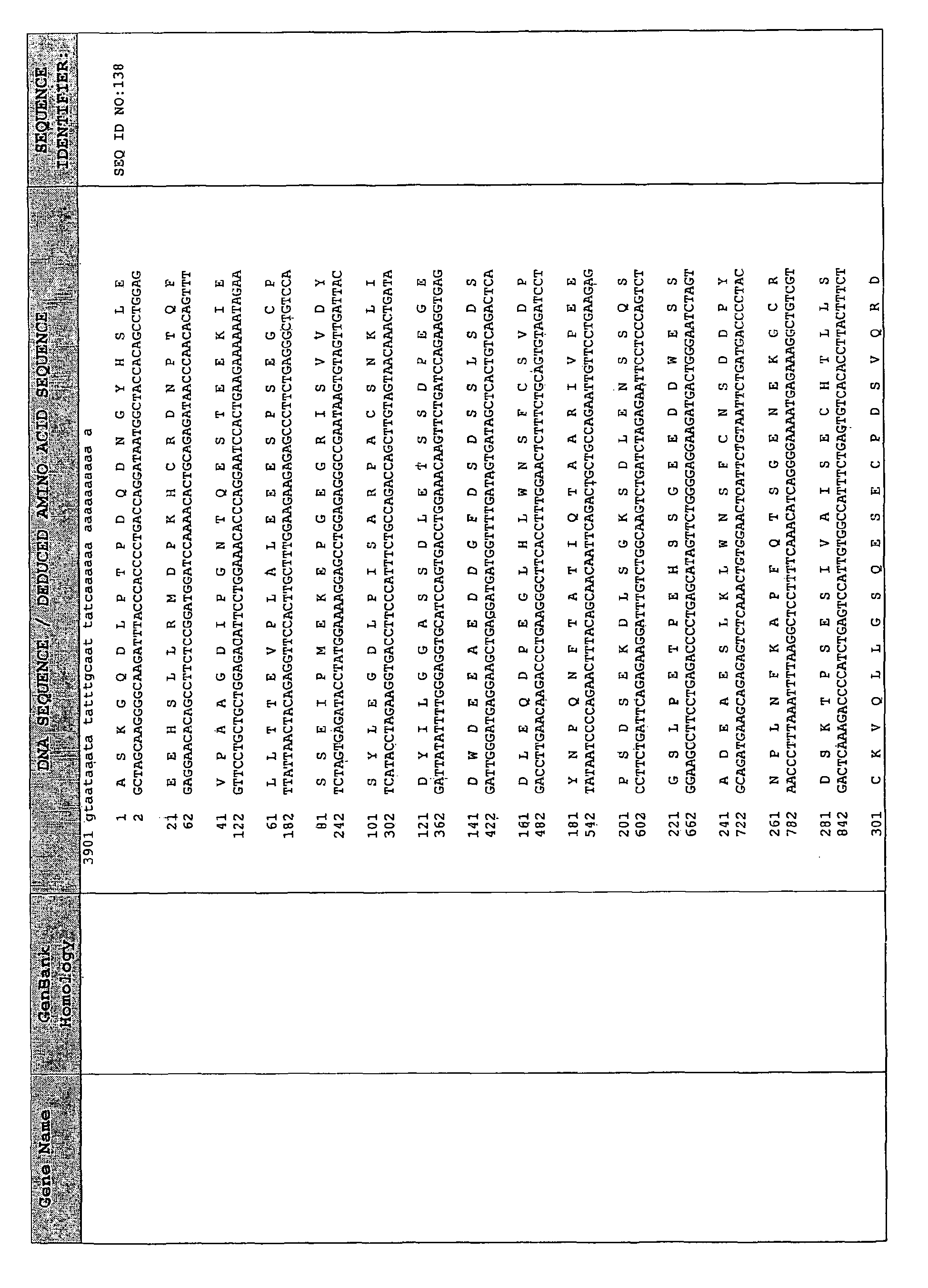

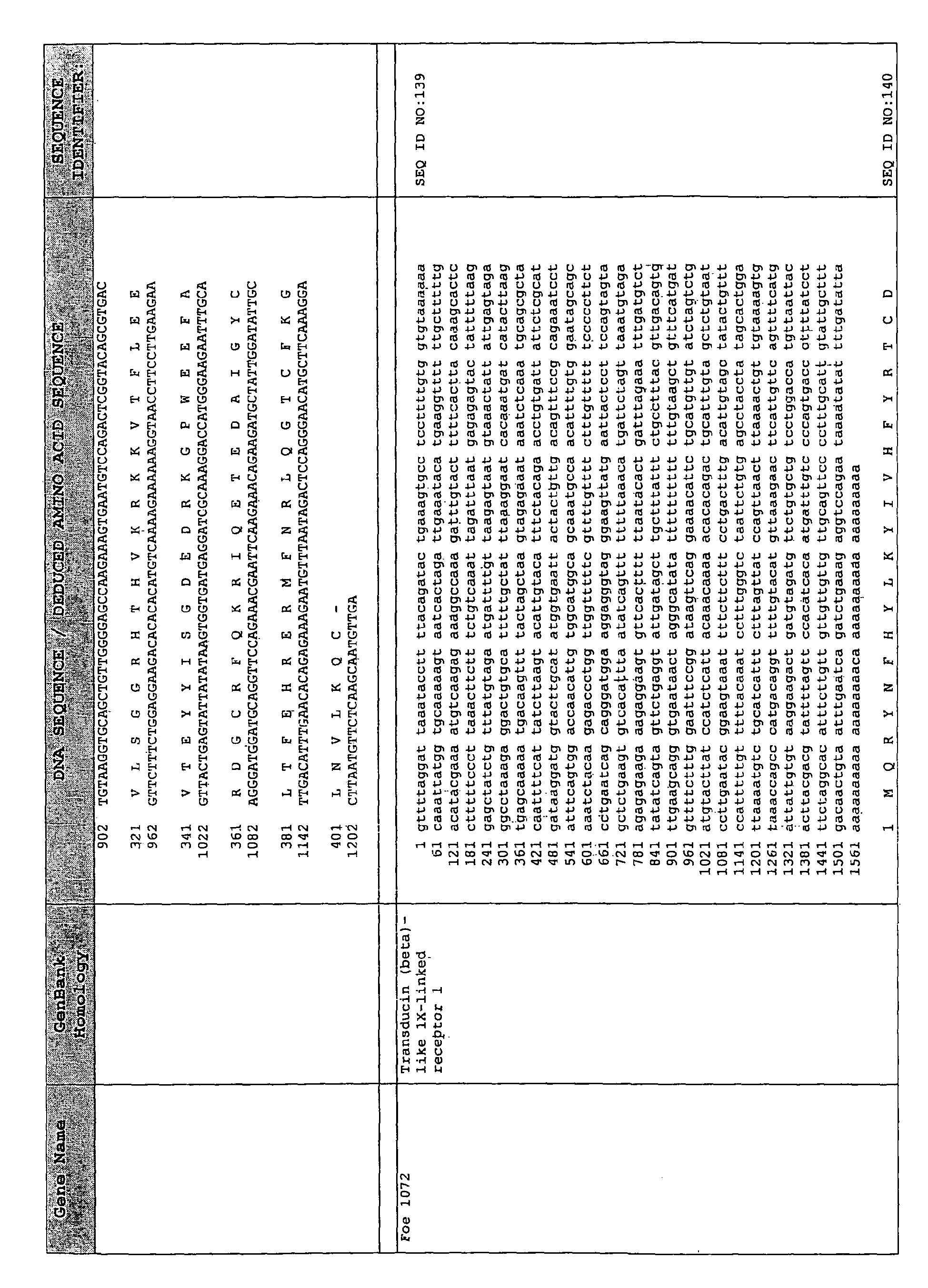

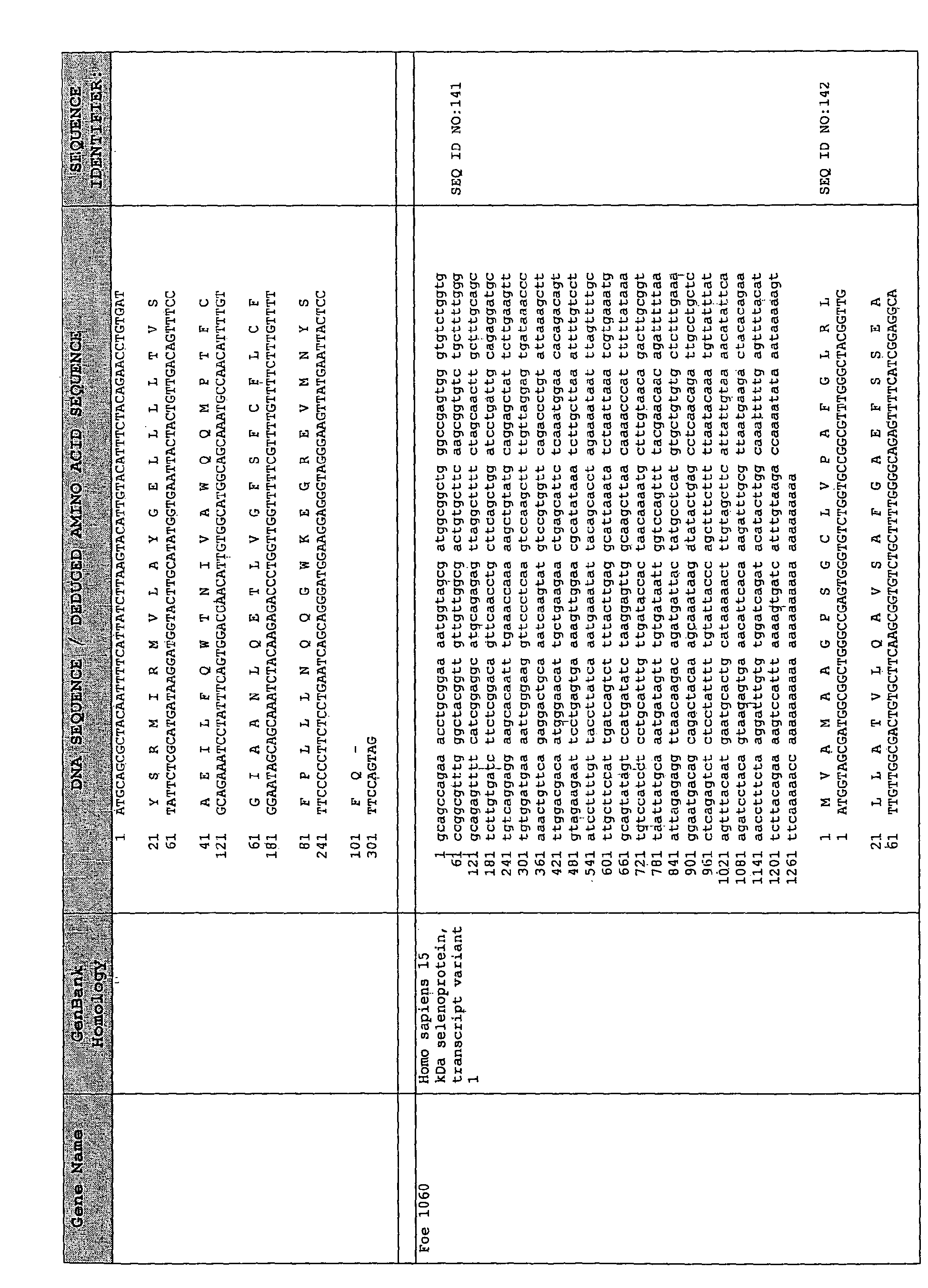

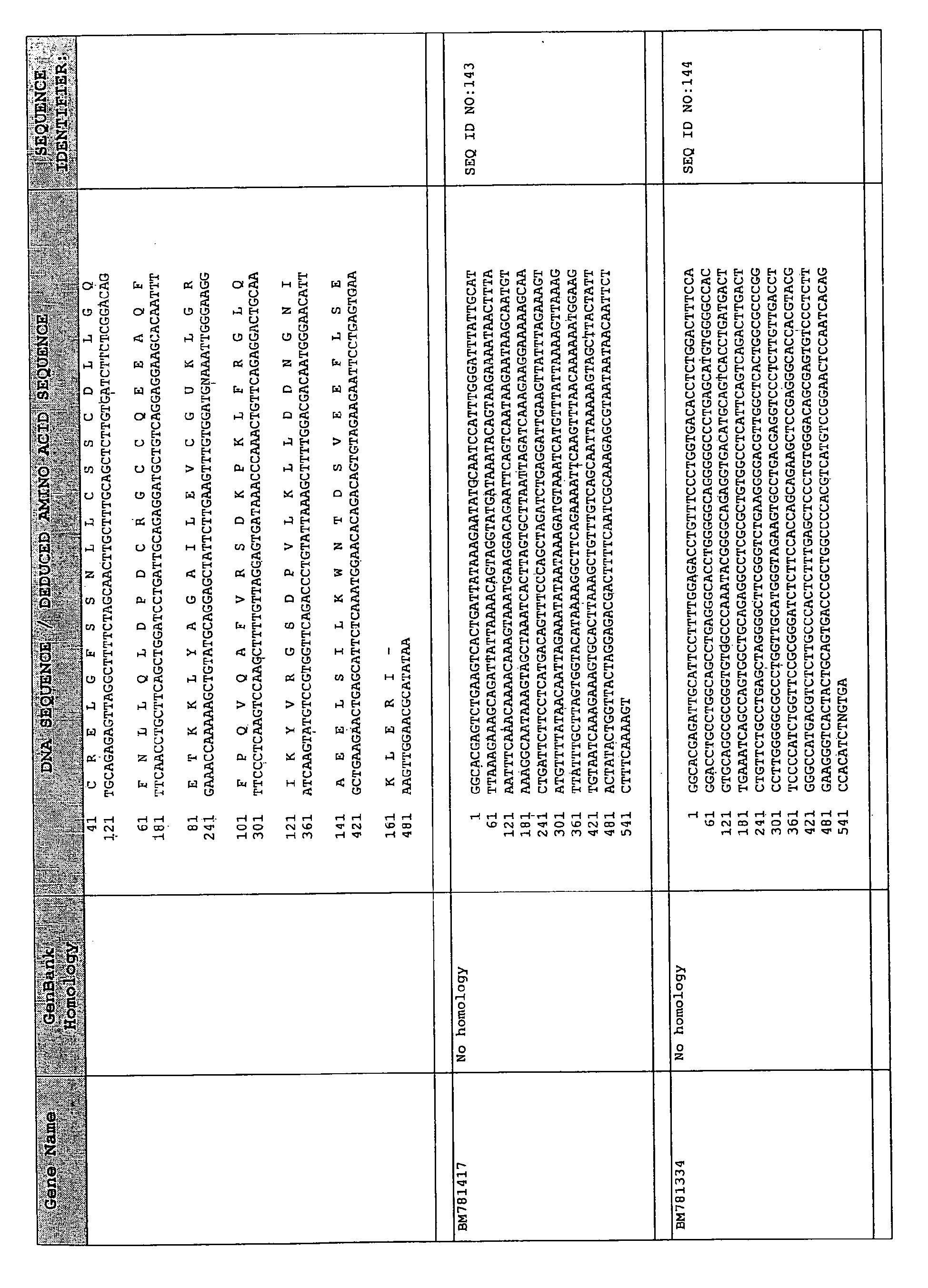

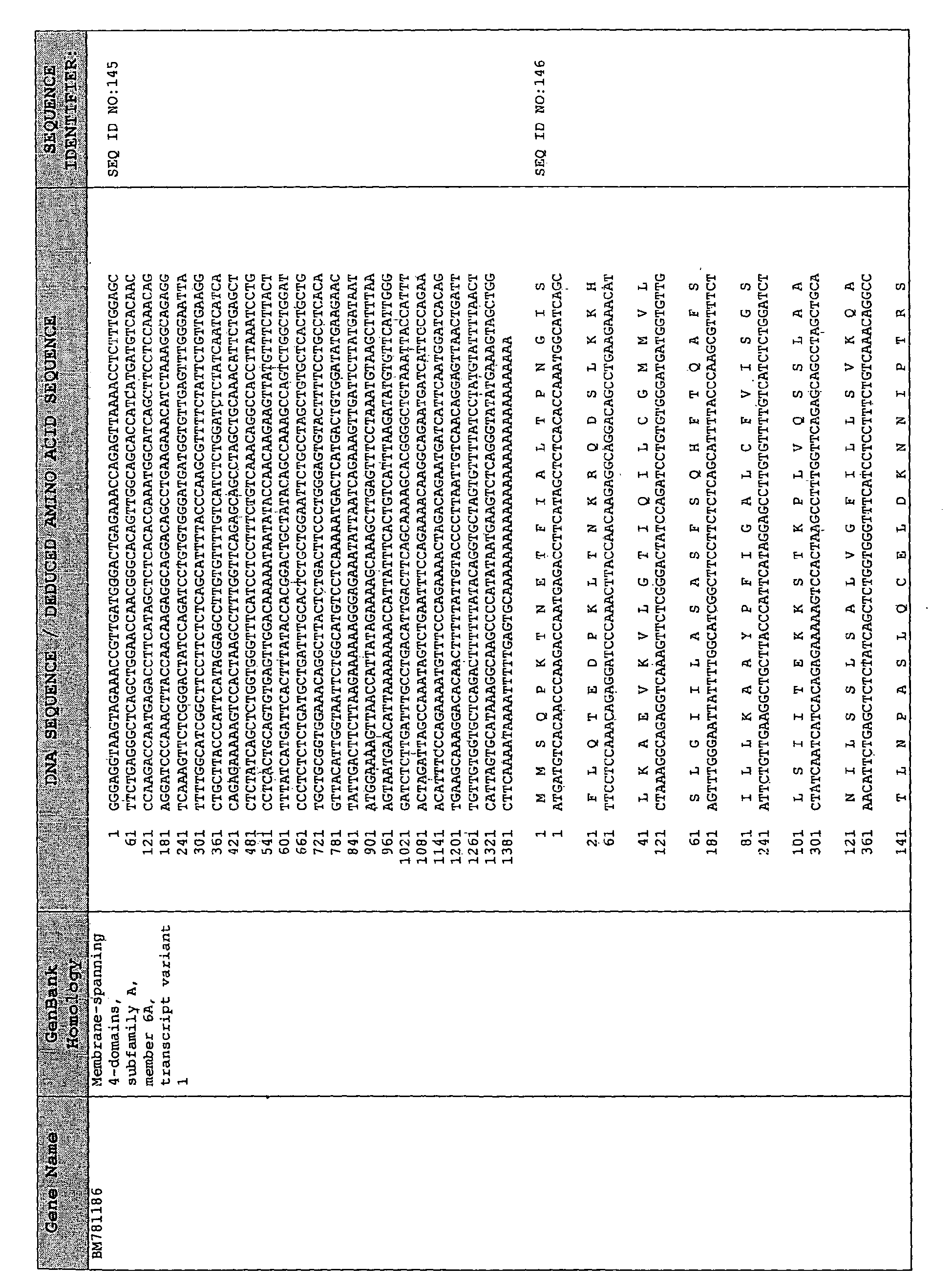

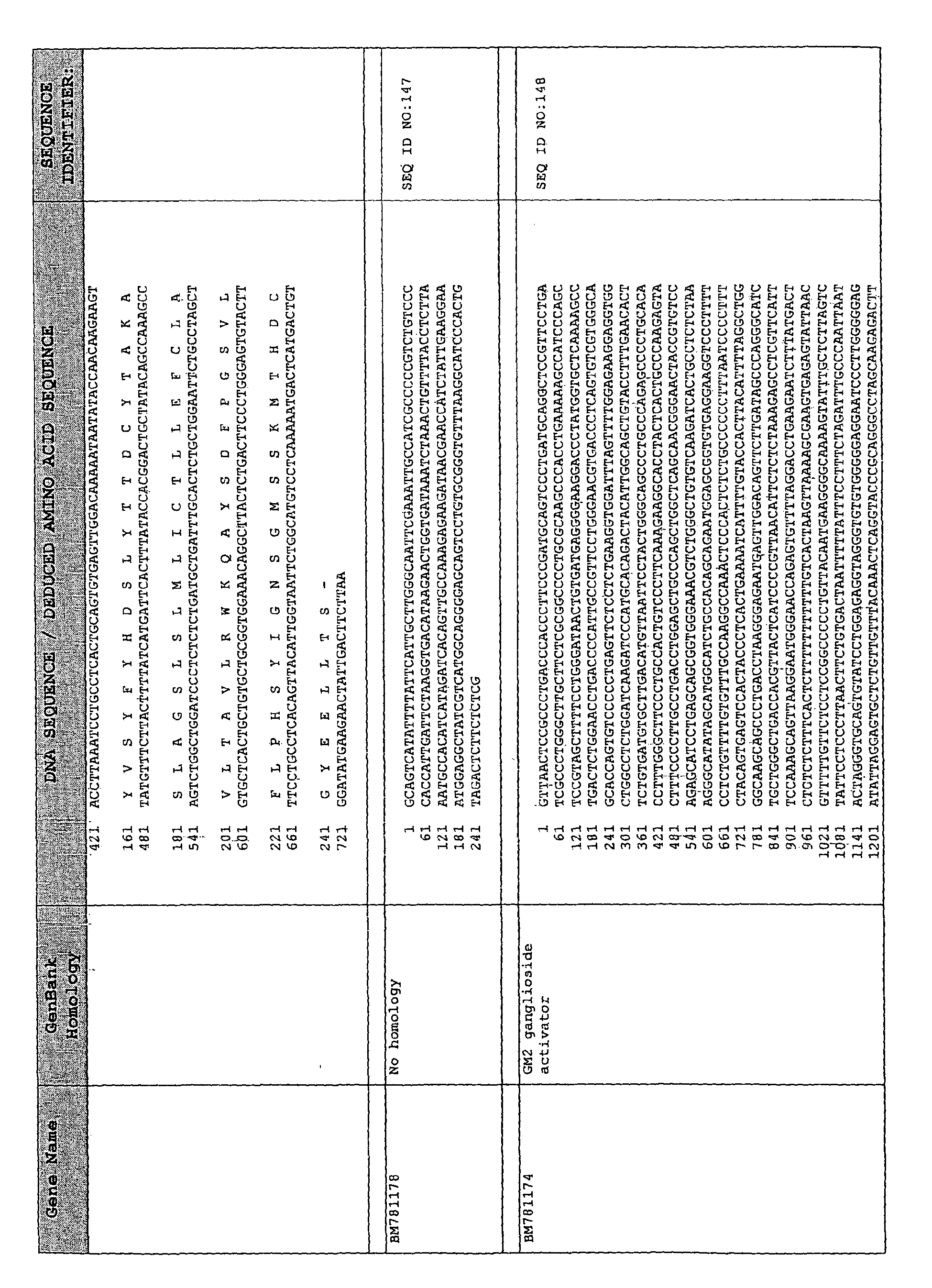

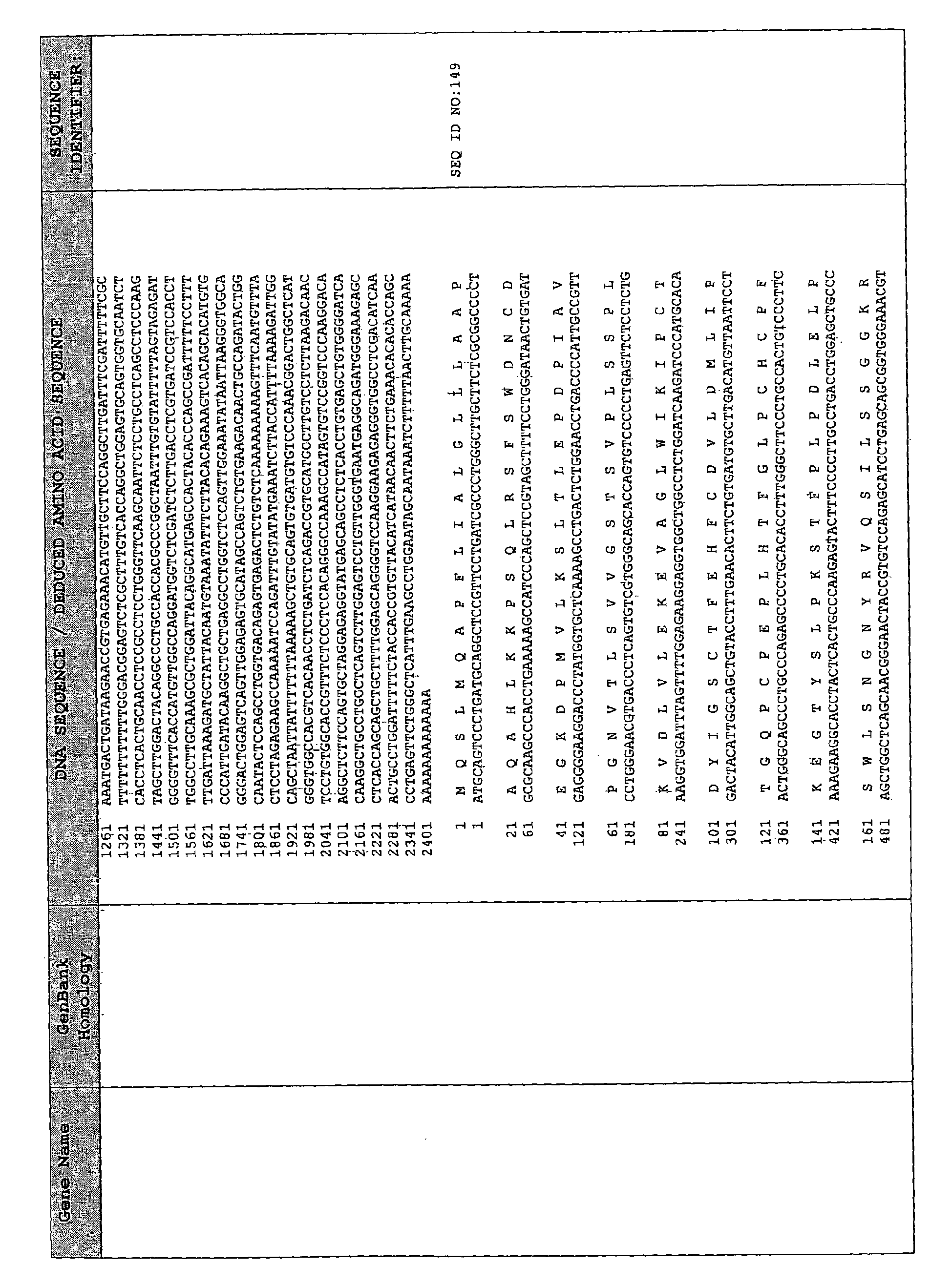

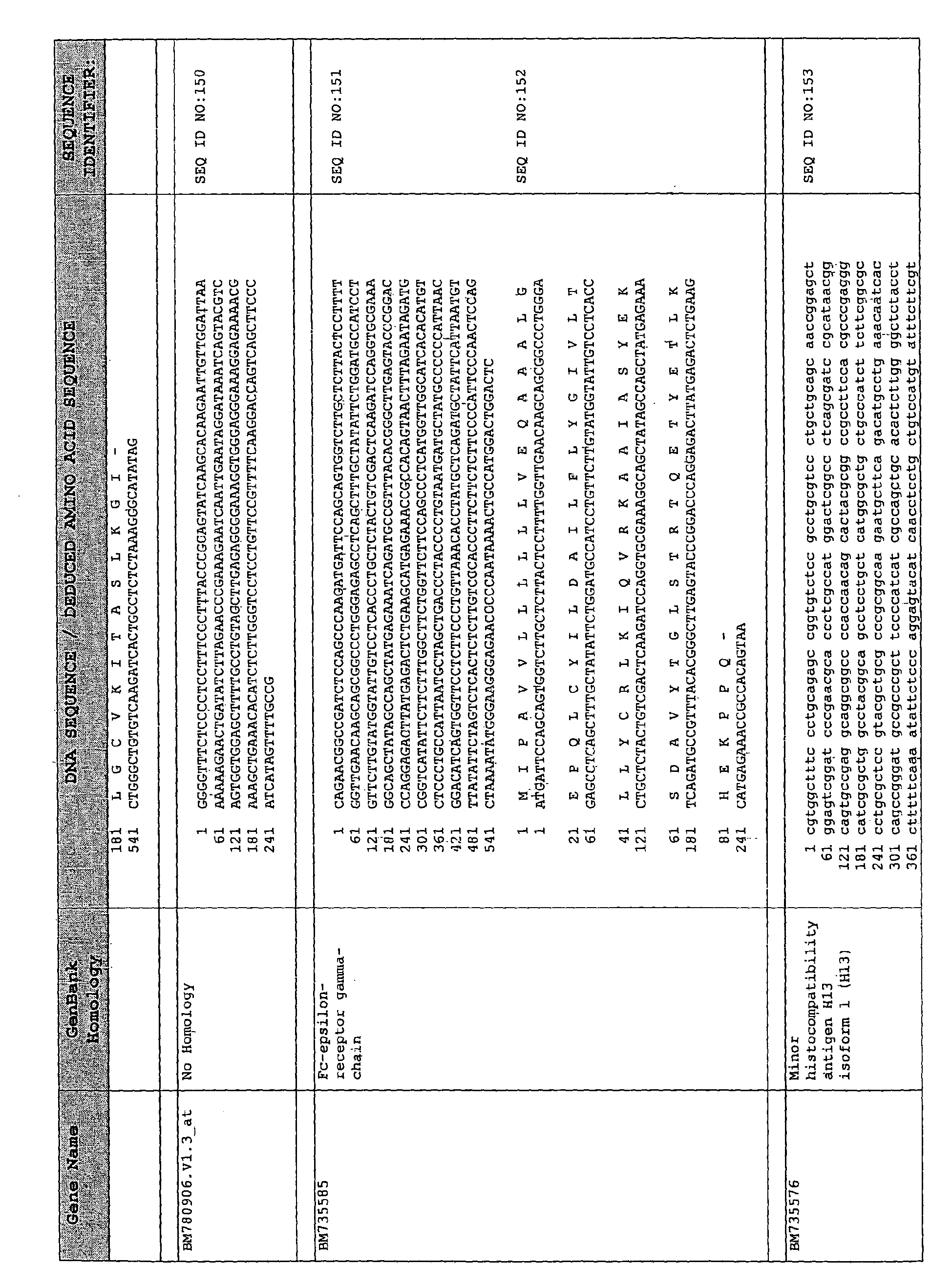

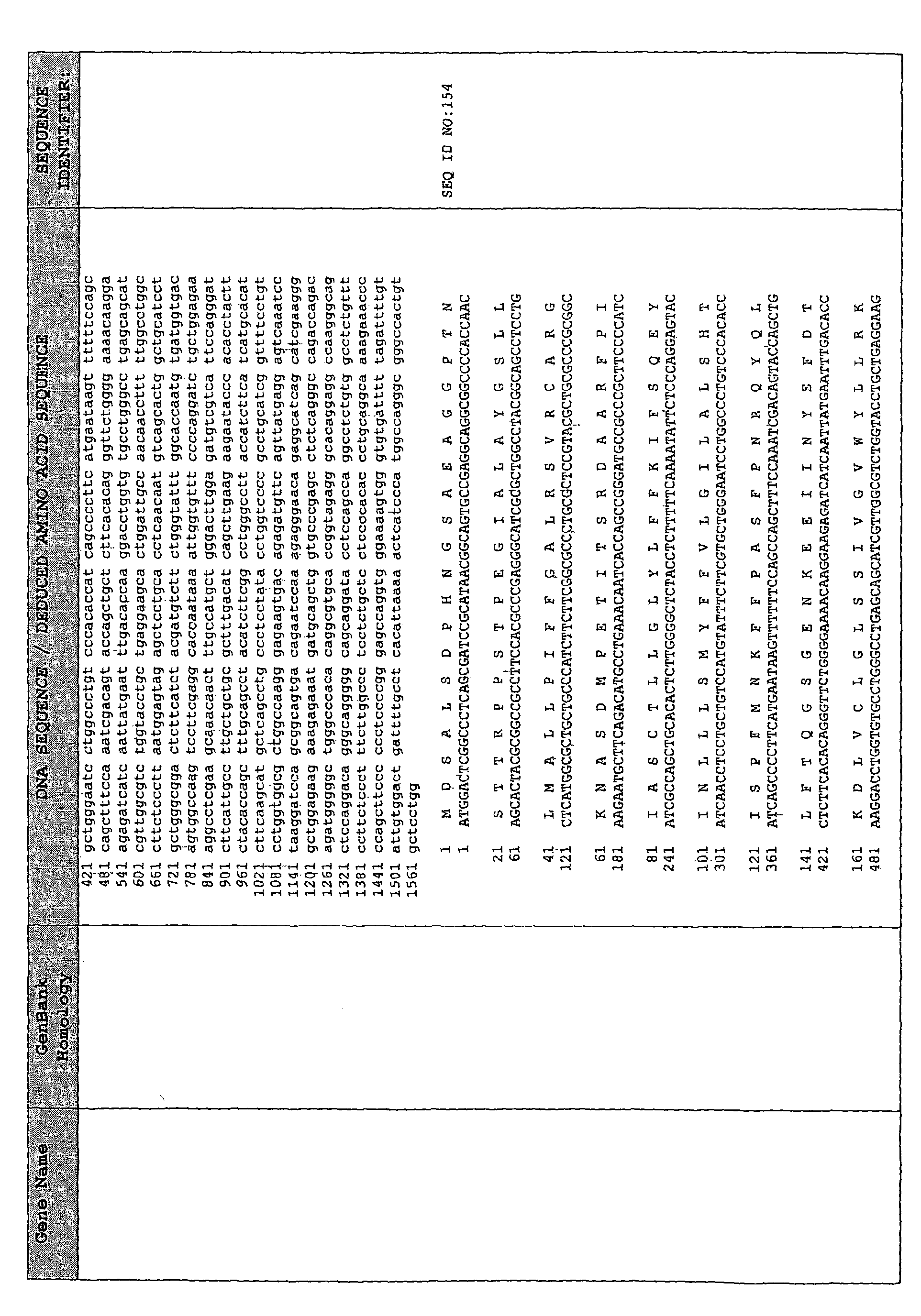

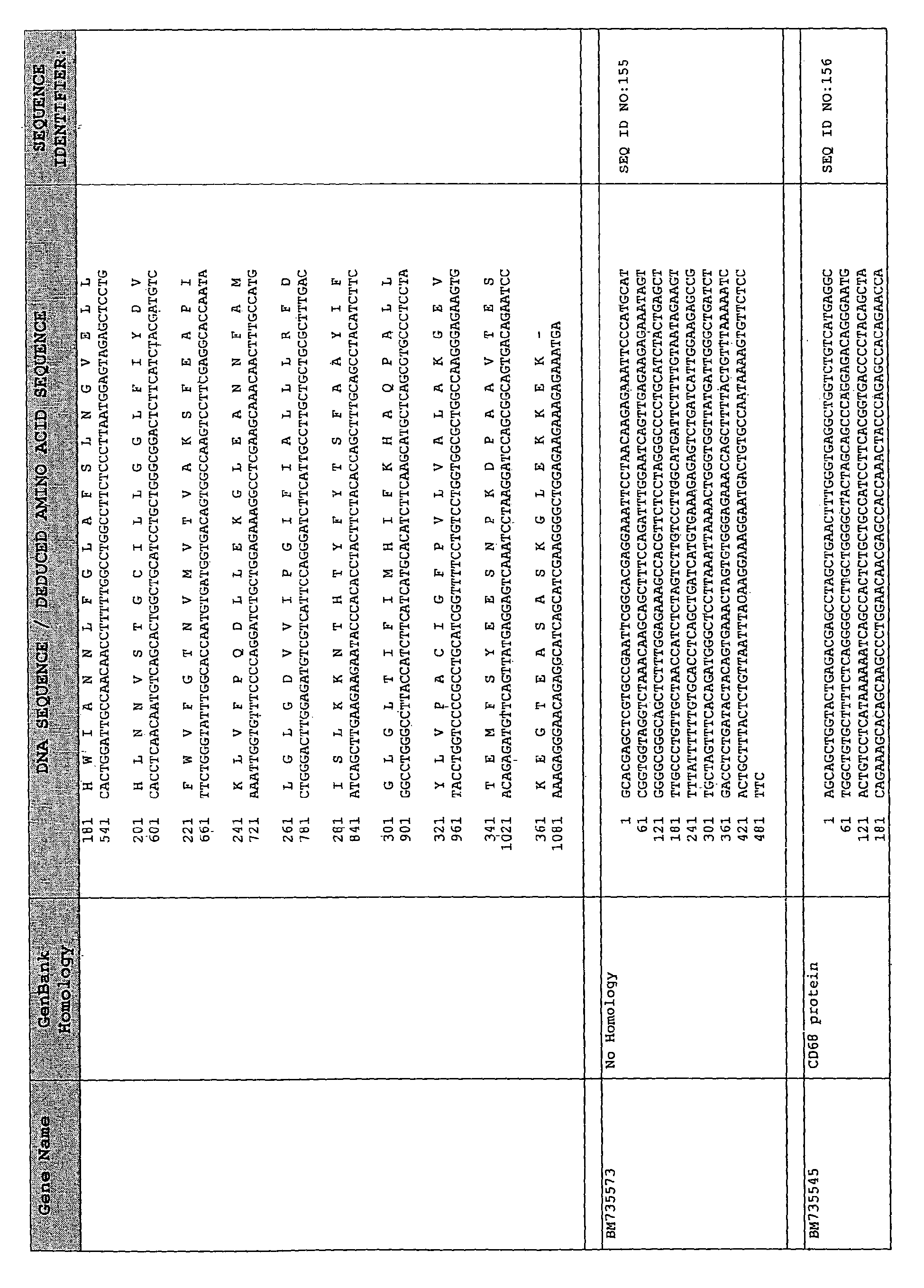

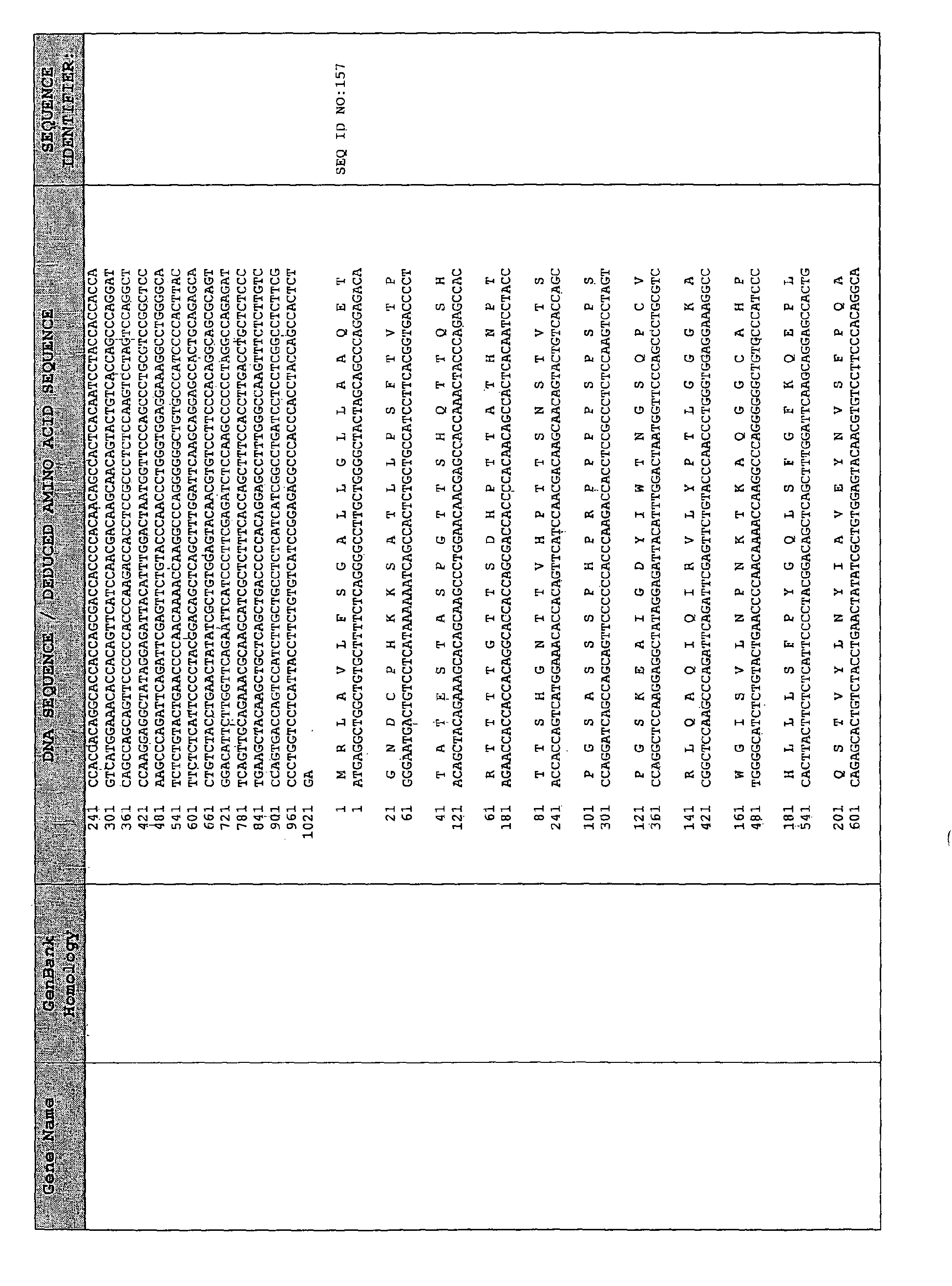

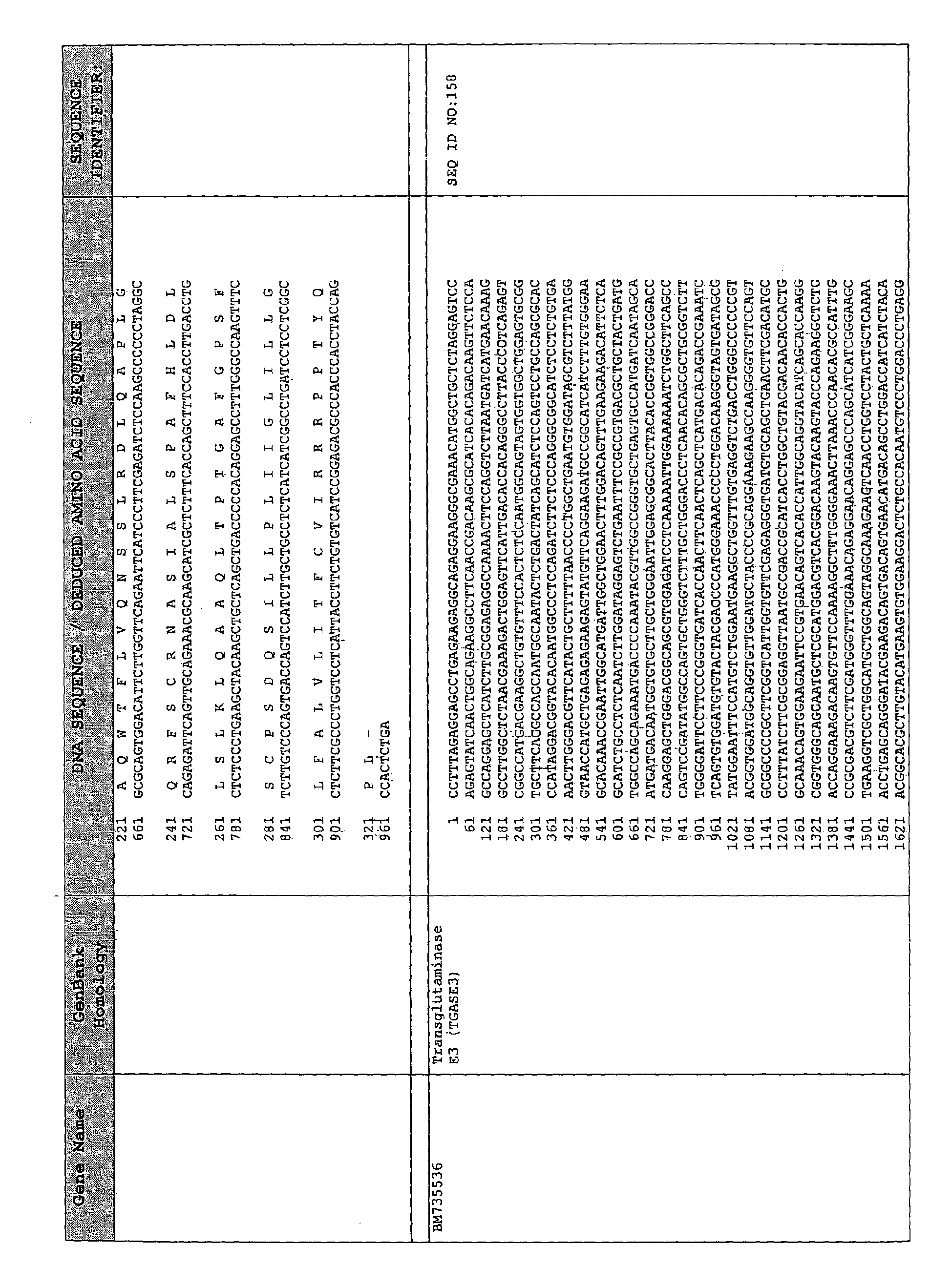

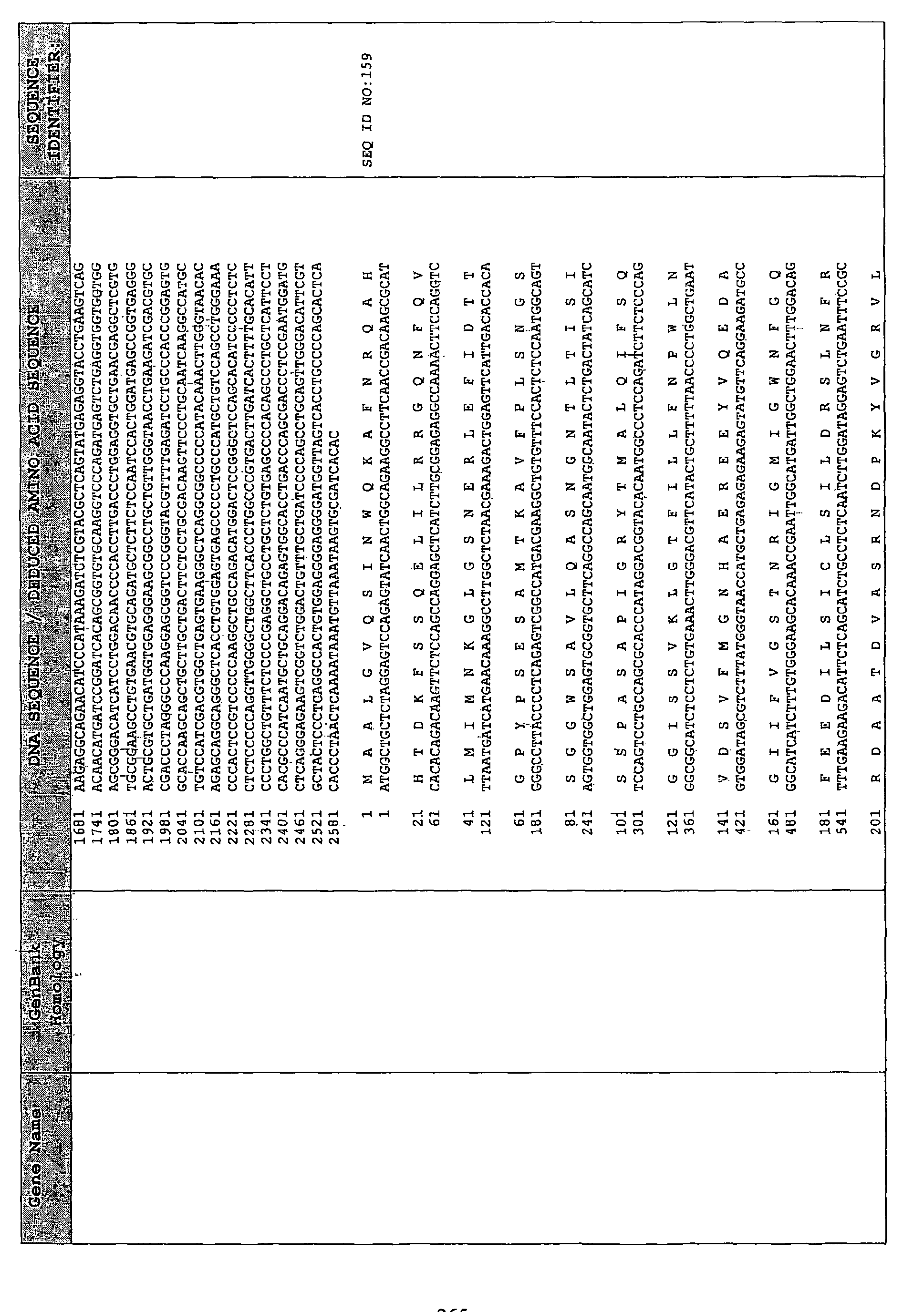

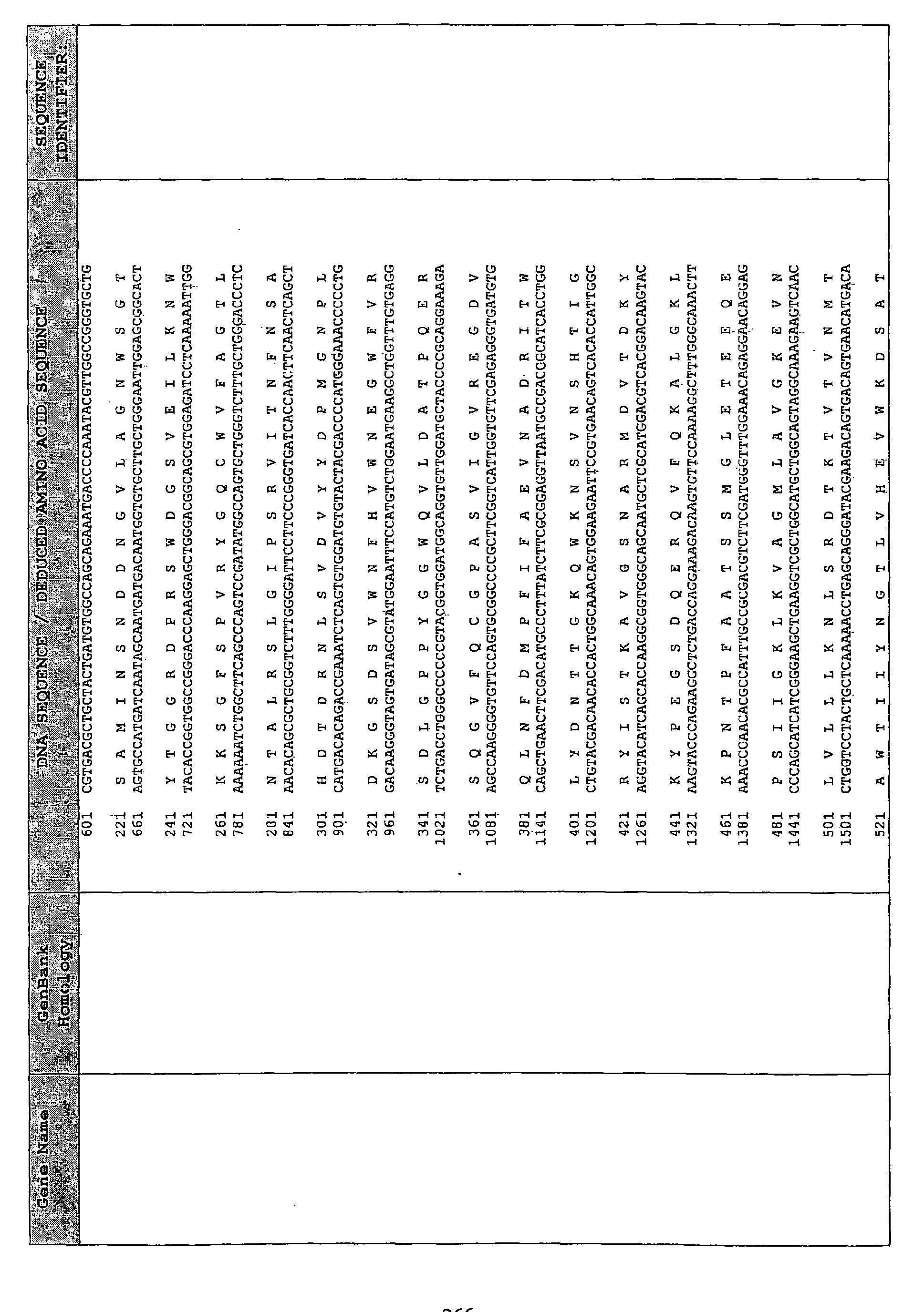

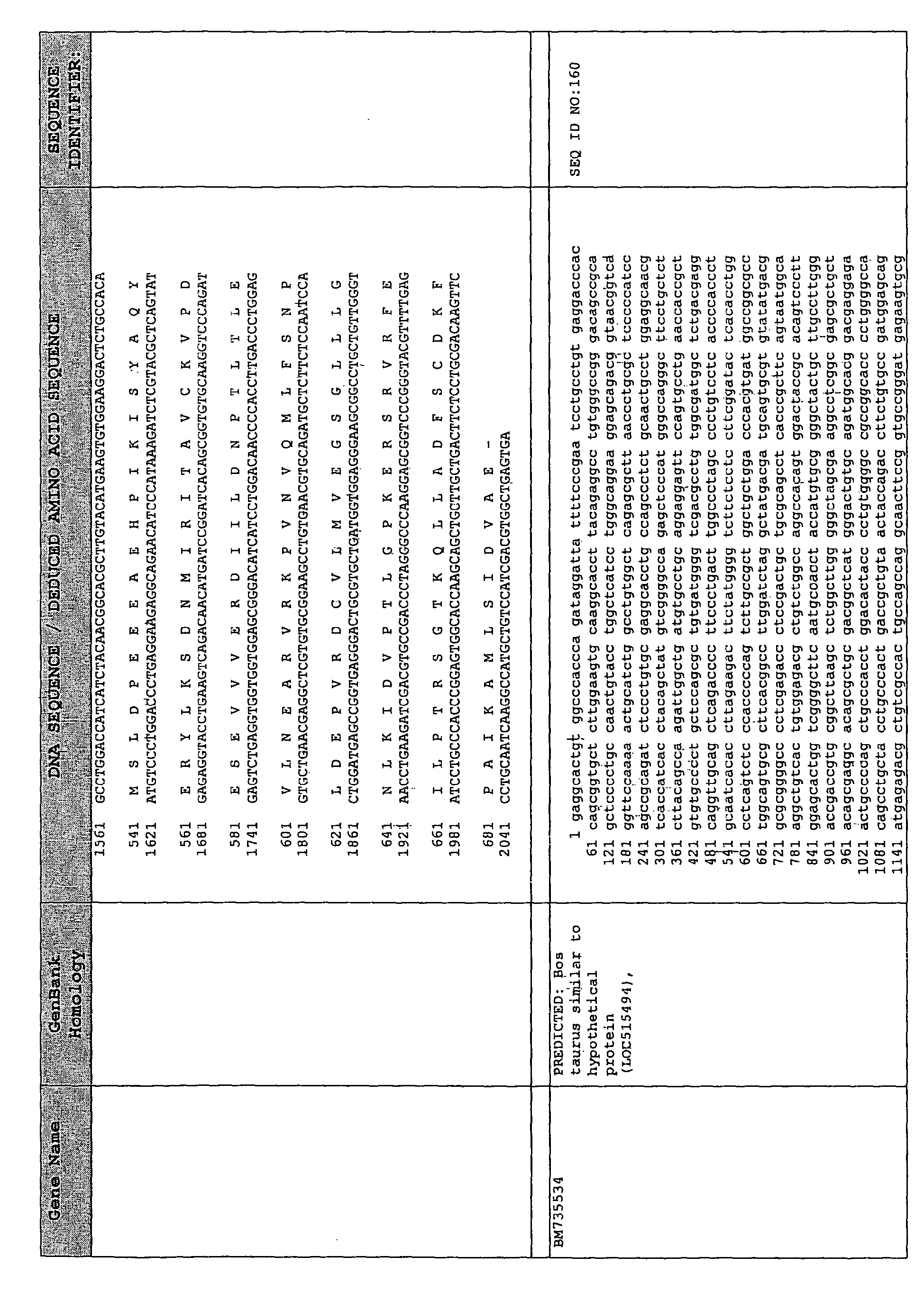

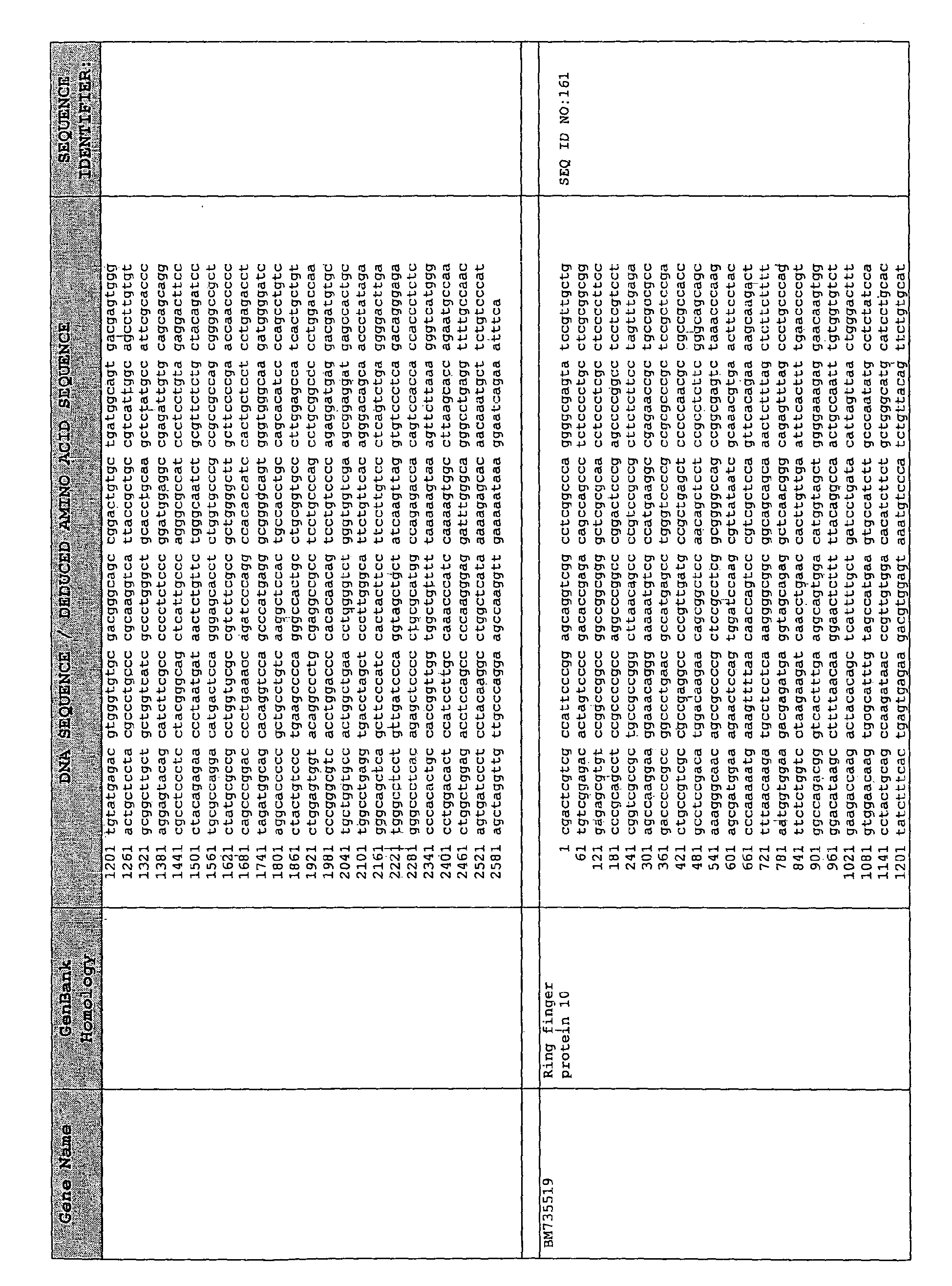

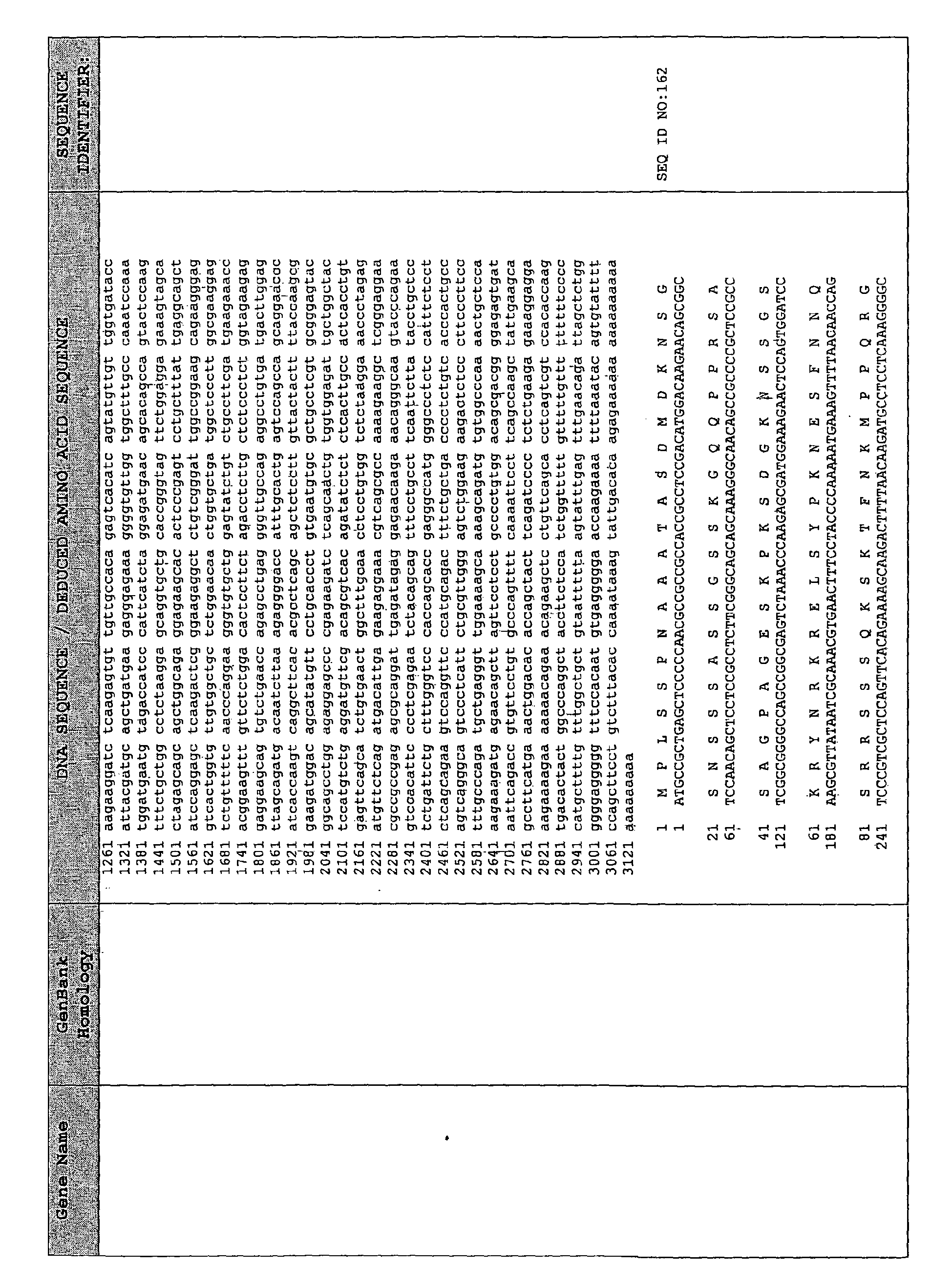

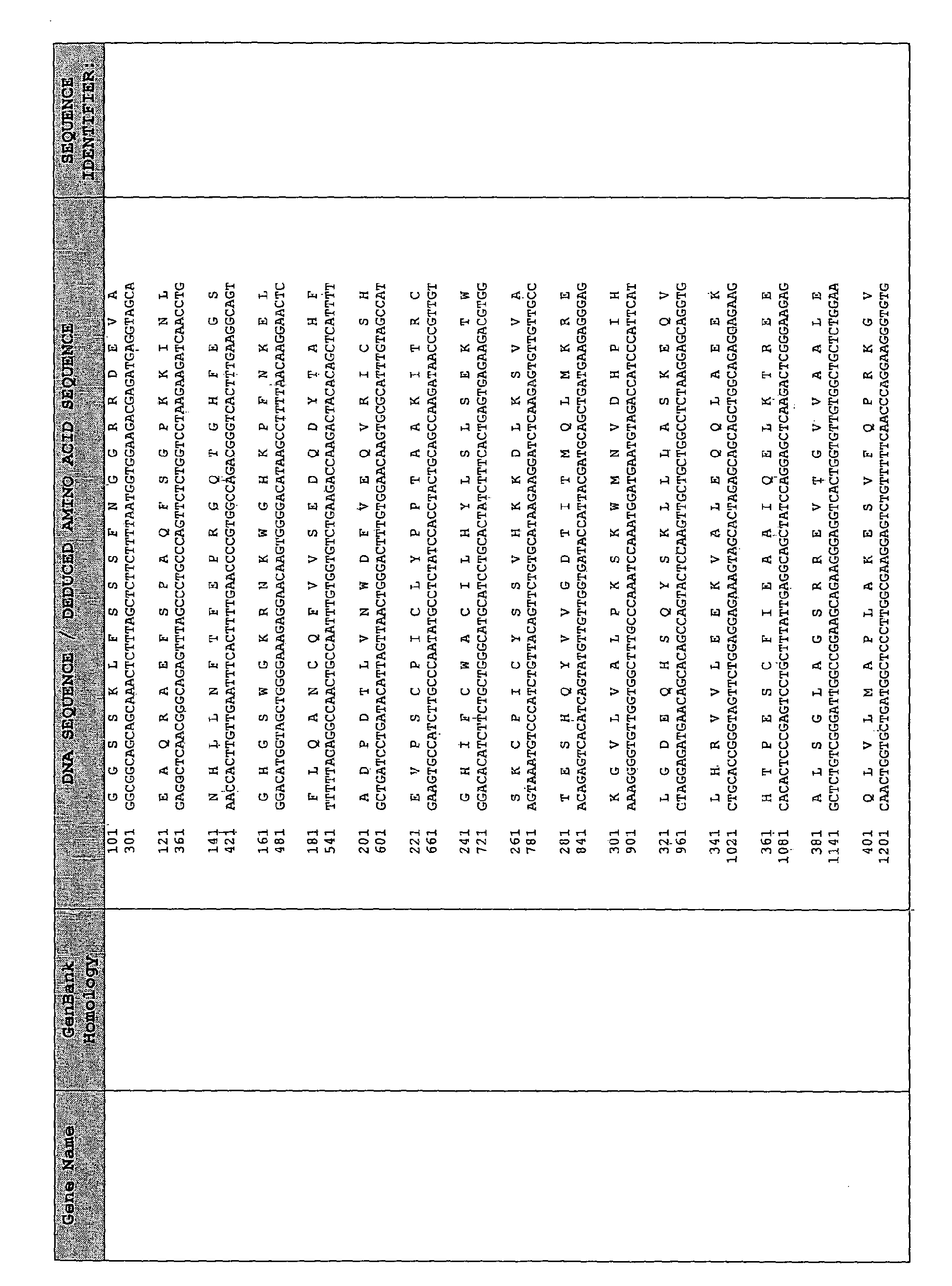

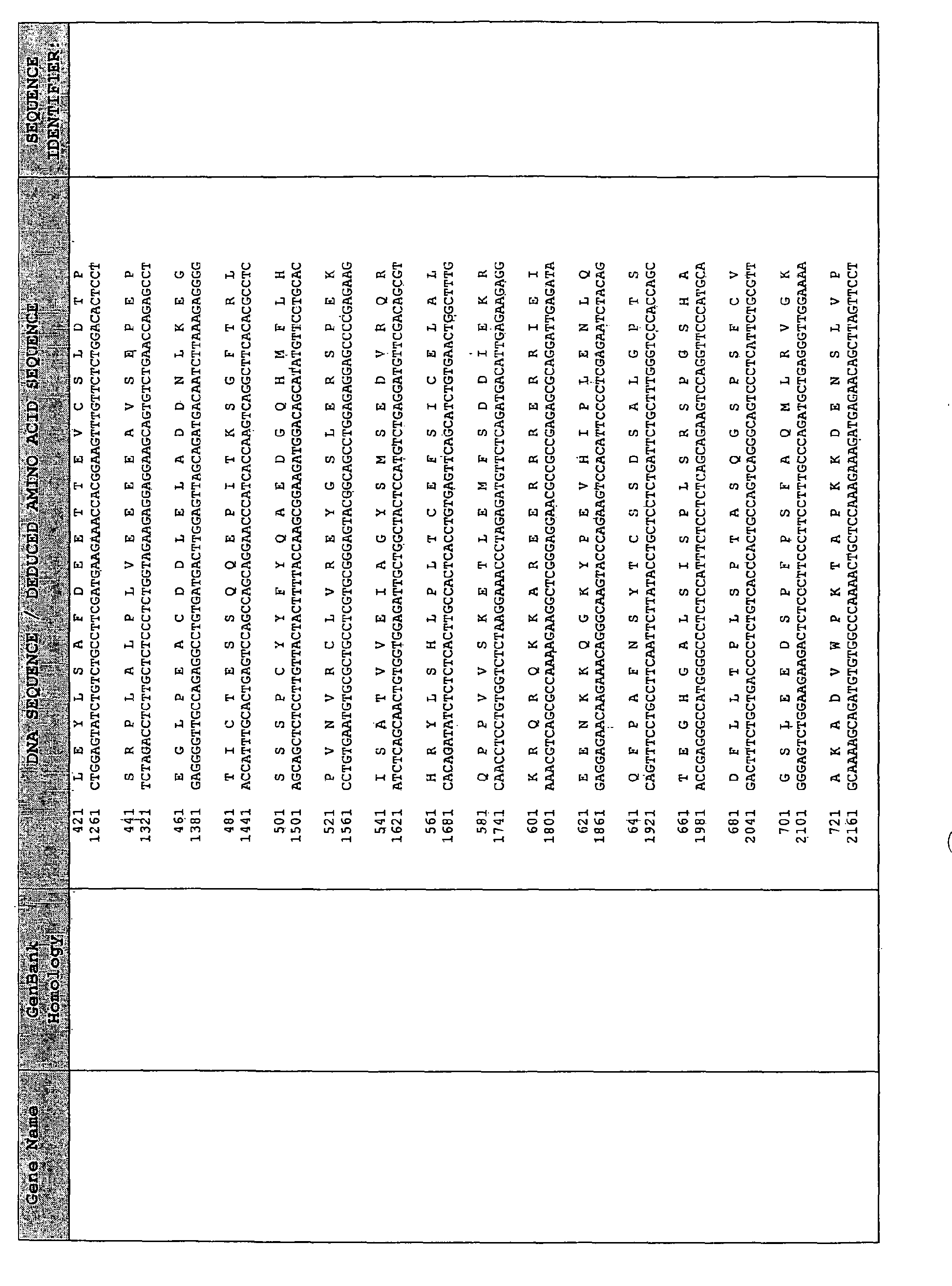

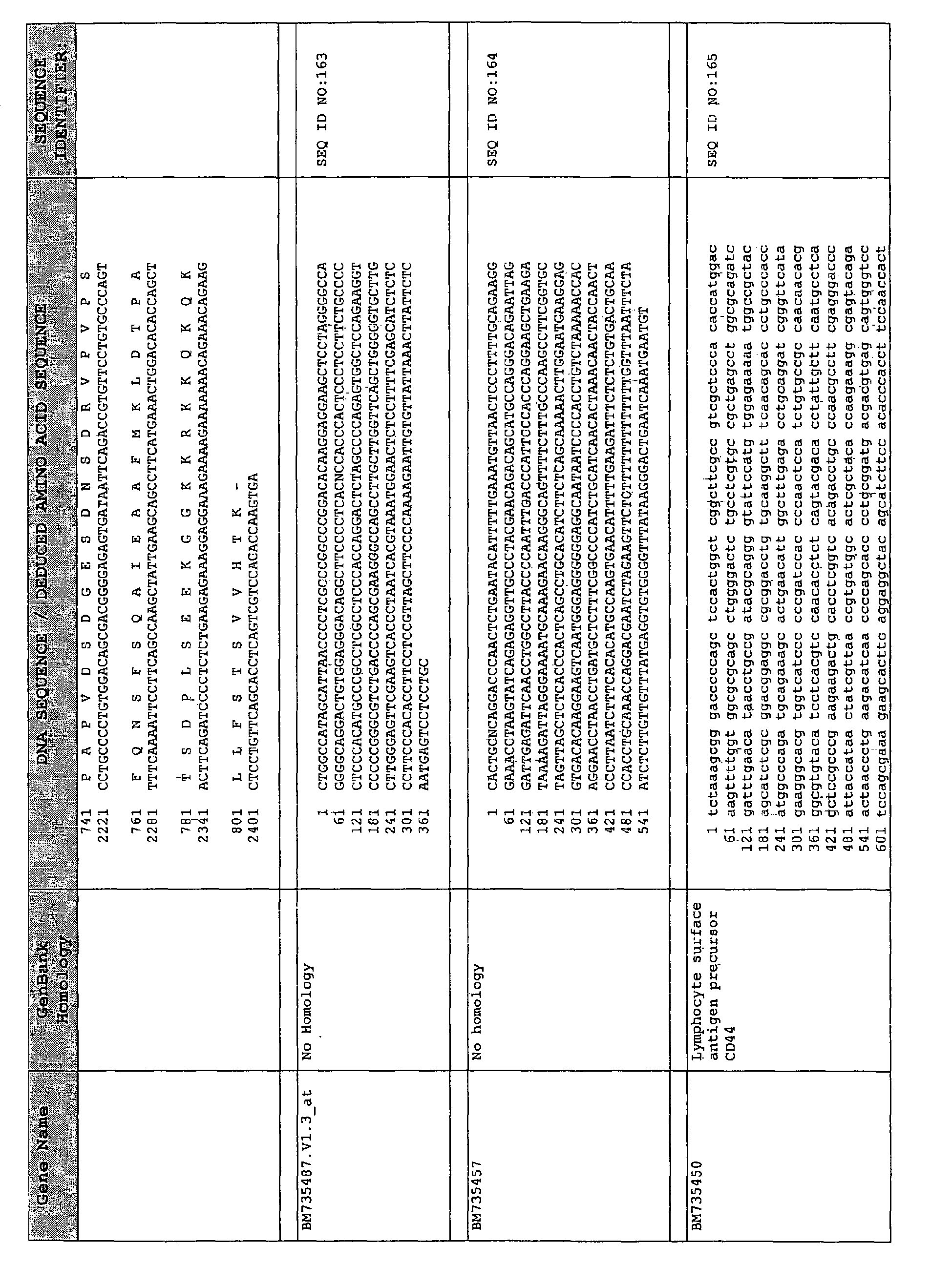

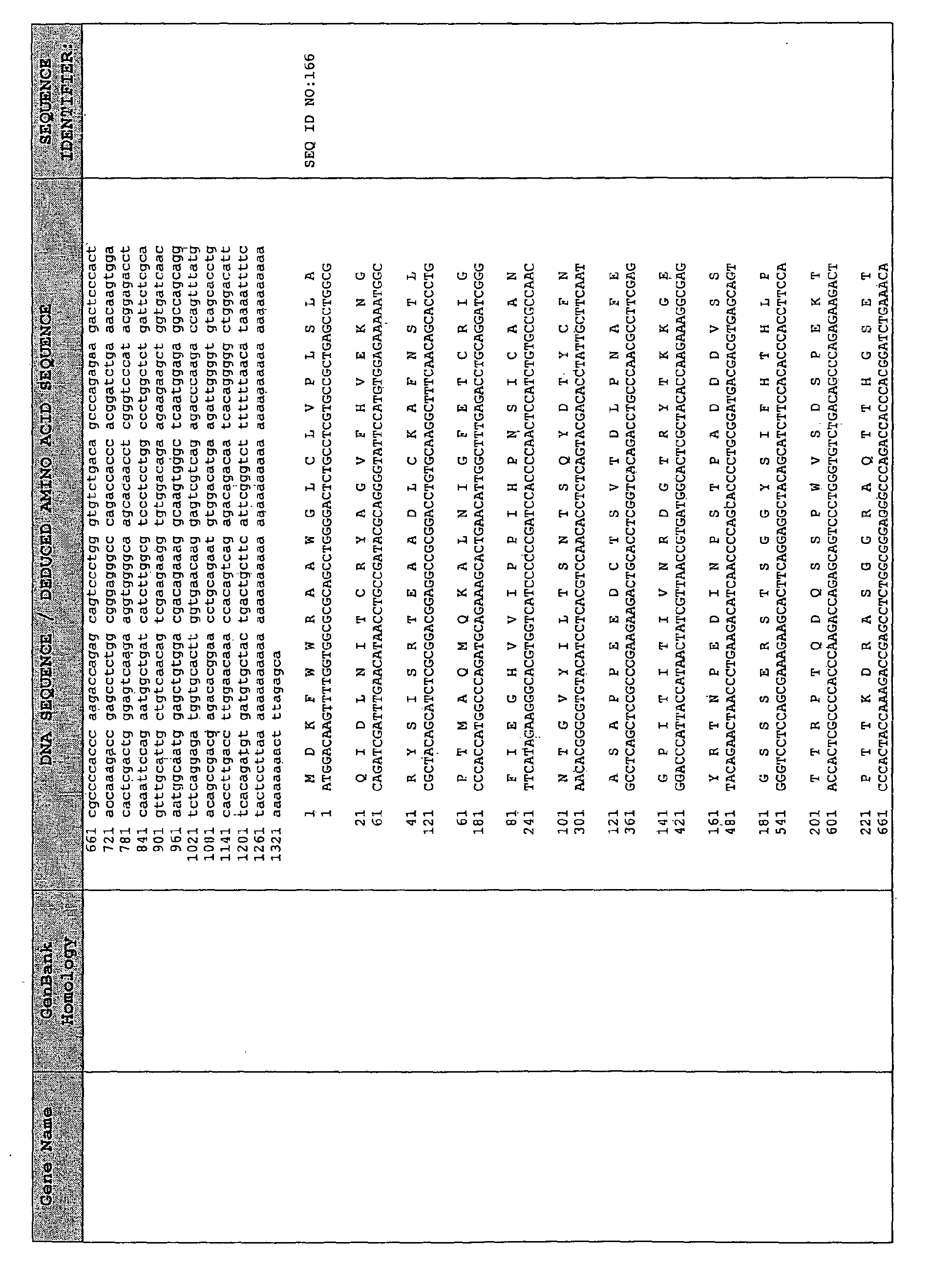

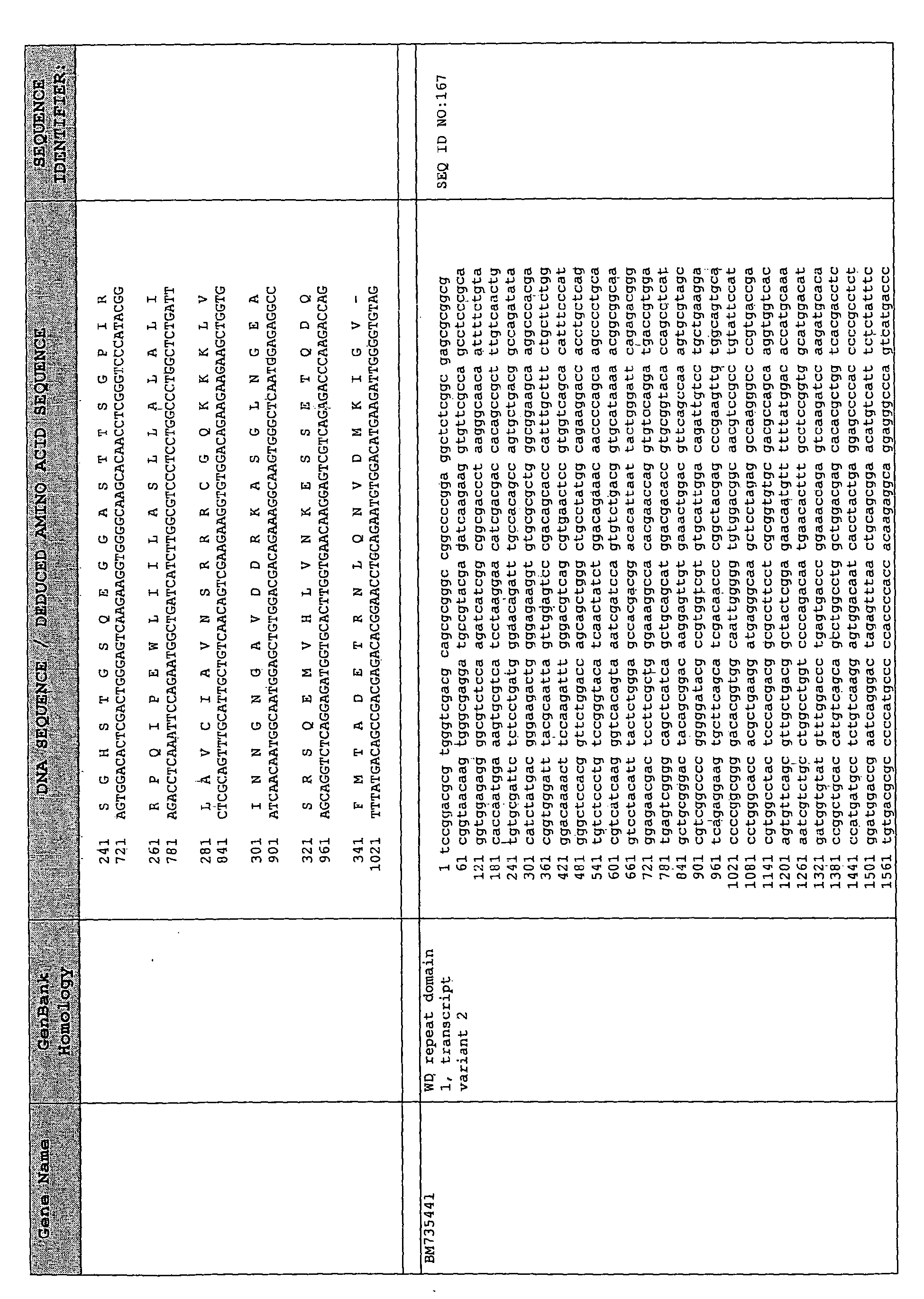

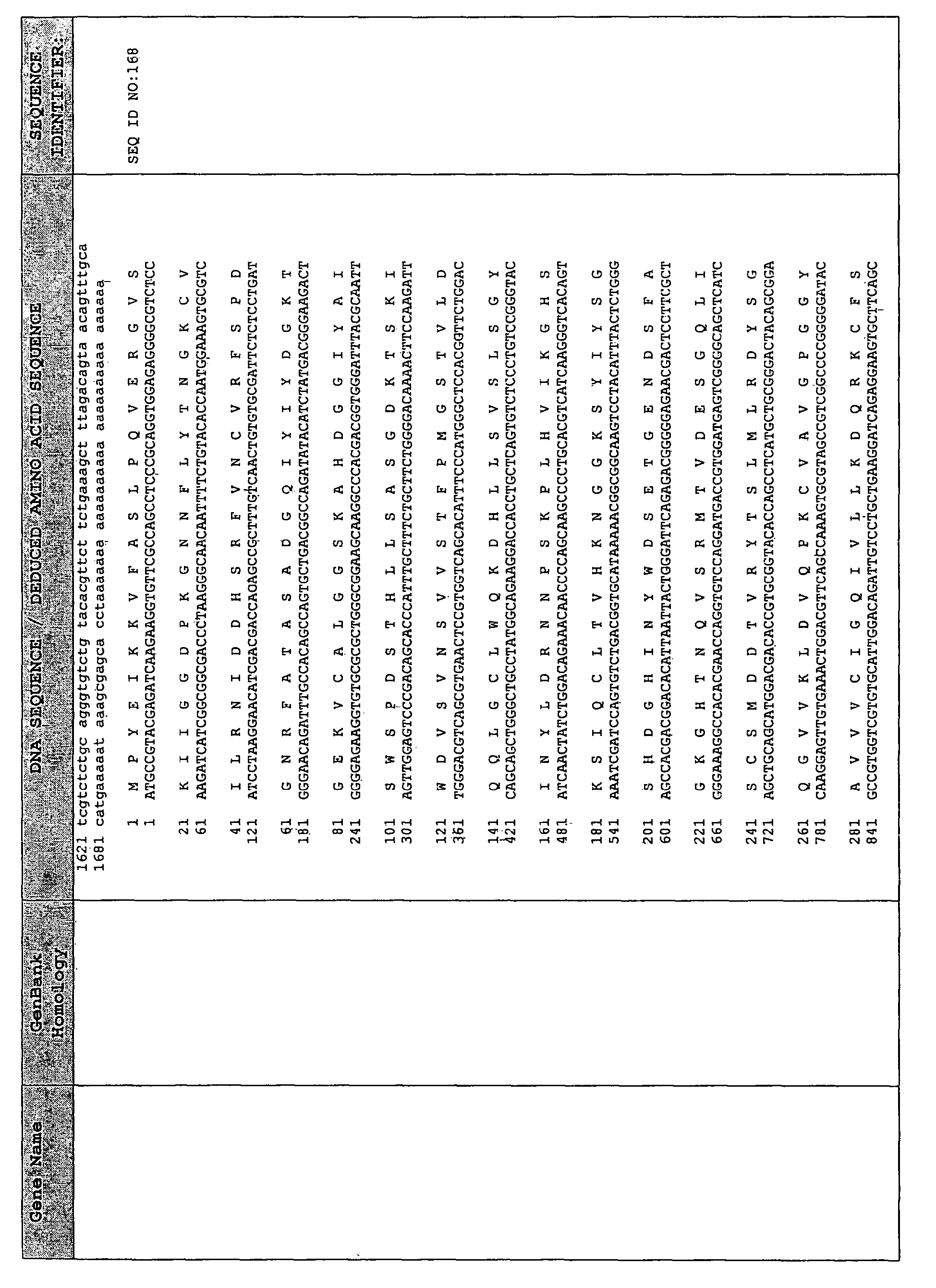

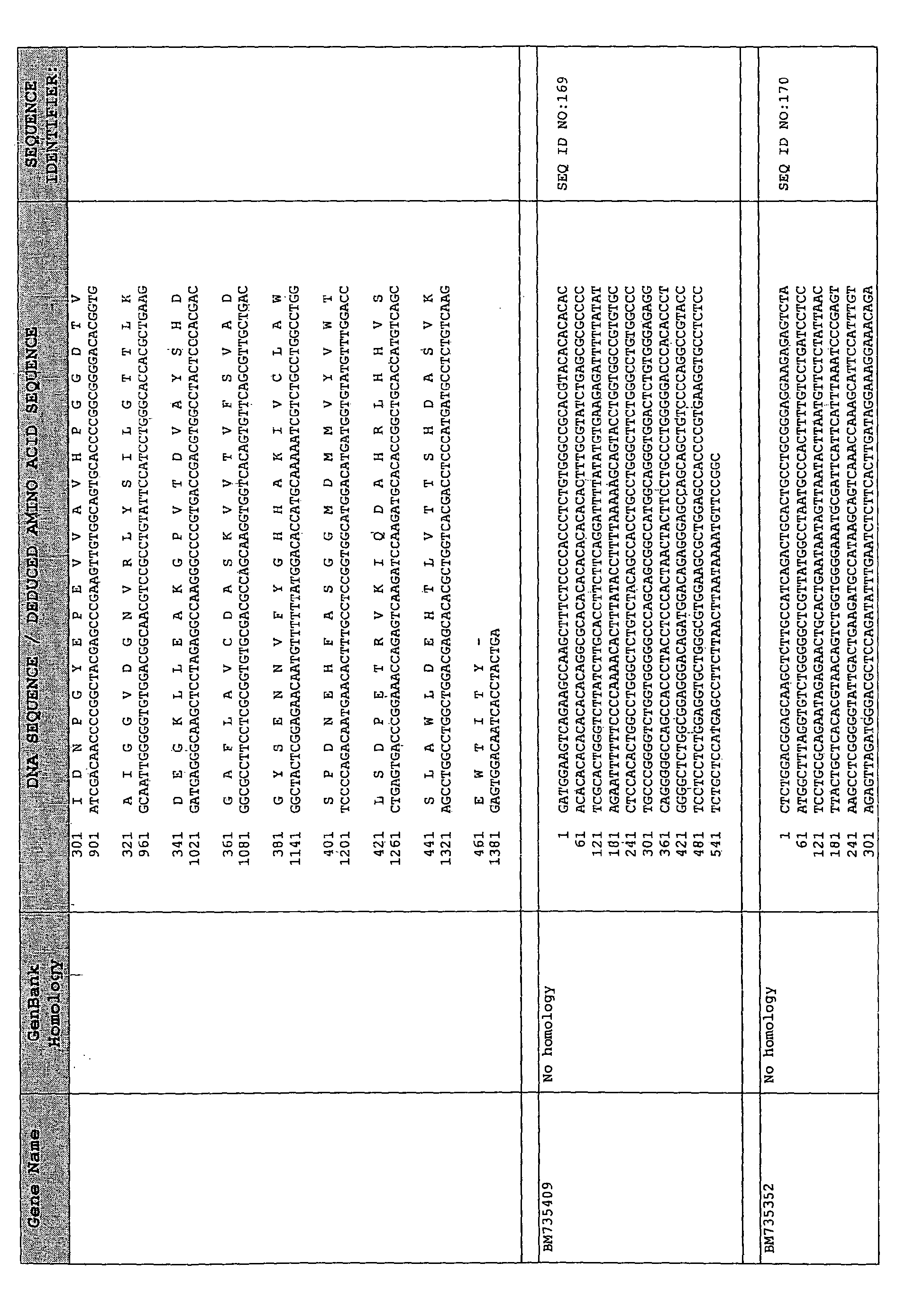

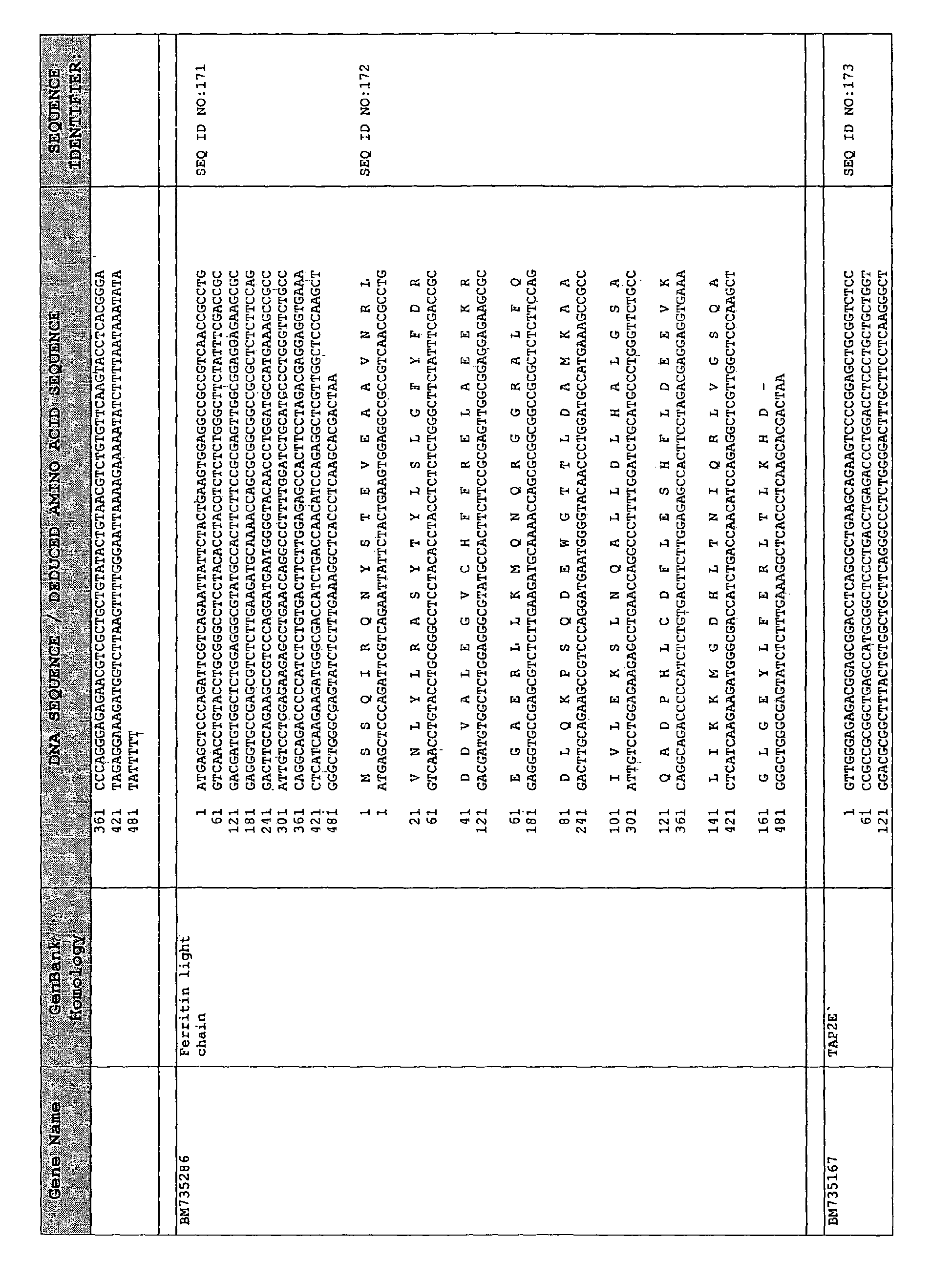

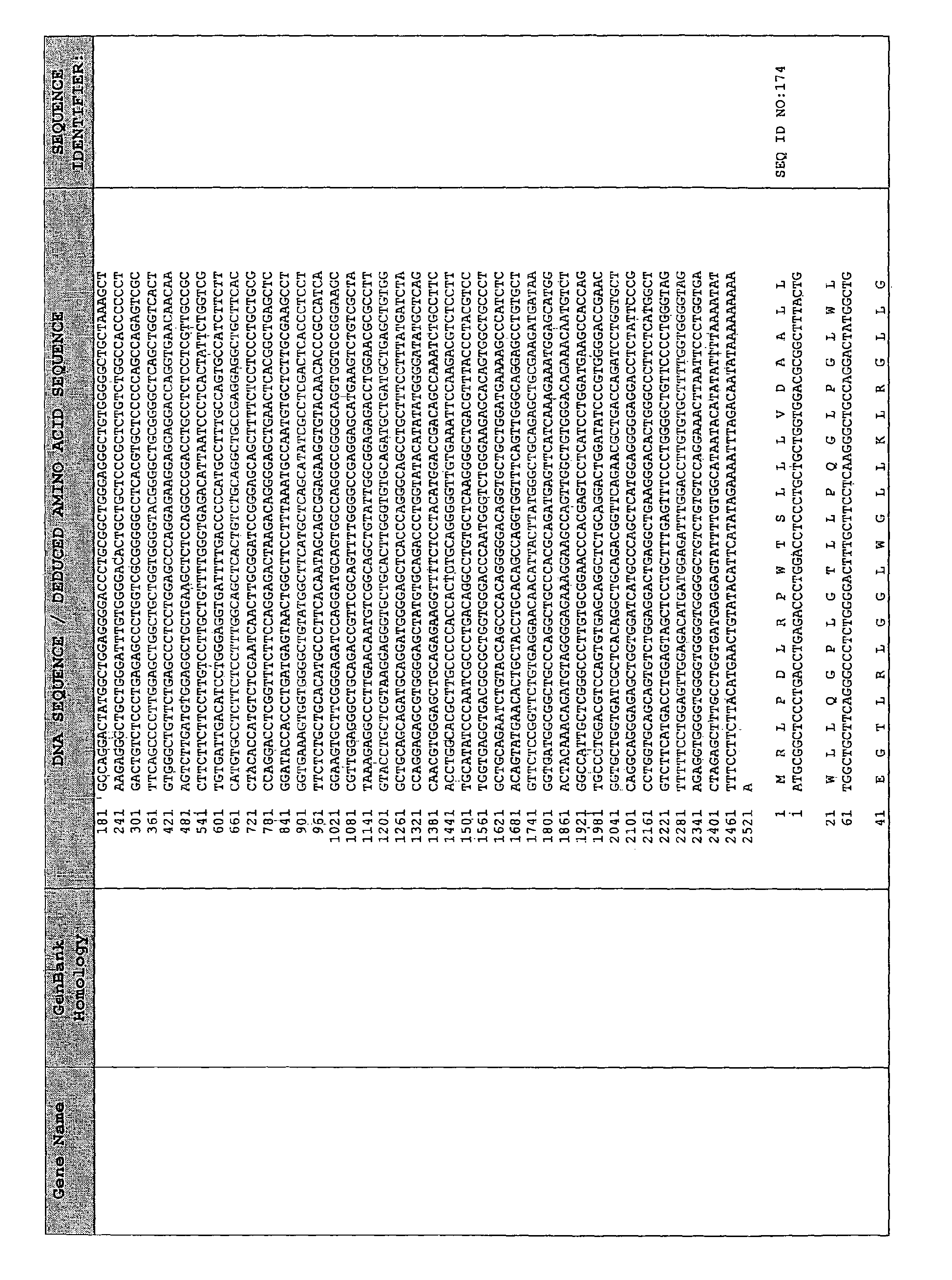

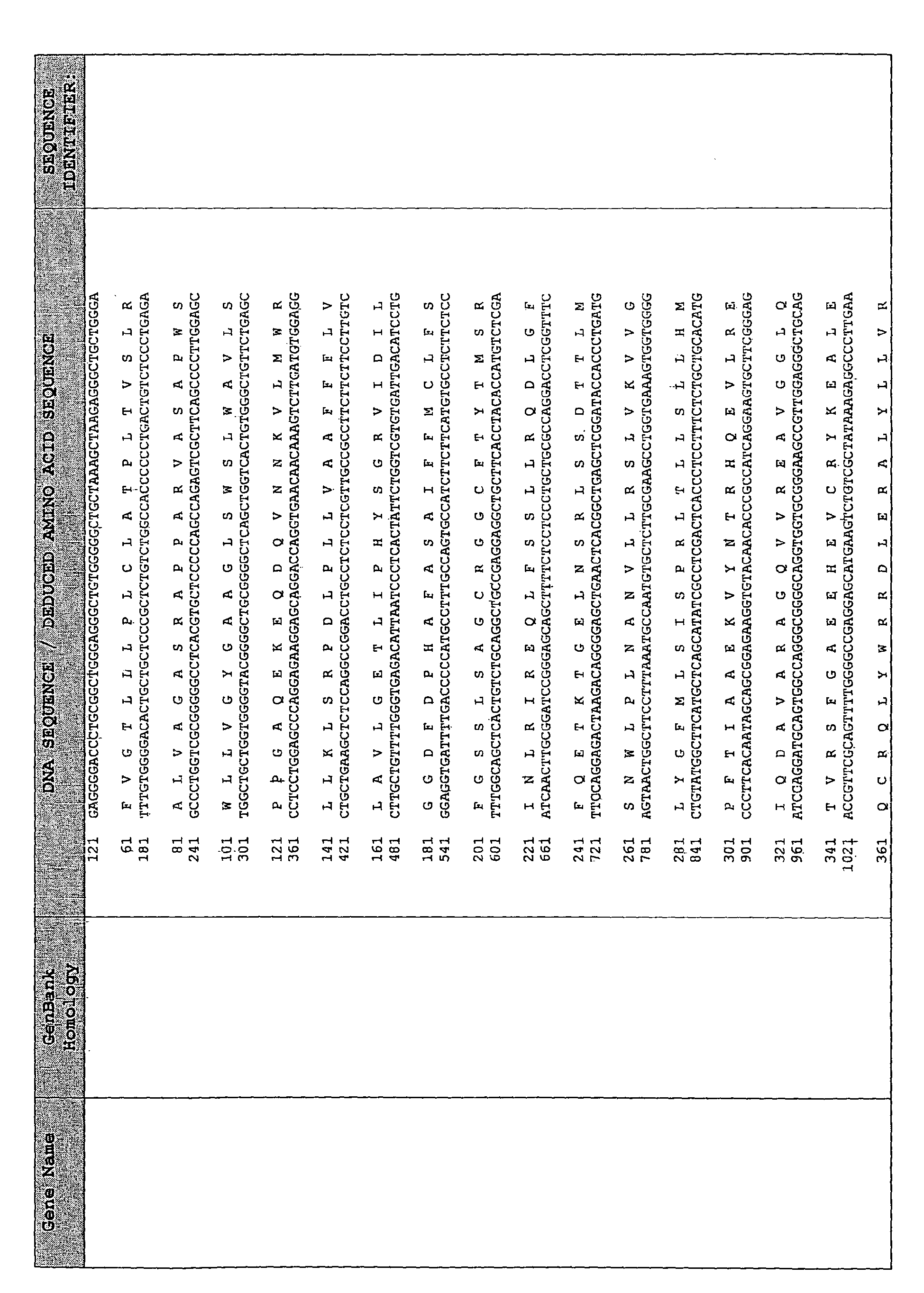

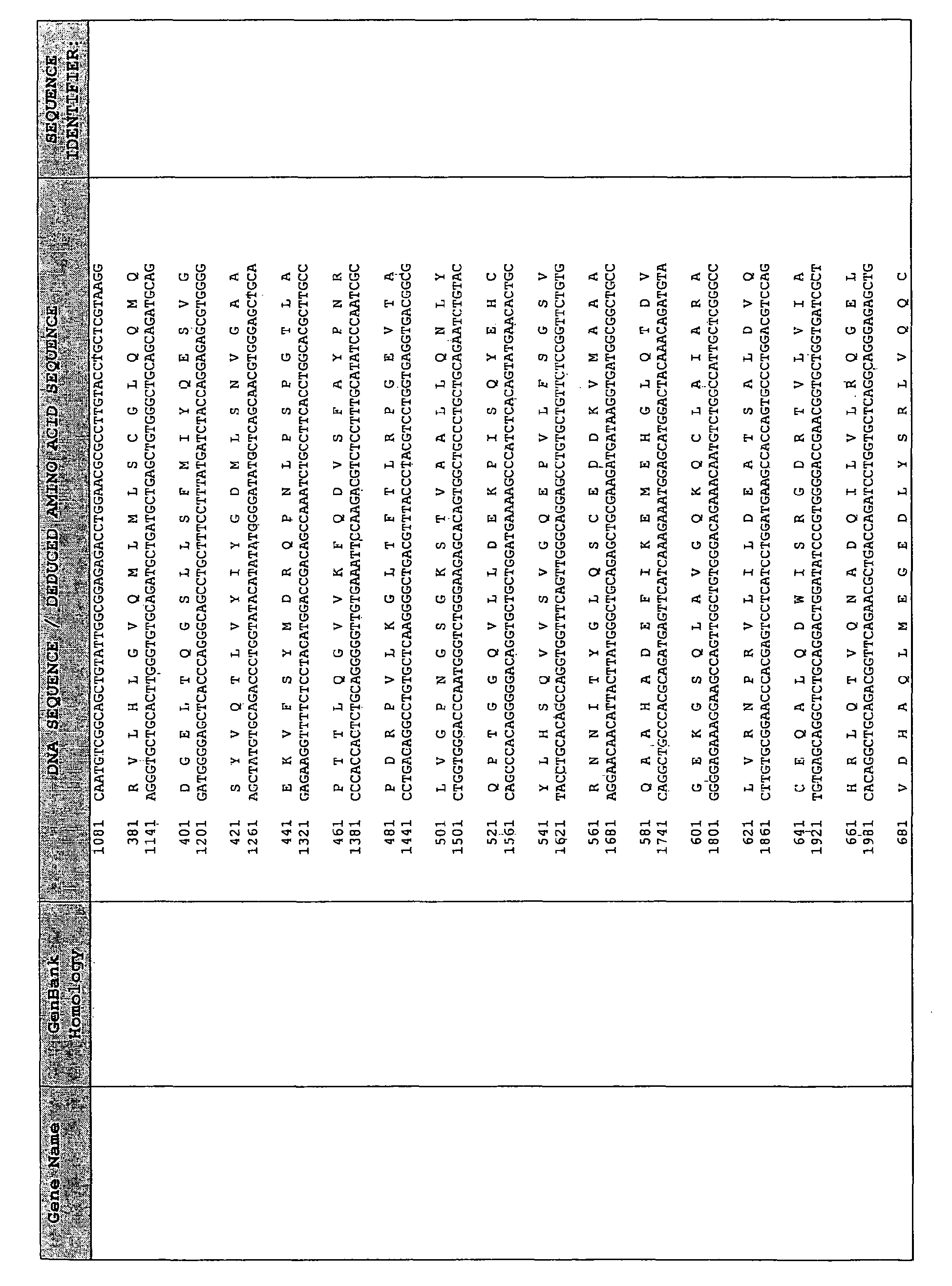

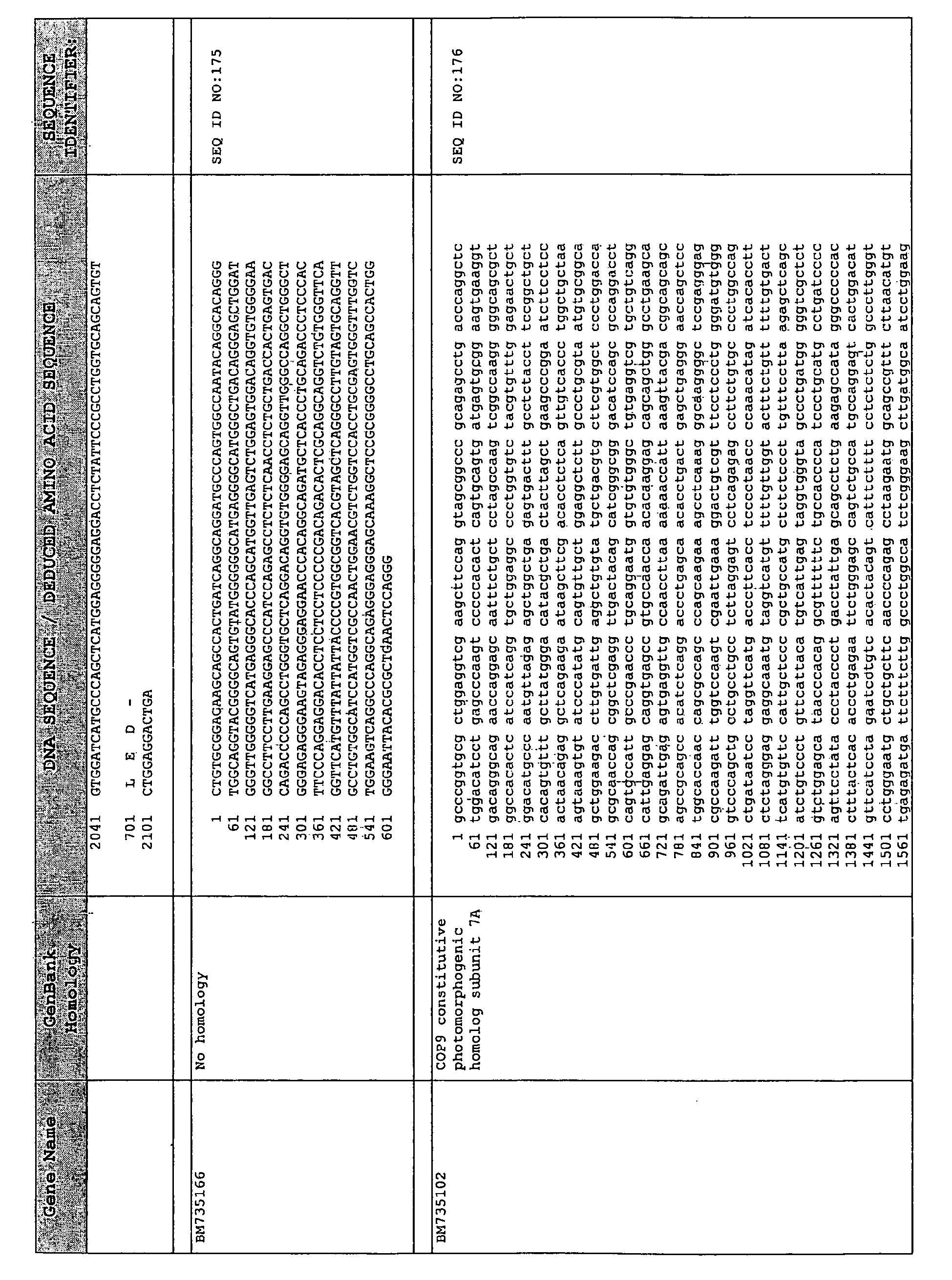

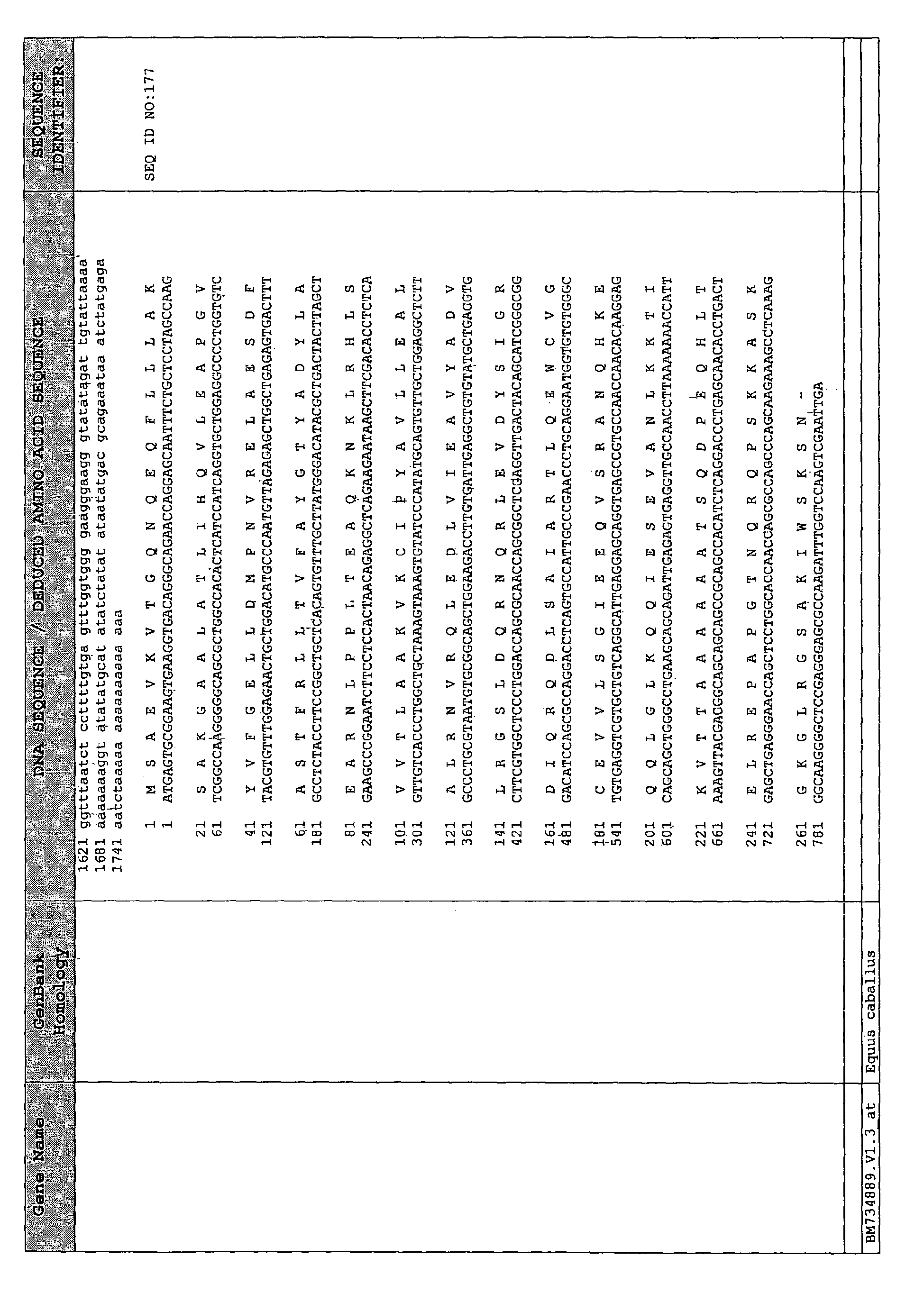

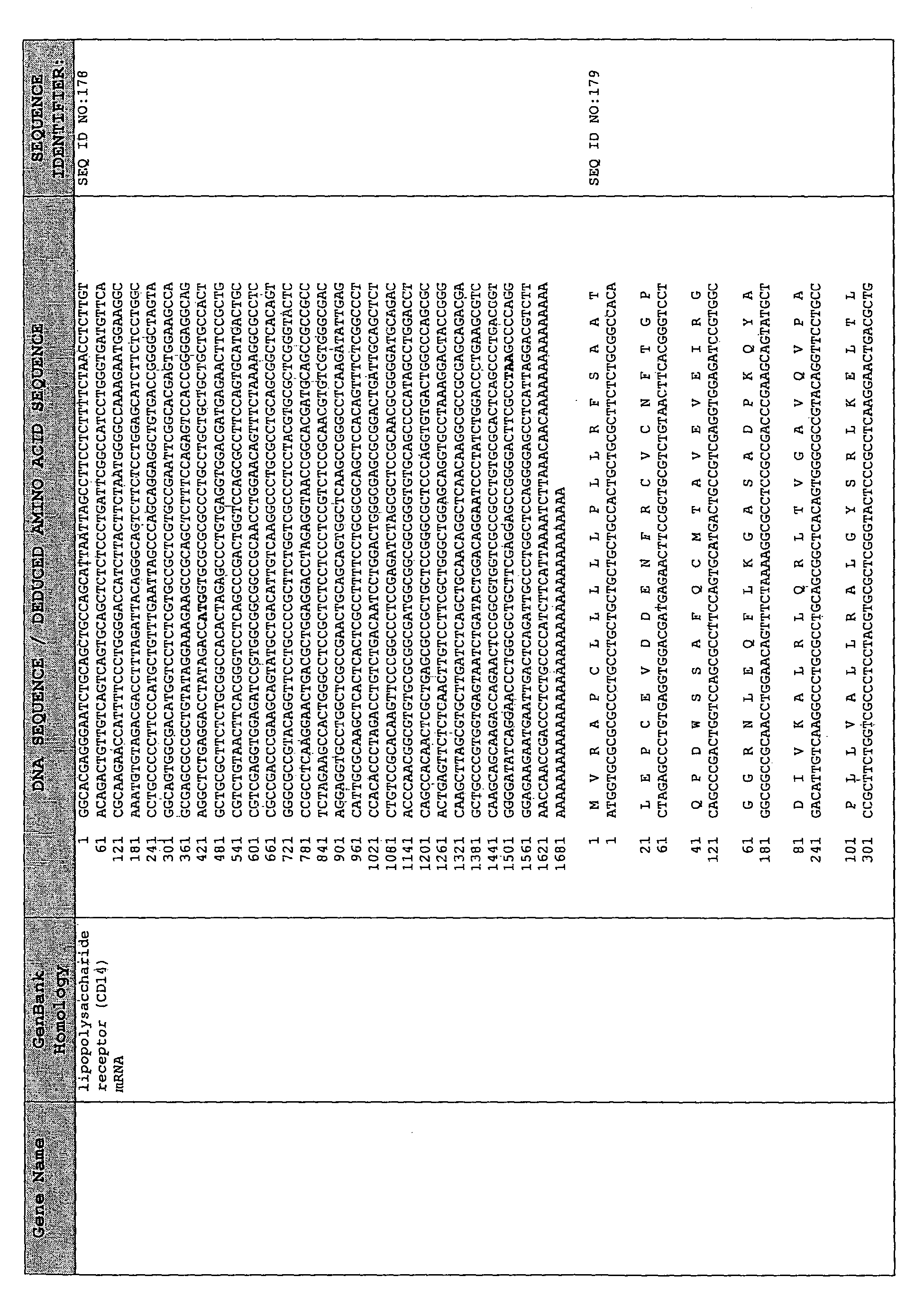

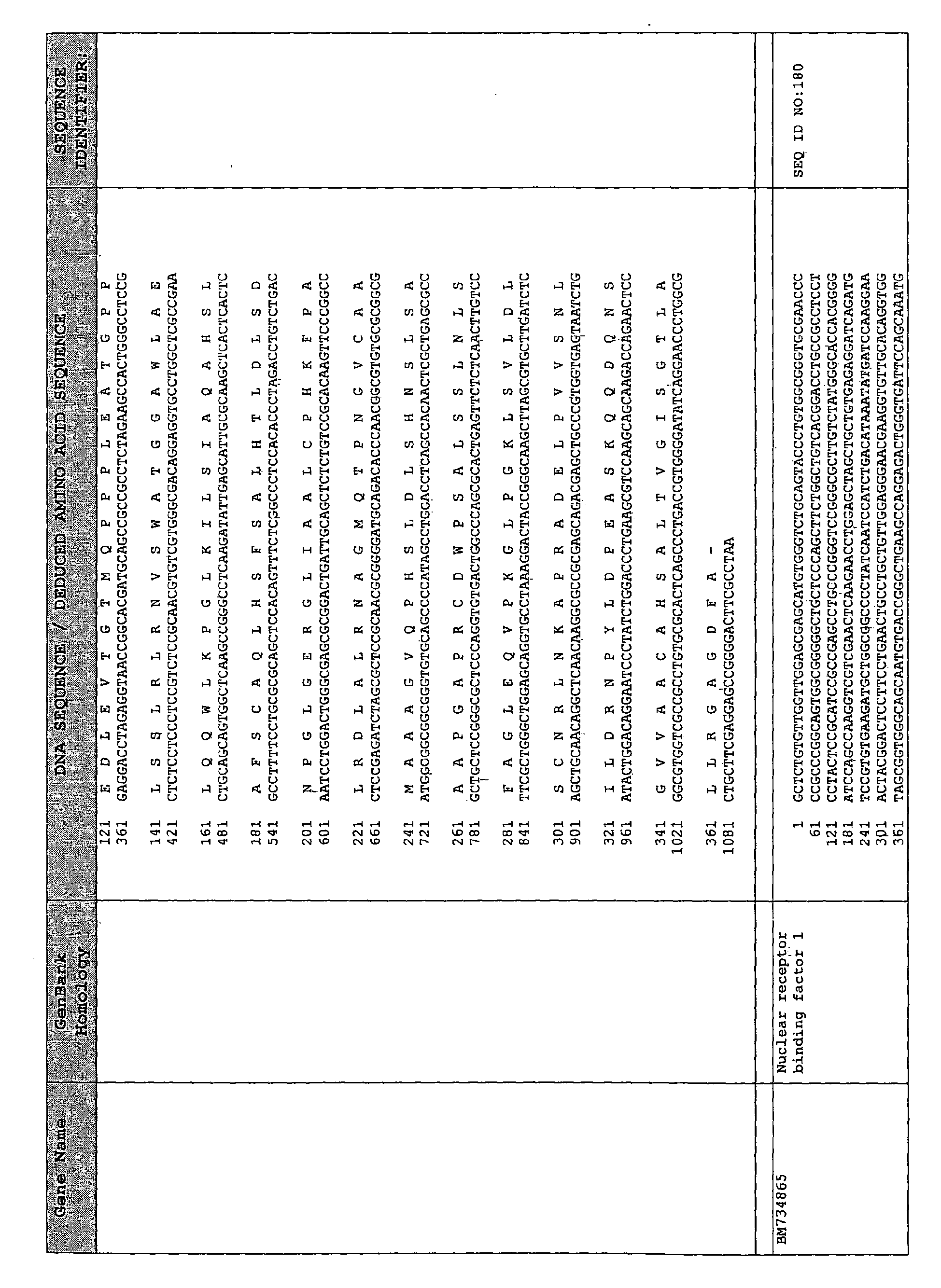

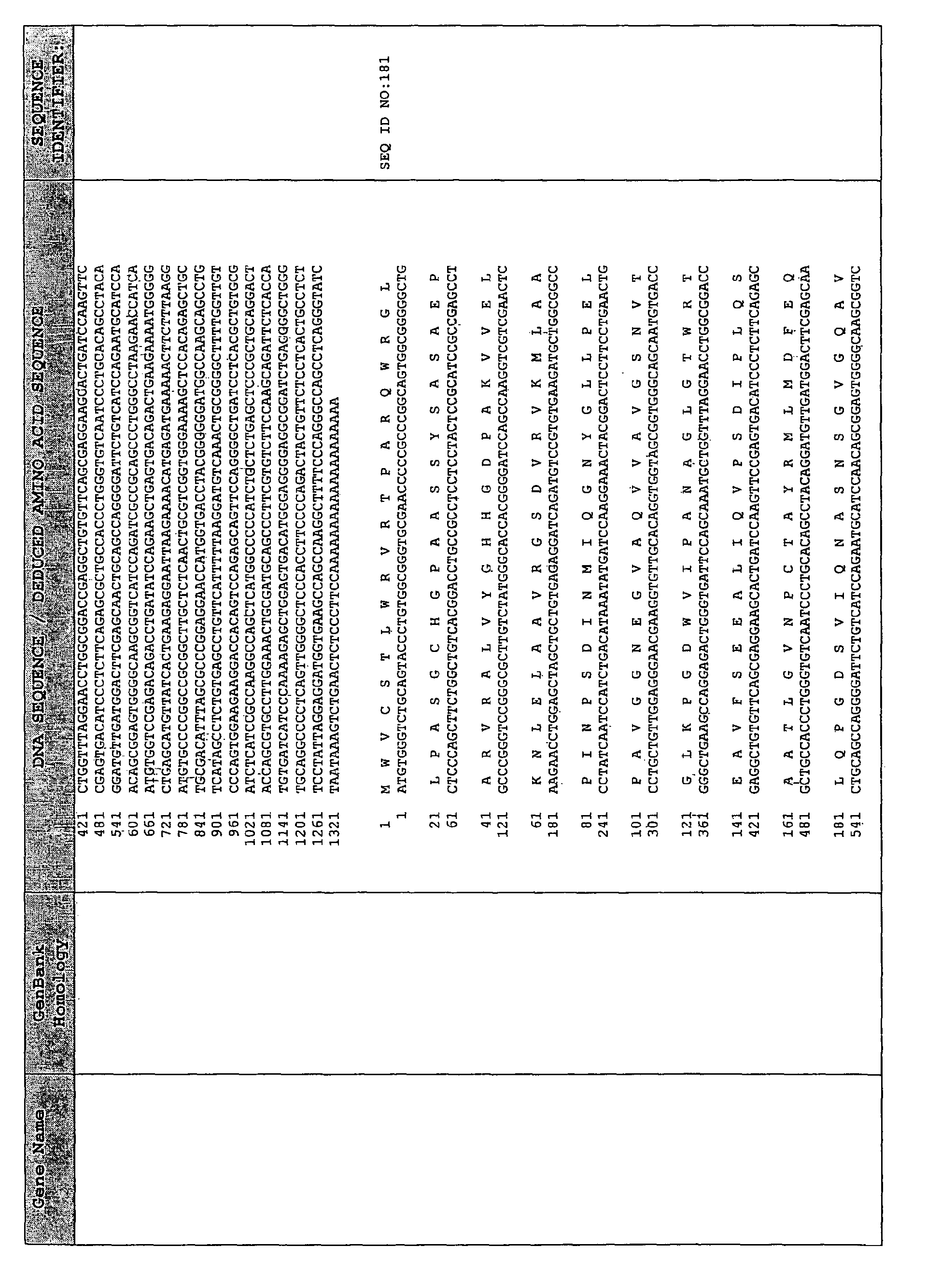

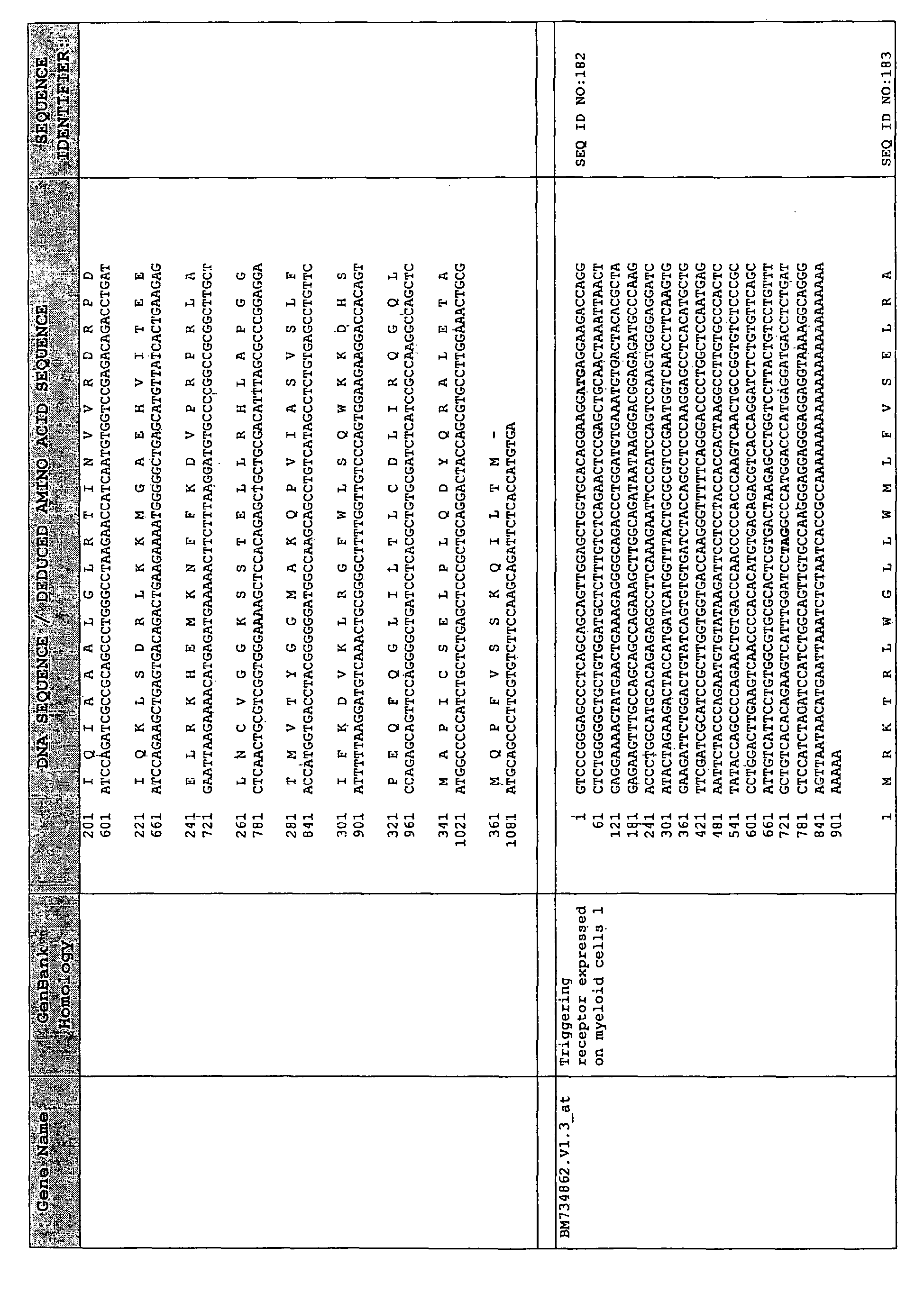

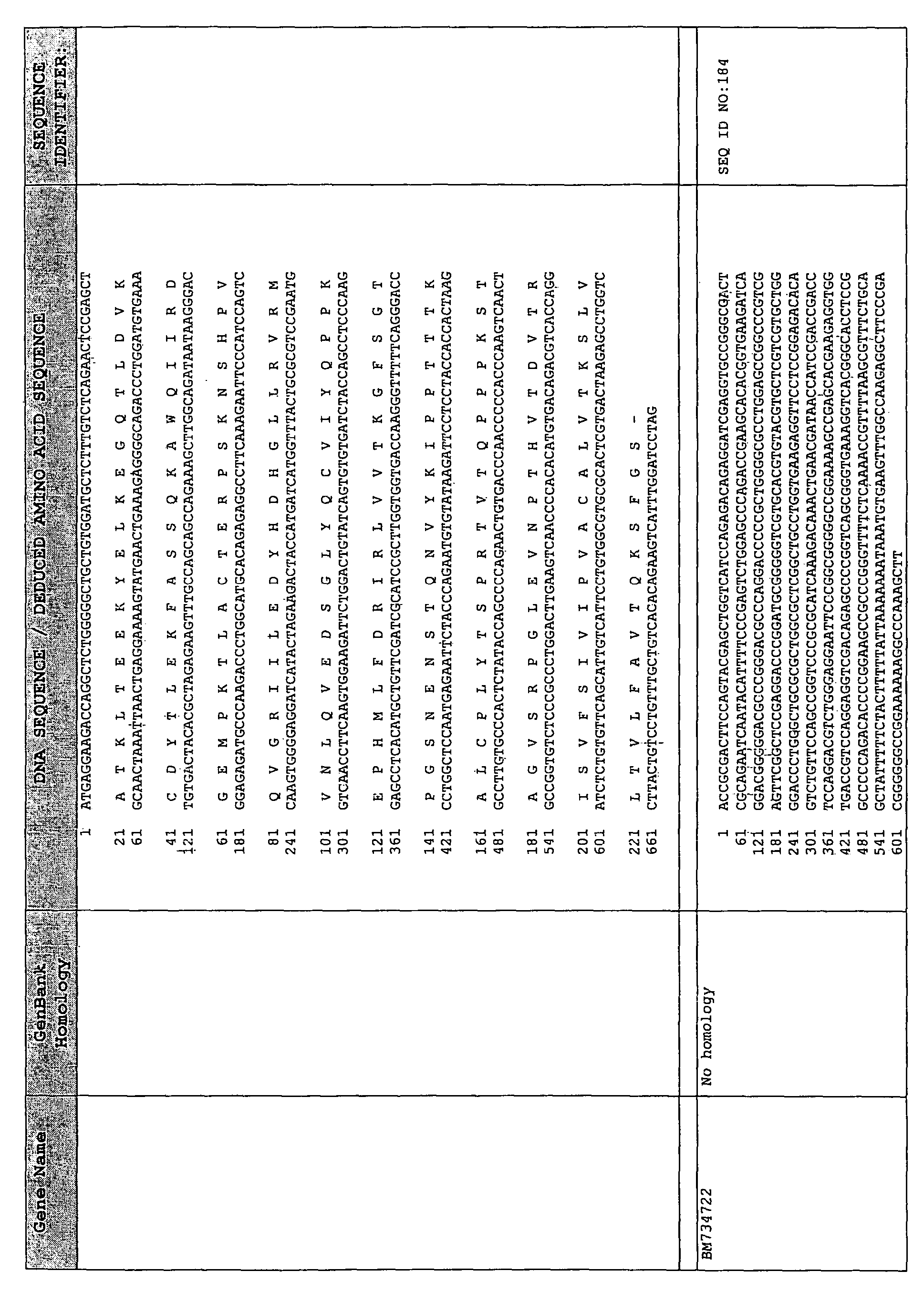

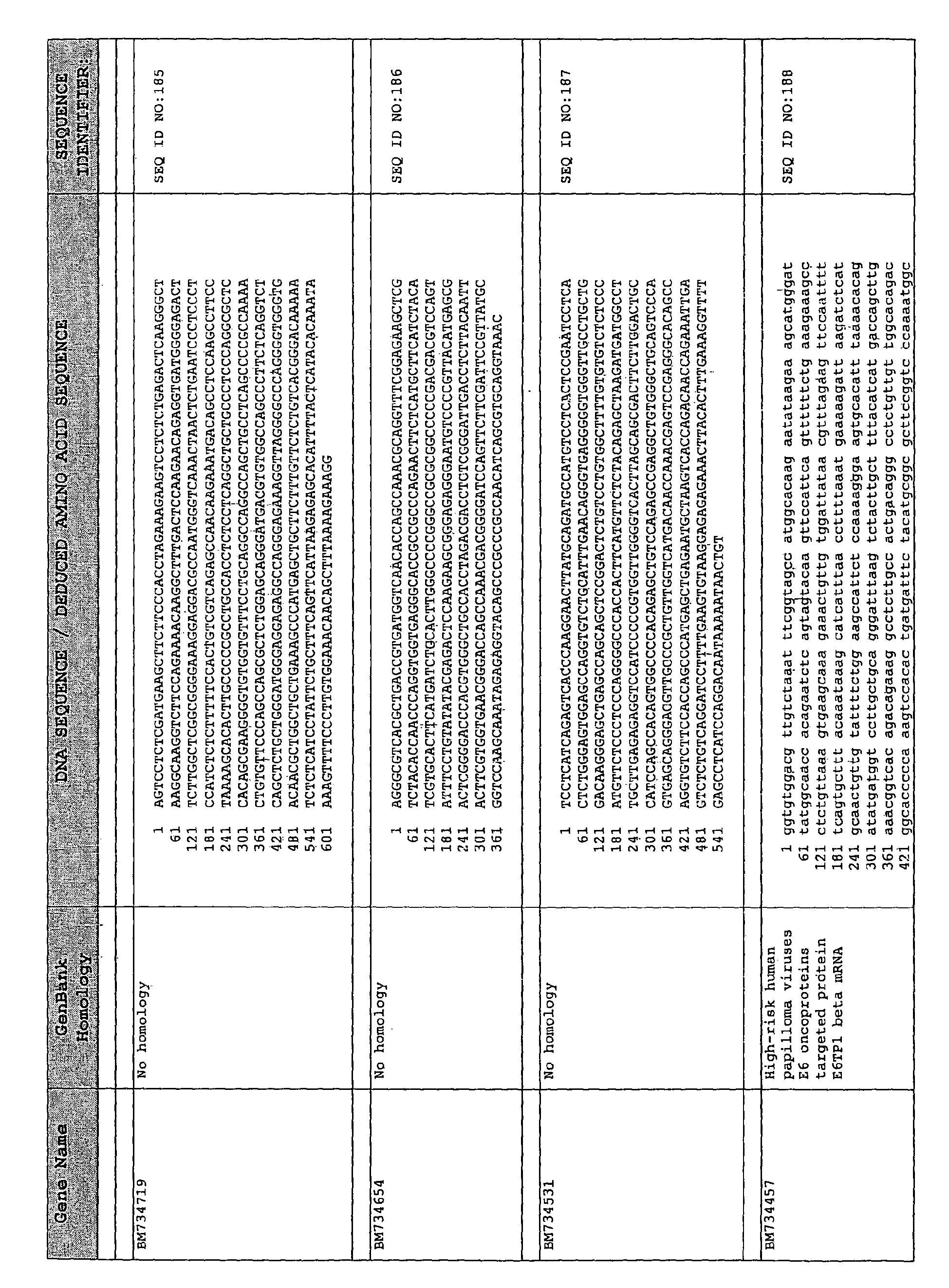

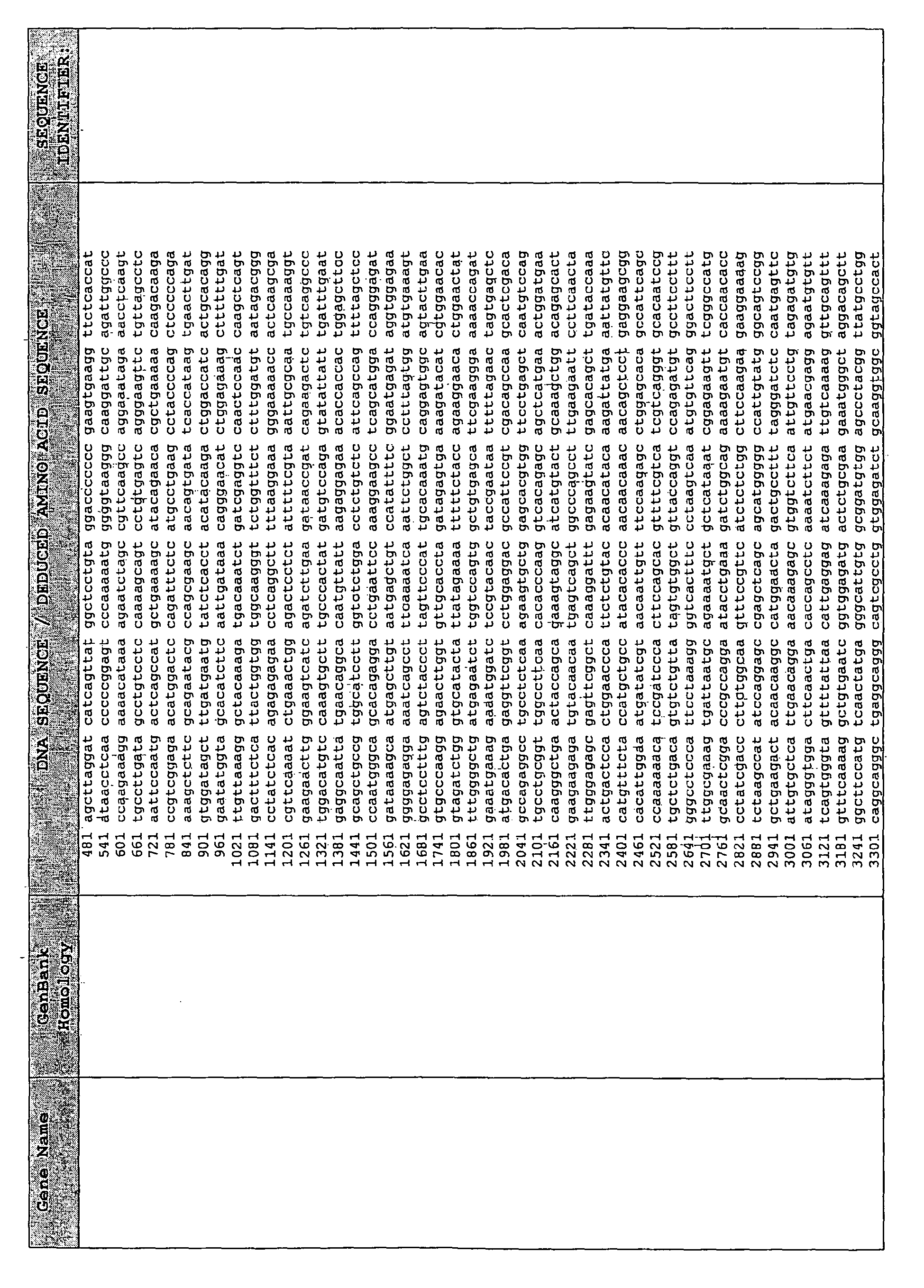

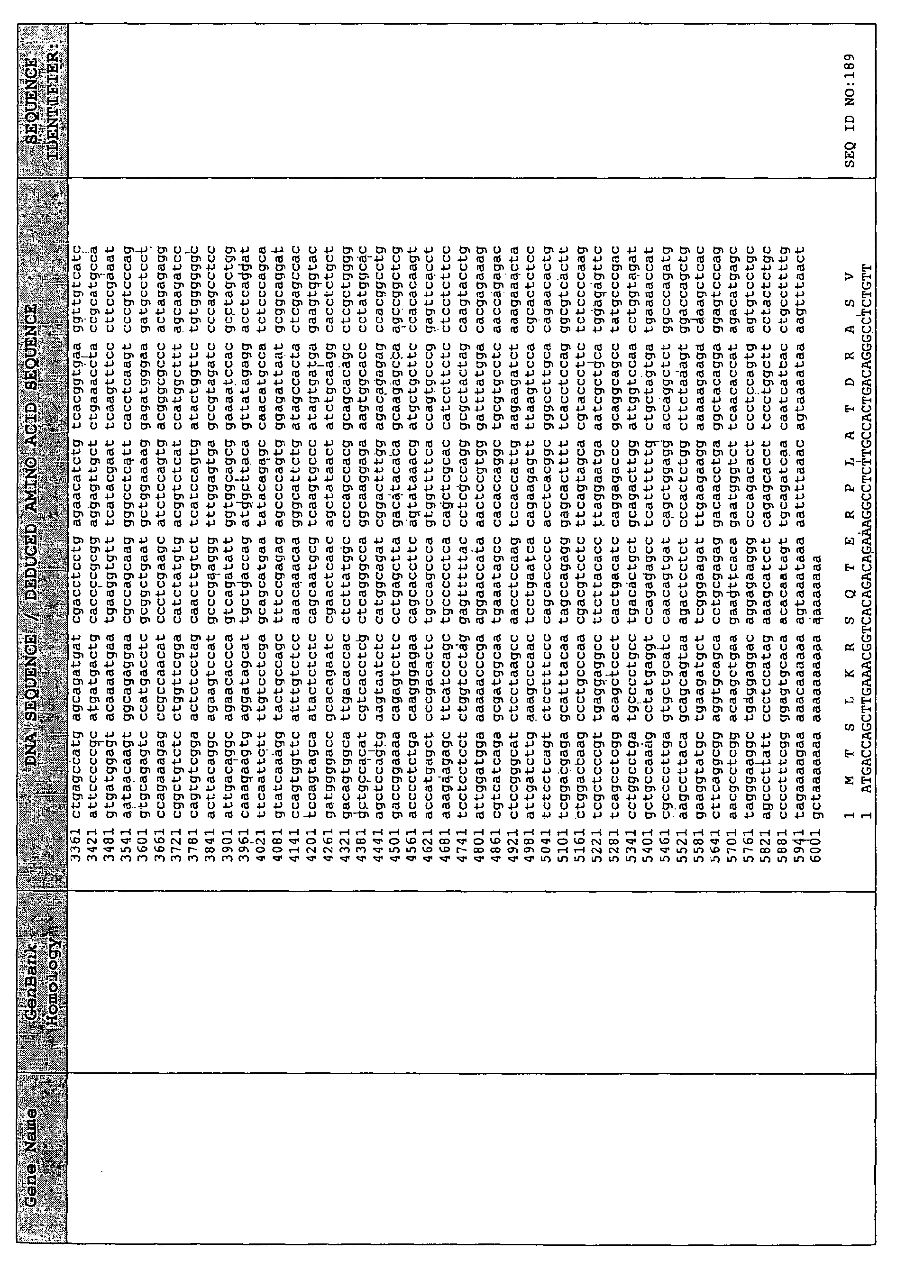

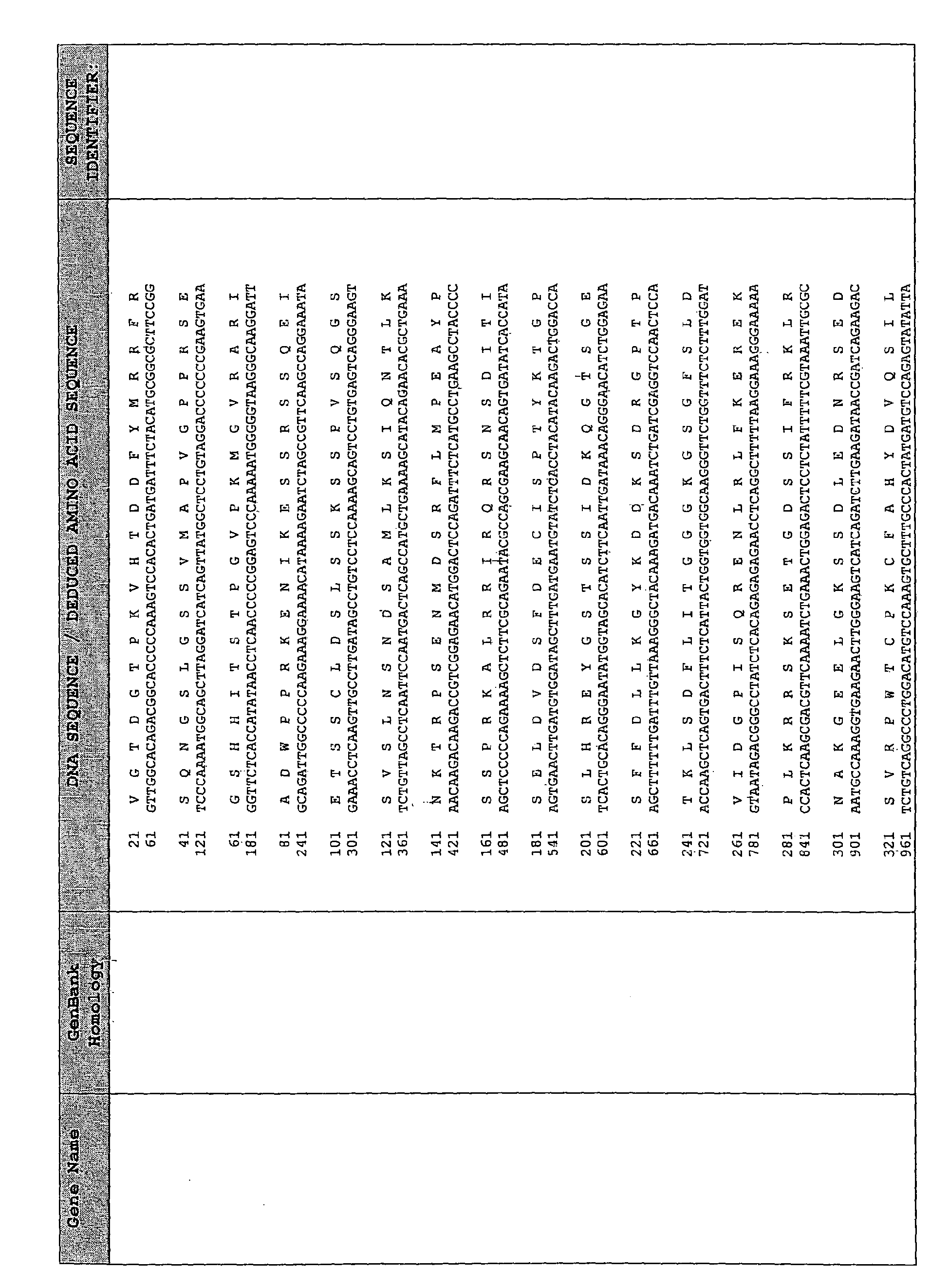

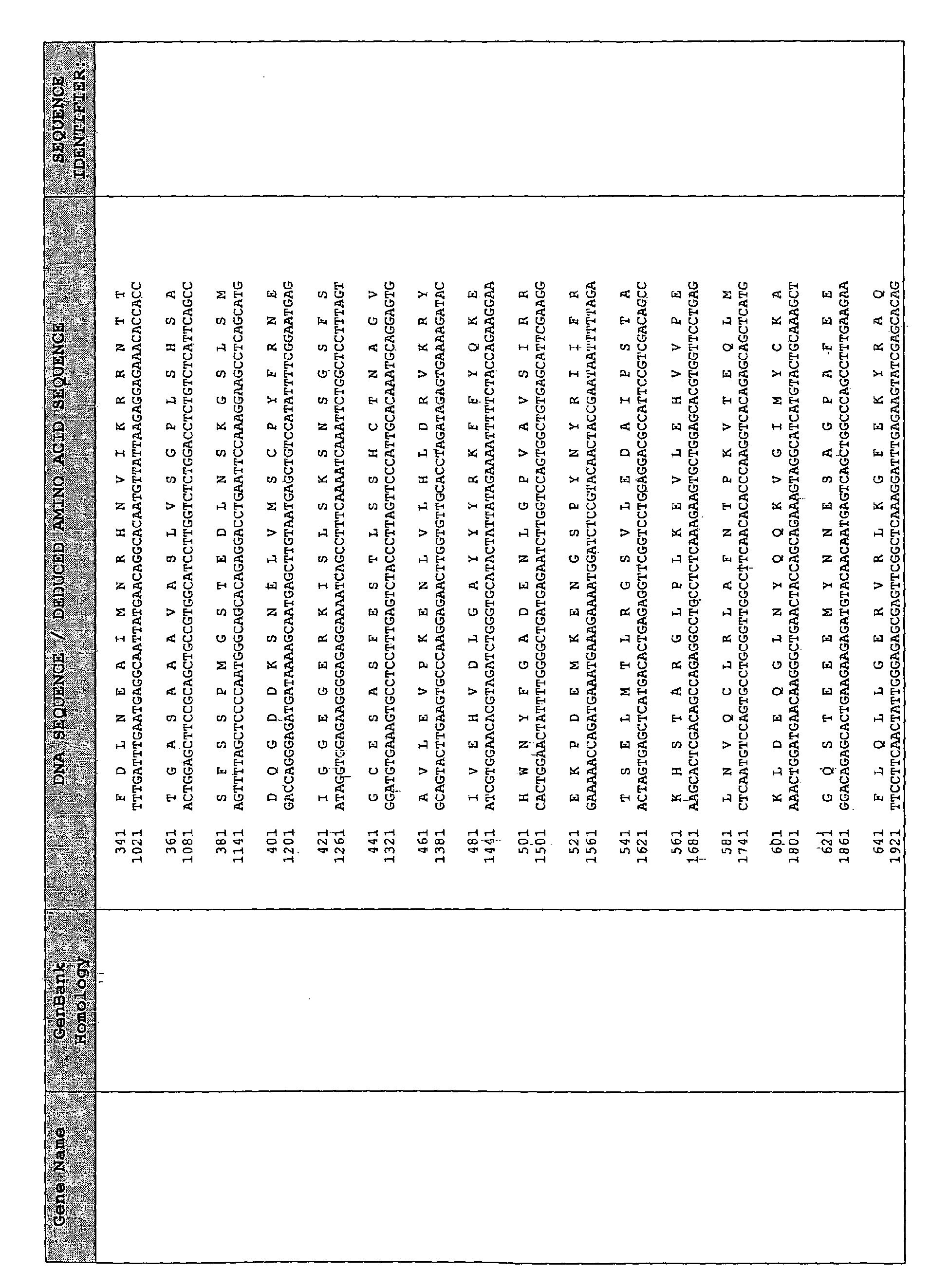

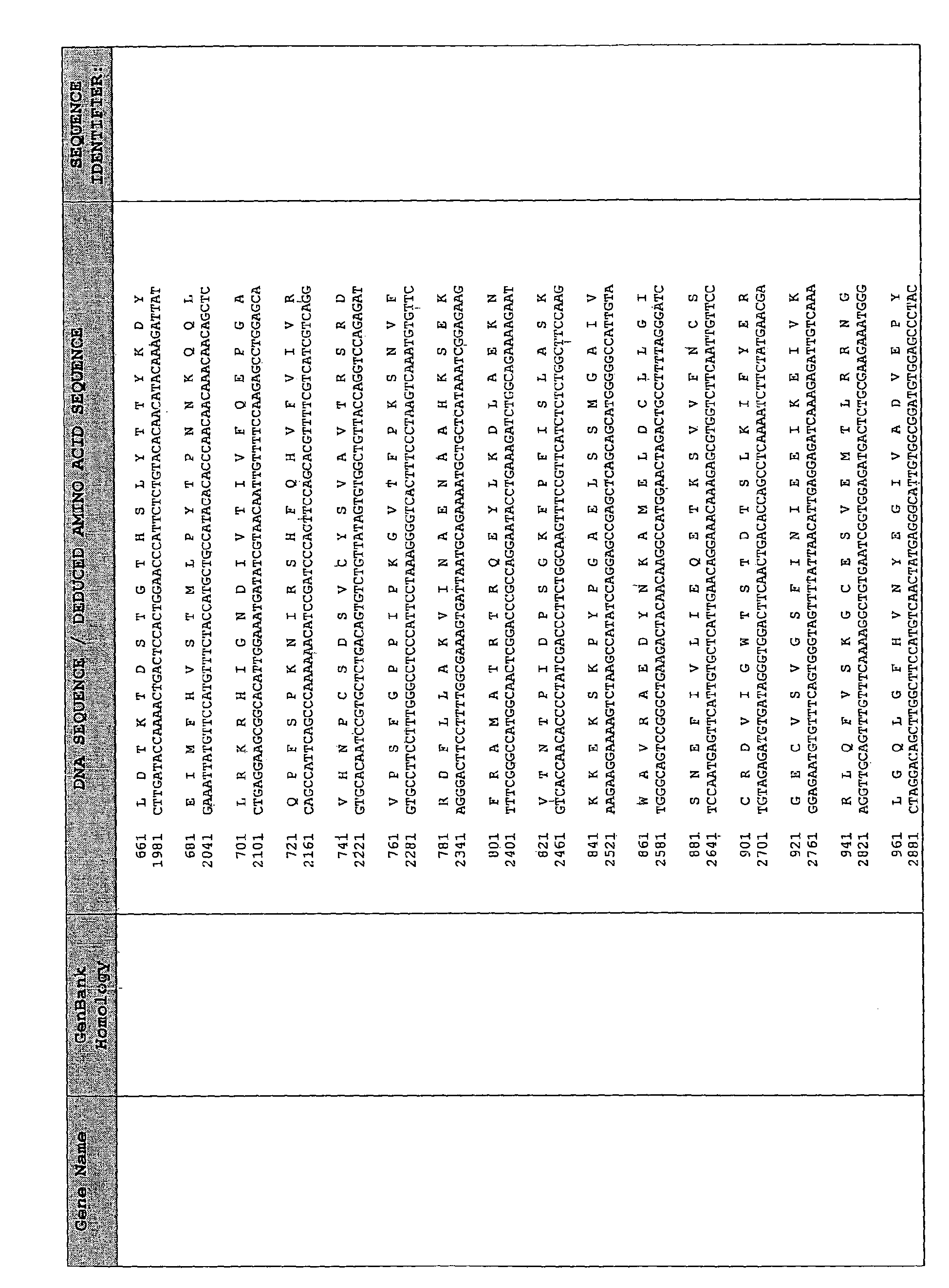

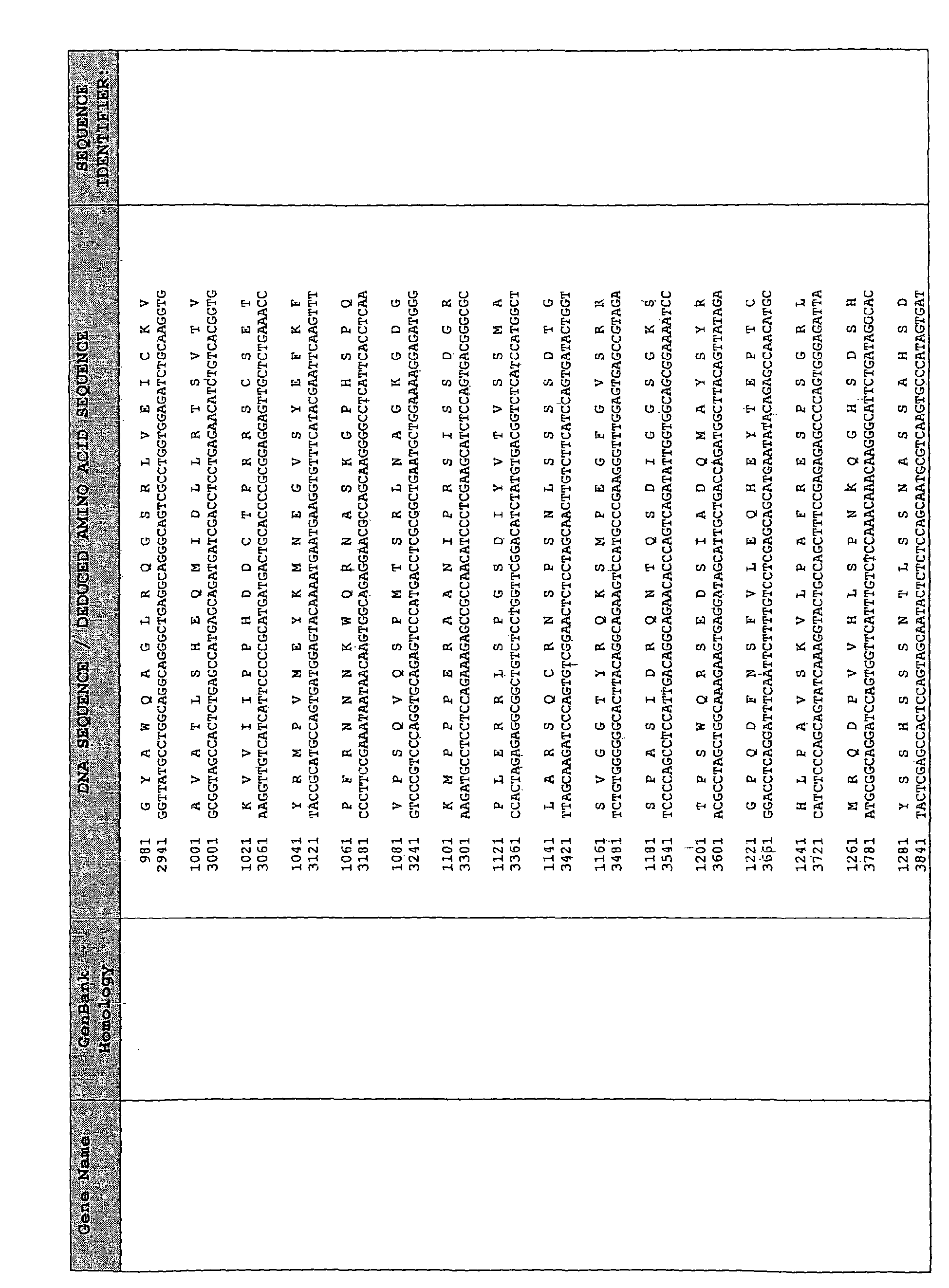

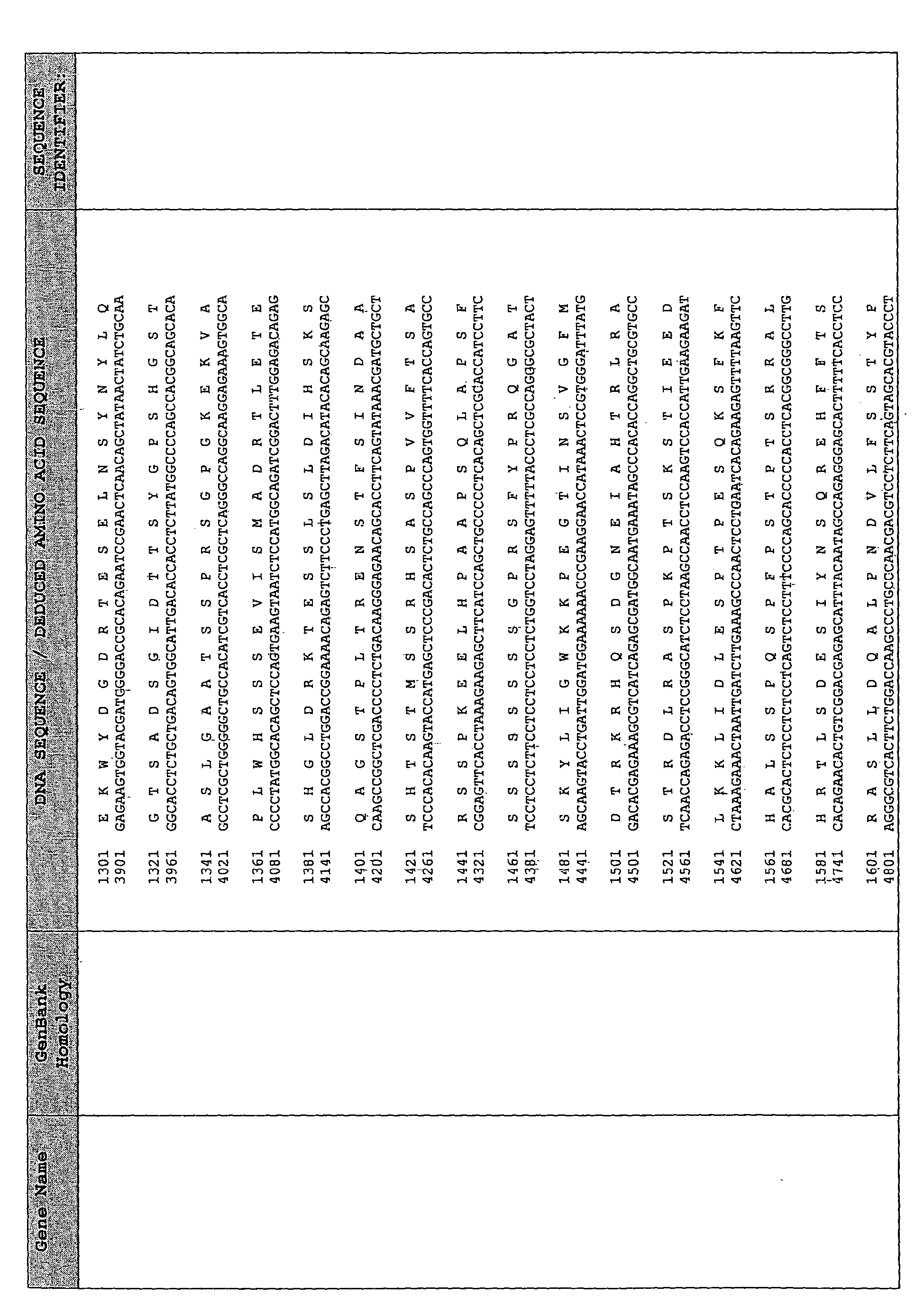

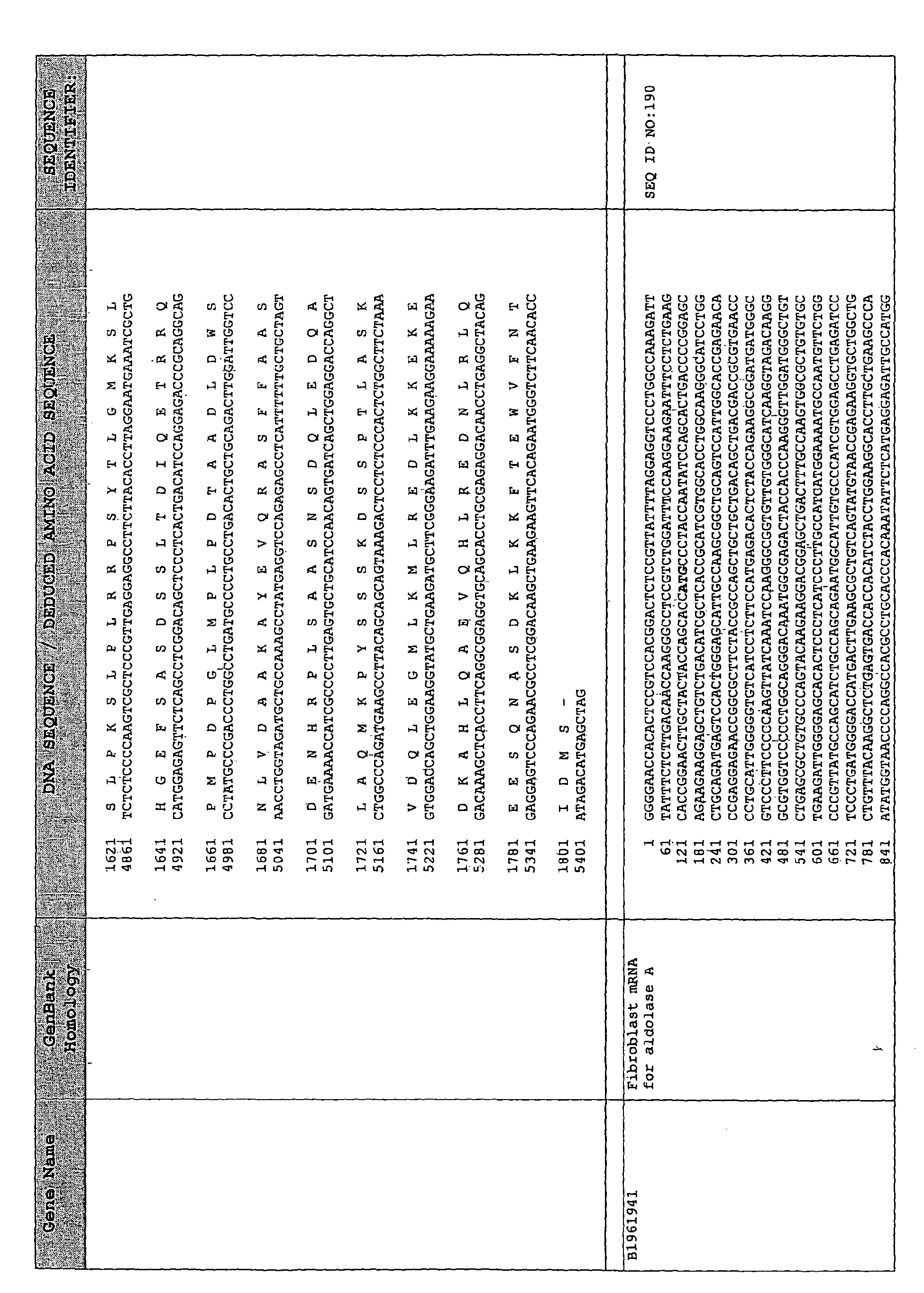

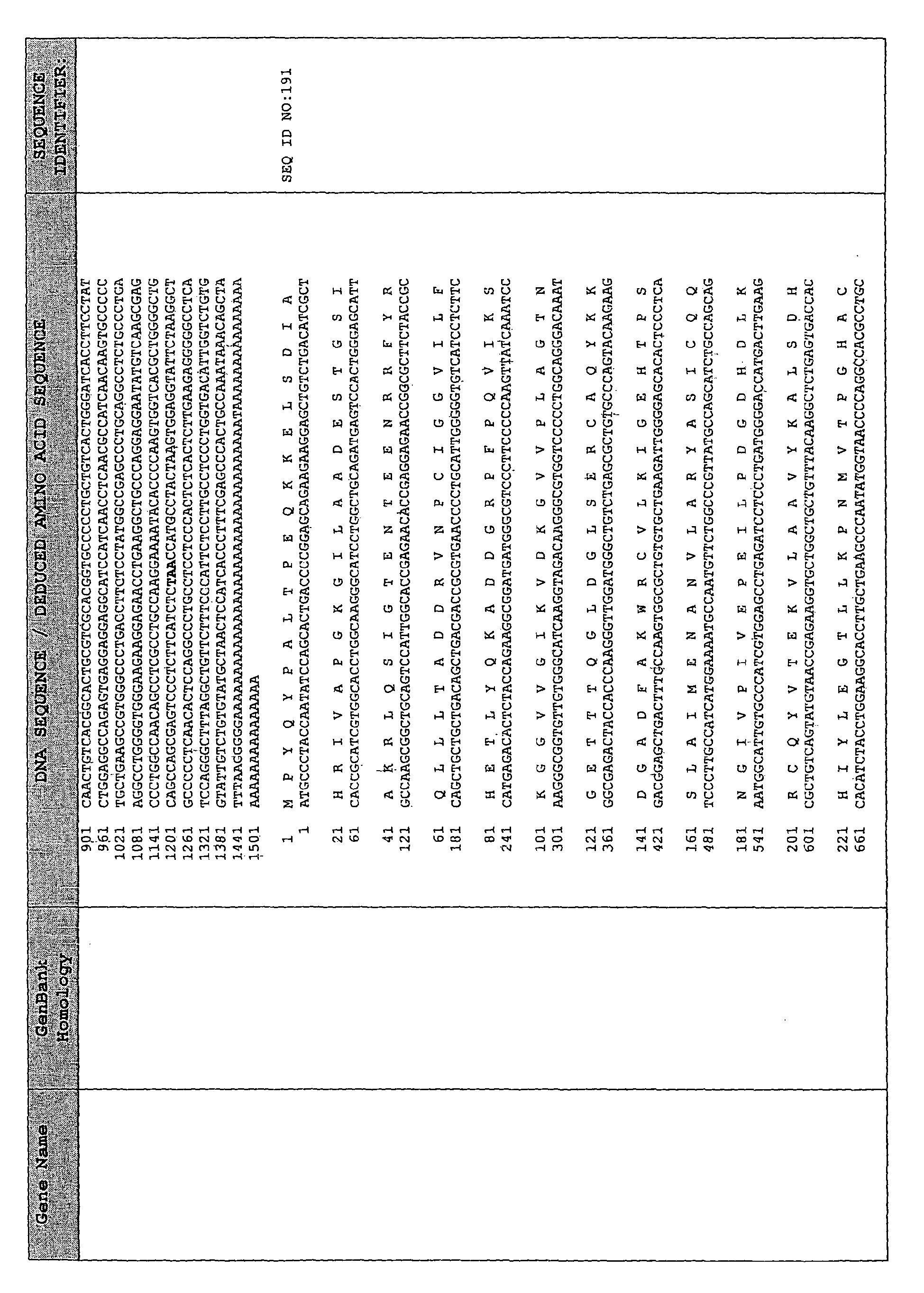

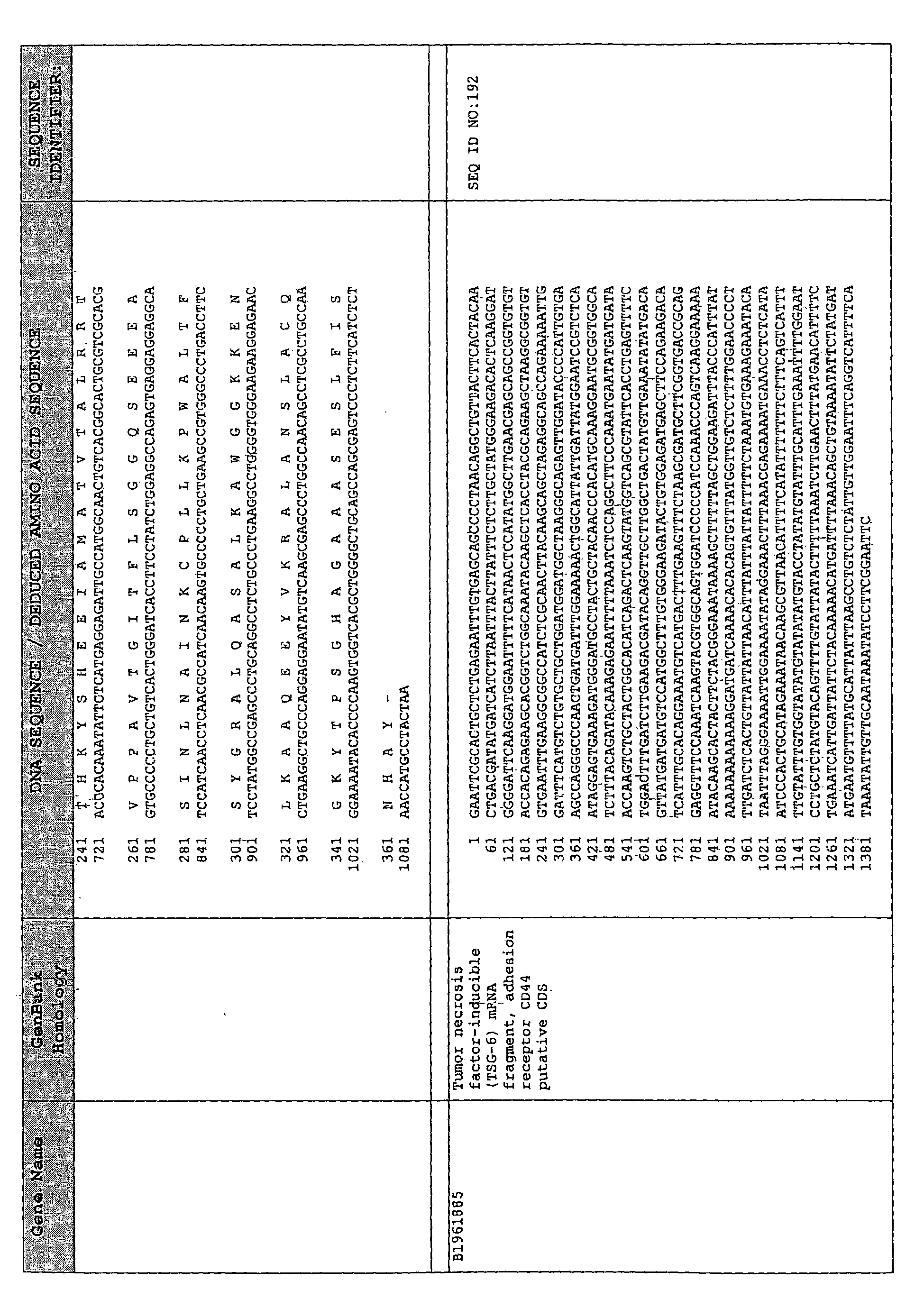

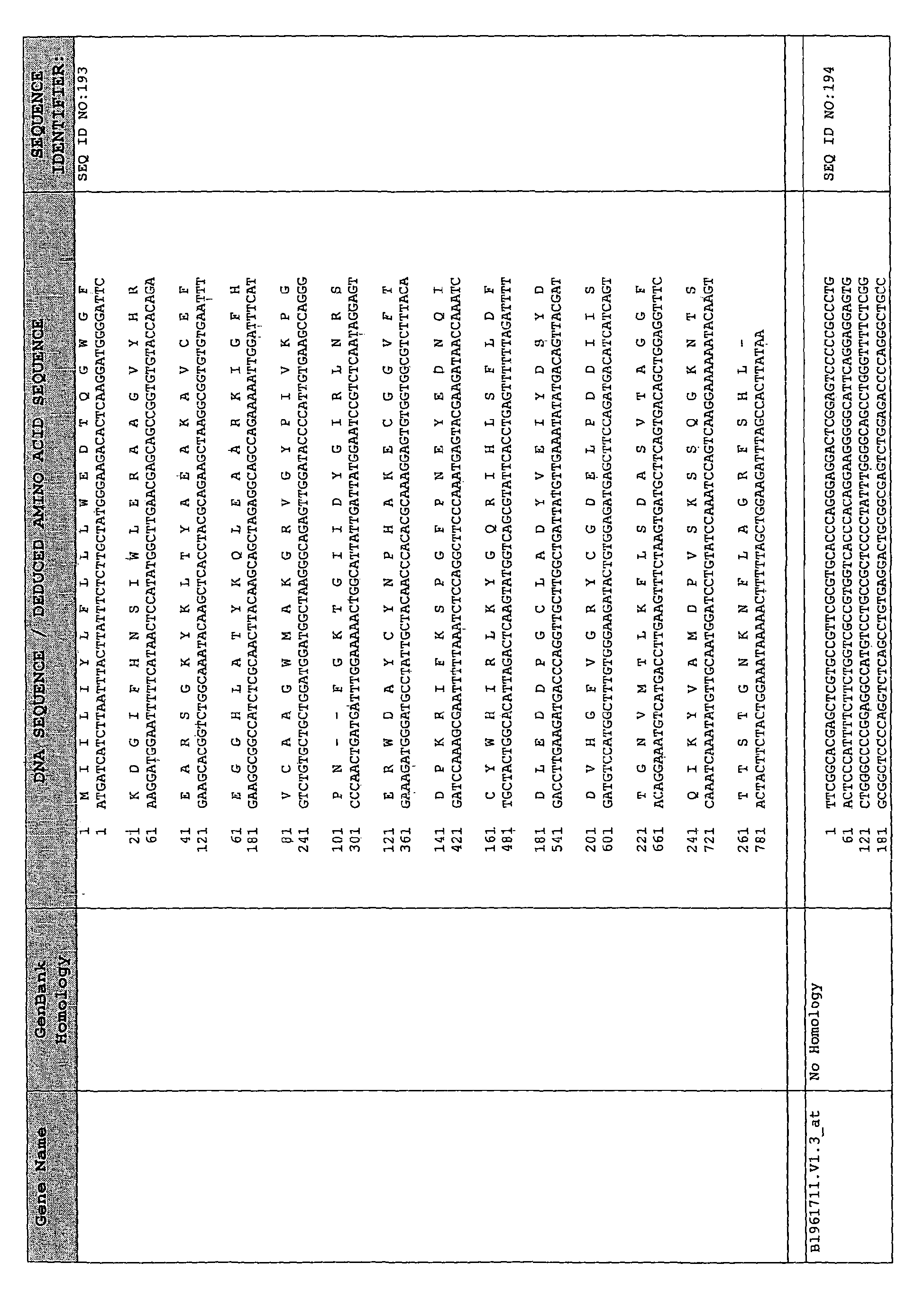

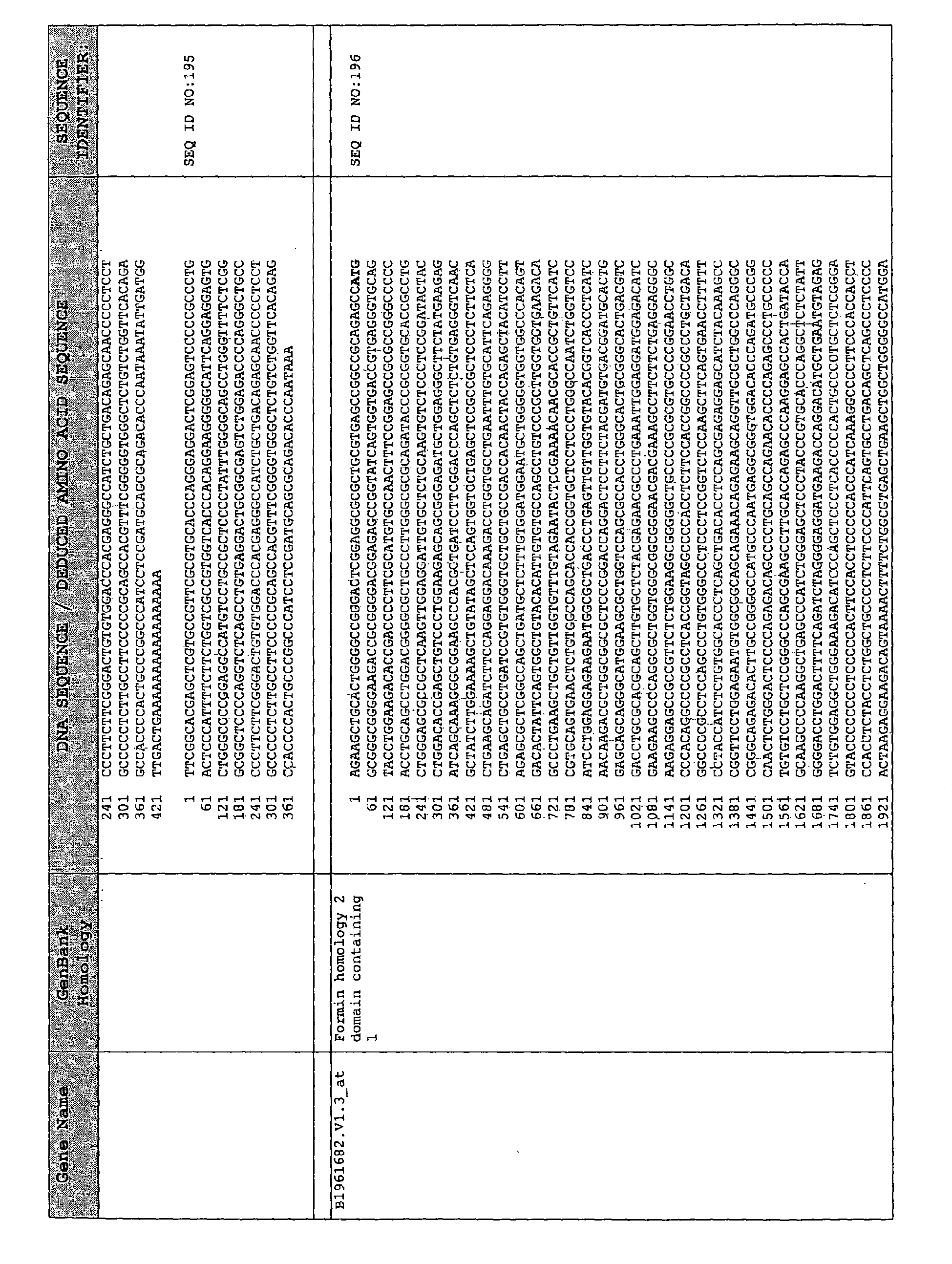

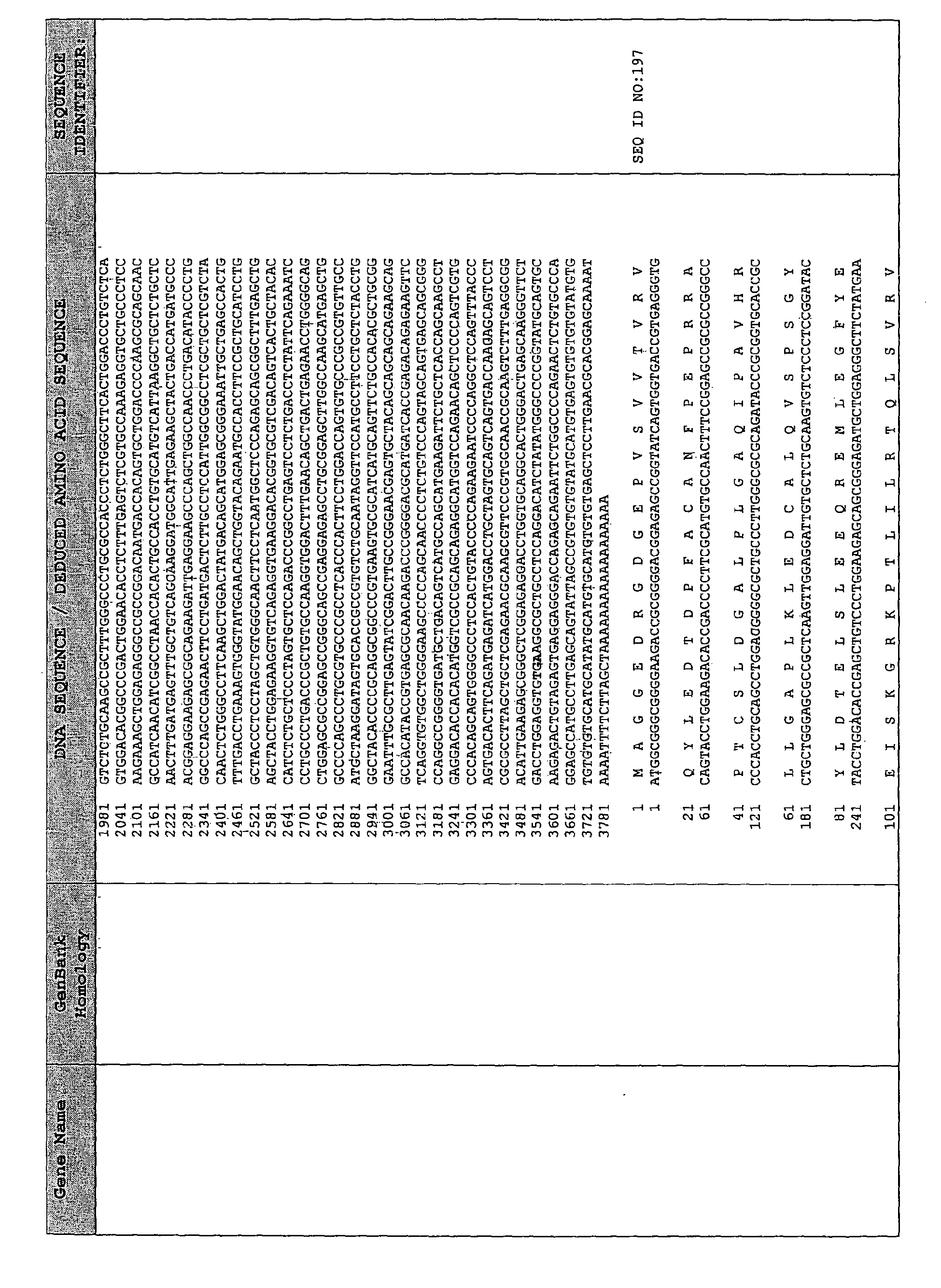

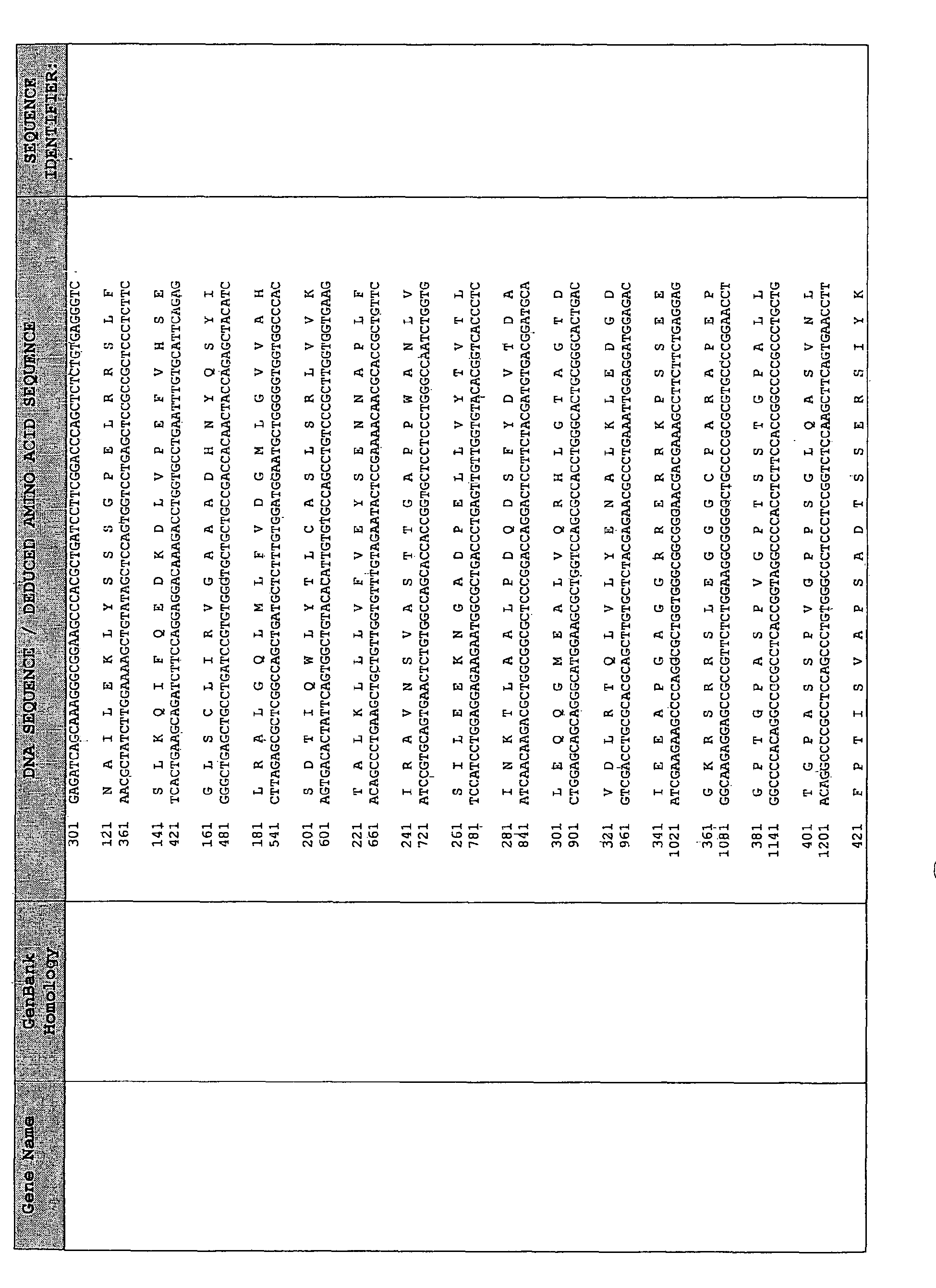

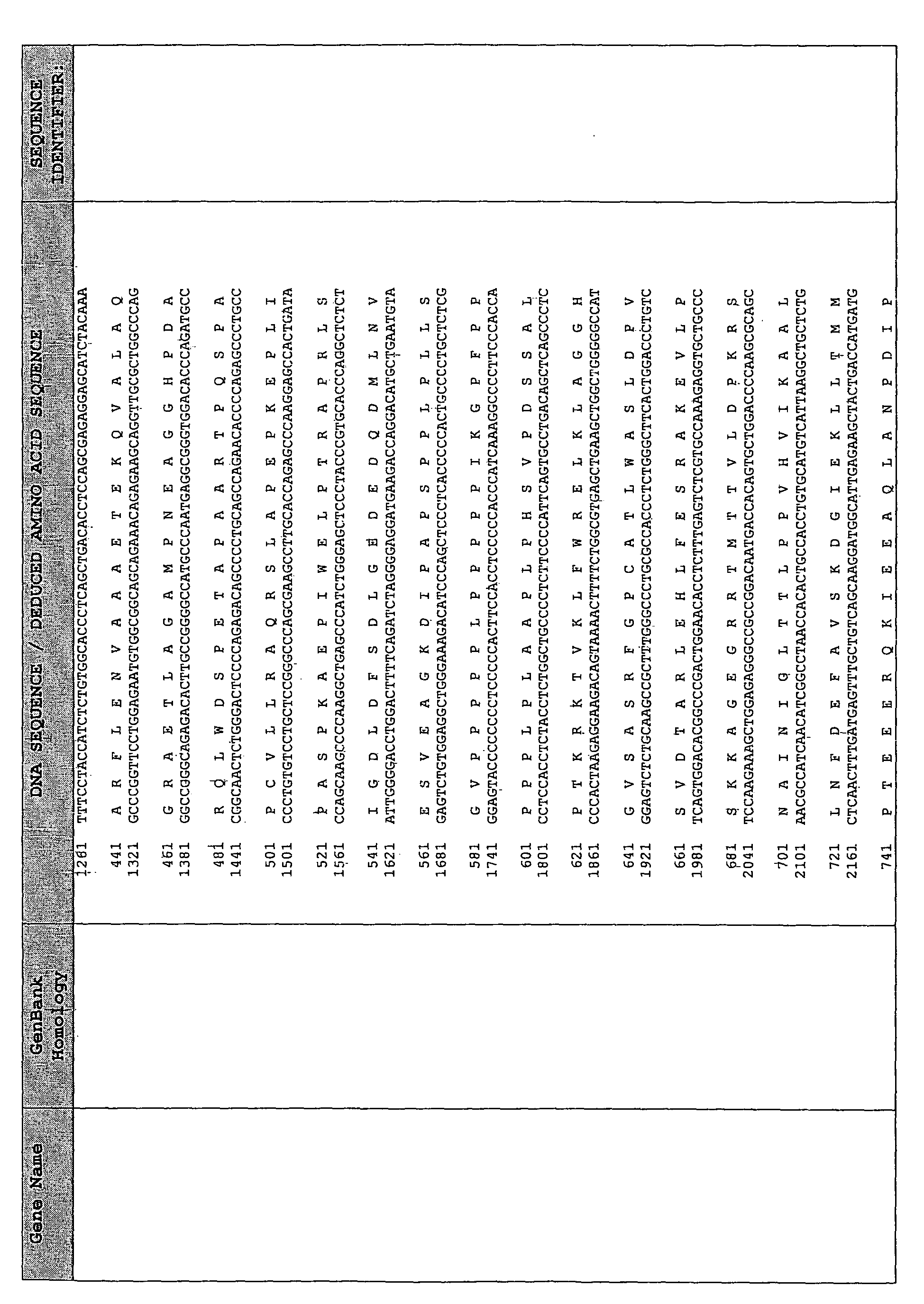

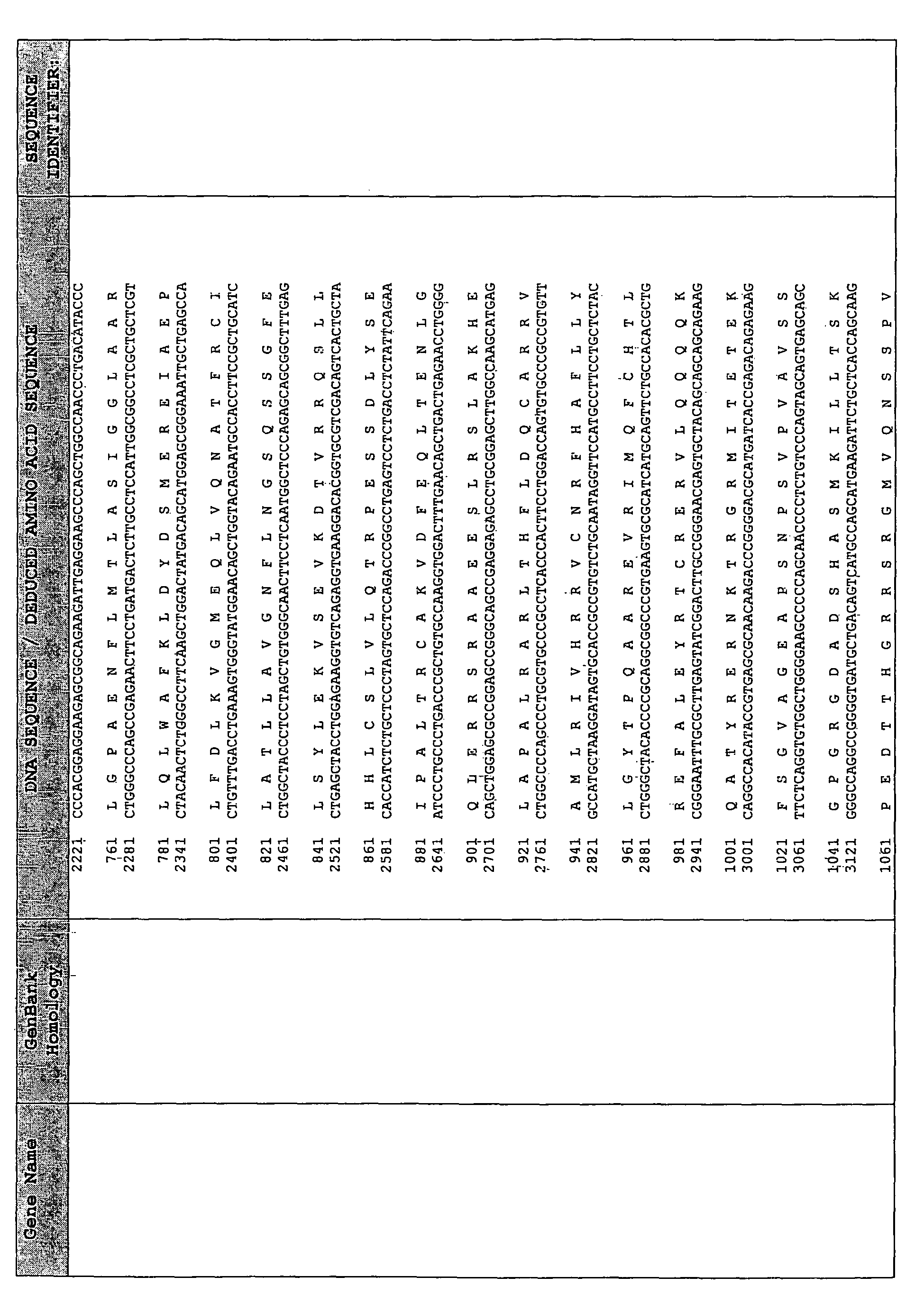

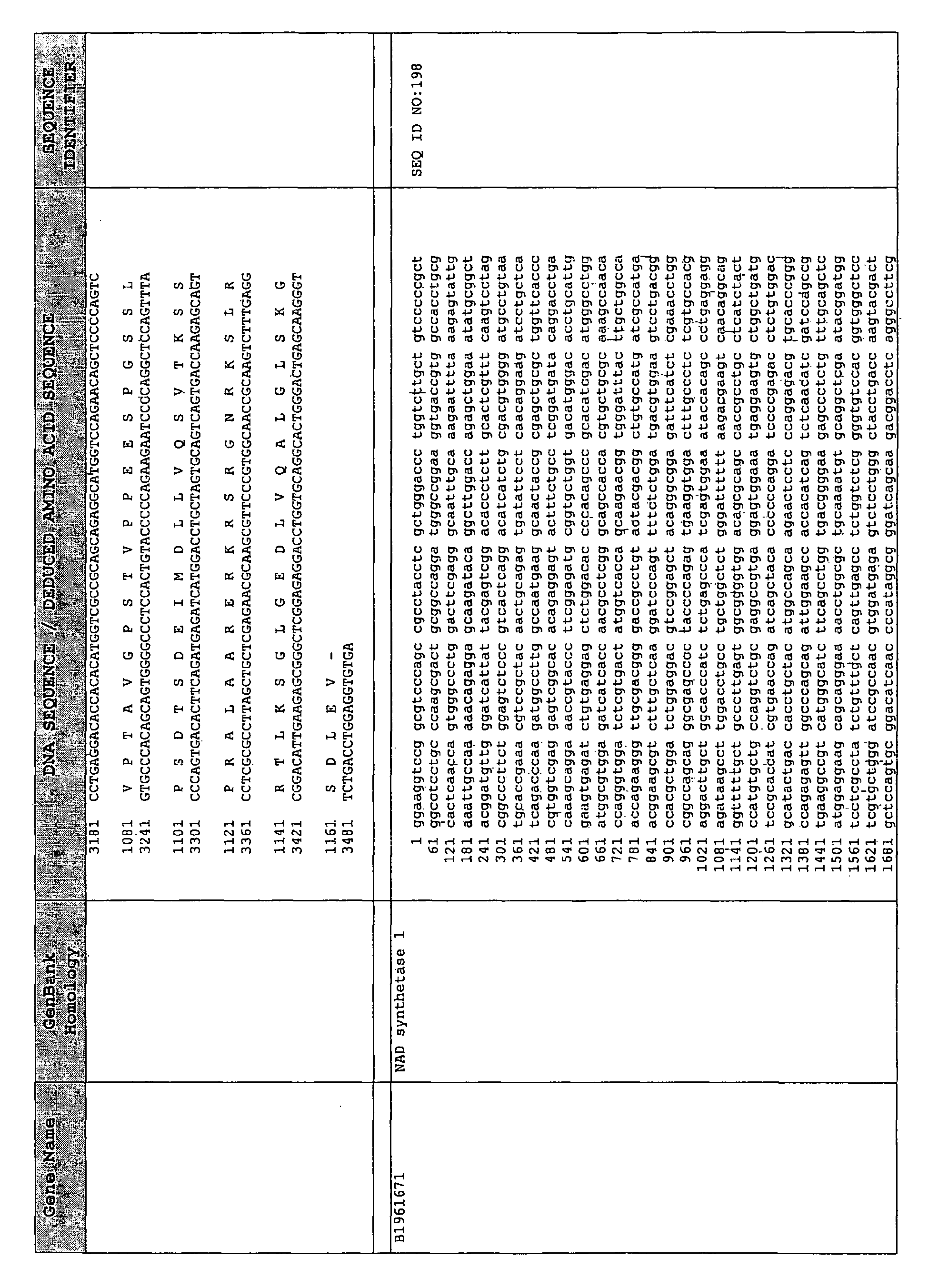

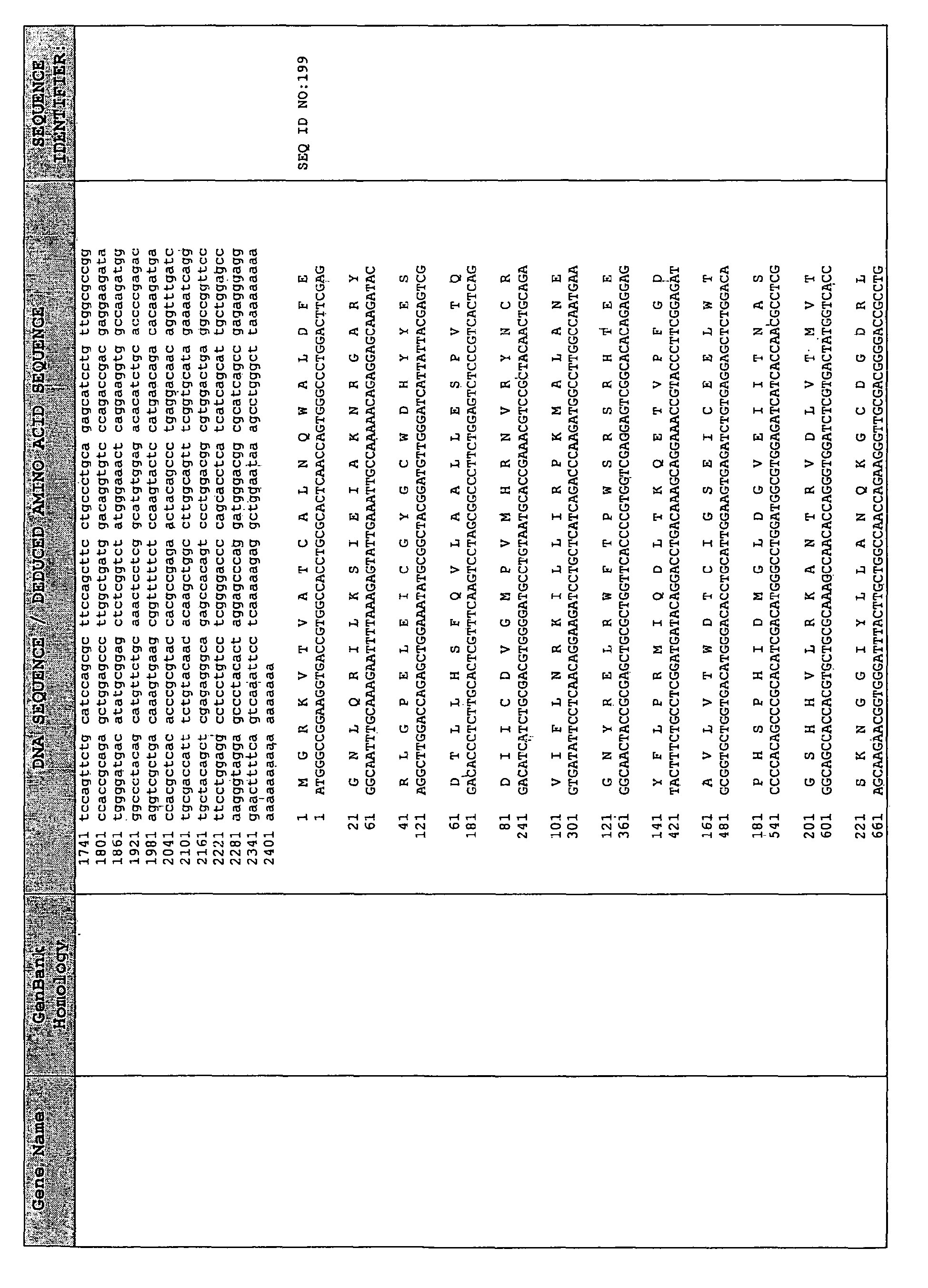

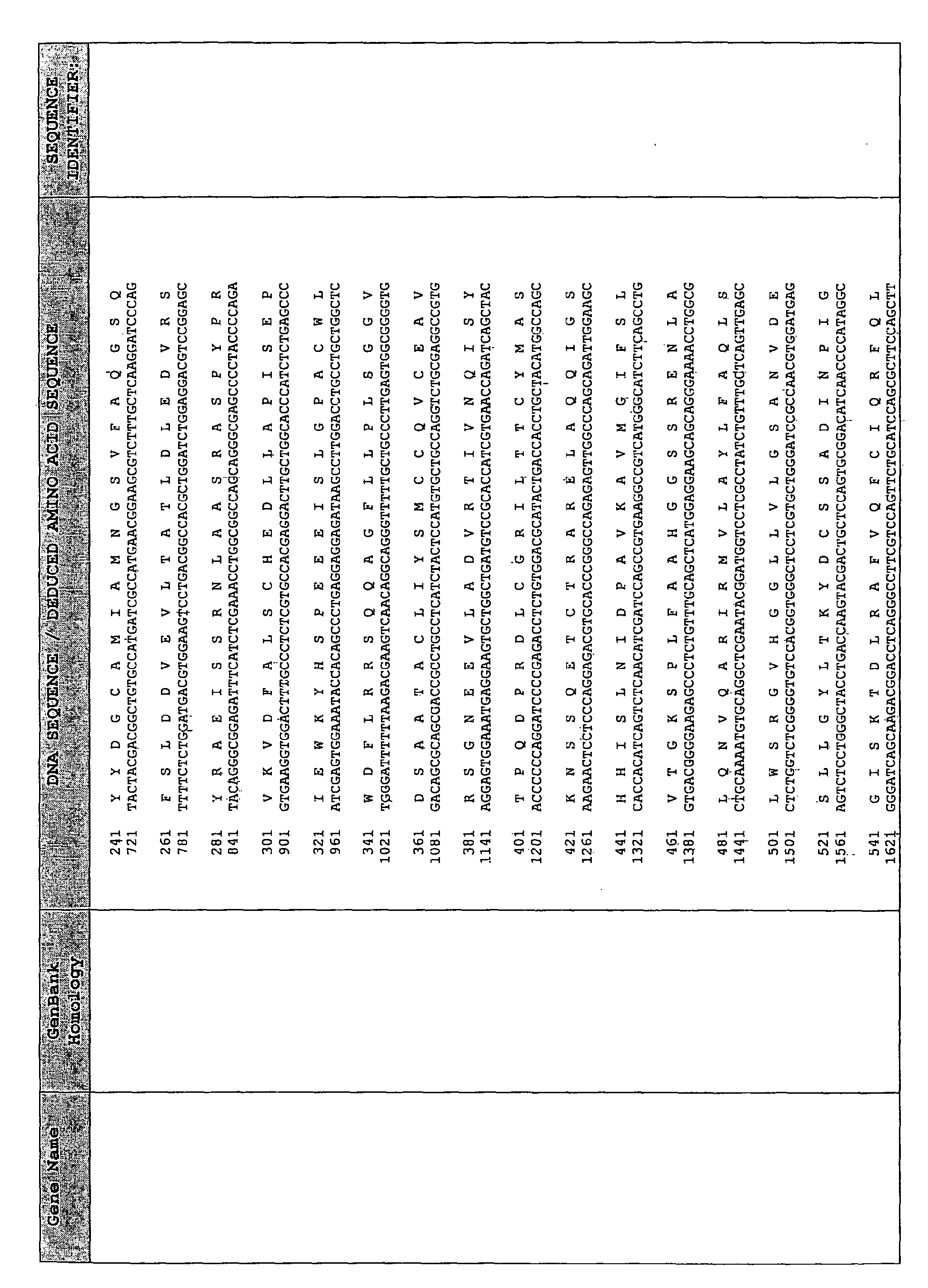

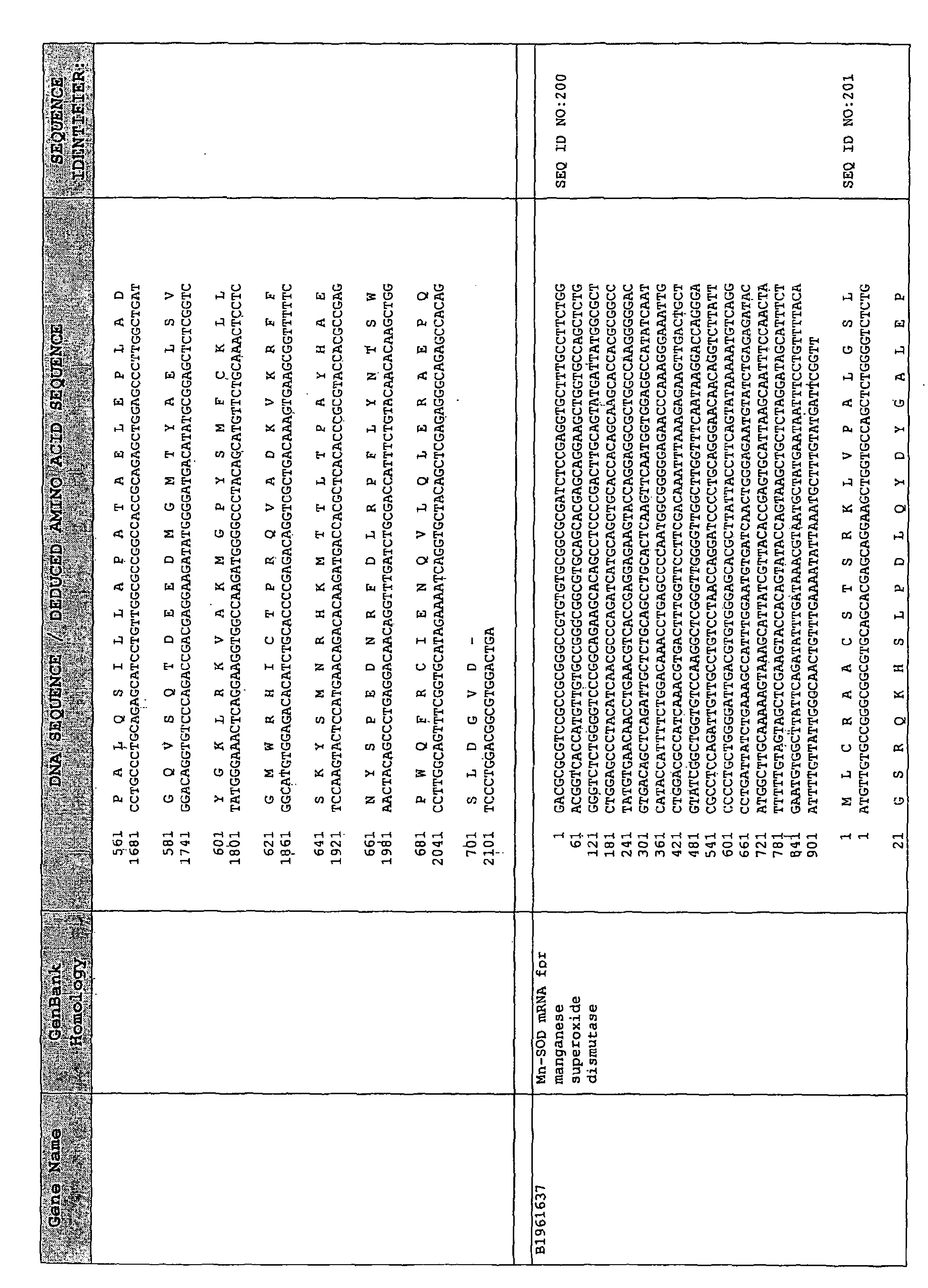

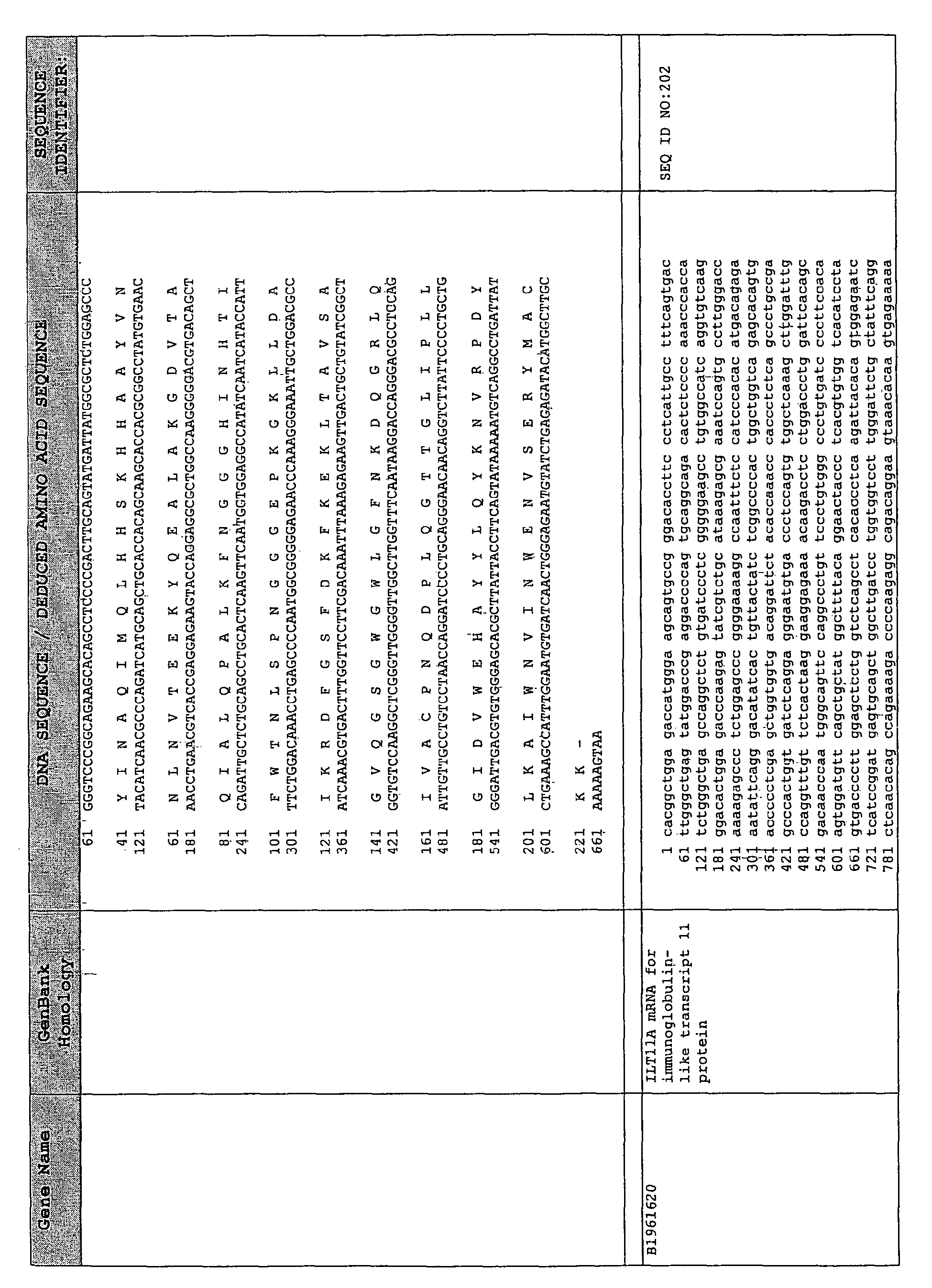

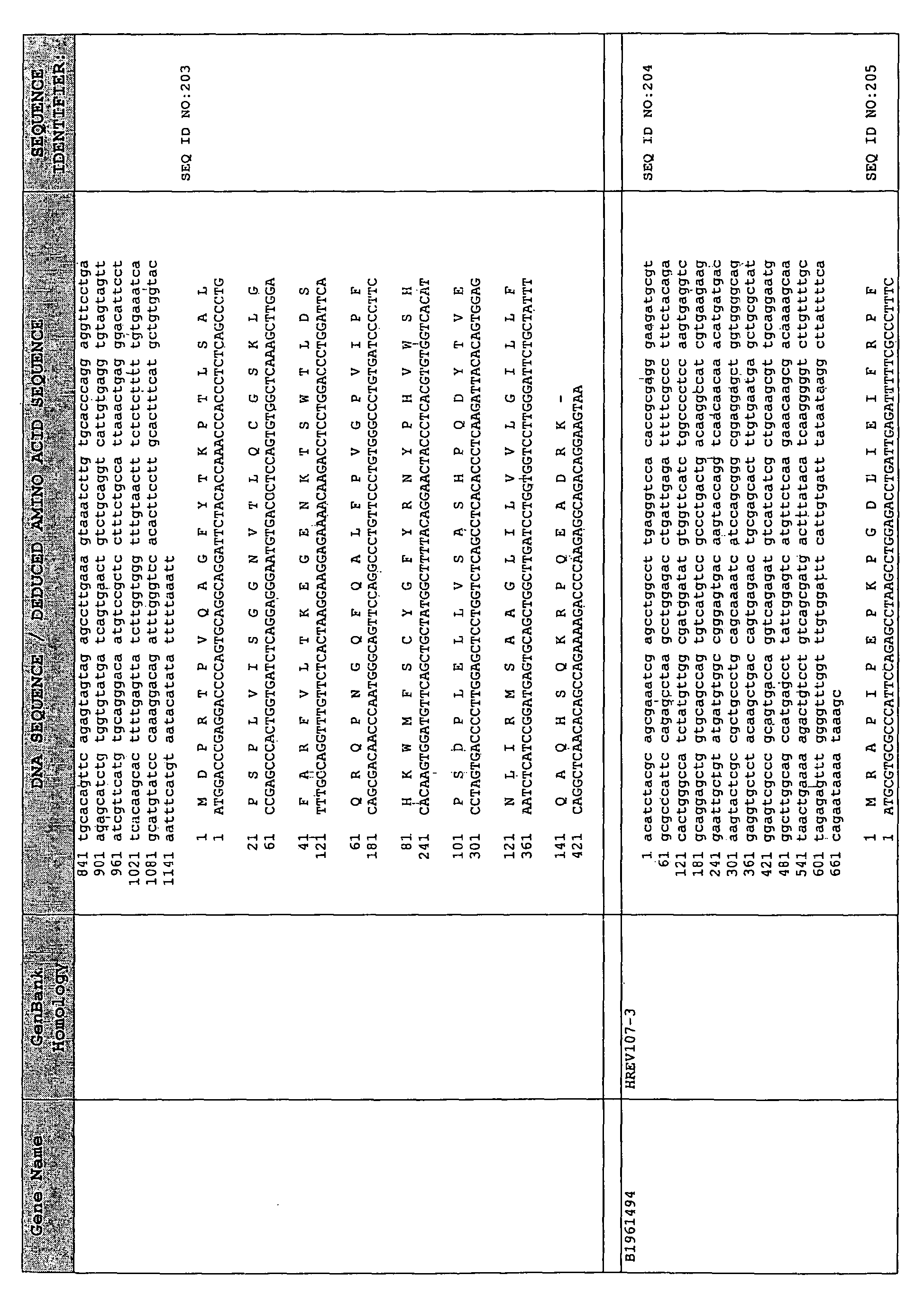

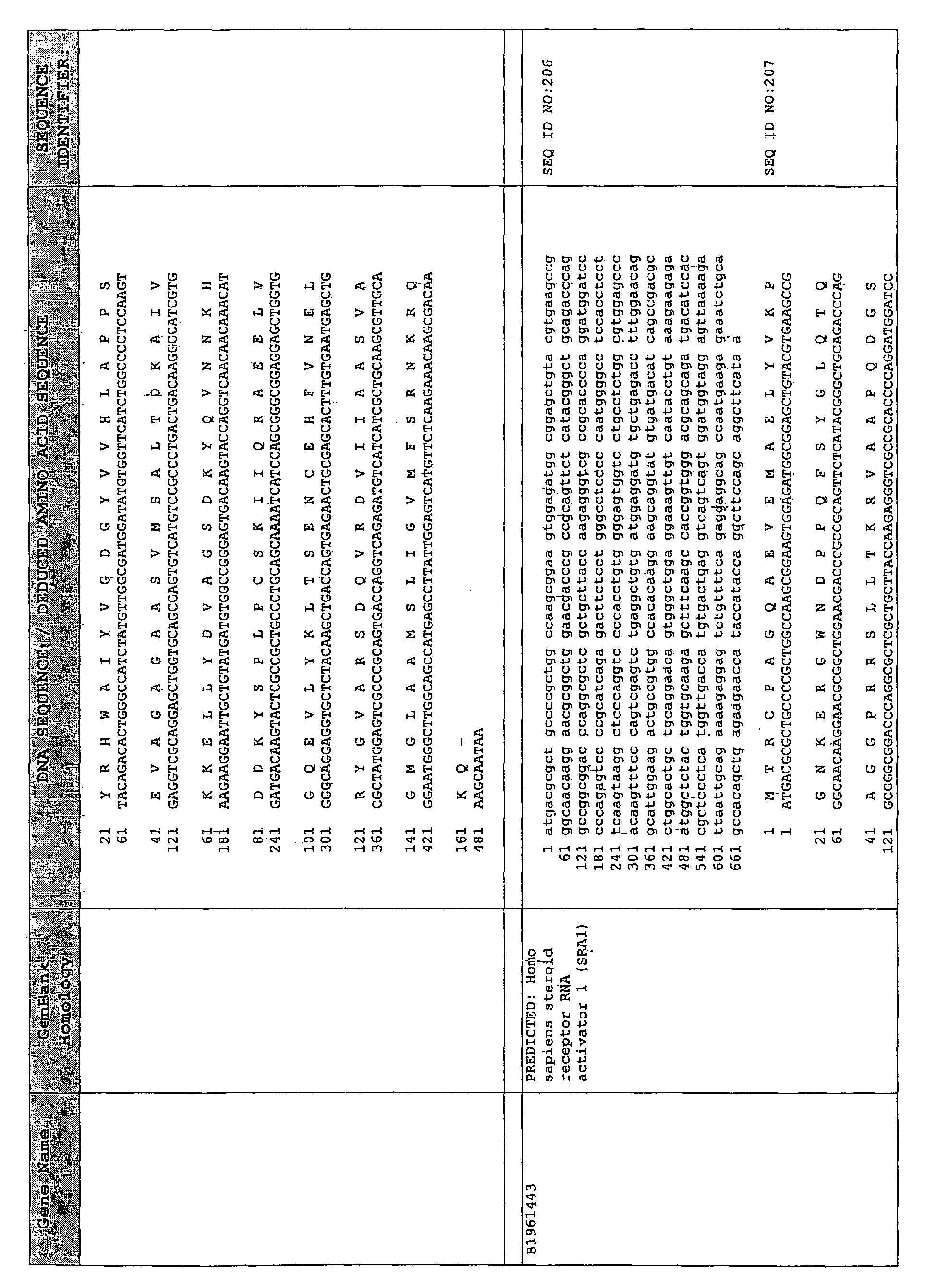

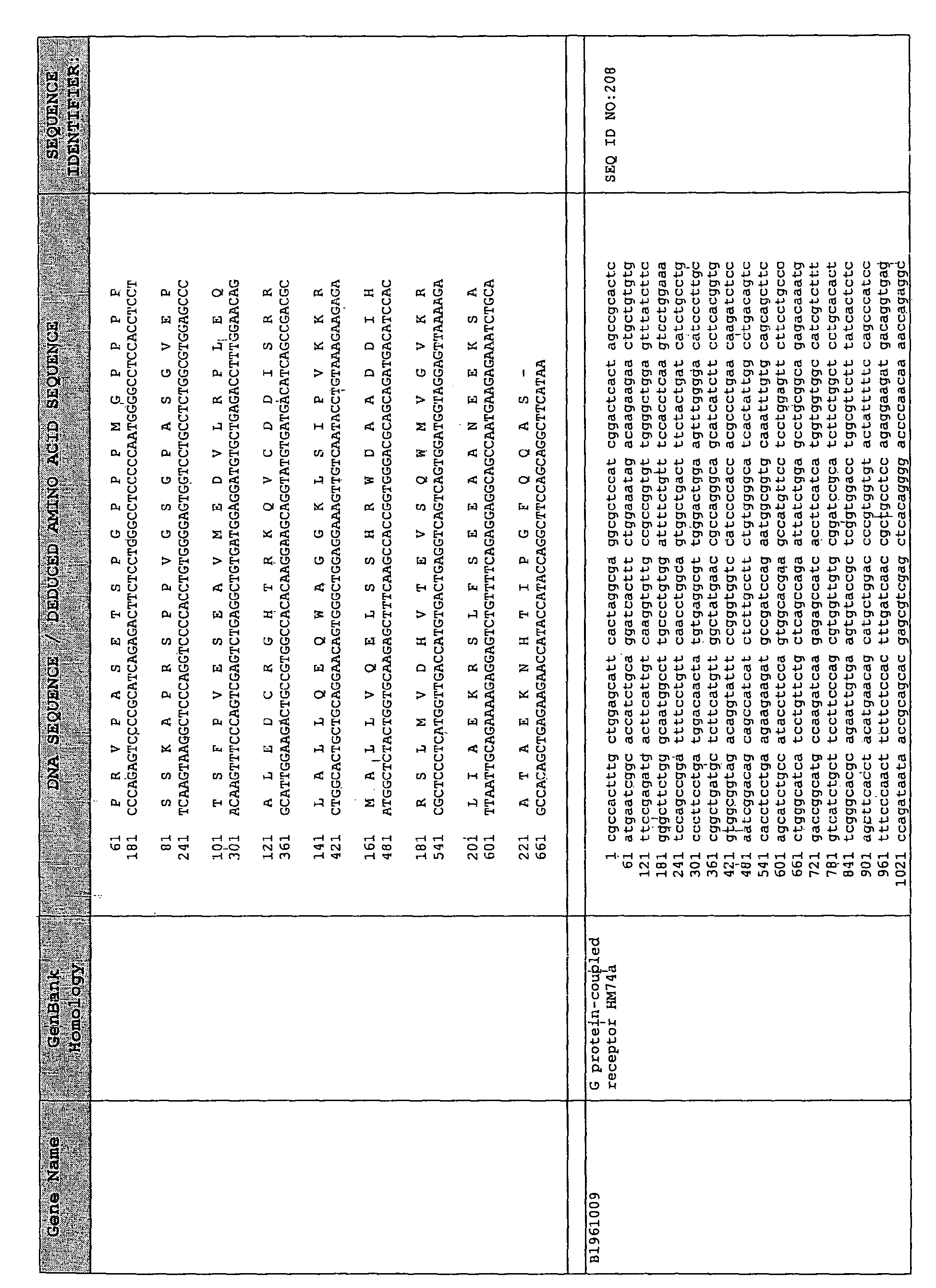

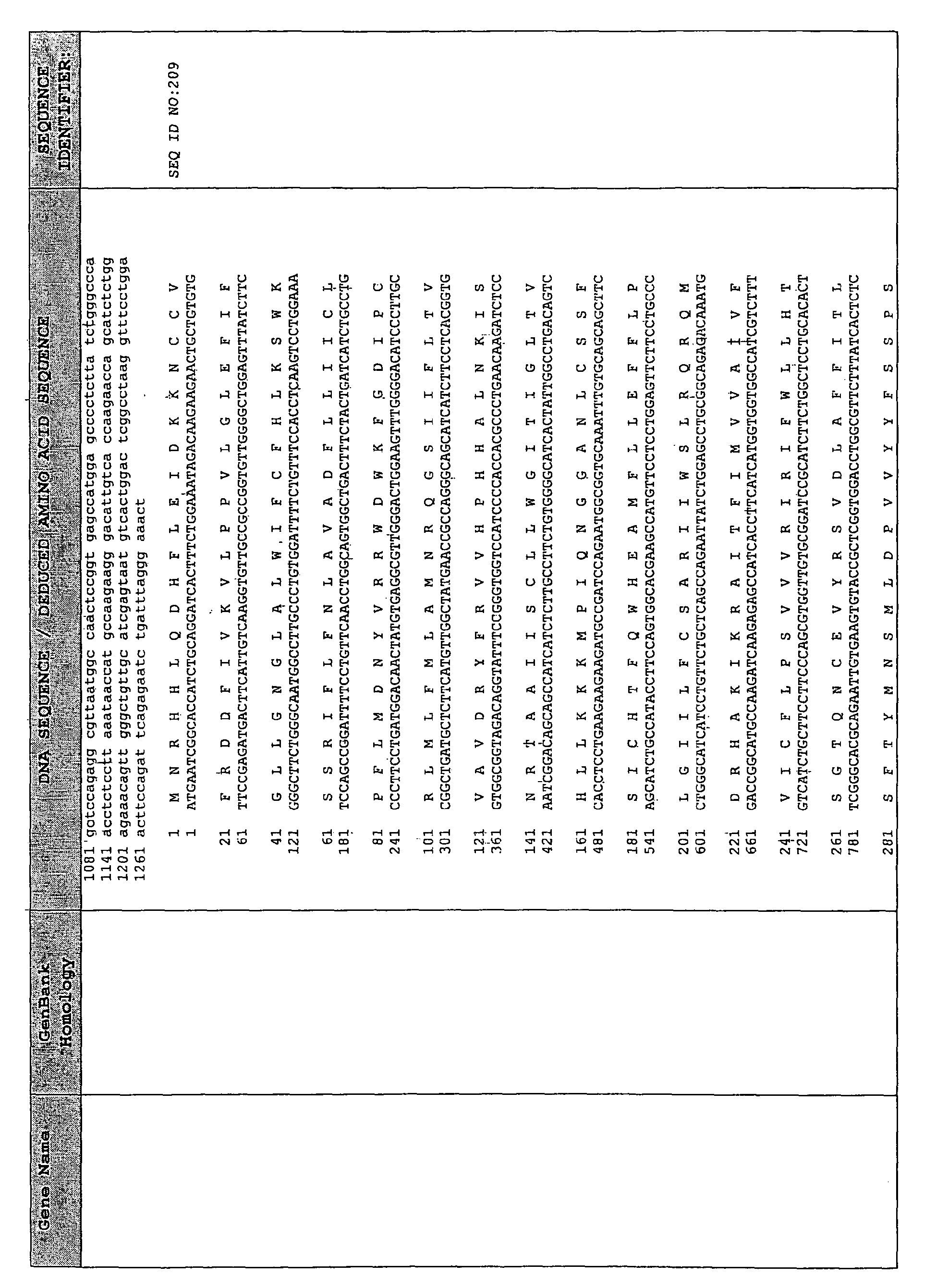

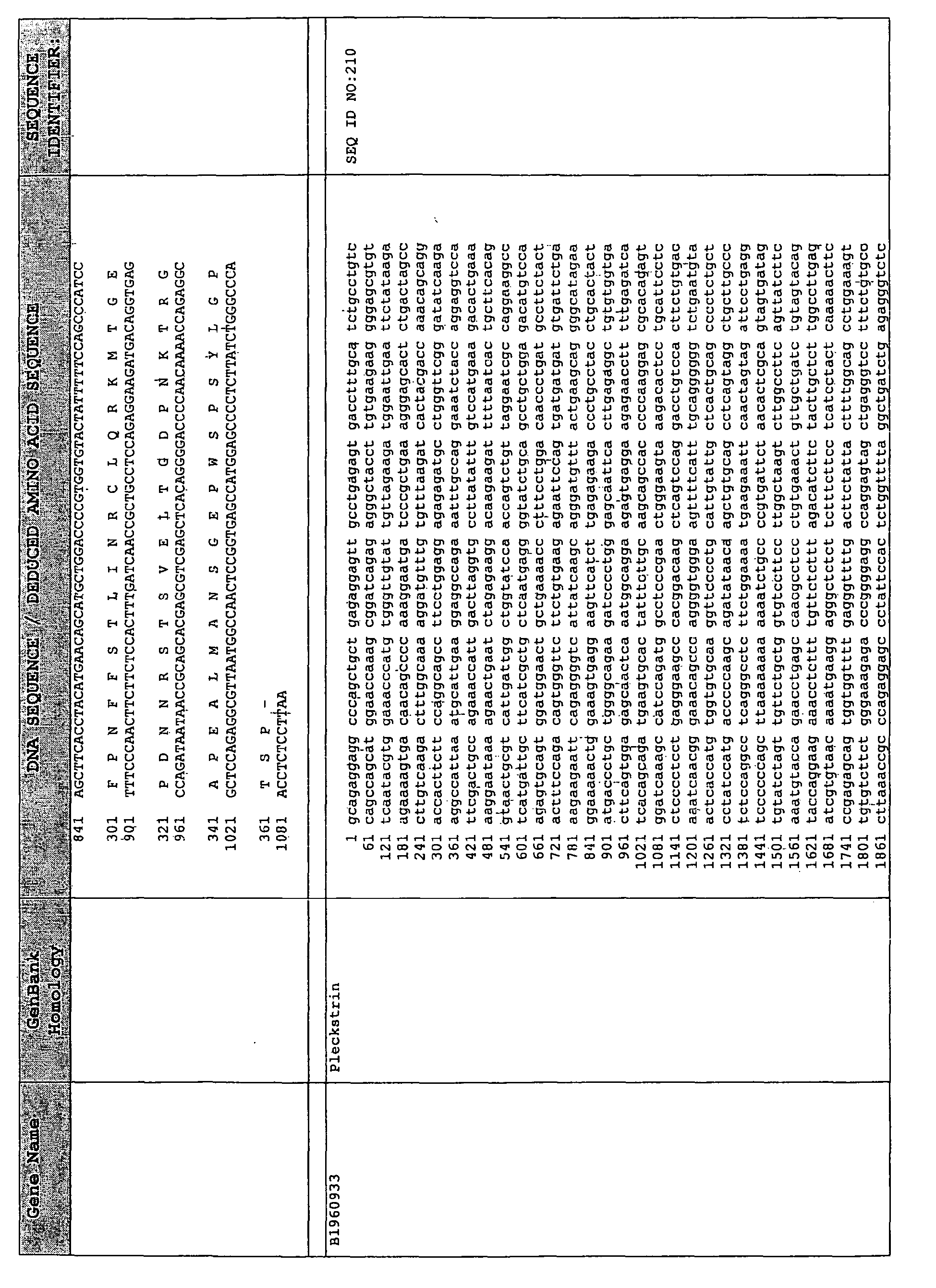

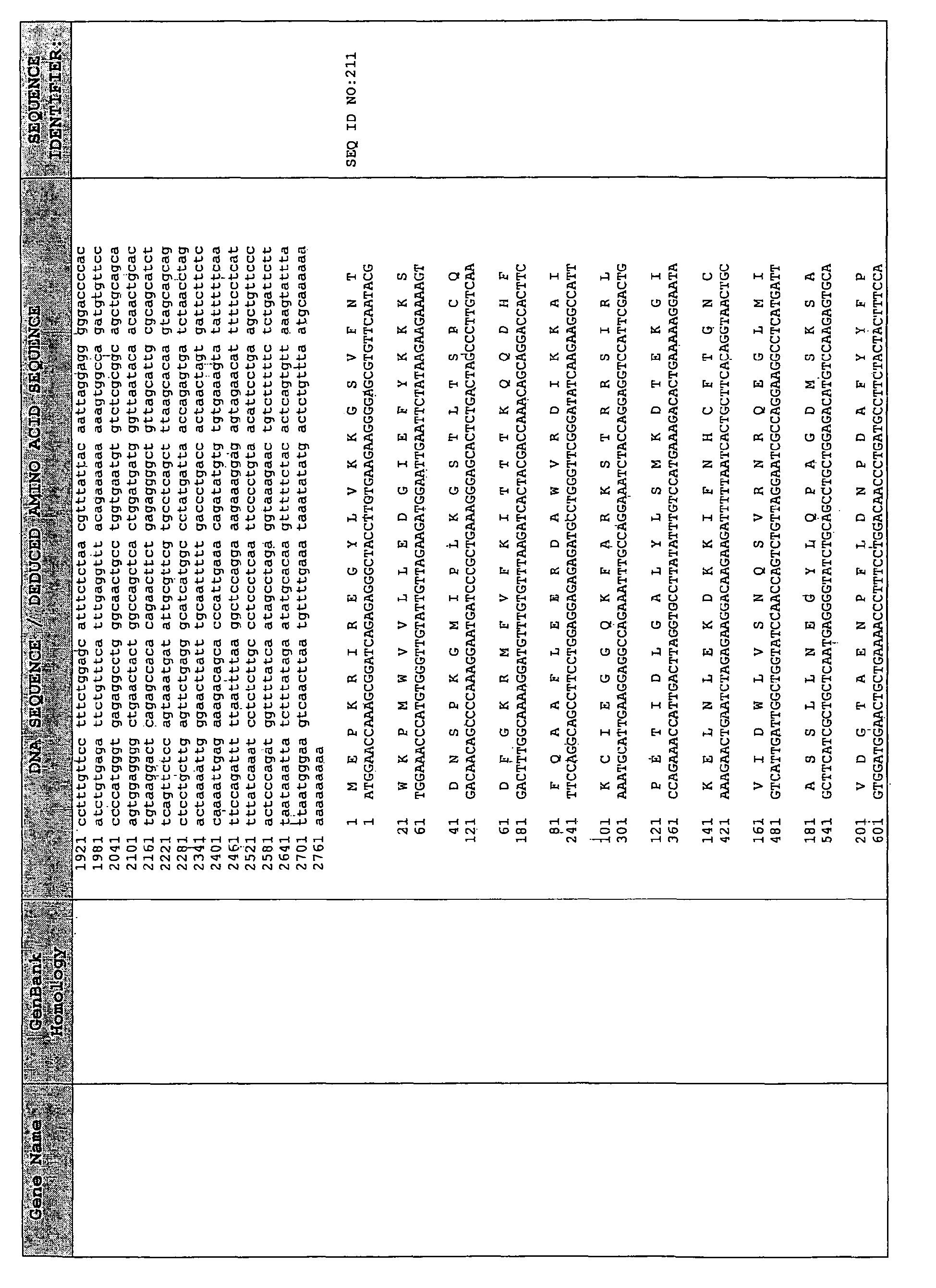

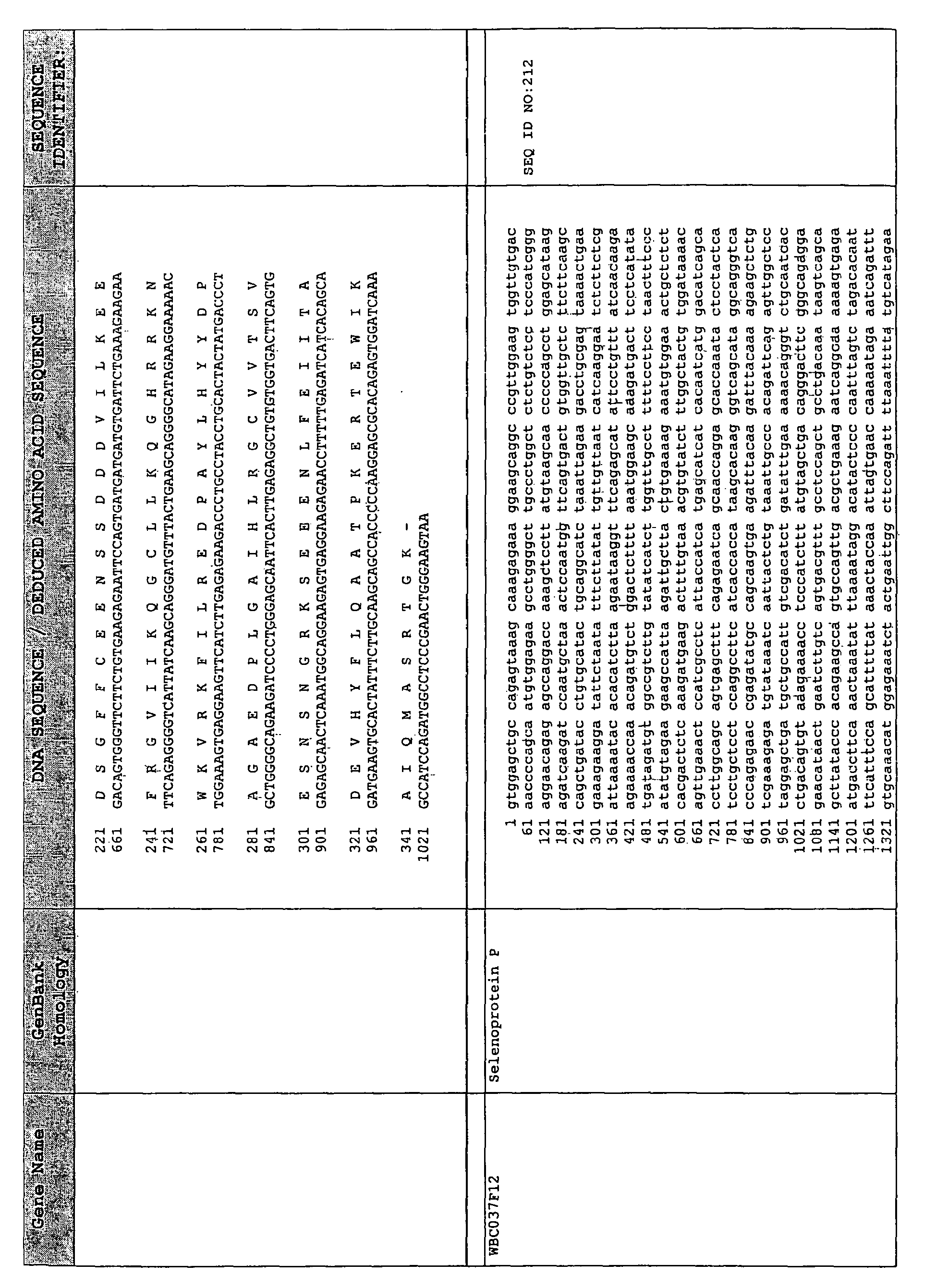

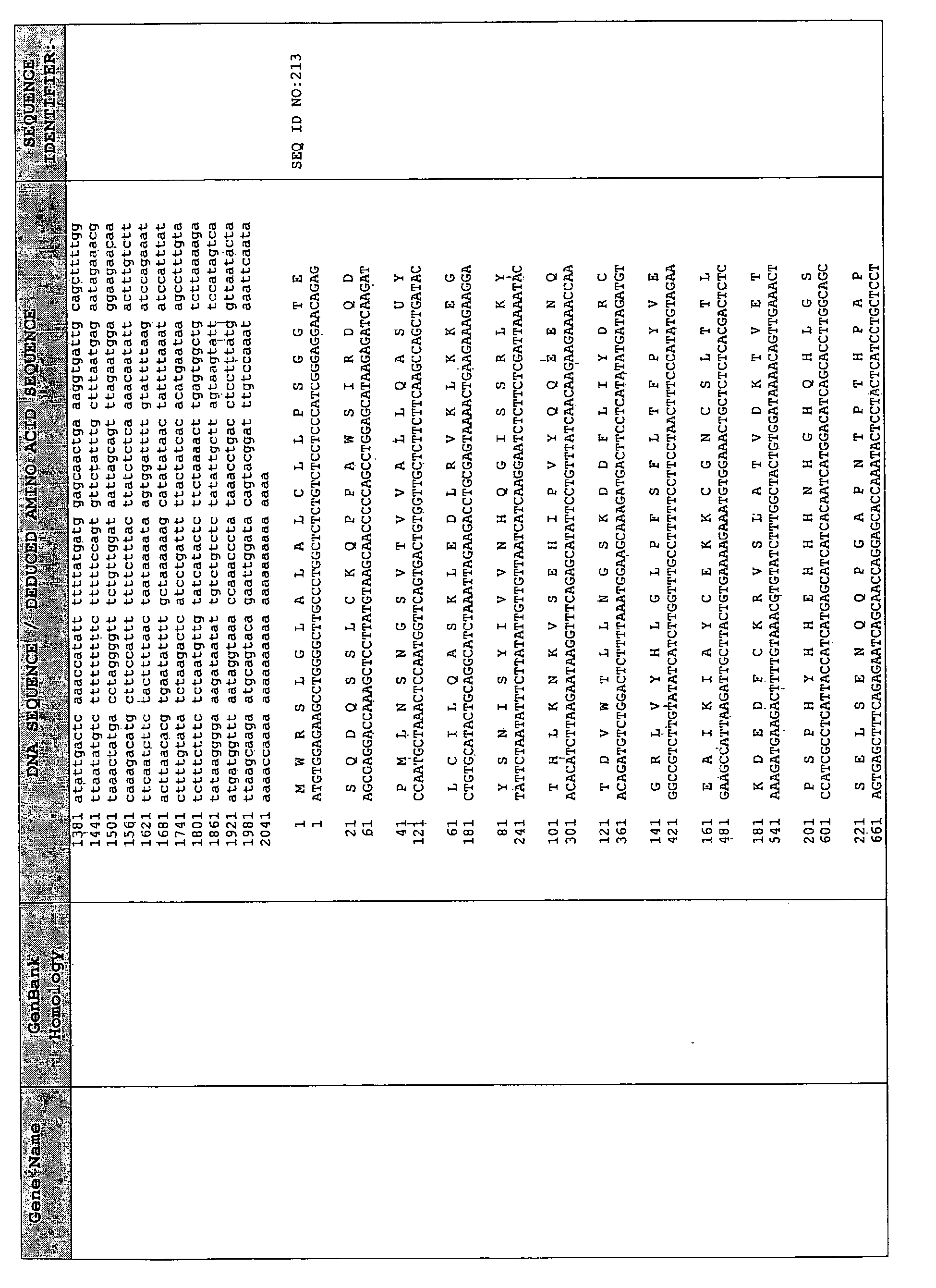

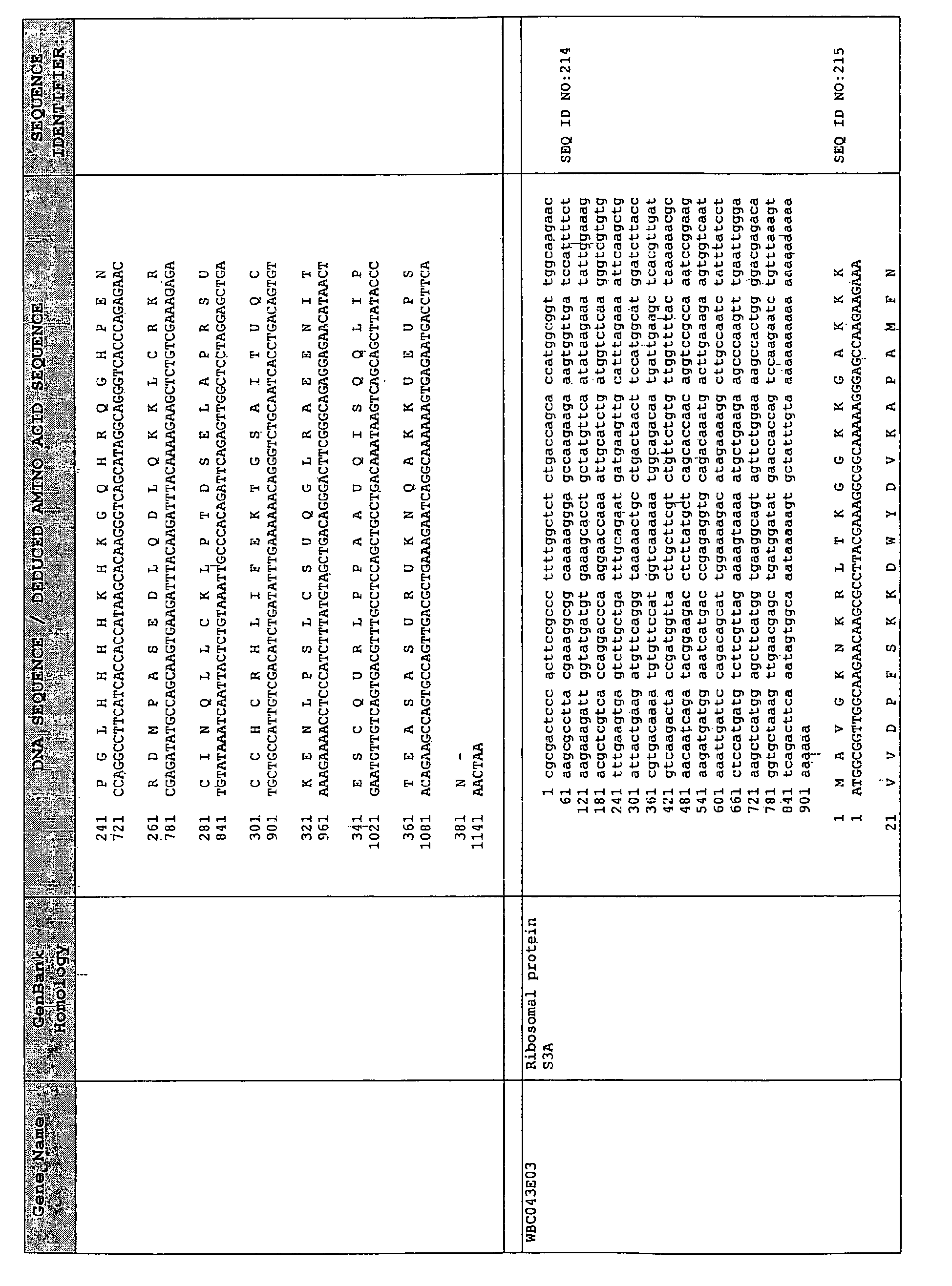

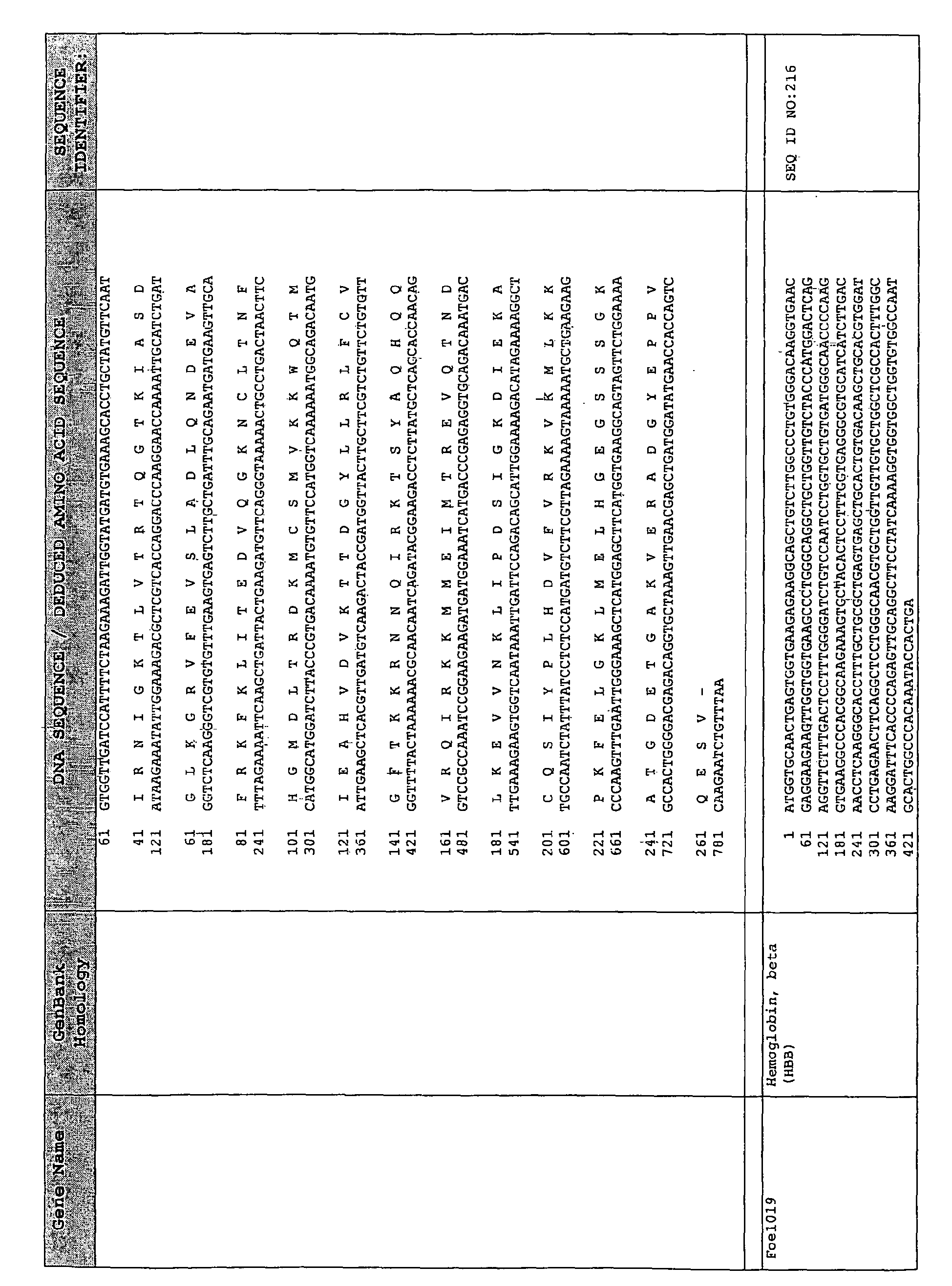

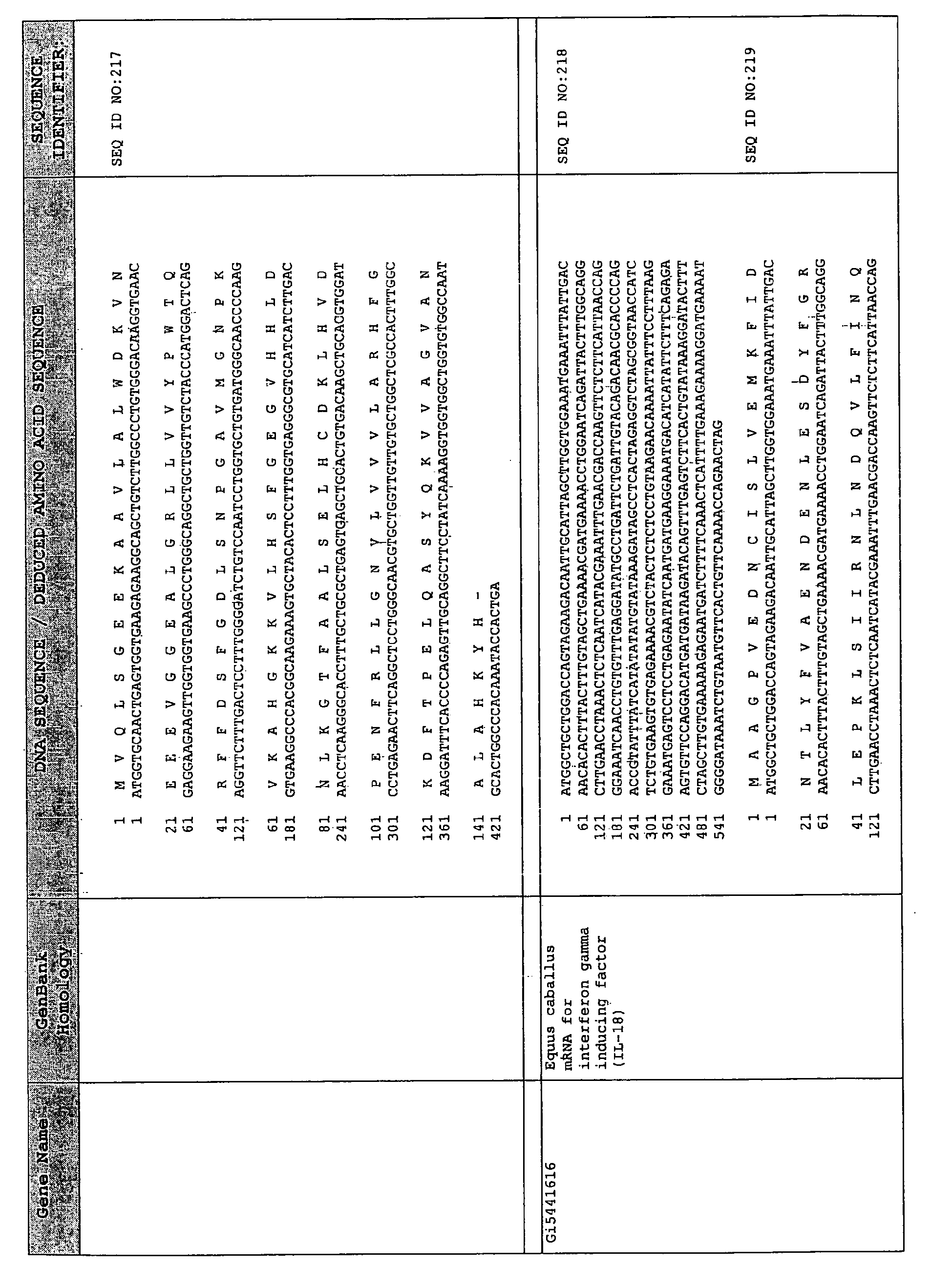

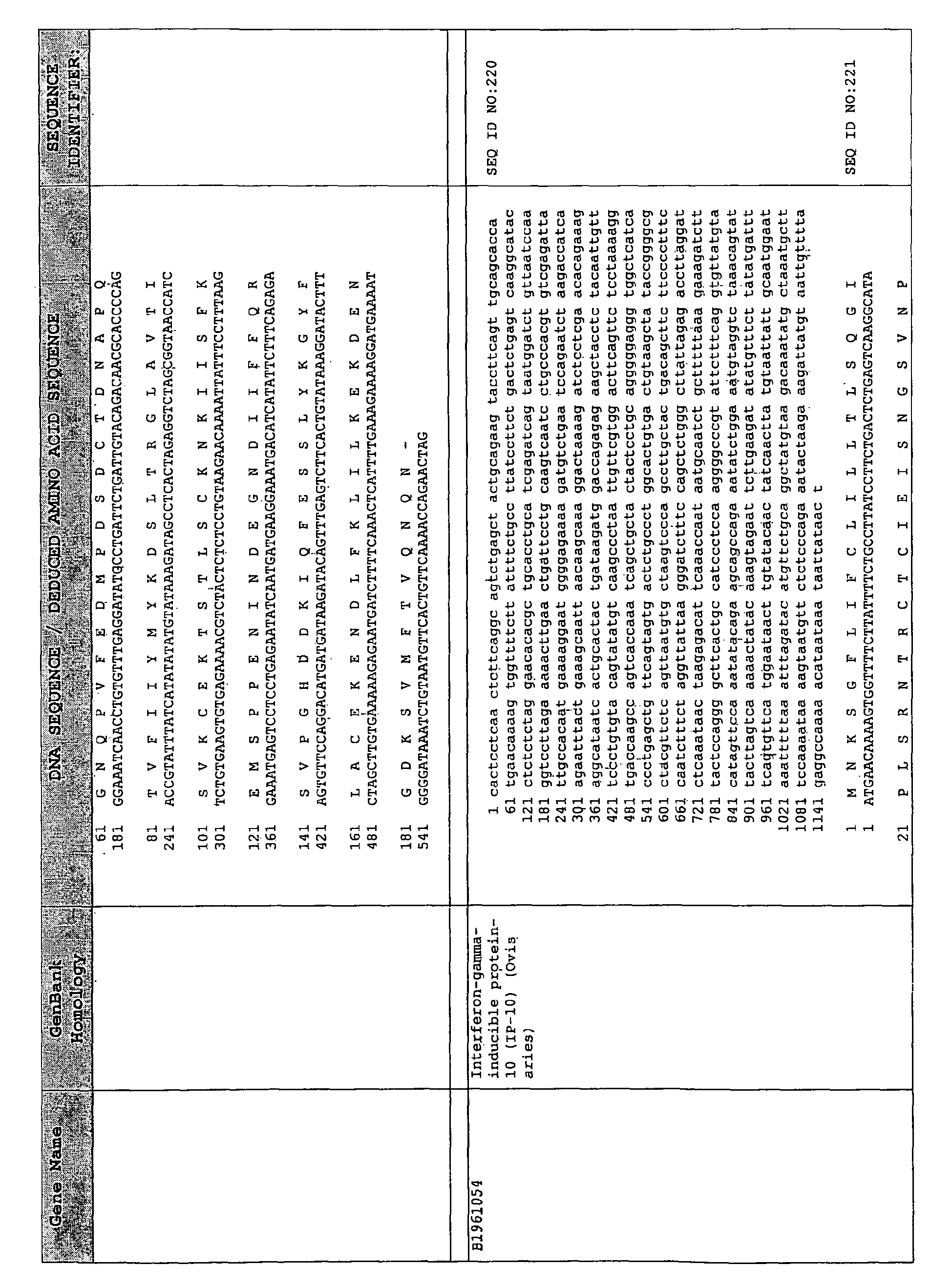

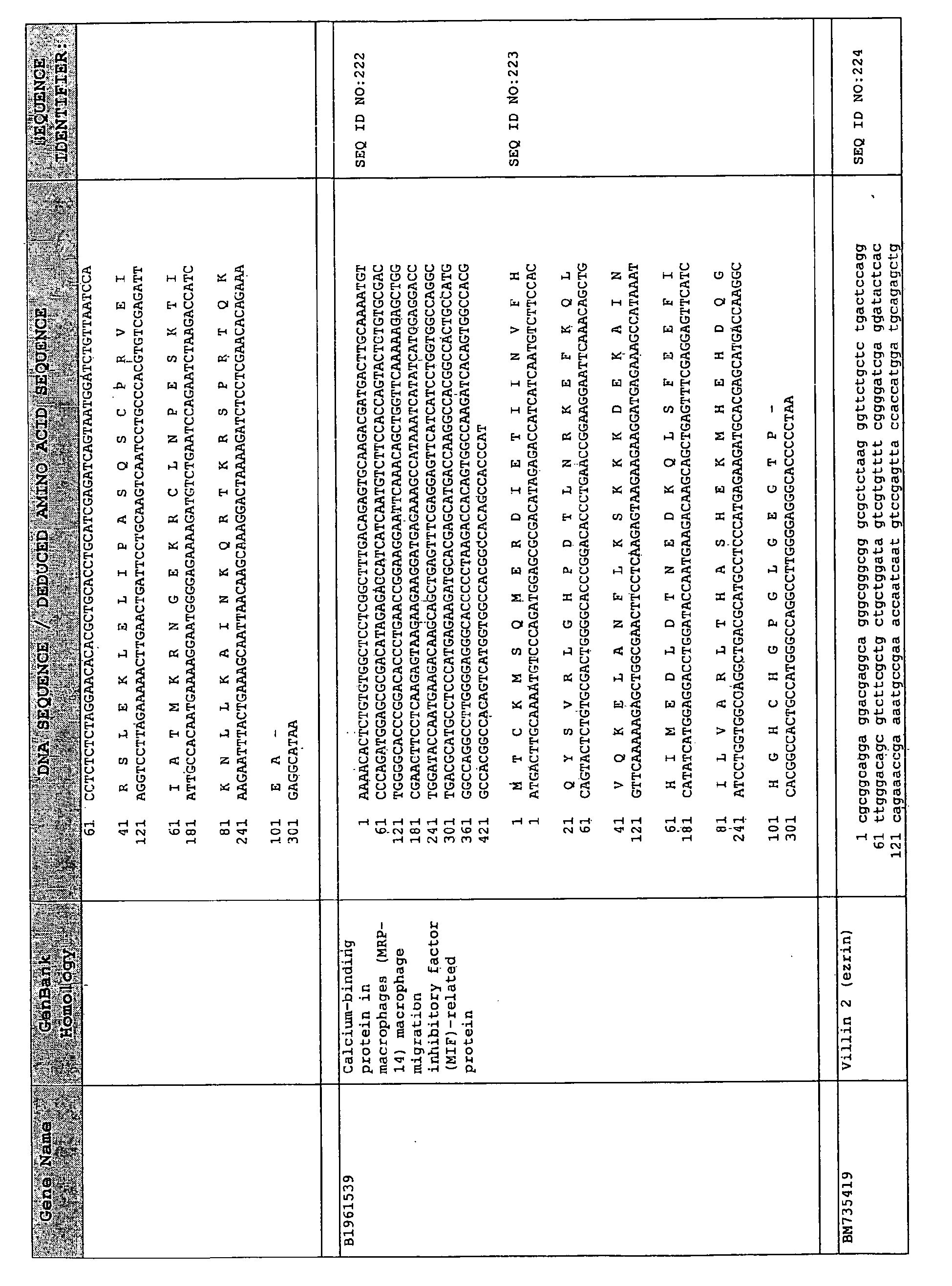

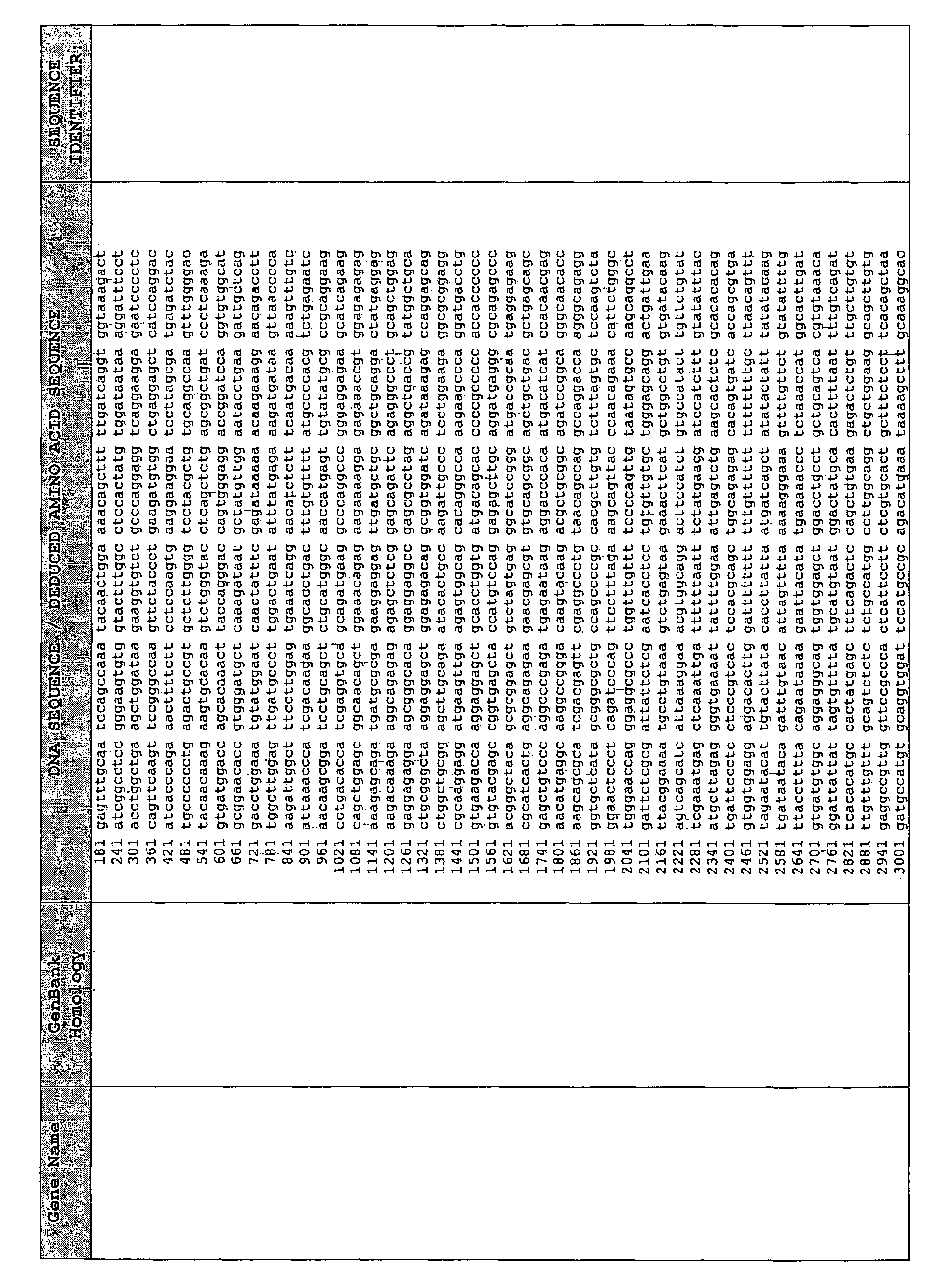

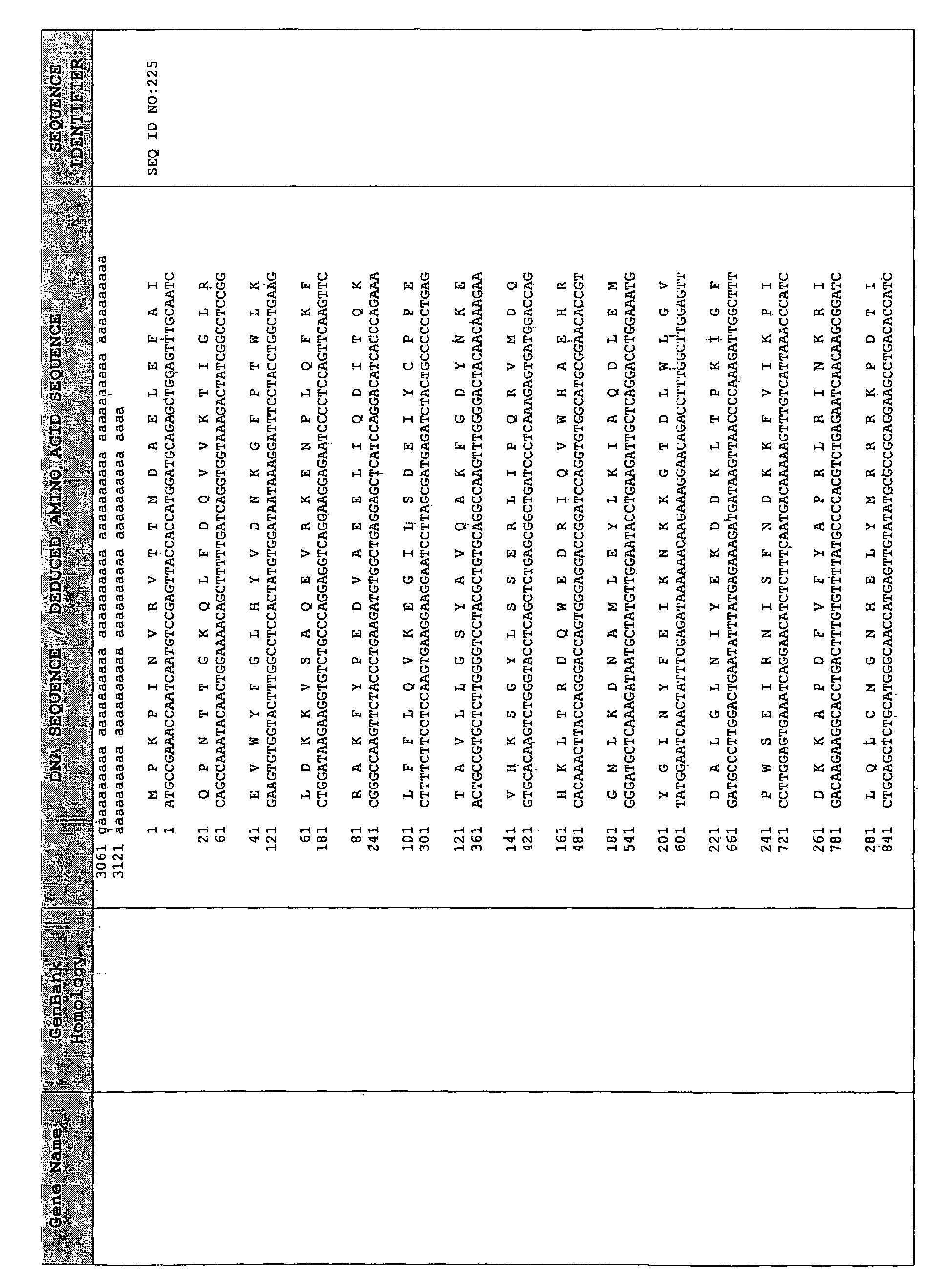

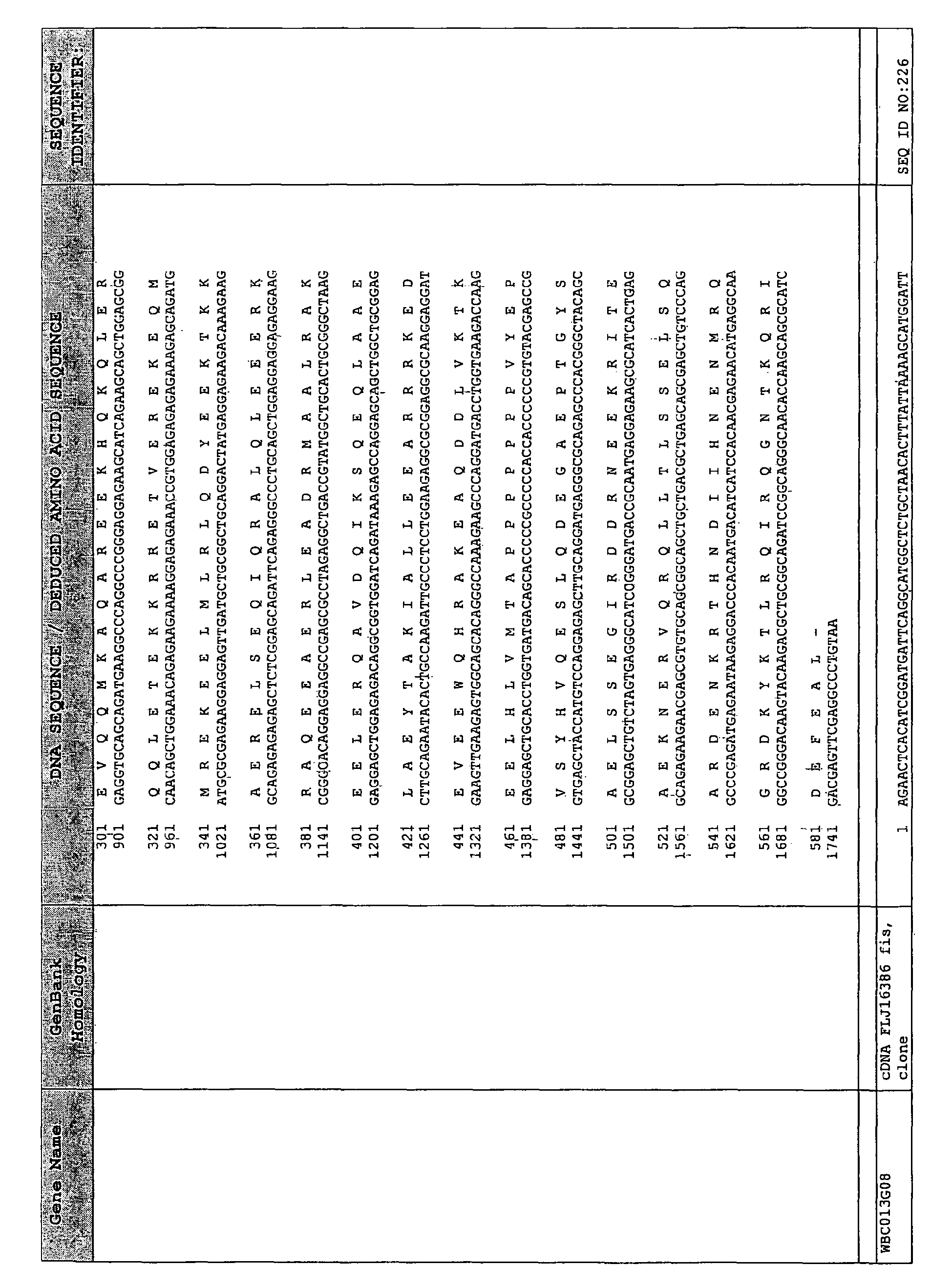

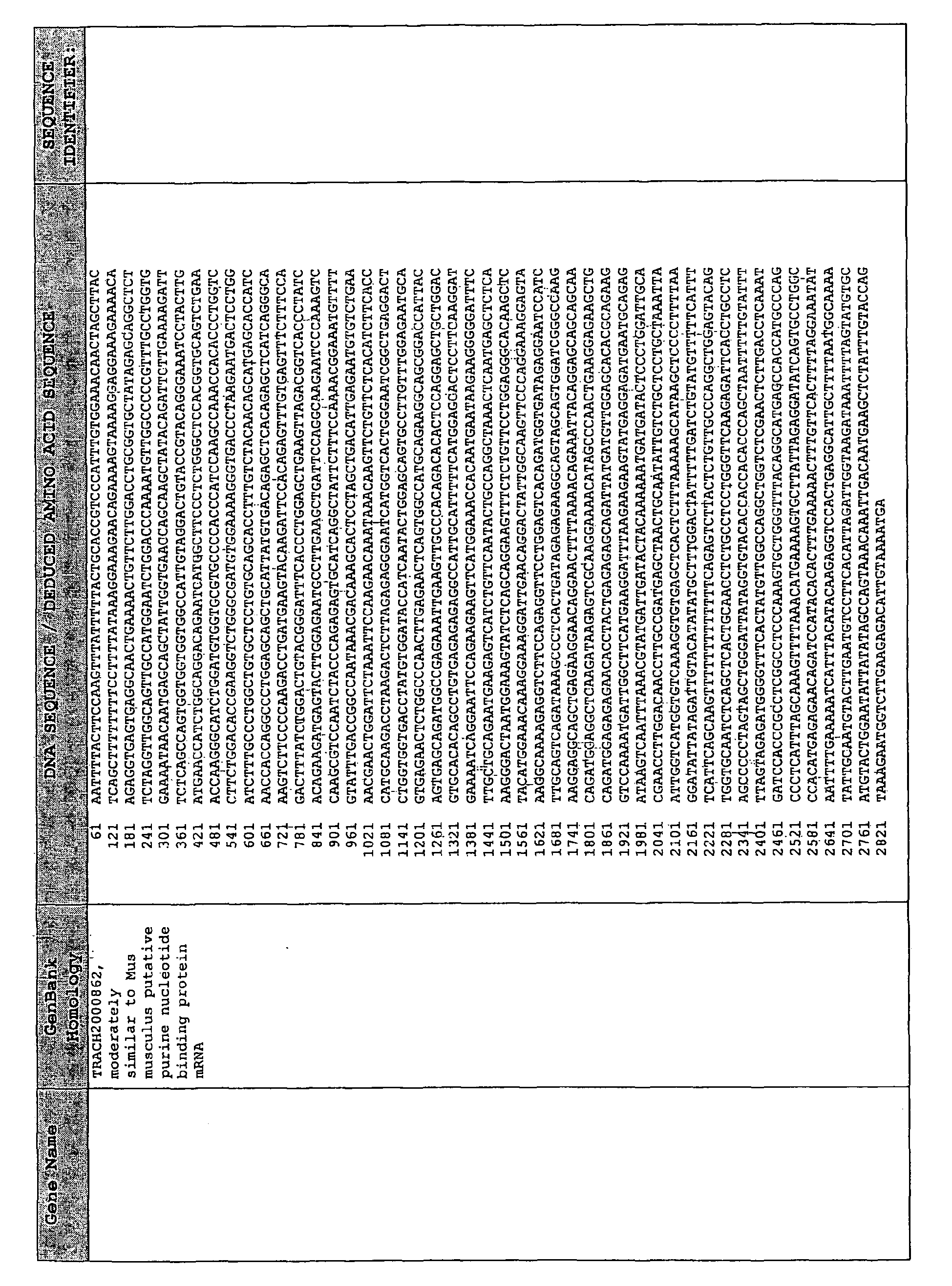

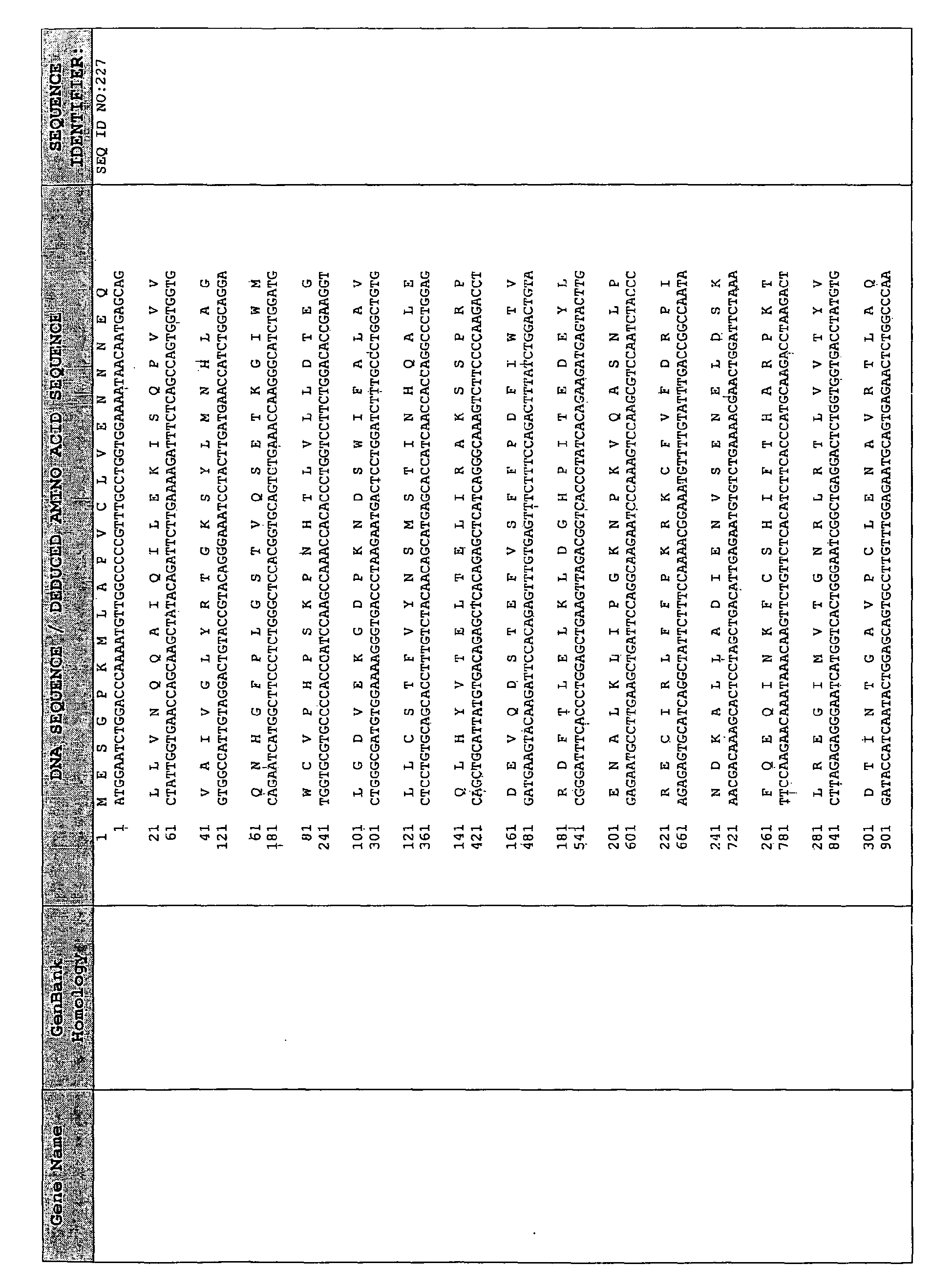

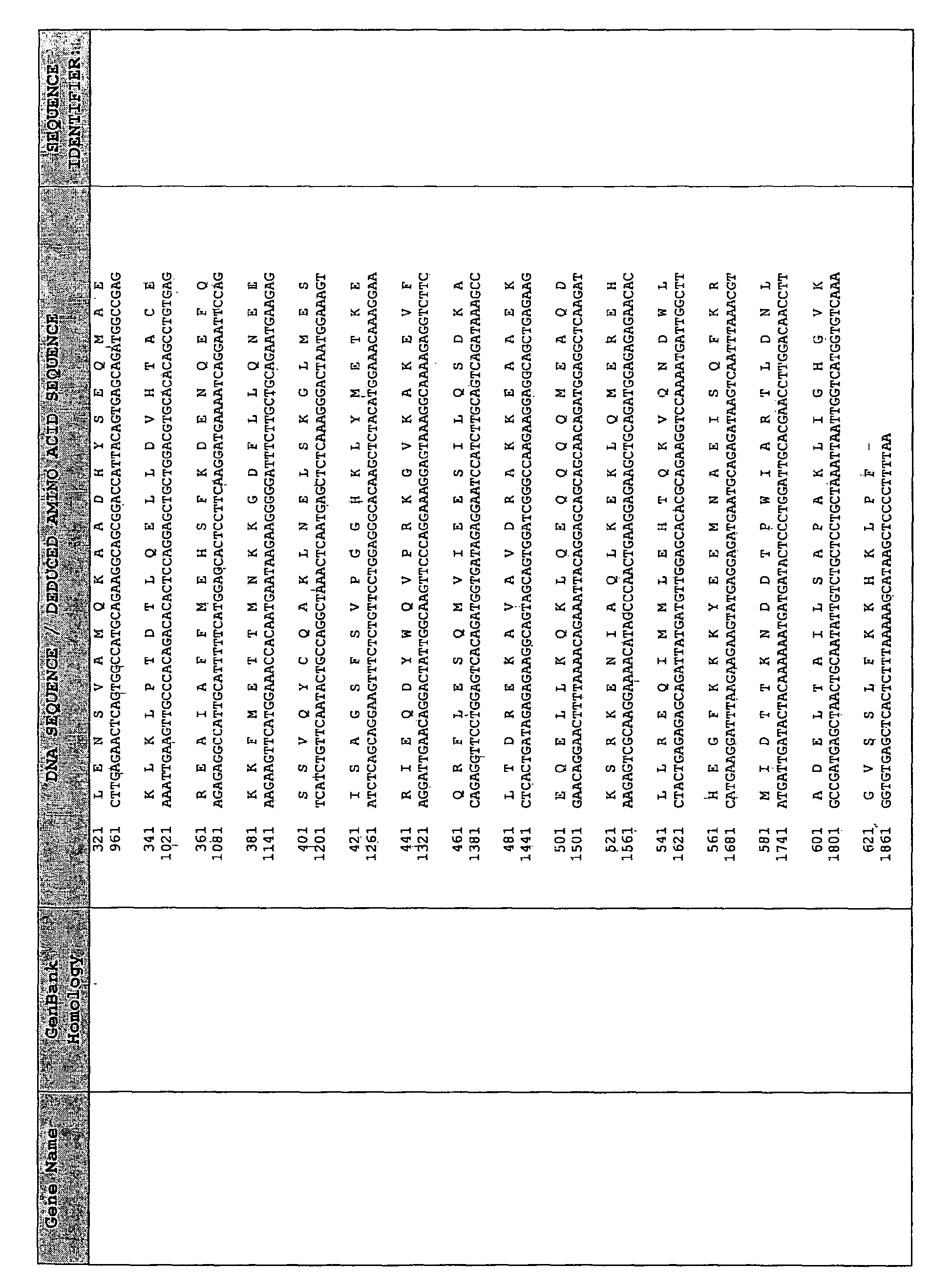

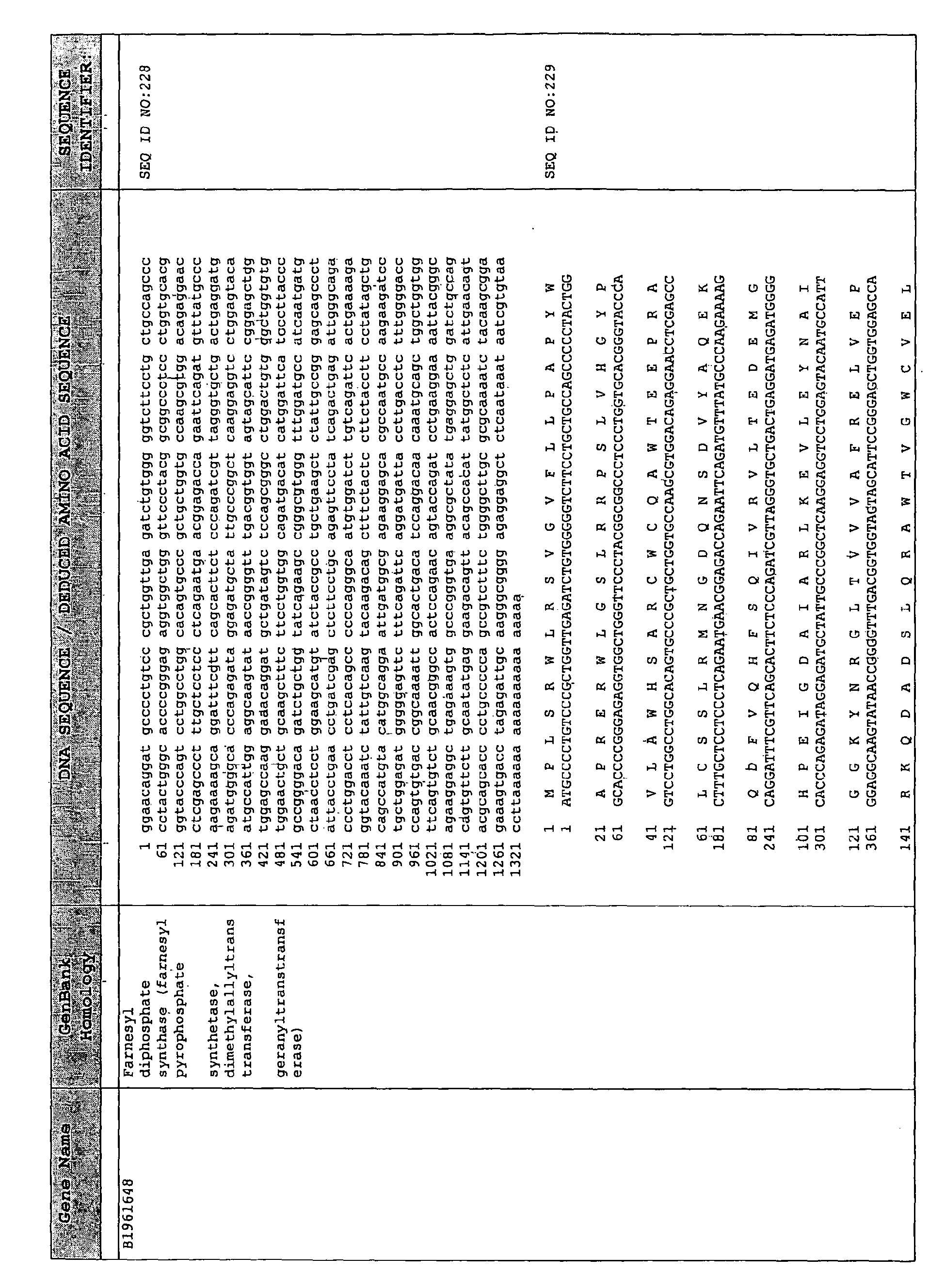

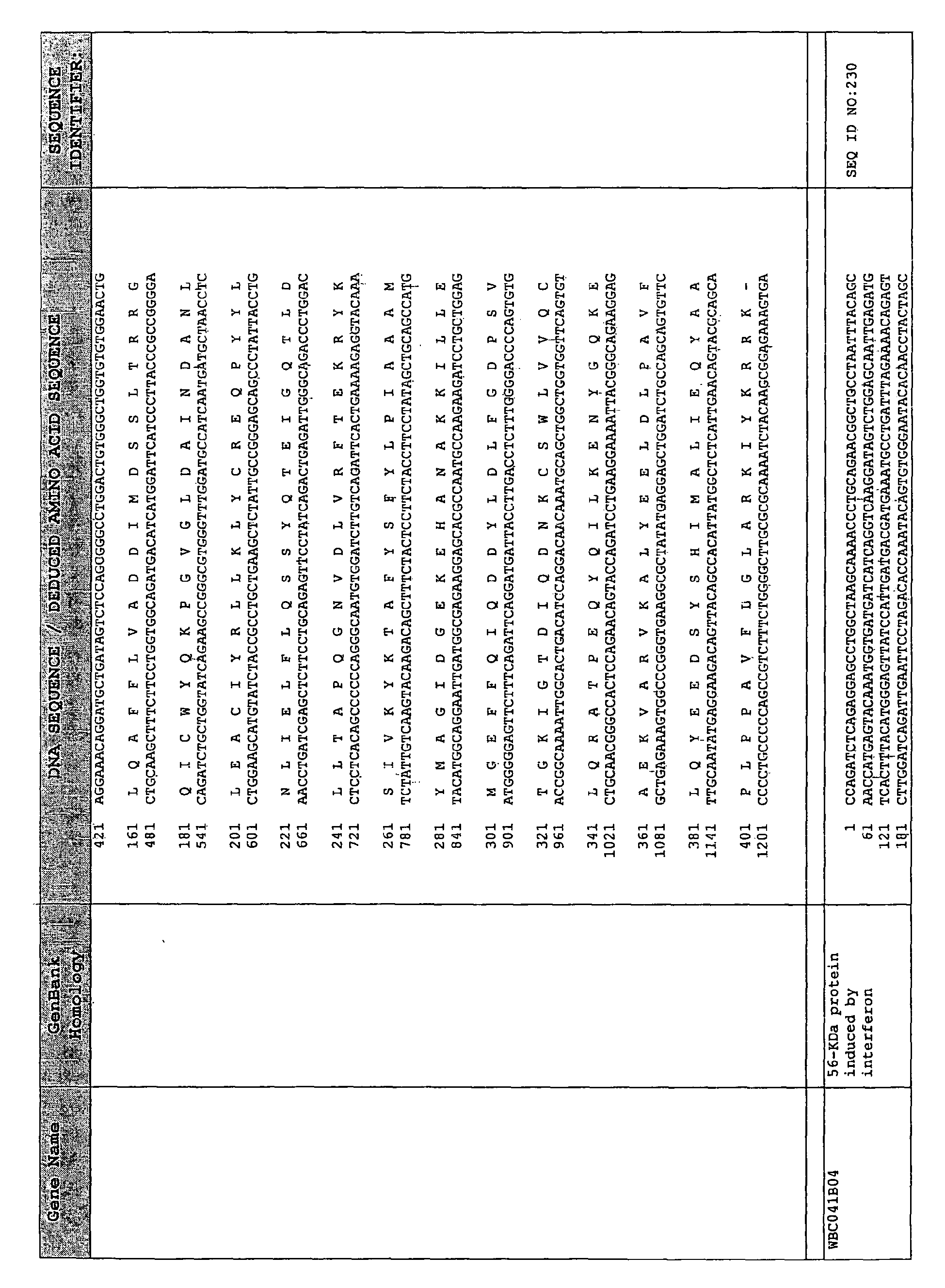

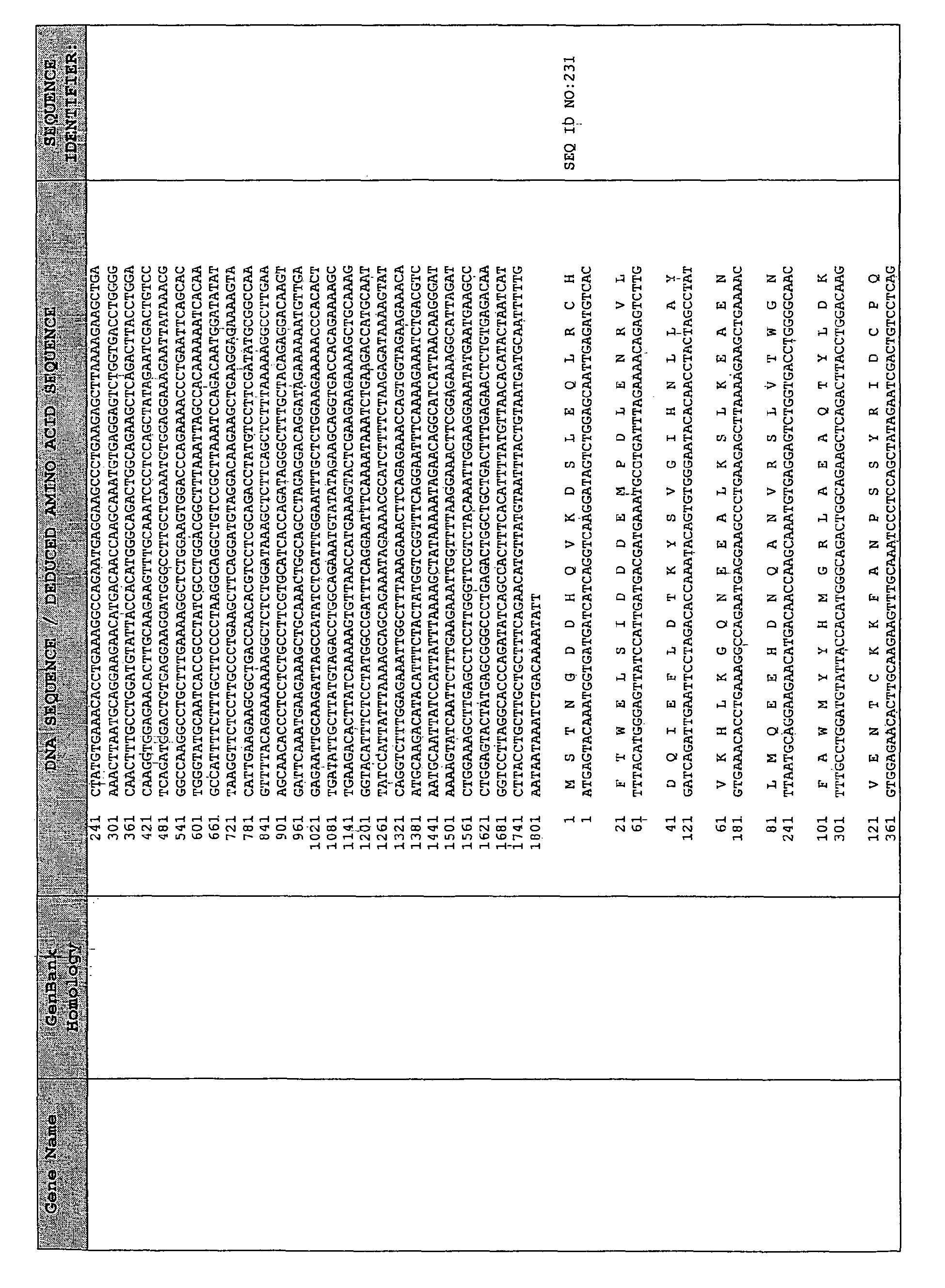

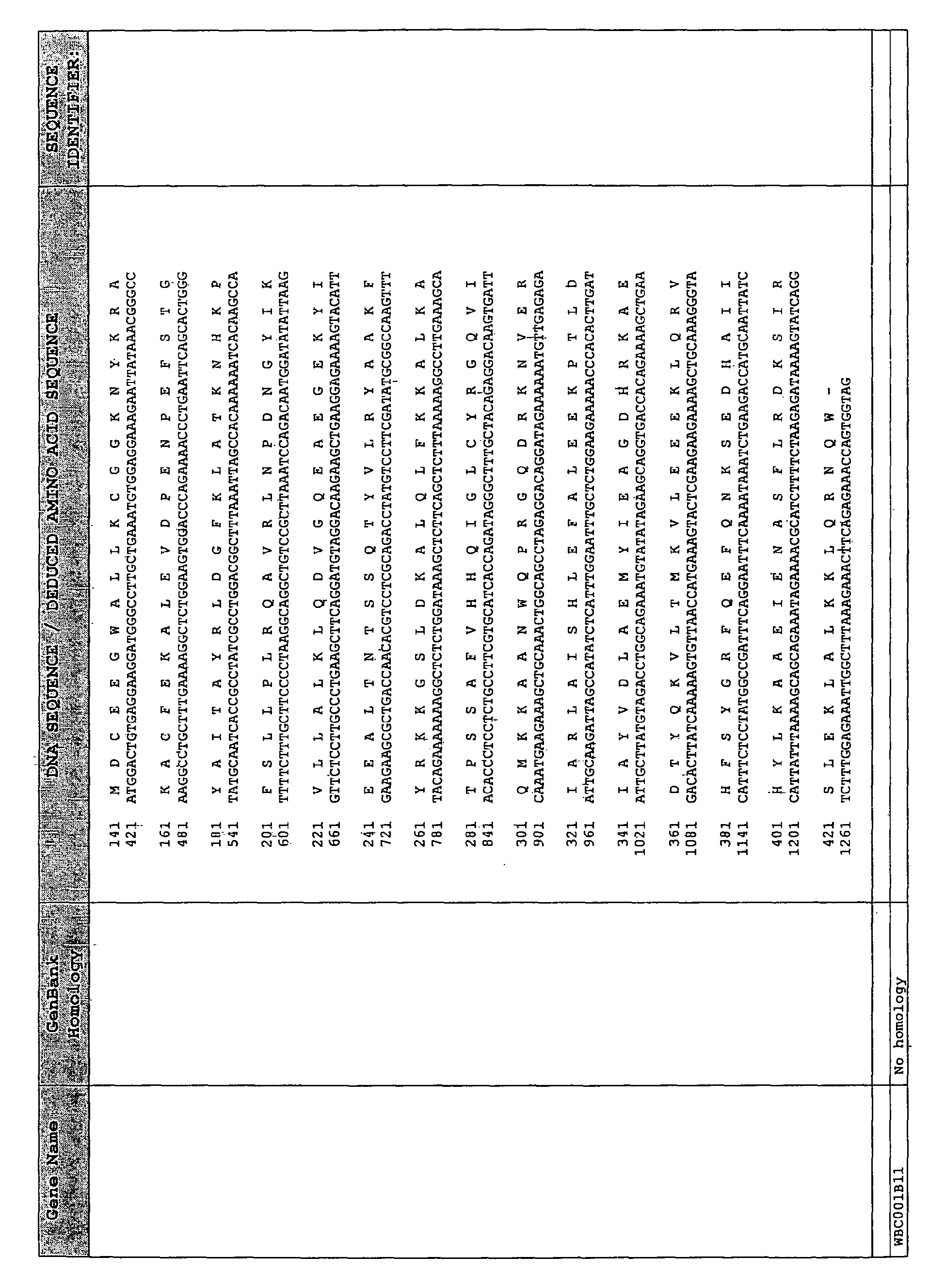

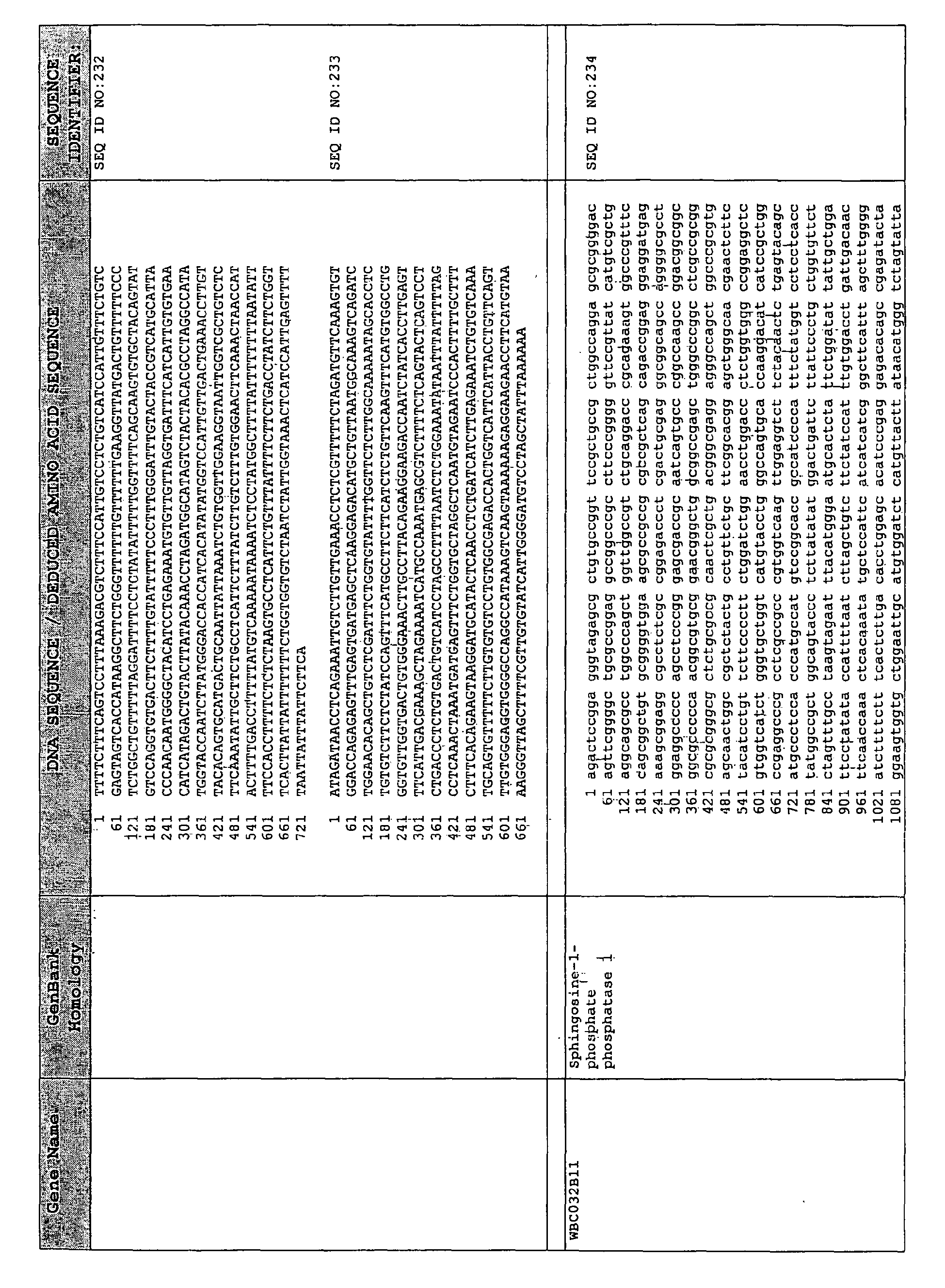

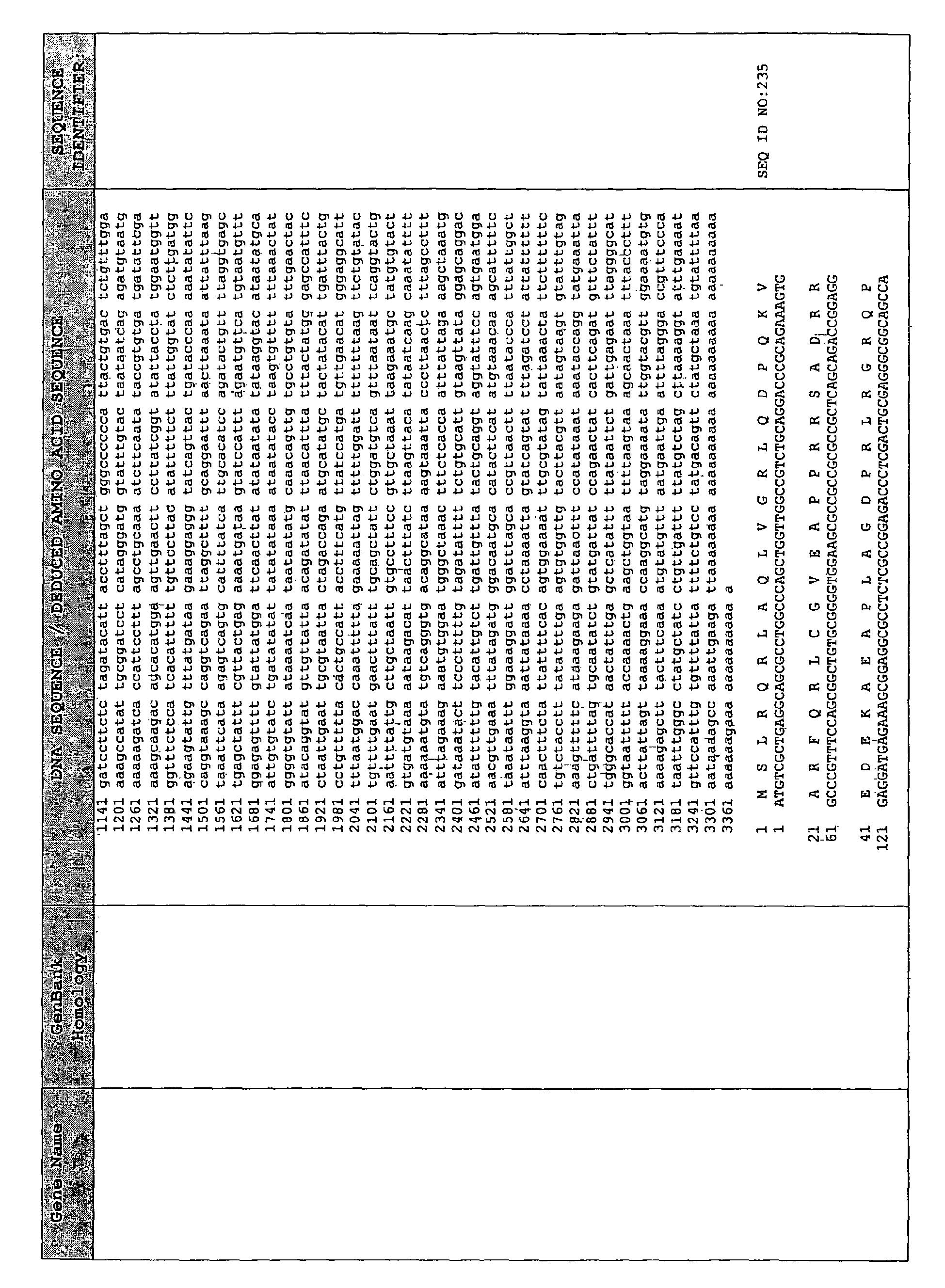

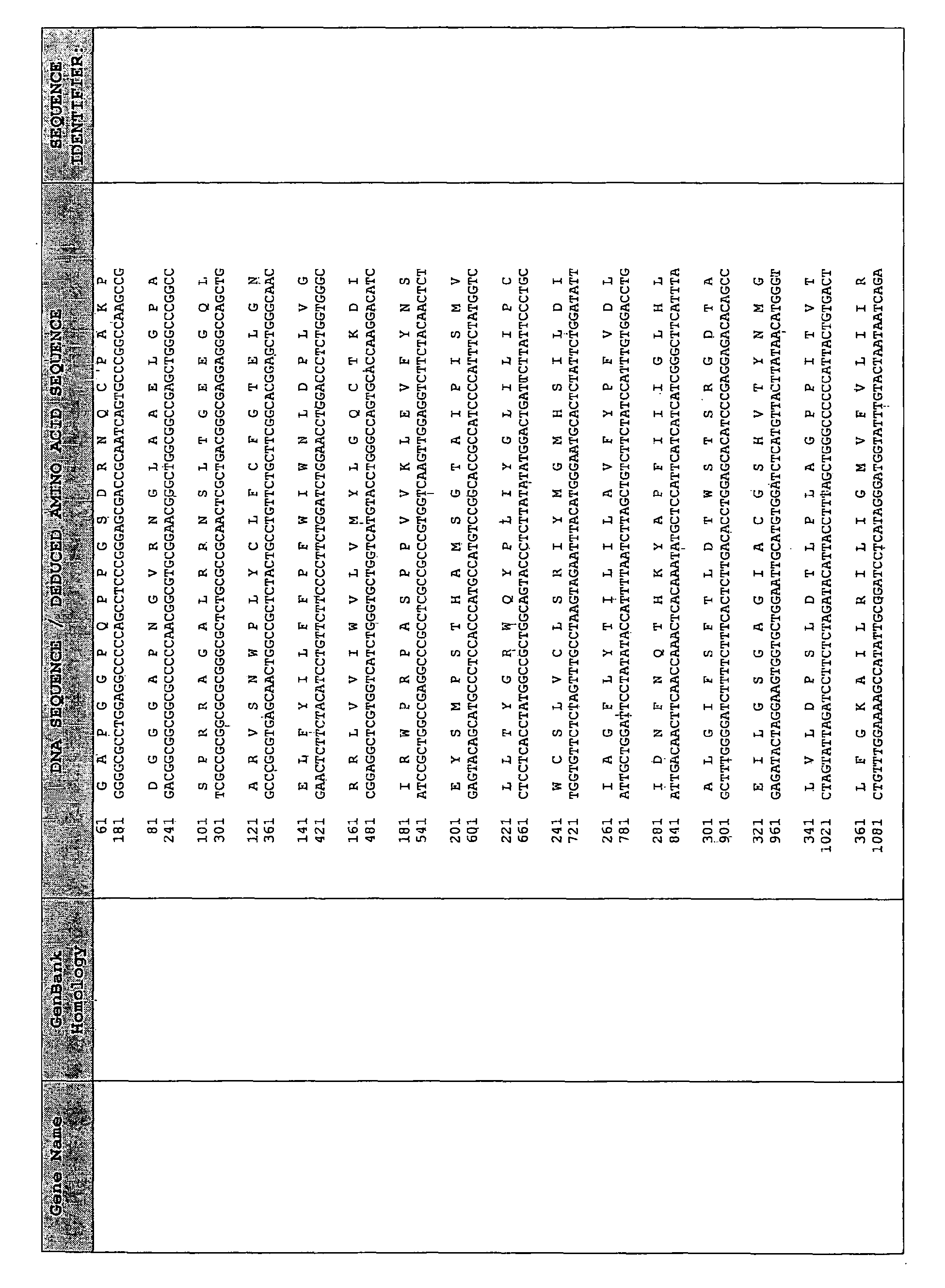

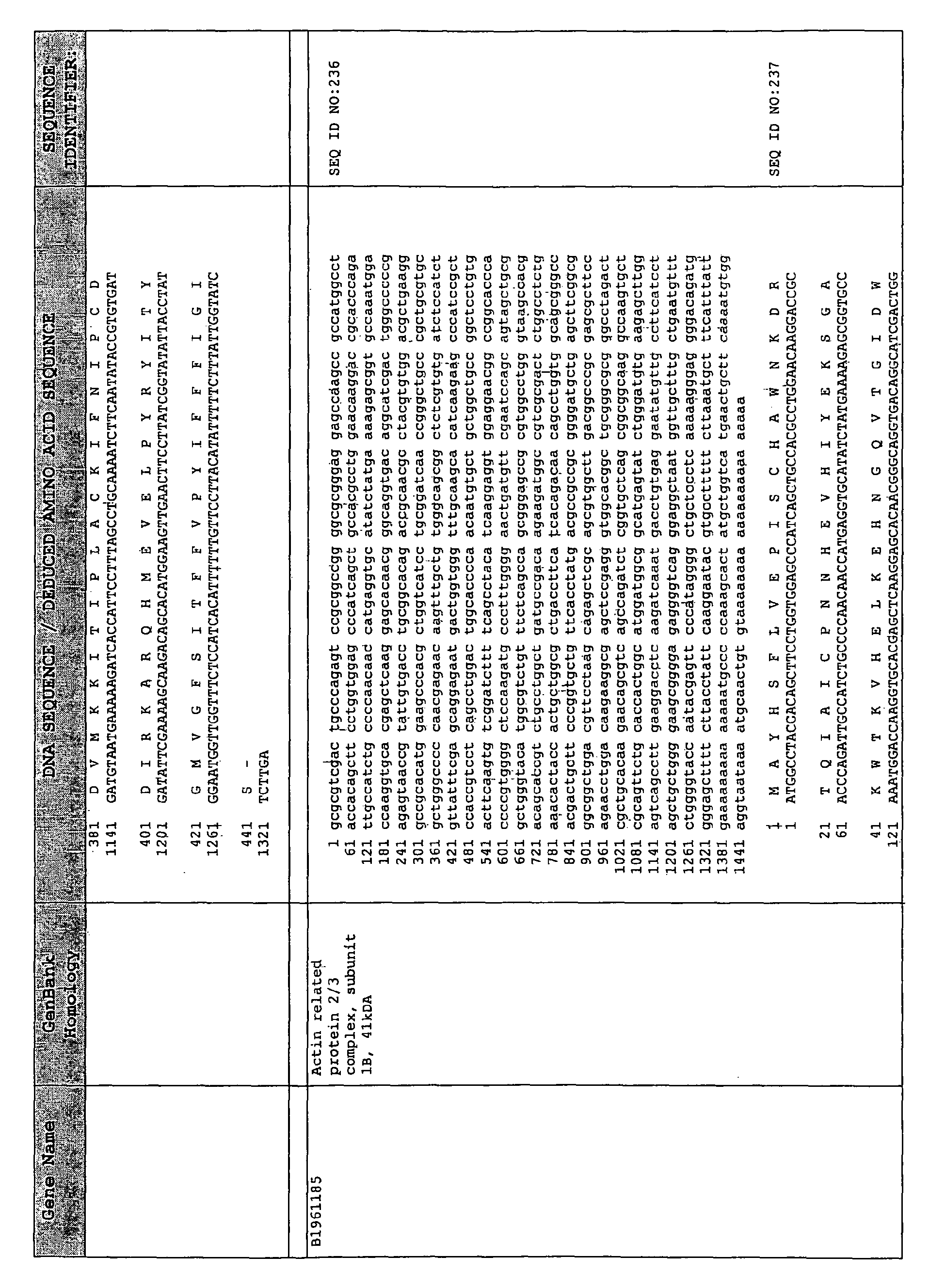

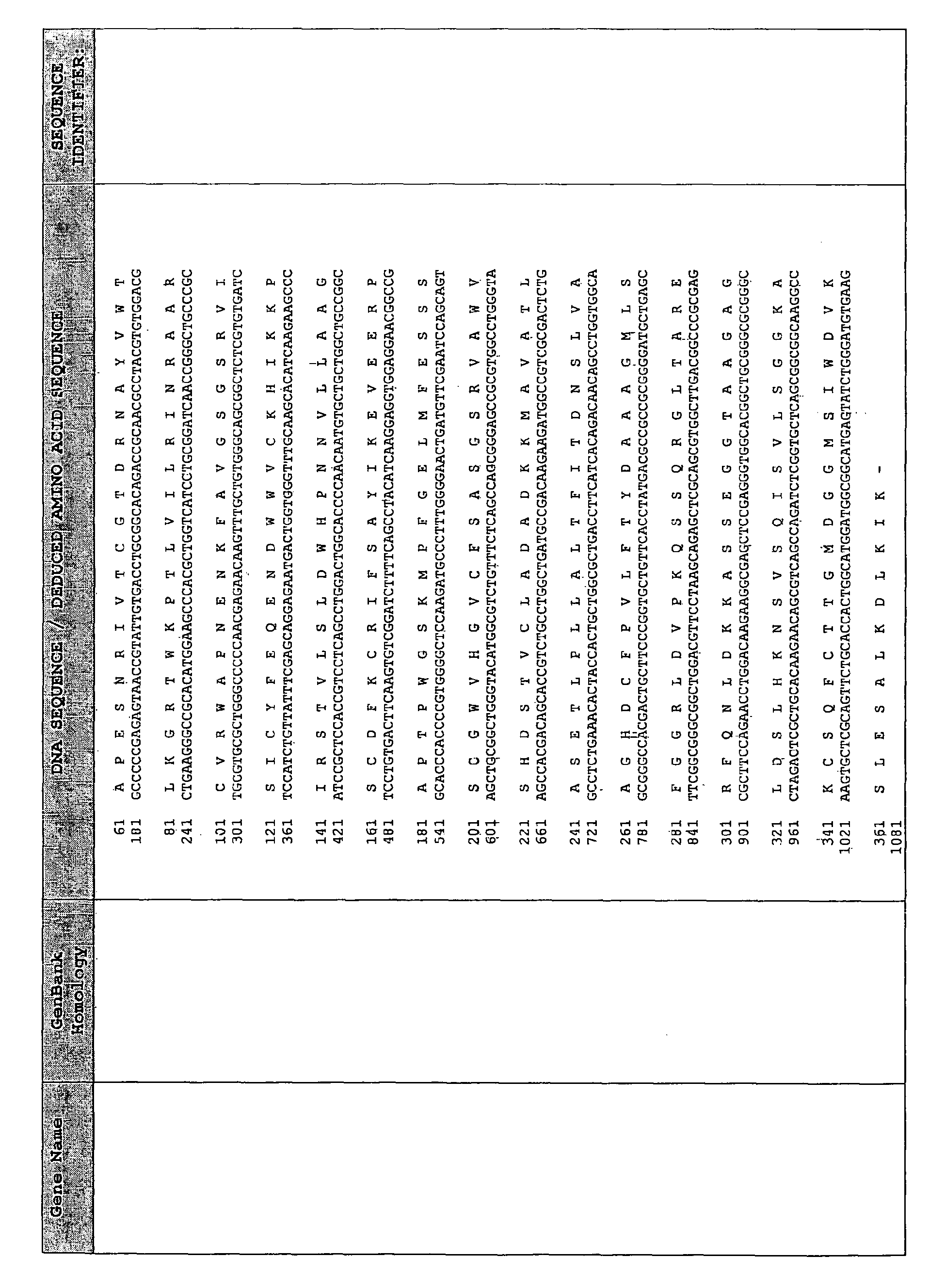

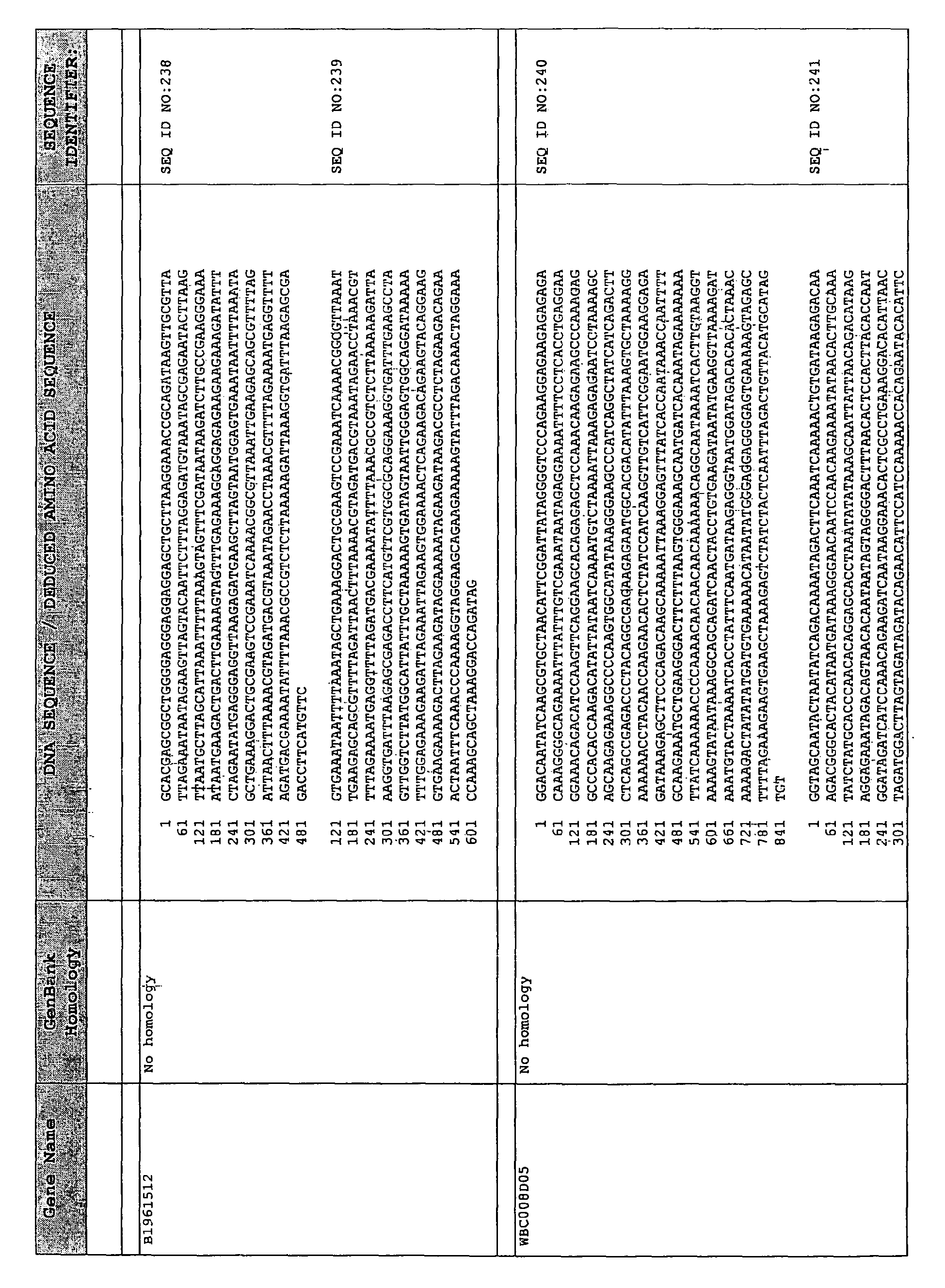

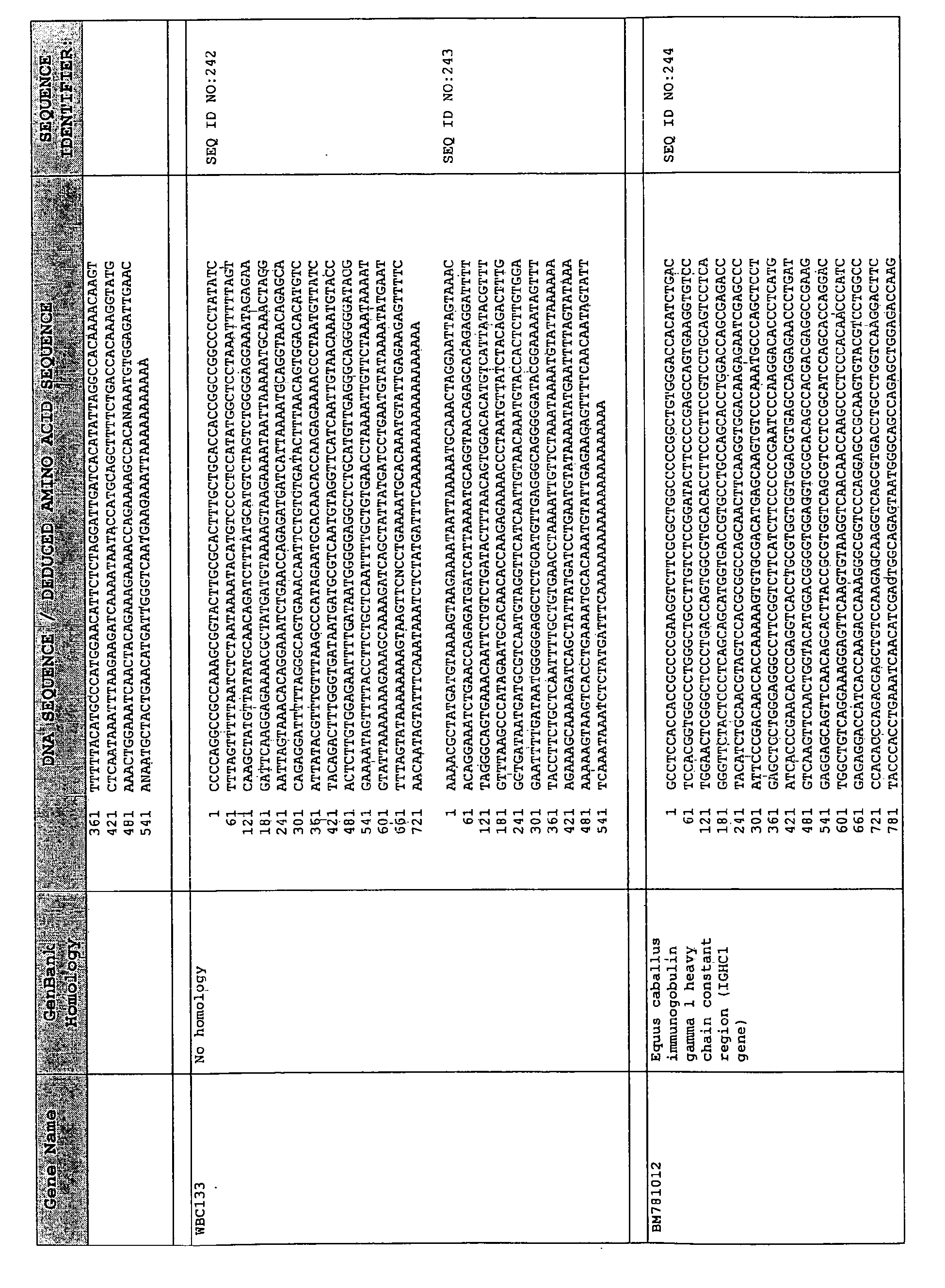

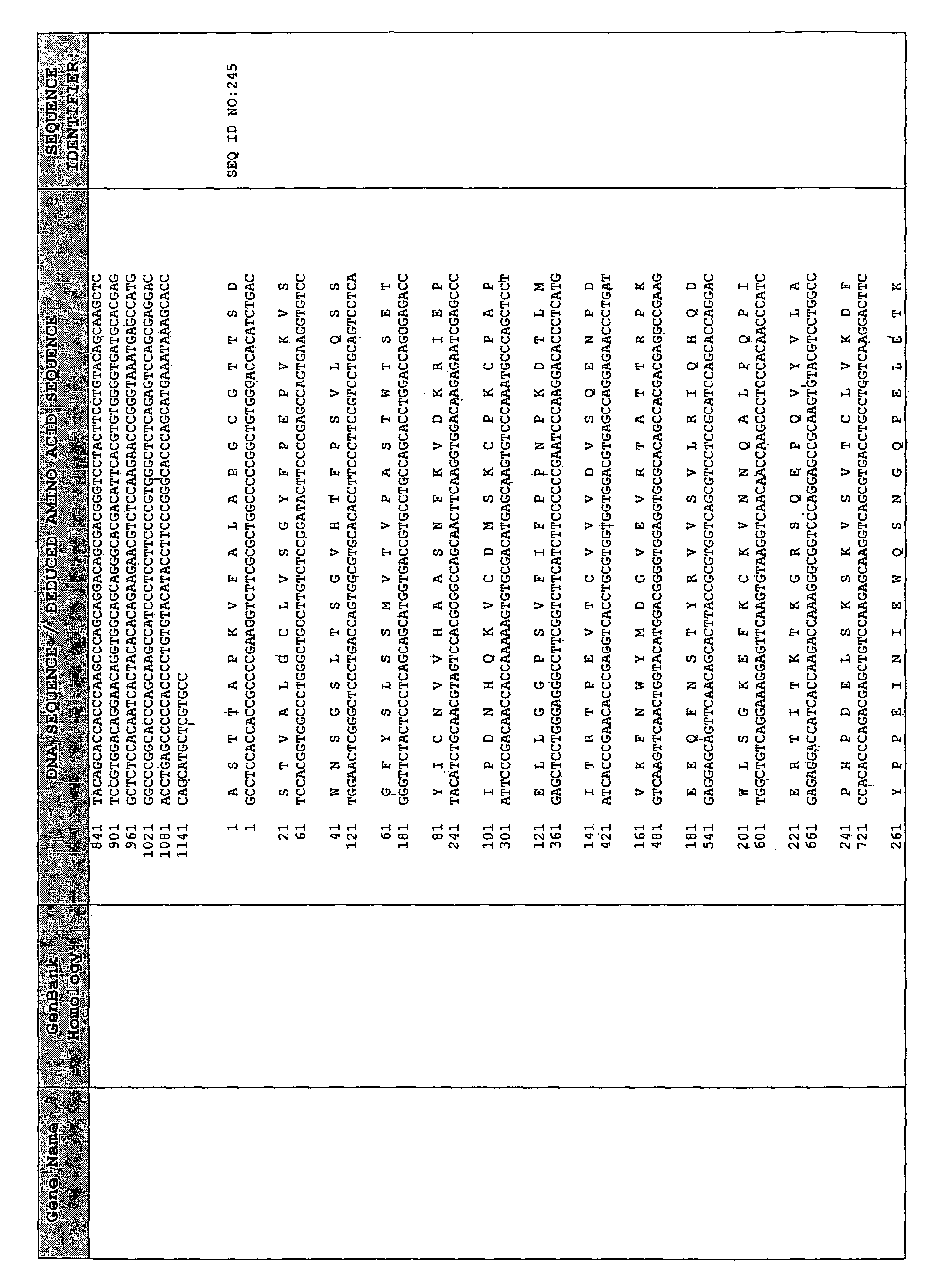

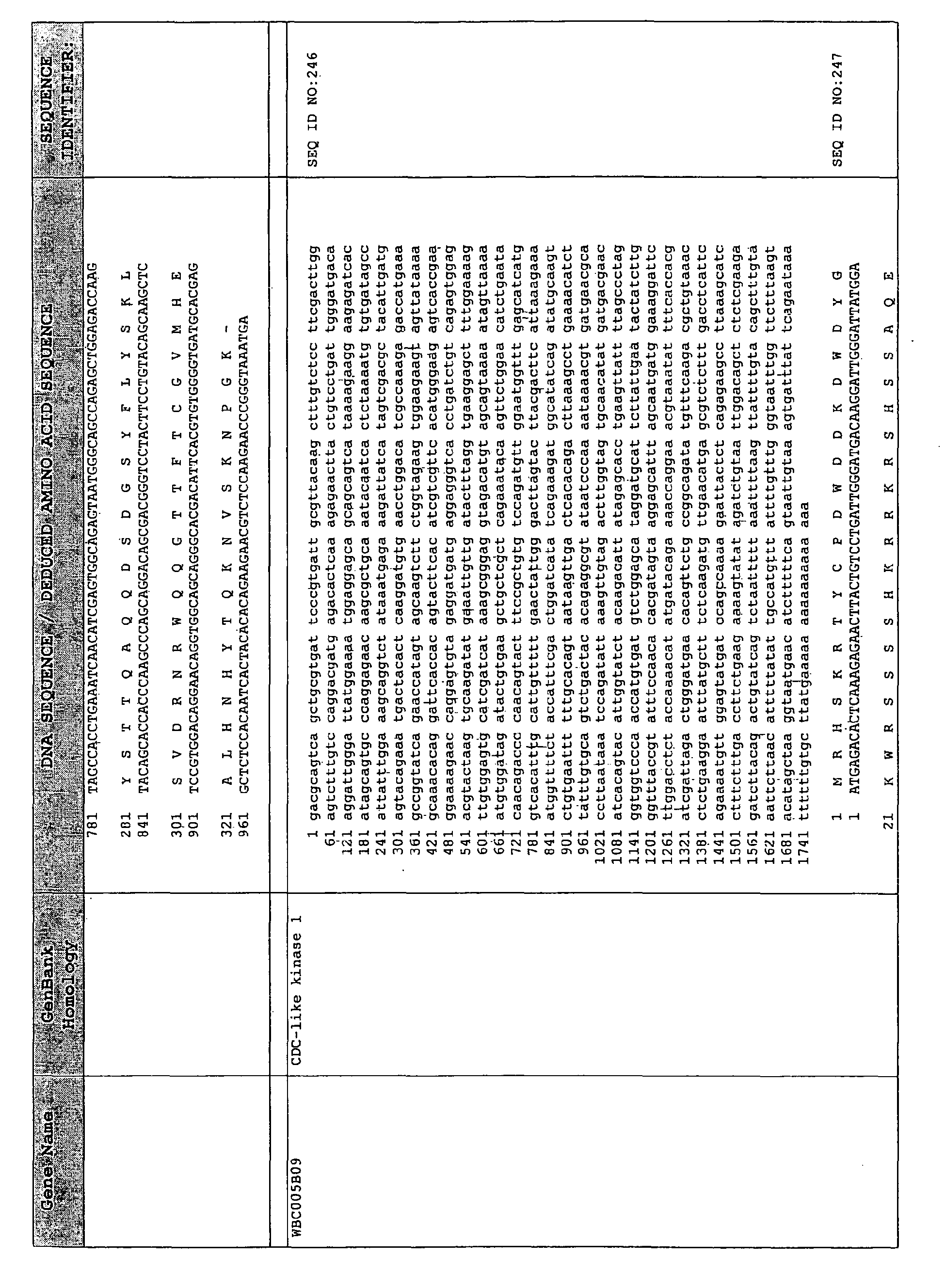

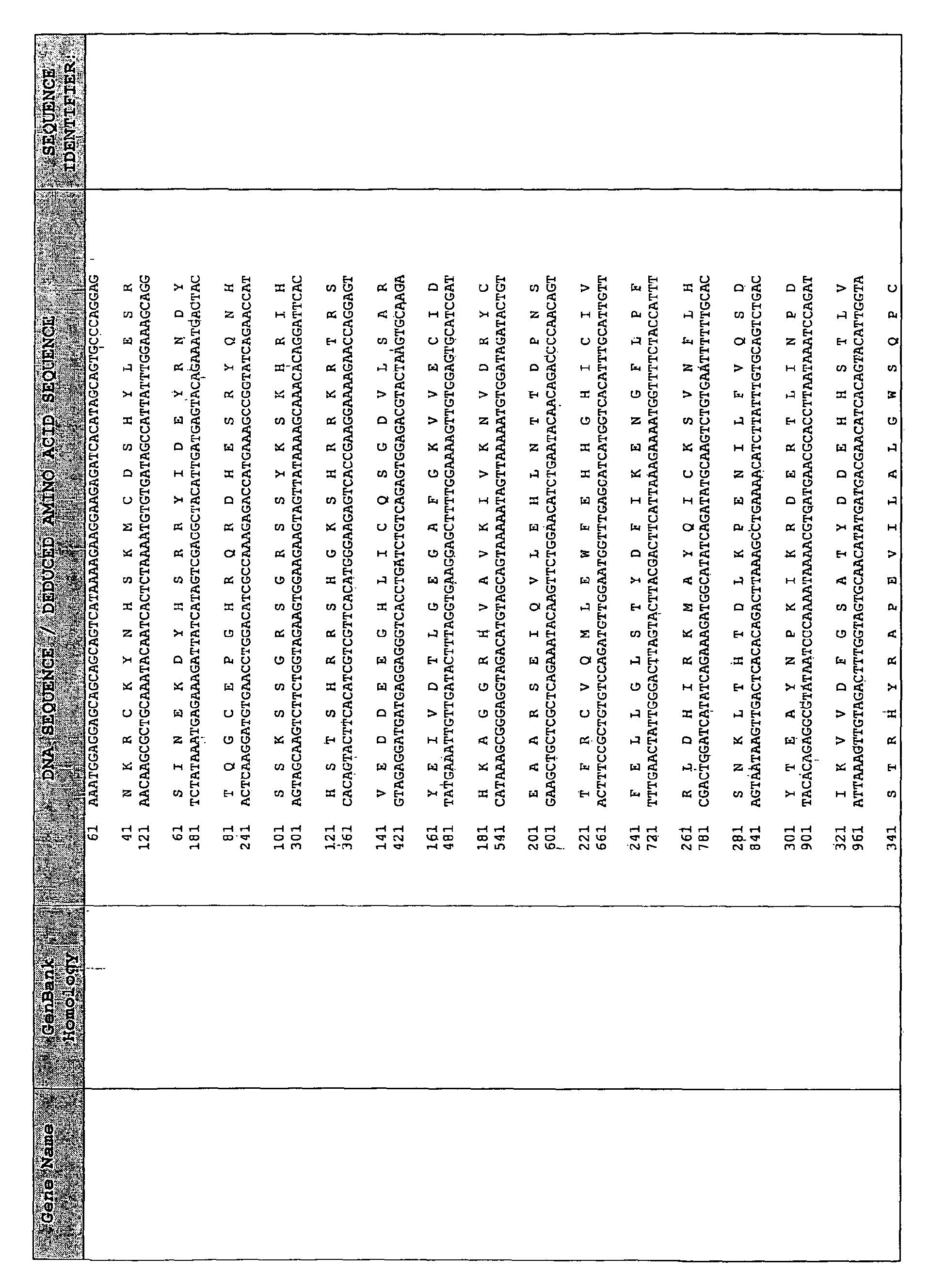

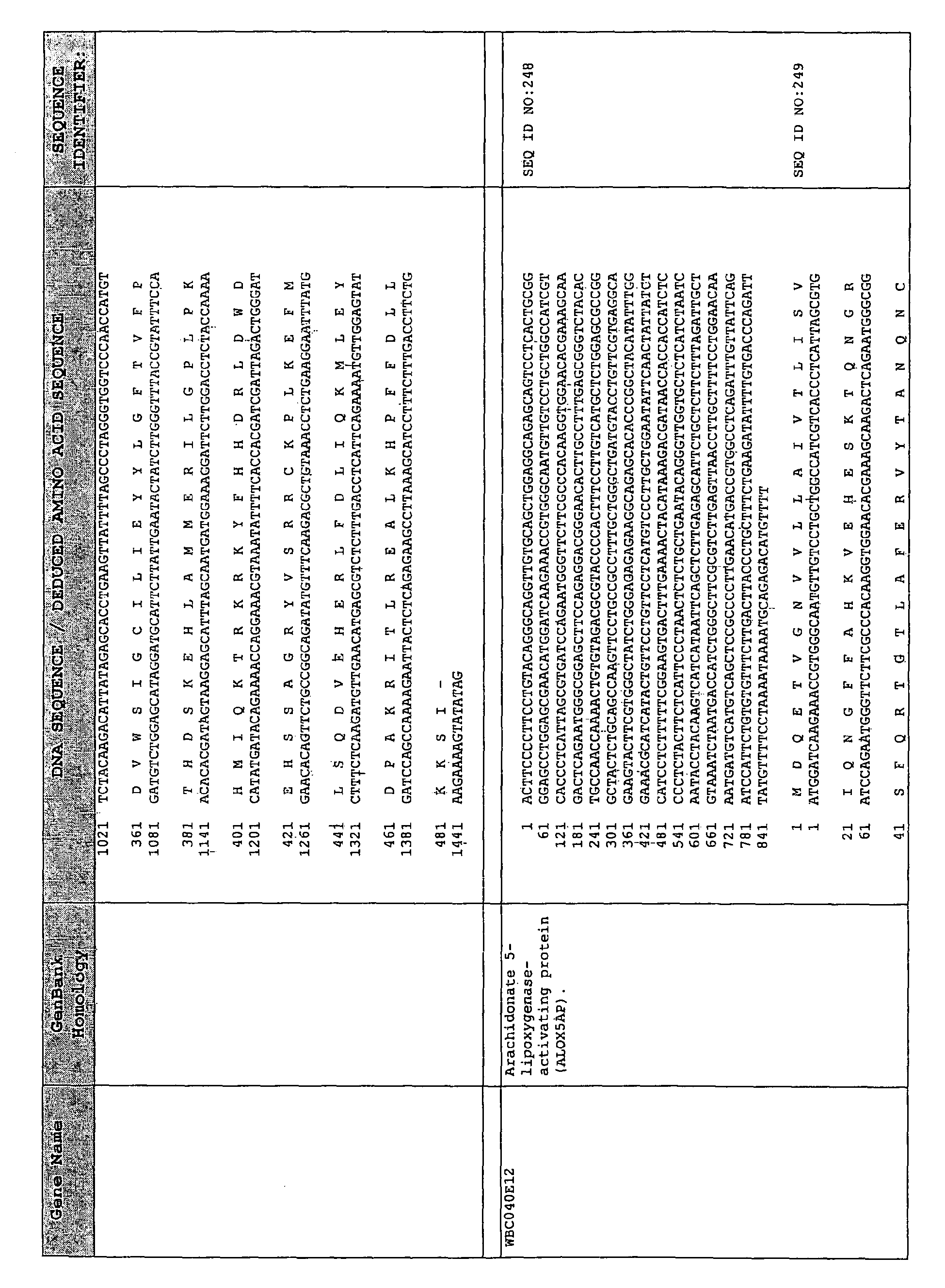

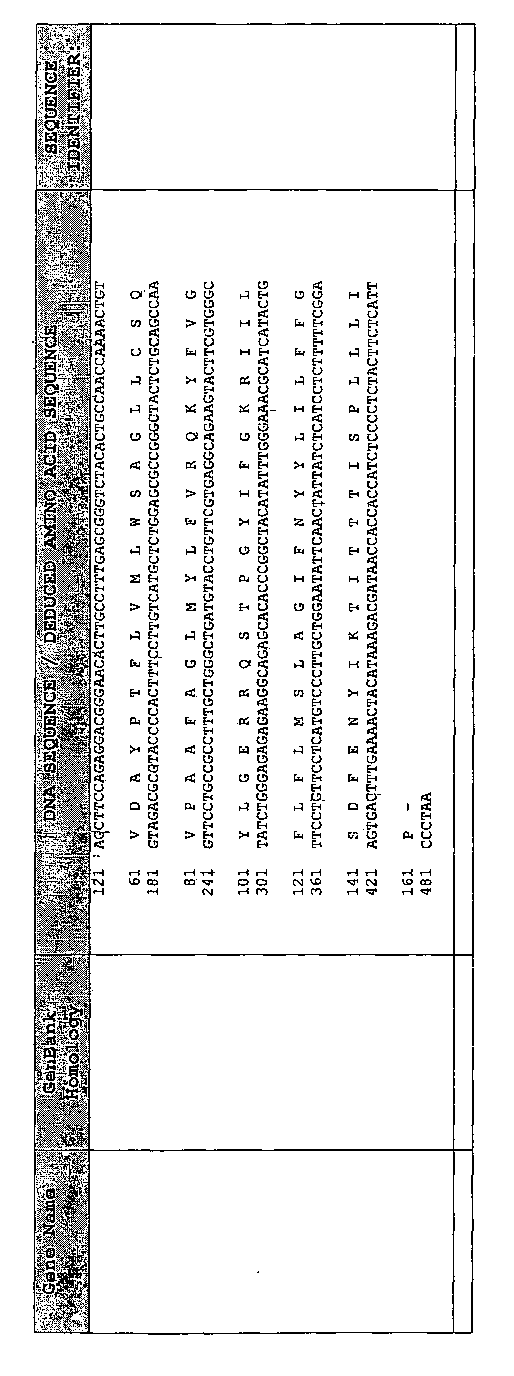

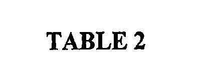

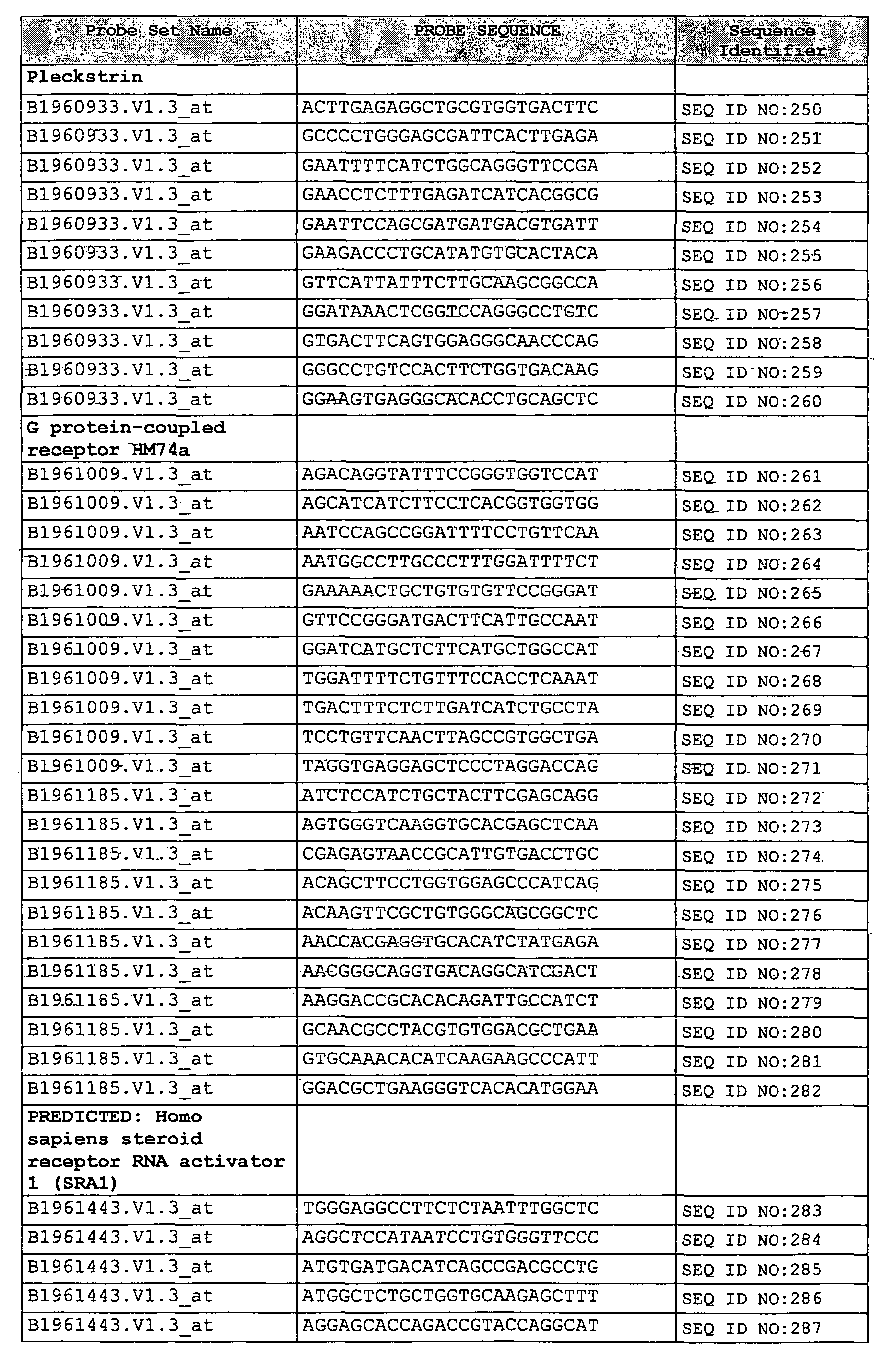

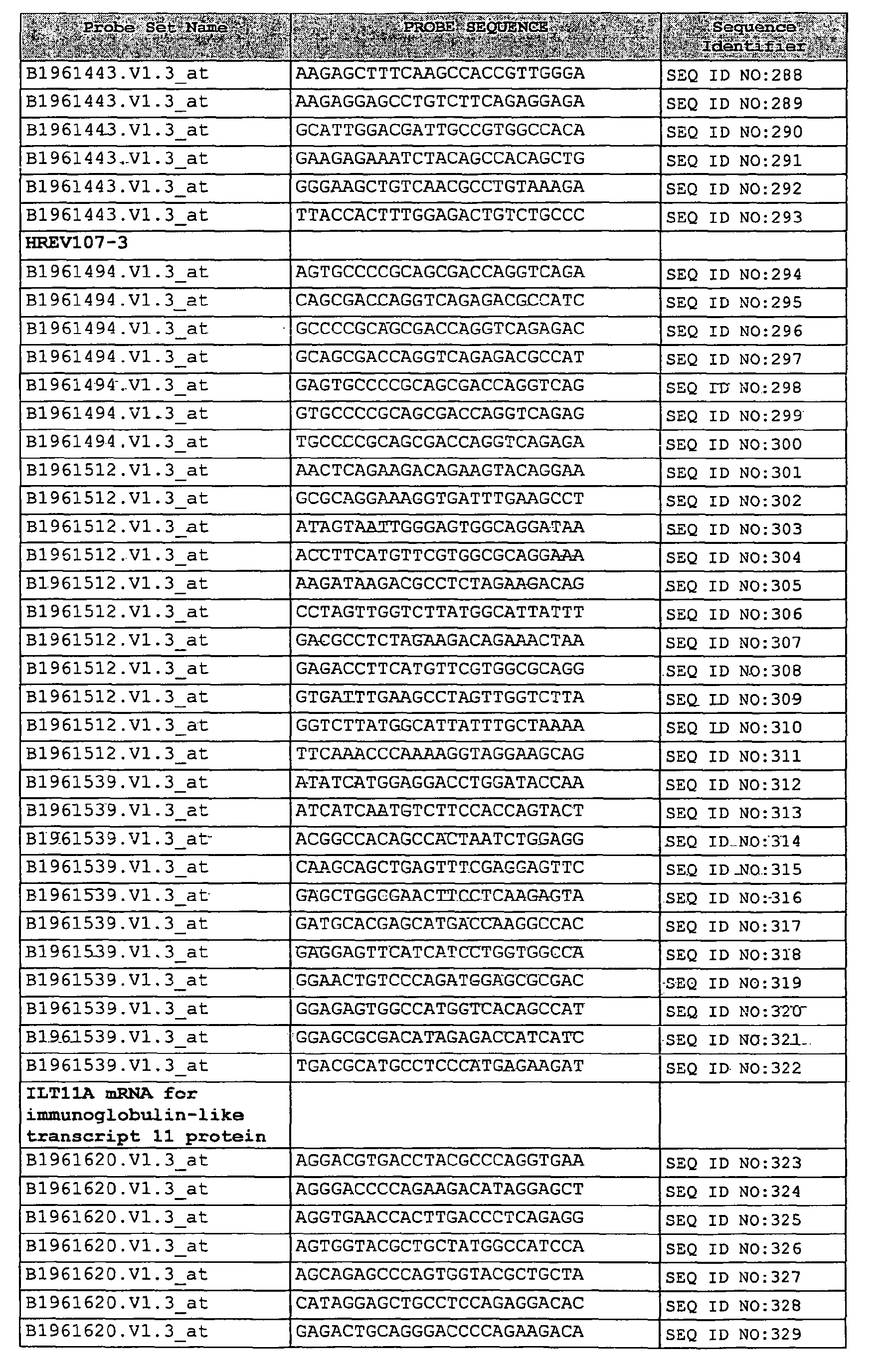

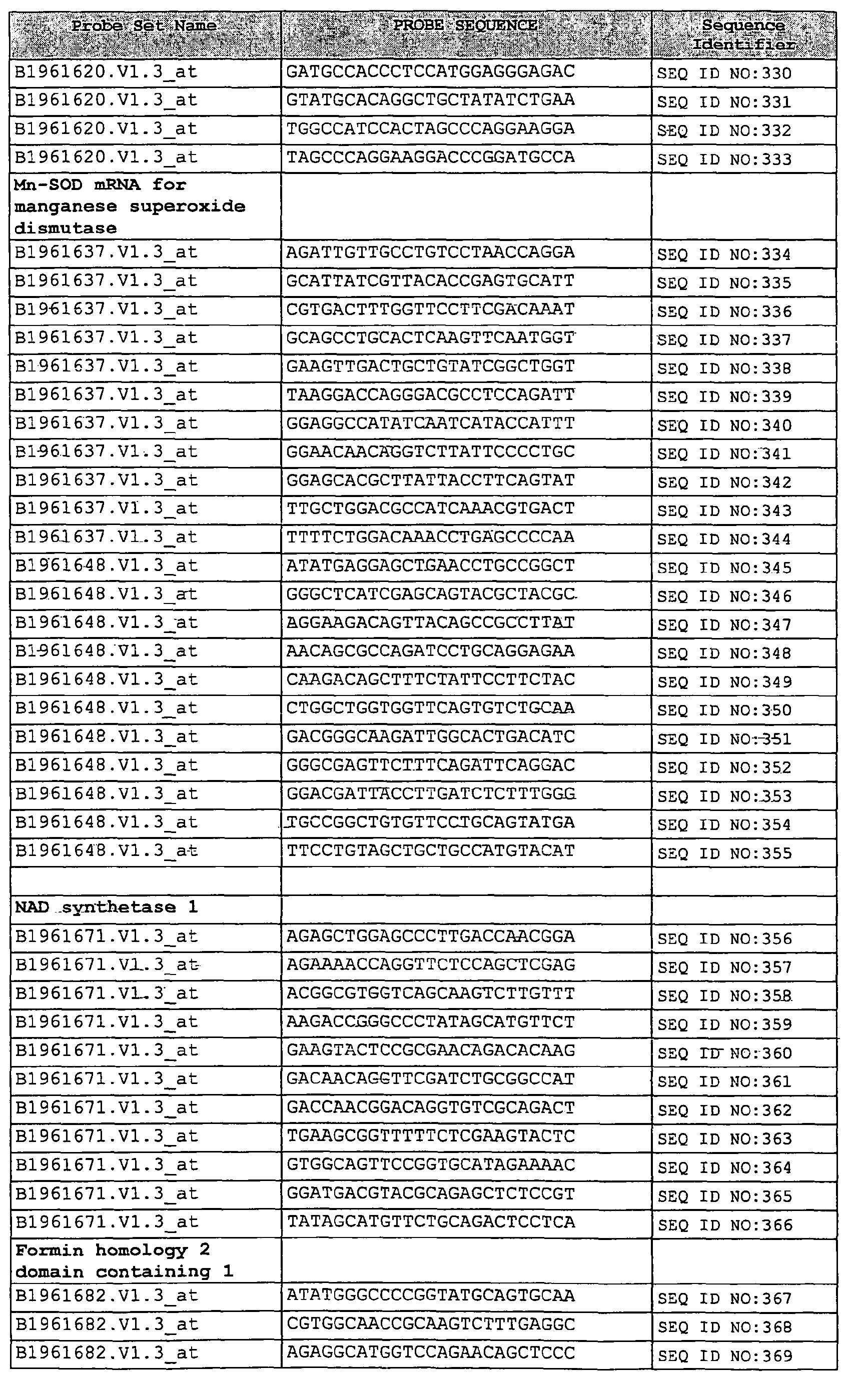

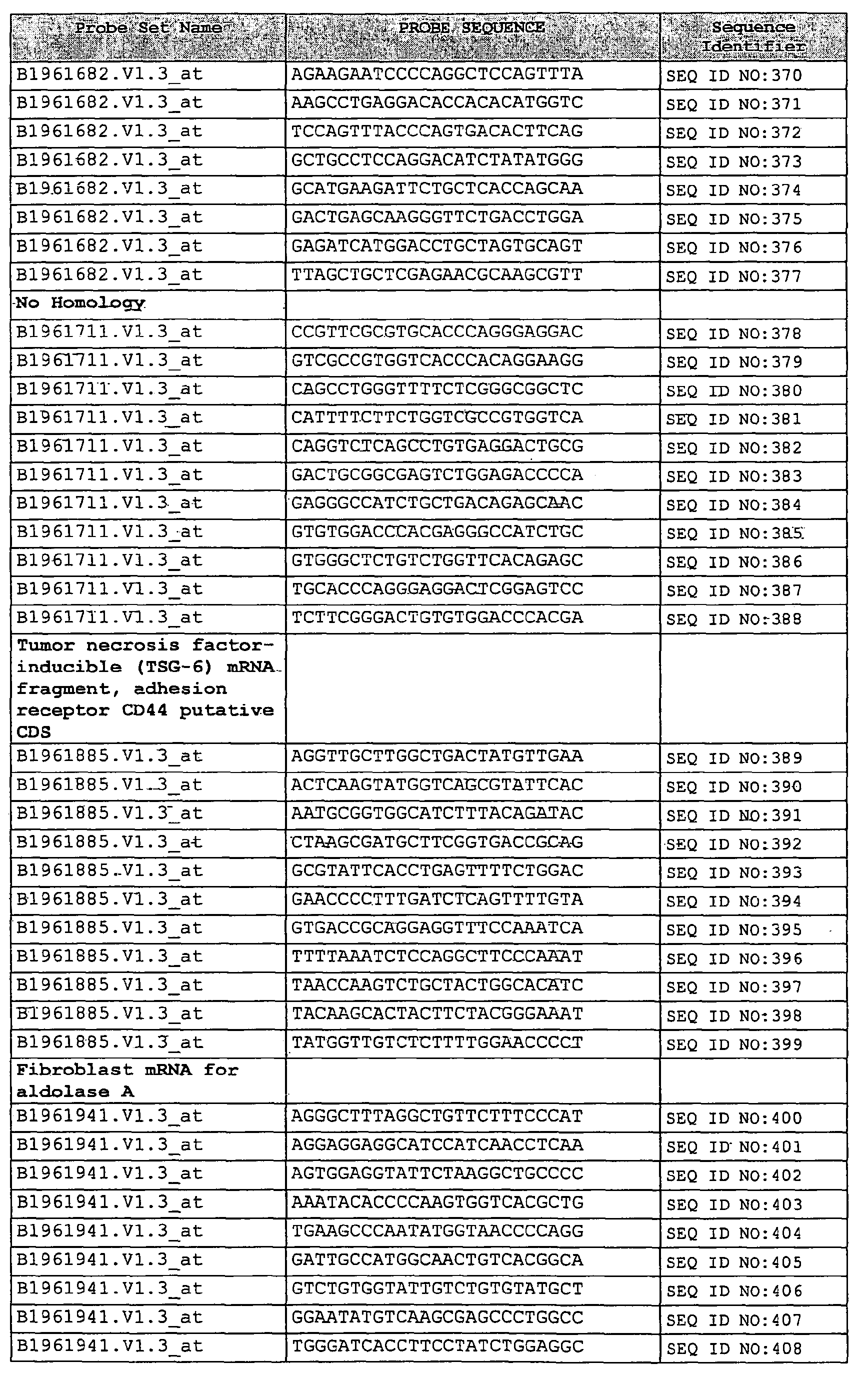

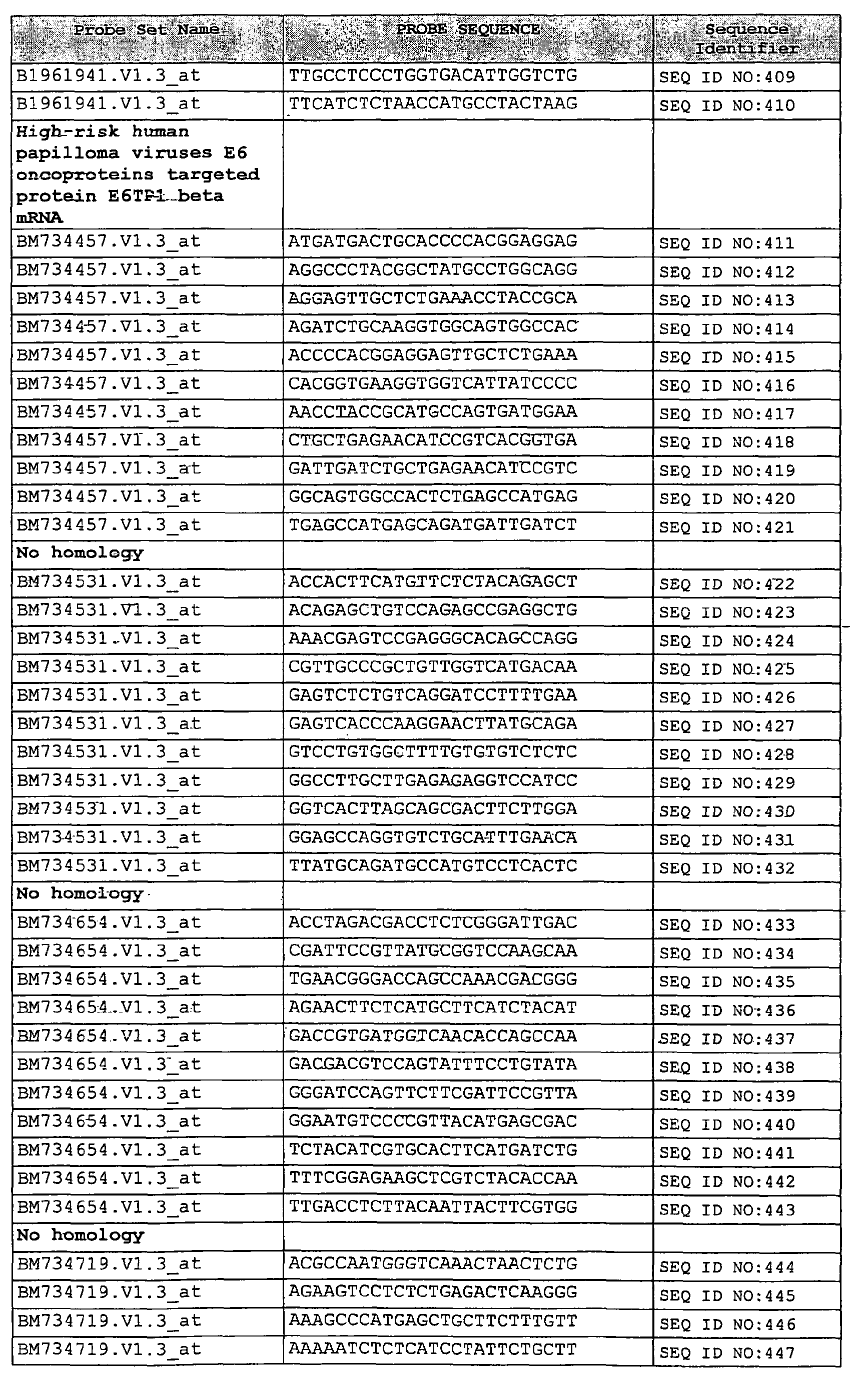

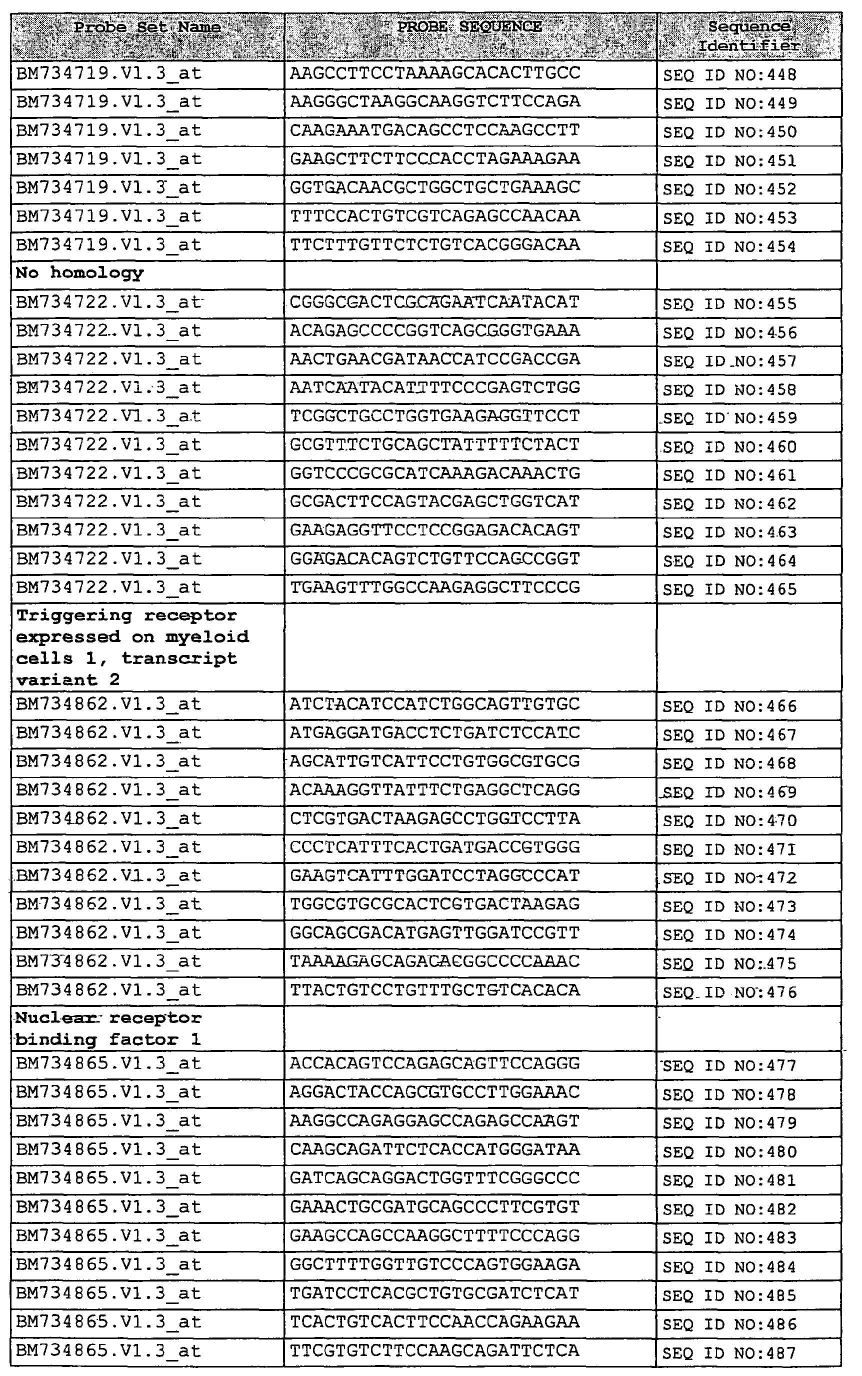

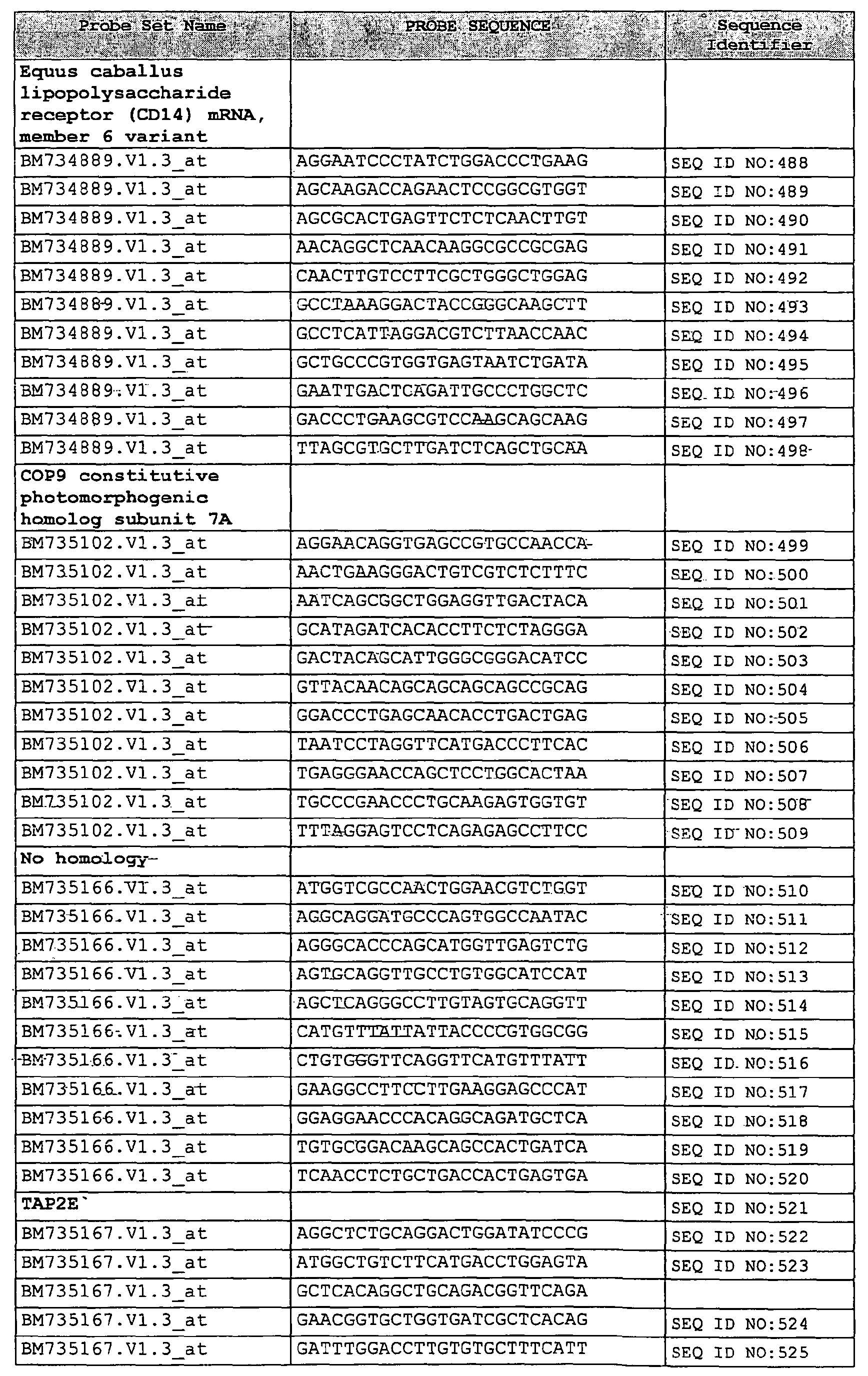

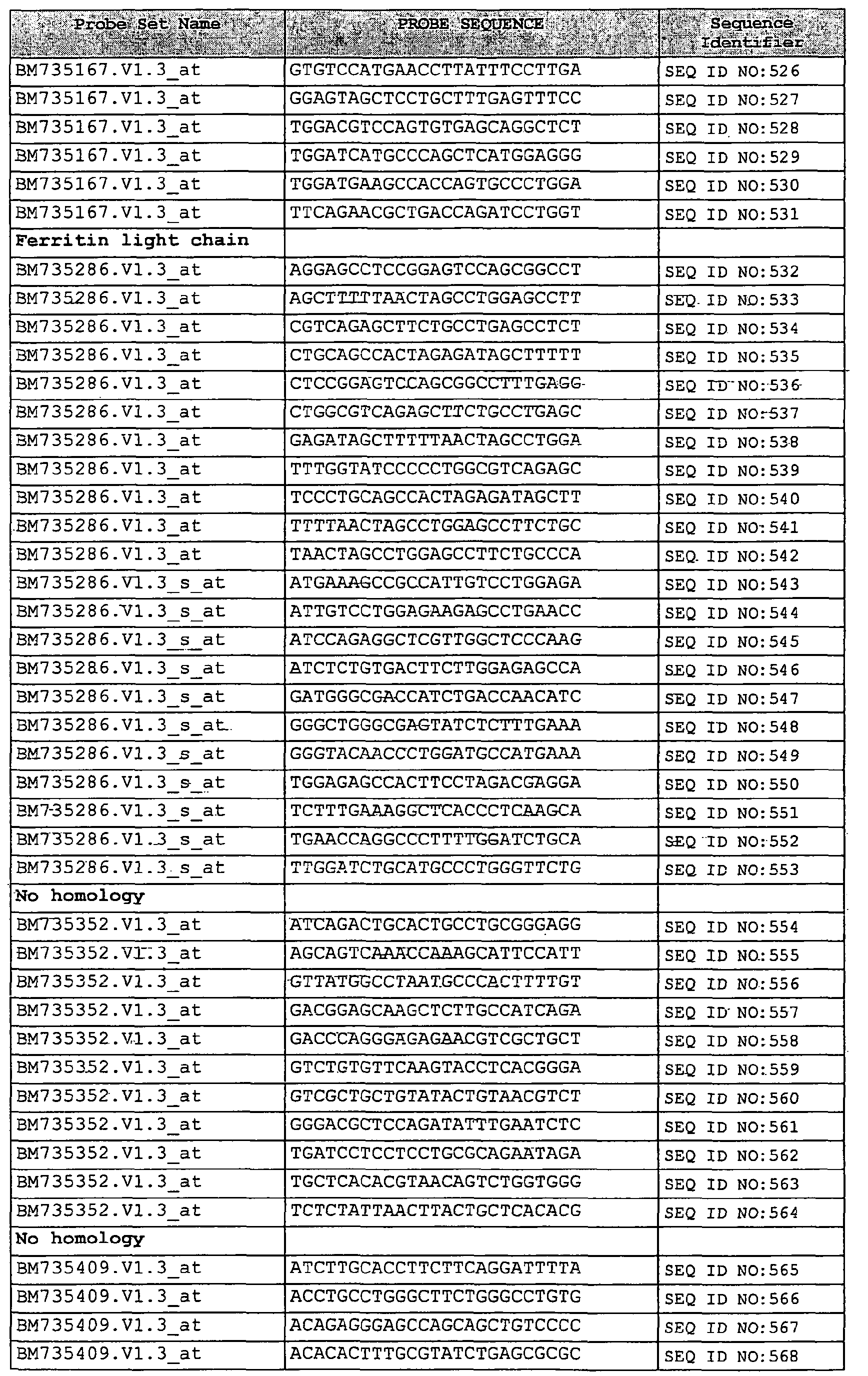

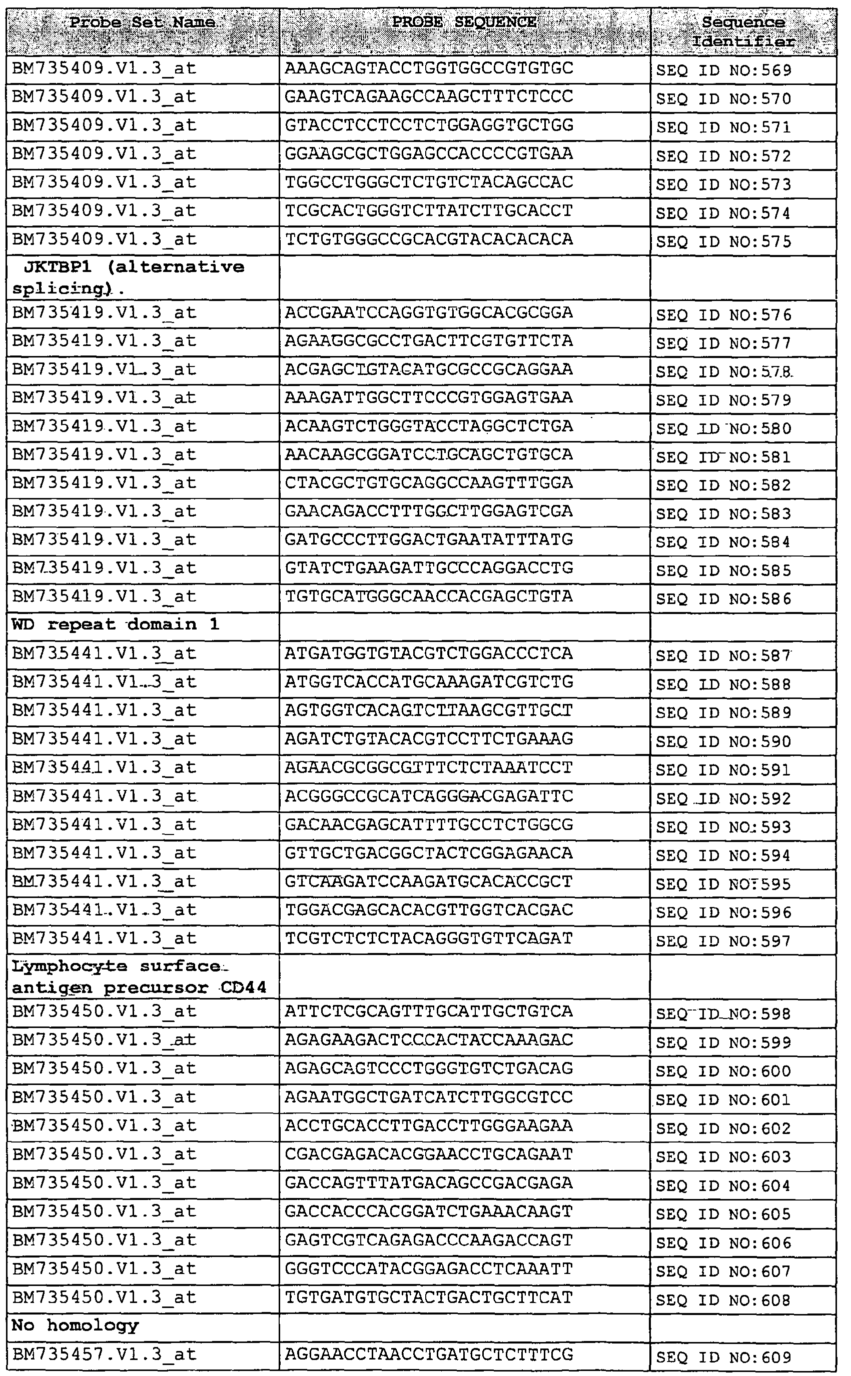

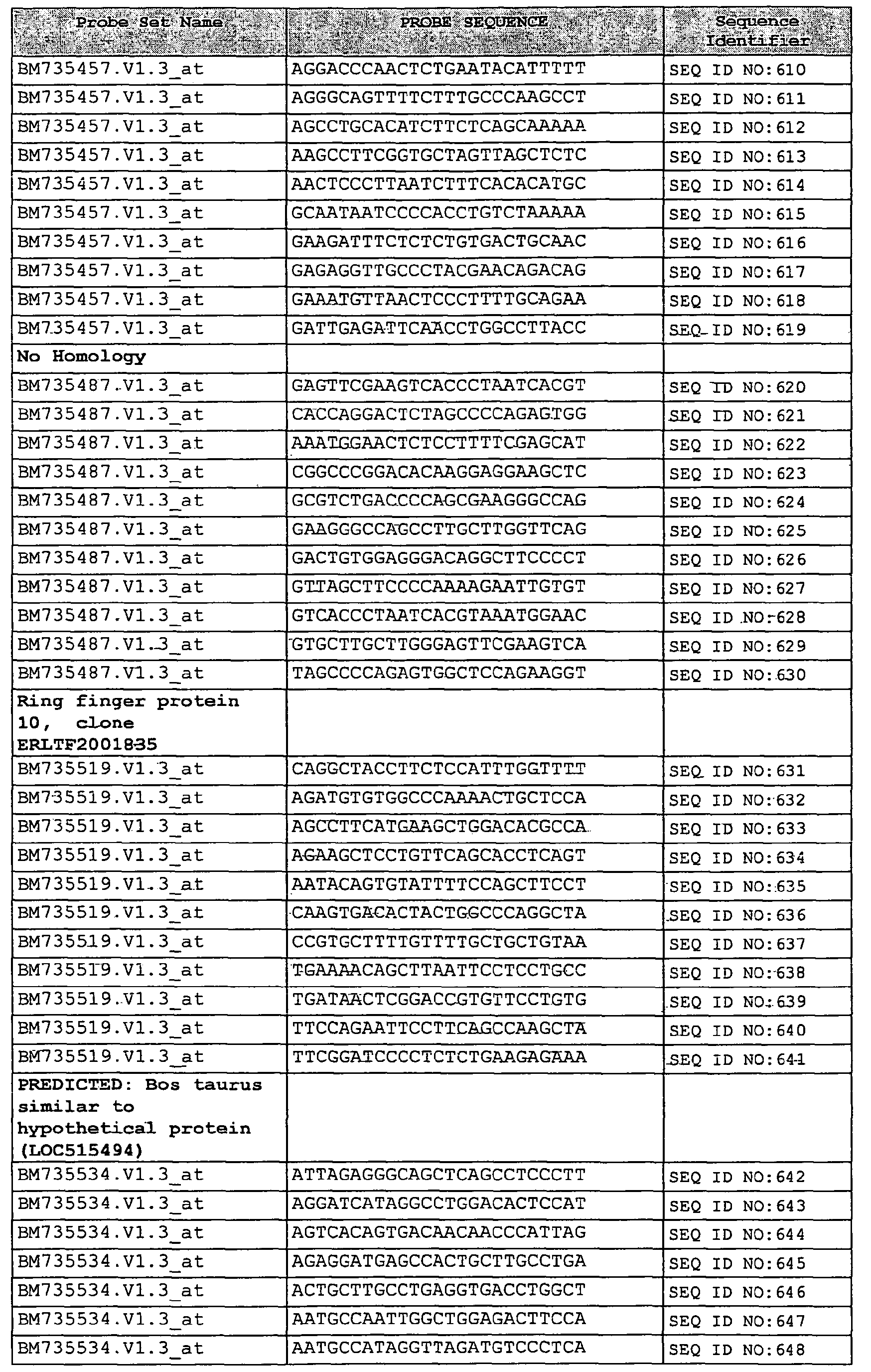

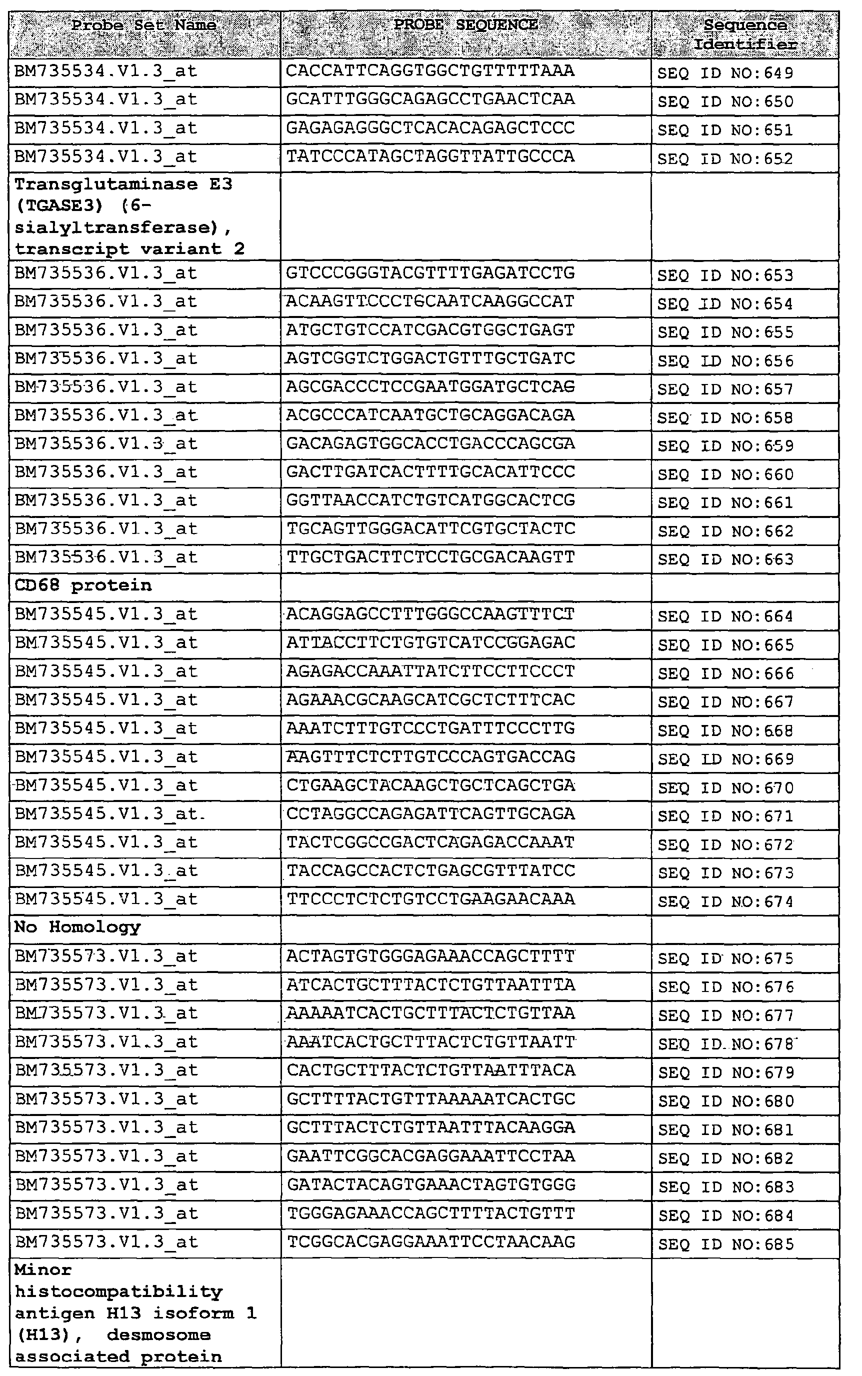

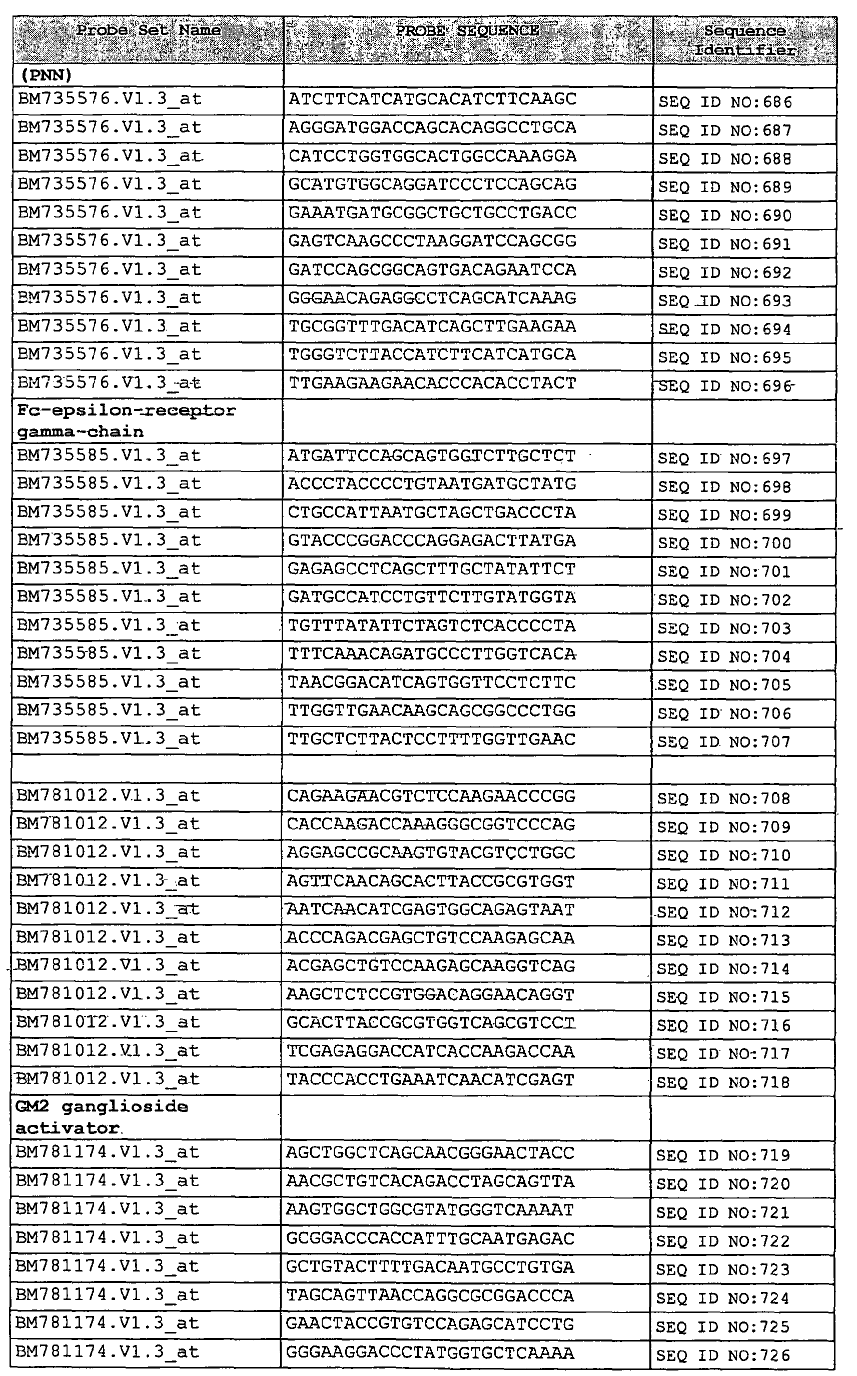

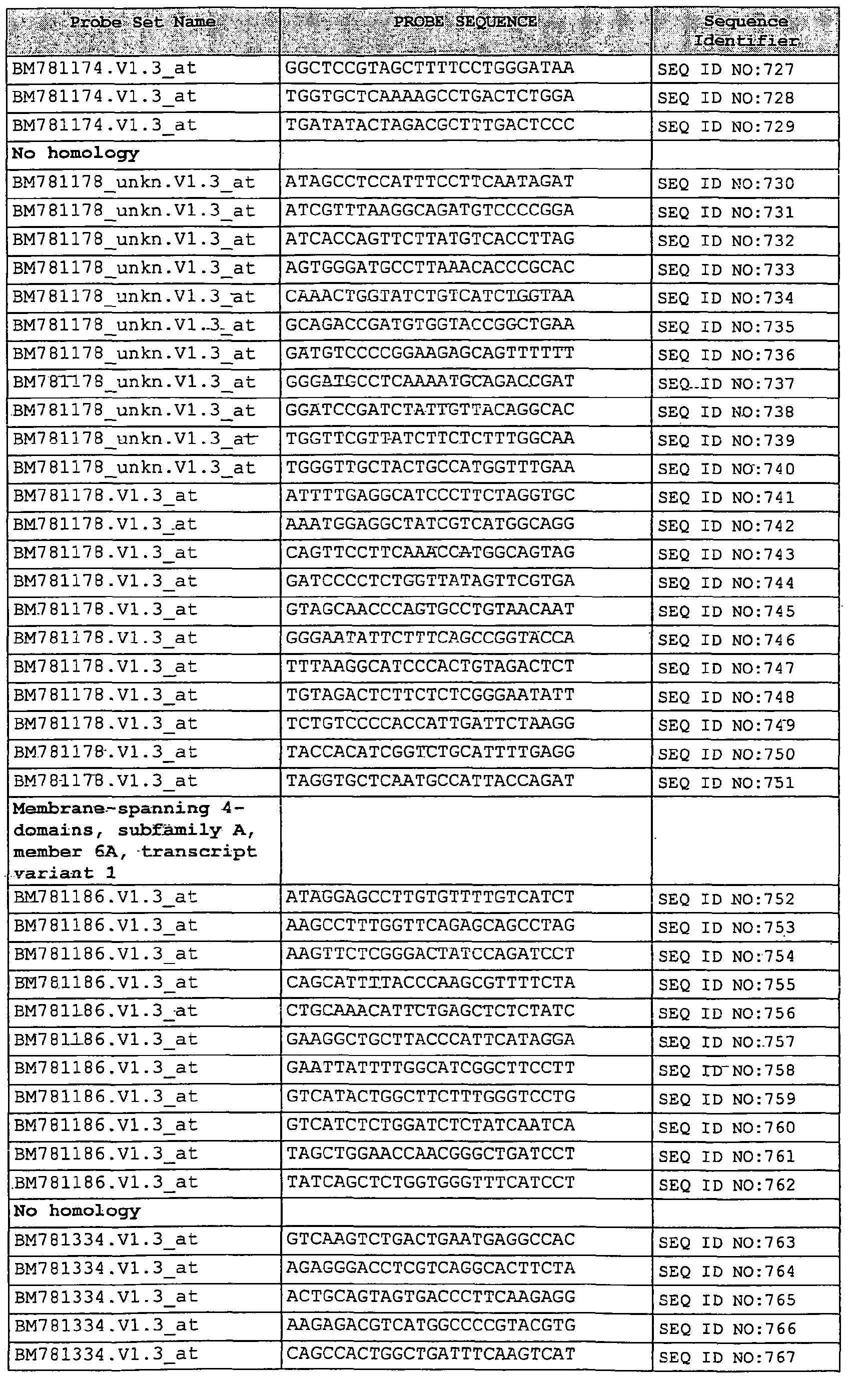

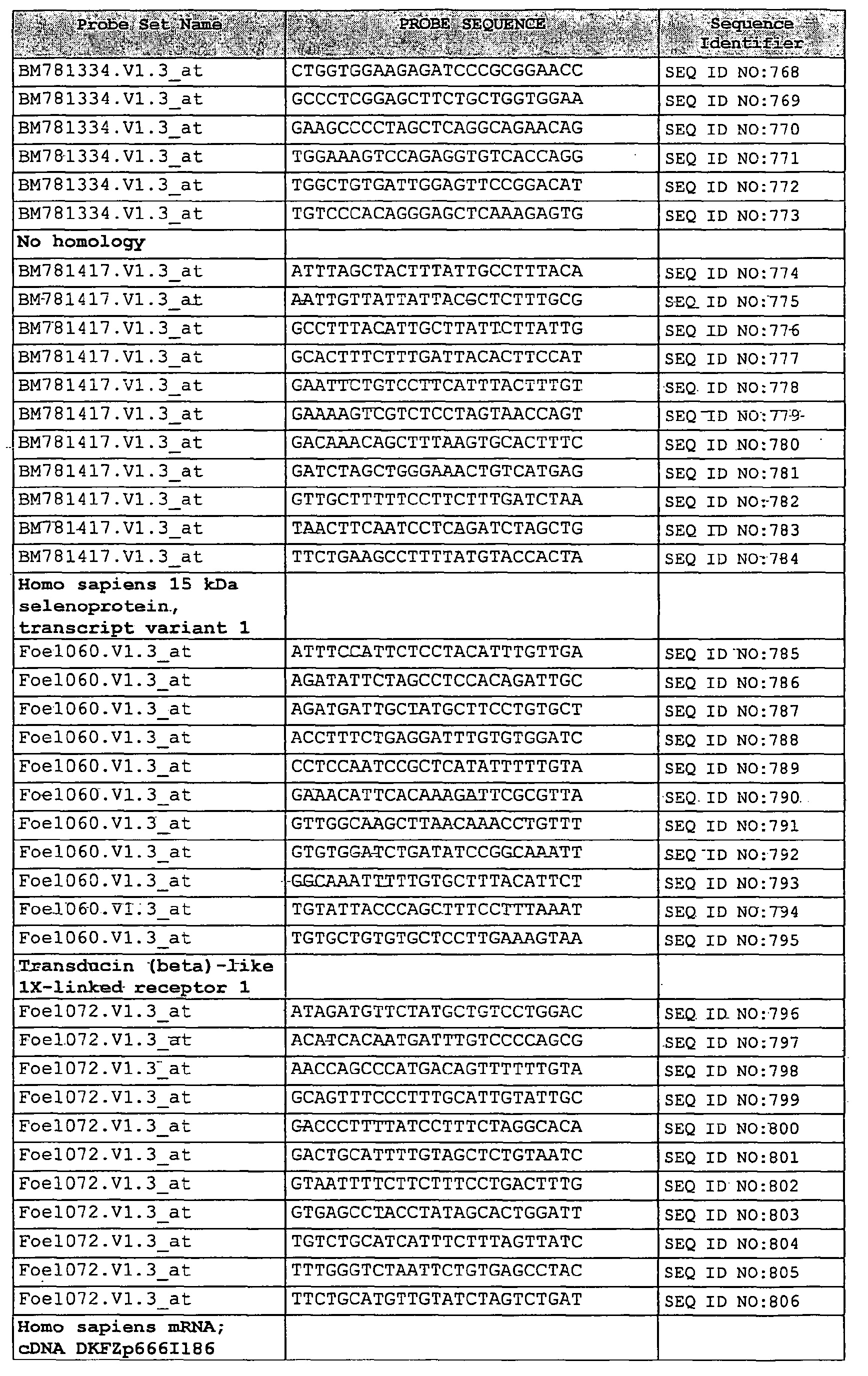

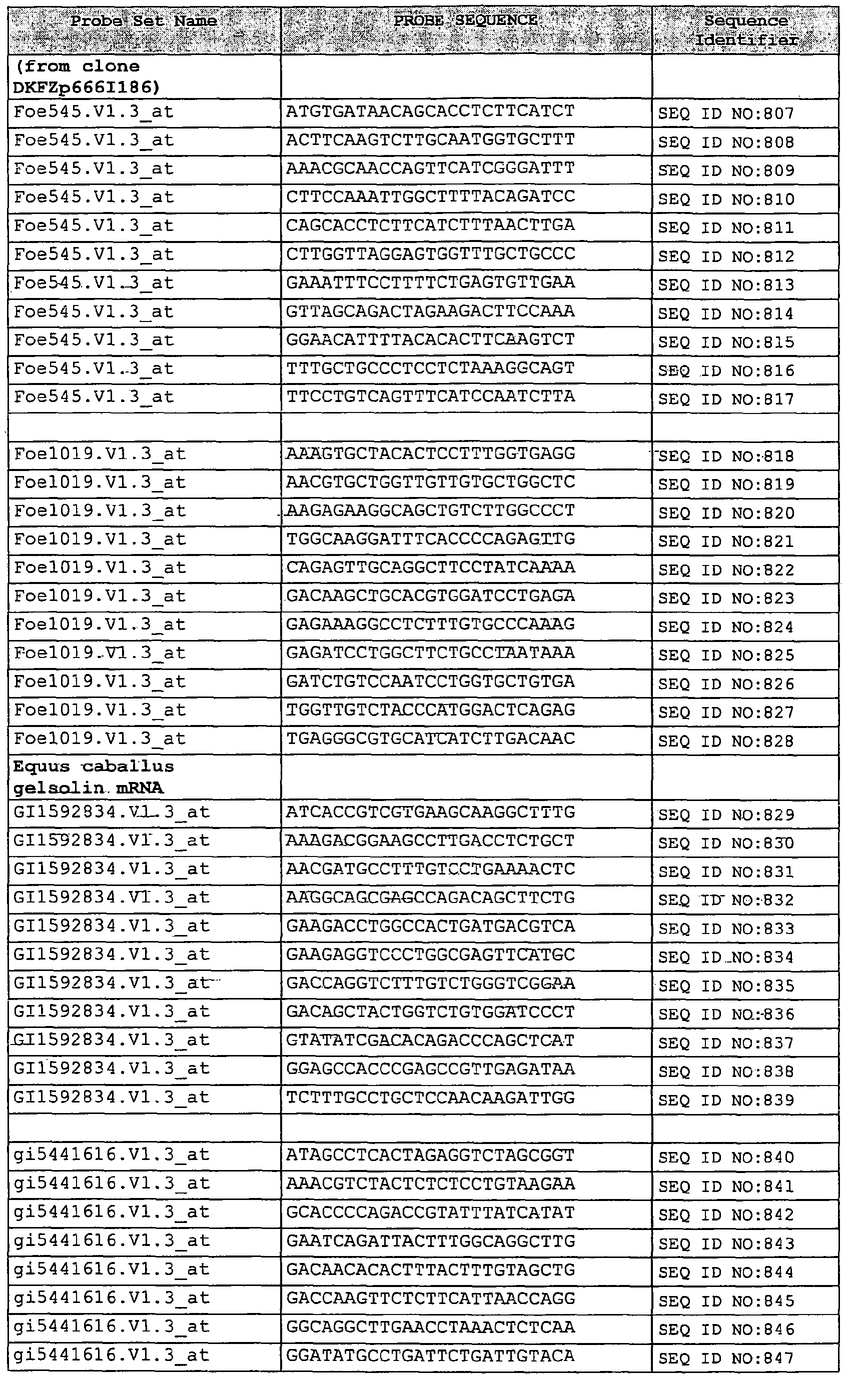

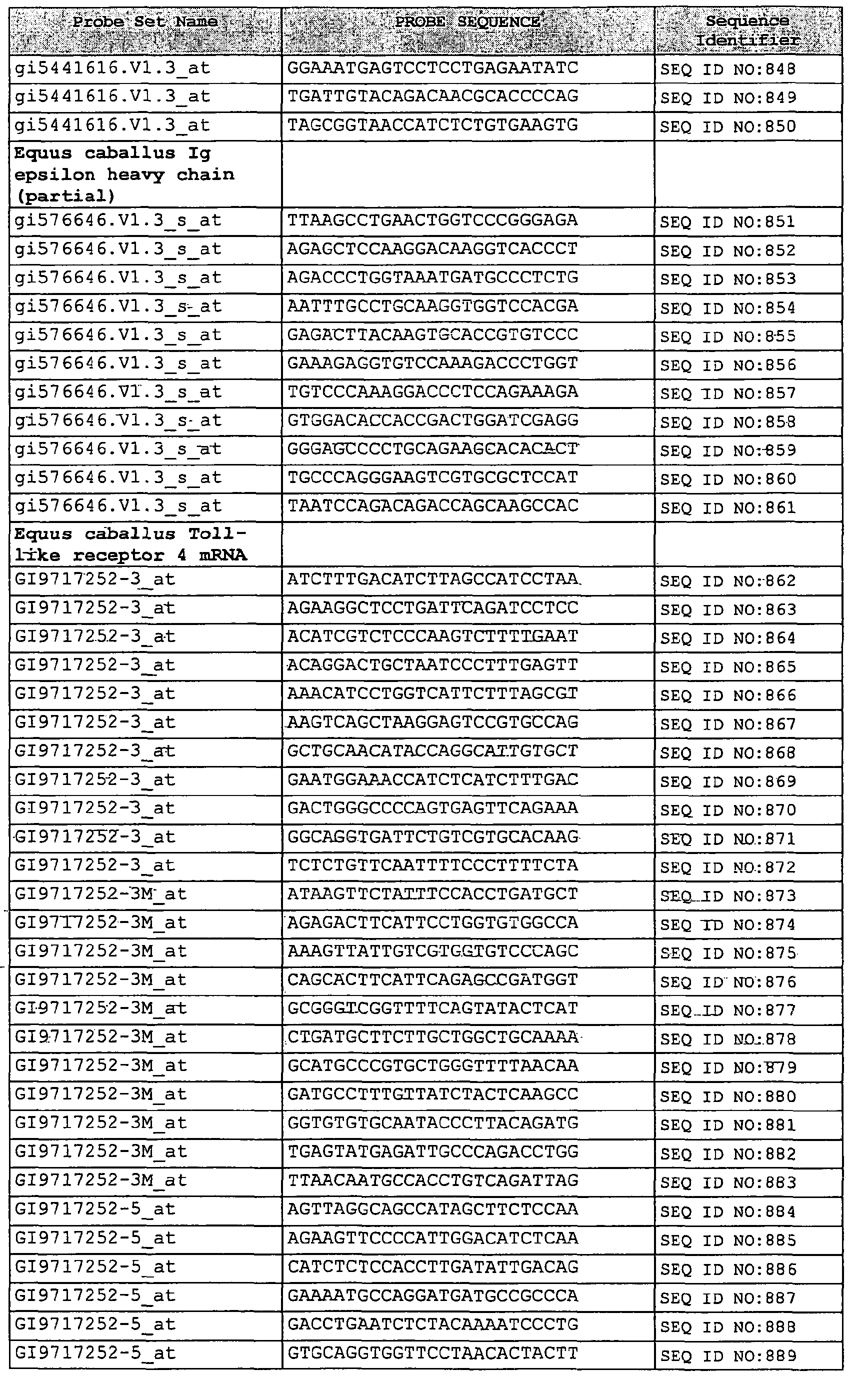

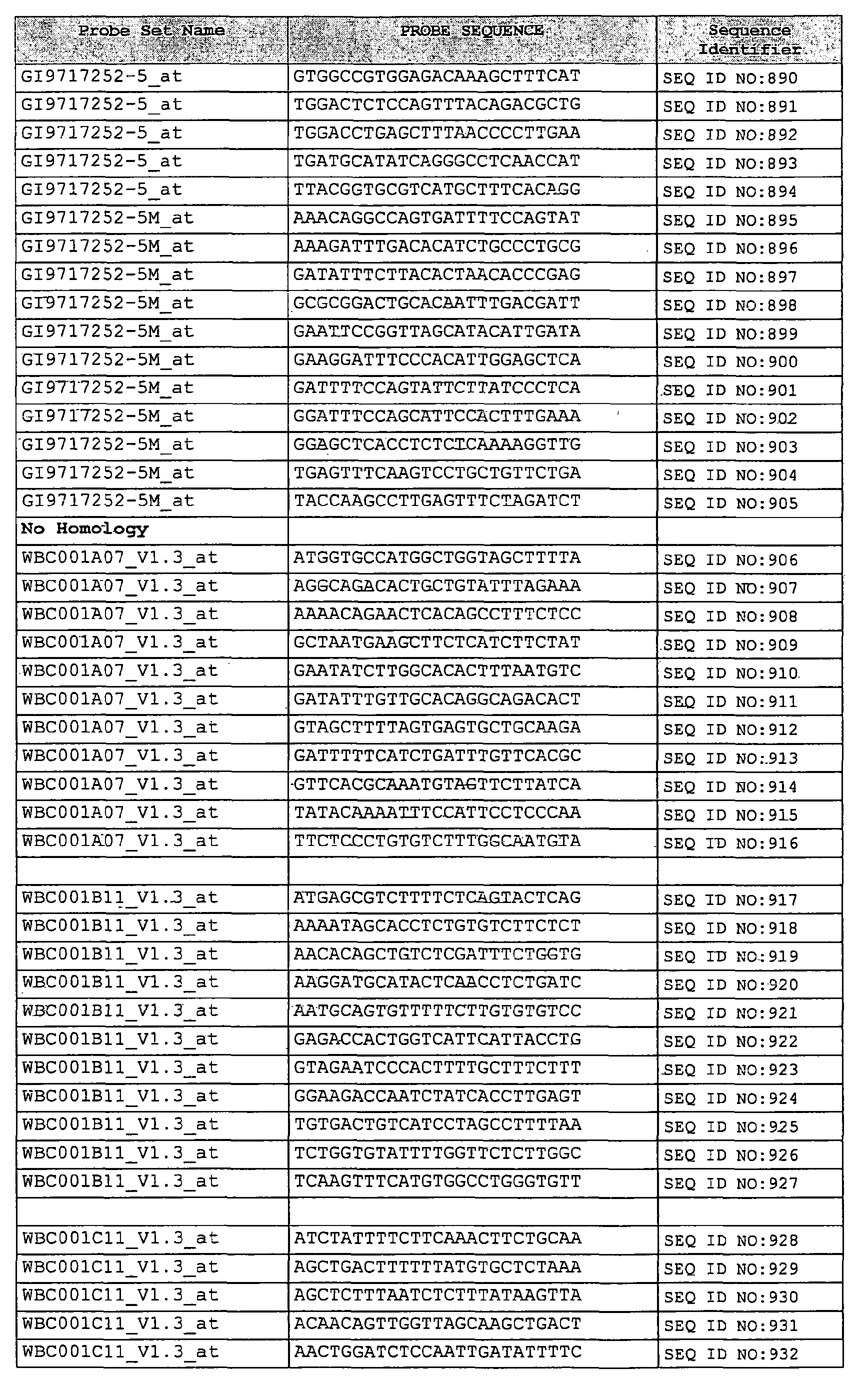

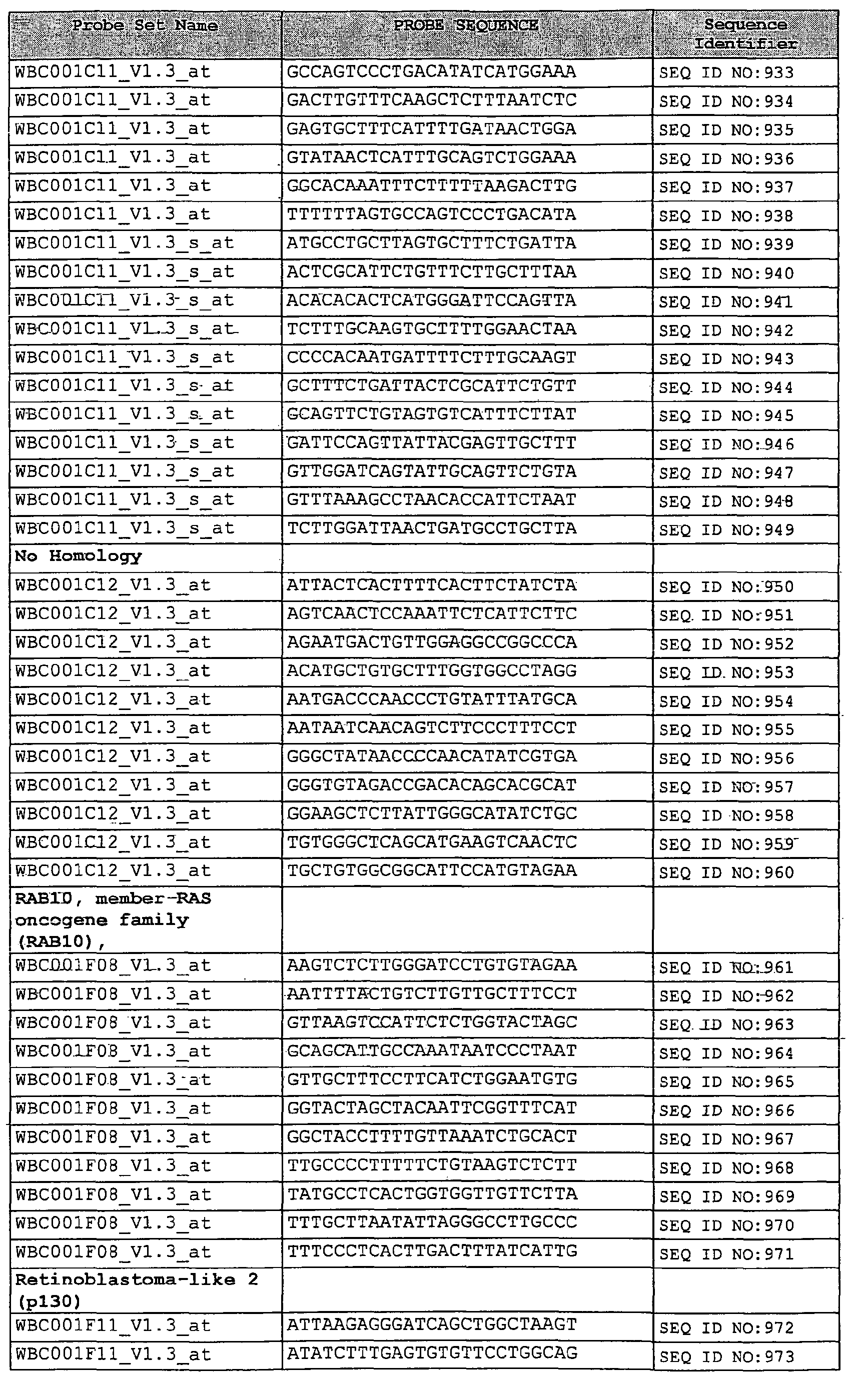

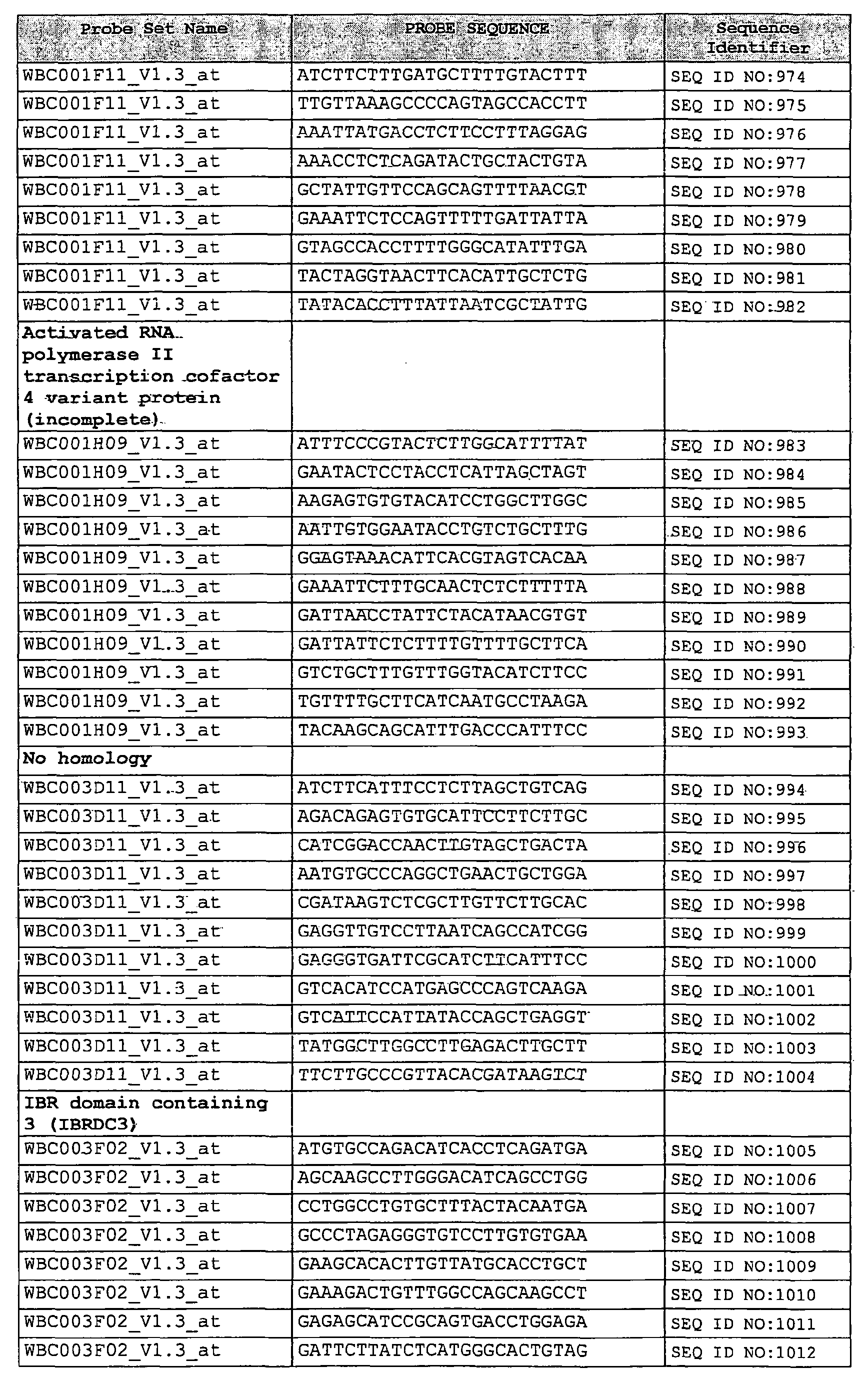

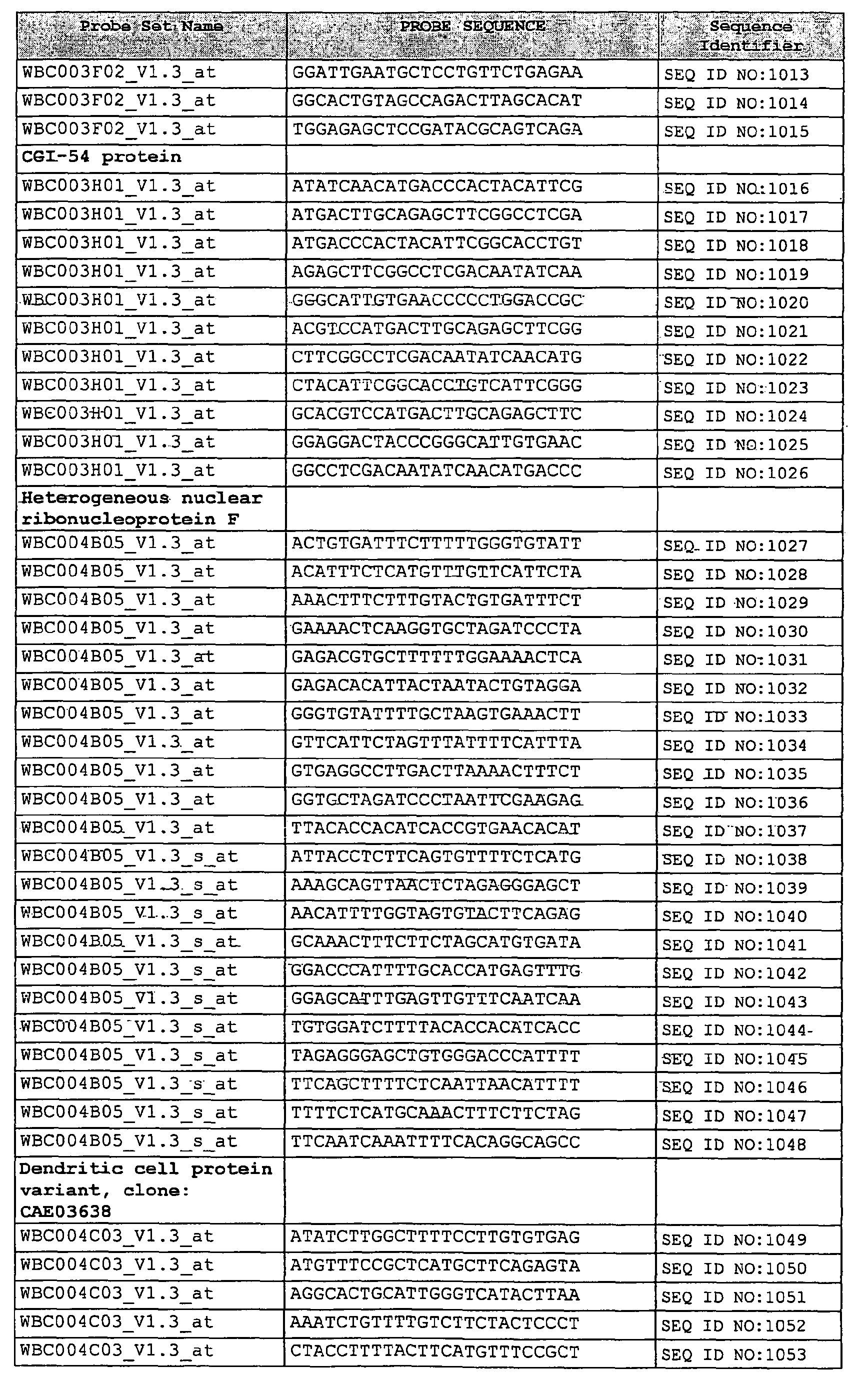

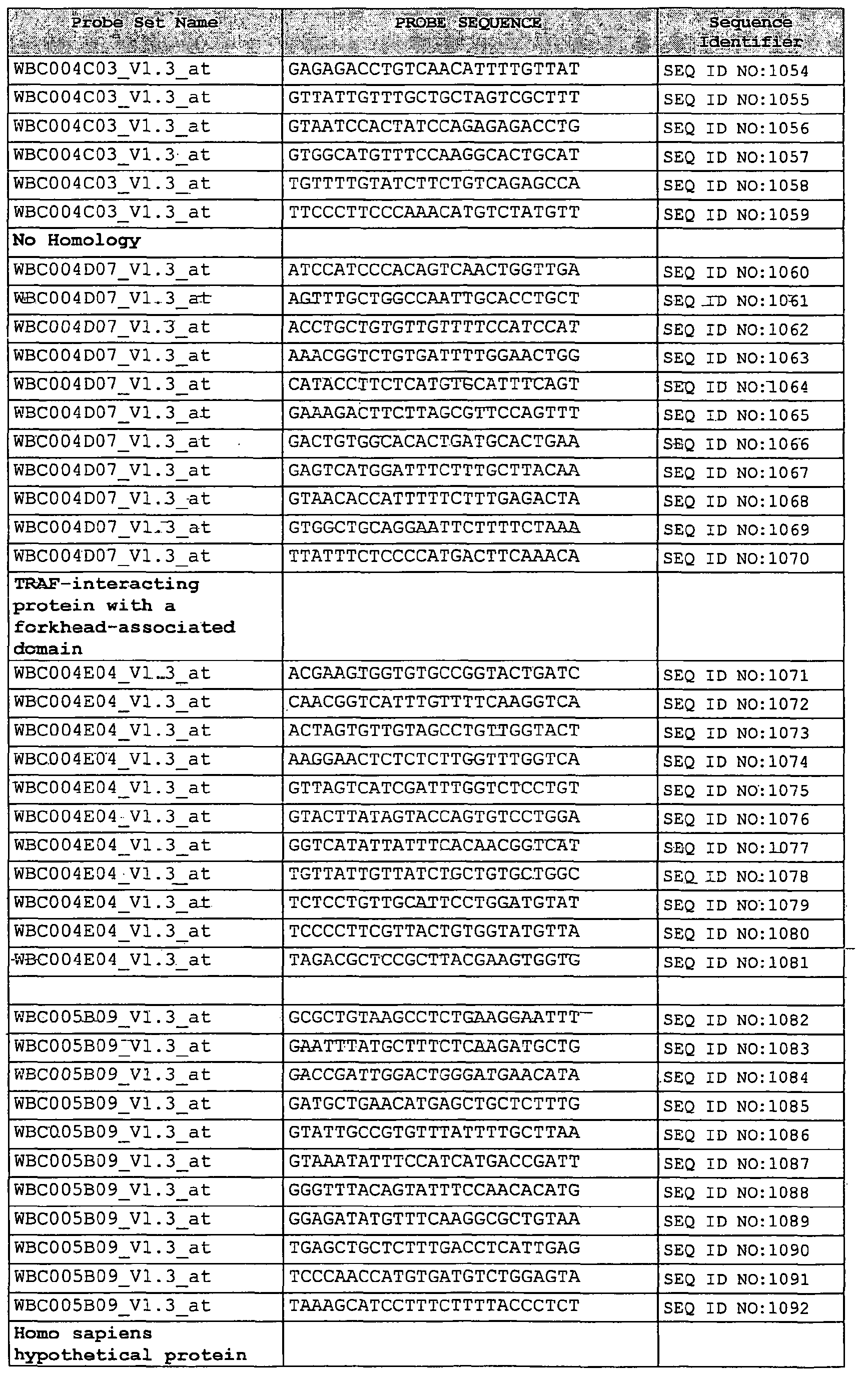

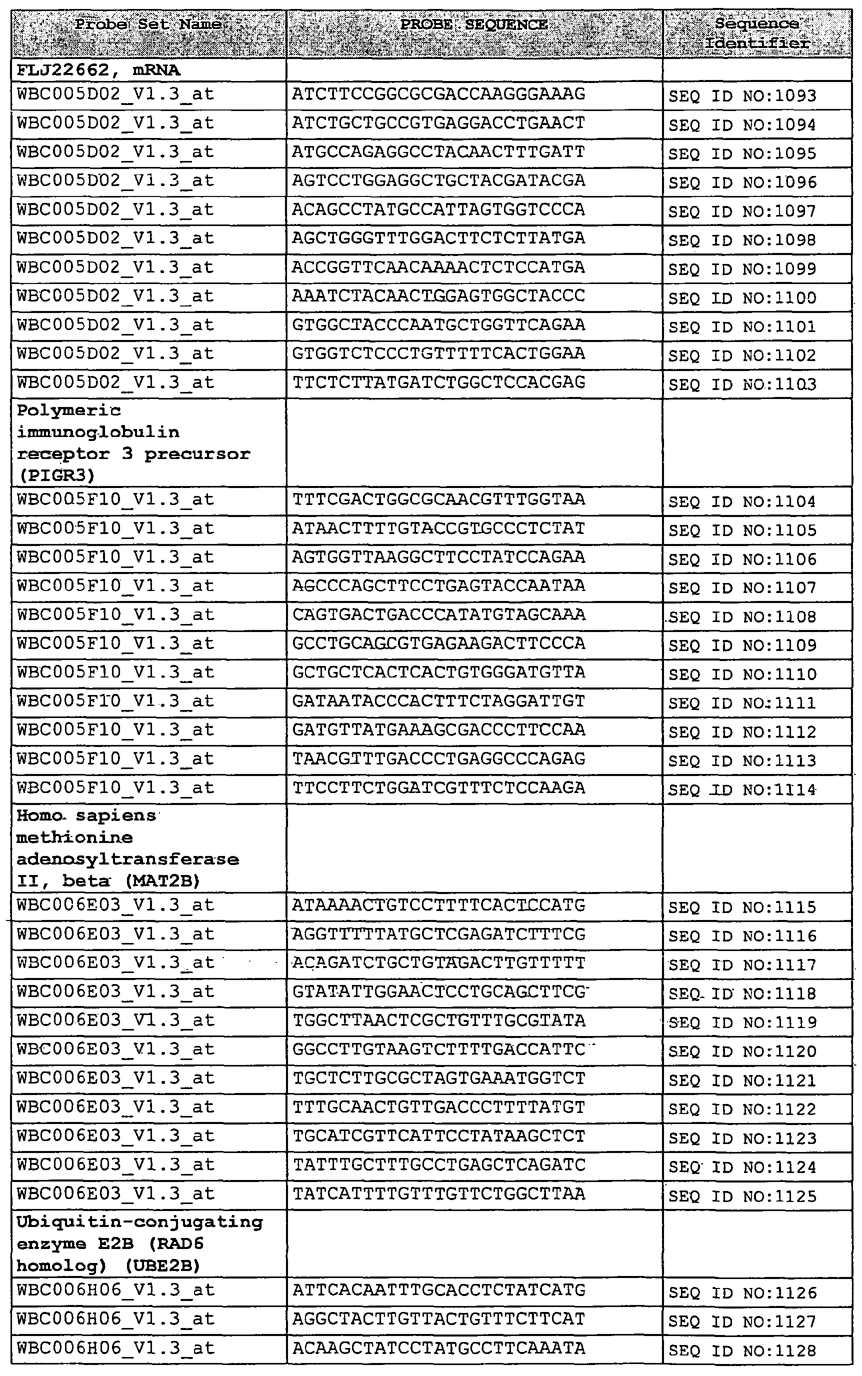

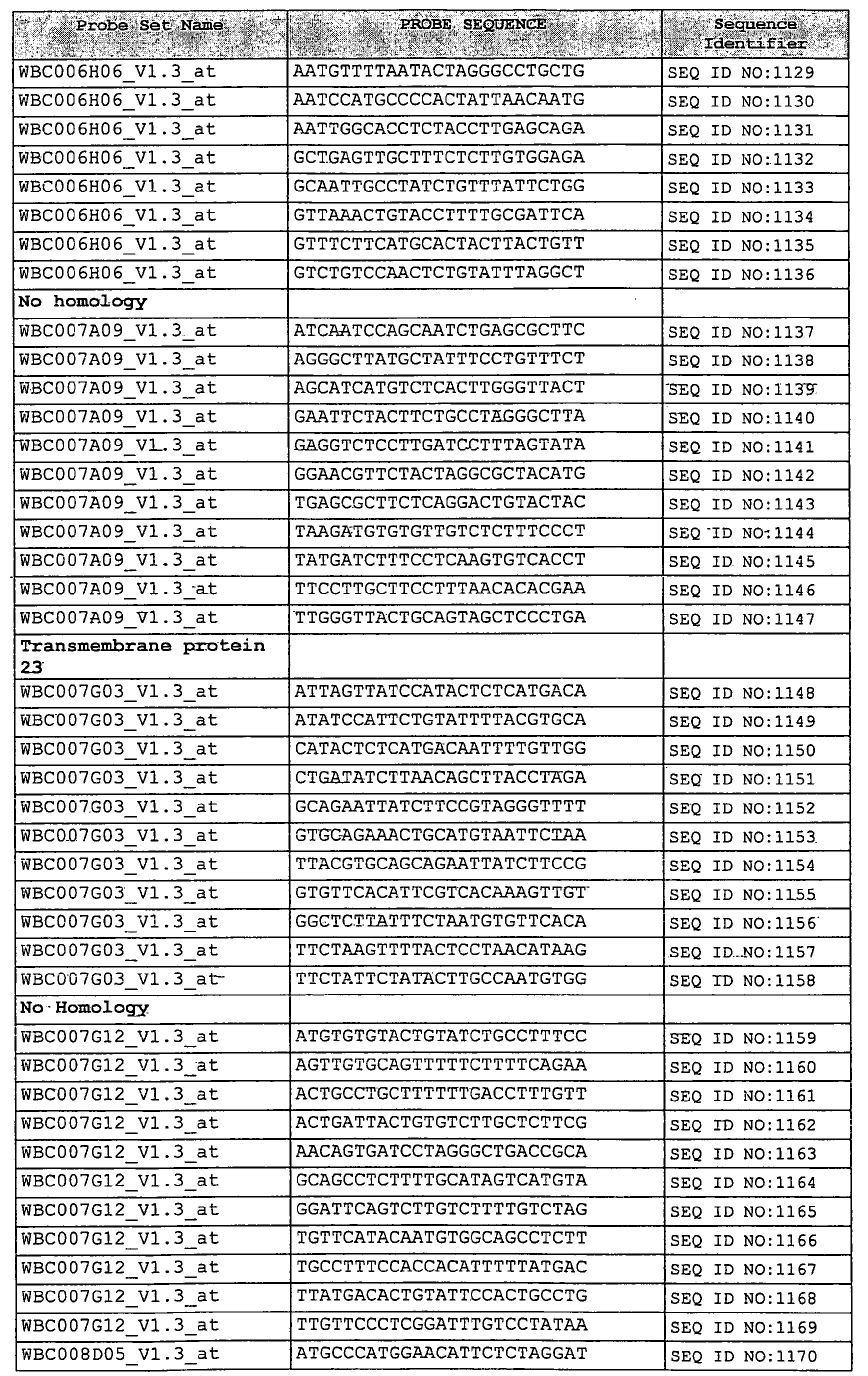

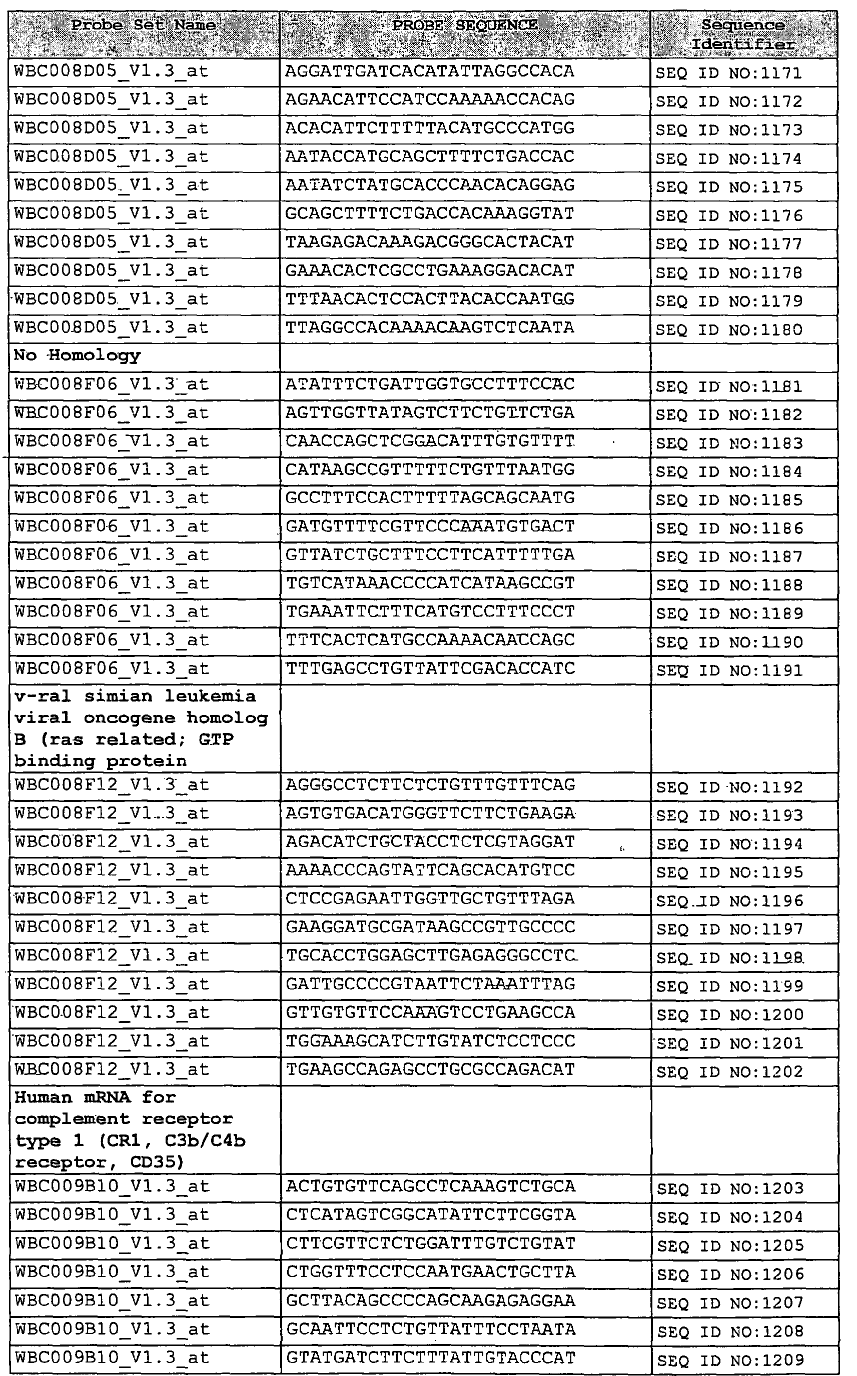

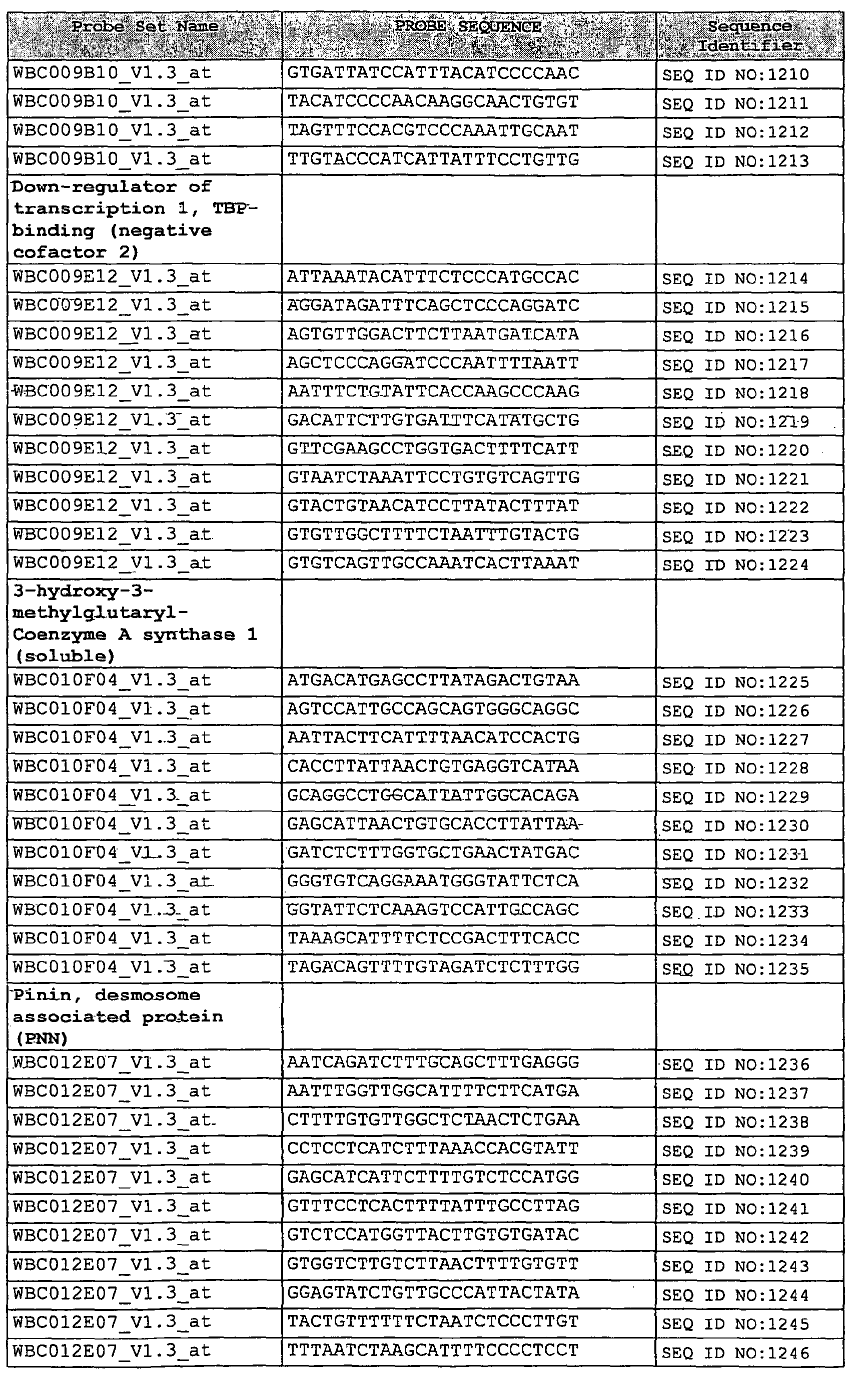

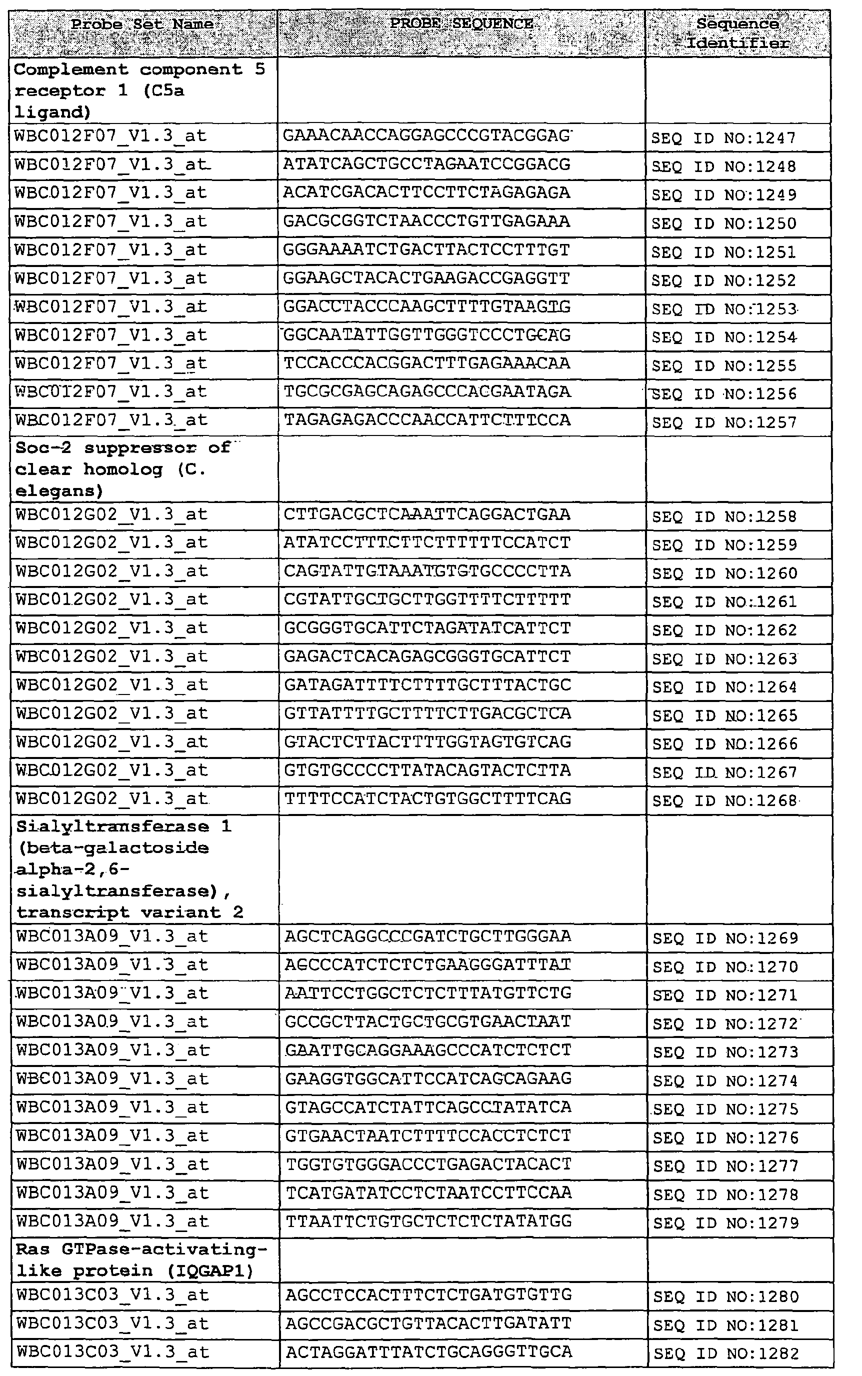

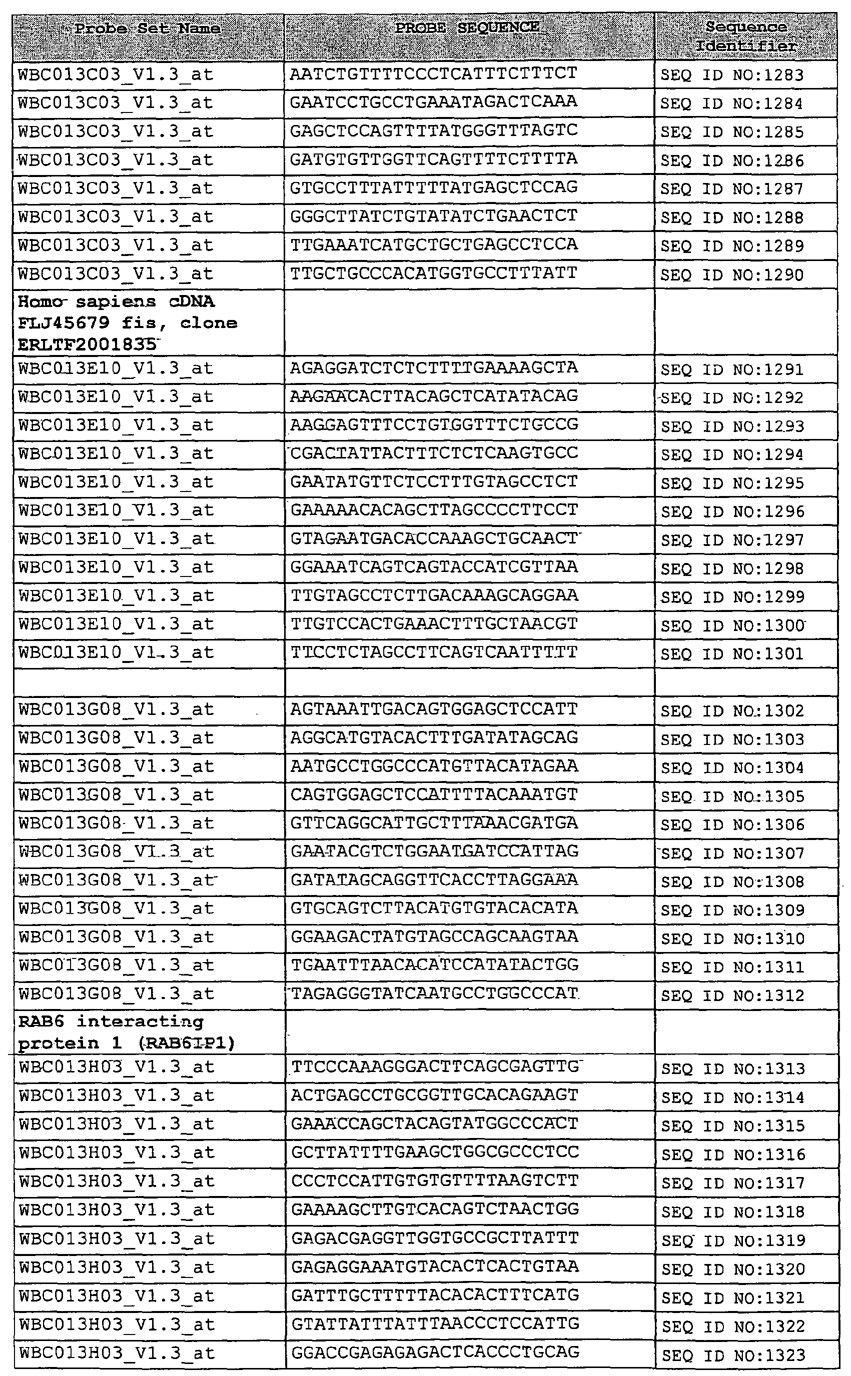

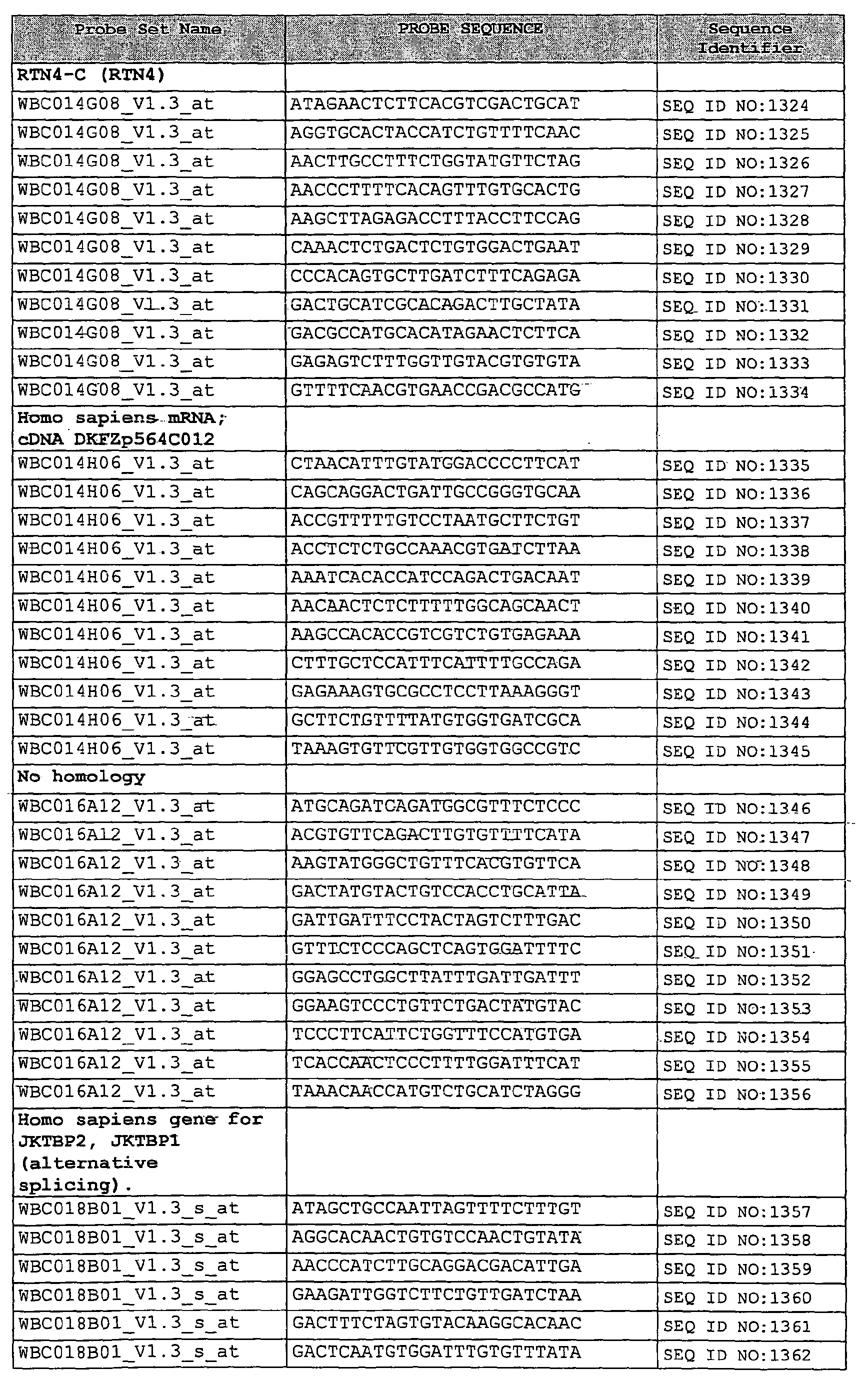

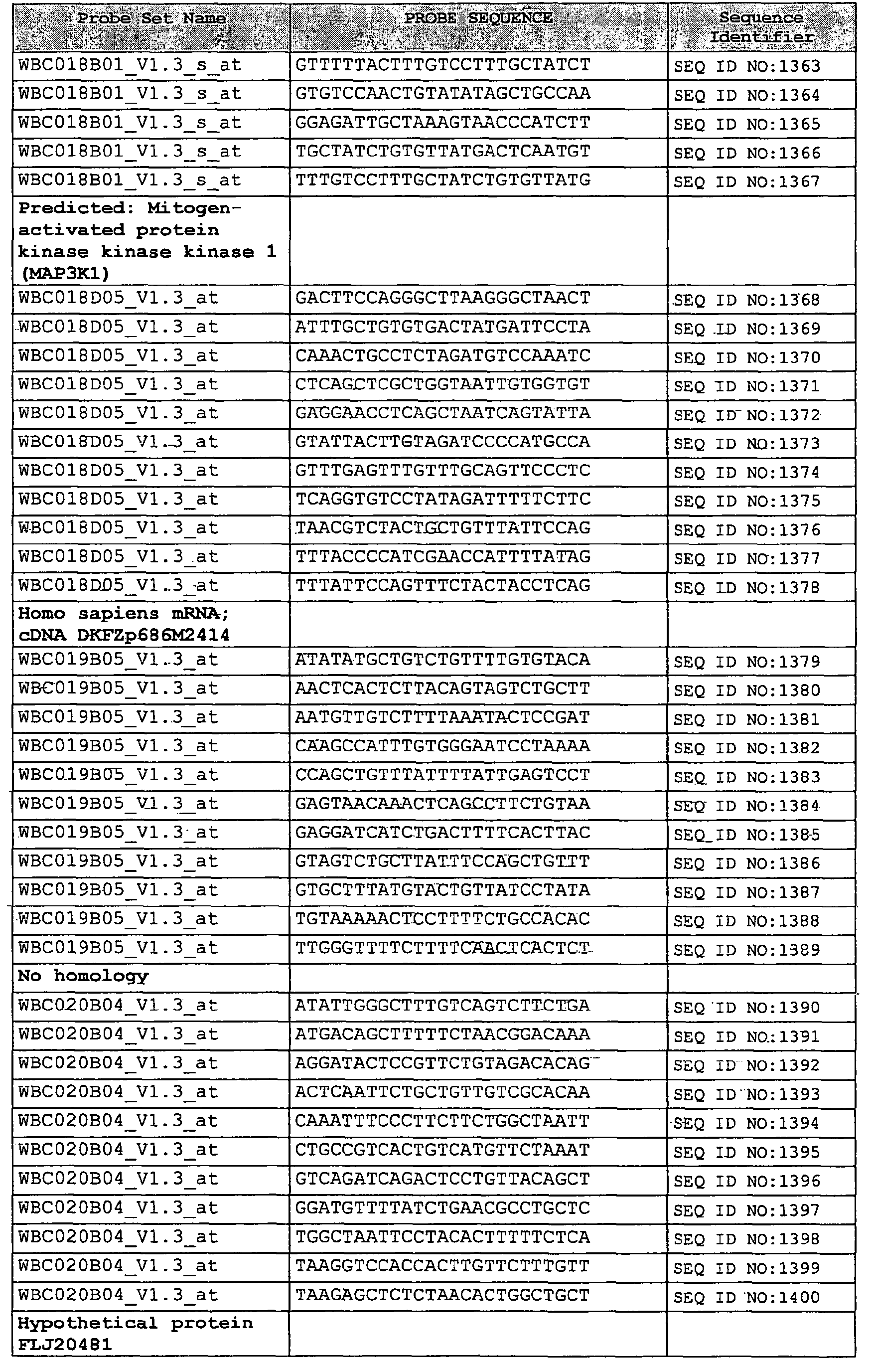

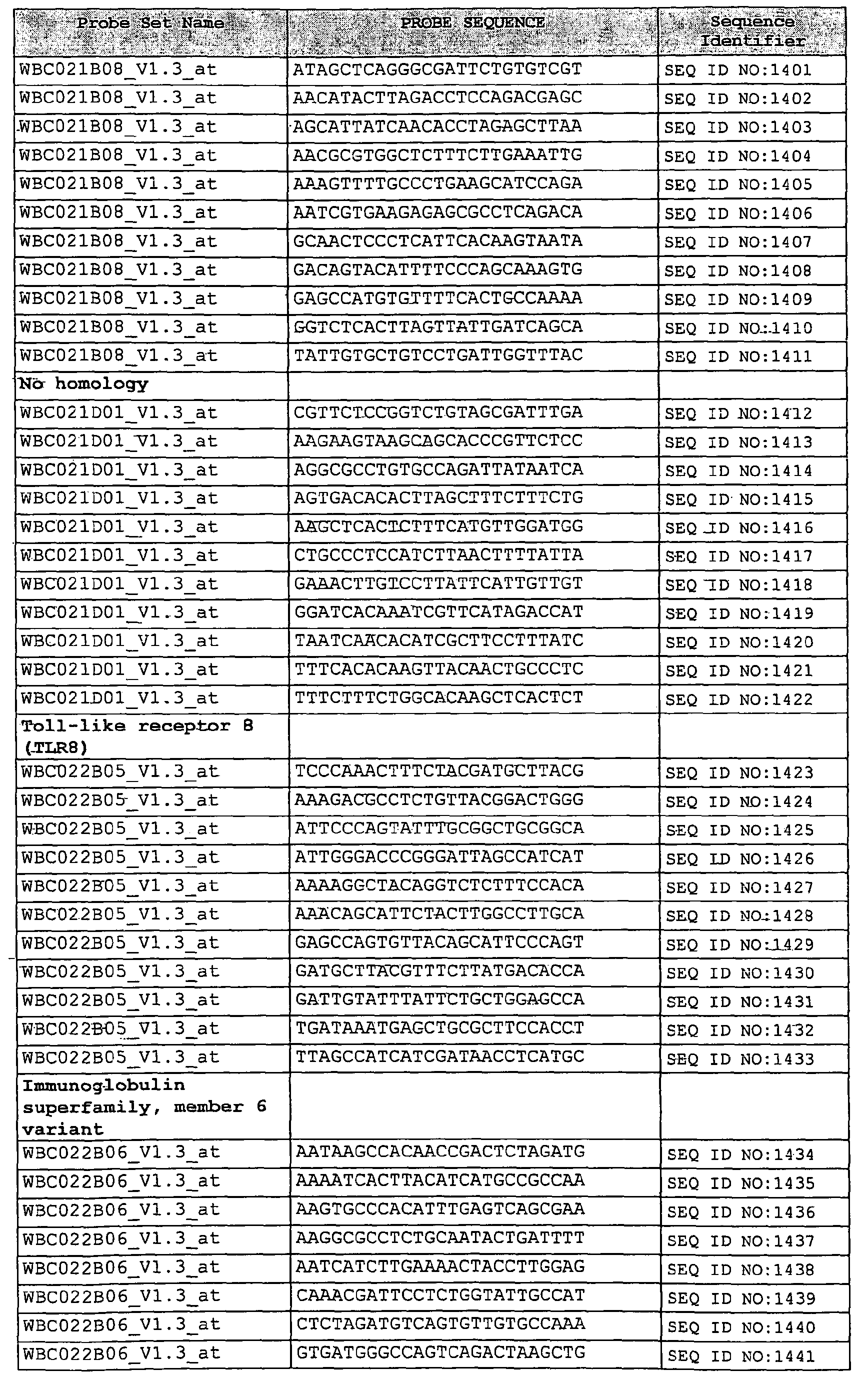

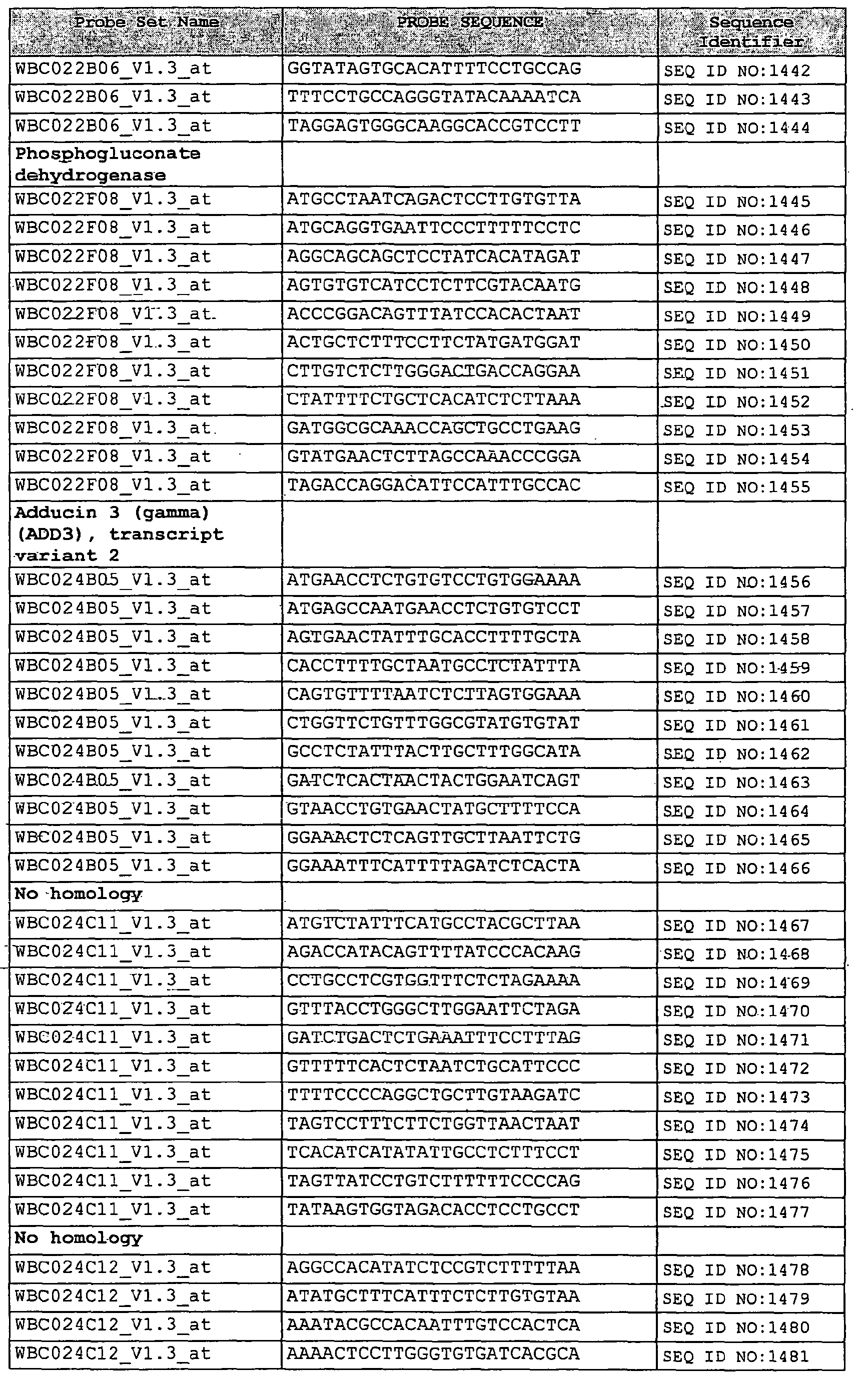

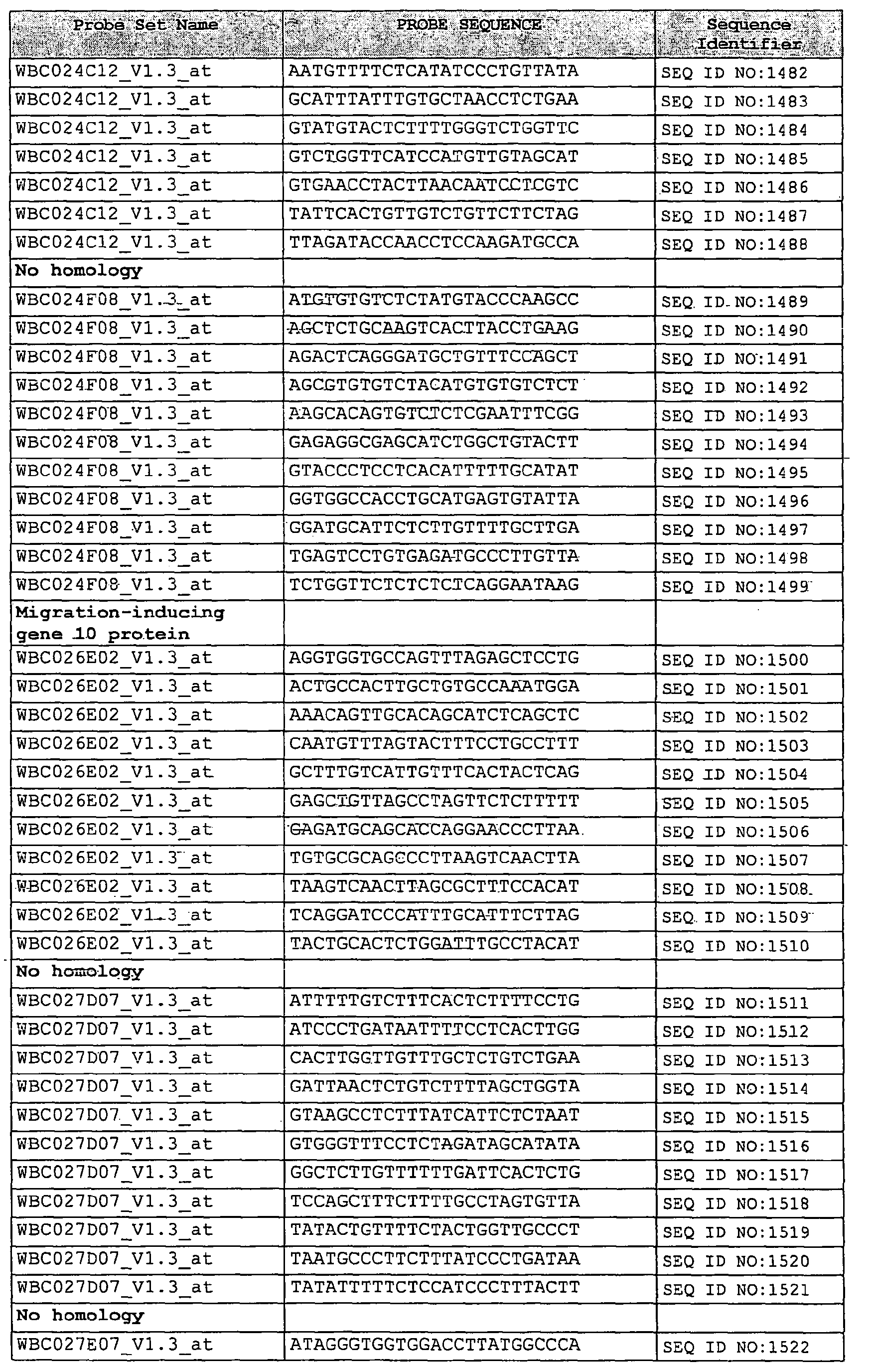

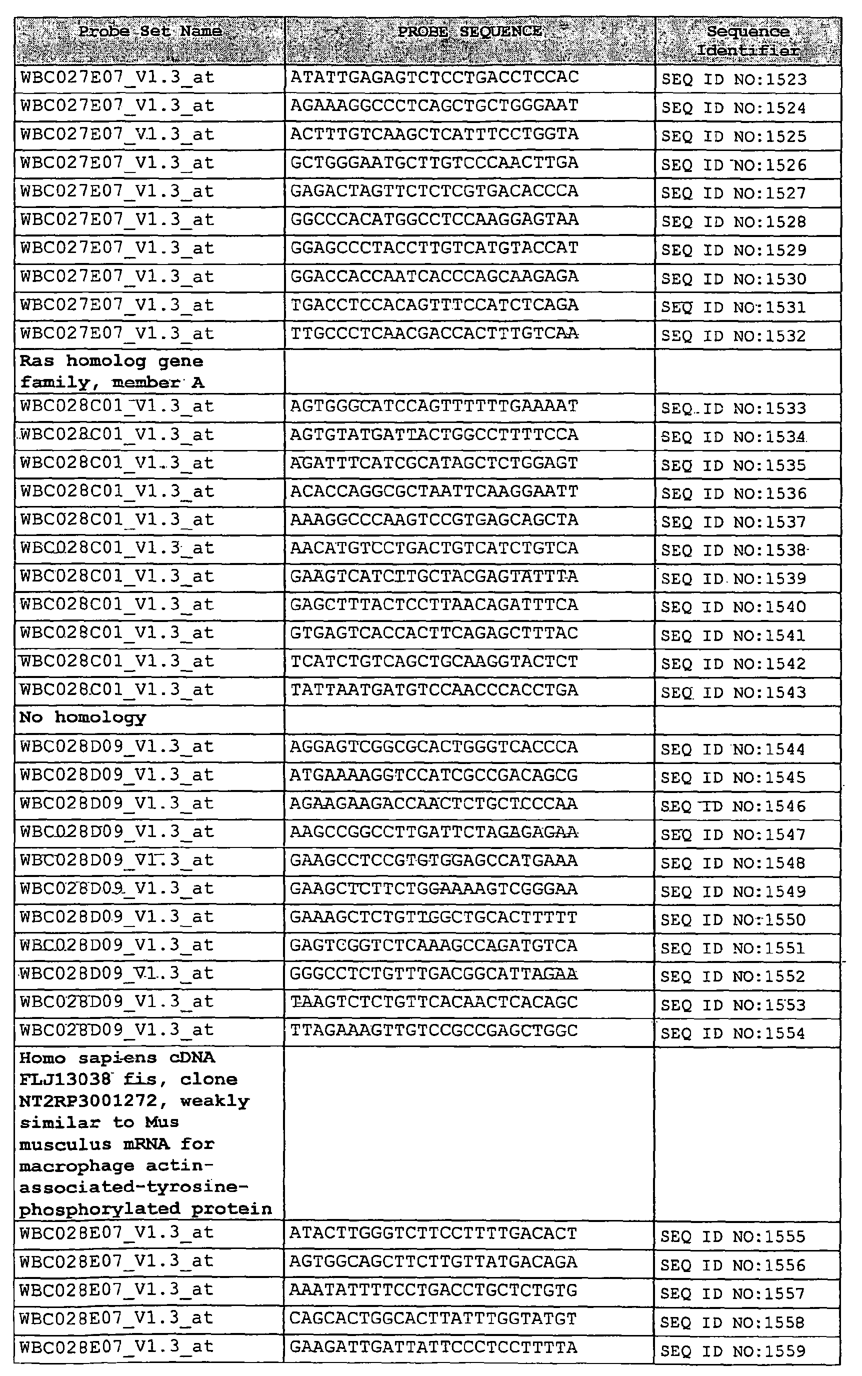

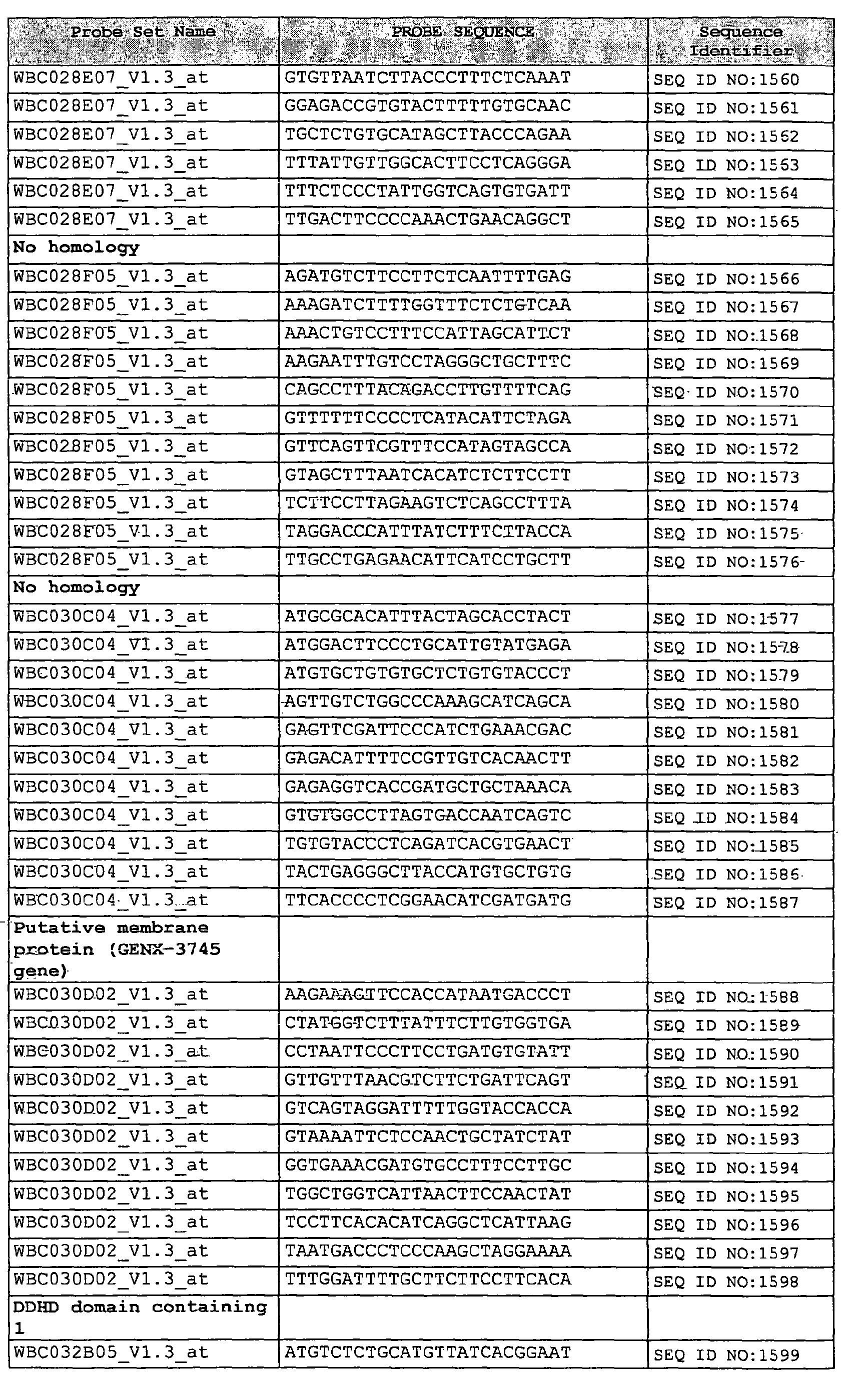

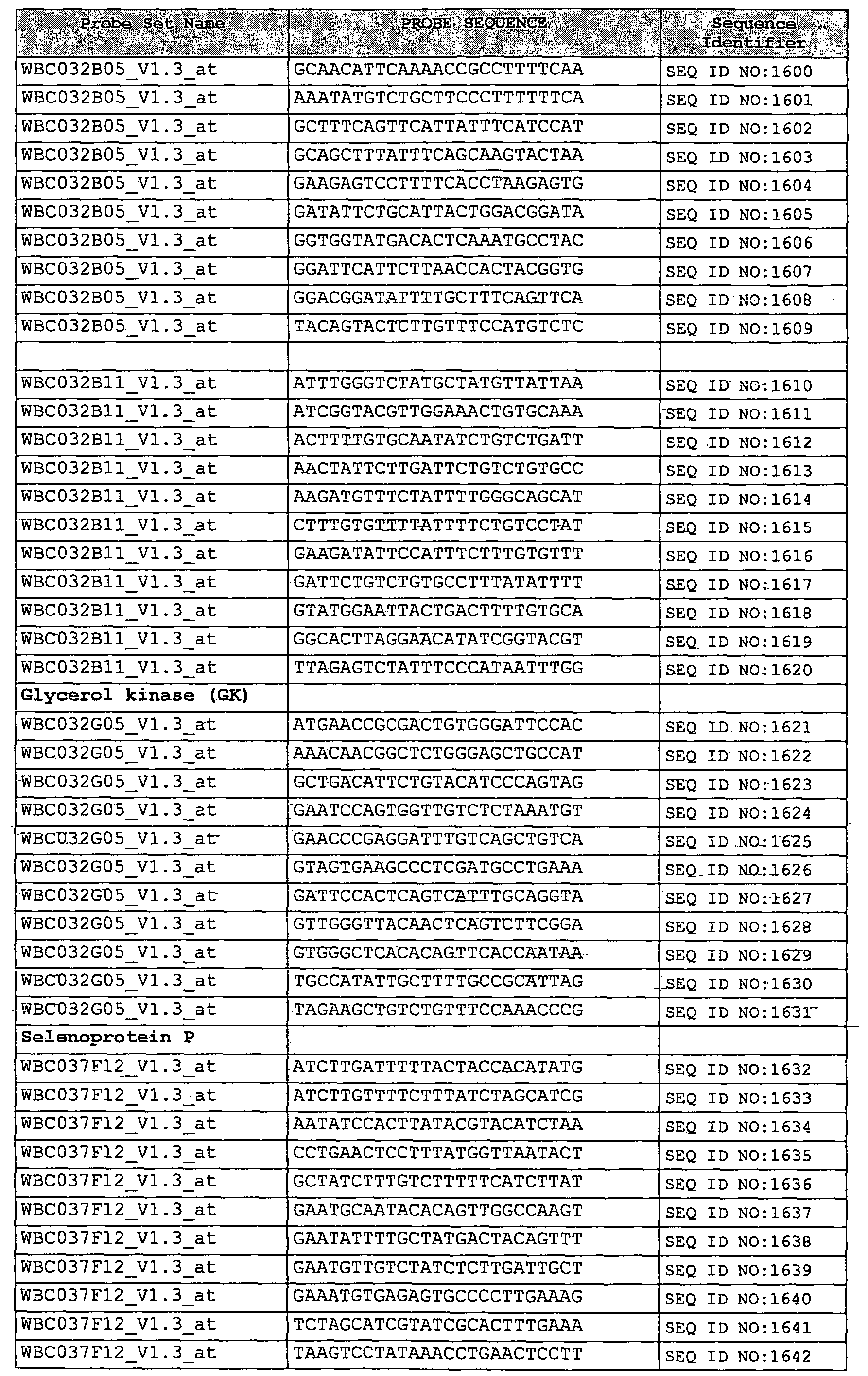

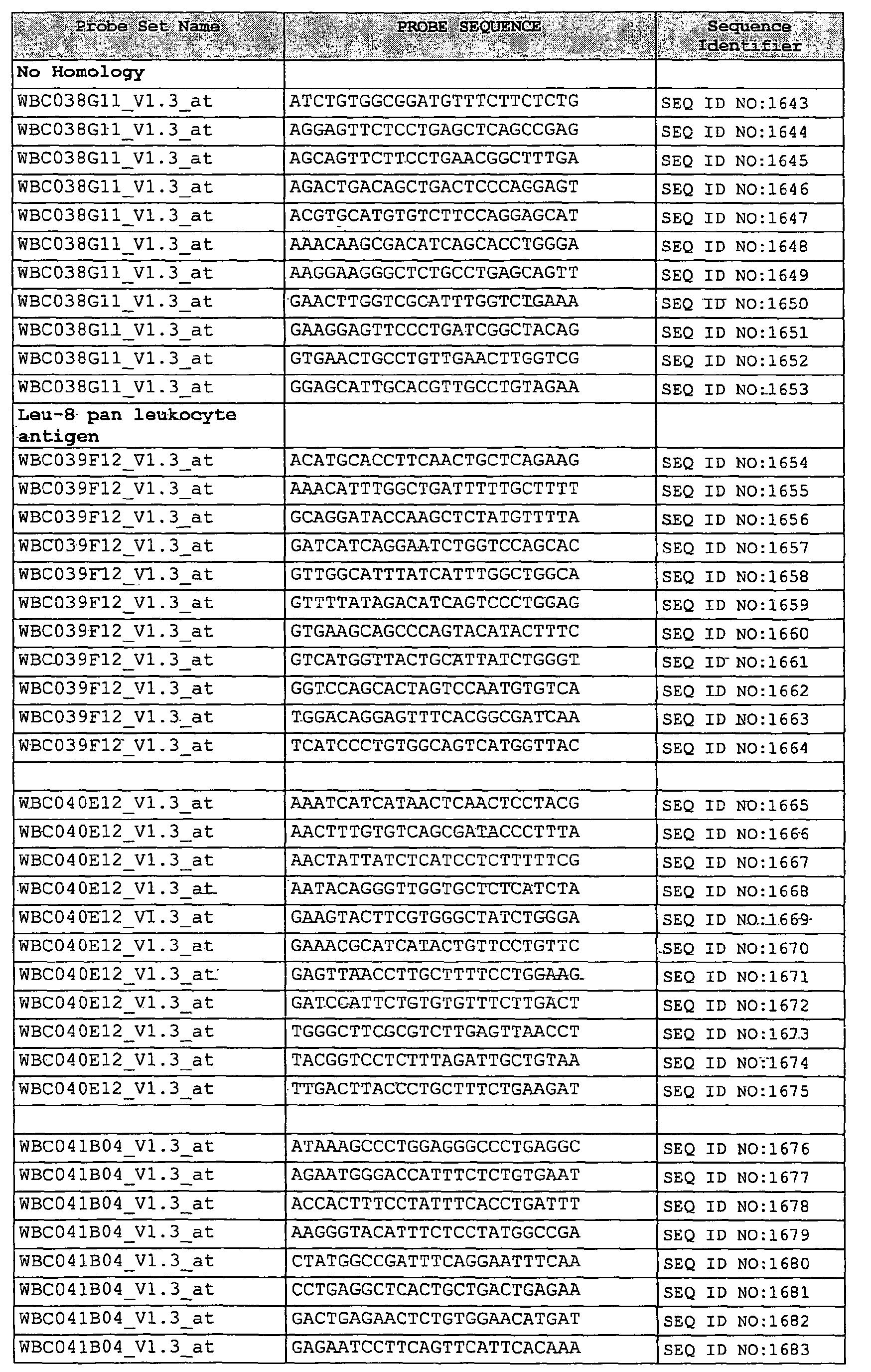

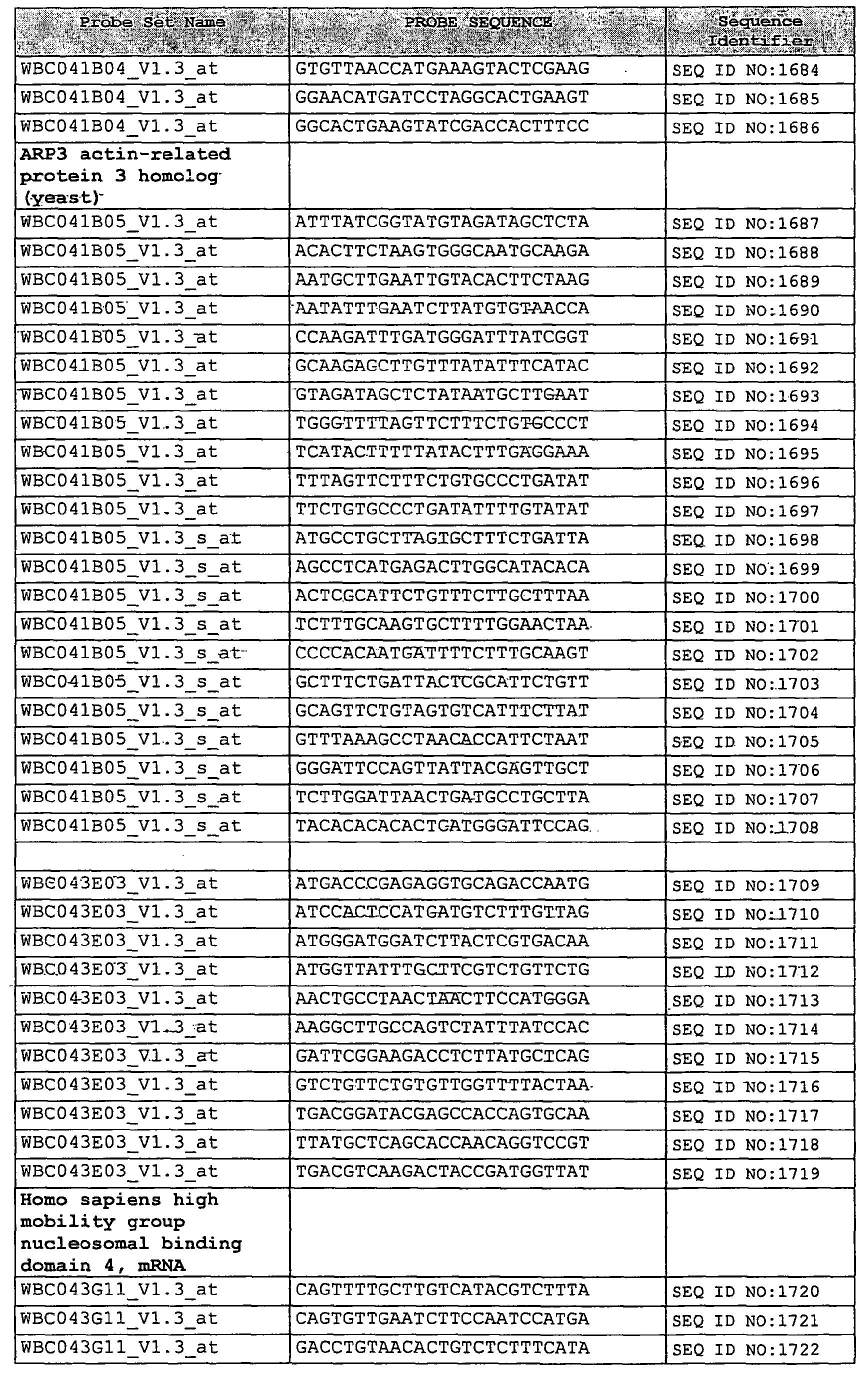

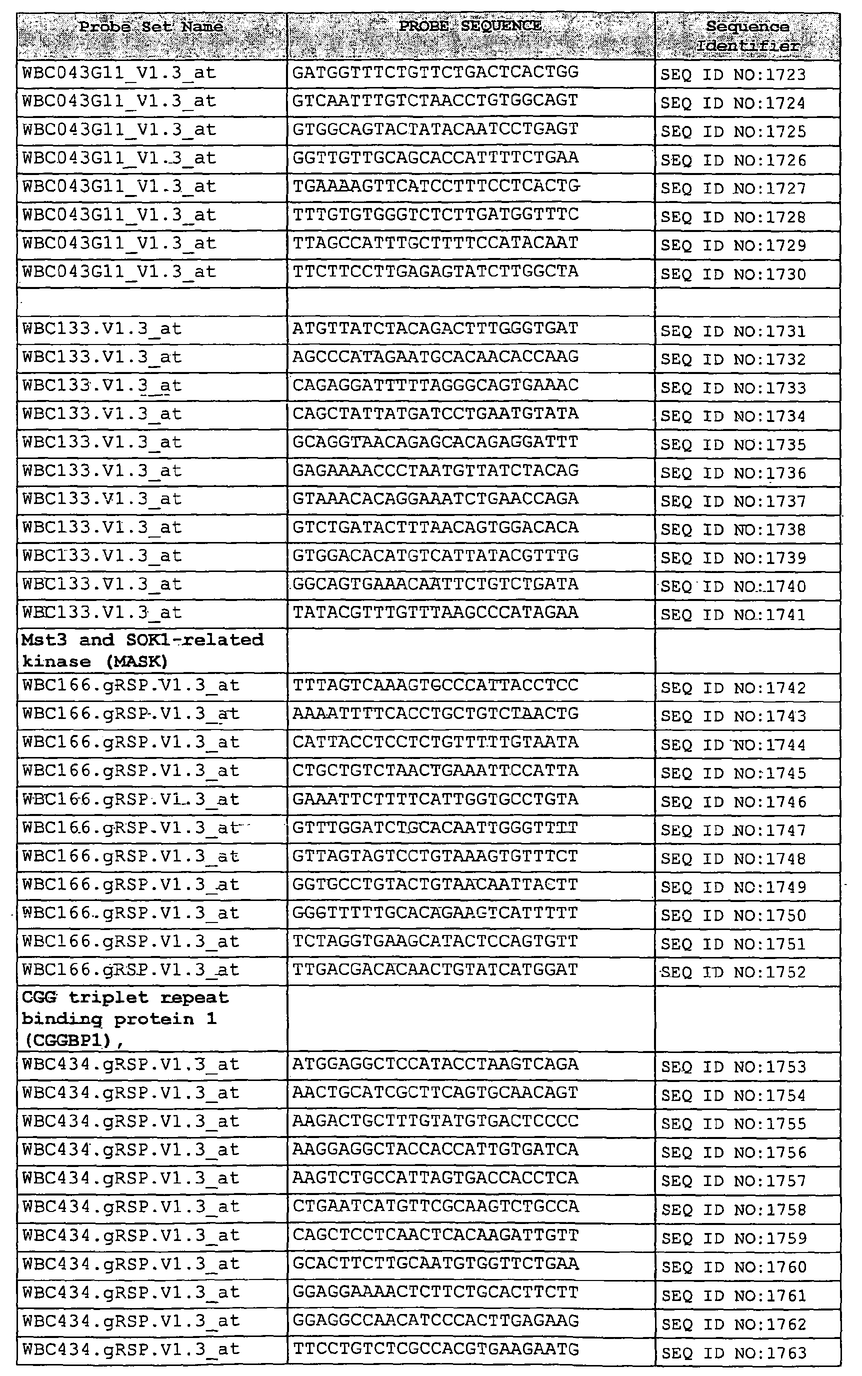

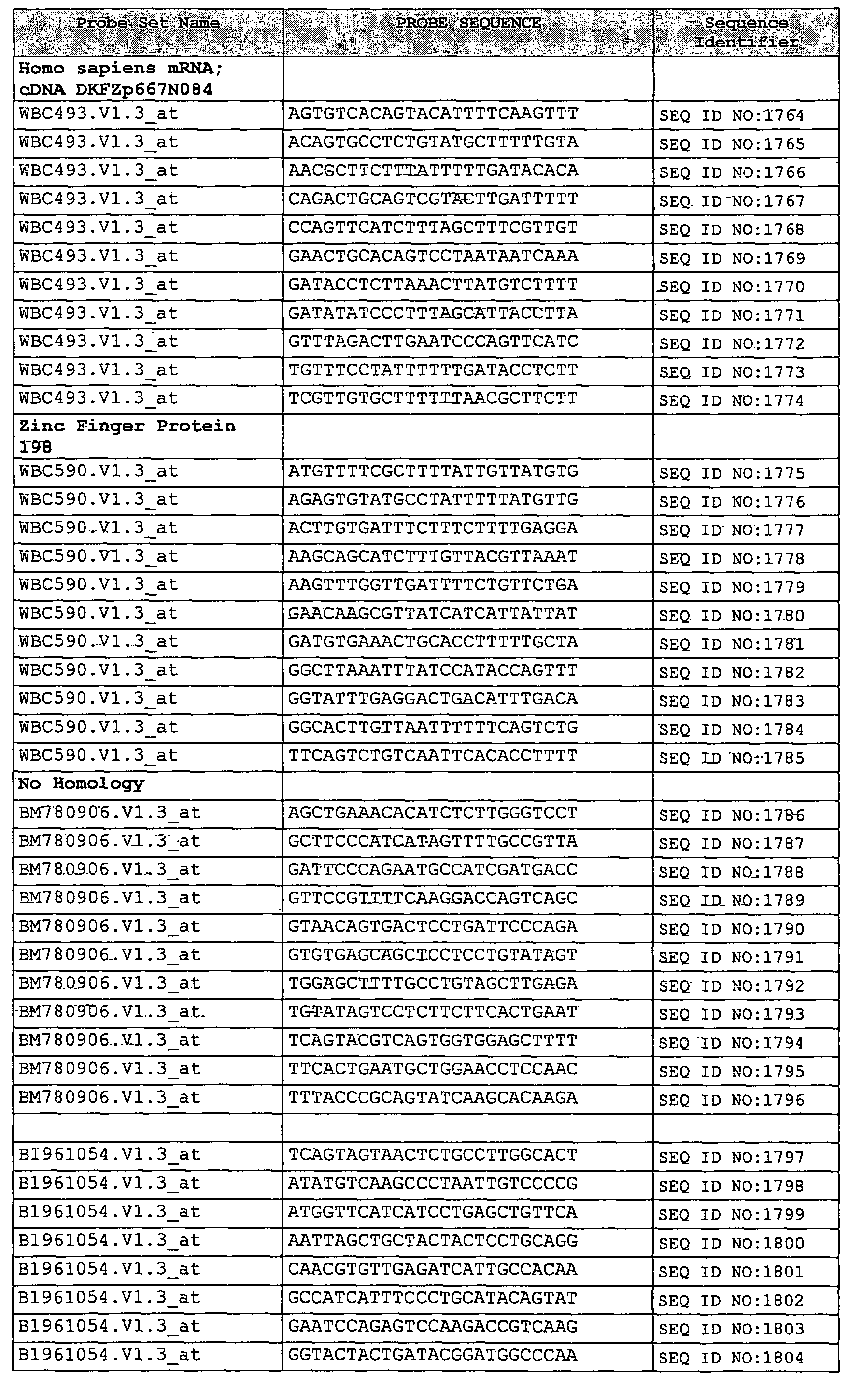

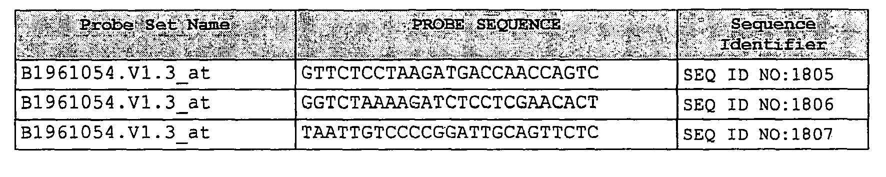

**TABLE 3**

| **AMINO ACID SUB-CLASSIFICATION** | |
|---|---|
| **Sub-classes** | **Amino acids** |
| Acidic | Aspartic acid, Glutamic acid |
| Basic | Noncyclic: Arginine, Lysine; Cyclic: Histidine |
| Charged | Aspartic acid, Glutamic acid, Arginine, Lysine, Histidine |
| Small | Glycine, Serine, Alanine, Threonine, Proline |
| Polar/neutral | Asparagine, Histidine, Glutamine, Cysteine, Serine, Threonine |
| Polar/large | Asparagine, Glutamine |
| Hydrophobic | Tyrosine, Valine, Isoleucine, Leucine, Methionine, Phenylalanine, Tryptophan |
| Aromatic | Tryptophan, Tyrosine, Phenylalanine |
| Residues that influence chain orientation | Glycine and Proline |

**TABLE 4**

| **EXEMPLARY AND PREFERRED AMINO ACID SUBSTITUTIONS** | | |
|---|---|---|
| **Origonal Residue** | **Exemplary Substitutions** | **Preferred Substitutions** |
| Ala | Val, Leu, Ile | Val |
| Arg | Lys, Gln, Asn | Lys |
| Asn | Gln, His, Lys, Arg | Gln |
| Asp | Glu | Glu |
| Cys | Ser | Ser |
| Gln | Asn, His, Lys, | Asn |
| Glu | Asp, Lys | Asp |
| Gly | Pro | Pro |
| His | Asn, Gln, Lys, Arg | Arg |
| Ile | Leu, Val, Met, Ala, Phe, Norleu | Leu |
| Leu | Norleu, Ile, Val, Met, Ala, Phe | Ile |
| Lys | Arg, Gln, Asn | Arg- |
| Met | Leu, Ile, Phe | Leu |
| Phe | Leu, Val, Ile, Ala | Leu |
| Pro | Gly | Gly |
| Ser | Thr | Thr |
| Thr | Ser | Ser |
| Trp | Tyr | Tyr |
| Tyr | Trp, Phe, Thr; Ser | Phe |
| Val | Ile, Leu, Met,-Phe, Ala, Norleu | Leu |

**TABLE 5**

| **PRIORITY RANKING OF GENES BASED ON P VALUES** | | | | | | | | | | | | | | |
|---|---|---|---|---|---|---|---|---|---|---|---|---|---|---|
| **Gene Name** | **Day** | **P. Value** | **Day** | **P. Value** | **Day** | **P. Value** | **Day** | **P. Value** | **Day** | **P. Value** | **Day** | **P. Value** | **Day** | **P. Value** |
| BM734889 | 0 | 5.62E-020 | 2 | 1.14E-09 | 4 | 7.81E-07 | 7 | 6.7E-09 | | | | | | |
| WBC005D02 | 0 | 8.19E-020 | 2 | 1.32E-12 | 4 | 1.59E-06 | 7 | 5.94E-05 | | | | | | |
| BM734862 | 0 | 1.13E-13 | 2 | 4.21E-06 | 4 | 0.047483 | 7 | 8.48E-06 | | | | | | |
| B1961941 | 0 | 1.45E-13 | 2 | 1.75E-12 | 4 | 7.44E-05 | 7 | 4.09E-08 | | | | | | |
| WBC001C12 | 9 | 3.77E-12 | 11 | 1.84E-05 | 14 | 0.000645 | | | | | | | | |
| BM735487 | 0 | 4E-12 | 2 | 1.07E-10 | 4 | 3.01E-05 | 7 | 1.27E-14 | | | | | | |
| BM734722 | 0 | 4.27E-11 | 2 | 5.64E-10 | 4 | 3.22E-07 | 7 | 4.34E-11 | 9 | 0.005597 | 11 | 0.007828 | | |
| WBC008F06 | 9 | 8.8E-10 | 11 | 6.43E-07 | 14 | 0.037099 | | | | | | | | |
| B1961620 | 0 | 3.23E-09 | 2 | 1.46E-05 | 4 | 0.002125 | 7 | 6.48E-09 | 9 | 0.000103 | 11 | 0.012906 | 14 | 0.00011 |
| BM735585 | 0 | 5.47E-09 | 2 | 6.71E-10 | 4 | 0.000125 | 7 | 2.85E-10 | | | | | | |
| WBC022F08 | 0 | 5.61E-09 | 2 | 5.81E-12 | 7 | 0.000304 | | | | | | | | |
| BM735573 | 0 | 7.07E-09 | 2 | 2.72E-07 | 4 | 0.001579 | | | | | | | | |
| B1961682 | 0 | 7.71E-09 | 2 | 0.024969 | 7 | 0.000375 | 14 | 0.005519 | | | | | | |
| WBC021B08 | 2 | 9.14E-09 | 7 | 1.24E-07 | 9 | 2.61E-09 | 11 | 1.81E-06 | | | | | | |
| WBC032G05 | 0 | 1.38E-08 | 2 | 3.35E-05 | 7 | 0.000114 | 11 | 0.008059 | | | | | | |
| BM781178 | 0 | 1.38E-08 | 2 | 1.35E-05 | 4 | 0.012404 | | | | | | | | |
| gi576646 | 0 | 1.87E-08 | 2 | 9.73E-06 | 4 | 0.000899 | 7 | 1.12E-06 | 9 | 9.62E-07 | 14 | 5.64E-05 | | |
| GI1592834 | 0 | 2.53E-08 | 2 | 7.16E-08 | 7 | 0.000149 | | | | | | | | |
| B1961443 | 0 | 3.82E-08 | 7 | 0.000109 | 9 | 0.021125 | 14 | 0.021335 | | | | | | |
| WBC030C04 | 0 | 4.93E-08 | 2 | 3.01E-10 | 4 | 1.22E-09 | 7 | 7.54E-12 | 9 | 3.84E-05 | 14 | 0.035821 | | |
| BM781334 | 0 | 6.32E-08 | 7 | 0.000608 | 9 | 0.027236 | 14 | 0.033821 | | | | | | |
| B1961885 | 0 | 6.83E-08 | 2 | 4.67E-06 | 7 | 0.000802 | | | | | | | | |
| BM781174 | 0 | 8.96E-08 | 2 | 0.000313 | 4 | 0.003936 | 7 | 0.033205 | | | | | | |
| WBC434 | 2 | 1.08E-07 | 4 | 0.005926 | 7 | 2.94E-11 | 9 | 3.71E-08 | 11 | 0.001727 | | | | |
| B1961671 | 0 | 1.14E-07 | 2 | 1.51E-05 | 4 | 0.000753 | 7 | 7.49E-08 | 9 | 4.37E-05 | 11 | 0.02579 | 14 | 3.46E-05 |
| WBC024F08 | 2 | 1.78E-07 | 7 | 3.09E-08 | 9 | 0.000111 | 11 | 0.00276 | 17 | 0.021939 | | | | |
| WBC007G12 | ' 0 | 2.09E-07 | 2 | 0.022205 | 7 | 0.003859 | | | | | | | | |
| WBC001F11 | 9 | 2.15E-07 | 11 | 6.5E-05 | 14 | 0.001586 | | | | | | | | |
| WBC003D11 | 2 | 2.81E-07 | 7 | 0.000171 | 9 | 0.009387 | | | | | | | | |
| WBC022B05 | 2 | 5.27E-07 | 7 | 1.65E-07 | 9 | 0.001287 | | | | | | | | |
| BM780906 | 0 | 6.11E-07 | 2 | 2.93E-06 | 7 | 0.000897 | | | | | | | | |
| WBC012F07 | 0 | 8.91E-07 | 2 | 3.32E-07 | 7 | 1.59E-06 | | | | | | | | |
| BM734865 | 0 | 9.12E-07 | 2 | 1.05E-05 | 7 | 7.16E-06 | 9 | 2.22E-05 | 11 | 0.00057 | 14 | 0.046599 | | |
| WBC014G08 | 9 | 1.04E-06 | 11 | 0.006248 | 14 | 0.001349 | | | | | | | | |
| WBC028C01 | 0 | 1.15E-06 | 2 | 0.002318 | 7 | 0.008866 | | | | | | | | |
| WBC013E10 | 2 | 1.24E-06 | 4 | 0.03163 | 7 | 0.000149 | 9 | 0.014568 | | | | | | |
| WBC009B10 | 2 | 1.55E-06 | 7 | 0.008422 | 9 | 0.000196 | | | | | | | | |
| BM734654 | 0 | 1.57E-06 | 2 | 4.23E-05 | 4 | 0.002656 | 7 | 1.42E-05 | | | | | | |
| BM735536 | 0 | 1.97E-06 | 2 | 2.54E-12 | 4 | 6.46E-09 | 7 | 7.41E-14 | 9 | 2.01E-11 | 11 | 0.000125 | | |
| WBC003F02 | 7 | 2.42E-06 | 9 | 5.83E-06 | 11 | 2.53E-06 | | | | | | | | |
| WBC012E07 | 0 | 3.05E-06 | 4 | 0.018001 | 7 | 4.76E-07 | 9 | 4.2E-06 | 14 | 0.000272 | | | | |
| WBC014H06 | 2 | 3.33E-06 | 7 | 1.81E-08 | 9 | 5.88E-09 | 11 | 0.002383 | | | | | | |
| BM735576 | 0 | 3.53E-06 | 2 | 0.021658 | 7 | 2.73E-05 | 9 | 0.00685 | | | | | | |
| BM735352 | 2 | 4.38E-06 | 7 | 4.37E-06 | 9 | 0.005436 | 11 | 0.039799 | | | | | | |
| WBC007A09 | 0 | 6.08E-06 | 2 | 1.2E-06 | 7 | 9.74E-06 | 9 | 0.000112 | 11 | 0.000195 | | | | |
| BM735457 | 2 | 6.8E-06 | 7 | 6.34E-05 | 9 | 1.91E-08 | | | | | | | | |
| WBC005F10 | 0 | 8.6E-06 | 2 | 2.42E-09 | 7 | 1.08E-05 | | | | | | | | |
| WBC020B04 | 0 | 8.98E-06 | 2 | 0.00137 | 7 | 0.022142 | | | | | | | | |
| WBC028E07 | 2 | 1.14E-05 | 7 | 0.000466 | 9 | 0.001507 | 11 | 9.31E-06 | | | | | | |
| BM735534 | 0 | 1.21E-05 | 2 | 4.54E-08 | 4 | 0.03855 | 7 | 3.37E-10 | 9 | 0.045945 | | | | |
| WBC038G11 | 0 | 1.45E-05 | 2 | 5.1E-05 | 7 | 0.000481 | | | | | | | | |
| B1961711 | 0 | 1.62E-05 | 2 | 0.044085 | 7 | 1.69E-05 | | | | | | | | |
| B1961637 | 2 | 1.66E-05 | 7 | 6.29E-08 | 9 | 0.00037 | 11 | 0.007214 | | | | | | |
| WBC024C12 | 0 | 1.85E-05 | 2 | 0.008355 | 7 | 9.02E-06 | | | | | | | | |
| BM735450 | 0 | 1.92E-05 | 2 | 0.009622 | 4 | 0.003305 | 7 | 3.46E-05 | | | | | | |
| WBC006H06 | 7 | 1.97E-05 | 9 | 1.55E-05 | 14 | 0.005638 | | | | | | | | |
| WBC013H03 | 0 | 2.2E-05 | 2 | 3.3E-05 | 7 | 0.000509 | | | | | | | | |
| WBC030D02 | 7 | 2.28E-05 | 9 | 3.74E-08 | 11 | 4.8E-05 | 14 | 0.000181 | | | | | | |
| WBC006E03 | 9 | 2.92E-05 | 11 | 0.004223 | 14 | 6.36E-05 | | | | | | | | |
| WBC022B06 | 2 | 3.06E-05 | 7 | 6.19E-06 | 11 | 0.026743 | | | | | | | | |
| WBC028F05 | 0 | 3.15E-05 | 2 | 7.49E-07 | 4 | 0.003321 | 7 | 1.68E-09 | 9 | 7.18E-07 | 11 | 7.49E-05 | | |
| WBC009E12 | 9 | 3.25E-05 | 11 | 0.004629 | 14 | 0.0005 | | | | | | | | |
| WBC003H01 | 0 | 3.31E-05 | 7 | 0.000992 | | 0.014301 | | | | | | | | |
| WBC026E02 | 0 | 3.43E-05 | 2 | 1.29E-07 | 7 | 0.021843 | | | | | | | | |
| WBC018D05 | 7 | 3.47E-05 | 9 | 0.027649 | 14 | 0.008898 | | | | | | | | |
| BM735166 | 0 | 3.88E-05 | 4 | 0.00401 | 7 | 0.000304 | 14 | 0.000601 | | | | | | |
| WBC028D09 | 0 | 4.84E-05 | 4 | 0.001241 | 7 | 6.41E-08 | 9 | 0.000202 | | | | | | |
| BM734531 | 0 | 4.96E-05 | 2 | 5.36E-08 | 7 | 0.000196 | | | | | | | | |
| WBC013A09 | 9 | 5.04E-05 | 11 | 6.13E-05 | 14 | 0.006365 | | | | | | | | |
| WBC010F04 | 4 | 5.04E-05 | 7 | 4.2E-05 | 9 | 1.68E-08 | 14 | 0.035866 | | | | | | |
| B 1961109 | 0 | 5.05E-05 | 2 | 1.51E-05 | 4 | 0.03924 | 7 | 1.31E-05 | 9 | 0.000187 | 11 | 8.7E-05 | 14 | 0.046035 |
| WBC001F08 | 7 | 5.49E-05 | 9 | 2.05E-05 | 11 | 0.006456 | 14 | 2.88E-06 | | | | | | |
| BM734457 | 7 | 5.65E-05 | 9 | 2.45E-06 | 14 | 0.004615 | | | | | | | | |
| WBC166 | 9 | 6.43E-05 | 11 | 0.000632 | 14 | 0.000648 | | | | | | | | |
| WBC024B05 | 7 | 8.41E-05 | 9 | 3.42E-09 | 11 | 2.11E-07 | 14 | 0.000172 | | | | | | |
| WBC004D07 | 9 | 8.87E-05 | 11 | 0.000708 | 14 | 0.002727 | | | | | | | | |
| WBC032B05 | 7 | 9.38E-05 | 9 | 1.47E-05 | 11 | 1.22E-05 | 14 | 1.97E-06 | | | | | | |
| WBC021D01 | 7 | 0.000118 | 9 | 0.000841 | 14 | 0.001455 | | | | | | | | |
| WBC008F12 | 0 | 0.000119 | 2 | 2.32E-08 | 4 | 0.002886 | 7 | 1.85E-08 | | | | | | |
| WBC027E07 | 0 | 0.000152 | 2 | 0.009696 | 7 | 0.003137 | | | | | | | | |
| BM735102 | 0 | 0.000152 | 7 | 0.000595 | 9 | 0.034995 | | | | | | | | |
| WBC041B05 | 9 | 0.000158 | 11 | 0.025083 | 14 | 0.022571 | | | | | | | | |
| WBC039F12 | 0 | 0.00019 | 2 | 3.68E-05 | 7 | 5.79E-09 | | | | | | | | |
| BM735286 | 0 | 0.000203 | 2 | 2.5E-05 | 4 | 0.015183 | 7 | 9.79E-05 | 11 | 0.618883 | | | | |
| WBC493 | 7 | 0.000215 | 9 | 0.000323 | 11 | 0.002796 | 14 | 6.25E-05 | | | | | | |
| WBC001C11 | 9 | 0.000252 | 11 | 0.002114 | 14 | 0.027481 | | | | | | | | |
| WBC019B05 | 0 | 0.000256 | 2 | 5.5E-05 | 4 | 0.000479 | 7 | 1.87E-12 | 9 | 8.09E-08 | 11 | 0.000637 | 14 | 1.43E-05 |
| G19717252 | 2 | 0.000267 | 7 | 0.001244 | 11 | 0.005664 | | | | | | | | |
| WBC043G11 | 7 | 0.000268 | 9 | 0.003323 | 14 | 0.003005 | | | | | | | | |
| Foe545 | 9 | 0.00027 | 11 | 0.008383 | 14 | 0.000604 | | | | | | | | |
| BM735519 | 0 | 0.000272 | 2 | 9.28E-07 | 7 | 0.001646 | | | | | | | | |
| BM735409 | 0 | 0.0003 | 4 | 0.005135 | 7 | 3.7E-09 | | | | | | | | |
| WBC004C03 | 0 | 0.0003 | 2 | 0.000373 | 7 | 1.14E-06 | 9 | 0.015975 | 14 | 0.008483 | | | | |
| WBC001A07 | 9 | 0.000309 | 11 | 0.031959 | 14 | 0.011618 | | | | | | | | |
| BM734719 | 2 | 0.000313 | 4 | 1.5E-05 | 7 | 2.69E-06 | 9 | 0.00355 | 11 | 0.012181 | | | | |
| WBC018B01 | 7 | 0.000344 | 9 | 0.048555 | 14 | 0.015517 | | | | | | | | |
| Foe1072 | 7 | 0.000351 | 9 | 1.38E-05 | 14 | 0.028866 | | | | | | | | |
| B1961494 | 7 | 0.000382 | 11 | 1.52E-06 | 14 | 9.11E-06 | | | | | | | | |
| WBC007G03 | 9 | 0.000385 | 11 | 0.026939 | 14 | 0.013456 | | | | | | | | |
| WBC004E04 | 7 | 0.000412 | 9 | 2.33E-09 | 11 | 7.14E-07 | 14 | 2.53E-05 | | | | | | |
| BM735545 | 0 | 0.000476 | 2 | 1.02E-06 | 4 | 1.1E-05 | 7 | 0.001412 | 11 | 0.007853 | 14 | 0.036358 | | |
| BM735167 | 2 | 0.000484 | 7 | 9.77E-05 | 9 | 6.49E-13 | 11 | 1.52E-05 | | | | | | |
| B1960933 | 7 | 0.0005016 | 9 | 0.007447 | 11 | 0.048364 | | | | | | | | |
| BM735441 | 0 | 0.000541 | 2 | 0.00586 | 7 | 0.000179 | | | | | | | | |
| Foe1060 | 9 | 0.000551 | 11 | 0.010201 | 14 | 0.02297 | | | | | | | | |
| WBC590 | 9 | 0.000596 | 11 | 0.02401 | 14 | 0.001841 | | | | | | | | |
| WBC001H09 | 7 | 0.00061 | 9 | 0.021894 | 14 | 0.022122 | | | | | | | | |
| WBC027D07 | 0 | 0.000667 | 2 | 1.12E-06 | 4 | 3E-05 | 7 | 8.55E-015 | 9 | 2.94E-06 | 11 | 0.001128 | 14 | 2.75E-07 |
| WBC013C03 | 9 | 0.000778 | 11 | 0.000615 | 14 | 0.002414 | | | | | | | | |
| WBC024C11 | 2 | 0.000799 | 4 | 4.91E-06 | 7 | 4E-10 | 9 | 1.81E-06 | 14 | 8.89E-06 | | | | |
| WBC016A12 | 0 | 0.000806 | 2 | 0.005596 | 4 | 0.026774 | 7 | 0.000604 | 9 | 0.002168 | | | | |
| BM781186 | 0 | 0.000863 | 2 | 0.000524 | 4 | 0.002977 | 7 | 0.016418 | | | | | | |
| WBC012G02 | 7 | 0.000863 | 9 | 0.000161 | 14 | 0.000843 | | | | | | | | |
| BM781417 | 0 | 0.000961 | 7 | 0.0002 | 9 | 0.002666 | 11 | 0.012894 | 14 | 0.004028 | | | | |

**TABLE 6**

| **GENE PRIORITY ORDER BASED ON T VALUE** | | | | | |
|---|---|---|---|---|---|
| **Gene Name** | **Day** | **M** | **t** | **P.Value** | **B** |
| WBC008F06_V1.3_at | 9 | -0.53075 | -7.92738 | 8.8E-10 | 21.27623 |
| WBC007G12_V1.3_at | 0 | -0.34772 | -6.96751 | 2.09E-07 | 16.05095 |
| WBC001F11_V1.3_at | 9 | -0.53926 | -6.96303 | 2.15E-07 | 16.03718 |
| WBC014G08_V1.3_at | 9 | -0.50112 | -6.67446 | 1.04E-06 | 14.53887 |
| WBC013E10_V1.3_at | 2 | -0.44979 | -6.64137 | 1.24E-06 | 14.37086 |
| WBC012E07_V1.3_at | 0 | -0.46294 | -6.47256 | 3.05E-06 | 13.50699 |
| WBC024C12_V1.3_at | 0 | -0.44376 | -6.12738 | 1.8-5E-05 | 11.79847 |
| WBC006H06_V1.3_at | 7 | -0.42431 | -611448 | 1.97E-05 | 11.73829 |
| WBC030D02_V1.3_at | 7 | -0.47564 | -6.08505 | 2.28E-05 | 11.595508 |
| WBC006E03_V1.3_at | 9 | -0.48246 | -6.03644 | 2.92E-05 | 11.35935 |
| WBC028F05_V1.3_at | 0 | -0.48118 | -6.02243 | 3.15E-05 | 11.29056 |
| WBC009E12_V1.3_at | 9 | -0.7531 | -6.01545 | 3.25E-05 | 11.25809 |
| WBC018D05_V1.3_at | 7 | -0.51854 | -6.00257 | 3.47E-05 | 11.19618 |
| WBC010F04_V1.3_at | 4 | -0.76635 | -5.9324 | 5.04E-05 | 10.76647 |
| WBC013A09_V1.3_at | 9 | -0.32137 | -5.92857 | 5.04E-05 | 10.84143 |
| WBC001F08_V1.3_at | 7 | -031702 | -5.91162 | 5.49E-05 | 10.76031 |
| WBC166.gRSP.V1.3_at | 9 | -0.70207 | -5.87988 | 6.43E-05 | 10.60961 |
| WBC024B05_V1.3_at | 7 | -0.4822 | -5.82594 | 8.41E-05 | 10.35363 |
| WBC004D07_V1.3_at | 9 | -0.38487 | -5.81519 | 8.87E-05 | 10.30359 |
| WBC032B05_V1.3_at | 7 | -0.42227 | -5.80372 | 9.38E-05 | 10.24879 |
| WBC021D01_V1.3_at | 7 | -0.37385 | -5.75613 | 0.000118 | 10.02519 |
| WBC041B05_V1.3_at | 9 | -0.60661 | -5.69821 | 0.000158 | 9.755808 |
| WBC493.V1.3_at | 7 | -0.75113 | -5.63393 | 0.000215 | 9.456588 |
| WBC001C11_V1.3_s_at | 9 | -0.459 | -5.60169 | 0.000252 | 9.309492 |
| WBC019B05_V1.3_at | 0 | -0.57447 | -5.60118 | 0.060256 | 9.309371 |
| Foe545.V1.3_at | 9 | -0.40775 | -5.58788 | 0.00027 | 9.246056 |
| WBC043G11_V1.3_at | 7 | -0.3362 | -5.58774 | 0.000268 | 9.243826 |
| WBC004C03_1.3_at | 0 | -0.41087 | -5.56794 | 0.0003 | 9.157128 |
| WBC001A07_V1.3_at | 9 | -0.53398. | -5.55969 | 0.000309 | 9.116868 |
| BM734719.V1.3_at | 2 | -0.41143 | -5.55799 | 0.000313 | 9.108264 |
| WBC018B01_V1.3_at | 7 | -0.67086 | -5.53604 | 0.000344 | 9.00706 |
| Foe1072.V1.3_at | 7 | -0.49276 | -5.5316 | 0.000351 | 8.986814 |
| WBC007G03_V1.3_at | 9 | -0.77183 | -5.51378 | 0.000385 | 8.90749 |
| WBC004E04_V1.3_at | 7 | -0.3488 | -5.4981 | 0.000412 | 8.834262 |
| Foe1060.V1.3_at | 9 | -0.4171 | -5.43881 | 0.000551 | 8.568091 |
| WBC590.V1.3_at | 9 | -0.46943 | -5.42207 | 0.000596 | 8.492765 |
| WBC001H09_V1.3_at | 7 | -0.55531 | -5.41491 | 0.00061 | 8.458322 |
| WBC027D07_V1.3_at | 0 | -0.39411 | -5.40118 | 0.000667 | 8.402682 |
| WBC013C03_V1.3_at | 9 | -0.29677 | -5.36572 | 0.000778 | 8.240336 |
| WBC024C11_V1.3_at | 2 | -0.29424 | -5.36158 | 0.000799 | 8.220603 |
| WBC016A12_V1.3_at | 0 | -0.48188 | -5.36119 | 0.000806 | 8.224168 |
| WBC012G02_V1.3_at | 7 | -0.53236 | -5.34123 | 0.000863 | 8.128662 |
| BM781417.V1.3_at | 0 | -0.54661 | -5.32386 | 0.000961 | 8.05831 |
| BM781186.V1.3_at | 0 | 0.607083 | 5.346723 | 0.000863 | 8.159792 |
| BM735441.V1.3_at | 0 | 0.253862 | 5.44514 | 0.000541 | 8.600034 |
| B1960933.V1.3_at | 7 | 0.716823 | 5.454949 | 0.000506 | 8.638769 |
| BM735167.V1.3_at | 2 | 0.14248 | 5.46725 | 0.000484 | 8.69542& |
| BM735545.V1.3_at | 0 | 0.589494 | 5.471981 | 0.000476 | 8.721067 |
| B1961494.V1.3_at | 7 | 0.354654 | 5.513986 | 0.000382 | 8.906528 |
| BM735409.V1.3_at | 0 | 0.269757 | 5.568459 | 0.0003 | 9.159479 |
| BM735519.V1.3_at | 0 | 0.323515 | 5.588238 | 0.000272 | 9.250006 |
| G197172523M_at | 2 | 0.63348 | 5.591229 | 0.000267 | 9.260645 |
| BM735286.V1.3_at | 0 | 0.247435 | 5.648946 | 0.000203 | 9.529213 |
| WBC039F12_V1.3_at | 0 | 0.351171 | 5.66273 | 0.00019 | 9.592888 |
| BM735102.V1.3_at | 0 | 0.356539 | 5.707416 | 0.000152 | 9.800038 |
| WBC027E07_V1.3_at | 0 | 0.290616 | 5.708292 | 0.000152 | 9.804113 |
| WBC008F12_V1.3_at | 0 | 0.269626 | 5.75662 | 0.000119 | 10.02939 |
| BM734457.V1.3_at | 7 | 0.430304 | 5.905849 | 5.65E-05 | 10.73278 |
| B1961109.V1.3_at | -0 | 0.72204 | 5.929105 | 5.05E-05 | 10.84356 |
| BM734531.V1:3_at | 0 | 0.633646 | 5.932699 | 4.96E-05 | 10.86069 |
| WBC028D09_V1.3_at | 0 | 0.2918.09 | 5.937444 | 4.84E-05 | 10.88332 |
| BM735166.V1.3_at | 0 | 0.357974 | 5.981577 | 3.88E-05 | 11.09433 |
| WBC026E02_V1.3_at | 0 | 0.339222 | 6.005673 | 3.43E-05- | 11.20996 |
| WBC003H01_V1.3_at | 0 | 0.398243 | 6.012941 | 3.31E-05 | 11.24489 |
| WBC022B06_V1.3_at | 2 | 0.431945 | 6.027528 | 3.06E-05 | 11.31647 |
| WBC013H03_V1.3_at | 0 | 0.472666 | 6.093231 | 2.2E-05 | 11.6326 |
| BM735450.V1.3_at | 0 | 0.292831 | 6.119368 | 1.92E-05 | 11.75951 |
| B1961637.V1.3_at | 2 | 0.525483 | 6..147262 | 1.66E-05 | 11.89799 |
| B1961711.V1.3_at | 0 | 0.364045 | 6.152405 | 1.62E-05 | 11.92041 |
| WBC038G11_V1.3_at | 0 | 0.409732 | 6.174059 | 1.45E-05 | 12.02617 |
| BM735534.V1.3_at | 0 | 0.372736 | 6.209232 | 1.21E-05 | 12.19843 |
| WBC028E07_V1.3_at | 2 | 0.582508 | 6.220488 | 1.14E-05 | 12.25714 |
| WBC020B04_V1.3_at | 0 | 0.418213 | 6.266942 | 8.98E-06 | 12.48239 |
| WBC005F10_V1.3_at | 0 | 0.472342 | 6.27517 | 8.6E-06 | 12.523 |
| BM735457.V1.3_at | 2 | 0.410421 | 6.319517 | 6.8E-06 | 12.74701 |
| WBC007A09_V1.3_at | 0 | 0.568251 | 6.341687 | 6.08E-06 | 12.85253 |
| BM735352.V1.3_at | 2 | 0.628248 | 6.403566 | 4.38E-06 | 13.16644 |
| BM7358576.V1.3_at | 0 | 0.472577 | 6.445128 | 3.53E-06 | 13.36913 |
| WBC014H06_V1.3_at | 2 | 0.785821 | 6.455537 | 3.33E-06 | 13.42745 |
| WBC003F02_V1.3_at | 7 | 0.367974 | 6.516236 | 2.42E-06 | 13.73495 |
| BM735536.V1.3_at | 0 | 1.024189 | 6.55447 | 1.97E-06 | 13.92057 |
| BM734654.V1.3_at | 0 | 0.802667 | 6.597475 | 1.57E-06 | 14.13893 |
| WBC009B10_V1.3_at | 2 | 0.664414 | 6.599032 | 1.55E-06 | 14.15456 |
| WBC028C01_V1.3_at | 0 | 0.290434 | 6.655002 | 1.15E-06 | 14.43232 |
| BM734865.V1.3_at | 0 | 0.656049 | 6.698282 | 9.12E-07 | 14.65401 |
| WBC012F07_V1.3_at | 0 | 0.584337 | 6.702508 | 8.91E-07 | 14.6757 |
| BM790906.V1.3_at | 0 | 0.416052 | 6.772214 | 6.11E-07 | 15.03457 |
| WBC022B05_V1.3_at | 2 | 0.646312 | 6.799413 | 5.27E-07 | 15.18522 |
| WBC003D11_V1.3_at | 2 | 1.337346 | 6.914252 | 2.81E-07 | 15.78363 |
| WBC024F08_V1.3_at | 2 | 0.518276 | 6.997403 | 1.78E-07 | 16.22033 |
| B1961671.V1.3_at | 0 | 0.453662 | 7.077614 | 1.14E-07 | 16.63083 |
| WBC434.gRSP.V1.3_at | 2 | 0.448914 | 7.087078 | 1.08E-07 | 16.69443 |
| BM781174.V1.3_at | 0 | 0.618002 | 7.120547 | 8.96E-08 | 16.85824 |
| B1961885.V1.3_at | 0 | 0.983887 | 7.169149 | 6.83E-08 | 17.11655 |
| BM781334.V1.3_at | 0 | 0.475758 | 7.183024 | 6.32E-08 | 17.19046 |
| WBC030C04_V1.3_at | 0 | 0.602389 | 7.22709 | 4.93E-08 | 17.42568 |
| B1961443.V1.3_at | 0 | 0.505935 | 7.272533 | 3.82E-08 | 17.66902 |
| G11592834.V1.3_at | 0 | 0:611795 | 7.345617 | 2.53E-08 | 18.06199 |
| gi576646.V1.3_s_at | 0 | 1.280838 | 7.399041 | 1.87E-08 | 18.35048 |
| BM781178_unkn.V1.3_at | 0 | 0.386001 | 7.452231 | 1.38E-08 | 18.63874 |
| WBC032C05_V1.3_at | 0 | 0.596733 | 7.452338 | 1.38E-08 | 18.63932 |
| WBC021B08_V1.3_at | 2 | 0.792947 | 7.524122 | 9.14E-09 | 19.04904 |
| B1961682.V1.3_at | 0 | 0.545432 | 7.553677 | 7.71E-09 | 19.19129 |
| BM735573.V1.3_s_at | 0 | 0.483434 | 7.568776 | 7.07E-09 | 19.27384 |
| WBC022F08_V1.3_at | 0 | 0.553469 | 7.608824 | 5.61E-09 | 19.49316 |
| BM735585.V1.3_at | 0 | 0.402875 | 7.613483 | 5.47E-09 | 19.51871 |
| B1961620.V1.3_at | 0 | 0.584057 | 7.704254 | 3.23E-09 | 20.01797 |
| BM734722.V1.3_at | 0 | 0.736865 | 8.435653 | 4.27E-11 | 24.13395 |
| BM735487.V1.3_at | 0 | 0.445341 | 8.825278 | 4E-12 | 26.38603 |
| WBC001C12_V1.3_at | 9 | 0.76454 | 8.834995 | 3.77E-12 | 26.47277 |
| B1961941.V1.3_at | 0 | 0.478102 | 9.36128 | 1.45E-13 | 29.53961 |
| BM734862.V1.3_at | 0 | 0.727226 | 9.401378 | 1.13E-13 | 29.77781 |
| WBC005D02_V1.3_at | 0 | 0.680964 | 11.60693 | 8.19E-020 | 43.19762 |
| BM734889.V1.3_at | 0 | 1.083673 | 11.66465 | 5.62E-020 | 43.55399 |

| | | | | | |
|---|---|---|---|---|---|
| The priority ranking of genes is based on increasing value of t value for the first day each gene is significant (p<0.001) following stress induction, and for genes that were significant for at least three sampling times. | | | | | |

**TABLE 7**

| **TWO GENES SELECTED** | | | | |
|---|---|---|---|---|
| **Genes** | | **Sensitivity** | **Specificity** | **Success** |
| WBC001F11 | B1961443 | 0.926829268 | 0.802816901 | 0.881443299 |
| WBC030D02 | BM735536 | 0.918699187 | 0.802816901 | 0.87628866 |
| WBC030D02 | BM735536 | 0.918699187 | 0.802816901 | 0.87628866 |
| B1961443 | WBC027D07 | 0.910569106 | 0.816901408 | 0.87628866 |
| B1961443 | WBC030D02 | 0.910569106 | 0.816901408 | 0.87628866 |
| BM735536 | BM734865 | 0.886178862 | 0.816901408 | 0.860824742 |
| BM735536 | B1961494 | 0.894308943 | 0.802816901 | 0.860824742 |
| BM735409 | B1961443 | 0.926829263 | 0.746478873 | 0.860824742 |
| WBC010F04 | WBC003H01 | 0.918699187 | 0.76056338 | 0.860824742 |
| BM734865 | BM735536 | 0.886178862 | 0.816901408 | 0.860824742 |
| WBC001C11 | B1961443 | 0.918699187 | 0.746478873 | 0.855670103 |
| BM735576 | BM735536 | 0.902439024 | 0.774647887 | 0.855670103 |
| B1961443 | WBC030C04 | 0.886178862 | 0.802816901 | 0.855670103 |
| WBC004D07 | B1961443 | 0.894308943 | 0.774647887 | 0.850515464 |
| B1961443 | WBC003D11 | 9.866918699 | 0.816901408 | 0.850515464 |
| B1961443 | WBC004D07 | 0.894308943 | 0.774647887 | 0.850595464 |
| WBC012G02 | WBC028D09 | 0.894308943 | 0.774647887 | 0.850515464 |
| BM735536 | WBC019B05 | 0.886178862 | 0.788732394 | 0.850515464 |
| B1961443 | BM734719 | 0.886178862 | 0.788732394 | 0.850515464 |
| B1961443 | WBC021B08 | 0.886178862 | 0.788732394 | 0.850515464 |

**TABLE 8**

| **THREE GENES SELECTED** | | | | | |
|---|---|---|---|---|---|
| **Genes** | | | **Sensitivity** | **Specificity** | **Success** |
| B1961443 | WBC004E04 | B1961620 | 0.910569106 | 0.873239437 | 0.896907216 |
| B1961443 | BM735441 | BM735536 | 0.943089431 | 0.802816301 | 0.891752577 |
| WBC003H01 | WBC004E04 | BM735536 | 0.93495935 | 0.802816901 | 0.886597938 |
| B1961443 | B1961494 | Foe545 | 0.910569106 | 0.845070423 | 0.886597938 |
| WBC028D09 | B1961443 | BM735487 | 0.918699187 | 0.830985915 | 0.886597938 |
| Foe545 | WBC013E10 | B1961443 | 0.93495935 | 0.802816901 | 0.886597938 |
| WBC027E07 | WBC010F04 | B1961443 | 0.910569106 | 0.830985915 | 0.881443299 |
| B1961108 | WBC013C03 | BM735536 | 0.910569106 | 0.816901408 | 0.87628866 |
| BM735487 | WBC028D09 | WBC021D01 | 0.894308943 | 0.845070423 | 0.87628866 |
| WBC041B05 | WBC028D09 | WBC019B05 | 0.910569106 | 0.816901408 | 0.87628866 |
| WBC030D02 | BM735536 | WBC018D05 | 0.902439024 | 0.816901408 | 0.871134021 |
| B1961443 | WBC03OD02 | WBC012F07 | 0.910569106 | 0.802816901 | 0.871134021 |
| WBC003D11 | B1961620 | B1961443 | 0.894308943 | 0.830985915 | 0.871134021 |
| Foe545 | WBC007G03 | B1961443 | 0.918699187 | 0.788732394 | 0.871134021 |
| WBCD03D11 | B1961443 | WBC590 | 0.894308943 | 0.830985915 | 0.871134021 |
| WBC003D11 | WBCD01C12 | B1961443 | 0.894308943 | 0.816901408 | 0.865979381 |
| B1961443 | BM735585 | WBC001F11 | 0.894308943 | 0.816901408 | 0.865979381 |
| WBC009E12 | BM735536 | BM735167 | 0.894308943 | 0.816901408 | 0.865979381 |
| WBC028E07 | BM735536 | WBC493 | 0.902439024 | 0.802816901 | 0.865979381 |
| B1961682 | B1961443 | WBC493 | 0.910569106 | 0.788232394 | 0.865979381 |

**TABLE 9**

| **FOUR GENES SELECTED** | | | | | | |
|---|---|---|---|---|---|---|
| **Genes** | | | | **Sensitivity** | **Sensitivity** | **Success** |
| B1961443 | WBC019B05 | WBC024C12 | BM735585 | 0.93495935 | 0.85915493 | 0.907216495 |
| WBC006E03 | WBC030C04 | WBC003D11 | B1961443 | 0.926829268 | 0.85915493 | 0.902061856 |
| WBC021D01 | BM735536 | B1961443 | WBC020B04 | 0.93495935 | 0.845070423 | 0.902061856 |
| B1961443 | BM734862 | BM735536 | WBC007G03 | 0.93495935 | 0.845070423 | 0.902061856 |
| BM735536 | B1961671 | WBC038G11 | WBC003H01 | 0.93495935 | 0.830985915 | 0.896907216 |
| WBC027D07 | B1961711 | B1961443 | BM735487 | 0.926829268 | 0.830985915 | 0.891752577 |
| BM734722 | B1961443 | WBC028E07 | WBC030D02 | 0.886178862 | 0.901408451 | 0.891752577 |
| B1961885 | B1961443 | 81961620 | WBC041B05 | 0.910569106 | 0.85915493 | 0.891752577 |
| BM735536 | B1961109 | Foe545 | WBC001F11 | 0.910569106 | 0.845070423 | 0.886597938 |
| B1960933 | B1961885 | B1961443 | WBC012G02 | 0.902439024 | 0.85915493 | 0.886597938 |
| WBC007A09 | WBC166 | WBC028D09 | WBC005F10 | 0.910569106 | 0.845070423 | 0.886597938 |
| B1961443 | BM735457 | WBC030C04 | WBC008F06 | 0.886178862 | 0.873239437 | 0.881443299 |
| WBC024F08 | BM735536 | WBC022B05 | B1961109 | 0.902439024 | 0.845070423 | 0.831443299 |
| WBC019B05 | BM735167 | WBCD08F12 | BM735102 | 0.894308943 | 0.85915493 | 0.881443299 |
| WBC028F05 | WBC003H01 | B1961443 | BM735536 | 0.918699187 | 0.816901408 | 0.881443299 |
| WBC005D02 | BM781174 | WBC028D09 | WBC166 | 0.910569106 | 0.830985915 | 0.881443299 |
| G11592834 | BM735534 | B1961443 | WBC004E04 | 0.902439024 | 0.845070423 | 0.881443299 |
| WBC001F08 | BM734457 | B1961443 | WBC039F12 | 0.902439024 | 0.830985915 | 0.87628866 |
| WBC010F04 | WBC007G03 | BM735102 | B1961443 | 0.918699187 | 0.802816901 | 0.87628866 |
| BM735536 | WBC038G11 | BM781334 | BM734-865 | 0.894308943 | 0.845070423 | 0.87628866 |

**TABLE 10**

| **FIVE GENES SELECTED** | | | | | | | |
|---|---|---|---|---|---|---|---|
| **Genes sensitivity** | | | | | **Sensitivity** | **Specificity** | **Success** |
| Foe1072 | BM735441 | B1961885 | B1961443 | WBC009B10 | 0.926829268 | 0.887323944 | 0.912371134 |
| B1961885 | WBC041B05 | BM735534 | B1961443 | WBC024F08 | 0.93495935 | 0.85915493 | 0.907216495 |
| BM735409 | WBC010F04 | B1961443 | B1960933 | BM734719 | 0.93495935 | 0.85915493 | 0.907216495 |
| WBC019B05 | B1961443 | WBC012E07 | BM735585 | WBC027D07 | 0.93495935 | 0.85915493 | 0.907216495 |
| B1961885 | B1961443 | WBC003D11 | WBC041B05 | WBC006E03 | 0.926829268 | 0.85915493 | 0.902061856 |
| WBC003F02 | B1961443 | BM735167 | WBC032G05 | WBC493 | 0.918699187 | 0.873239437 | 0.902061856 |
| BM734719 | WBC024C11 | WBC010F04 | B1961443 | B1961941 | 0.943089431 | 0.830985915 | 0.902061856 |
| WBC013C03 | BM735536 | WBC032G05 | WBC019B05 | WBC166 | 0.93495935 | 0.845070423 | 0.902061856 |
| BM735487 | WBC027D07 | WBC016A12 | WBC001F11 | B1961443 | 0.926829268 | 0.845070423 | 0.896907216 |
| BM781334 | B1961443 | WBC015B05 | WBC026E02 | WBC032G05 | 0.918699187 | 0.85915493 | 0.896907216 |
| WBC030C04 | BM734889 | WBC001F08 | B1961443 | Foe1060 | 0.918699187 | 0.85915493 | 0.896907216 |
| WBC493 | B1961443 | WBC009E12 | BM735534 | BM734889 | 0.926829268 | 0.845070423 | 0.896907216 |
| BM734719 | WBC003H01 | WBC014G08 | B1961443 | BM735534 | 0.894308943 | 0.901408451 | 0.896907216 |
| BM781334 | BM734889 | BM735536 | BM735534 | WBC013C03 | 0.918699187 | 0.845070423 | 0.891752577 |
| WBC001A07 | BM735487 | WBC030D02 | WBC013A09 | B1961443 | 0.910569106 | 0.85915493 | 0.891752577 |
| WBC016A12 | WBC038G11 | B1961443 | WBC010F04 | WBC004E04 | 0.926829268 | 0.830985915 | 0.891752577 |
| WBC004C03 | 81961443 | WBC027E07 | WBC001F11 | WBC010F04 | 0.910569106 | 0.845070423 | 0.886597938 |
| WBC032G05 | BM735573 | WBC003H01 | WBC004C03 | Foe545 | 0.926829268 | 0.816901408 | 0.886597938 |
| BM735636 | B1961682 | B1961494 | WBC009E12 | WBC021B08 | 0.918699187 | 0.830985915 | 0.886597938 |
| B1961443 | WBC013C03 | WBC030D62 | BM736467 | BM735534 | 0.902439024 | 0.85915493 | 0.886597938 |

**TABLE 11**

| **SIX GENES SELECTED** | | | | | | | | |
|---|---|---|---|---|---|---|---|---|
| **Gene** | | | | | | **Sensitivity** | **Specificity** | **Success** |
| WBC006H06 | B1961443 | BM735536 | WBC019B05 | WBC007G03 | WBC009E12 | 0.951219512 | 0.873239437 | 0.922680412 |
| WBC013C03 | BM781334 | WBC032G05 | BM735536 | WBC043G11 | WBC010F04 | 0.951219512 | 0.845070423 | 0.912371134 |
| B1961443 | WBC166 | WBC006H06 | WBC012E07 | BM735536 | BM735167 | 0.951219512 | 0.830985915 | 0.907216495 |
| WBC001H09 | BM735536 | WBC027D07 | WBC009B10 | WBC028D09 | B1961443 | 0.918699187 | 0.887323944 | 0.907216495 |
| WBC028D09 | WBC434 | Foe545 | WBC001F11 | B1961443 | BM735573 | 0.93495935 | 0.85915493 | 0.907216495 |
| BM781178 | B1961671 | WBC028D09 | WBC005F10 | BM781417 | BM735536 | 0.93495935 | 0.845070423 | 0.902061856 |
| WBC013E10 | WBC166 | BM781417 | BM735460 | B1961494 | B1961443 | 0.93495935 | 0.845070423 | 0.902061856 |
| WBC028F05 | BM735102 | BM735534 | B1961443 | WBC019B05 | B1961885 | 0.918699187 | 0.873239437 | 0.902061856 |
| WBC003H01 | BM734531 | B1961109 | gi576646 | WBC019B05 | BM735536 | 0.926829268 | 0.85915493 | 0.902061856 |
| BM735409 | BM735352 | BM735536 | WBC028D09 | WBC014H06 | BM734865 | 0.910569106 | 0.873239437 | 0.896907216 |
| WBC019B05 | B1961443 | WBC028F05 | WBC021D01 | WBC003D11 | WBC012E07 | 0.902439024 | 0.887323944 | 0.896907216 |
| WBC028D09 | BM781417 | WBC019B05 | BM735536 | WBC039F12 | WBC004D07 | 0.918699187 | 0.85915493 | 0.896907216 |
| WBC026E02 | Foe1072 | WBC008F06 | B1961885 | B1961443 | WBC028F05 | 0.910569106 | 0.873239437 | 0.896907216 |
| BM735536 | BM734457 | WBC028D09 | BM780906 | BM735487 | B1961671 | 0.910569106 | 0.873239437 | 0.896907216 |
| WBC028C01 | BM734722 | B1961620 | WBC013C03 | BM735534 | BM735536 | 0.918699187 | 0.85915493 | 0.896907216 |
| WBC493 | B1961682 | WBC001C11 | WBC012E07 | WBC003D11 | B1961443 | 0.918699187 | 0.85915493 | 0.896907216 |
| WBC005D02 | BM735536 | BM734457 | WBC003F02 | BM781334 | Foe545 | 0.93495935 | 0.830985915 | 0.896907216 |
| B1961443 | WBC021D01 | BM781417 | B1961494 | BM735585 | BM735457 | 0.910569106 | 0.85915493 | 0.891752577 |
| WBC434 | BM734457 | B1961443 | B1961941 | BM736534 | WBC030C04 | 0.93495935 | 0.816901408 | 0.891752577 |
| BM780906 | WBC030D02 | WBC001C12 | Foe545 | B1961443 | B1961109 | 0.918699187 | 0.845070423 | 0.891752577 |

**TABLE 12**

| **SEVEN GENES SELECTED** | | | | | | | | | |
|---|---|---|---|---|---|---|---|---|---|
| **Genes** | | | | | | | **Sensitivity** | **Specificity** | **Success** |
| BM735536 | WBC016A12 | B1961637 | B1961941 | B1961443 | BM734722 | WBC030D02 | 0.93495935 | 0.873239437 | 0.91237113 |
| B1961682 | Foe545 | WBC001F11 | BM734862 | WBC003D11 | B1961443 | WBC028F05 | 0.93495935 | 0.873239437 | 0.91237113 |
| BM734889 | B1961443 | WBC434 | Foe1072 | WBC004C03 | B1961885 | WBC007G03 | 0.93495935 | 0.873239437 | 0.91237113 |
| WBC006E03 | BM735573 | B1961443 | WBC001H09 | B1961682 | WBC019B05 | WBC022B06 | 0.943089431 | 0.85915493 | 0.91237113 |
| BM734862 | WBC013C03 | WBC032G05 | WBC004D07 | WBC028D09 | WBC008F06 | BM735536 | 0.93495036 | 0.85915493 | 0.90721649 |
| WBC004D07 | WBC003H01 | WBC001A07 | BM735487 | BM735536 | BM734865 | BM780906 | 0.926829268 | 0.873239437 | 0.90721649 |
| B1961443 | WBC012F07 | gi576646 | BM734862 | WBC019B05 | WBC022B06 | WBC001F08 | 0.943089431 | 0.845070423 | 0.90721649 |
| WBC003F02 | BM735536 | WBC003H01 | Foe545 | WBC003D11 | WBC004E04 | BM735573 | 0.943089431 | 0.830985915 | 0.90206186 |
| WBC005D02 | WBC028D09 | B1961637 | B1961443 | WBC030D02 | BM735576 | BM735457 | 0.894308943 | 0.915492958 | 0.90206186 |
| WBC014G08 | WBC007G12 | BM735102 | WBC001F08 | WBC006E03 | gi576646 | BM735536 | 0.951219512 | 0.816901408 | 0.90206186 |
| B1961494 | BM781334 | BM735585 | BM7355536 | WBC009B10 | B1961620 | WBC041B05 | 0.926829268 | 0.85915493 | 0.90206186 |
| BM735585 | BM735102 | B1961443 | WBC001F11 | WBC008F06 | WBC003D11 | WBC027D07 | 0.910569106 | 0.887323944 | 0.90206186 |
| BM735536 | WBC001F08 | WBC004C03 | WBC007G12 | B1961494 | B1961682 | BM735286 | 0.93495935 | 0.845070423 | 0.90206186 |
| WBC038G11 | BM781334 | GI1592834 | WBC019B05 | WBC027E07 | WBC007A09 | B1961671 | 0.926829268 | 0.85915493 | 0.90206186 |
| B1961671 | B1961443 | WBC004C03 | WBC004D07 | BM735536 | WBC012E07 | WBC021D01 | 0.910569106 | 0.873239437 | 0.89690722 |
| WBC009B10 | WBC038G11 | B1961682 | B1961443 | WBC001C11 | WBC006E03 | WBC030C04 | 0.926829268 | 0.845070423 | 0.89690722 |
| BM735573 | WBC019B05 | WBC028E07 | WBC003H01 | WBC006H06 | BM735441 | BM734654 | 0.943089431 | 0.816901408 | 0.89690722 |
| WBC003H01 | WBC041B05 | B1961443 | BM735457 | BM735585 | WBC007G12 | BM735441 | 0.918699187 | 0.85915493 | 0.89690722 |
| Foe1072 | BM734865 | WBC026E02 | WBC010F04 | B1961443 | WBC022B06 | WBC001F11 | 0.910569106 | 0.873239437 | 0.89690722 |
| WBC027E07 | BM735536 | WBC008F06 | BM735450 | WBC013A09 | WBC032G05 | WBC007G03 | 0.918699187 | 0.85915493 | 0.89690722 |

**TABLE 13**

| **EIGHT GENES SELECTED** | | | | | | | | | | |
|---|---|---|---|---|---|---|---|---|---|---|
| **Genes** | | | | | | | | **Sensitivity** | **Specificity** | **Success** |
| B1961443 | B1961941 | WBC009E12 | BM735545 | BM735585 | BM735536 | BM734457 | WBC043G11 | 0.93495935 | 0.90140845 | 0.92268 |
| BM734865 | BM735536 | B1960933 | B1961443 | WBC027E07 | WBC007G12 | WBC019B05 | G11592834 | 0.943089431 | 0.88732394 | 0.92268 |
| BM735536 | B1961443 | BM734862 | WBC013C03 | WBC028C01 | BM734719 | BM735286 | BM735585 | 0.943089431 | 0.88732394 | 0.92268 |
| B1961109 | B1961443 | BM734654 | BM735576 | WBC032G05 | WBC003D11 | WBC019B05 | WBC013C03 | 0.943089431 | 0.87323944 | 0.917526 |
| WBC001C11 | B1961711 | BM734862 | BM735536 | WBC039F12 | WBC004C03 | B1961443 | BM735166 | 0.93495935 | 0.87323944 | 0.912371 |
| BM734531 | WBC041B05 | Foe545 | B1961620 | WBC027D07 | B1961637 | WBC021B08 | B1961443 | 0.926829268 | 0.88732394 | 0.912371 |
| WBC021D01 | WBC006H06 | B1961109 | WBC001F11 | WBC019B05 | BM735536 | B1961443 | WBC005D02 | 0.93495935 | 0.85915493 | 0.907216 |
| B1961443 | BM735573 | B1961885 | BM734457 | WBC041B05 | WBC009B 10 | B1961620 | WBC003F10 | 0.926829268 | 0.87323944 | 0.907216 |
| WBC019B05 | B1961620 | WBC041B05 | WBC032B05 | WBC013C03 | WBC013H03 | B1961443 | BM734531 | 0.926829268 | 0.87323944 | 0.907216 |
| B1961885 | BM735573 | WBC028E07 | WBC026E02 | WBC003H01 | BM735536 | WBC013C03 | B1961682 | 0.910569106 | 0.90140845 | 0.907216 |
| BM735536 | WBC007G03 | BM734457 | WBC003H01 | WBC041B05 | WBC016A12 | WBC022B06 | BM735573 | 0.93495935 | 0.85915493 | 0.907216 |
| WBC010F04 | WBC493 | BM734719 | B1961682 | WBC019B05 | WBC006H06 | B1961443 | WBC032G05 | 0.918699187 | 0.88732394 | 0.907216 |
| WBC030C04 | B1961443 | WBC016A12 | BM735166 | WBC019B05 | WBC005F10 | GI1592834 | B1961885 | 0.910569106 | 0.88732394 | 0.902062 |
| BM735441 | WBC009B10 | WBC003H01 | WBC003D11 | BM735536 | B1961671 | WBC010F04 | WBC027E07 | 0.93495935 | 0.84507042 | 0.902062 |
| WBC019B05 | WBC022B05 | B1961682 | WBC024C12 | WBC028E07 | B1961443 | GI9717252-3M | WBC001F08 | 0.93495935 | 0.84507042 | 0.902062 |
| WBC001C11 | WBC007G12 | BM781334 | WBC030C04 | BM781174 | BM735409 | BM735536 | WBCQ04D07 | 0.910569106 | 0.88732394 | 0.902062 |
| WBC013H03 | WBC028D09 | B1961885 | WBC009E12 | B1961941 | WBC005F10 | WBC001F11 | B1961671 | 0.926829268 | 0.85915493 | 0.902062 |
| WBC005D02 | BM734865 | GI9717252-3M | WBC012E07 | BM735536 | WBC043G11 | BM735441 | WBC004D07 | 0.93495935 | 0.84507042 | 0.902062 |
| WBC013C03 | BM735536 | BM781178 | WBC003H01 | gi576646 | WBC028F05 | WBC003F02 | Foe545 | 0.943089431 | 0.83098592 | 0.902062 |
| B1961620 | GI1592834 | BM735585 | WBC009B10 | WBC007G03 | B1961443 | BM781417 | gi576646 | 0.902439024 | 0.90140845 | 0.902062 |

**TABLE 14**

| **NINE GENES SELECTED** | | | | | | | | | | | |
|---|---|---|---|---|---|---|---|---|---|---|---|
| **Genes** | | | | | | | | | **Sensitivity** | **Specificity** | **Success** |
| WBC019B05 | WBC003F02 | BM781334 | WBC004D07 | WBC013E10 | WBC434 | B1961443 | WBC010F04 | WBC007G03 | 0.95121951 | 0.901408 | 0.93299 |
| WBC028C01 | WBC001F08 | BM735536 | WBC013H03 | BM735573 | WBC012F07 | WBC013A09 | WBC005F10 | BM735409 | 0.95121951 | 0.859155 | 0.917526 |
| B1961443 | WBC007G03 | B1961109 | B1961637 | BM735585 | WBC001F08 | BM735450 | BM735536 | BM734457 | 0.92682927 | 0.887324 | 0.912371 |
| WBC001H09 | WBC020B04 | BM735536 | BM735102 | BM734531 | WBC003H01 | BM735457 | WBC001A07 | WBC019B05 | 0.92682927 | 0.887324 | 0.912371 |
| BM781186 | WBC001A07 | B1961443 | WBC019B05 | BM735167 | WBC022B05 | WBC013C03 | WBC018B01 | B1961941 | 0.91056911 | 0.915493 | 0.912371 |
| WBC024C12 | BM735536 | WBC016A12 | BM735102 | B1961443 | Foe545 | WBC006H06 | WBC028C01 | WBC004E04 | 0.93495935 | 0.873239 | 0.912371 |
| WBC018D05 | WBC010F04 | BM734722 | GI9717252-3M | WBC039F12 | BM735519 | BM781174 | WBC019B05 | B1961443 | 0.92682927 | 0.873239 | 0.907216 |
| WBC003H01 | WBC006H06 | WBC012G02 | WBC014H06 | WBC004C03 | WBC019B05 | WBC007A09 | WBC020B04 | BM735536 | 0.93495935 | 0.859155 | 0.907216 |
| WBC001A07 | BM735573 | WBC012G02 | B1961443 | WBC043G11 | gi576646 | BM735536 | BM734719 | WBC024B05 | 0.93495935 | 0.859155 | 0.907216 |
| WBC004C03 | WBC005D02 | BM734865 | WBC024B05 | BM735536 | WBC014H06 | WBC013C03 | WBC012E07 | BM734719 | 0.93495935 | 0.859155 | 0.907216 |
| BM735534 | BM735457 | WBC019B05 | BM735166 | WBC434 | B1961443 | WBC038G11 | WBC005D02 | BM735585 | 0.92682927 | 0.873239 | 0.907216 |
| WBC024B05 | BM735536 | WBC038G11 | WBC010F04 | BM735457 | Foe1072 | WBC003H01 | B1961443 | WBC006E03 | 0.93495935 | 0.84507 | 0.902062 |
| WBC004D07 | B1961941 | WBC022B05 | WBC022B06 | BM735534 | B1961443 | WBC010F04 | BM735167 | WBC004E04 | 0.92682927 | 0.859155 | 0.902062 |
| WBC032G05 | BM735409 | WBC004C03 | WBC012F01 | B1961620 | B1961443 | WBC019B05 | WBC021D01 | WBC016A12 | 0.91056911 | 0.887324 | 0.902062 |
| B1961682 | BM735450 | WBC028F05 | BM735102 | B1961443 | WBC001F08 | WBC021D01 | BM735487 | WBC030C04 | 0.91056911 | 0.887324 | 0.902062 |
| WBC041B05 | BM735585 | B1961443 | WBC028E07 | WBC020B04 | B1961637 | WBC013E10 | WBC010F04 | BM781178 | 0.92682927 | 0.859155 | 0.902062 |
| Foe545 | Foe1060 | BM735536 | BM735167 | BM735585 | WBC009E12 | GI1592834 | WBC024C12 | WBC006H06 | 0.92682927 | 0.859155 | 0.902062 |
| BM735536 | WBC038G11 | BM735534 | WBC004C03 | WBC003H01 | BM735457 | WBC007G12 | B1961620 | WBC004D07 | 0.91869919 | 0.873239 | 0.902062 |
| BM735166 | WBC003H01 | WBC043G11 | BM734722 | WBC022B05 | BM734531 | BM735536 | WBC008F06 | WBC434 | 0.92682927 | 0.859155 | 0.902062 |
| WBC012E07 | BM735167 | WBC004D07 | WBC013H03 | WBC019B05 | WBC021B08 | B1961443 | WBC022F08 | BM735450 | 0.94308943 | 0.830986 | 0.902062 |

**TABLE 15**

| **NETEN GENES SELECTED** | | | |
|---|---|---|---|
| **Genes** | **Sensitivity** | **Specificity** | **Success** |
| BM735536; WBC030C04; WBC019B05, BM734531; WBC018B01; BM735166; WBC006E03; WBC007A09; WBC018D05; B1961885 | 0.95122 | 0.859155 | 0.917526 |
| BM734719; BM735534; B1961443; B1960933; WBC026E02; BM735536; BM735573; WBC022B05; WBC019B05; WBC001F11 | 0.926829 | 0.901408 | 0.917526 |
| WBC004D07; BM735450; WBC004C03; B1961711; Foe1072; WBC039F12; B1961443; WBC013H03; WBC032G05; WBC001F08 | 0.934959 | 0.873239 | 0.912371 |
| BM734531; WBC028C01; BM735536; BM734722; WBC019B05; WBC041B05; BM735166; WBC0013H03; BM735487; WBC032G05 | 0.943089 | 0.859155 | 0.912371 |
| BM735102; WBC434; BM734531; WBC005D02; WBC007G03; WBC010F04; BM781417; BM735441; BM734719; B1961443 | 0.934959 | 0.873239 | 0.912371 |
| WBC032B05; WBC005F10; WBC028D09; B1961443; Foe1072; WBC027E07; WBC434; B1960933; BM734654; B1961885 | 0.943089 | 0.859155 | 0.912371 |
| WBC019B05; WBC043G11; B1961941; BM781186; B1961682; WBC018D05; WBC024C12; WBC012F07; WBC001F08; WBC003D11 | 0.934959 | 0.859155 | 0.907216 |
| WBC010F04; B1961443; BM735585; WBC434; WBC493; WBC022B06; WBC013H03; BM735352; WBC027D07; WBC001A07 | 0.926829 | 0.873239 | 0.907216 |
| BM734865; WBC021B08; BM735573; BM735536; WBC001F08; WBC007G03; B1961637; BM735519; WBC032G05; WBC001H09 | 0.934959 | 0.859155 | 0.907216 |
| WBC008F06; WBC434; B1961443; BM735487; WBC166; WBC012F07; BM735536; Foe1072; WBC007G12; WBC004D07 | 0.934959 | 0.859155 | 0.907216 |
| BM734862; BM734654; WBC001C12; Foe1072; BM734889; B1961443; BM735487; WBC039F12; BM735519; WBC001F08 | 0.934959 | 0.859155 | 0.907216 |
| WBC008F12; WBC001C12; WBC043G11; BM734862; Foe1060; WBC013C03; WBC022B05; WBC007G12; WBC009E12; BM735536 | 0.943089 | 0.84507 | 0.907216 |
| WBC021D01; BM781174; B1961443; Foe1060; BM781334; WBC024B05; Foe545; WBC028E07; WBC026E02; WBC005D02 | 0.910569 | 0.901408 | 0.907216 |
| WBC590; WBC010F04; BM735576; WBC021B08; BM735573; WBC003D11; WBC027D07; WBC008F12; Foe545; B1961443 | 0.918699 | 0.887324 | 0.907216 |
| WBC007G03; BM735585; B1961443; WBC009B10; GI1592834; BM734722; BM735536; BM735519; BM735409; WBC022B06 | 0.910569 | 0.887324 | 0.902062 |
| WBC043G11; BM781417; B1961443; WBC005F10; BM780906; BM735166; WBC028F05; BM735573; WBC019B05; WBC003D11 | 0.910569 | 0.887324 | 0.902062 |
| WBC493; BM735286; WBC004C03; BM735167; BM735536; BM734722; WBC003H01; BM735487; B1961711; 1; BM735576 | 0.934959 | 0.84507 | 0.902062 |
| WBC006E03; WBC043G11; WBC024C11; BM735576; WBC004E04; WBC021B08; BM735536; WBC01QF04; B1961443; WBC166 | 0.926829 | 0.859155 | 0.902062 |
| WBC020B04; BM781186; WBC003H01; BM781174; BM735573; BM735536; WBC028D09; B1961682; BM735519; WBC012E07 | 0.918699 | 0.873239 | 0.902062 |
| BM735536; WBC022B05; WBC590; BM735519; BM781174; B1961443; B1961494; WBC039F12; WBC005F10; WBC021B08 | 0.926829 | 0.859155 | 0.902062 |

**TABLE 16**

| **TWENTY GENES SELECTED** | | | |
|---|---|---|---|
| **Genes** | **Sensitivity** | **Specificty** | **Success** |
| WBC013C03; WBC019B05; WBC041B05; B1961637; BM780906; WBC004C03; WBC030D02; WBC434; BM781178; WBC032G05; BM781186; WBC018B01; BM781334; B1961885; BM734722; WBC010F04; WBC030C04; WBC038G11; WBC012E07; WBC008F06 | 0.95935 | 0.887324 | 0.93299 |
| B1960933; WBC019B05; B1961443; WBC007A09; WBC010F04; WBC02C11; WBC434; WBC018D05; WBC013E10; WBC009E12; BM781186; WBC018B01; BM781334; B1961885; BM734722; WBC01OF04; WBC030C04; WBC038G11; WBC012E07; WBC008F06 | 0.95935 | 0.887324 | 0.93299 |
| WBC013A09; BM735441; WBC028E07; WBC003D11; BM734531; BM735573; WBC028D09; WBC005F10; WBC030C04; WBC021B08; BM735487; BM781417; B1961494; B1961109; BM734531; WBC005D02; B1961637; WBC028E07; BM735352; BM735167 | 0.95122 | 0.901408 | 0.93299 |
| BM735536; WBC006H06; WBC018B01; WBC019B05; WBC003D11; BM735166; WBC009E12; BM735167; WBC493; BM735352; WBC028C01; WBC009B10; WBC014G08; WBC019B05; BM735585; BM735450; BM781334; BM735536; Foc545; WBC001C11 | 0.943089 | 0.901408 | 0.927835 |
| WBC019B05; BM734719; WBC434; WBC028F05; B1961443; BM73SS73; Foe1072; WBC001F08; BM735519; WBC03A09; BM781174; WBC043G11; WBC032G05; WBC041B05; WBC006E03; WBC001H09; WBC007G12; WBC004D07; B1901637; WBC004C03 | 0.934959 | 0.915493 | 0.927835 |
| BM734722; WBC030D02; BM735166; WBC022B06; BM735167; BM735441; WBC006E03; BM734531; WBC032G05; WBC012G02; WBC166; WBC021B08; WBC024F08; WBC013E10; BM734654; BM735409; BM734531; BM735536; WBC043G11; B1960933 | 0.943089 | 0.887324 | 0.92268 |
| WBC004E04; BM735536; WBC001F11; WBC018B01; WBC024F08; WBC009E12; WBC001F08; gi576646; BM735576; BM735457; BM735457; WBC024F08; WBC013C03; WBC018B01; WBC166; WBC001F08; BM735536; BM735409; Foe1060; WBC028F03 | 0.943089 | 0.887324 | 0.92268 |
| WBC008F12; WBC032G05; WBC010F04; WBC001F11; WBC018B01; B1960933; WBC012E07; BM735450; WBC022B06; BM735441; WBC019B05; WBC009B10; B1961109; BM734457; BM7345531; BM735545; WBC001C12; WBC024C12; WBC006E03; BM781334 | 0.95122 | 0.873239 | 0.92268 |
| B1961443; WBC001A07; WBC013E10; B1960933; WBC005F10; Foe545; WBC012F07; WBC010F04; WBC004D07; BM735487; Foe545; WBC434; WBC019B05; BM735167; WBC028C01; BM735576; BM734862; WBC009B10; Foe1072; WBC012F07 | 0.943089 | 0.887324 | 0.92268 |
| WBC003H01; BM735457; WBC004C03; BM734457; WBC006H06; WBC020B04; B1961443; WBC019B05; BM735536; WBC038G11; BM734531; WBC027D07; WBC032G05; WBC004C03; WBC007G03; WBC032B05; WBC001F11; WBC003F02; BM735536; WBC003H01 | 0.926829 | 0.915493 | 0.92268 |
| WBC019B05; WBC018B01; BM734722; WBC030D02; B1961109; BM735536; G11592834; WBC003D11; BM735573; WBC026E02; BM735573; BM734719; BM781417; WBC005D02; WBC012F07; WBC024C11; WBC004D07; BM735487; BM734865; WBC024B05 | 0.934959 | 0.901408 | 0.92268 |
| BM734865; BM735102; WBC001F08; B1961443; BM780906; Foe1072; WBC038G11; B1961637; WBC019B05; WBC024B05; WBC018D05; BM781178; WBC001F08; B1961443; WBC028F05; WBC013A09; WBC014G08; BM735487; Foe545; WBC012E07 | 0.926829 | 0.915493 | 0.92268 |
| B1961494; BM735536; WBC038G11; WBC004E04; WBC039F12; BM735167; WBC001F08; WBC004C03; BM734722; WBC019B05; WBC003H01; BM735457; BM735536; WBC043G11; WBC001C11; G19717252-3M; WBC004D07; WBC032G05; WBC016A12; WBC026E02 | 0.943089 | 0.887324 | 0.92268 |
| WBC003D11; BM734457; B1961443; BM735450; BM734531; WBC004C03; WBC012G02; BM734889; BM735585; WBC018B01; WBC007A09; GI1592834; BM781186; B1961682; BM734531; BM735352; WBC001H09; WBC493; WBC024B05; WBC005D02 | 0.918699 | 0.915493 | 0.917526 |
| WBC024C11; WBC001A07; WBC434; WBC032G05; WBC028E07; WBC004C03; WBC027D07; BM734531; gi576646; BM734654; WBC019B05; BM735409; BM735487; WBC005F10; WBC005D02; WBC014G08; WBC012F07; WBC007G12; WBC010F04; B1961671 | 0.943089 | 0.873239 | 0.917526 |
| WBC009B10; Foe545; Foe1060; WBC027E07; WBC012G02; BM735457; WBC019B05; BM735409; GI1592834; WBC030C04; BM734457; WBC030C04; WBC010F04; WBC003H01; BM735102; BM735545; BM781417; BM781174; WBC014G08; WBC007A09 | 0.943089 | 0.873239 | 0.917526 |
| BM735441; WBC010F04; WBC008F12; BM735573; B1961443; WBC012F07; B1960933; WBC004D07; WBC043G11; WBC014H06; BM780906; WBC016A12; WBC041B05; BM781178; WBC010F04; WBC434; WBC005D02; WBC014H06; BM734865; WBC028D09 | 0.934959 | 0.887324 | 0.917526 |
| BM735573; WBC007G03; G11592834; BM734722; B1961711; WBC021B08; BM735536; WBC493; WBC019B05; B1961443; WBC024C11; WBC006H06; WBC493; WBC013C03; BM734719; BM735487; WBC019B05; WBC024F08; WBC016A12; WBC004E04 | 0.926829 | 0.901408 | 0.917526 |
| WBC590; WBC028F05; BM735166; B1961885; BM735519; WBC018B01; WBC019B05; B1961637; WBC021B08; B1961941; WBC001F08; WBC026E02; BM735534; BM735585; WBC006E03; WBC004E04; WBC009B10; WBC008F12; Foe1072; WBC018B01 | 0.943089 | 0.873239 | 0.917526 |
| WBC027D07; BM735534; BM735487; BM791334; WBC013A09; WBC020D09; WBC590; WBC024F08; WBC024C12; B1961941; WBC006E03; WBC005D02; B1961711; WBC009E12; WBC003H01; WBC026E02; WBC166; WBC001F11; BM735536; BM735167 | 0.926829 | 0.901408 | 0.917526 |

**TABLE 17**

| **STRESS MARKER GENE ONTOLOGY** | | | | | |
|---|---|---|---|---|---|
| **Gene** | **Genbank Homology** | **UNIPROT** | **CELLULAR COMPONENT** | **MOLECULAR FUNCTION** | **BIOLOGICAL PROCESS** |
| WBC590 | Zinc Finger Protein 198 | Q5W0T3 | nucleus | zinc ion binding | - |
| WBC493 | Homo sapiens mRNA; cDNA DKFZp667N0.84 | NA | | | |
| WBc434 | CGG triplet repeat binding protein 1 (CGGBP1), | 015183 | nucleus | double-stranded DNA binding | - |
| WBC166 | Mst3 and SOK1-related kinase (MASK) | Q9P289 | - | ATP binding, protein serine/threonine kinase activity, protein-tyrosine kinase activity | protein amino acid phosphorylation |
| WBC043G11.bFSP_20021 401.esd | Homo sapiens high mobility group nucleosomal binding domain 4, mRNA | Q53XL9 | - | - | - |
| WBC041B05 | ARP3 actin-related protein 3 homology (yeast) | Q59FV6 | - | - | - |
| WBC039F12 | Leu-8 pan leukocyte antigen | NA | | | |
| WBC03BG11_V1.3_at | No Homology | NA | | | |
| WBC032G05 | Glycerol kinase (GK) | NA | | | |
| WBC032B05 | DDHD domain containing 1 | NA | | | |
| WBC030DO2 | Putative membrane protein (GENX-3745 gene) | Q9NY35 | - | - | - |
| WBC030C04 | No homology | NA | | | |
| WBC028F05 | No homology | NA | | | |
| WBC028E07 | Homo sapiens cDNA FLJ13038 fis, clone NT2RP3001272, weakly similar to Mus musculus mRNA for macrophage actin-associated-tyrosine-phosphorylated protein | NA | | | |
| WBC028D09 | No homology | NA | | | |
| WBC028C01-V1.3_at | Ras homolog gene family, member A | Q5U024 | - | - | - |
| WBC027E07 | No homology | NA | | | |
| WBC027D07 | No homology | NA | | | |
| WBC026E02 | Migration-inducing gene 10 protein | Q5J7M1 | - | - | - |
| WBC024F08 | No homology | NA | | | |
| WBC024C12 | No homology | NA | | | |
| WBC024C11 | No homology | NA | | | |
| WBC024B05 | Adducin 3 (gamma) (ADD3), transcript variant 2 | Q5VU08 | - | - | - |
| WBC022F08 | Phosphogluconate dehydrogenase | P52209 | - | electron transporter activity | pentose-phosphate shunt, oxidative branch |
| WBC022B06 | Immunoglobulin superfamily, member 6 variant | NA | | | |
| WBC022B05 | Toll-like receptor 8 (TLR8) | Q9NR97 | integral to membrane | receptor activity, Toll binding | detection of virus, I-kappaB kinase/NF-kappaB cascade, innate immune response |
| WBC021D01 | No homology | NA | | | |
| WBC021B08 | Hypothetical protein FLJ20481 | Q7L5N7 | - | acyltransferase activity, calcium ion binding | metabolism |
| WBC020B04 | No homology | NA | | | |
| WBC019B05 | Homo sapiens mRNA; cDNA DKFZp686M2414 | NA | | | |
| WBC018D05 | Predicted: Mitogen-activated protein kinase kinase kinase 1 (MAP3K1) | NA | | | |
| WBC018B01_V1.3_s_at | Homo sapiens gene for JKTBP2, JKTBP1 (alternative splicing). | NA | | | |
| WBC016Al2 | No homology | NA | | | |
| WBC014H06 | Homo sapiens mRNA; cDNA DKFZp564C012 | Q9H0V1 | - | - | - |
| WBC014G08_V1.3_at | RTN4-C (RTN4) | Q6IPN0 | endoplasmic reticulum | unknown | - |
| WBC013H03_V1.3_at | RAB6 interacting protein (RAB6IP1) | Q6IQ26 | - | - | - |
| WBC013E10 | Homo sapiens cDNA FLJ45679 fis, clone ERLTF2001835 | NA | | | |
| WBC013C03_V1.3_at - | Ras GTPase-activating-like protein (IQGAP1) | P46940 | actin filament | calmodulin binding | GTPase activator activity, GTPase inhibitor activity, signal transduction |
| WBC013A09 | Sialyltransferase 1 (beta-gaiactoside alpha-2,6-sialyltransferase), transcript variant 2 | P15907 | integral to membrane | beta-galactoside alpha-2,6-sialyltransferase activity | growth, humoral immune response, oligosaccharide metabolism, protein modification |
| KBC012G02 | Soc-2 suppressor of clear homolog (C. elegans) | Q5VZS9 | | | |
| WBC012F07 | Complement component 5 receptor 1 (C5a ligand) | NA | | | |
| WBC012EO7 | Pinin, desmosome associated protein (PNN) | Q99738 | intercellular junction, intermediate filament, plasma membrane | structural molecule activity | cell adhesion |
| WBC010F04 | 3-hydroxy-3-methylglutaryl-Coenzyme A synthase 1 (soluble) | Q01581 | cytoplasm, soluble fraction | hydroxymethylglut aryl-CoA synthase activity | lipid metabolism |
| WBC009E12 | Down-regulator of transcription 1, TBP-binding (negative cofactor 2) | Q01658 | - | DNA binding, transcription corepressor activity, transcription factor binding | Negative regulation of transcription from RNA polymerase II promoter |
| WBC009B10_V1.3_at (CR1, | Human mRNA for complement receptor type 1 C3b/C4b receptor, CD35) | P17927 | integral to plasma membrane | complement receptor activity | complement activation |
| WBC008F12 | v-rai simian leukemia viral oncogene homolog B (ras related; GTP binding protein | Q7T383 | - | GTP binding | Small GTPase mediated signal transduction |
| WBC008F06_V1.3_at | No Homology | NA | | | |
| WBC007G12_V1.3_at | No Homology | NA | | | |
| WBC007G03 | Transmembrane protein 23 cDNA clone MGC:17342 IMAGE:4342258 also called Phosphatidylcholine:ceramide cholinephosphotransferase 1 (Sphingomyelin synthase 1) (Mob protein | Q86VZ5 | Cellular component, integral to golgi | ceramide cholinephosphotra nsferase activity | Sphingomyelin biosynthesis |
| WBC007A09 | No homology | NA | | | |
| WBC006H06 | Ubiquitin-conjugating enzyme E2B (RAD6 homolog) (UBE2B) | | | | |
| WBC006E03_V1.3_at | Homo sapiens methionine adenosyltransferas() II, beta (MAT2B) | | Intracellular | Protein binding | S-adenosylmethionine biosynthesis |
| WBC005F10 | Polymeric immunoglobulin receptor 3 precursor (PTGR3) | Q8NHL4 | - | receptor activity | - |
| WBC005D02_V1.3_at | Homo sapiens hypothetical protein FLJ22662, mRNA | Q6P4A8 | - | - | - |
| WBC004E04 | TRAF-interacting protein with a forkhead-associated domain | Q96CG3 | nucleus | - | - |
| WBC004D07-V1.3_at | No Homology | NA | | | |
| WBC004B05 | Heterogeneous nuclear ribonucleoprotein F | P52597 | heterogeneous nuclear ribonucleoprotein complex | RNA binding | RNA processing |
| WBC004C03 | Dendritic cell protein variant, clone: CAE03630 | Q53HL6 ?clone CAE0363 8 | - | - | - |
| WBC003H01 | CGI-54 protein | Q9Y282 | - | - | - |
| WBC003F02 | IBR domain containing 3 (IBRDC3) | NA | | | |
| WBC003D11 | No homology | NA | | | |
| WBC001H09 | Activated RNA polymerase II transcription cofactor 4 variant protein (incomplete) | Q59G24 | - | - | - |
| WBC001F11 | Retinoplastoma-like 2 (p130) | Q08999 | - | protein binding | - |
| WBC001F08 | RAB10, member RAS oncogene family (RAB10), | P61026 | - | - | - |
| WBC001C12-V1.3_at | No Homology | NA | | | |
| WBC001C11 (Same as WBC041B05) | ARP3 actin-related protein 3 homolog (yeast) | Q59FV6 | - | - | - |
| WBC001A07_V1.3_at | No Homology | NA | | | |
| GI9717252 | Equus caballus Toll-like receptor 4 mRNA | Q5XWB9 | membrane | transmembrane receptor activity | inflammatory response |
| GI1592834 | Equus caballus gelsolin mRNA | Q6X9X6 | - | actin binding | - |
| Gi576646 | Equus caballus Ig epsilon heavy chain (partial) | NA | | | |
| Foe 545 | Homo sapiens mRNA, cDNA DKFZp6661186 (from clone DKFZp666I186) | Q658M2 | - | - | - |
| Foe 1072 | Transducin (beta)-like 1X-linked receptor 1 | NA | | | |
| Foe 1060 | Homo sapiens 15 kDa selenoprotein, transcript variant 1 | | Endoplasmic reticulum lumen | Protein binding, Se binding | Post-translational protein folding. |
| BM781417 | No homology | NA | | | |
| BM781334 | No homology | NA | | | |
| BM781186 | Membrane-spanning 4-domains, subfamily A, member 6A, transcript variant 1 | | Integral to membrane | Receptor activity | Signal transduction |
| BM781178 | No homology | NA | | | |
| BM761174 | GM2 ganglioside activator | | Lysosome | Sphingolipid activator protein activity | Lipid metabolism |
| BM780906.V1.3_at | No Homology | NA | | | |
| BM735585 | Fc-epsilon-receptor gamma-chain | | Integral to plasma membrane | Receptor activity | Humoral response. |
| BM735576 | Minor histocompatibility antigen H13 isoform 1 (H13) | | Integral to membrane | Peptidase activity | D-alanyl-D-alanine endopeptidase activity |
| BM735573 | No Homology | NA | | | |
| BM735545 | CD68 protein | | Lysosome, membrane | NA | NA |
| BM735536 | Transglutaminase E3 (TGASE3) | | NA | NA | NA |
| BM735534 | PREDICTED: Bos taurus similar to hypothetical protein (LOC515494), | | NA | NA | NA |
| BM735519 | Ring finger protein 10 | NA | | | |
| BM735487.V1.3_at | No Homology | NA | | | |
| BM735457 | No homology | NA | | | |
| BM735450 | Lymphocyte surface antigen precursor CD44 | | Type I membrane protein | Cell surface receptor | Lymphocyte homing |
| BM735441 | WD repeat domain 1, transcript variant 2 | | Cytoskeleton | Protein binding | Actin binding |
| BM735409 | No homology | NA | | | |
| BM735352 | No homology | NA | | | |
| BM735286 | Ferritin light chain | | Ferritin complex | Iron ion binding | Iron homeostasis |
| BM735167 | TAP2E | | NA | NA | NA |
| BM735166 | No homology | NA | | | |
| BM735102 | COP9 constitutive photomorphogenic homolog subunit 7A | | Signalasome complex | Unknown | Unknown |
| BM734889.V1.3_at | Equus caballus lipopolysaccharide receptor (CD14) mRNA | | Plasma membrane | Peptidoglycan receptor activity | Apoptosis, signal transduction, phagocytosis. |
| BM734865 | Nuclear receptor binding factor 1 | | NA | NA | NA |
| BM734862.V1.3_at | Triggering receptor expressed on myeloid cells 1 | | Receptor activity | Humoral immune response. | Intracelluar signalling cascade |
| BM734722 | No homology | NA | | | |
| BM734719 | No homology | NA | | | |
| BM734654 | No homology | NA | | | |
| BM734531 | No homology | NA | | | |
| BM734457 | High-risk human papilloma viruses E6 oncoproteins targeted protein E6TP1 beta mRNA | | NA | NA | NA |
| B1961941 | Fibroblast mRNA for aldolase A | | NA | Fructose-bisphosphate aldolase activity | Glycolysis |
| B1961885 | Tumor necrosis factor-inducible (TSG-6) mRNA fragment, adhesion receptor CD44 putative CDS | | Extracellular region | Protein binding | Inflammtory response, cell adhesion, protein binding. |
| B1961711.V1.3_at | No Homology | NA | | | |
| B1961682.V1.3_at | Formin homology 2 domain containing 1 | | Nucleus, cytoplasm | Actin binding | Cell organisation and biogenesis. |
| B1961671 | NAD synthetase 1 | | | ATP binding | NAD biosynthesis |
| B1961637 | Mn-SOD mRNA for manganese superoxide dismutase | | Mitochondrian | Superoxide dismutase activity | Response to oxidative stress |
| B1961620 | ILT11A mRNA for immunoglobulin-like transcript 11 protein | | NA | NA | NA |
| B1961494 | HREV107-3 | | NA | Tumor suppressor, associated with cell death. | NA |
| B1961443 | PREDICTED: Homo sapiens steroid receptor RNA activator 1 (SRA1) | | NA | NA | NA |
| B1961009 | G protein-coupled receptor HM74a | | Integral to membrane | Receptor activity | G-protein coupled receptor protein signaling pathway |
| B1960933 | Pleckstrin | | NA | Calcium ioni binding | NA |
| WBC037F12 | Selenoprotein P | | Extracellular region | Selenium binding | Response to oxidative stress |
| WBC043E03 | Ribosomal protein S3A | | Ribosome | Constitutive component of ribosome | Protein biosynthesis |
| Foe1019 | Hemoglobin, beta (HBB) | | | | Oxygen transport |
| Gi5441616 | Equus caballus mRNA for interferon gamma inducing factor (IL-18) | | Extracellular region | Cytokine activity | Interferon gamma induction |
| B1961054 | Interferon-gamma-inducible protein-10 (IP-10) (ovis aries) | | Extracellular regioin | Chemokine activity | Immune response. |
| B1961539 | Calcium-binding protein in macrophages (MRP-14) macrophage migration inhibitory factor (MIF)-related protein | | NA | Signal transducer activity | Cell-cell signalling, inflammatory response. |
| BM735419 | Villin 2 (ezrin) | | Membrane bound, (extracellular) | Connection of cytoskeleton to plasma membrane | NA |
| WBC013G08 | cDNA FLJ16386 fis, clone TRACH2000862, moderately similar to Mus musculus putative purine nucleotide binding protein mRNA | | NA | NA | NA |
| B1961648 | Farnesyl diphosphate synthase (farnesyl pyrophosphate synthetase, dimethylallyltranstransferase, geranyltranstransferase) | | Cyotplasmic | Catalytic | Cholesterol biosynthesis |
| WBC041B04 | 56-KDa protein induced by interferon | NA | NA | NA | NA |
| NBC001B11 | No homology | NA | NA | NA | NA |
| WBC032B11 | Sphingosine-1-phosphate phosphatase 1 | | Endoplasmic reticulum | Enzymatic activity | Regulates S1P levels |
| B1961185 | Actin related protein 2/3 complex, subunit 1B, 41kDA | | ARP2/3 protein complex | Cytoskeleton | Cell motility |
| B1961512 | No homology | NA | | | |
| WBC008D05 | No homology | NA | | | |
| WBC133 | No homology | NA | | | |
| BM781012 | Equus caballus immunogobulin gamma 1 heavy chain constant region (IGHC1 gene) | NA | NA | NA | NA |
| WBC005B09 | CDC-like kinase 1 | | | Non-membrane spanning protein tyrosine kinase activity | Regulation of cell cycle |
| WBC040E12 | Arachidonate 5-lipoxygenase-activating protein (ALOX5AP). | | Integral to membrane | Enzyme activator activity | Inflammatory response. |

The invention also relates to the following aspects as defined in the following numbered paragraphs:
1. A method for determining the presence or degree of a physiological response to stress or a related condition in a test subject, comprising detecting in the test subject aberrant expression of at least one stress marker gene selected from the group consisting of:
   (a) a gene having a polynucleotide expression product comprising a nucleotide sequence that shares at least 50% sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof;
   (b) a gene having a polynucleotide expression product comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a gene having a polynucleotide expression product comprising a nucleotide sequence that encodes a polypeptide that shares at least 50% sequence similarity with at least a portion of the sequence set forth in SEQ ID NO: 22, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 15 contiguous amino acid residues of that sequence; and (d) a gene having a polynucleotide expression product comprising a nucleotide sequence that hybridizes to the sequence of (a), (b), (c) or a complement thereof, under at least low stringency conditions.
2. A method according to paragraph 1, comprising comprise detecting aberrant expression of a stress marker polynucleotide selected from the group consisting of: (a) a polynucleotide comprising a nucleotide sequence that shares at least 50% sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof; (b) a polynucleotide comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a polynucleotide comprising a nucleotide sequence that encodes a polypeptide that shares at least 50% sequence similarity with at least a portion of the sequence set forth in SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.
3. A method according to paragraph 1, comprising detecting aberrant expression of a stress marker polynucleotide selected from the group consisting of: (i) a polynucleotide comprising an amino acid sequence that shares at least 50% (sequence similarity with the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (ii) a polynucleotide comprising a portion of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 5 contiguous amino acid residues of that sequence; (iii) a polypeptide comprising an amino acid sequence that shares at least 30% similarity with at least 15 contiguous amino acid residues of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; and (iv) a polypeptide comprising a portion of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 5 contiguous amino acid residues of that sequence and is immuno-interactive with an antigen-binding molecule that is immuno-interactive with a sequence of (i), (ii) or (iii).
4. A method according to paragraph 1, wherein the aberrant expression is detected by: (1) measuring in a biological sample obtained from the test subject the level or functional activity of an expression product of at least one stress marker gene and (2) comparing the measured level or functional activity of each expression product to the level or functional activity of a corresponding expression product in a reference sample obtained from one or more normal subjects or from one or more subjects not under stress, wherein a difference in the level or functional activity of the expression product in the biological sample as compared to the level or functional activity of the corresponding expression product in the reference sample is indicative of the presence of a physiological response to stress in the test subject.
5. A method according to paragraph 4, further comprising diagnosing the presence, stage or degree of a physiological response to stress in the test subject when the measured level or functional activity of the or each expression product is 10% lower than the measured level or functional activity of the or each corresponding expression product.
6. A method according to paragraph 5, wherein the presence of a physiological response to stress is determined by detecting a decrease in the level or functional activity of at least one stress marker polynucleotide selected from (a) a polynucleotide comprising a nucleotide sequence that shares at least 50% sequence identity with the sequence set forth in any one of SEQ ID NO: 1, 3, 4, 7, 9, 11, 19, 21, 24, 25, 33, 34, 38, 39, 40, 41, 42, 50, 51, 52, 56, 57, 59, 62, 63, 66, 70, 71, 73, 75, 79, 81, 83, 89, 90, 91, 92, 93, 97, 99, 105, 107, 108, 111, 119, 121, 122, 123, 129, 130, 137, 139, 140, 141, 142, 143 or 185, or a complement thereof; (b) a polynucleotide comprising a nucleotide sequence that encodes a polypeptide comprising the amino acid sequence set forth in any one of SEQ ID NO: 2, 8, 10, 12, 20, 22, 43, 58, 60, 67, 71, 72, 74, 76, 80, 82, 84, 94, 98, 100, 106, 112, 120, 122, 123, 124 or 138; (c) a polynucleotide comprising a nucleotide sequence that encodes a polypeptide that shares at least 50% sequence similarity with at least a portion of the sequence set forth in SEQ ID NO: 2, 8, 10, 12, 20, 22, 43, 58, 60, 67, 71, 72, 74, 76, 80, 82, 84, 94, 98, 100, 106, 112, 120, 122, 123, 124 or 138, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.
7. A method according to paragraph 4, further comprising diagnosing the presence, stage or degree of a physiological response to stress in the test subject when the measured level or functional activity of the or each expression product is 10% higher than the measured level or functional activity of the or each corresponding expression product.
8. A method according to paragraph 7, wherein the presence of a physiological response to stress is determined by detecting an increase in the level or functional activity of at least one stress marker polynucleotide selected from (a) a polynucleotide comprising a nucleotide sequence that shares at least 50% sequence identity with the sequence set forth in any one of SEQ ID NO: 5, 13, 15, 16, 17, 23, 26, 28, 29, 30, 32, 35, 37, 44, 46, 48, 52, 54, 55, 64, 68, 77, 85, 87, 95, 96, 101, 103, 113, 115, 117, 118, 125, 126, 131, 133, 135, 144, 145, 147, 148, 150, 151, 153, 155, 156, 158, 160, 161, 163, 164, 165, 167, 169, 170, 171, 173, 175, 176, 178, 180, 182, 183, 184, 186, 187, 188, 190, 192, 194, 195, 196, 198, 200, 202, 204, 206 or 210, or a complement thereof; (b) a polynucleotide comprising a nucleotide sequence that encodes a polypeptide comprising the amino acid sequence set forth in any one of SEQ ID NO: 6, 14, 18, 27, 31, 36, 45, 47, 49, 53, 65, 69, 78, 86, 88, 102, 104, 114, 116, 132, 134, 136, 146, 149, 152, 154, 157, 159, 162, 166, 168, 172, 174, 177, 179, 181, 189, 191, 193, 197, 199, 201, 203, 205, 207 or 211; (c) a polynucleotide comprising a nucleotide sequence that encodes a polypeptide that shares at least 50% sequence similarity with at least a portion of the sequence set forth in SEQ ID NO: 6, 14, 18, 27, 31, 36, 45, 47, 49, 53, 65, 69, 78, 86, 88, 102, 104, 114, 116, 132, 134, 136, 146, 149, 152, 154, 157, 159, 162, 166, 168, 172, 174, 177, 179, 181, 189, 191, 193, 197, 199, 201, 203, 205, 207 or 211, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.
9. A method according to paragraph 4, further comprising diagnosing the absence of a physiological response to stress when the measured level or functional activity of the or each expression product is the same as or similar to the measured level or functional activity of the or each corresponding expression product.
10. A method according to paragraph 4, wherein the measured level or functional activity of an individual expression product varies from the measured level or functional activity of an individual corresponding expression product by no more than about 5%.
11. A method according to paragraph 4, comprising measuring the level or functional activity of individual expression products of at least about 2 stress marker genes.
12. A method according to paragraph 4, comprising measuring the level or functional activity of individual expression products of at least 1 level one correlation stress marker gene selected from: (a) a polynucleotide comprising a nucleotide sequence that shares at least 50% sequence identity with the sequence set forth in any one of SEQ ID NO: 89, 90, 103, 125, 126, 163, 178, 182, 184 or 190, or a complement thereof; (b) a polynucleotide comprising a nucleotide sequence that encodes a polypeptide comprising the amino acid sequence set forth in any one of SEQ ID NO: 104, 179, 183 or 189; (c) a polynucleotide comprising a nucleotide sequence that encodes a polypeptide that shares at least 50% sequence similarity with at least a portion of the sequence set forth in SEQ ID NO: 104, 179, 183 or 189, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.
13. A method according to paragraph 4, comprising measuring the level or functional activity of individual expression products of at least 1 level two correlation stress marker gene selected from: (a) a polynucleotide comprising a nucleotide sequence that shares at least 50% sequence identity with the sequence set forth in any one of SEQ ID NO: 17, 23, 44, 52, 133, 135, 144, 147, 148, 151, 155, 192, 196, 202 or 206, or a complement thereof; (b) a polynucleotide comprising a nucleotide sequence that encodes a polypeptide comprising the amino acid sequence set forth in any one of SEQ ID NO: 18, 20, 45, 53, 134, 136, 149, 152, 193, 197 or 207; (c) a polynucleotide comprising a nucleotide sequence that encodes a polypeptide that shares at least 50% sequence similarity with at least a portion of the sequence set forth in SEQ ID NO: 18, 20, 45, 53, 134, 136, 149, 152, 193, 197 or 207, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.
14. A method according to paragraph 4, comprising measuring the level or functional activity of individual expression products of at least 1 level three correlation stress marker gene selected from: (a) a polynucleotide comprising a nucleotide sequence that shares at least 50% sequence identity with the sequence set forth in any one of SEQ ID NO: 5, 30, 37, 48, 54, 55, 64, 66, 70, 77, 79, 85, 91, 92, 95, 96,101, 115, 117, 118, 121, 150, 153, 158, 164, 170, 180, 186 or 198, or a complement thereof; (b) a polynucleotide comprising a nucleotide sequence that encodes a polypeptide comprising the amino acid sequence set forth in any one of SEQ ID NO: 6, 31, 49, 65, 67, 78, 80, 86, 102, 116, 122, 154, 159, 181 or 199; (c) a polynucleotide comprising a nucleotide sequence that encodes a polypeptide that shares at least 50% sequence similarity with at least a portion of the sequence set forth in SEQ ID NO: 6, 31, 49, 65, 67, 78, 80, 86, 102, 116, 122, 154, 159, 181 or 199, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.
15. A method according to paragraph 4, comprising measuring the level or functional activity of individual expression products of at least 1 level four correlation stress marker gene selected from: (a) a polynucleotide comprising a nucleotide sequence that shares at least 50% sequence identity with the sequence set forth in any one of SEQ ID NO: 7, 15, 16, 19, 21, 24, 25, 26, 35, 38, 39, 42, 46, 57, 68, 73, 81, 83, 97, 99, 107, 113, 123, 160, 165, 175, 187, 188, 194, 195 or 200, or a complement thereof; (b) a polynucleotide comprising a nucleotide sequence that encodes a polypeptide comprising the amino acid sequence set forth in any one of SEQ ID NO: 20, 22, 27, 29, 36, 42, 43, 58, 69, 74, 82, 84, 98, 100, 108, 114, 124, 166, 189 or 201; (c) a polynucleotide comprising a nucleotide sequence that encodes a polypeptide that shares at least 50% sequence similarity with at least a portion of the sequence set forth in SEQ ID NO: 20, 22, 27, 29, 36, 42, 43, 58, 69, 74, 82, 84, 98, 100, 108, 114, 124, 166, 189 or 201, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.
16. A method according to paragraph 4, comprising measuring the level or functional activity of individual expression products of at least 1 level five correlation stress marker gene selected from: (a) a polynucleotide comprising a nucleotide sequence that shares at least 50% sequence identity with the sequence set forth in any one of SEQ ID NO: 71, 3, 9, 11, 13, 32, 33, 34, 40, 41, 50, 51, 56, 59, 62, 63, 71, 75, 87, 93, 105, 111, 119, 127, 129, 130, 131, 137, 139, 141, 143, 145, 156, 161, 167, 169, 171, 173, 176, 185, 204 or 210, or a complement thereof; (b) a polynucleotide comprising a nucleotide sequence that encodes a polypeptide comprising the amino acid sequence set forth in any one of SEQ ID NO: 2, 4, 12, 14, 60, 61, 72, 76, 88, 94, 106, 112, 120, 128, 132, 138, 140, 142, 146, 157, 162, 168, 172, 174, 177, 205 or 211; (c) a polynucleotide comprising a nucleotide sequence that encodes a polypeptide that shares at least 50% sequence similarity with at least a portion of the sequence set forth in SEQ ID NO: 2, 4, 12, 14, 60, 61, 72, 76, 88, 94, 106, 112, 120, 128, 132, 138, 140, 142, 146, 157, 162, 168, 172, 174, 177, 205 or 211, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.
17. A method according to paragraph 4, wherein the biological sample comprises blood.
18. A method according to paragraph 4, wherein the biological sample comprises peripheral blood.
19. A method according to paragraph 4, wherein the biological sample comprises leukocytes.
20. A method according to paragraph 4, wherein the expression product is a RNA molecule.
21. A method according to paragraph 4, wherein the expression product is a polypeptide.
22. A method according to paragraph 4, wherein the expression product is the same as the corresponding expression product.
23. A method according to paragraph 4, wherein the expression product is a variant of the corresponding expression product.
24. A method according to paragraph 4, wherein the expression product or corresponding expression product is a target RNA or a DNA copy of the target RNA whose level is measured using at least one nucleic acid probe that hybridizes under at least low stringency conditions to the target RNA or to the DNA copy, wherein the nucleic acid probe comprises at least 15 contiguous nucleotides of a stress marker polynucleotide.
25. A method according to paragraph 24, wherein the measured level or abundance of the target RNA or its DNA copy is normalized to the level or abundance of a reference RNA or a DNA copy of the reference RNA that is present in the same sample.
26. A method according to paragraph 24, wherein the nucleic acid probe is immobilized on a solid or semi-solid support.
27. A method according to paragraph 24, wherein the nucleic acid probe forms part of a spatial array of nucleic acid probes.
28. A method according to paragraph 24, wherein the level of nucleic acid probe that is bound to the target RNA or to the DNA copy is measured by hybridization.
29. A method according to paragraph 24, wherein the level of nucleic acid probe that is bound to the target RNA or to the DNA copy is measured by nucleic acid amplification.
30. A method according to paragraph 24, wherein the level of nucleic acid probe that is bound to the target RNA or to the DNA copy is measured by nuclease protection assay.
31. A method according to paragraph 24, wherein the probe for detecting the stress marker polynucleotide comprises a sequence as set forth in any one oof SEQ ID NO: 250-1807.
32. A method according to paragraph 24, wherein the expression product or corresponding expression product is a target polypeptide whose level is measured using at least one antigen-binding molecule that is immuno-interactive with the target polypeptide.
33. A method according to paragraph 24, wherein the measured level of the target polypeptide is normalized to the level of a reference polypeptide that is present in the same sample.
34. A method according to paragraph 24, wherein the antigen-binding molecule is immobilized on a solid or semi-solid support.
35. A method according to paragraph 24, wherein the antigen-binding molecule forms part of a spatial array of antigen-binding molecule.
36. A method according to paragraph 24, wherein the level of antigen-binding molecule that is bound to the target polynucleotide is measured by immunoassay.
37. A method according to paragraph 4, wherein the expression product or corresponding expression product is a target polypeptide whose level is measured using at least one substrate for the target polypeptides with which it reacts to produce a reaction product.
38. A method according to paragraph 37, wherein the measured functional activity of the target polypeptide is normalized to the functional activity of a reference polypeptide that is present in the same sample.
39. A method according to paragraph 4, wherein a system is used to perform the method, which comprises at least one end station coupled to a base station, wherein the base station is caused (a) to receive subject data from the end station *via* a communications network, wherein the subject data represents parameter values corresponding to the measured or normalized level or functional activity of at least one expression product in the biological sample, and (b) to compare the subject data with predetermined data representing the measured or normalized level or functional activity of at least one corresponding expression product in the reference sample to thereby determine any difference in the level or functional activity of the expression product in the biological sample as compared to the level or functional activity of the corresponding expression product in the reference sample.
40. A method according to paragraph 39, wherein the base station is further caused to provide a diagnosis for the presence, absence or degree of a physiological response to stress.
41. A method according to paragraph 43, wherein the base station is further caused to transfer an indication of the diagnosis to the end station via the communications network.
42. A method according to paragraph 1, wherein detection of the aberrant expression is indicative of the presence or risk of a physiological response to stress.
43. A method according to paragraph 1, wherein the test subject is a horse.
44. A method for treating, preventing or inhibiting the development of stress in a subject, the method comprising detecting aberrant expression of at least one stress marker gene in the subject, and administering to the subject an effective amount of an agent that treats or ameliorates the symptoms or reverses or inhibits the development of stress in the subject, wherein the stress marker gene is selected from the group consisting of: (a) a gene having a polynucleotide expression product comprising a nucleotide sequence that shares at least 50% sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof; (b) a gene having a polynucleotide expression product comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a gene having a polynucleotide expression product comprising a nucleotide sequence that encodes a polypeptide that shares at least 50% sequence similarity with at least a portion of the sequence set forth in SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 15 contiguous amino acid residues of that sequence; and (d) a gene having a polynucleotide expression product comprising a nucleotide sequence that hybridizes to the sequence of (a), (b), (c) or a complement thereof, under at least low stringency conditions.
45. An isolated stress marker polynucleotide selected from (a) a polynucleotide comprising a nucleotide sequence that shares at least 50% sequence identity with the sequence set forth in any one of SEQ ID NO: 15, 16, 23, 24, 25, 28, 29, 32, 33, 34, 37, 38, 39, 40, 41, 50, 51, 54, 55, 62, 63, 89, 90, 91, 92, 95, 96, 107, 108, 117, 118, 125, 126, 129, 130, 143, 144, 147, 150, 155, 163, 164, 169, 170, 175, 184, 185, 186, 187, 194, 195, 232, 233, 238, 239, 240, 241, 242, or 243, or a complement thereof; (b) a polynucleotide comprising a portion of the sequence set forth in any one of SEQ ID NO: 15, 16, 23, 24, 25, 28, 29, 32, 33, 34, 37, 38, 39, 40, 41, 50, 51, 54, 55, 62, 63, 89, 90, 91, 92, 95, 96, 107, 108, 117, 118, 125, 126, 129, 130, 143, 144, 147, 150, 155, 163, 164, 169, 170, 175, 184, 185, 186, 187, 194, 195, 232, 233, 238, 239, 240, 241, 242, or 243, or a complement thereof, wherein the portion comprises at least 15 contiguous nucleotides of that sequence or complement; (c) a polynucleotide that hybridizes to the sequence of (a) or (b) or a complement thereof, under at least low stringency conditions; and (d) a polynucleotide comprising a portion of any one of SEQ ID NO: 15, 16, 23, 24, 25, 28, 29, 32, 33, 34, 37, 38, 39, 40, 41, 50, 51, 54, 55, 62, 63, 89, 90, 91, 92, 95, 96, 107, 108, 117, 118, 125, 126, 129, 130, 143, 144, 147, 150, 155, 163, 164, 169, 170, 175, 184, 185, 186, 187, 194, 195, 232, 233, 238, 239, 240, 241, 242, or 243, or a complement thereof, wherein the portion comprises at least 15 contiguous nucleotides of that sequence or complement and hybridizes to a sequence of (a), (b) or (c), or a complement thereof, under at least low stringency conditions.
46. A nucleic acid construct comprising a stress marker polynucleotide as claimed in paragraph 45, in operable connection with a regulatory element that is operable in a host cell.
47. An isolated host cell containing a nucleic acid construct as claimed in paragraph 46.
48. A probe comprising a nucleotide sequence that hybridizes under at least low stringency conditions to a polynucleotide as claimed in paragraph 45.
49. A probe as claimed in paragraph 48, wherein the probe comprise a nucleotide sequence that is capable of hybridizing to at least a portion of any one of SEQ ID NO: 15, 16, 23, 24, 25, 28, 29, 32, 33, 34, 37, 38, 39, 40, 41, 50, 51, 54, 55, 62, 63, 89, 90, 91, 92, 95, 96, 107, 108, 117, 118, 125, 126, 129, 130, 143, 144, 147, 150, 155, 163, 164, 169, 170, 175, 184, 185, 186, 187, 194, 195, 232, 233, 238, 239, 240, 241, 242 or 243 under at least low stringency conditions, wherein the portion is at least 15 nucleotides in length.
50. A probe as claimed in paragraph 49, comprising a sequence as set forth in any one of SEQ ID NO: 250-1807.
51. A solid or semi-solid support comprising at least one probe as claimed in paragraph 48 immobilized thereon.
52. Use of:
   (i) one or more stress marker polynucleotides selected from the group consisting of (a) a polynucleotide comprising a nucleotide sequence that shares at least 50% (and at least 51% to at least 99% and all integer percentages in between) sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof; (b) a polynucleotide comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at 15 contiguous amino acid residues of that sequence; and (d) a polynucleotide comprising a nucleotide sequence that hybridizes to the sequence of (a), (b), (c) or a complement thereof, under at least low stringency conditions; or
   (ii) one or more probes comprising a nucleotide sequence that hybridizes under at least low stringency conditions to a stress marker polynucleotide according to (i); or
   (iii) one or more stress marker polypeptides selected from the group consisting of: (1) a polypeptide comprising an amino acid sequence that shares at least 50% (and at least 51% to at least 99% and all integer percentages in between) sequence similarity with the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (2) a polypeptide comprising a portion of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 5 contiguous amino acid residues of that sequence; (3) a polypeptide comprising an amino acid sequence that shares at least 30% (and at least 31% to at least 99% and all integer percentages in between) similarity with at least 15 contiguous amino acid residues of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; and (4) a polypeptide comprising a portion of the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 5 contiguous amino acid residues of that sequence and is immuno-interactive with an antigen-binding molecule that is immuno-interactive with a sequence of (1), (2) or (3); or
   (iv) one or more antigen-binding molecules that are immuno-interactive with a stress marker polypeptide according to (iii),
   In the manufacture of a kit for diagnosing the presence of a physiological response to stress in a subject.
53. A method according to paragraph 52, wherein the physiological response to stress is selected from the group consisting of: physical stress, mood disorders, anxiety disorders, inflammation; pain; chronic fatigue syndrome; stress-induced headache; cancer; human immunodeficiency virus (HIV) infections; neurodegenerative diseases; gastrointestinal diseases; eating disorders; supranuclear palsy; amyotrophic lateral sclerosis; a decrease in immune function or immunosuppression; hemorrhagic stress; stress-induced psychotic episodes; euthyroid sick syndrome; syndrome of inappropriate antidiarrhetic hormone (ADH); overeating or obesity; infertility; head traumas; spinal cord trauma; ischemic neuronal damage; excitotoxic neuronal damage; epilepsy; cardiovascular disorders; stroke; immune dysfunctions; restraint; behavioural (operant) conditioning; muscular spasms; urinary incontinence; senile dementia of the Alzheimer's type; multiinfarct dementia; amyotrophic lateral sclerosis; chemical dependencies and addictions; drug and alcohol withdrawal symptoms; osetoporosis; psychosocial dwarfism; hypoglycaemia; hair loss; abnormal circadian rhythm; and disorders related to abnormal circadian rhythm.
54. A method for diagnosing the presence of a physiological response to stress in a test subject, comprising detecting in the test subject aberrant expression of at least one stress marker polynucleotide selected from the group consisting of: (a) a polynucleotide comprising a nucleotide sequence that shares at least 50% sequence identity with the sequence set forth in any one of SEQ ID NO: SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof; (b) a polynucleotide comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a polynucleotide comprising a nucleotide sequence that encodes a polypeptide that shares at least 50% sequence similarity with at least a portion of the sequence set forth in SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249, wherein the portion comprises at least 15 contiguous amino acid residues of that sequence; and (d) a polynucleotide comprising a nucleotide sequence that hybridizes to the sequence of (a), (b), (c) of a complement thereof, under at least low stringency conditions.

## Claims

1. A method for monitoring immune function in a subject, the method comprising:
- comparing expression of at least one stress marker gene in a leukocyte sample obtained from the subject to expression of at least one corresponding stress marker gene in a control leukocyte sample obtained from a normal subject or from a subject not under stress, wherein a difference in the expression between the sample and the control indicates presence of stress and reduced immune function (or immunosuppression), wherein a similarity in the expression between the sample and the control indicates absence of stress and normal immune function and wherein the at least one stress marker gene is selected from the group consisting of: (a) a gene having a polynucleotide expression product comprising a nucleotide sequence that shares at least 90% sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof; (b) a gene having a polynucleotide expression product comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a gene having a polynucleotide expression product comprising a nucleotide sequence that encodes a polypeptide that shares at least 90% sequence similarity with the sequence set forth in SEQ ID NO: 22, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; and (d) a gene having a polynucleotide expression product comprising a nucleotide sequence that hybridizes to the sequence of (a), (b), (c) or a complement thereof, under high stringency conditions.

2. Use of at least one stress marker gene in a method for treating or preventing the development of immunosuppression in a subject, wherein the method comprises:
- comparing expression of at least one stress marker gene in a leukocyte sample obtained from the subject to expression of at least one corresponding stress marker gene in a control leukocyte sample obtained from a normal subject or from a subject not under stress, wherein a difference in the expression between the sample and the control indicates presence of stress and immunosuppression, wherein a similarity in the expression between the sample and the control indicates absence of stress and normal immune function; and
- on the basis that the sample tests positive for stress and immunosuppression, managing the environment of the subject to prevent or minimize exposure of the subject to a causative stressor and/or administering to the subject an effective amount of an agent that treats or ameliorates the symptoms or reverses or inhibits the development of stress in the subject,
wherein the at least one stress marker gene is selected from the group consisting of:
(a) a gene having a polynucleotide expression product comprising a nucleotide sequence that shares at least 90% sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof;
(b) a gene having a polynucleotide expression product comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a gene having a polynucleotide expression product comprising a nucleotide sequence that encodes a polypeptide that shares at least 90% sequence similarity with the sequence set forth in SEQ ID NO: 22, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; and (d) a gene having a polynucleotide expression product comprising a nucleotide sequence that hybridizes to the sequence of (a), (b), (c) or a complement thereof, under high stringency conditions.

3. Use of at least one stress marker gene in a method for assessing the capacity of the immune system of a subject to produce an immunogenic response to a selected antigen, wherein the method comprises:
- comparing expression of at least one stress marker gene in a leukocyte sample obtained from the subject to expression of at least one corresponding stress marker gene in a control leukocyte sample obtained from a normal subject or from a subject not under stress; and
- determining that the subject has a normal capacity to produce the immunogenic response when a similarity in the expression between the sample and the control is detected, or
- determining that the subject has an impaired capacity to produce the immunogenic response when a difference in the expression between the sample and the control is detected,
wherein the at least one stress marker gene is selected from the group consisting of:
(a) a gene having a polynucleotide expression product comprising a nucleotide sequence that shares at least 90% sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof;
(b) a gene having a polynucleotide expression product comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a gene having a polynucleotide expression product comprising a nucleotide sequence that encodes a polypeptide that shares at least 90% sequence similarity with the sequence set forth in SEQ ID NO: 22, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; and (d) a gene having a polynucleotide expression product comprising a nucleotide sequence that hybridizes to the sequence of (a), (b), (c) or a complement thereof, under high stringency conditions.

4. Use of at least one stress marker gene in a method for eliciting an immune response to a selected antigen in a subject via administration of a composition comprising the antigen, wherein the method comprises:
- comparing expression of at least one stress marker gene in a leukocyte sample obtained from the subject to expression of at least one corresponding stress marker gene in a control leukocyte sample obtained from a normal subject or from a subject not under stress;
- determining that the subject has a normal capacity to produce the immunogenic response when a similarity in the expression between the sample and the control is detected, or
- determining that the subject has an impaired capacity to produce the immunogenic response when a difference in the expression between the sample and the control is detected; and
- on the basis that the sample tests positive for normal capacity to produce the immunogenic response, administering the composition to the subject, or
- on the basis that the sample tests positive for impaired capacity to produce the immunogenic response, managing the environment of the subject to prevent or minimize exposure of the subject to a causative stressor and/or administering to the subject an effective amount of an agent that reverses or inhibits the development of stress in the subject, and administering the composition to the subject,
wherein the at least one stress marker gene is selected from the group consisting of:
(a) a gene having a polynucleotide expression product comprising a nucleotide sequence that shares at least 90% sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof;
(b) a gene having a polynucleotide expression product comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a gene having a polynucleotide expression product comprising a nucleotide sequence that encodes a polypeptide that shares at least 90% sequence similarity with the sequence set forth in SEQ ID NO: 22, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; and (d) a gene having a polynucleotide expression product comprising a nucleotide sequence that hybridizes to the sequence of (a), (b), (c) or a complement thereof, under high stringency conditions.

5. Use of at least one stress marker gene in a method for improving an immune response to a selected antigen in a subject to whom/which has been administered a composition comprising the antigen, wherein the method comprises:
- comparing expression of at least one stress marker gene in a leukocyte sample obtained from the subject to expression of at least one corresponding stress marker gene in a control leukocyte sample obtained from a normal subject or from a subject not under stress;
- determining that the subject has a normal capacity to produce the immunogenic response when a similarity in the expression between the sample and the control is detected, or
- determining that the subject has an impaired capacity to produce the immunogenic response when a difference in the expression between the sample and the control is detected; and
- on the basis that the sample tests positive for impaired capacity to produce the immunogenic response, managing the environment of the subject to prevent or minimize exposure of the subject to a causative stressor and/or administering to the subject an effective amount of an agent that reverses or inhibits the development of stress in the subject, whereby the management or administration leads to normal expression of the or each stress marker gene in the subject.

6. A method or use according to any one of claims 1 to 5, comprising: (1) measuring in the sample the level or functional activity of an expression product of the at least one stress marker gene and (2) comparing the measured level or functional activity of each expression product to the level or functional activity of a corresponding expression product in the control.

7. A method use according to claim 6, wherein the expression product is a stress marker polynucleotide selected from the group consisting of (a) a polynucleotide comprising a nucleotide sequence that shares at least 90% sequence identity with the sequence set forth in any one of SEQ ID NO: 1, 3, 4, 5, 7, 9, 11, 13, 15, 16, 17, 19, 21, 23, 24, 25, 26, 28, 29, 30, 32, 33, 34, 35, 37, 38, 39, 40, 41, 42, 44, 46, 48, 50, 51, 52, 54, 55, 56, 57, 59, 62, 63, 64, 66, 68, 70, 71, 73, 75, 77, 79, 81, 83, 85, 87, 89, 90, 91, 92, 93, 95, 96, 97, 99, 101, 103, 105, 107, 108, 109, 111, 113, 115, 117, 118, 119, 121, 123, 125, 126, 127, 129, 130, 131, 133, 135, 137, 139, 141, 143, 144, 145, 147, 148, 150, 151, 153, 155, 156, 158, 160, 161, 163, 164, 165, 167, 169, 170, 171, 173, 175, 176, 178, 180, 182, 184, 185, 186, 187, 188, 190, 192, 194, 195, 196, 198, 200, 202, 204, 206, 208, 210, 212, 214, 216, 218, 220, 222, 224, 226, 228, 230, 232, 233, 234, 236, 238, 239, 240, 241, 242, 243, 244, 246 or 248, or a complement thereof; (b) a polynucleotide comprising a nucleotide sequence that encodes a polypeptide comprising the amino acid sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; (c) a polynucleotide comprising a nucleotide sequence that encodes a polypeptide that shares at least 90% sequence similarity with the sequence set forth in SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249; and (d) a polynucleotide comprising a nucleotide sequence that hybridizes to the sequence of (a), (b), (c) or a complement thereof, under high stringency conditions.

8. A method or use according to claim 6,whererin the expression product is a stress marker polypeptide comprising an amino acid sequence that shares at least 90% sequence similarity with the sequence set forth in any one of SEQ ID NO: 2, 6, 8, 10, 12, 14, 18, 20, 22, 27, 31, 36, 43, 45, 47, 49, 53, 58, 60, 61, 65, 67, 69, 72, 74, 76, 78, 80, 82, 84, 86, 88, 94, 98, 100, 102, 104, 106, 110, 112, 114, 116, 120, 122, 124, 128, 132, 134, 136, 138, 140, 142, 146, 149, 152, 154, 157, 159, 162, 166, 168, 172, 174, 177, 179, 181, 183, 189, 191, 193, 197, 199, 201, 203, 205, 207, 209, 211, 213, 215, 217, 219, 221, 223, 225, 227, 229, 231, 235, 237, 245, 247 or 249.

9. A method or use according to claim 6, wherein the presence of stress or immunosupression or impaired capacity to produce the immunogenic response is indicated when the measured level or functional activity of the or each expression product is 10% lower than the measured level or functional activity of the or each corresponding expression product.

10. A method or use according to claim 6, wherein the presence of stress or immunosupression or impaired capacity to produce the immunogenic response is determined by detecting a decrease in the sample relative to the control in the level or functional activity of at least one stress marker polynucleotide selected from (a) a polynucleotide comprising a nucleotide sequence that shares at least 90% sequence identity with the sequence set forth in any one of SEQ ID NO: 1, 3, 4, 7, 9, 11, 19, 21, 24, 25, 33, 34, 38, 39, 40, 41, 42, 50, 51, 56, 57, 59, 62, 63, 66, 70, 71, 73, 75, 79, 81, 83, 89, 90, 91, 92, 93, 97, 99, 105, 107, 108, 111, 119, 121, 122, 123, 129, 130, 137, 139, 140, 141, 142, 143 or 185, or a complement thereof; (b) a polynucleotide comprising a nucleotide sequence that encodes a polypeptide comprising the amino acid sequence set forth in any one of SEQ ID NO: 2, 8, 10, 12, 20, 22, 43, 58, 60, 67, 71, 72, 74, 76, 80, 82, 84, 94, 98, 100, 106, 112, 120, 122, 123, 124 or 138; (c) a polynucleotide comprising a nucleotide sequence that encodes a polypeptide that shares at least 90% sequence similarity with the sequence set forth in SEQ ID NO: 2, 8, 10, 12, 20, 22, 43, 58, 60, 67, 71, 72, 74, 76, 80, 82, 84, 94, 98, 100, 106, 112, 120, 122, 123, 124 or 138; and (d) a polynucleotide comprising a nucleotide sequence that hybridizes to the sequence of (a), (b), (c) or a complement thereof, under high stringency conditions.

11. A method or use according to claim 6, wherein the presence of stress or immunosupression or impaired capacity to produce the immunogenic response is indicated when the measured level or functional activity of the or each expression product is 10% higher than the measured level or functional activity of the or each corresponding expression product.

12. A method or use according to claim 6, wherein the presence of stress or immunosupression or impaired capacity to produce the immunogenic response is determined by detecting an increase in the sample relative to the control in the level or functional activity of at least one stress marker polynucleotide selected from (a) a polynucleotide comprising a nucleotide sequence that shares at least 90% sequence identity with the sequence set forth in any one of SEQ ID NO: 5, 13, 15, 16, 17, 23, 26, 28, 29, 30, 32, 35, 37, 44, 46, 48, 52, 54, 55, 64, 68, 77, 85, 87, 95, 96, 101, 103, 113, 115, 117, 118, 125, 126, 131, 133, 135, 144, 145, 147, 148, 150, 151, 153, 155, 156, 158, 160, 161, 163, 164, 165, 167, 169, 170, 171, 173, 175, 176, 178, 180, 182, 183, 184, 186, 187, 188, 190, 192, 194, 195, 196, 198, 200, 202, 204, 206 or 210, or a complement thereof; (b) a polynucleotide comprising a nucleotide sequence that encodes a polypeptide comprising the amino acid sequence set forth in any one of SEQ ID NO: 6, 14, 18, 27, 31, 36, 45, 47, 49, 53, 65, 69, 78, 86, 88, 102, 104, 114, 116, 132, 134, 136, 146, 149, 152, 154, 157, 159, 162, 166, 168, 172, 174, 177, 179, 181, 189, 191, 193, 197, 199, 201, 203, 205, 207 or 211; (c) a polynucleotide comprising a nucleotide sequence that encodes a polypeptide that shares at least 90% sequence similarity with the sequence set forth in SEQ ID NO: 6, 14, 18, 27, 31, 36, 45, 47, 49, 53, 65, 69, 78, 86, 88, 102, 104, 114, 116, 132, 134, 136, 146, 149, 152, 154, 157, 159, 162, 166, 168, 172, 174, 177, 179, 181, 189, 191, 193, 197, 199, 201, 203, 205, 207 or 211; and (d) a polynucleotide comprising a nucleotide sequence that hybridizes to the sequence of (a), (b), (c) or a complement thereof, under high stringency conditions.

13. A method or use according to claim 7, wherein the absence of stress or immunosupression or impaired capacity to produce the immunogenic response or the presence of normal immune function or normal capacity to produce the immunogenic response is indicated when the measured level or functional activity of the or each expression product is the same as or similar to the measured level or functional activity of the or each corresponding expression product.

14. A method or use according to claim 13, wherein the measured level or functional activity of an individual expression product varies from the measured level or functional activity of an individual corresponding expression product by no more than about 5%.

15. A method or use according to claim 6, comprising measuring the level or functional activity of individual expression products of at least about 2 stress marker genes.

16. A method or use according to claim 6, comprising measuring the level or functional activity of individual expression products of at least 1 level one correlation stress marker gene selected from: (a) a polynucleotide comprising a nucleotide sequence that shares at least 90% sequence identity with the sequence set forth in any one of SEQ ID NO: 89, 90, 103, 125, 126, 163, 178, 182, 184 or 190, or a complement thereof; (b) a polynucleotide comprising a nucleotide sequence that encodes a polypeptide comprising the amino acid sequence set forth in any one of SEQ ID NO: 104, 179, 183 or 189; (c) a polynucleotide comprising a nucleotide sequence that encodes a polypeptide that shares at least 90% sequence similarity with the sequence set forth in SEQ ID NO: 104, 179, 183 or 189; and (d) a polynucleotide comprising a nucleotide sequence that hybridizes to the sequence of (a), (b), (c) or a complement thereof, under high stringency conditions.

17. A method or use according to claim 6, comprising measuring the level or functional activity of individual expression products of at least 1 level two correlation stress marker gene selected from: (a) a polynucleotide comprising a nucleotide sequence that shares at least 90% sequence identity with the sequence set forth in any one of SEQ ID NO: 17, 23, 44, 52, 133, 135, 144, 147, 148, 151, 155, 192, 196, 202 or 206, or a complement thereof; (b) a polynucleotide comprising a nucleotide sequence that encodes a polypeptide comprising the amino acid sequence set forth in any one of SEQ ID NO: 18, 20, 45, 53, 134, 136, 149, 152, 193, 197 or 207; (c) a polynucleotide comprising a nucleotide sequence that encodes a polypeptide that shares at least 90% sequence similarity with the sequence set forth in SEQ ID NO: 18, 20, 45, 53, 134, 136, 149, 152, 193, 197 or 207; and (d) a polynucleotide comprising a nucleotide sequence that hybridizes to the sequence of (a), (b), (c) or a complement thereof, under high stringency conditions.

18. A method or use according to claim 6, comprising measuring the level or functional activity of individual expression products of at least 1 level three correlation stress marker gene selected from: (a) a polynucleotide comprising a nucleotide sequence that shares at least 90% sequence identity with the sequence set forth in any one of SEQ ID NO: 5, 30, 37, 48, 54, 55, 64, 66, 70, 77, 79, 85, 91, 92, 95, 96, 101, 115, 117, 118, 121, 150, 153, 158, 164, 170, 180, 186 or 198, or a complement thereof; (b) a polynucleotide comprising a nucleotide sequence that encodes a polypeptide comprising the amino acid sequence set forth in any one of SEQ ID NO: 6, 31, 49, 65, 67, 78, 80, 86, 102, 116, 122, 154, 159, 181 or 199; (c) a polynucleotide comprising a nucleotide sequence that encodes a polypeptide that shares at least 90% sequence similarity with the sequence set forth in SEQ ID NO: 6, 31, 49, 65, 67, 78, 80, 86, 102, 116, 122, 154, 159, 181 or 199; and (d) a polynucleotide comprising a nucleotide sequence that hybridizes to the sequence of (a), (b), (c) or a complement thereof, under high stringency conditions.

19. A method or use according to claim 6, comprising measuring the level or functional activity of individual expression products of at least 1 level four correlation stress marker gene selected from: (a) a polynucleotide comprising a nucleotide sequence that shares at least 90% sequence identity with the sequence set forth in any one of SEQ ID NO: 7, 15, 16, 19, 21, 24, 25, 26, 28, 35, 38, 39, 42, 46, 57, 68, 73, 81, 83, 97, 99, 107, 113, 123, 160, 165, 175, 187, 188, 194, 195 or 200, or a complement thereof; (b) a polynucleotide comprising a nucleotide sequence that encodes a polypeptide comprising the amino acid sequence set forth in any one of SEQ ID NO: 20, 22, 27, 29, 36, 42, 43, 58, 69, 74, 82, 84, 98, 100, 108, 114, 124, 166, 189 or 201; (c) a polynucleotide comprising a nucleotide sequence that encodes a polypeptide that shares at least 90% sequence similarity with the sequence set forth in SEQ ID NO: 20, 22, 27, 29, 36, 42, 43, 58, 69, 74, 82, 84, 98, 100, 108, 114, 124, 166, 189 or 201; and (d) a polynucleotide comprising a nucleotide sequence that hybridizes to the sequence of (a), (b), (c) or a complement thereof, under high stringency conditions.

20. A method or use according to claim 6, comprising measuring the level or functional activity of individual expression products of at least 1 level five correlation stress marker gene selected from: (a) a polynucleotide comprising a nucleotide sequence that shares at least 90% sequence identity with the sequence set forth in any one of SEQ ID NO: 71, 3, 9, 11, 13, 32, 33, 34, 40, 41, 50, 51, 56, 59, 62, 63, 71, 75, 87, 93, 105, 111, 119, 127, 129, 130, 131, 137, 139, 141, 143, 145, 156, 161, 167, 169, 171, 173, 176, 185, 204 or 210, or a complement thereof; (b) a polynucleotide comprising a nucleotide sequence that encodes a polypeptide comprising the amino acid sequence set forth in any one of SEQ ID NO: 2, 4, 12, 14, 60, 61, 72, 76, 88, 94, 106, 112, 120, 128, 132, 138, 140, 142, 146, 157, 162, 168, 172, 174, 177, 205 or 211; (c) a polynucleotide comprising a nucleotide sequence that encodes a polypeptide that shares at least 90% sequence similarity with the sequence set forth in SEQ ID NO: 2, 4, 12, 14, 60, 61, 72, 76, 88, 94, 106, 112, 120, 128, 132, 138, 140, 142, 146, 157, 162, 168, 172, 174, 177, 205 or 211; and (d) a polynucleotide comprising a nucleotide sequence that hybridizes to the sequence of (a), (b), (c) or a complement thereof, under high stringency conditions.

21. A method or use according to any preceding claim, wherein the sample comprises blood.

22. A method or use according to any preceding claim, wherein the sample comprises peripheral blood.

23. A method or use according to any preceding claim, wherein the expression product is a RNA molecule.

24. A method or use according to any preceding claim, wherein the expression product is a polypeptide.

25. A method or use according to claim 6, wherein the expression product is the same as the corresponding expression product.

26. A method or use accordinp to claim 6, wherein the expression product is a variant of the corresponding expression product.

27. A method or use according to claim 6, wherein the expression product or corresponding expression product is a target RNA or a DNA copy of the target RNA whose level is measured using at least one nucleic acid probe that hybridizes under high stringency conditions to the target RNA or to the DNA copy, wherein the nucleic acid probe comprises at least 15 contiguous nucleotides of a stress marker polynucleotide.

28. A method or use according to claim 27, wherein the measured level or abundance of the target RNA or its DNA copy is normalized to the level or abundance of a reference RNA or a DNA copy of the reference RNA that is present in the same sample.

29. A method or use according to claim 27, wherein the nucleic acid probe is immobilized on a solid or semi-solid support.

30. A method or use according to claim 27, wherein the nucleic acid probe forms part of a spatial array of nucleic acid probes.

31. A method or use according to claim 27, wherein the level of nucleic acid probe that is bound to the target RNA or to the DNA copy is measured by hybridization.

32. A method or use according to claim 27, wherein the level of nucleic acid probe that is bound to the target RNA or to the DNA copy is measured by nucleic acid amplification.

33. A method or use according to claim 27, wherein the level of nucleic acid probe that is bound to the target RNA or to the DNA copy is measured by nuclease protection assay.

34. A method or use according to claim 27, wherein the probe for detecting the stress marker polynucleotide comprises a sequence as set forth in any one of SEQ ID NO: 250-1807.

35. A method or use according to claim 6, wherein the expression product or corresponding expression product is a target polypeptide whose level is measured using at least one antigen-binding molecule that is immuno-interactive with the target polypeptide.

36. A method or use according to claim 35, wherein the measured level of the target polypeptide is normalized to the level of a reference polypeptide that is present in the same sample.

37. A method or use according to claim 35, wherein the antigen-binding molecule is immobilized on a solid or semi-solid support.

38. A method or use according to claim 35, wherein the antigen-binding molecule forms part of a spatial array of antigen-binding molecule.

39. A method or use according to claim 35, wherein the level of antigen-binding molecule that is bound to the target polypeptide is measured by immunoassay.

40. A method or use according to claim 6, wherein the expression product or corresponding expression product is a target polypeptide whose level is measured using at least one substrate for the target polypeptide with which it reacts to produce a reaction product.

41. A method or use according to claim 40, wherein the measured functional activity of the target polypeptide is normalized to the functional activity of a reference polypeptide that is present in the same sample.
